# EUROPEAN PATENT APPLICATION

(11) **EP 2 520 585 A1**
(43) Date of publication of application: **07.11.2012**
(21) Application number: 11305540.4
(22) Date of filing: 06.05.2011
(51) Int. Cl.: C07K 14/44

(54) **Identification of region of RON2 of Apicomplexan parasites suitable for targeting by vaccines and drugs**

(71) Applicant: Centre National de la Recherche Scientifique (CNRS), 75016 Paris (FR); University Of Victoria Innovation And Development Corporation, Victoria, BC V8W 3W2 (CA)
(72) Inventor: Lebrun, Maryse, 34270 Saint Mathieu de Tréviers (FR); Tonkin, Michelle, Victoria, BC V8W 3N5 (CA); Boulanger, Martin, Victoria, BC V8W 3N5 (CA); Roques, Magali, 34090 Montpellier (FR)
(74) Representative: Faivre Petit, Frédérique

(57) **Abstract**

The present invention relates to Apicomplexan rhoptry neck protein 2 (RON2) polypeptide fragments, or homologous polypeptides thereof. The present invention is also directed to a composition comprising such polypeptides for their uses as a medicament, particularly for the prevention or the treatment of Apicomplexan parasite infection. The present invention also encompasses specific antibodies directed against these polypeptides, and method of diagnosis of Apicomplexan parasite infection implementing these polypeptides or antibodies. The invention further comprises a construct for its use in a method for crystallization of the Apicomplexan co-structure AMA1-RON2 complex. The present invention also relates to a computer-readable data storage material. Finally, the invention concerns a method for *in silico* or in *vitro* identifying or screening a potential inhibitor of vertebrate, preferably mammalian or avian, host cell invasion by an Apicomplexan parasite.

## Description

The present invention relates to Apicomplexan rhoptry neck protein 2 (RON2) polypeptide fragments, or homologous polypeptides thereof. The present invention is also directed to a composition comprising such polypeptides for their uses as a medicament, particularly for the prevention or the treatment of Apicomplexan parasite infection. The present invention also encompasses specific antibodies directed against these polypeptides, and method of diagnosis of Apicomplexan parasite infection implementing these polypeptides or antibodies. The invention further comprises a construct for its use in a method for crystallization of the Apicomplexan co-structure AMAI-RON2 complex. The present invention also relates to a computer-readable data storage material. Finally, the invention concerns a method for *in silico* or in *vitro* identifying or screening a potential inhibitor of vertebrate, preferably mammalian or avian, more preferably human host cell invasion by an Apicomplexan parasite.

Apicomplexan parasites cause widespread diseases including malaria (Plasmodium) and toxoplasmosis (Toxoplasma). The success of these obligate intracellular pathogens is largely due to their unique, conserved mechanism of active invasion (reviewed in¹) involving the coordinated secretion of proteins from the microneme and rhoptry organelles². Recently, the host cell invasion process was likened to a 'kiss and spit' mechanism³ where the parasite provides both ligand and receptor in a synchronised process⁴ reminiscent of the Intimin - Tir assembly used by enteropathogenic Escherichia coli⁵. It is noteworthy, however, that this is the first example of such a strategy employed by a eukaryotic or intracellular pathogen.

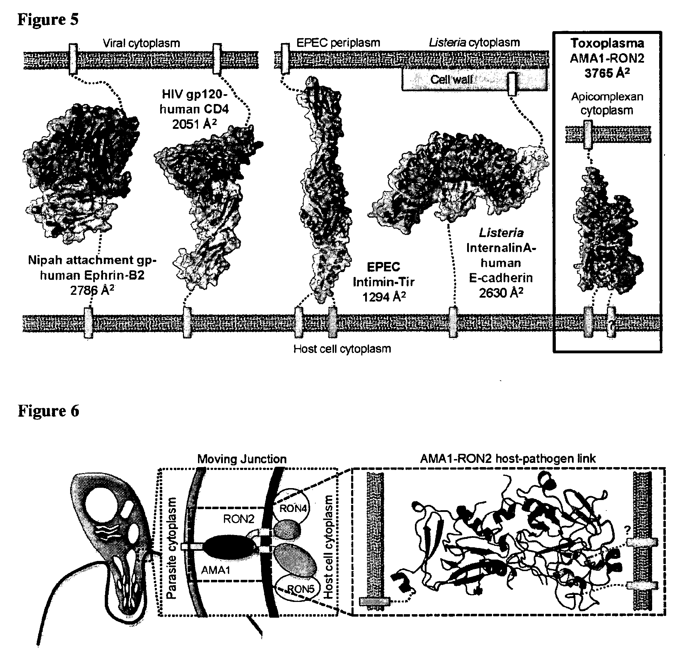

The molecular basis of active invasion by Apicomplexans relies on secretion of a rhoptry neck complex consisting of, at minimum, RONs 2, 4 and 5 into the host cell where RON2 is integrated into the host plasma membrane and RONs 4 and 5 are localized to its cytoplasmic face⁴. A surface exposed ectodomain of RON2 serves as the receptor⁶ for the multifunctional, micronemal secreted AMA¹⁷⁻¹² displayed on the parasite cell surface (Fig. 6). The assembled AMA1-RON complex forms a ring like structure known as the Moving Junction (MJ), which migrates from the anterior to the posterior of the parasite¹³ leading to internalization of the parasite into a parasitophorous vacuole (PV). In addition to linking the parasite and host cell membranes, the MJ acts as a diffusion barrier excluding host transmembrane proteins from the PV¹⁴, thereby preventing degradation of intracellular parasites¹⁵. Thus, the fate of the parasite in the host cell is largely dependent on its ability to actively invade in a MJ dependent process.

It is intriguing that AMA1 and RONs 2, 4 and 5 are conserved across Apicomplexan^{4,16-21}, suggesting the potential of a broadly conserved host cell invasion mechanism. In support of this prediction, we have recently shown that the AMA1-RON2 interaction is equally important for the invasive process by Toxoplasma gondii and Plasmodium falciparum⁶. Coincidentally, structural studies of TgAMA1 from our lab ²², Plasmodium vivax (PvAMA1)²³ and P. falciparum (PfAMA1)^{24,25} revealed a conserved hydrophobic trough in the ectodomain that displays structural hallmarks of a receptor binding surface and thus may be involved in coordinating the binding of RON2. Defining a detailed blueprint of MJ complex formation is therefore critical to both an in-depth understanding of host cell invasion by these important global pathogens, and the strategic design of vaccines and therapeutics.

Since the ectoplasmic region of AMA1 is a promising target for a vaccine candidate against Apicomplexan infection, it is important to understand the structure and the function of said AMA1-RON2 interaction.

Therefore, there is a need in the art to provide a crystal co-structure and to elucidate the three-dimensional co-structure of the AMA1-RON2 interaction of Apicomplexan parasites such as Toxoplasma or Plasmodial parasites and to use such co-structures or model structures in therapeutic strategies, such as drug design and/or to identify anti-Apicomplexan vaccine candidates capable of inhibiting the host cells infection in human. This is the object of the present invention.

The inventors have determined and report herein the comprehensive structural and functional characterization of the Apicomplexan parasite AMA1-RON2, such as TgAMA1-TgRON2 and PfAMA1-PfRON2 assembly from which the inventors have demonstrated mechanism of host cell invasion by Apicomplexan parasites.

So, in a first aspect, the present invention is directed to a purified or isolated polypeptide selected from the group of polypeptides consisting of the following polypeptides:
a)
   i) a polypeptide having at most a 75 amino-acid length and comprising the sequence of the rhoptry neck protein RON2-2 domain of an Apicomplexan parasite, said RON2-2 domain designating the region located between the two last putative transmembrane domains of RON2 peptide and extending into 14 amino-acids residues of the last transmembrane domain; or
   ii) a fragment of said polypeptide as defined in part a) i) above and comprising at least the subfragment of said RON2-2 peptide having the sequence comprised between the two cysteine residues located into said RON2-2 domain, cysteine residues inclusive; and
b) a polypeptide homologous to the polypeptide as defined in part a) above, said homologous polypeptide having a sequence presenting an identity of at least 50 %, preferably at least 55 %, 60 %, 65 %, 70 %, 80 %, 85 %, 90 %, 95 % or 97,5 %, with that RON2-2 domain and wherein at least two amino-acids residues capable of forming a disulfide bond are present.

Preferably, by putative transmembrane domain, it is intended to designate an hydrophobic domain that potentially span membrane, and extending into 14 amino-acids residues of last.

Such hydrophobic domain that potentially span membrane can be predicted by software wellknown by the skilled person, for example, but non limited to, the TopPred™ software (http://mobyle.pasteur.fr/cgi-bin/portal.py?#forms::toppred).

Preferably, said two amino-acids residues capable of forming a disulfide bond are separated by a fragment having 20, preferably 19, 18, 17, 16, 15, 14, 13, 12, 11, 10, 9, 8 or 7 amino-acids residues, length. More preferably separated by a fragment having 11, 10 or 9 amino-acids residues length.

Preferably, said RON2-2 domain is a domain having an amino-acids sequence selected from the group consisting of the sequences:
SEQ NO: 72: DIX₁QX₂₋₄DX₅GX₆₋₇PX₈₋₁₀CX₁₁₋₁₉CX₂₀₋₂₉; or
SEQ ID NO: 73: DIX₁QX₂₋₄DX₅GX₆₋₇PX₈₋₁₀CX₁₁₋₂₀CX₂₁₋₃₀; or
SEQ ID NO: 74: DIX₁QX₂₋₄DX₅GX₆₋₇PX₈₋₁₀CX₁₁₋₂₁CX₂₂₋₃₁, wherein two, preferably one, amino-acids residue selected from the group of the D, I, Q, D, G and P amino-acids residues respectivelly located at position 1, 2, 4, 8,10 and 13 of the sequence SEQ ID NO: 72, SEQ ID NO: 73 or SEQ ID NO: 74 can be any amino-acid residue, and
wherein X₁ to X₃₁ designates any amino acid (polypeptide amino-acids sequence representation in one letter code), or a fragment thereof or homologous polypeptide thereof as above defined in paragraph a) ii) and b).

More preferably, said RON2-2 domain is a domain having an amino-acids sequence selected from the group consisting of the sequences:
SEQ ID NO: 72: DIX₁QX₂₋₄DX₅GX₆₋₇PX₆₋₇PX₁₁₋₁₉CX₂₀₋₂₉; or
SEQ ID NO: 73: DIX₁QX₂₋₄DX₅GX₆₋₇PX₈₋₁₀CX₁₁₋₂₀CX₂₁₋₃₀; or
SEQ ID NO: 74: DIX₁QX₂₋₄DX₅GX₆₋₇PX₈₋₁₀CX₁₁₋₂₁CX₂₂₋₃₁,
wherein X₁ to X₃₁ designates any amino acid.

As used herein, the terms "polypeptide", "protein" and "peptide" are synonym

In a general manner, by amino acid sequence having at least 50 %, preferably, 59 %, 60 %, 65 %, 70 %, 80 %, 85 %, 90 %, 95 % or 97,5 % , identity with a reference amino acid sequence, those having, with respect to the reference sequence, certain modifications, in particular a deletion, addition or substitution of at least one amino acid, a truncation or an elongation are preferred. In the case of a substitution of one or more consecutive or nonconsecutive amino acid(s), the substitutions are preferred in which the substituted amino acids are replaced by "equivalent" amino acids. The expression "equivalent amino acids" is aimed here at indicating any amino acid capable of being substituted with one of the amino acids of the base structure without, however, essentially modifying the biological activities of the reference polypeptide and such as will be defined later, especially in the examples.

These equivalent amino acids can be determined either by relying on their structural homology with the amino acids which they replace, or on results of comparative trials of biological activity between the different polypeptides capable of being carried out.

Particularly, it is preferred that the homologous polypeptides according to the invention having amino acid sequence having at least 50 %, preferably, 59 %, 60 %, 65 %, 70 %, 80 %, 85 %, 90 %, 95 % or 97,5 % , identity with a reference amino acid sequence, are capable:
i) to specifically bind the AMA1 ectoplasmic domain, said AMA1 ectoplasmic domain comprising at least the subdomains DI and DII of the ectoplasmic domain of said AMA1 polypeptide, particularly by using the ELISA "binding assay" method as depicted in the examples herein (see Example 1); or
-ii) to inhibit the formation of the AMA1/RON2-2 complex resulting from the contact between a RON2-2 polypeptide and an AMA1 polypeptide fragment comprising at least the subdomains DI and DII of the ectoplasmic domain of said AMA1 polypeptide particularly by using the competitive ELISA assay as depicted in the examples herein (see Example 1, "binding assay "") or
- iii) to inhibit the host cells invasion by Apicomplexan parasite, particularly by using the "Invasion assay " method as depicted in the examples herein (see Examle 1);

By way of example, mention is made of the possibilities of substitution capable of being carried out without resulting in a profound modification of the biological activity of the reference RON2-2 (see paragraphs i), ii) and iii) above for at least one the preferred biological activities which has to be preserved. More preferably, at least 50 %, 60 %, 70 %, 80 %, 85 %, 90 %, 95 % or 100 % of the biological activity of the reference RON2-2 is preserved). Most preferably, more than 100 % of the biological activity found with the reference RON2-2 domain (or the wild type RON2-2 domain) is obtained wich such homologous polypeptides of the present invention.

For example, it is thus possible to replace leucine by valine or isoleucine, aspartic acid by glutamic acid, glutamine by asparagine, arginine by lysine, etc., the reverse substitutions being naturally envisageable under the same conditions.

By homologous polypeptides, it is also intended to designate mutated polypeptide having, with respect to the reference RON2-2 polypeptide sequence, at least one amino acid point mutation and at most 2, 3, 4, 5, 6, 7, 8, 9 and 10 point mutations, in particular a deletion, addition or substitution of amino acid. In the case of a substitution of one or more consecutive or nonconsecutive amino acid(s), the substitutions are preferred in which the substituted amino acids are replaced by "equivalent" amino acids as defined above.

Two amino-acids or nucleotidic sequences are said to be "identical" if the sequence of amino-acids or nucleotidic residues in the two sequences is the same when aligned for maximum matching. Sequence comparisons between two peptides or polynucleotides are typically performed by comparing sequences of two optimally aligned sequences. Optimal alignment of sequences for comparison may be conducted by homology alignment algorithm well known of the skilled person, or by visual inspection.

"Percentage of sequence identity" (or identity) is determined by comparing two optimally aligned sequences over a comparison window, where the portion of the peptide (also for polynucleotide) sequence in the comparison window may comprise additions or deletions (i.e., gaps) as compared to the reference sequence (which does not comprise additions or deletions) for optimal alignment of the two sequences. The percentage is calculated by determining the number of positions at which the identical amino-acid residue or nucleic acid base occurs in both sequences to yield the number of matched positions, dividing the number of matched positions by the total number of positions in the window of comparison and multiplying the result by 100 to yield the percentage of sequence identity. The definition of sequence identity given above is the definition that would use one of skill in the art. The definition by itself does not need the help of any algorithm, the said algorithms being helpful only to achieve the optimal alignments of sequences, rather than the calculation of sequence identity.

From the definition given above, it follows that there is a well defined and only one value for the sequence identity between two compared sequences which value corresponds to the value obtained for the best or optimal alignment.

It can be used for example the BLAST P "BLAST 2 sequence" (Tatusova et al., "Blast 2 sequences - a new tool for comparing protein and nucleotide sequences", FEMS Microbiol. Lett. 174:247-250) software that is available in the web site http://www.ncbi.nlm.nih.gov/gorf/bl2.html, which is habitually used by the inventors and in general by the skilled man for comparing and determining the identity between two sequences. The "open gap penalty" and « extension gap penalty » parameters that depends on the substitution matrix selected regarding the nature and the length of the sequence to be compared is directly selected by the software (i.e "5" and "2" respectively for substitution matrix BLOSUM-62). The identity percentage between the two sequences to be compared is directly calculated by the software.

In a preferred embodiment, the polypeptide according to the present invention is characterized in that said RON2-2 domain of an Apicomplexan parasite is a RON2-2 domain of an Apicomplexan parasite selected from the group consisting of Aconoidasida and Conoidasida class of Apicomplexan parasite, preferably selected from the group consisting of Plasmodium, Toxoplasma, Eimeria, Neospora, Babesia or Theileria, more preferably selected from the group of *Toxoplasma gondii*, *Plasmodium falciparum, Plasmodium berghei, Plasmodium knowlesi, Plasmodium vivax, Plasmodium yoelii, Babesia bovis, Eimeria tenella*, *Neospora caninum and Theileria parva* species.

In a further preferred embodiment, the polypeptide according to the present invention is characterized in that it is a synthetic or a recombinant polypeptide.

In a preferred embodiment, the polypeptide according to the present invention is characterized in that it comprises a disulfide bond between the two amino-acids residues capable of forming a disulfide bond present in said subfragment.

In a preferred embodiment, the polypeptide according to the present invention is characterized in that it comprises the RON2-2 domain of the RON2 polypeptide having the sequence selected from the sequences SEQ ID NOs. 1, 3, 5, 7, 9, 11, 13, 15, 17 and 19, or a fragment or a homologous sequence thereof as defined in the above paragraphs a) and b).

In a preferred embodiment, the polypeptide according to the present invention is characterized in that it comprises the RON2-2 domain having the sequence selected from the sequences SEQ ID NOs. 2, 4, 6, 8, 10, 12, 14, 16, 18 and 20, or a fragment or a homologous sequence thereof as defined in the above paragraphs a) and b).

In a preferred embodiment, the polypeptide according to the present invention is characterized in that a disulfide bound (or bridge) is formed between two conserved cysteine amino-acids residues located into the RON2-2 domain.

These cysteine amino-acids residues are for example the cysteine residues located respectively in position aa1313 and aa1323 of the sequence SEQ ID NO:1 (*T*. *gondii* RON2 polypeptide) or in position aa2037 and aa2049 of the SEQ ID NO:3 (*P.falciparum* RON2 polypeptide). These two cysteine positions can be easily located from the sequences of the RON2-2 polypeptide SEQ ID NOs. 2, 4, 6, 8, 10, 12, 14, 16, 18 and 20.

In a more preferred embodiment, the polypeptide according to the present invention is characterized in that it has at most 64, preferably at most 63, 62, 61, 60, 55, 50, 45, 44, 43, 42, 41 and more preferably 40, 39, 38, 37 amino-acids length.

In another more preferred embodiment, the polypeptide according to the present invention is characterized in that it has at least 10 amino-acids length, preferably 11, 12, 13, 14, 15, 20, 25, 30 and more preferably at least 31, 32, 33, 34 or 35 amino-acids length.

In a further aspect, the present invention also comprises the isolated nucleic acid, DNA or RNA, coding for the polypeptide according to the present invention, or a complementary nucleic acid thereof.

Also is comprised in the present invention, a polypeptide according to the above invention, characterized in that it is recombinant polypeptide or a polypeptide which has been produced by chemical synthesis. These recombinant or synthetic polypeptides preferably comprises the disulfide bound formed between the two cysteines of the RON2-2 domain (or formed between two other natural or modified amino-acid capable to induce a disulfide bound whether these cysteine residues have been mutated, i.e.substitued, in the homologous polypeptide of the present invention).

For the production of the polypeptides of the present invention, it is possible to have recourse to genetic engineering procedures making use of micro-organisms transformed by a specific nucleic acid encoding the polypeptide.

Consequently, forms also part of the present invention the purified or isolated nucleic acids, such as DNA or RNA, encoding the polypeptides according to the invention.

Are also included in the present invention, cloning or expression vectors encoding the polypeptide according to invention, and the host cells transformed by said vectors.

A vector of the present invention can be either a prokaryotic or an eukaryotic vector, such as a plasmid or a virus. Expression vectors of the present invention include any DNA or RNA vectors that direct the expression of the recombinant polypeptide of the invention in the host cell, including bacterial, fungal, yeast, insect, other animal such as mammal, and plant cells.

The invention aims especially at cloning and/or expression vectors which contain a nucleotide sequence according to the invention.

The vectors according to the invention preferably contain elements which allow the expression and/or the secretion of the protein in a determined host cell. The vector must therefore contain a promoter, signals of initiation and termination of translation, as well as appropriate regions of regulation of transcription. It must be able to be maintained in a stable manner in the host cell and can optionally have particular signals which specify the secretion of the translated protein. These different elements are chosen and optimized by the person skilled in the art according to the host cell used. To this effect, the nucleotide sequences according to the invention can be inserted into autonomous replication vectors in the chosen host, or in integrative vectors of the chosen host.

Such vectors are prepared by methods currently used by the person skilled in the art, and the resulting vectors can be introduced into an appropriate host by standard methods, such as lipofection, electroporation, thermal shock, or chemical methods.

The vectors according to the invention are, for example, vectors of plasmidic or viral origin. They are useful for transforming host cells in order to clone or to express the nucleotide sequences according to the invention.

An expression vector of the present invention can be transformed into any suitable host cell to form a recombinant cell. A suitable host cell of the present invention includes any cell capable of expressing a nucleic acid of the present invention inserted into the expression vector. For example, a prokaryotic expression vector can be transformed into a bacterial host cell.

The invention likewise comprises the host cells transformed by or comprising a vector according to the invention.

In another aspect, the present invention is directed to a composition comprising a polypeptide according to the present invention, or a nucleic acid according to the present invention, for its use as a medicament, as an immunogen or as a vaccine.

These polypeptides of the present invention, particularly as immunogens or vaccines according to the invention, are capable of triggering *in vivo* the synthesis of specific antibodies, particularly directed against the antigenic epitope situated into the RON2-2 domain and comprising the subfragment located bewteen the two cysteine amino-acides residues as defined above.

In a preferred embodiment, said composition comprising a polypeptide according to the present invention, or a nucleic acid according to the present invention, is used as a pharmaceutical composition for the prevention or the treatment of Apicomplexan parasite infection.

Is also comprised in the present invention, the use of the polypeptide according to the invention for the manufacture of a composition for the prevention or the treatment of Apicomplexan parasite infection or pathology associated to this infection.

The present invention is also directed to a method of protecting a vertebrate, subject, preferably a mammalian or avian subject, more preferably a human, against infection by Apicomplexan parasite, the said method comprising administering to the said mammal the polypeptide or the nucleic acid according to the invention.

In a more preferred embodiment, said composition comprising a polypeptide according to the present invention, or a nucleic acid according to the present invention, is used as a pharmaceutical composition for the prevention or the treatment of toxoplasmosis, malaria, neosporosis, coccidiosis, or babesiosis or theileriosis.

In another aspect, the present invention relates to composition for stimulating an immune response against infection by an Apicomplexan parasite in a mammal, said composition comprising a polypeptide or a nucleic acid according to the invention.

Preferably, the said polypeptide or the said nucleic acid according to the invention are formulated as a vaccine in a pharmaceutically effective carrier.

The invention also relates to compositions comprising the polypeptides according to the invention with carrier molecules (natural or synthetic), pharmaceutically acceptable. Preferably, said composition according to the invention will be mixed with an excipient and/or a pharmaceutically acceptable vehicle.

In the present description, pharmaceutically acceptable vehicle is intended to indicate a compound or a combination of compounds entering into a pharmaceutical composition not provoking secondary reactions and which allows, for example, facilitation of the administration of the active compound(s), an increase in its lifespan and/or in its efficacy in the body, an increase in its solubility in solution or else an improvement in its conservation. These pharmaceutically acceptable vehicles are well known and will be adapted by the person skilled in the art as a function of the nature and of the mode of administration of the active compound(s) chosen.

Preferably, these compounds will be administered by the systemic route, in particular by the intravenous route, by the intramuscular, intradermal, intraperitoneal or subcutaneous route, or by the oral route. In a more preferred manner, the composition comprising the antibodies or the polypeptides according to the invention will be administered several times, in a sequential manner.

Their modes of administration, dosages and optimum pharmaceutical forms can be determined according to the criteria generally taken into account in the establishment of a treatment adapted to a patient such as, for example, the age or the body weight of the patient, the seriousness of his/her general condition, the tolerance to the treatment and the secondary effects noted.

An isolated or purified polypeptide of the present invention can be used as an immunogen to generate antibodies that bind the specific epitope contained in said polypeptide using standard techniques for polyclonal and monoclonal antibody preparation.. The antigenic peptide comprises at least 10 amino acid residues of the subfragment situated between the two cysteine residues on the RON2-2 domain as defined above, such that an antibody raised against the peptide of the invention forms a specific immune complex with that RON2-2 polypeptide. Preferably, the antigenic peptide comprises at least 11, 12, 13, 14 or 15 amino acid residues, more preferably at least 20, 25, 30 or 32, amino acid residues, even more preferably at least 35, 36, 37, 38, 39 or 40 amino acid residues, of the involved RON2-2 polypeptide containing that antigenic epitope.

A RON2-2 polypeptidic immunogen typically is used to prepare antibodies by immunizing a suitable subject, (e.g., rabbit, goat, mouse or other mammal) with the immunogen. An appropriate immunogenic preparation can contain, for example, recombinantly expressed or a chemically synthesized RON2-2 polypeptide, or fragment thereof, of the present invention. The preparation can further include an adjuvant, such as Freund's complete or incomplete adjuvant, alum or similar immunostimulatory agent.

In another aspect, the present invention is directed to a method for the production of isolated or purified antibodies, or a functional fragment thereof, capable of inhibiting the interaction between the AMA1 polypeptide and the RON2 polypeptide during host cell invasion step by Apicomplexan parasite, characterized in that said method comprises the step of:
a) immunizing a suitable non-human mammal subject with an immunogen composition comprising a polypeptide according to the present invention, said immunogen composition optionally further includes an adjuvant; and
b) selecting the antibodies which are capable of specifically binding the RON2-2 domain of RON2 Apicomplexan parasite, preferably said RON2-2 domain exhibiting the epitope comprising a fragment of at least 10 amino-acids residues of the subfragment of said RON2-2 peptide having the sequence comprised between the two cysteine residues located into said RON2-2 domain, or homologous fragment thereof as defined in part a) and b) above when defining the polypeptide according to the present invention.

In a more preferred embodiment, the immunogens in step a) are selected from the polypeptides having the sequence SEQ ID NOs. 2, 4, 6, 8, 10, 12, 14, 16, 18 or 20.

In another aspect, the present invention is directed to an isolated or purified antibody, or one of its functional fragments, susceptible to be obtained or obtained by the method according to the present invention, said antibody or one of its said fragments being capable of specifically binding the epitope formed by the subfragment of said RON2-2 peptide having the sequence comprised between the two cysteine residues located into said RON2-2 domain, or homologous fragment thereof as defined in claim 1, part a) and b) above when defining the polypeptide according to the present invention.

In another aspect, the present invention is directed to an isolated or purified antibody, or one of its functional fragments, susceptible to be obtained or obtained by the method according to the present invention, said antibody or one of its said fragments being capable of specifically binding the AMA1 polypeptide and disrupting the AMA1-RON2-2 binding.

Antibodies, monoclonal, polyclonal, chimeric or humanized antibodies, which are specifically directed against the polypeptide of the present invention as defined above are comprised in the invention.

In a more preferred embodiment, the method according to the present invention is a method for the production of isolated or purified monoclonal antibodies.

The antibodies according to the present invention are preferably specific monoclonal antibodies, especially of murine, chimeric or humanized origin, which can be obtained according to the standard methods well known to the person skilled in the art.

In general, for the preparation of monoclonal antibodies or their functional fragments, especially of murine origin, it is possible to refer to classical techniques which are well known of the skilled artisan.

By functional fragment of an antibody according to the invention, it is intended to indicate in particular an antibody fragment, such as Fv, scFv (sc for single chain), Fab, F(ab')2, Fab' or scFv-Fc. Said fragments have at least the sequences of the 6 characteristic CDRs of the heavy and light chain of the parent full length antibody and have in particular the capacity to recognize and to bind to the same RON2-2 epitope recognized by the complete parent antibody.

In a particular aspect, the antibody or one of its functional fragments, according to the present invention, is characterized in that it is a chimeric antibody and, can optionally comprises the light chain and heavy chain constant regions derived from a human antibody to form a humanized antibody, particularly when it is intended to use said antibody as therapeutic antibody.

The antibodies or their fragments of chimeric type according to the invention can be prepared by using the techniques of genetic recombination.

Antibody, or one of its functional fragments, according to the invention, or obtained by the process according to the invention, or a nucleic acid according to the invention, as a medicament forms part of the present invention, particularly for the manufacture of a pharmaceutical composition for the prevention or the treatment of Apicomplexan parasite infection.

In another aspect, the present invention is directed to a method of *in vitro* diagnosis of Apicomplexan parasite infection from a biological sample, characterized in that said biological sample is contacted with:
- an antibody susceptible to be obtained or obtained by the method according to the present invention, or an antibody according to the present invention; or
- a polypeptide according to the present invention,
optionally, said antibody or polypeptide is labeled.

Said antibody or said polypeptide, or one of their fragments, can be present in the form of an immunoconjugate or of a labeled antibody or polypeptide so as to obtain a detectable and/or quantifiable signal.

The labeled antibodies or polypeptide according to the invention, or their fragments include, for example, antibodies or polypeptides called immunoconjugates which can be conjugated, for example, with enzymes such as peroxidase, alkaline phosphatase, beta-D-galactosidase, glucose oxydase, glucose amylase, carbonic anhydrase, acetylcholinesterase, lysozyme, malate dehydrogenase or glucose 6-phosphate dehydrogenase or by a molecule such as biotin, digoxygenin or 5-bromodeoxyuridine. Fluorescent labels can be likewise conjugated to the antibodies or polypeptdides according to the invention and especially include fluorescein and its derivatives, fluorochrome, rhodamine and its derivatives, GFP (GFP for "Green Fluorescent Protein"), dansyl, umbelliferone, etc.. In such conjugates, the antibodies or polypeptides of the invention, or their functional fragments, can be prepared by methods known to the person skilled in the art. They can be coupled to the enzymes or to the fluorescent labels directly or by the intermediary of a spacer group or of a linking group such as a polyaldehyde, like glutaraldehyde, ethylenediaminetetraacetic acid (EDTA), diethylene-triaminepentaacetic acid (DPTA), or in the presence of coupling agents such as those mentioned above for the therapeutic conjugates. The conjugates containing labels of fluorescein type can be prepared by reaction with an isothiocyanate.

Other conjugates can likewise include chemoluminescent labels such as luminol and the dioxetanes, bioluminescent labels such as luciferase and luciferin, or else radioactive labels.

In a particular embodiment, the polypeptides or the antibodies, or their functional fragments, according to the invention, can be employed in a process for the detection and/or the quantification of Apicomplexan polypeptides, or antibodies directed against these Apicomplexan polypeptides, in a biological sample, for the monitoring of the efficacy of a prophylactic and/or therapeutic Apicomplexan parasite infection treatment

Preferably, the biological sample is formed by a biological fluid, such as serum, whole blood, cells, a tissue sample or biopsies of human origin.

Any procedure or conventional test can be employed in order to carry out such a detection and/or dosage. Said test can be a competition or sandwich test, or any test known to the person skilled in the art dependent on the formation of an immune complex of antibody-antigen type.

By way of example, a preferred method brings into play immunoenzymatic processes according to the ELISA technique, by immunofluorescence, or radioimmunoassay (RIA) technique or equivalent.

In another aspect, the present invention is directed to a kit comprising the following elements:
a)
   - an antibody susceptible to be obtained or obtained by the method according to the present invention, or an antibody according to the present invention; or
   - a polypeptide according to the present invention,
      optionally, said antibody or polypeptide is labeled;
b) optionally, the suitable reagents for the formation of a immunological reaction; and
c) optionally, the reagents allowing the demonstration of antigen/antibody and/or antibody/polypeptide complexes produced by the immunological reaction.

In another aspect, the present invention is directed to a construct for its use for crystallization of the Apicomplexan co-structure AMA1-RON2 complex, said construct comprising the following elements:
a) a polypeptide according to the present invention; and, preferably
b) an AMA1 polypeptide fragment comprising at least the subdomains DI and DII of the ectoplasmic domain of said AMA1 polypeptide, preferably said AMA1 polypeptide fragment is the AMA1 ectoplasmic domain comprising the DI, DII and, optionally DIII subdomains,
said RON2-2 and AMA1 polypeptide fragment being originating from the same species of Apicomplexan parasite.

In another aspect, the present invention is directed to a construct for its use for crystallization of the Apicomplexan co-structure AMA1-RON2 complex, said construct comprising the following elements:
a) a polypeptide according to the present invention, said polypeptide being capable of crossreacting with the AMA1 ectodomain of at least two different species of Apicomplexan parasite; and, preferably
b) an AMA1 polypeptide fragment comprising at least the subdomains DI and DII of the ectoplasmic domain of said at least AMA1 polypeptide which crossreacts with said polypeptide as defined in a), preferably said AMA1 polypeptide fragment is the AMAI ectoplasmic domain comprising the DI, DII and, optionally DIII subdomains,
said RON2-2 and AMA1 polypeptide fragment being originating from different species of Apicomplexan parasite.

In another aspect, the present invention is directed to a method for the preparation of a crystal of a polypeptide complex, preferably for diffraction measurements analysis, said method comprising contacting a polypeptide according to the present invention and an AMA1 polypeptide fragment comprising at least the domains DI and DII of said AMA1 polypeptide, and wherein, said RON2 and AMA1 polypeptide fragment being originating from the same species of Apicomplexan parasite, preferably from *Toxoplasma gondii* or *Plasmodium falciparum* species.

Are preferred, the AMA1 fragments or derived thereof having the sequence SEQ ID NOs. 21 and 22 when a crystal of the co-structure AMA1-RON2 of *Plasmodium falciparum* is intended to be prepared.

Are also preferred, the AMA1 fragments or derived thereof having the sequence SEQ ID NO. 75 and the fragment aa 36-456 of the SEQ ID NO. 23 when a crystal of the co-structure AMA1-RON2 of *Toxoplasma gondii* is intended to be prepared.

SEQ ID NO. 75 (one letter code)

GSAMGSASTSGNPFQANVEMKTFMERFNLTHHHQSGIYVDLGQDKEVDGTLY
REPAGLCPIWGKHIELQQPDRPPYRNNFLEDVPTEKEYKQSGNPLPGGFNLNFV
TPSGQRISPFPMELLEKNSNIKASTDLGRCAEFAFKTVAMDKNNKATKYRYPFV
YDSKKRLCHILYVSMQLMEGKKYCSVKGEPPDLTWYCFKPRKSVTENHHLIYG
SAYVGENPDAFISKCPNQALRGYRFGVWKKGRCLDYTELTDTVIERVESKAQC
WVKTFENDGVASDQPHTYPLTSQASWNDWWPLHQSDQPHSGGVGRNYGFYY
VDTTGEGKCALSDQVPDCLVSDSAAVSYTAAGSLSEETPNFIIPSNPSVTPPTPET
ALQCTADKFPDSFGACDVQACKRQKTSCVGGQIQSTSVCTADEQNECGSNTAA
ALVPR

These isolated polypeptide having the sequence SEQ NOs. 21, 22, 75 and the fragment aa 36-456 of the SEQ NO. 23 form part of the present invention.

The crystal which has been obtained or obtainable by the methods according to the invention forms also part of the present invention.

In another aspect, the present invention relates to a computer-readable data storage material encoded with computer-readable data comprising the atomic structure coordinates of the crystal according to the present invention, preferably the three-dimensional structure of the co-structure AMA1-RON2 Apicomplexan parasite, preferably from *Toxoplasma* genus, such as *Toxoplama gondii* or from *Plasmodium* genus, such as *Plasmodium falciparum*, which substantially conforms to the data given in the examples.

According to the present invention, the crystal of the Apicomplexan co-structure AMA1-RON2 complex can be used to determine the ability of a compound to bind to the AMA1 site associated to the RON2-2 binding, or to inhibit said AMA1-RON2 complex formation.

This ability is predicted by a structure-based, drug-design method that is based on the three-dimensional structure of the co-structure AMA1-RON2 complex crystal obtained by the inventors. The predicted binding of the chemical compound to the crystal of the present invention is determined by methods standard in the art.

Potential compounds which can bind to the AMA1 site associated to the RON2-2 binding, or to inhibit said AMA1-RON2 complex formation, can inhibit the host cells (particularly erythrocytes) invasion. These potential compounds can be designed using structure-based drug design. Until the discovery of the three-dimensional structure of the present invention, no information was available for structure-based development of therapeutic compounds based on the co-structure of the AMA1-RON2 complex. Such rational development could not be executed de novo from available linear amino acid sequence information. Structure-based drug design refers to the use of computer simulation to predict a conformational interaction between the three-dimensional AMA1 ectodomain-RON2 co-structure of the present invention and consequently, a potential therapeutic compound.

For example, generally, for a protein to effectively interact with a therapeutic compound, it is necessary that the three-dimensional structure of the therapeutic compound assume a compatible conformation that allows the compound to bind to the protein in such a manner that a desired result is obtained upon binding. In this case, the knowledge of the three-dimensional AMA1 ectodomain-RON2 co-structure enables a skilled artisan to design a therapeutic compound having such a compatible conformation.

Thus, present invention also provides:
- methods for identifying, for selecting or for screenind ligands of this AMA1 ectodomain site associated to the RON2-2 binding, said method using the said crystal co-structure analysis, and to design pharmaceutical compounds capable of interacting or binding to that domain AMA1, particularly to certain amino-acid residues of domain identified as contributing to a relevant domain/epitope, as Tyr213 and F197 in *T.gondii* RON2 sequence; or
- methods for identifying, for selecting or for screening vaccine or drug candidates that could be used to inhibit host cells, particularly erythocytes, invasion by, and/or growth inhibition of, Apicomplexan parasites and that can used in pharmaceutical compositions for the prevention or treatment Apicomplexan parasite infection or their associated diseases such as toxoplasmosis, malaria, neosporosis, coccidiosis, babesiosis or theileriosis.

So, in another aspect, the present invention relates to a computer-readable data storage material encoded with computer-readable data comprising atomic structure coordinates of the polypeptide AMA1-RON2 complex obtained by the method above for the preparation of a crystal of a polypeptide complex according to the present invention.

In another aspect, the present invention is directed to a method for *in silico* screening a potential inhibitor of the host cell invasion of mammal by an Apicomplexan parasite comprising the step of:
- selecting a potential inhibitor by performing rational drug design with the three-dimensional structure of the Apicomplexan co-structure AMA1-RON2 complex, determined from the crystal obtained by the method according to the present invention, or by using the computer-readable data storage material according to the present invention, the said selecting being optionally performed in conjunction with computer modeling.

In another aspect, the present invention is directed to an *in vitro* method for screening or identifying a potential inhibitor of the host cell invasion of mammal by an Apicomplexan parasite, said method comprising:
a) optionally, selecting a potential inhibitor by performing the *in silico* method according to the present invention;
b) determining whether this potential inhibitor is capable to specifically bind a polypeptide according to the present invention, or to the AMA1 polypeptide, or fragment thereof comprisng at least the subdomains DI and DII of the ectoplasmic domain of said AMA1 polypeptide, or/and to inhibit the formation of the AMA1/RON2 complex resulting from the contact between a polypeptide according to the present invention and an AMA1 polypeptide fragment comprising at least the subdomains DI and DII of the ectoplasmic domain of said AMA1 polypeptide; and
c) optionally,
   - administrating the potential inhibitor to a non-human animal model which has been infected by an Apicomplexan parasite or contacting *in vitro* the potential inhibitor with a cell model by using a cellular-based assay capable of reproducing the invasion of mammal host cell by Apicomplexan parasite; and
d) optionally, detecting the ability of the potential inhibitor for inhibiting the host cell invasion and/or parasite growth.

Finally, the present invention is directed to a *in vitro* method for screening a potential inhibitor of the host cell invasion of mammal by an Apicomplexan parasite, said method comprising the steps of:
a) contacting said potential inhibitor with :
   -i) a polypeptide according to the present invention, or with an AMA1 polypeptide, or a fragment thereof comprising at least the subdomains DI and DII of the ectoplasmic domain of said AMA1 polypeptide, or
   -ii) an AMA1/RON2-2 complex resulting from the contact between a polypeptide according to the present invention and an AMA1 polypeptide fragment comprising at least the subdomains DI and DII of the ectoplasmic domain of said AMA1 polypeptide;
b) determining whether this potential inhibitor is capable to:
   -i) specifically bind the polypeptide according to the present invention, or bind said AMA1 polypeptide, or a fragment thereof, or/and
   -ii) specifically inhibit the formation of the AMA1/RON2-2 complex; and
c) optionally,
   - administrating the potential inhibitor to a non-human animal model which has been infected by an Apicomplexan parasite or contacting *in vitro* the potential inhibitor with a cell model by using a cellular-based assay capable of reproducing the invasion of mammal host cell by Apicomplexan parasite; and
d) optionally, detecting the ability of the potential inhibitor for inhibiting the host cell invasion and/or parasite growth.

The following figures and their legends are illustrative of embodiments of the present invention and are not meant to limit the scope of the invention as encompassed by the claims.

### Description of the Figures

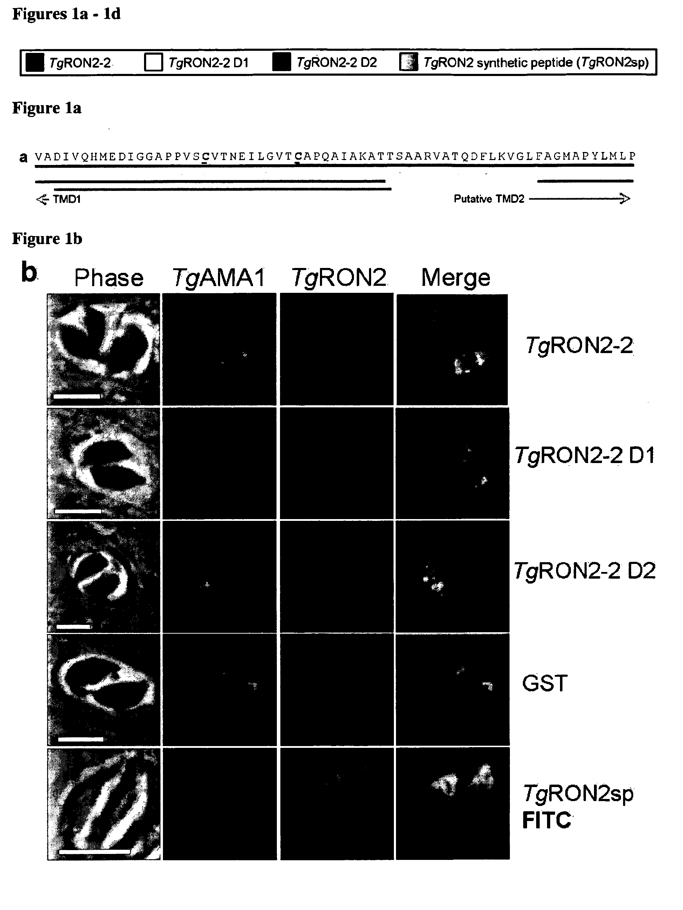

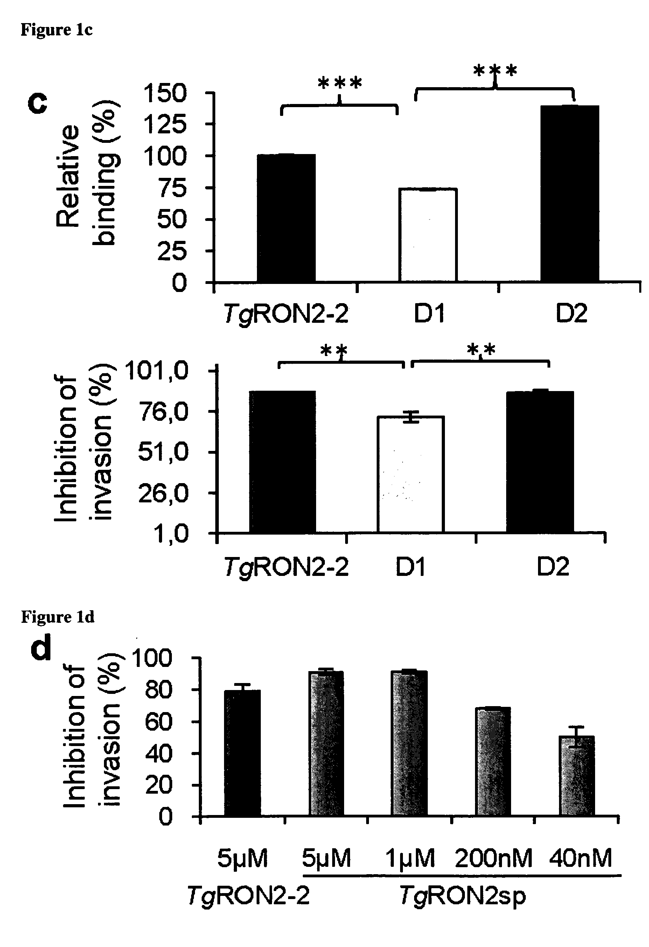

**Figure 1****:** Defining the TgRON2 recognition sequence for TgAMA1
   a, TgRON2-2 deletion constructs; TMD, transmembrane domain. b, TgRON2 constructs label TgAMA1 in micronemes of intracellular parasites. Scale bar, 5 µm. c, SPR-based binding of TgRON2 constructs to TgAMA1 (top). TgRON2 constructs inhibit T. gondii invasion of HFF cells (bottom). d, TgRON2sp displays similar invasion inhibition properties to TgRON2-2. Student's t-test; ** p<0.01, *** p<0.001; means ± s.e.m. for n = 2, and n = 3.

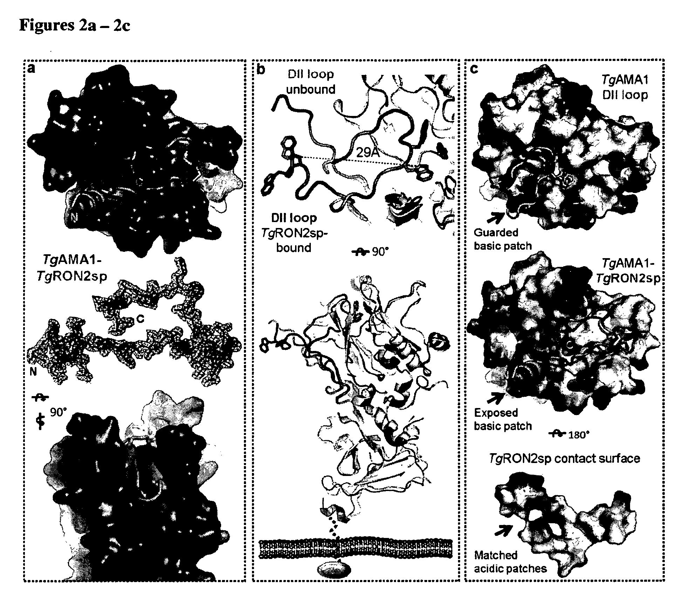

**Figure 2****:** TgRON2sp induces conformational change in TgAMA1
   a, Top and end-on views of TgRON2sp (green) bound in the TgAMA1 apical groove (purple). Electron density map of TgRON2sp shown in green and contoured at 1.0σ
   (center). b, Top and side views of TgRON2sp showing the 29Å displacement of the TgAMA1 DII loop from its ordered position in the apo TgAMA1 structure. c, Electrostatic renderings of the TgAMA1 apical surface guarded by the DII loop (top panel - orange) and in the TgRON2sp bound form (middle panel). Corresponding electrostatic view of TgRON2sp (lower panel).

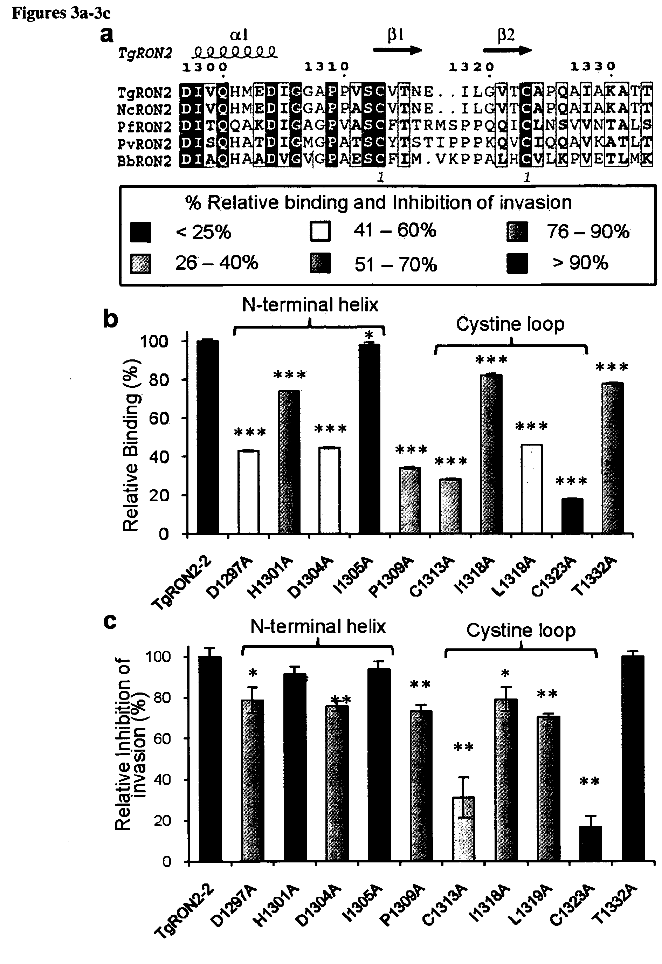

**Figure 3****:** TgRON2sp hot spot residues in TgAMA1 binding
   a, Alignment of Apicomplexan RON2 sequences corresponding to TgRON2sp; secondary structure and numbering based on TgRON2. b, SPR-measured relative binding of TgRON2-2 mutants to TgAMA1. c, Effect of TgRON2-2 mutants (6 µM) in inhibiting invasion by T. gondii. Student's t-test; * p<0.05,** p<0.01, *** p<0.001; means ± s.e.m. for n = 2 and n = 3.

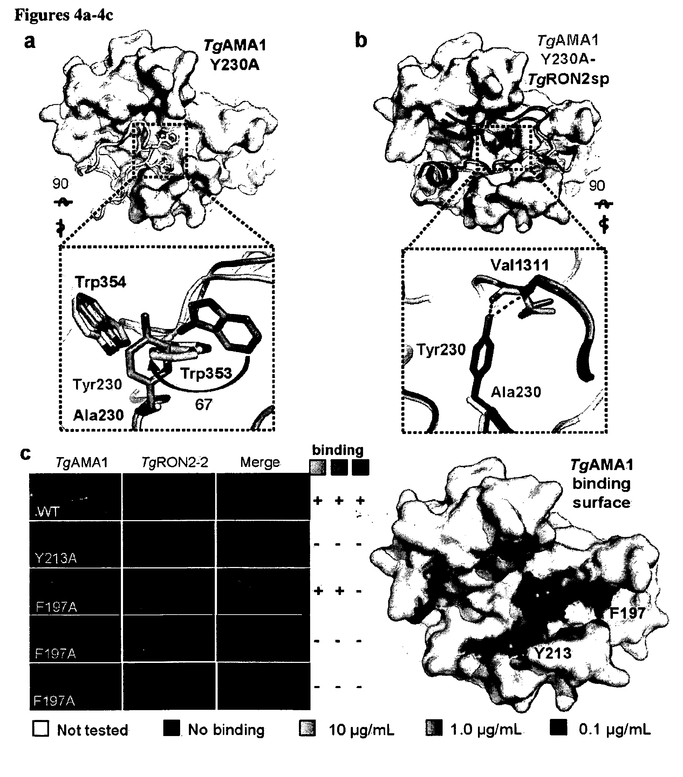

**Figure 4****:** TgAMA1 contributions to TgRON2sp complex formation
   a, Apical view of apo TgAMA1 Y230A DI surface with secondary structure DII loop (yellow). Inset - overlay with apo TgAMA1 (orange) shows reorganization of the DII loop. b, Apical view of TgAMA1 Y230A in complex with TgRON2sp (dark grey). Inset - overlay with TgAMAl-TgRON2sp (purple-green) highlights limited role for Tyr230 in forming a bifurcated hydrogen bond. c, Binding consequences of TgAMA1 mutants and synergistic TgRON2-2 mutants in cell-based assays (left) and spatially represented on the surface of TgAMA1 (right).
**Figure 5****:** A novel host-pathogen protein ligand-receptor complex
   Pathogen derived proteins (left - viral; center - bacterial and right - TgAMA1-TgRON2sp representing the first protozoan complex) are shown in purple and host cell proteins shown in green. Buried surface areas are shown in Å2. PDB IDs left to right: 2VSM, 3LQA, 1F02, 1O6S, 2Y8T.
**Figure 6****:** The TgAMAl-TgRON2 complex is the essential link between an Apicomplexan parasite and its target host cell.
   Left - schematic of an Apicomplexan parasite in the process of active host cell invasion and parasitophorous vacuole formation. Inset center - the close junction between parasite and host cell which migrates from anterior to posterior during invasion is termed the moving junction (MJ). The critical, highly conserved components of the MJ include the transmembrane microneme-derived protein AMA1 (apical membrane antigen 1) presented on the parasite surface and serving as the receptor for rhoptry-derived RON proteins (rhoptry neck proteins 2, 4, and 5) secreted and assembled at the host cell surface. Inset right - the TgAMAl-TgRON2sp co-structure presented in this work is the first step in structurally defining how the AMA1-RONs complex assembles, and represents the bridge between host and pathogen necessary for active invasion through the MJ constriction.

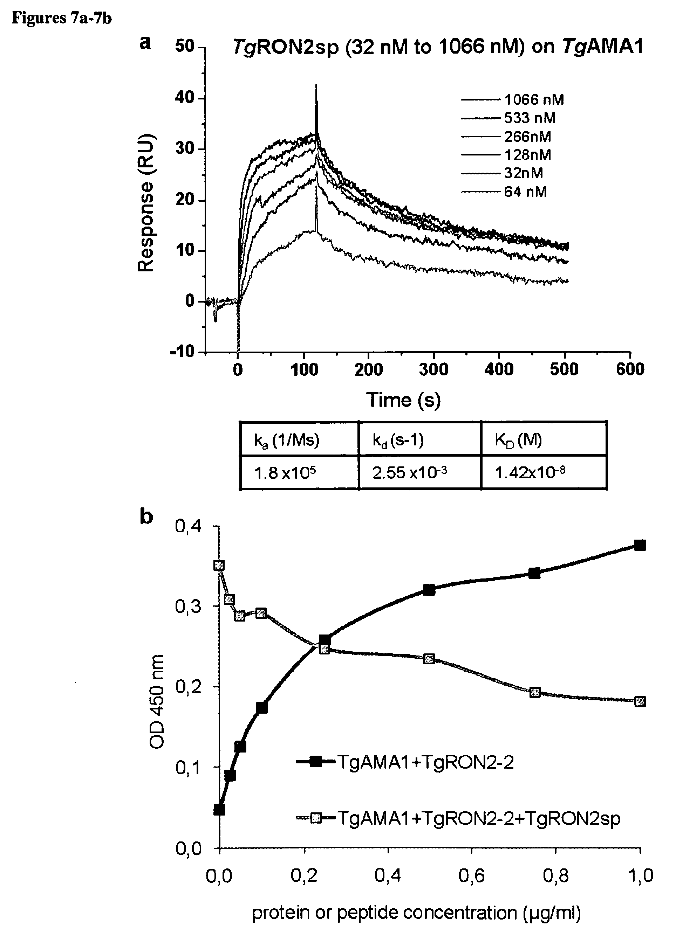

**Figure 7****:** Affinity of TgRON2sp for TgAMA1
   (a) SPR based binding kinetics of TgRON2sp on TgAMA1: binding sensorgrams of TgRON2sp at 6 concentrations (from 33 nM to 1066 nM) to TgAMA1 covalently immobilized (2500-3000 RU) on a CM5 chip. All sensorgrams were obtained by subtracting the signal from the control flowcell. The flow rate was 50 µl/min. (b) Incubation with TgRON2sp prevents the binding of TgRON2-2 to TgAMA1 in a dose-dependent fashion. Competitive ELISAs were performed by a pre-incubation for 15 min of 1 µg/mL TgRON2-2 and increasing concentrations of TgRON2sp peptide (0 to 1 µg/mL) before being added to each well coated with 1 µg/mL of TgAMA1. Values represent means ± SD, n=2, from a representative experiment out of 2 independent assays.

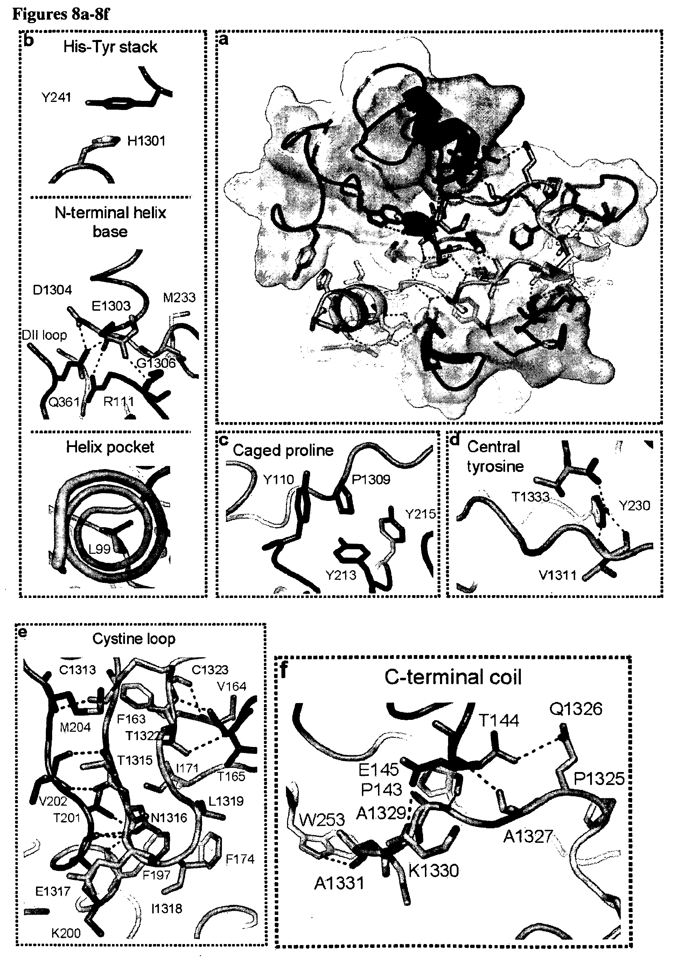

**Figure 8****:** Detailed atomic interactions that define the TgAMA1-TgRON2sp interface
   (a) TgAMA1 is shown in purple, TgRON2sp in green and buried solvent molecules as blue spheres. (b) Top - TgAMA1 Tyr241 forms a pi-pi stacking interaction with His1301 on TgRON2sp. Middle - The base of the TgRON2sp helix is anchored by a combination of 5 hydrogen bonds incorporating backbone and side chain interactions. Bottom - TgAMA1 Leu99, conserved in sequence and structure with PfAMA1 and PvAMA1, forms the base of the pocket occupied by the N-terminal helix of TgRON2sp. (c) Shape complementarity is observed as TgRON2sp Pro1309 is caged by a trio of TgAMA1 tyrosines and thus occupies the pocket left vacant by the displacement of Trp354 with the DII loop. (d) The hydroxyl group of the tyrosine central to the TgAMA1 apical groove (Tyr230) orients the amidated C-terminus threonine of the peptide (Thr1333) with a single hydrogen bond and also the TgRON2sp coil connector with 2 backbone hydrogen bonds. (e) Ten of the twenty-three predicted hydrogen bonds are dedicated to ordering the previously disordered Loop 2 of TgAMA1 to form the contiguous antiparallel beta-sheet and anchoring the disulfide bond. (f) Pro1325 enhances shape complementarity while 5 hydrogen bonds are spread over 5 residues of the C-terminal coil of TgRON2sp and likely responsible for ordering this region of the peptide.

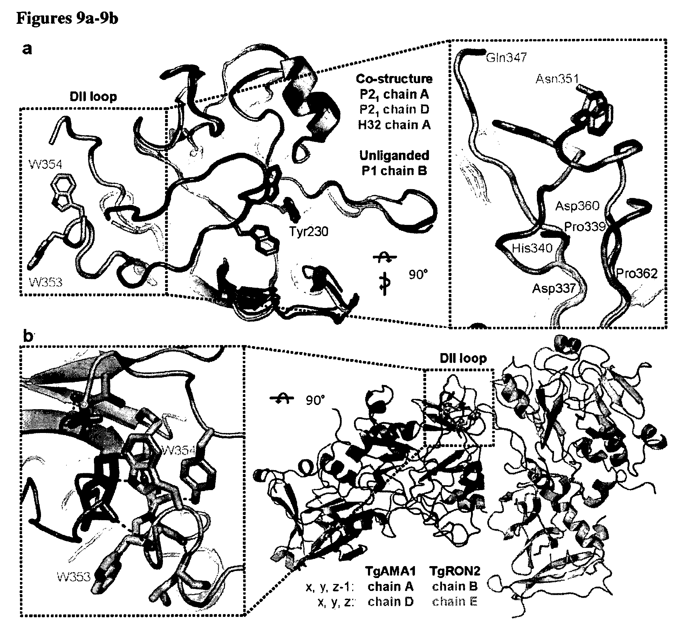

**Figure 9****:** Crystal contacts and an alternate crystal form of TgAMA1-TgRON2sp support the disordered structure of the displaced DII loop
   (a) Overlaying the two chains of the co-structure in the monoclinic spacegroup (A - purple; D - violet) with the additional single TgAMA1 chain from the hexagonal spacegroup (forest green) and chain B of unliganded TgAMA1 (blue) reveals differences in the ordered structure of the displaced DII loop. In the unliganded structure, the DII loop is modeled in its entirety with the two tryptophans at the tip of the loop (Trp353/354) anchored by the apical groove central tyrosine (Tyr230). Inset - The last positionally conserved residues defining the base of the DII loop between all 4 structures are Asp337 at the N-terminal end and Pro362 at the C-terminal end. The loop is nearly completely modeled in chain D of the P21 crystal form (missing Ala348-Trp350), but the loop in chain A is only modeled to Pro339 and Asp360. A hexagonal crystal form with one complex in the asymmetric unit revealed crystal packing with the DII loops directed into large solvent channels and subsequently disordered; the extent of ordered structure closely mirrors that seen in chain A of the monoclinic crystal form, with the base of the DII loop modeled to His340 and Asp360. (b) A closer inspection of the crystal packing in the P21 spacegroup shows that a nearby complex closely borders on the DII loop of chain D. Inset - a detailed view of this interface reveals that crystal contacts are likely responsible for the more extensively modeled the displaced DII loop of chain D.

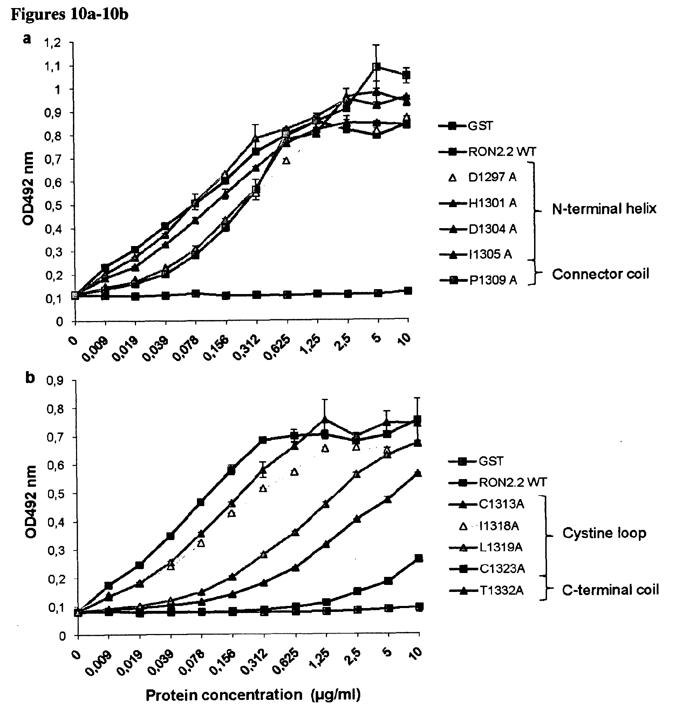

**Figure 10****:** Binding analysis of TgRON2-2 mutants by ELISA
   ELISAs were carried out with increasing concentrations of recombinant TgRON2-2 mutants on TgAMA1 coated at 1 µg/mL. GST alone was used as a control. Bound TgRON2-2 protein was detected using rat anti-GST (1:1000) followed by an anti-rat IgG horseradish peroxidase conjugate (1:2500) (ZyMAX, Invitrogen). (a) Mutants in the N-terminal helix of and the coil connecting the helix and cystine loop. (b) Mutants in the cystine loop and the C-terminal coil. Values represent means ± SD, n=2, from a representative experiment out of 3 independent assays. Colouring based on the SPR data presented in Fig. 3b shows a close correlation between the data provided for each assay.

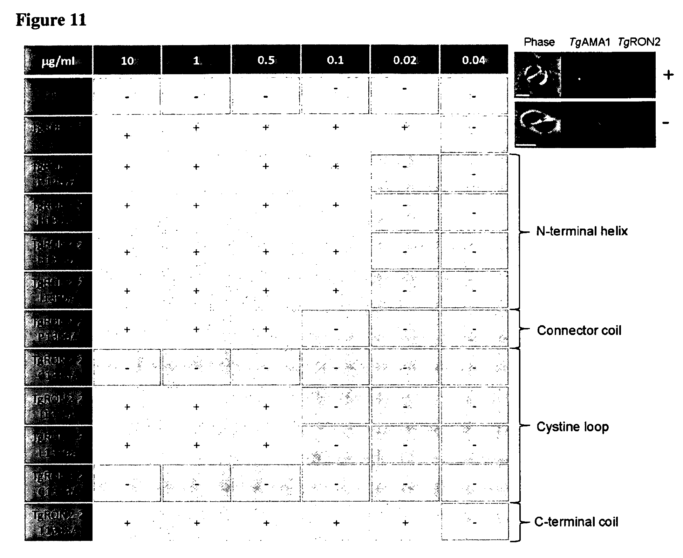

**Figure 11****:** *In vitro* binding analysis of TgRON2-2 mutants
   TgRON2-2 binds specifically to exposed TgAMA1. Fixed intracellular tachyzoites were permeabilized and incubated with TgRON2-2 at various concentrations (0.04 to 10 µg/mL). Binding to TgAMA1 in micronemes was assessed by immunofluorescence using anti-GST antibody (labelling of TgRON2-2) and anti-ectodomain B3.90 antibody (labelling of TgAMA1). Scale bar, 5µm.

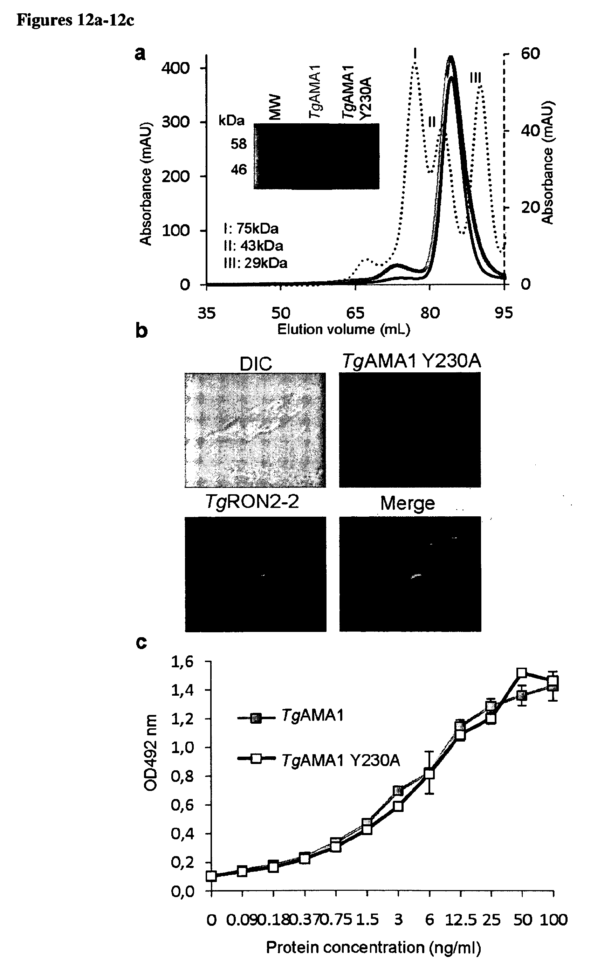

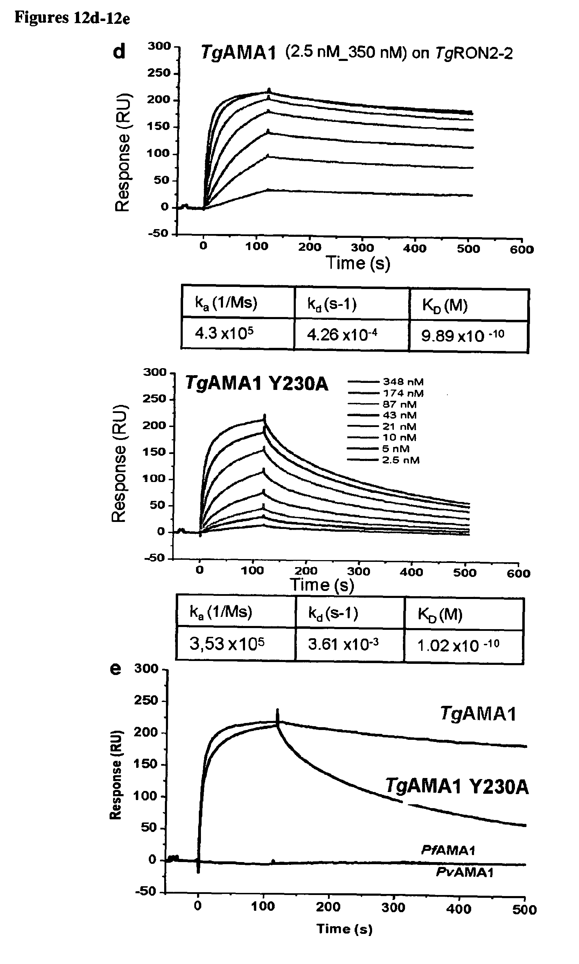

**Figure 12****:** Preliminary analysis of TgAMA1 Y230A binding to TgRON2-2
   (a) Elution of Ni-affinity purified native and mutant TgAMA1 off the size exclusion column revealed similar elution profiles suggesting the structural core of the mutant was conserved. Inset - SDS-PAGE showed the two proteins migrating in a similar pattern at the expected molecular weight of 48 kDa. (b) TgRON2-2 co-localizes with TgAMA1 Y230A expressed on the surface of BHK-21 cells. (c) ELISA was carried out with increasing concentrations of TgRON2-2 and recombinant TgAMA1 (orange) or TgAMA1 Y230A (yellow) proteins coated at 1 µg/mL. Bound TgRON2-2 protein was detected using rat anti-GST (1:1000) followed by an anti-rat IgG horseradish peroxidase conjugate (1:2500) (ZyMAX, Invitrogen). Binding was visualized using SIGMAFAST-OPD (o-phenylenediamine) tablet (Sigma Aldrich) and absorbance read at 492 nm. Values represent means ± SD, n=2, from a representative experiment out of 2 independent assays. (d) Multi concentration SPR binding study of TgAMA1 and TgAMA1 Y230A on TgRON2-2: binding sensorgrams of TgAMA1 (top) and TgAMA1 Y230A (bottom) at 6 concentrations (from 2.5 nM to 348 nM) to TgRON2-2 (GST fusion) captured at 612 RU on immobilized anti-GST. All sensorgrams were obtained by subtracting the signal from the control flowcell. The flow rate was 50 µL/min. (e) Simplified SPR sensorgram of TgRON2-2 on TgAMA1 (350 nM) highlights the higher dissociation rate of TgAMA1 Y230A relative to TgAMA1. Note that PfAMA1 and PvAMA1 have no cross-reactivity with TgRON2-2.

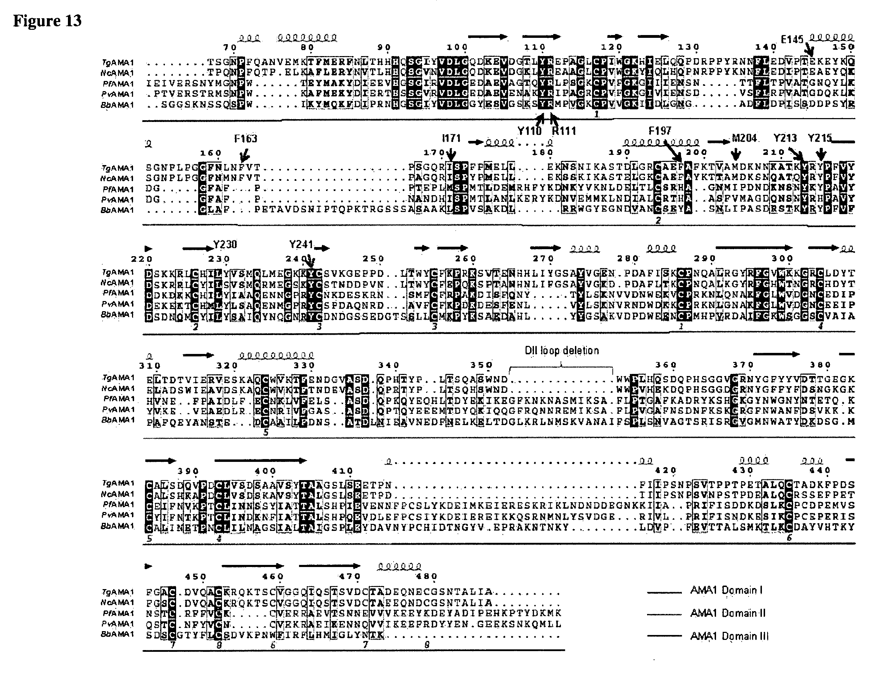

**Figure 13****:** AMA1 sequence alignment of the fully processed 3-domain extracellular region using Kalign and ESPript
   Accession numbers for aligned AMA1 sequences are as follows: T. gondii (ME49_055260), Neospora caninum (BAF45372), P. falciparum (XP_001348015.1), P. vivax (XP_001615447), and Babesia bovis (AAS58045.1), with the P vivax sequence modified to represent that crystallized by Pizarro et al 2005. Secondary structure and numbering is based on TgAMA1. Mutants in this study are indicated, and coloured as in Fig. 4c.

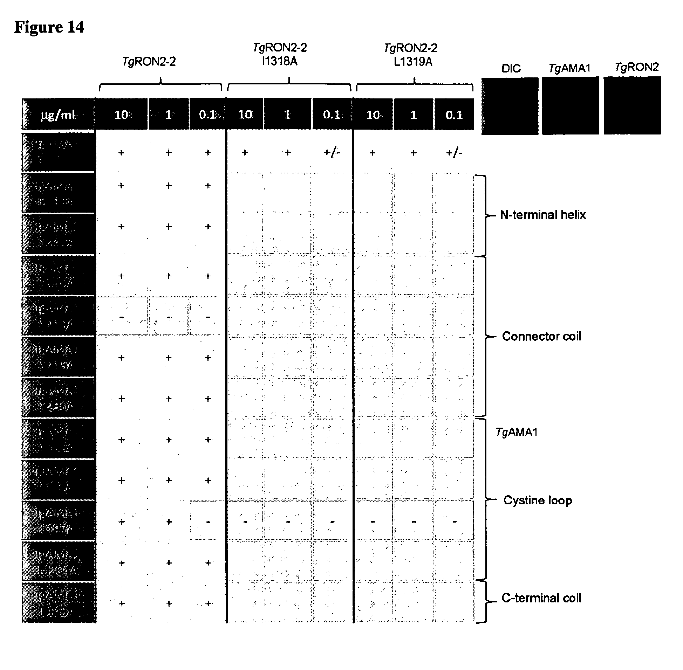

**Figure 14****:** *In vitro* binding analysis of TgAMA1 mutants
   Transiently transfected BHK-21 cells expressing TgAMA1 or a mutated version of TgAMA1 at their surface were incubated with TgRON2-2 or mutated version of TgRON2-2 at concentrations ranging from 0.1 µg/mL to 10 µg/mL, for 1 h. After five washes in PBS to remove unbound proteins, cells were fixed in 4% PAF and further processed for IFA. Binding was considered as negative when cells expressing a strong signal of TgAMA1 at their surface (using anti-ectodomain B3.90 antibody) were absolutely negative for the detection of TgRON2-2 using anti-GST antibody.

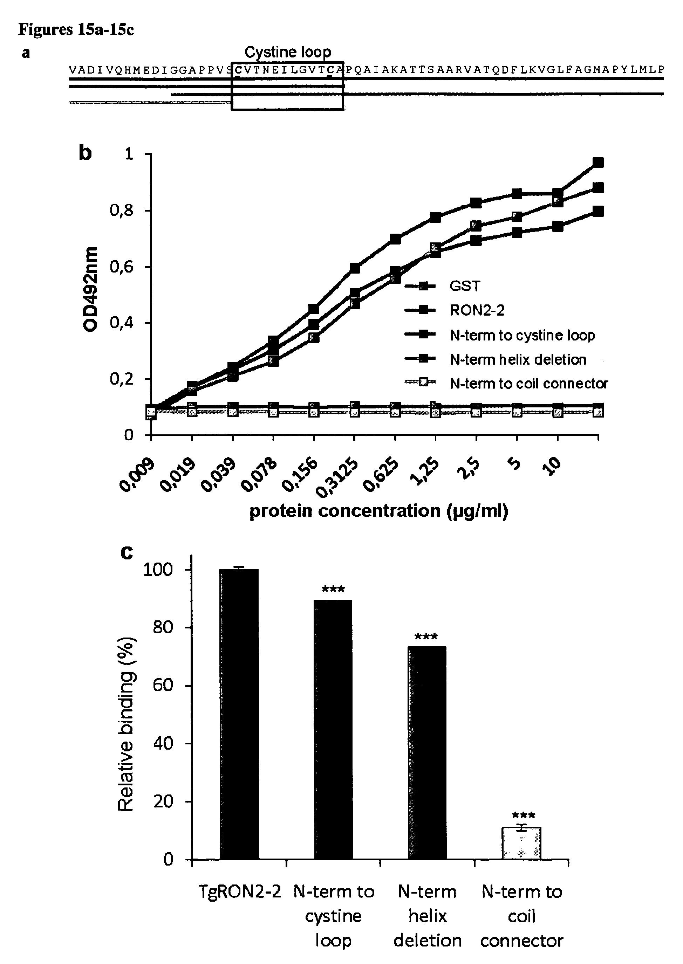

**Figure 15****:** Demonstration of the cystine loop as the critical substructure for TgAMA1-TgRON2sp complex formation
   (a) TgRON2-2 (red) with three additional truncation constructs honing in on the role of individual substructures; region after the cystine loop (purple), N-terminal helix (pink), and cystine loop (cyan). (b) an ELISA assay and (c) a SPR analysis shows a limited role for the complete section of TgRON2-2 from the end of the cystine loop to the C-terminus (purple), and a construct missing just the N-terminal helix is still competent to bind TgAMA1. However, once the cystine loop is removed (cyan) there is no detectable binding, supporting the insufficiency of the N-terminal helix and coil connector alone to displace the DII loop. Together, these constructs highlight a key role for the cystine loop in mediating complex formation and support our proposed binding mechanism. Error bars in (b) represent SD, n=2, from a representative experiment out of 2 independent assays. SPR-measured binding of TgRON2 segments to TgAMA1 in (c) is expressed in % of binding regarding to TgRON2-2. Error bars in (c) represent SD; t-test, *** p<0.001 for *n*=3.

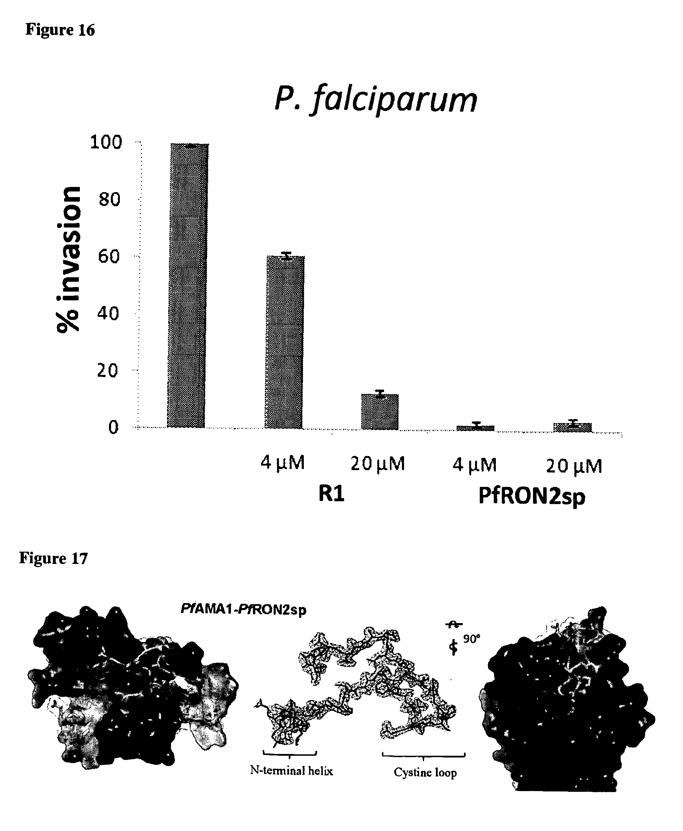

**Figure 16****:** Inhibition of host cell invasion by Plasmodium merozoites by AMA1-binding PfRON2-2 peptide
   Inhibition of the *P. falciparum* invasion of human red blood cells by PfRON2sp construct at different concentrations compaired with the R1 peptide.
Figure 17: PfAMA1-PfRON2sp co-structure

### Example 1: Material and Method

### A) Recombinant proteins, and peptides

Recombinant TgAMA1 and TgAMA1 Y230A were produced in insect cells and purified according to previously published protocols²². Expression of pGEX-TgRON2-2 GST was performed in E. coli and purified as described previously⁶. Primers used for production of recombinant TgRON2-2 constructs and mutant versions of TgAMA1 are listed in Table 3. A 37 residue, disulfide cyclised TgRON2 peptide (TgRON2sp) and an N-terminal FITC-labelled version (TgRON2sp-FITC) corresponding to residues 1297 to 1333 were synthesized by Kinexus (Vancouver, Canada).

### B) Structure determination

Diffraction data were processed using Imosflm⁴⁰ and Scala⁴¹ in the CCP4 suite of programs⁴². Initial phases for TgAMAl-TgRON2sp and TgAMA1 Y230A (+/-TgRON2sp) were obtained by molecular replacement using PHASER⁴³ with the unliganded TgAMA1 structure (PDB 2X2Z). Tracing of TgRON2sp was performed manually in COOT44 and all refinements performed with Refmac5⁴⁵. Stereochemical analysis was performed with PROCHECK and SFCHECK in CCP4⁴².

SPR, cellular binding and invasion assays. SPR analysis was performed on a BIACORE 3000 at 25°C using running buffer HBS-EP at a flow rate of 50 µL/min. Kinetic and affinity data were calculated using BIAevaluation 4.1 software. Cell culture, site-directed mutagenesis, fluorescence imaging and ELISA techniques were conducted based on previously published protocols⁶.

### C) Molecular biology, recombinant protein production and peptide synthesis

The TgAMA1 Y230A construct was generated by site directed mutagenesis using the QuickChange II XL protocol (Stratagene). Recombinant TgAMA1 and TgAMA1 Y230A were produced in insect cells and purified according to a previously published protocol²². Expression of the pGEX-TgRON2-2 GST was performed using E. coli and purified as described previously⁶. Primers for recombinant RON2 constructs and mutant versions of TgRON2-2 are listed in Table 3. A 37 residue disulfide cyclized TgRON2 peptide (TgRON2sp - residues 1297 to 1333) and the corresponding N-terminal FITC-labelled version (TgRON2sp-FITC) were synthesized by Kinexus (Vancouver, Canada). Lyophilized peptides were solubilized in 100% DMSO and diluted in HBS (20 mM HEPES pH 7.5, 150 mM NaCl) for use in co-crystallization and functional studies.

### D) Crystallization and data collection

Initial crystals of TgAMAl-TgRON2sp and TgAMA1 Y230A-TgRON2sp were obtained after three months in 25% PEG1500, 100 mM MIB pH 4.0. The final 1.6 µL drops consisted of equal volumes protein (AMA1 (14 mg/mL) incubated with a twofold molar excess of TgRON2sp) and reservoir solution, and equilibrated against 100 µL of reservoir solution. Cryoprotection was performed in 12.5% glycerol for 20 s and flash cooled at 100K directly in the cryostream. Diffraction data to 1.95 Å (TgAMA1-TgRON2sp) and 2.55 Å (TgAMA1 Y230A-TgRON2sp) resolution were collected on beamline 08ID-1 (CMCF) at the Canadian Light Source (CLS; Saskatoon, SK). Diffraction quality crystals for TgAMA1 Y230A were grown in sitting drops consisting of 0.8 µL protein (14 mg/mL) and 0.8 µL reservoir solution (21% PEG3350, 100 mM Tris pH 7.4), and equilibrated against 100 µL of reservoir solution. Cryoprotection of the crystal was carried out in 12.5% glycerol for 20 s and flash cooled at 100K directly in the cryostream. Diffraction data to 2.35Å were collected on beamline 9-2 at the Stanford Synchrotron Radiation Laboratory (SSRL; Menlo Park, CA).

### E) Data processing, structure solution and refinement

Diffraction data were processed using Imosflm⁴⁰ and Scala⁴¹ in the CCP4 suite of programs⁴². Initial phases were obtained by molecular replacement using PHASER⁴³ with the unliganded TgAMA1 structure (PDB 2X2Z). Tracing of the RON2 peptide was performed manually in COOT44 with subsequent addition of solvent atoms and refinement in Refmac5⁴⁵. Stereochemical analysis was performed with PROCHECK and SFCHECK in CCP4⁴². Data processing and refinement statistics are presented in Table 1.

### F) Bioinformatics

RON2 sequences were aligned using ClustalW⁴⁶ and illustrated in ESPript⁴⁷. Acquisition numbers for aligned sequences are as follows: Toxoplasma gondii (TGME49_100100), Neospora caninum (NCLIV_064620), Plasmodium falciparum (PF14_0495), Plasmodium vivax (PVX_117880), and Babesia bovis (BBOV_I001630).

Binding assays. Techniques for parasite culture, site-directed mutagenesis, fluorescence imaging, and immuno blotting, as well as analysis of the interaction between TgAMA1 and TgRON2 in ELISA, mammalian BHK-21 cells and on RH tachyzoites were conducted as described in previously published protocols6. For binding assay using BHK21 cells, 0.1 µg/mL to 10 µg/mL of recombinant TgRON2 were used (no binding was observed below 0.1 µg/mL for wild-type TgRON2-2). For ELISA, TgAMA1 was coated at 1 µg/mL and TgRON2-2 recombinant proteins used at 0.0009 µg/mL to 10 µg/mL. For competitive ELISA assays, TgRON2-2 was used at 1 µg/mL and pre-incubated with increasing concentrations of TgRON2 synthetic peptide for 15 min before being added to each well. To test binding of recombinant TgRON2-2 proteins on tachyzoites, recombinant TgRON2 proteins were used at concentrations ranging from 0.04 µg/mL to 10 µg/mL. TgRON2sp-FITC was used at 2 µg/mL for 30 min on fixed and permeabilized intracellular tachyzoites.

SPR studies of the TgAMAl-TgRON2-2 assembly were performed on a BIACORE 3000 instrument (GE Healthcare, Biacore AB Uppsala, Sweden) at 25°C using running buffer HBS-EP (10 mM HEPES pH 7.4, 150 mM NaCl, 3 mM EDTA and 0.005% Biacore^{™} surfactant), at a flow rate of 50 µL/min. For GST-capture of recombinant TgRON2-2, monoclonal anti-GST (GE Healthcare) was covalently immobilized on a flow cell of a CM5 sensor chip with EDC/NHS according to the manufacturer's instructions. A control flow cell was prepared with the same chemical treatment without antibody. For affinity measurements, each concentration of TgAMA1 or TgAMA1 Y230A was injected following an injection of TgRON2-2 over 2 min at 910 nM. Regeneration of the flowcell between each run was achieved with a pulse of 10 mM HCl after a 400 s dissociation step. The binding level of TgAMA1(520 nM) on TgRON2-2 mutants was measured at 125 s, immediately after the end of injection. For determination of TgRON2sp affinity, TgAMA1 was covalently immobilized on a CM5 sensor chip by amine coupling and TgRON2-2sp was injected at different concentrations. A pulse of 100 mM HCl was used to regenerate the TgAMA1 coated surface. The kinetic and affinity data were calculated using the BIAevaluation 4.1 software using a Langmuir 1:1 fitting model.

### G) Invasion assays

Inhibition of invasion was performed as described previously⁶ with 200 µg/ml (6 µM) of recombinant TgRON2 proteins. Intracellular parasites were detected by immunofluorescence microscopy (IFA) using anti-ROP1 that labelled the nascent PV. When using TgRON2sp, concentrations ranging from 8 nM to 5 µM were used.

### H) Statistical analysis

Statistical analyses were performed in GraphPad Prism 4 for Windows, with Students's t-test (unpaired, equal variance, two-tailed test) for comparisons with data that fit a normal distribution.

### Example 2: Refining the TgAMA1 binding site on TgRON2-2

We have shown previously that a recombinant, truncated form of TgRON2 (TgRON2-2), corresponding to a predicted surface exposed region, is capable of binding TgAMA1⁶. While this study established the general location of RON2 recognized by AMA1, it is unlikely that the entire 65 residue sequence of TgRON2-2 is necessary for complex formation. Thus, as a first step towards refining our description of the TgAMAl-TgRON2 assembly, we generated deletion constructs targeting both N-(TgRON2-2 D1) and C-termini (TgRON2-2 D2) of the predicted TgRON2 extracellular loop (Fig. 1a). Despite an in vitro cellular binding assay showing that both deletion constructs were capable of binding TgAMA1 (Fig. 1b), surface plasmon resonance (SPR) and invasion studies revealed an important role for the N-terminal region of TgRON2-2 while the C-terminal region appeared to be dispensable (Fig. 1c). The central region of TgRON2-2 is also likely to be important for binding to TgAMA1 as it harbours a pair of highly conserved cysteines that may form a disulfide bond endowing this region with critical internal structure. Guided by these observations and secondary structure predictions, we synthesized a 37 amino acid, disulfide-cyclised peptide (TgRON2 synthetic peptide - TgRON2sp) corresponding to residues 1297-1333 (Fig 1a). We subsequently demonstrated the functional relevance of this peptide in binding TgAMA1 using cellular binding assays (Fig. 1b), host cell invasion-inhibition assays with nanomolar concentrations of TgRON2sp (Fig. 1d) (SPR determined KD of 1.42 x 10-8 M; Fig. 7a), and competition ELISA assays with TgRON2-2 (Fig. 7b). These studies, therefore, indicate that the 37 residue TgRON2sp retains the functional binding characteristics of TgRON2-2.

### Example 3: Structural rearrangement induced by TgRON2sp

Based on the identification and characterization of TgRON2sp we next sought to establish an architectural blueprint of the AMA1-RON2 complex. To this end, we crystallized TgAMA1 in complex with TgRON2sp and refined the structure to a resolution of 1.95 Å (Table 1). A preliminary difference map revealed clear electron density in the apical groove of TgAMA1 enabling accurate tracing of the entire TgRON2sp sequence (Fig. 2a). Intriguingly, the bound TgRON2sp did not simply dock into the center of the apical groove, but rather bound in a U-shaped conformation where its α-helical N-terminus extended through an ordered coil region along one side of the AMA1 groove to a disulfide-tethered beta-hairpin loop and then folded back along the opposite side of the groove through a coil region leading to its C-terminus. By adopting this U-shaped binding strategy, the TgAMAl-TgRON2sp interface results in an extensive buried surface area of 3765 Å2 (Fig. 8a-8f, Table 2) and benefits from significant shape complementarity (complexation significance score of 1.00²⁶). These features likely endow the MJ with the ability to withstand the mechanical sheer force associated with pulling the parasite through the constricted MJ ring.

A second noteworthy feature of the TgAMAl-TgRON2sp co-structure is the dramatic conformational change in the DII loop that defines one end AMA1 apical groove (Fig. 2b). This observation is intriguing for two primary reasons. First, the DII loop is predicted to be one of the most influential substructures involved in immune escape and RON2 coordination^{22,21,27,28} in T. gondii and Plasmodium spp and thus its rearrangement likely reflects a critical regulatory event in MJ assembly. Second, the DII loop is exceptionally well ordered in the apo TgAMA1 structure ²² where it is intimately associated with the ectodomain core through numerous interactions, including a pair of tryptophan residues that anchor the loop into hydrophobic pockets in the apical groove (Fig. 2b - orange). By inducing the conformational changes leading to displacement of the DII loop, TgRON2sp is able to expose the fully functional binding surface on TgAMA1 that displays enhanced shape and charge complementarity. For example, in its reconfigured form, the apical groove is less hydrophobic, instead relying on an extended basic patch to complements acidic patches on the TgRON2sp N-terminal helix and C-terminal tail (Fig. 2c). In addition to establishing an essential role for the DII loop in regulating access to the apical groove, the induced conformational changes also suggest a stepwise binding mechanism where contact is initiated between the end of the AMA1 apical groove opposite the DII loop, and the cystine loop region of RON2. Using this strategy, the binding energy harnessed from the initial interactions can be applied to reconfiguring the DII loop and completing binary complex formation.

### Example 4: Defining hot spot residues on TgRON2sp

Guided by our high-resolution TgAMA1-TgRON2sp co-structure (Fig. 8a-8f) and sequence alignments (Fig. 3a), we identified individual residues and substructures on TgRON2-2 and probed their functional relevance using SPR, ELISA, cellular binding and invasion inhibition assays. In general, alanine substitutions in the N-terminal helix (D1297, H1301, D1304 and I1305), the coil connecting the helix and cystine loop (P1309), the cystine loop (C1313, I1318, L1319 and C1323), and the C-terminal coil (T1332) all showed some degree of reduced binding to TgAMA1 as determined by SPR and ELISA (Fig. 3b, 10a-10b) consistent with an extensive, composite TgAMAl-TgRON2sp interface. Of particular note, residues D1297, D1304, P1309, C1313, and C1323 all broadly conserved across Apicomplexan were identified as critical for TgAMA1 binding as was the less conserved L1319 located between the cysteines in the cystine loop. To corroborate the SPR based binding studies, we also tested the ability of the TgRON2-2 mutants to bind TgAMA1 in the micronemes of T. gondii tachyzoites (Fig. 11) and inhibit host cell invasion by T. gondii (Fig. 3c). Collectively these data highlight an extensive binding surface and point toward the importance of the cysteine-dependent conformation of RON2 in promoting complex formation consistent with our proposed stepwise binding strategy.

### Example 5: Probing the hydrophobic groove of TgAMA1

The only previous attempt to identify residues on AMA1 critical for MJ assembly was based on a PfAMA1 mutagenesis study that implicated the central tyrosine (Tyr251) in the hydrophobic groove in complex formation²⁸. Thus, we began our investigation into defining residues on TgAMA1 essential for TgRON2 binding by probing the analogous central tyrosine (Tyr230). When we produced recombinant TgAMA1 Y230A (Fig. 12a) and measured binding to TgRON2-2 in cell-based (Fig. 12b) and ELISA (Fig. 12c) assays, no discernable difference in binding was observed. However, SPR analysis revealed an order of magnitude weaker binding between TgRON2-2 and TgAMA1 Y230A (KD of 10-8 M) relative to native TgAMA1 (KD of 10-9 M) (Fig. 12d, 12e). This difference is significantly less than what would be expected as a result of mutating a major "hot spot" residue. To investigate the structural basis of these observations, we solved the crystal structures of TgAMA1 Y230A in the apo and TgRON2sp bound forms (Table 1). While analysis of the TgAMA1 Y230A structure revealed a conserved core (r.m.s.d of 0.60Å over 384 α-carbons) relative to native TgAMA1²², an alternate conformation was observed for the tip of the DII loop proximal to the truncated tyrosine side chain (Fig. 4a). More specifically, Trp353 was rotated 67° to occupy the newly exposed space above Ala230 (Fig. 4a inset). Ultimately, however, binding of TgRON2sp resulted in a similar displacement of the DII loop as observed in the native complex (Fig. 4b) consistent with a largely conserved binary interface. The most notable effect resulting from substituting Tyr230 with an alanine is the loss of two (bifurcated) hydrogen bonds, out of twenty three observed in TgAMAl-TgRON2sp (Fig. 4b inset), that likely corresponds to the reduced affinity measured by SPR.

To further probe the role of specific AMA1 residues in promoting binary complex formation, we performed a detailed analysis of the buried surface areas and hydrogen bonding contributions (Fig. 8a-8f, Table 2), and filtered these through a sequence alignment (Fig. 13). Finally, we selected a set of eleven TgAMA1 residues (Fig. 13) to investigate and tested the ability of TgRON2-2 to bind these mutant forms of TgAMA1 expressed on the surface of BHK-21 cells (Fig. 4c, 14). While the individual substitutions of R111 and Y241 (base of the N-terminal helix); Y110 and Y215 (coil connector); F163, I171 and M204 (cystine loop); and E145 (C-term coil) to alanine showed no discernable binding effects, substitutions of F197 (base of cystine loop) or Y213 (base of tyrosine cage surrounding P1309) to alanine, impaired binding (Fig. 4c, 14). In fact, Y213A showed no binding at any of the concentrations tested suggesting that this highly conserved residue is a critical anchor point during complex formation. The F197A mutant showed a more moderated effect with binding disrupted only at the lowest concentration. Thus the F197A variant provided an opportunity to test the potential of synergistic TgAMAl-RON2 mutations. Total abrogation of complex formation was observed between TgAMA1 F197A and either TgRON2-2 I1318A or L1319A (tip of the cystine loop) (Fig. 4c, 14). These synergistic effects prompted us to generate three additional TgRON2-2 constructs comprising 1) from the N-terminal helix to cystine loop, 2) from the coil connector to C-terminus, and 3) from the N-terminal helix to coil connector (Fig. 15a-15c). Analysis of these constructs by ELISA demonstrated clear binding for the first two, but no interaction with the third, effectively isolating the most critical region of TgRON2-2 to the cystine loop (Fig. 15a-15c). Collectively, these results allow to refine our proposed mechanism of binding where the cystine loop in combination with the Tyr213-Pro1309 bridge act as a primary structural brace to anchor the peptide in the apical groove and promote displacement of the DII loop.

### Example 6: TgAMAl-TgRON2sp: a structural perspective

Among the diverse mechanisms employed by viruses, pathogenic bacteria, and protozoan parasites to enter host cells, one commonality is the utilization of protein complexes to promote host cell attachment and invasion (Fig. 6). Many of the current models describing these processes are derived from seminal structural studies including the Nipah virus attachment glycoprotein in complex with human Ephrin B2 (buried interface of 2786 Å2)³⁶, HIV gp120 with human CD4 (2051 Å2)³⁷, the enteropathogenic E. coli translocated intimin receptor (Tir) with intimin (1294 Å2)³⁸ and Listeria monocytogenes Internalin A with host cell E-cadherin (2630 Å2)³⁹. Our structural characterization of TgAMA1 in complex with TgRONsp is an important addition to these studies as it is the first such complex from a protozoan parasite and incorporates one of the most extensive buried surface areas (3765 Å2) of any host-pathogen binary protein complex. The induced conformational changes in TgAMA1 upon TgRON2sp also provide an illustrative framework to predict secondary roles associated with conformational changes such as protecting surfaces from exposure to the immune system. The extended structure of the DII loop that spans much of the AMA1 ectodomain (Fig. 2b, 9a-9b) may also poise it to function as a molecular switch transmitting a signal from the reorganized apical groove to the parasite cell surface. For example, RON2 binding could trigger such processes as the association of the AMA1 C-terminal tail with the glideosome to move the junction, or alternatively, facilitate intramembrane proteolytic processing of AMA1, which has recently been shown to trigger the transition of T. gondii from invasive to replicative mode⁹. While the prediction of a molecular switch is speculative, the structural characterization of the TgAMAl-TgRON2sp binary complex reported here provides a critical step towards establishing a structural link between the parasite and the host cell cytoskeleton during invasion.

### Example 7: PfAMA1-RON2 co-structure

The PfAMA1 construct used for crystallization with PfRON2 encompasses residues 104 to 438 (PlasmoDB Gene ID: PF11_0344, P. falciparum 3D7), with numbering based on the initiation methionine in the signal sequence. This construct is identical to that used for the original crystallization of apo PfAMA1 by Bai et al in 2005 ²⁵, and includes domains I and II of the fully processed N-terminal ectoplasmic region. The PfRON2 construct is based off of both a sequence alignment with the TgRON2 peptide used for successful co-crystallization with TgAMA1 and experimental data suggesting that this region is critical for erythrocyte invasion by P. falciparum (Lamarque et al 2011⁶). The 39 amino acid PfRON2 peptide includes residues 2021 to 2059 (PlasmoDB Gene ID: PF14_0495, P. falciparum 3D7), with numbering based on the initiation methionine in the signal sequence. The X-ray crystal co-structure of PfAMA1-PfRON2 peptide in P21 was solved to 2.20 Å and refined to an Rcryst of 17.5 and an Rfree of 23.5. Residues 108 to 438 are modeled for PfAMA1, including apical surface loops that were unmodeled in the apo structure, with the exception of the extended DII loop that is disordered between residues 350 and 388 due to its displacement upon PfRON2 binding. The PfRON2 peptide is modeled from residues 2023 to 2058, and adopts a U-shaped conformation in the apical hydrophobic groove of PfAMA1. The N-terminal region of the PfRON2 peptide is assembled into an alpha-helix that overlays exceptionally well with the displaced DII loop helix of PfAMA1. A segment of ordered coil connects this helix to a disulfide bound beta-hairpin structure anchored in the opposite end of the groove, and while the overall binding paradigm is conserved with TgAMAl-TgRON2 significant structural and chemical differences are localized to this cystine loop.

### Example 8: AMA1-binding PfRON2-2 peptide inhibits host cell invasion by Plasmodium merozoites

The synthetic peptide PfRON2sp that encompassed residues 104 to 438 (PlasmoDB Gene ID: PF11_0344, P. falciparum 3D7) was tested if it could also prevent Plasmodium merozoites entry into red blood cells. Highly synchronized *P. falciparum* 3D7 mature schizonts were incubated either with R1 peptide as a positive control of invasion inhibition, or PfRON2sp. Equal molar concentrations of recombinant proteins were used relative to R1 peptide concentration. Blood smears were performed 16 hours post-invasion and counting of ring-stage parasites allowed us to evaluate the invasion efficiency. As shown in Figure 16, R1 peptide was able to decrease *P. falciparum* 3D7 invasion capacities to 60 % at 10 µg/ml (4 µM) and to 12 % at 50 µg/ml (20 µM), which is consistent with what has already been reported (Harris KS, Casey JL, Coley AM, Masciantonio R, Sabo JK, et al. (2005) Binding hot spot for invasion inhibitory molecules on Plasmodium falciparum apical membrane antigen 1. Infect Immun 73: 6981-6989). Addition of PfRON2sp showed a significant higher inhibition at 4 µM or 20 µM than R1 peptide. This shows that PfRON2sp interferes with PfAMA1-PfRON2 interaction and block Plasmodium invasion similarly to what it has been observed for Toxoplasma. Moreover, it displays a very potent inhibitor effect.

Here we present a comprehensive structural and functional characterization of Toxoplasma AMA1 in complex with the receptor portion of the RON2 ectodomain. Structural analysis reveals that RON2 binding induces substantial conformational change in the apical surface of AMA1. Data from mutagenesis, binding and invasion studies support a stepwise assembly mechanism where a disulfide bound substructure on RON2 is initially braced against a conserved Tyr-Pro platform in the apical groove of AMA1, thereby effecting the necessary conformational changes to establish a functional binding site. The resulting buried surface area is amongst the largest reported for analogous host pathogen binary protein complexes and likely serves a strategic role in sufficiently stabilizing the assembly to resist the considerable mechanical forces encountered as the parasite enters the host cell through the constricted MJ ring. The structure of Toxoplasma AMA1-RON2 complex in conjunction with the structure of the analogous Plasmodium complex further demonstrate a crucial role for the RON2 cystine loop and associated binding surface on AMA1 in regulating cross species specificity. This structure of the TgAMAl-TgRON2 complex in conjunction with a model of the analogous Plasmodium complex offers an important perspective on host pathogen interactions in general as it represents the first protein-protein, ligand-receptor complex from a protozoan parasite.

These data provide the first structural blueprint of a host-protozoan parasite protein complex enabling us to establish a structural paradigm for host cell invasion and guiding the design of improved vaccines and invasion inhibitory small molecules targeting these devastating global pathogens.

**Table 1. Data collection and refinement statistics (Molecular replacement)**

| | | *Tg*AMA1 Y230A | *Tg*AMA1 Y230A-RON2sp | *Tg*AMA1-RON2sp |
|---|---|---|---|---|
| **Data collection** | | | | |
| | Spacegroup | P1 | P2₁ | P2₁ |
| | a, b, c (Å) | 67 52, 76 09, 88 78 | 70 52, 96 83, 78 33 | 69 86, 96 38, 78 35 |
| | α, β, γ (deg) | 71 89, 73 37, 73 50 | 90, 116 75, 90 | 90, 115 64, 90 |
| | Wavelength | 0 9795 | 0 9795 | 0 9795 |
| | Resoluhon (Å) | 38 59 - 2 35 (2 48 - 2 35) | 62 97 - 2 55 (2 69 - 2 55) | 45 00 - 1 95 (2 06 - 1 95) |
| | Measured reflections | 152470 | 173635 | 314359 |
| | Unique reflections | 63415 | 30766 | 68179 |
| | Redundancy | 2 4 (2 4) | 5 6 (5 6) | 4 6 (4 5) |
| | Completeness (%) | 96 9 (96 1) | 100 0 (100 0) | 100 0 (99 9) |
| | *I*/σ*(I)* | 7 2 (1 9) | 11 9 (4 1) | 10 6 (3 7) |
| | R_{merge}^{a} | 0 100 (0 456) | 0 108 (0 415) | 0 105 (0 479) |

| **Refinement Statistics** | | | | |
|---|---|---|---|---|
| | Resolution (Å) | 38 59 - 2 45 (2 51 - 2 45) | 56 70 - 2 55 (2 62 -2 55) | 39 81 - 1 95 (2 00 - 1 95) |
| | R_{cryt}^{b} / R_{free}^{c} | 0 212/0 305 (0 300/0 400) | 0 185/0 263 (0 218/0 293) | 0 170/0 228 (0 207/0 263) |
| | No of atoms | | | |
| | Protem (A/B/D/E) | 3158/3178/3211/3122 | 3037/252/3091/245 | 3082/260/3131/252 |
| | Solvent | 533 | 212 | 604 |
| | Bonc Acid | N/A | 8 | 8 |
| | Glycerol | N/A | 18 | N/A |

| B-values (Å²) | | | | |
|---|---|---|---|---|
| | Protem (A/B/D/E) | 25 53/29 12/28 75/32 20 | 28 37/31 41/36 33/50 93 | 19 98/21 19/25 12/30 88 |
| | Solvent | 27 91 | 33 51 | 33 09 |
| | Bone Acid | N/A | 37 80 | 44 10 |
| | Glycerol | N/A | 51 27 | N/A |

| rms deviation from ideality | | | | |
|---|---|---|---|---|
| | Bond lengths (Å) | 0 014 | 0 016 | 0 020 |
| | Bond angles (deg) | 1 622 | 1 695 | 1 943 |

| Ramachandran statistics | | | | |
|---|---|---|---|---|
| | Most favoured | 94 6% | 95 3% | 97 5% |
| | Allowed | 5 4% | 4 7% | 2 5% |
| | Disallowed | 0 0% | 0 0% | 0 0% |

| | | | | |
|---|---|---|---|---|
| Values m parentheses are for the highest resolution shell ^{a} R_{merge}= ∑*ₕₖₗ* ∑*ᵢ*\|I*_{hkl j}* -[I*ₕₖₗ*]\| / ∑*ₕₖₗ* ∑ᵢ I*_{hkl i}*, where [I*ₕₖₗ*] is the is the average of symmetry related observations of a unique reflection ^{b} R_{cryst}=Σ\|F_{obs}-F_{calc}/∑F_{fobs}, where Fobs and F_{calc} are the observed and the calculated structure factors, respectively ^{c} R_{free} is R using 5% of reflections randomly chosen and omitted from refinement | | | | |

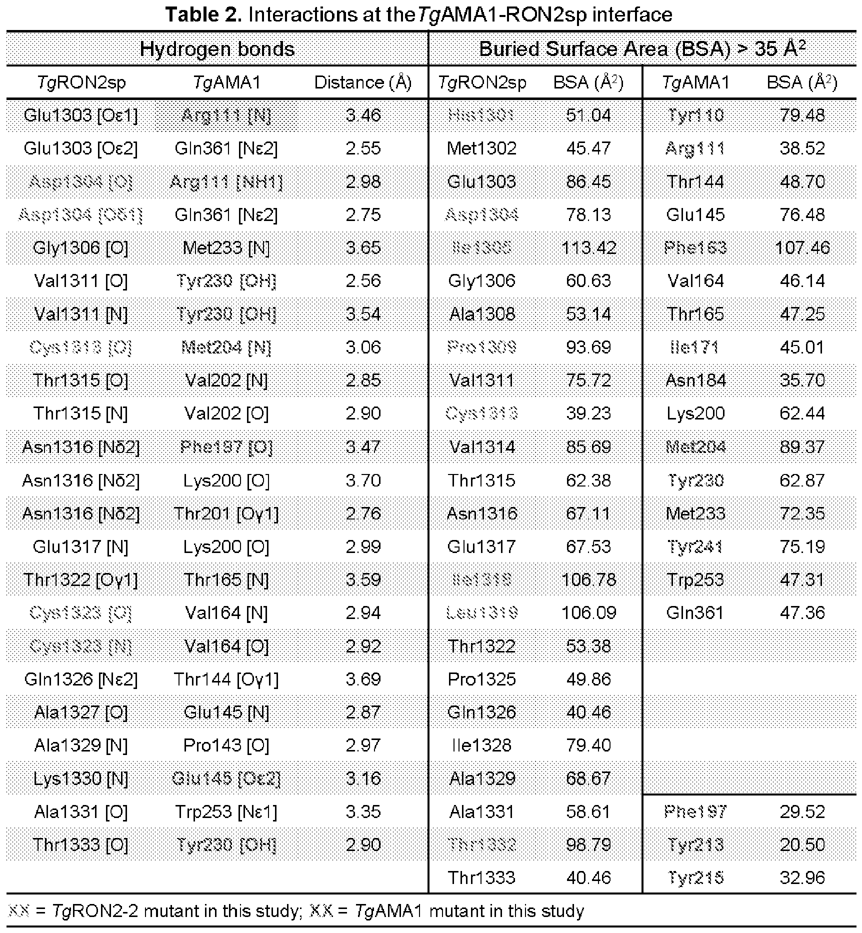

**Table 3. Primers used for mutagenesis and truncation construct generation**

| **Oligonucleotide name** | **Sequence** | **Construct/Mutant** |
|---|---|---|
| **ML567** | 5'-GATGGCACATTATACGCGGAGCCTGCGGGGTTG-3' | AMA1 R111A FOR |
| **ML568** | 5'-CAACCCCGCAGGCTCCGCGTATAATGTGCCATC-3 | AMA1 R111A REV |
| **ML559** | 5'-CTGATGGAGGGTAAAAAGGCCTGTTCAGTCAAGGGCGAAC-3' | AMA1 Y241A FOR |
| **ML560** | 5'-GTTCGCCCTTGACTGAACAGGCCTTTTTACCCTCCATCAG-3' | AMA1 Y241A REV |
| **ML593** | 5'-GTTGATGGCACATTAGCCCGGGAGCCTGCGGGGTT-3' | AMA1 Y110A FOR |
| **ML594** | 5'-AACCCCGCAGGCTCCCGGGCTAATGTGCCATCAAC-3' | AMA1 Y110A REV |
| **ML557** | 5'-CAATAAGGCGACGAAGGCCCGTTACCCATTTGTTTATGAC-3 | AMA1 Y213A FOR |
| **ML558** | 5'-GTCATAAACAAATGGGTAACGGGCCTTCGTCGCCTTATTG-3 | AMA1 Y213A REV |
| **ML533** | 5' GCGACGAAGTACCGTGCCCCATTTGTTTATGAC 3' | AMA1 Y215A FOR |
| **ML534** | 5'GTCATAAACAAATGG*GGC*ACGGTACTTCGTCGC3' | AMA1 Y215A REV |
| **ML591** | 5'-GAGGCTTCAACTTGAATGCCGTGACGCCTAGCG-3' | AMA1 F163A FOR |
| **ML592** | 5'-CGCTAGGCGTCACGGCATTCAAGTTGAAGCCTC-3 | AMA1 F163A REV |
| **ML563** | 5'-CCTAGCGGGCAGCGAGCTTCACCATTTCCGATG-3' | AMA1 I171A FOR |
| **ML564** | 5'-CATCGGAAATGGTGAAGCTCGCTGCCCGCTAGG-3' | AMA1 I171A REV |
| **ML561** | 5'-GGAGGTGCGCCGAGGCTGCCTTTAAGACGGTCG-3 | AMA1 F197A FOR |
| **ML562** | 5'-CGACCGTCTTAAAGGCAGCCTCGGCGCACCTCC-3 | AMA1 F197A REV |
| **ML589** | 5'-CCTTTAAGACGGTCGCTGCGGATAAAAACAATAAGGC-3' | AMA1 M204A FOR |
| **ML590** | 5'-GCCTTATTGTTTTTATCCGCAGCGACCGTCTTAAAGG-3' | AMA1 M204A REV |
| **ML565** | 5'-CTTGGAAGATGTTCCGACTGCAAAAGAATACAAACAGTCAGG-3 | AMA1 E145A FOR |
| **ML566** | 5'-CCTGACTGTTTGTATTCTTTTGCAGTCGGAACATCTTCCAAG-3' | AMA1 E145A REV |
| **M L 457** | 5'-CTGTGCCACATCCTCGCCGTATCGATGCAGCTG-3' | AMA1 Y230A FOR |
| **ML458** | 5'-CAGCTGCATCGATACGGCGAGGATGTGGCACAG-3' | AMA1 Y2301 REV |
| | | |
| **M L 525** | 5'-GTTCCGCGTGGATCCGTTGCACCGGTCTCCTGCGTGACGAAC-3' | TgRON2-2 D1 FOR |
| **M L 526** | 5'-GTTCGTCACGCAGGAGACCGGTGCAACGGATCCACGCGGAAC-3' | TgRON2-2 D1 REV |
| **ML 531** | 5'-CAGGCCATCGCCAAGGCCACCTTCGCAGGCATGGCGCCGTAC-3' | TgRON2-2 D2 FOR |
| **ML 532** | 5'-GTACGGCGCCATGCCTGCGAAGGTGGCCTTGGCGATGGCCTG-3' | TgRON2-2 D2 REV |
| **ML 459** | 5'-GCGCCGCCGGTCTCCGCCGTGACGAACGAGATC-3' | TgRON2-2 C1313A FOR |
| **ML 460** | 5'-GATCTCGTTCGTCACGGCGGAGACCGGCGGCGC-3' | TgRON2-2 C1313A REV |
| **ML 498** | 5'-ATCCTCGGAGTCACCGCCGCGCCGCAGGCCATC-3' | TgRON2-2 C1323A FOR |
| **ML 543** | 5'-GATGGCCTGCGGCGCGGCGGTGACTCCGAGGAT-3' | TgRON2-2 C1323A REV |
| **ML 569** | 5'-CGTGGATCCGTTGCAGCCATCGTGCAACATATG-3' | TgRON2-2 D1297A FOR |
| **ML 570** | 5'-CATATGTTGCACGATGGCTGCAACGGATCCACG-3' | TgRON2-2 D1297A REV |
| **ML 571** | 5'-GCAGACATCGTGCAAGCTATGGAAGACATTGGC-3' | TgRON2-2 H1301A FOR |
| **ML 572** | 5'-GCCAATGTCTTCCATAGCTTGCACGATGTCTGC-3' | TgRON2-2 H1301A REV |
| **ML 463** | 5'-GTGCAACATATGGAAGCCATTGGCGGCGCGCCG-3' | TgRON2-2 D1304A FOR |
| **ML 464** | 5'-CGGCGCGCCGCCAATGGCTTCCATATGTTGCAC-3' | TgRON2-2 D1304A REV |
| **M L 573** | 5'-CAACATATGGAAGACGCTGGCGGCGCGCCGCCG-3' | TgRON2-2 I1305A FOR |
| **ML 574** | 5'-CGGCGGCGCGCCGCCAGCGTCTTCCATATGTTG-3' | TgRON2-2 I1305A REV |
| **M L 461** | 5'-GACATTGGCGGCGCGGCGCCGGTCTCCTGCGTG-3' | TgRON2-2 P1309A FOR |
| **ML 462** | 5'-CACGCAGGAGACCGGCGCCGCGCCGCCAATGTC-3' | TgRON2-2 P1309A REV |
| **ML 575** | 5'-TGCGTGACGAACGAGGCCCTCGGAGTCACCTGC-3' | TgRON2-2 I1318A FOR |
| **ML 576** | 5'-GCAGGTGACTCCGAGGGCCTCGTTCGTCACGCA-3' | TgRON2-2 I1318A REV |
| **ML 577** | 5'-GTGACGAACGAGATCGCCGGAGTCACCTGCGCG-3' | TgRON2-2 L1319A FOR |
| **ML 578** | 5'-CGCGCAGGTGACTCCGGCGATCTCGTTCGTCAC-3' | TgRON2-2 L1319A REV |
| **ML 579** | 5'-GCCATCGCCAAGGCCGCCACGAGCGCCGCGAGA-3' | TgRON2-2 T1332A FOR |
| **ML 580** | 5'-TCTCGCGGCGCTCGTGGCGGCCTTGGCGATGGC-3' | TgRON2-2 T1332A REV |

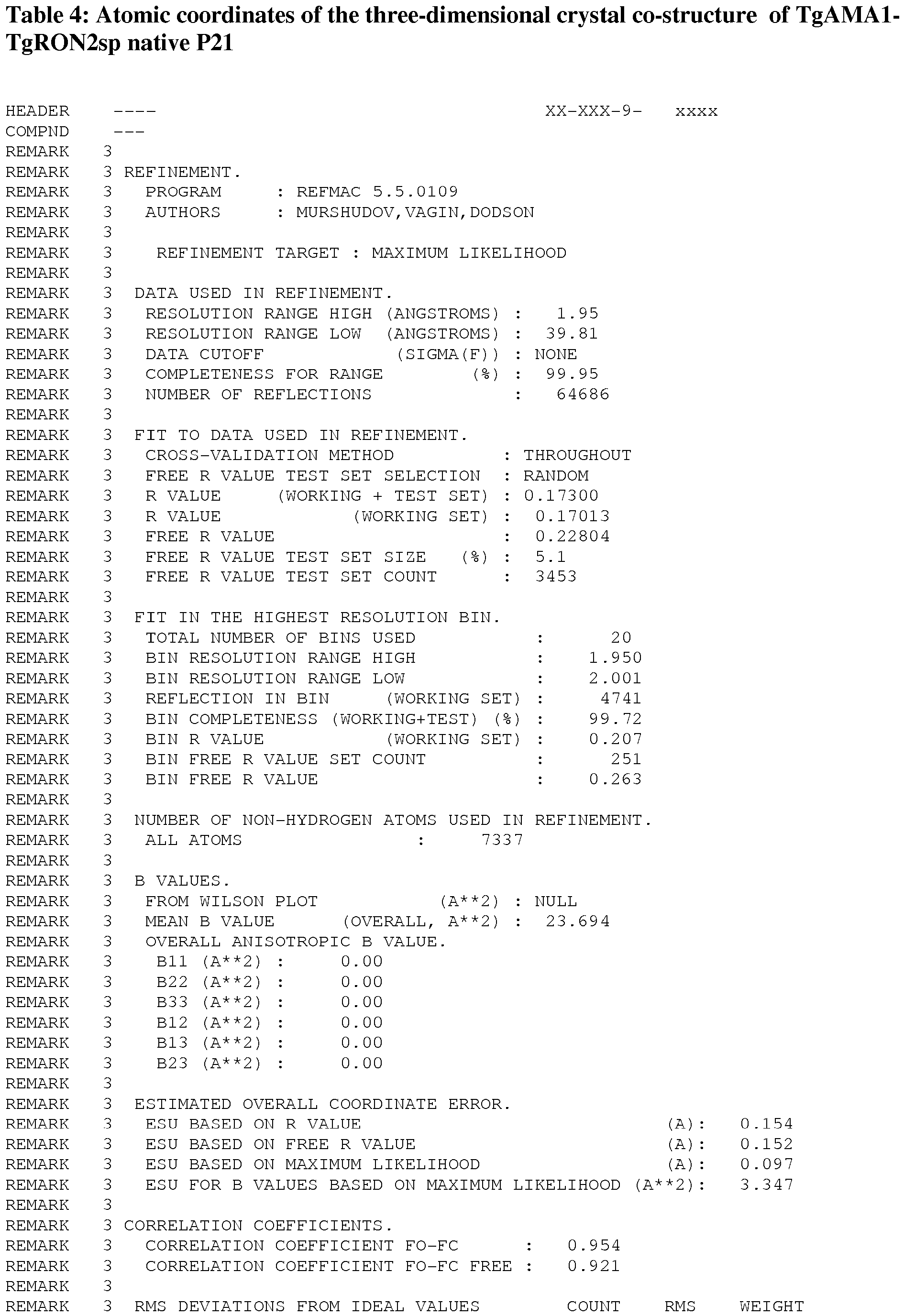

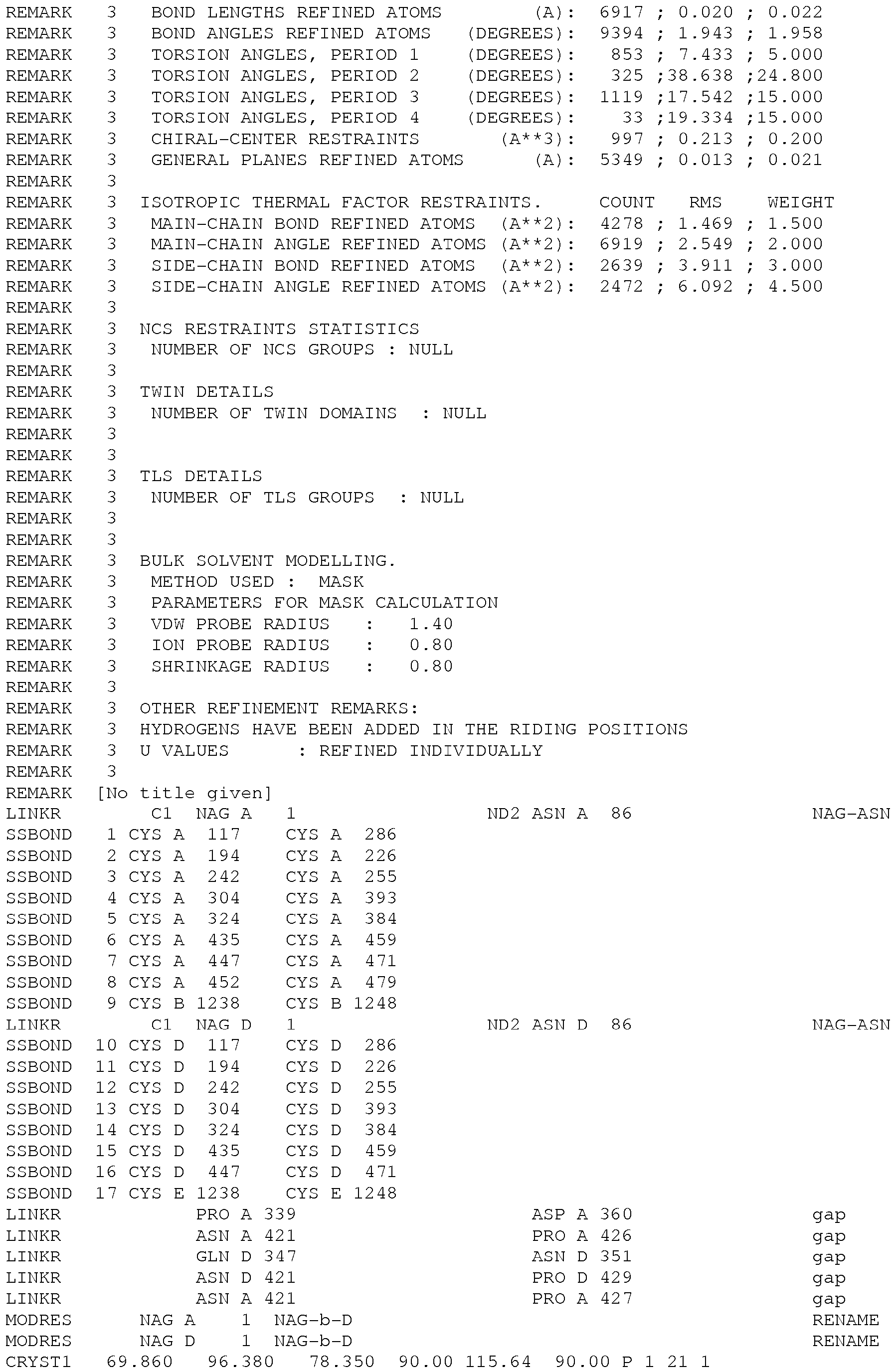

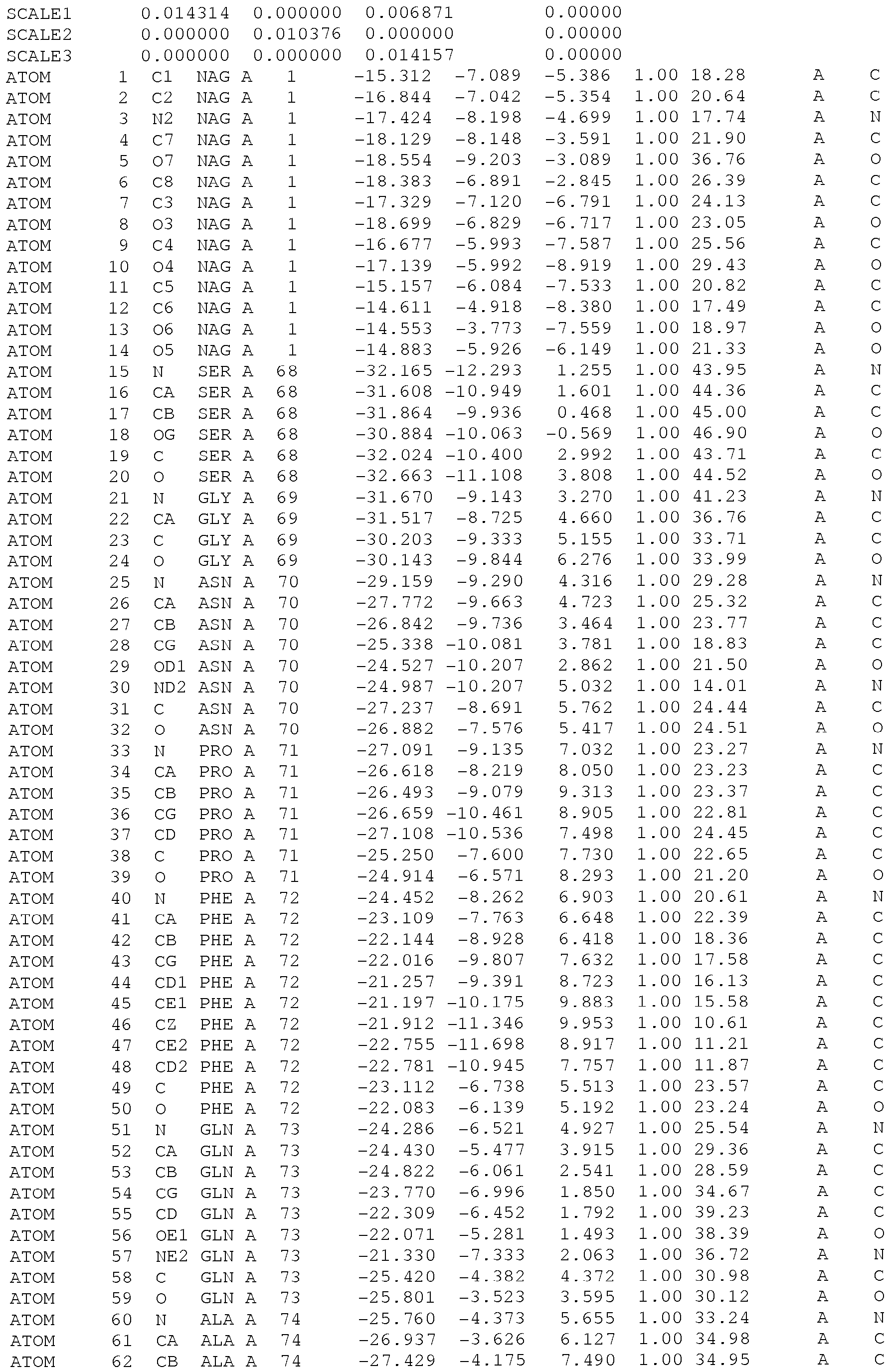

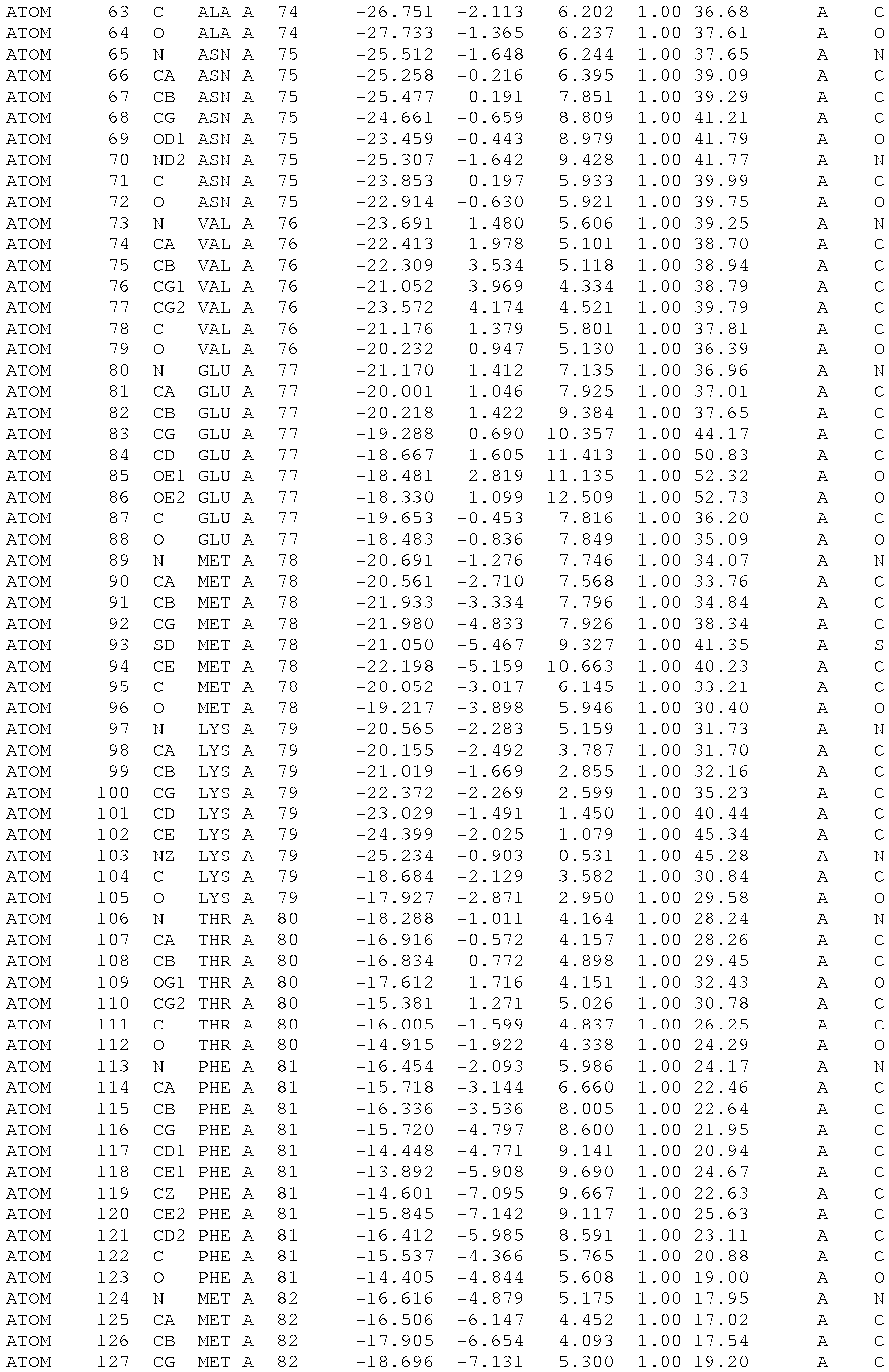

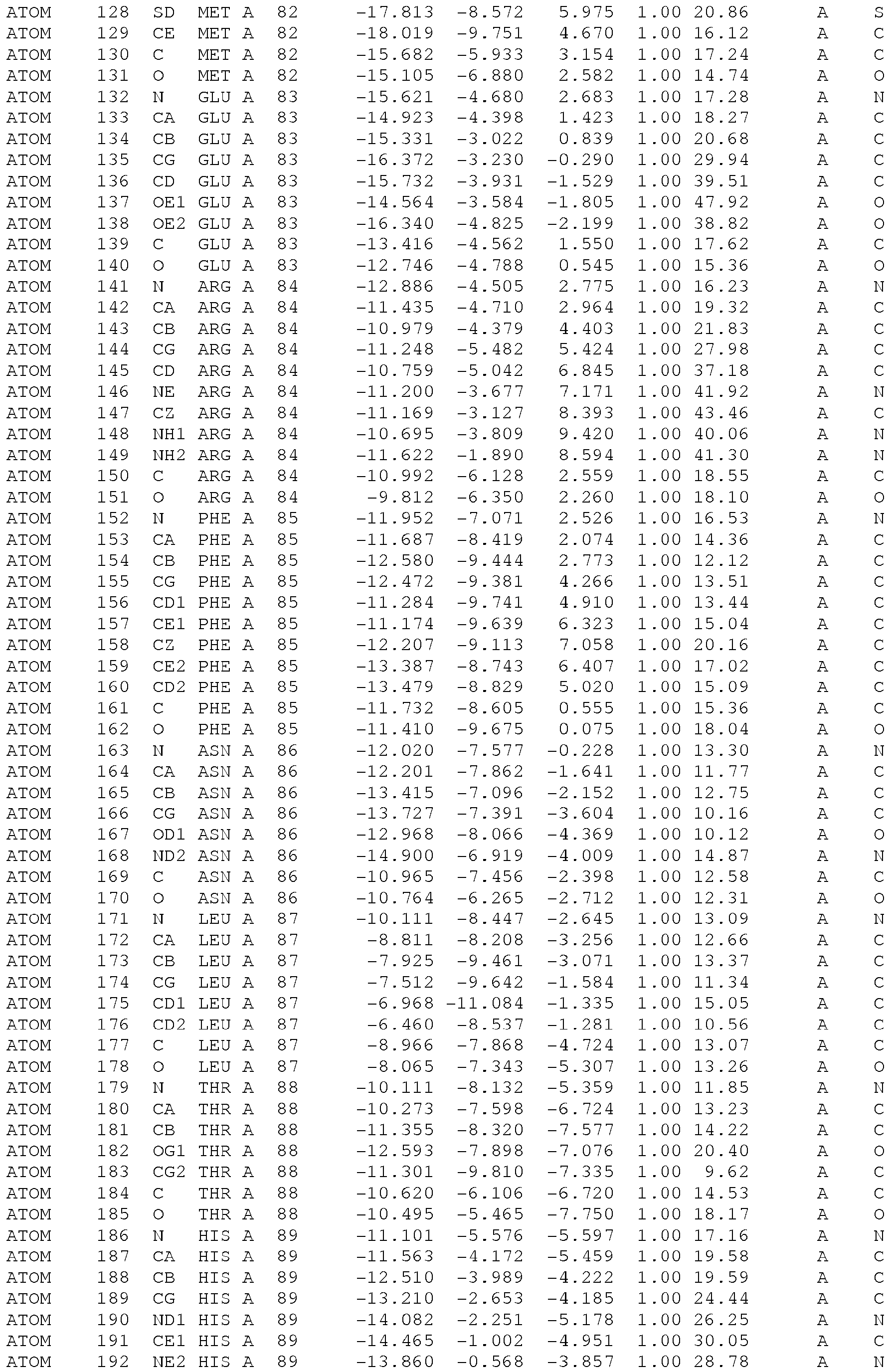

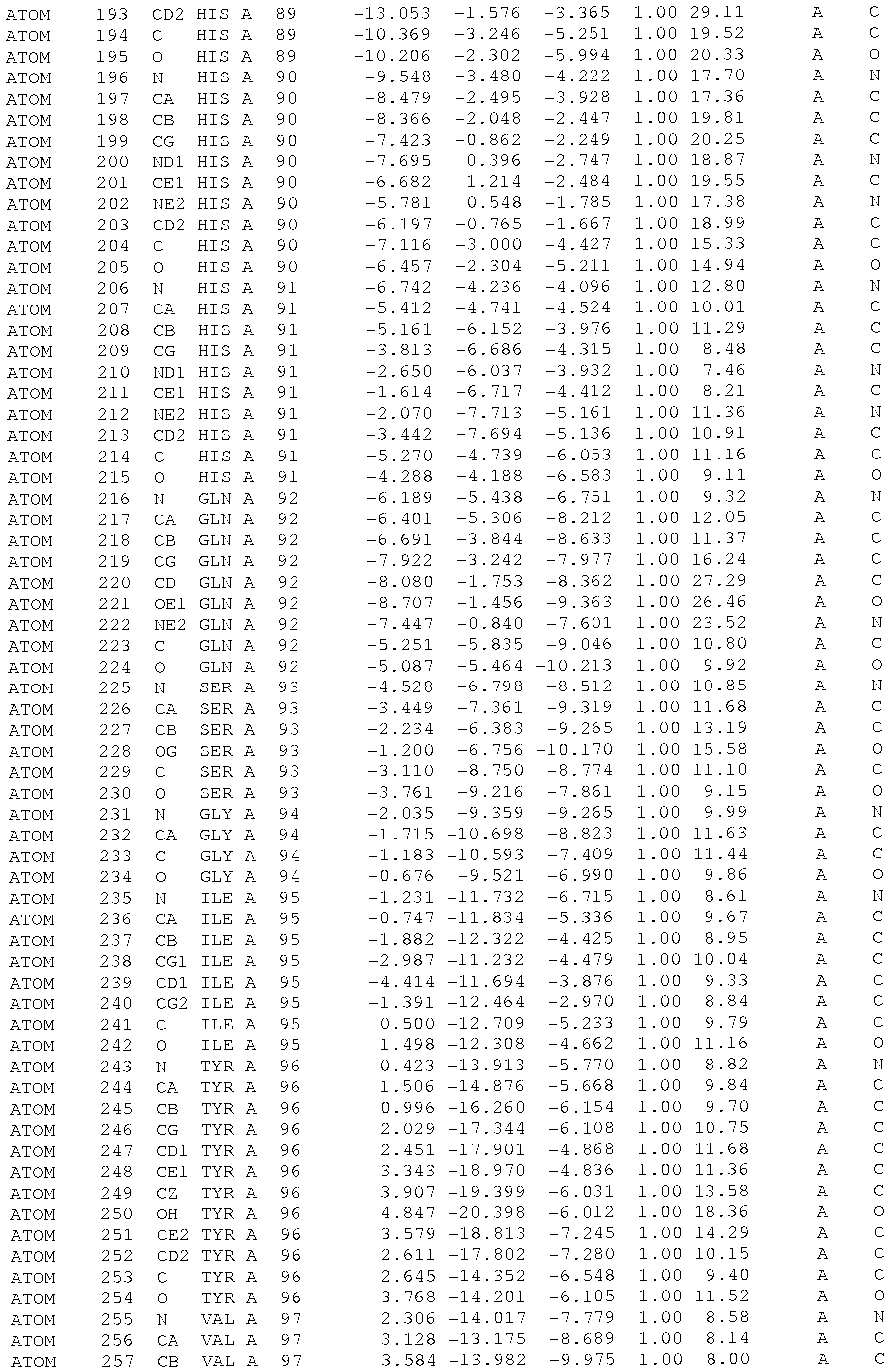

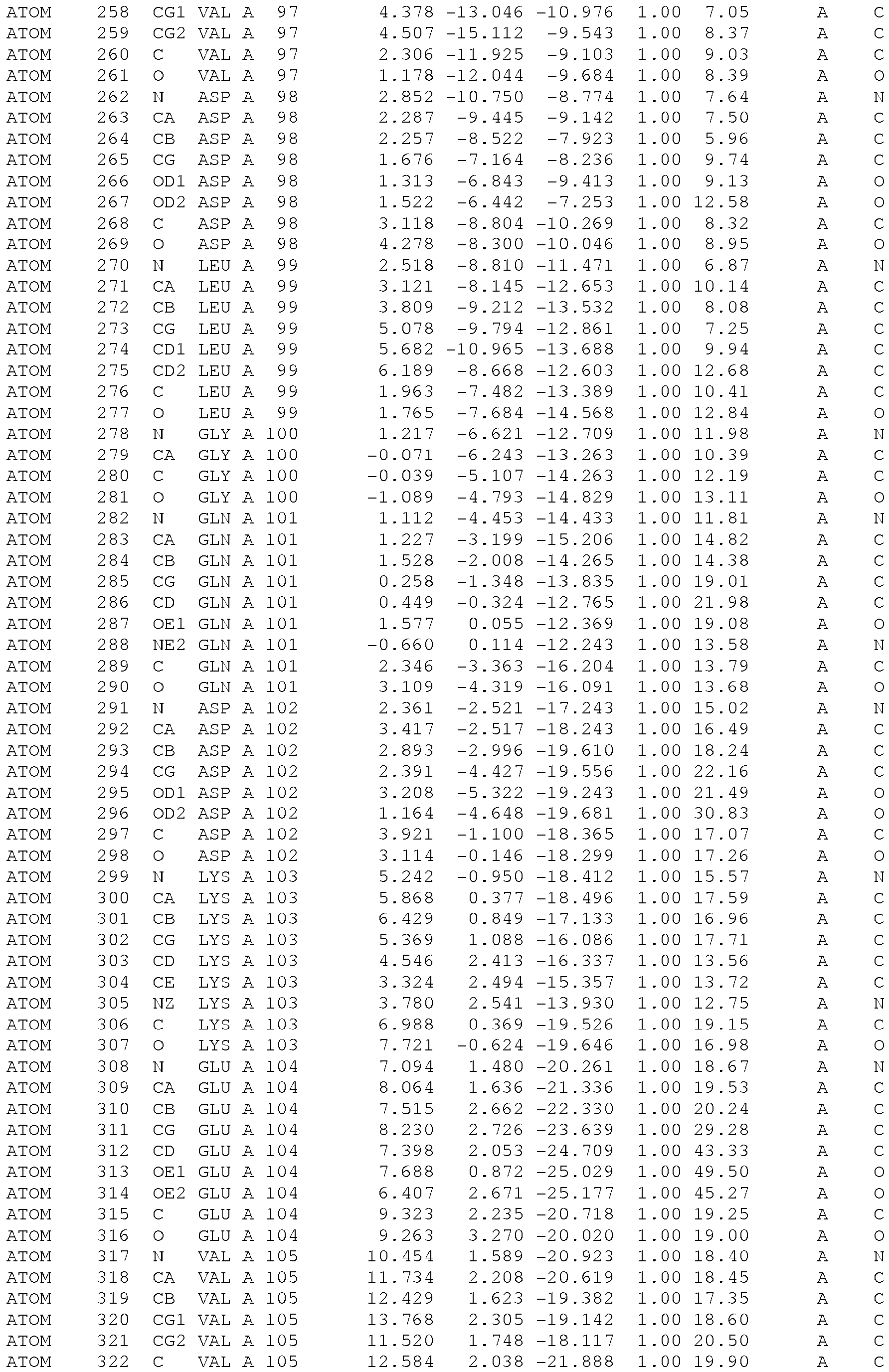

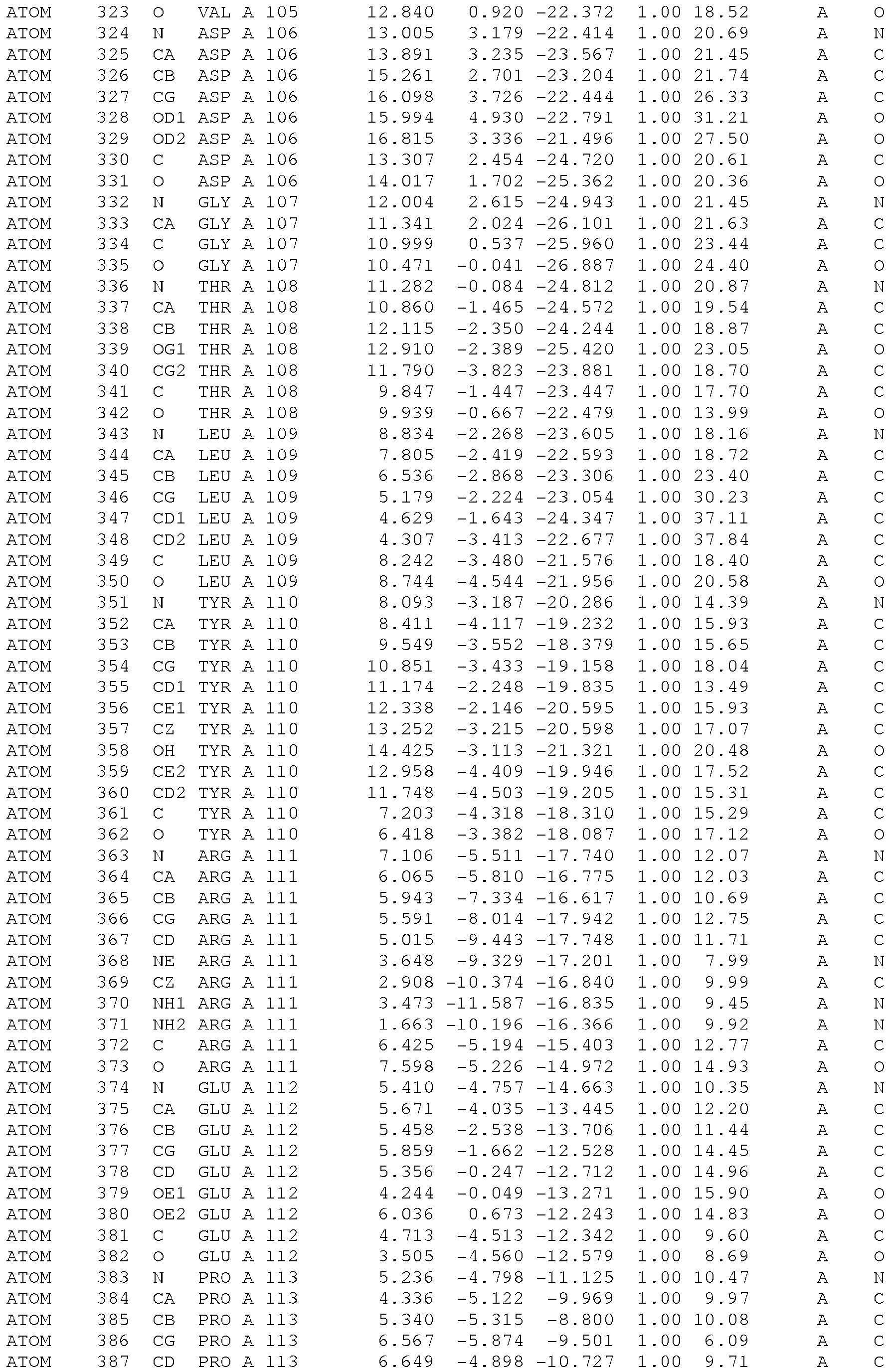

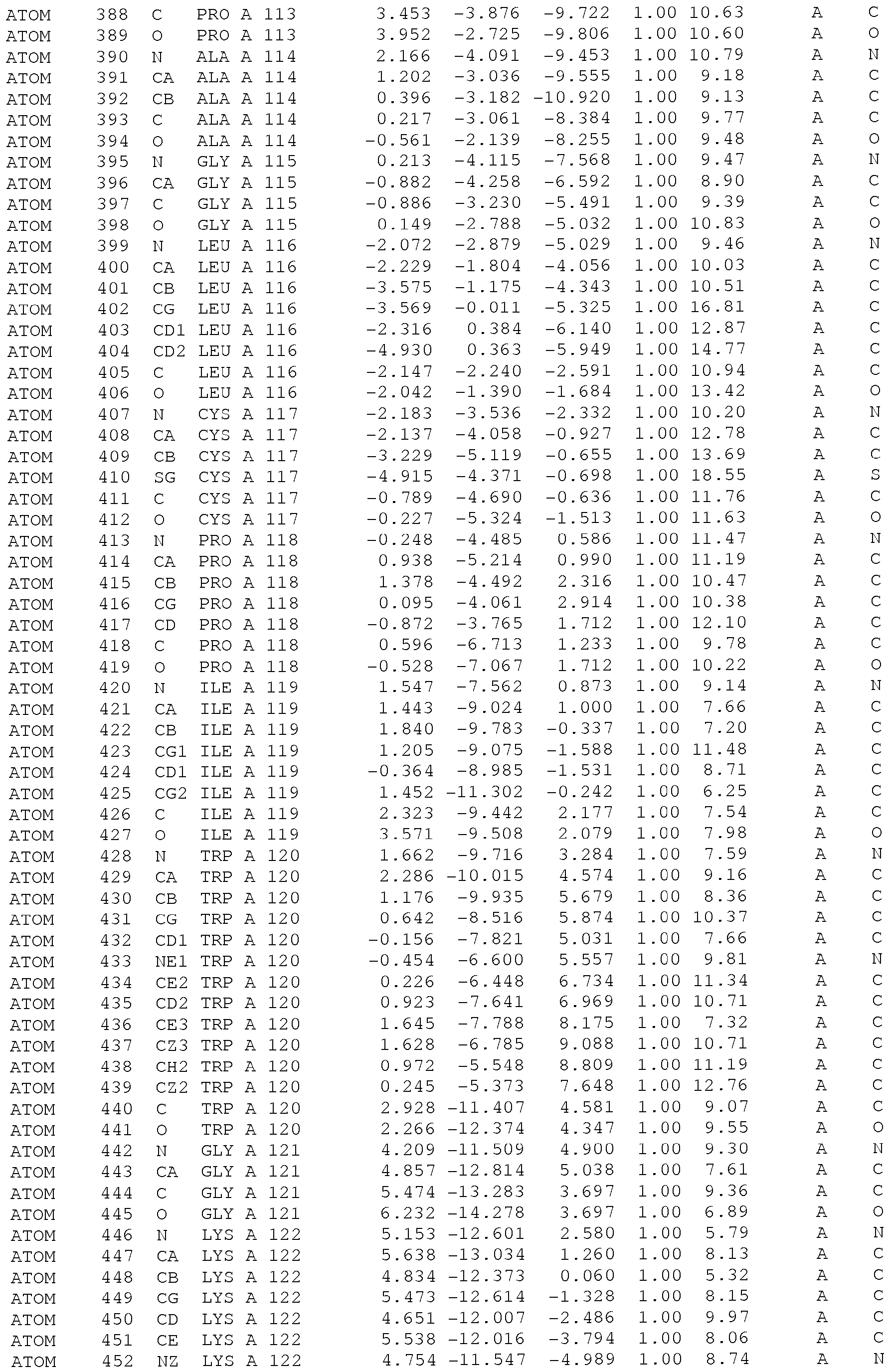

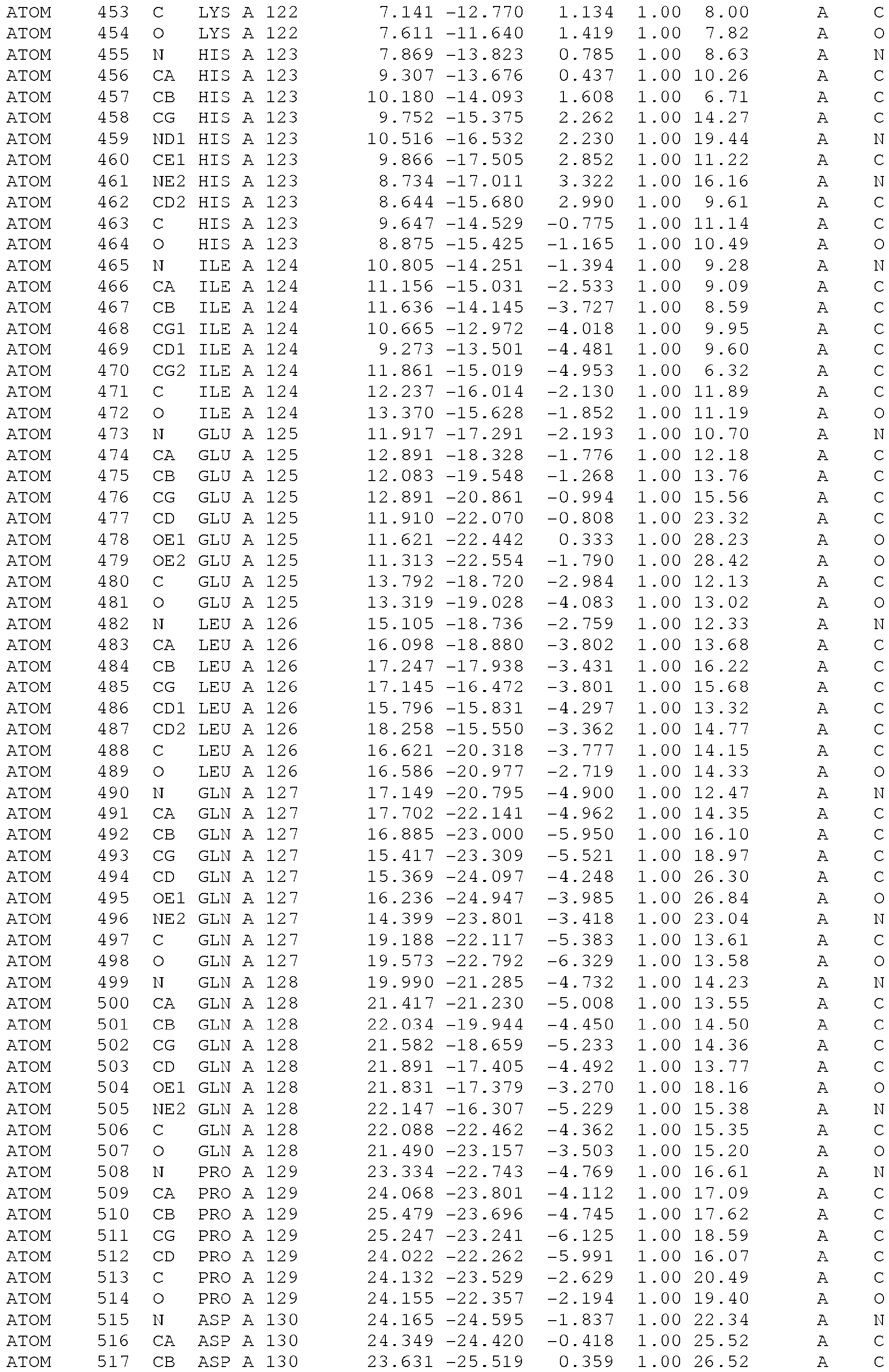

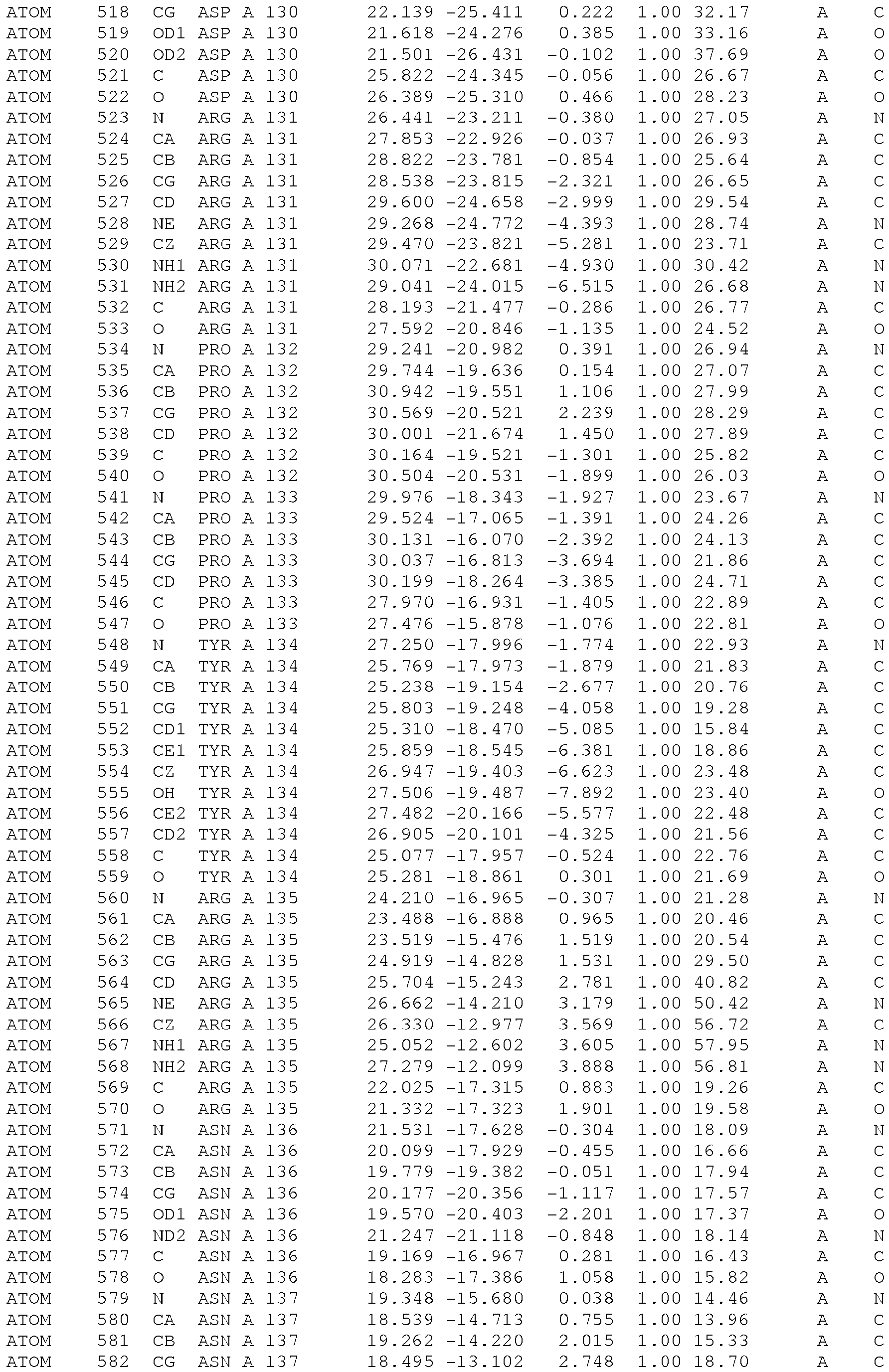

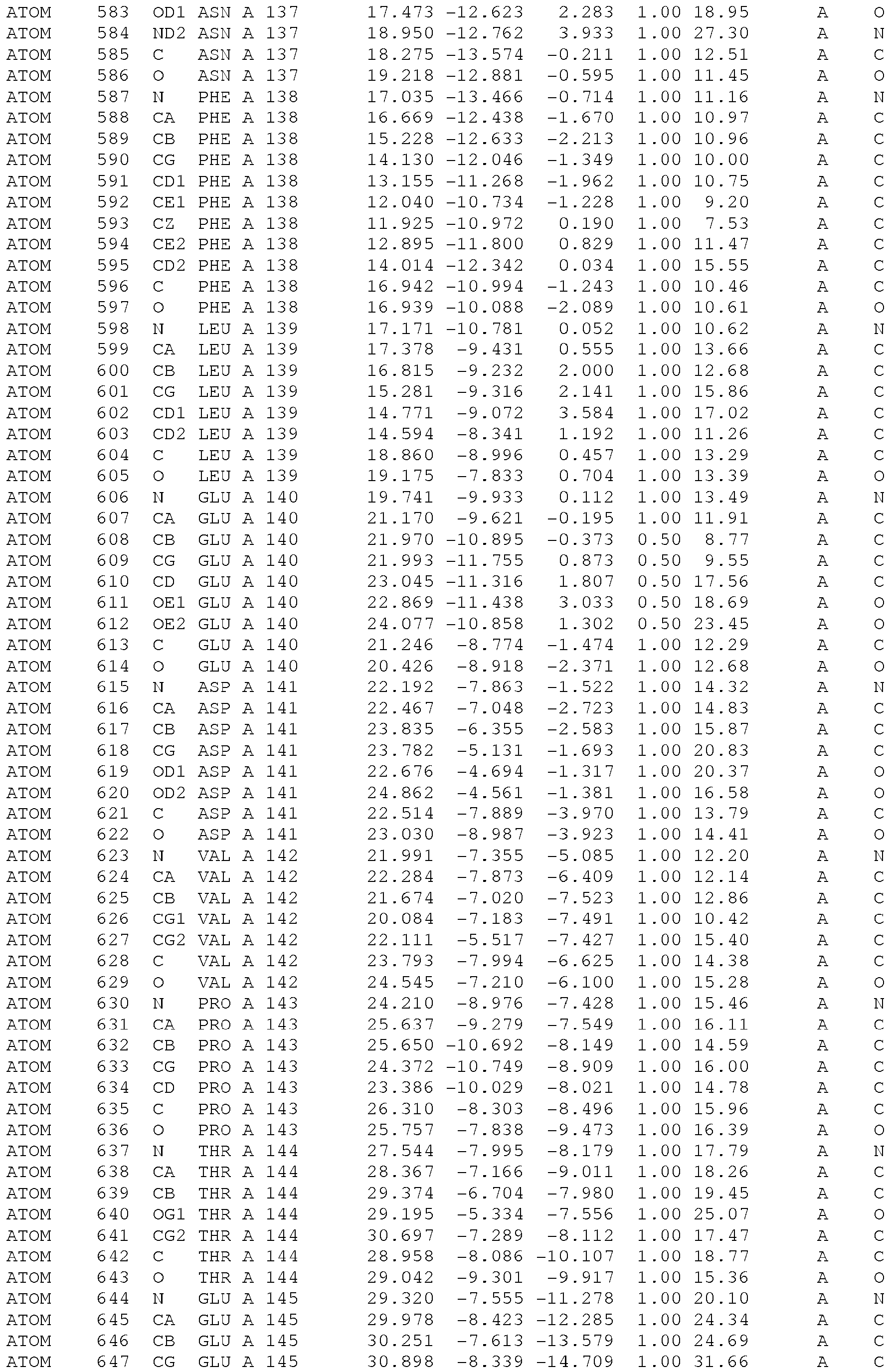

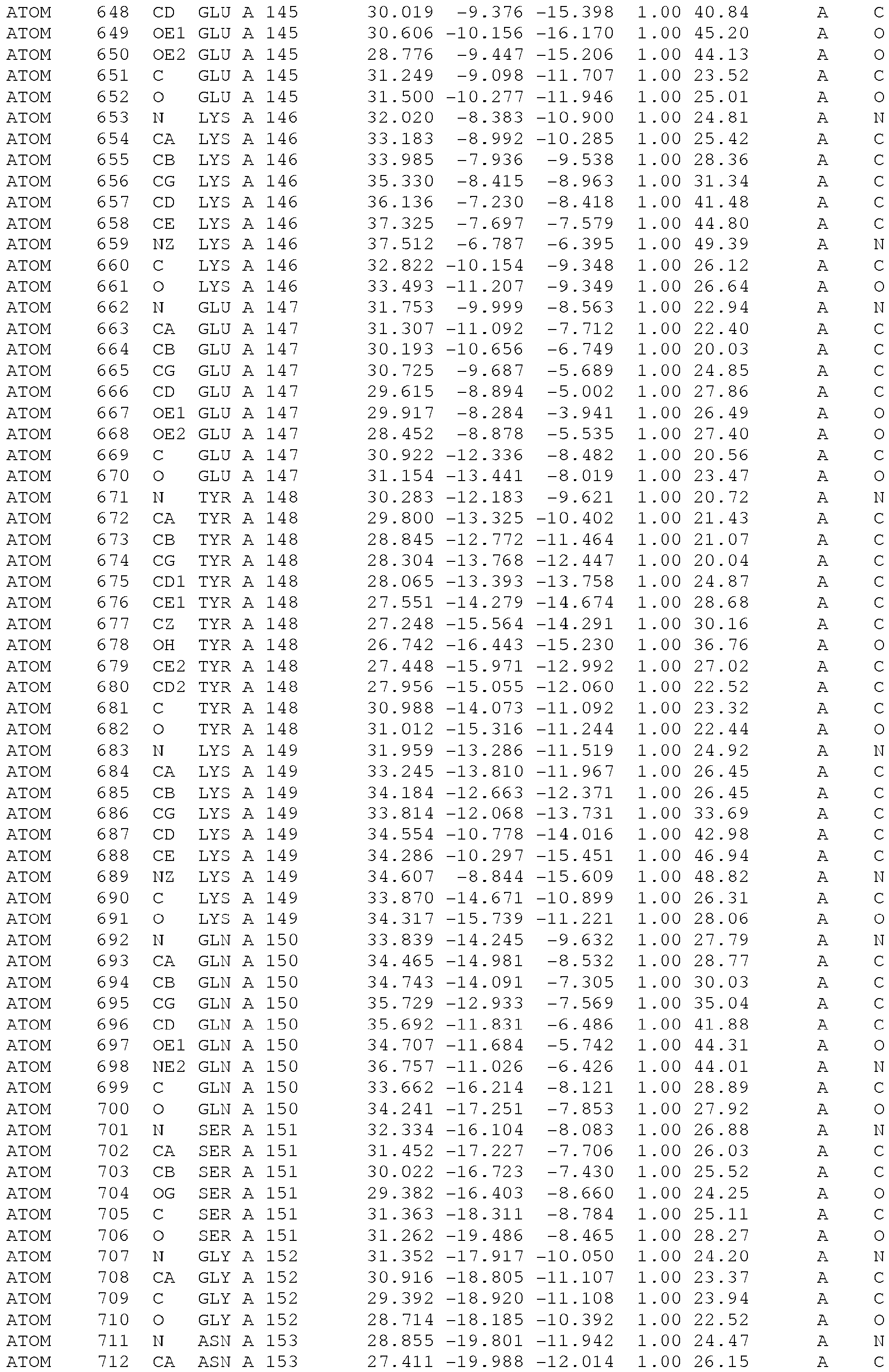

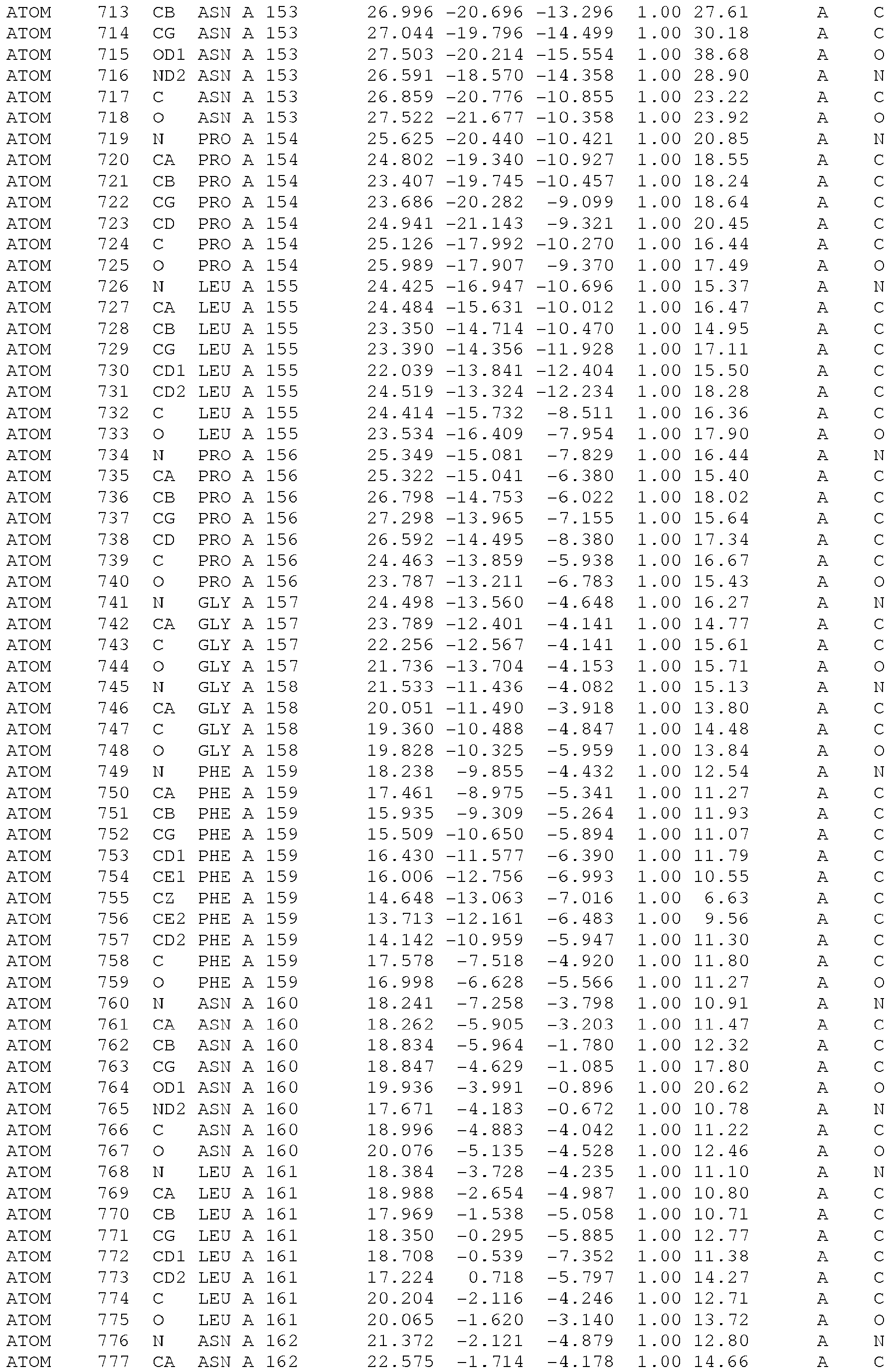

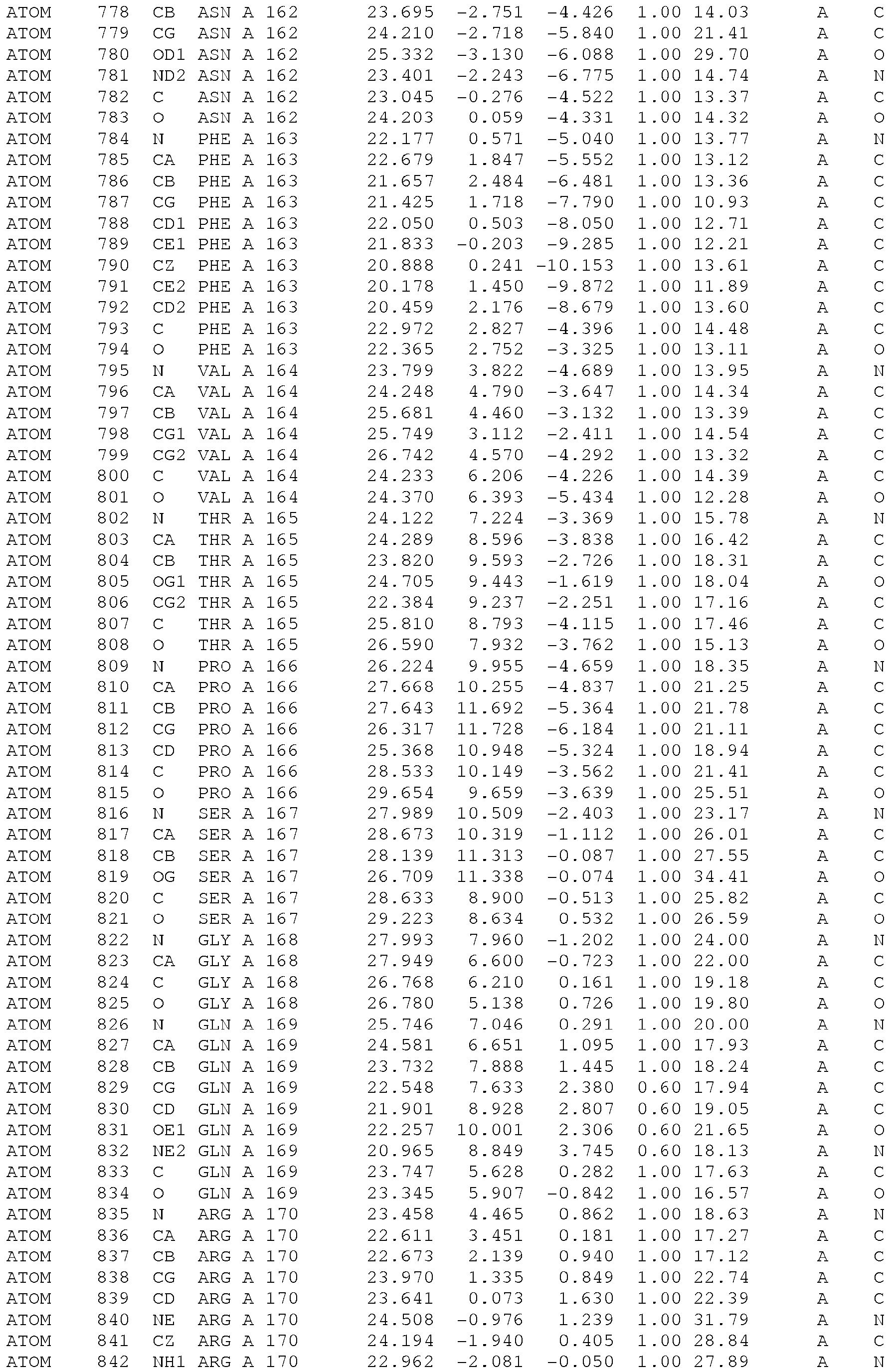

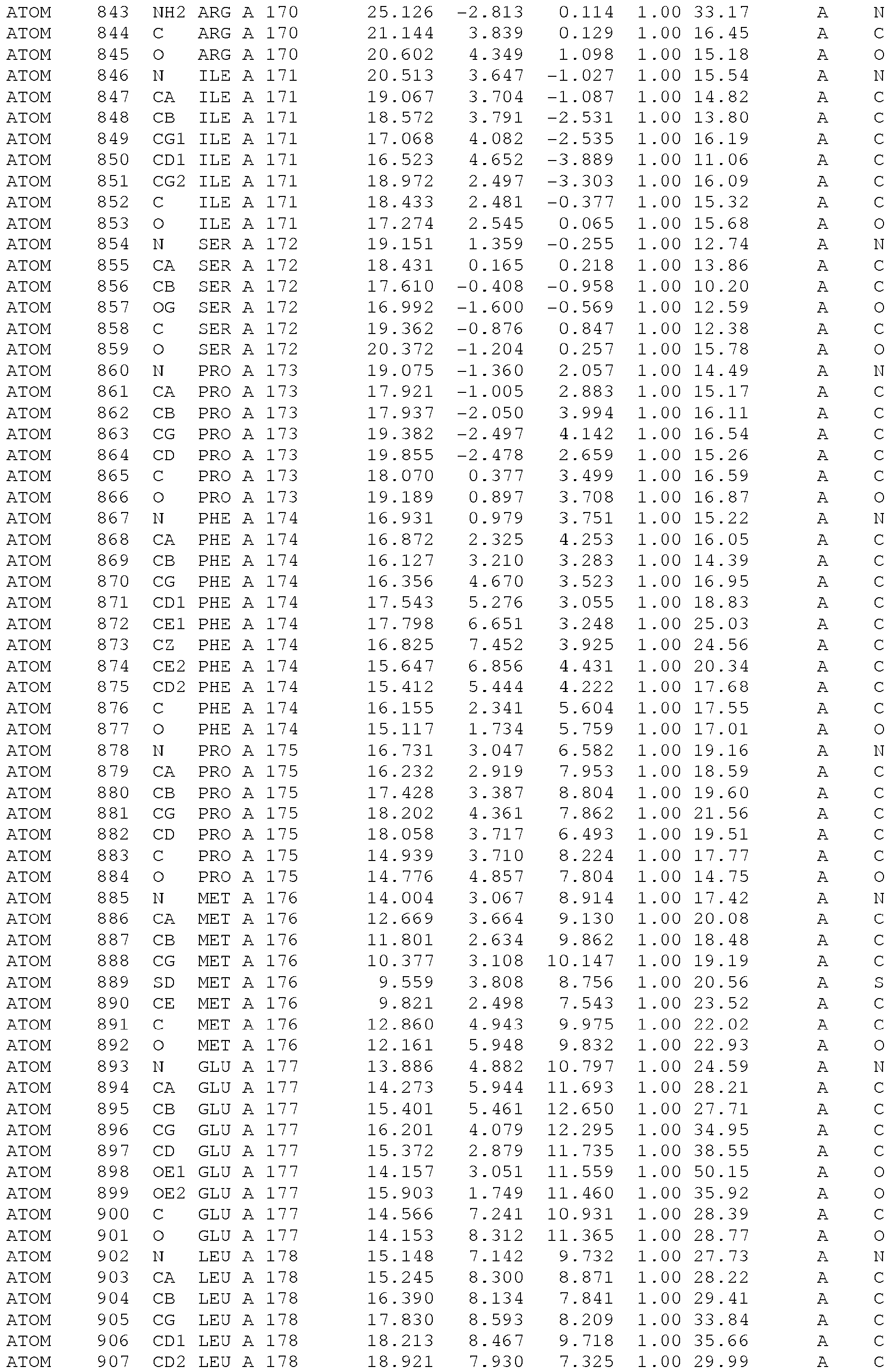

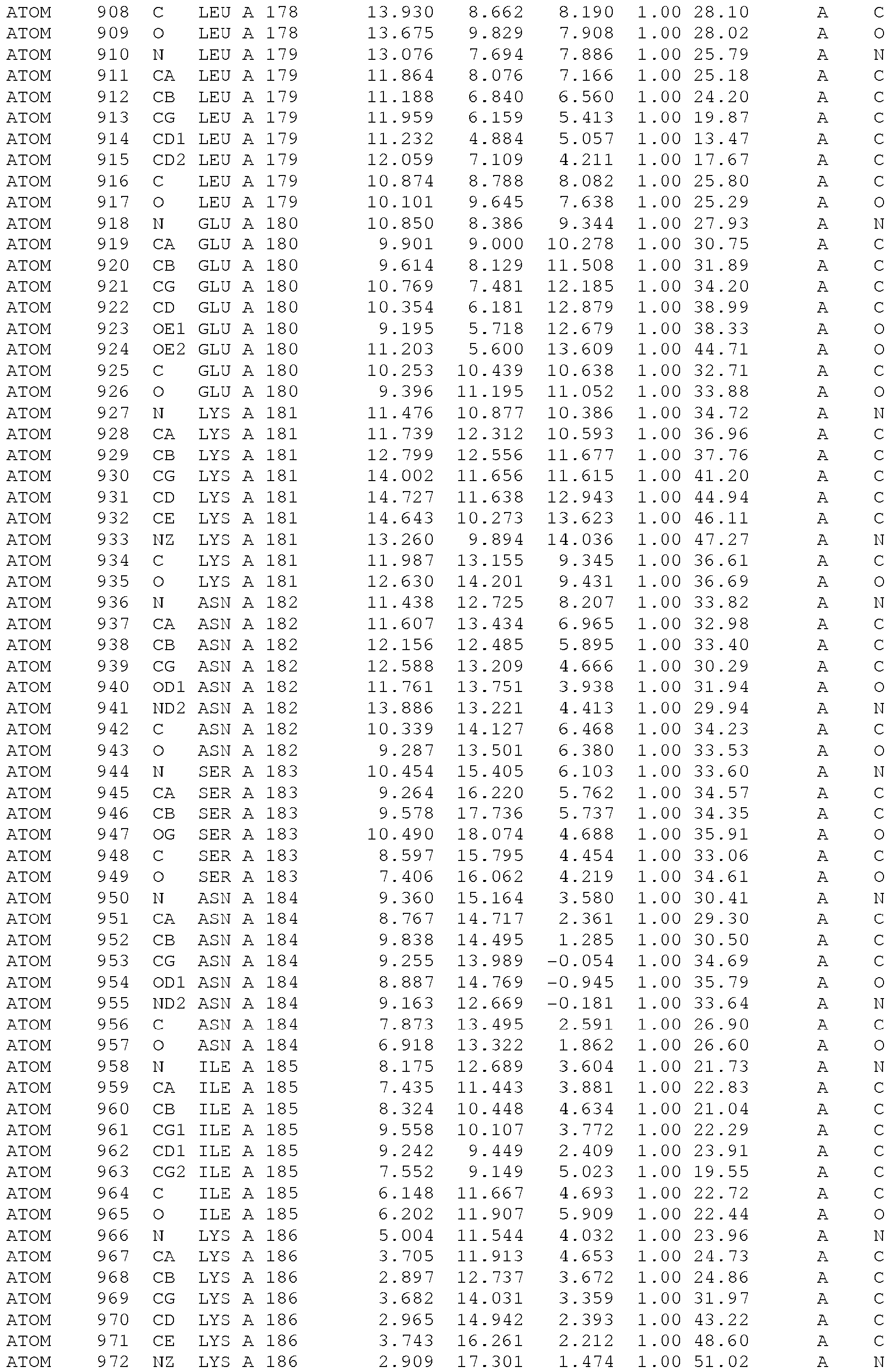

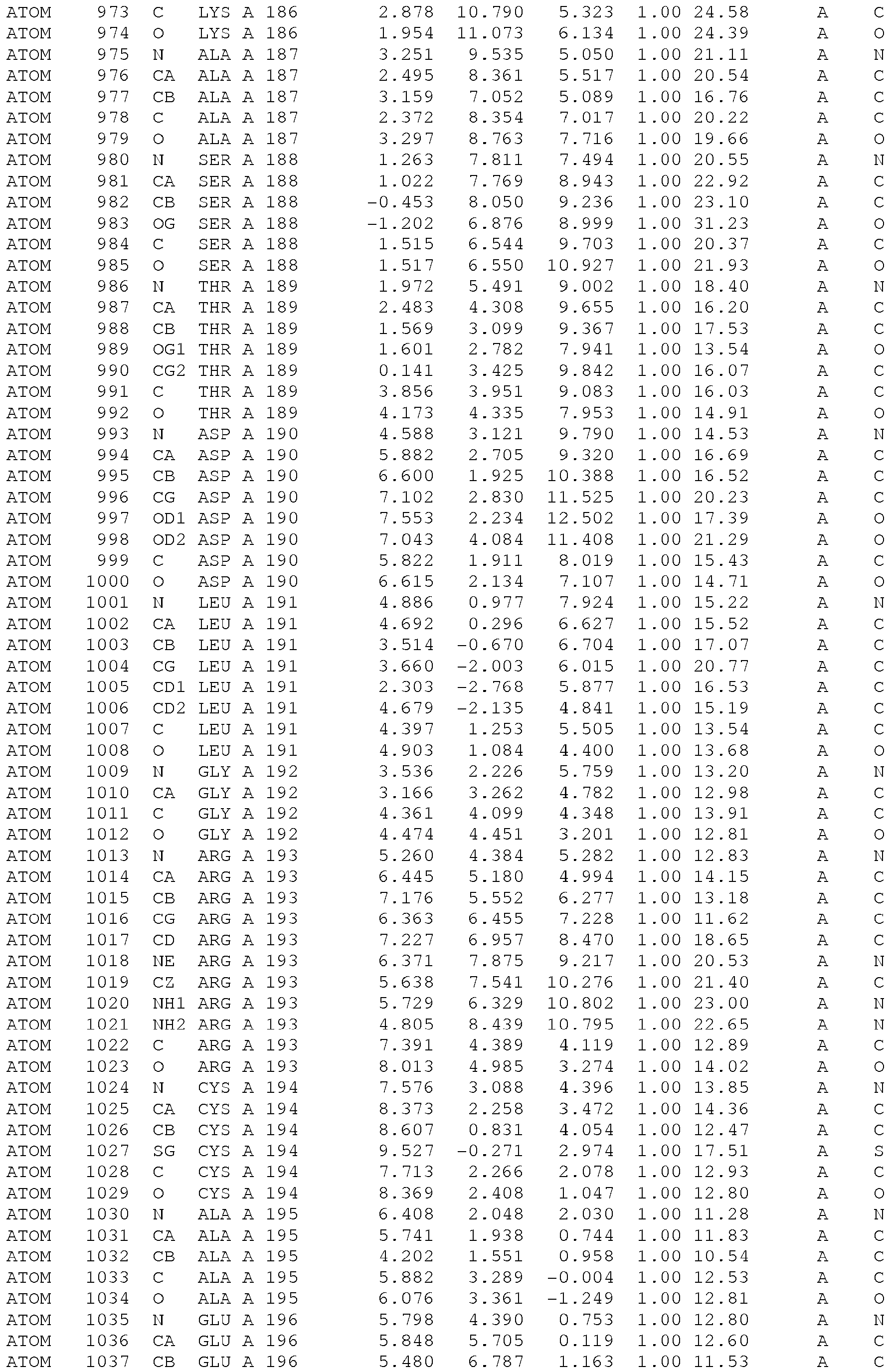

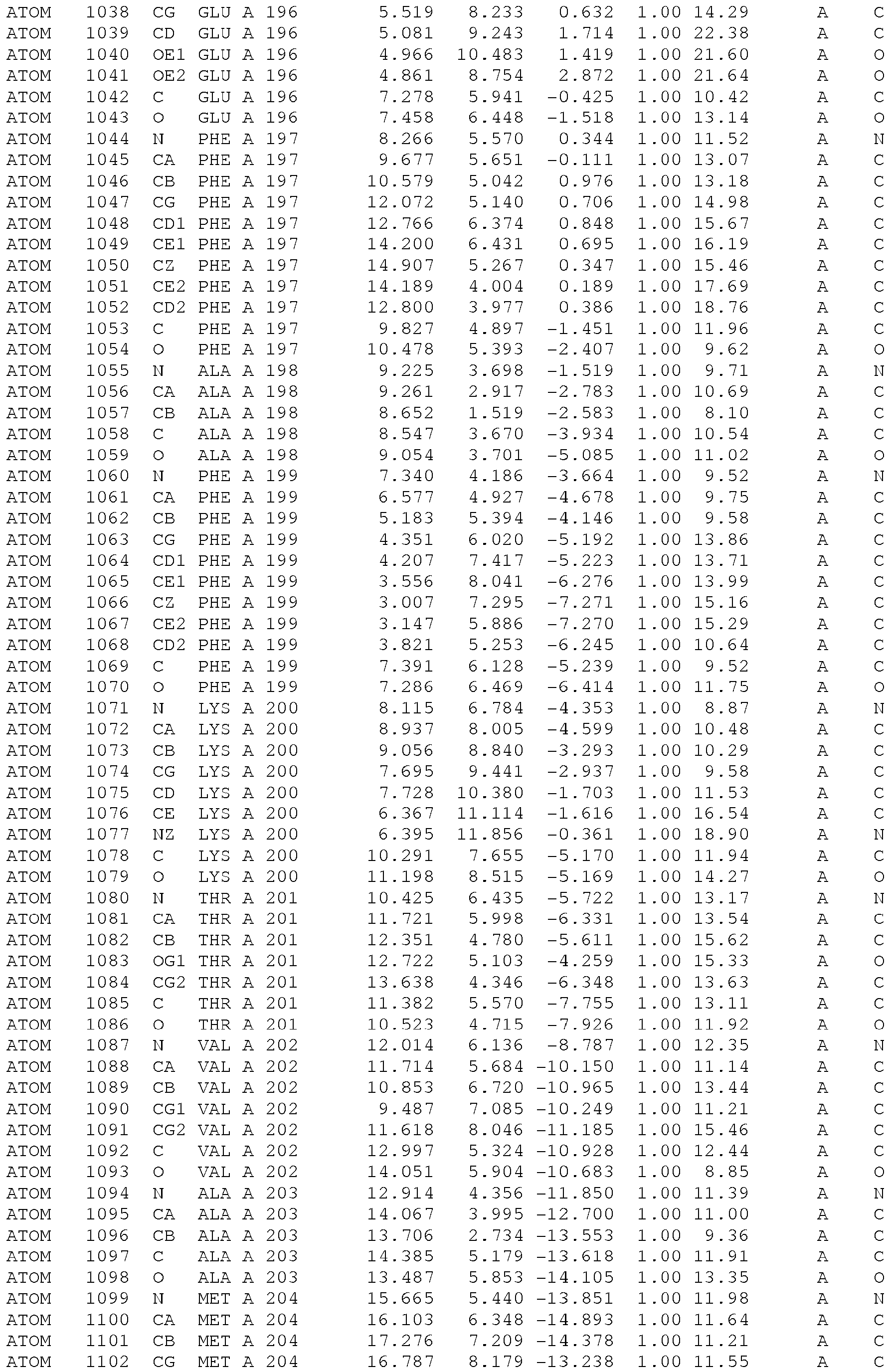

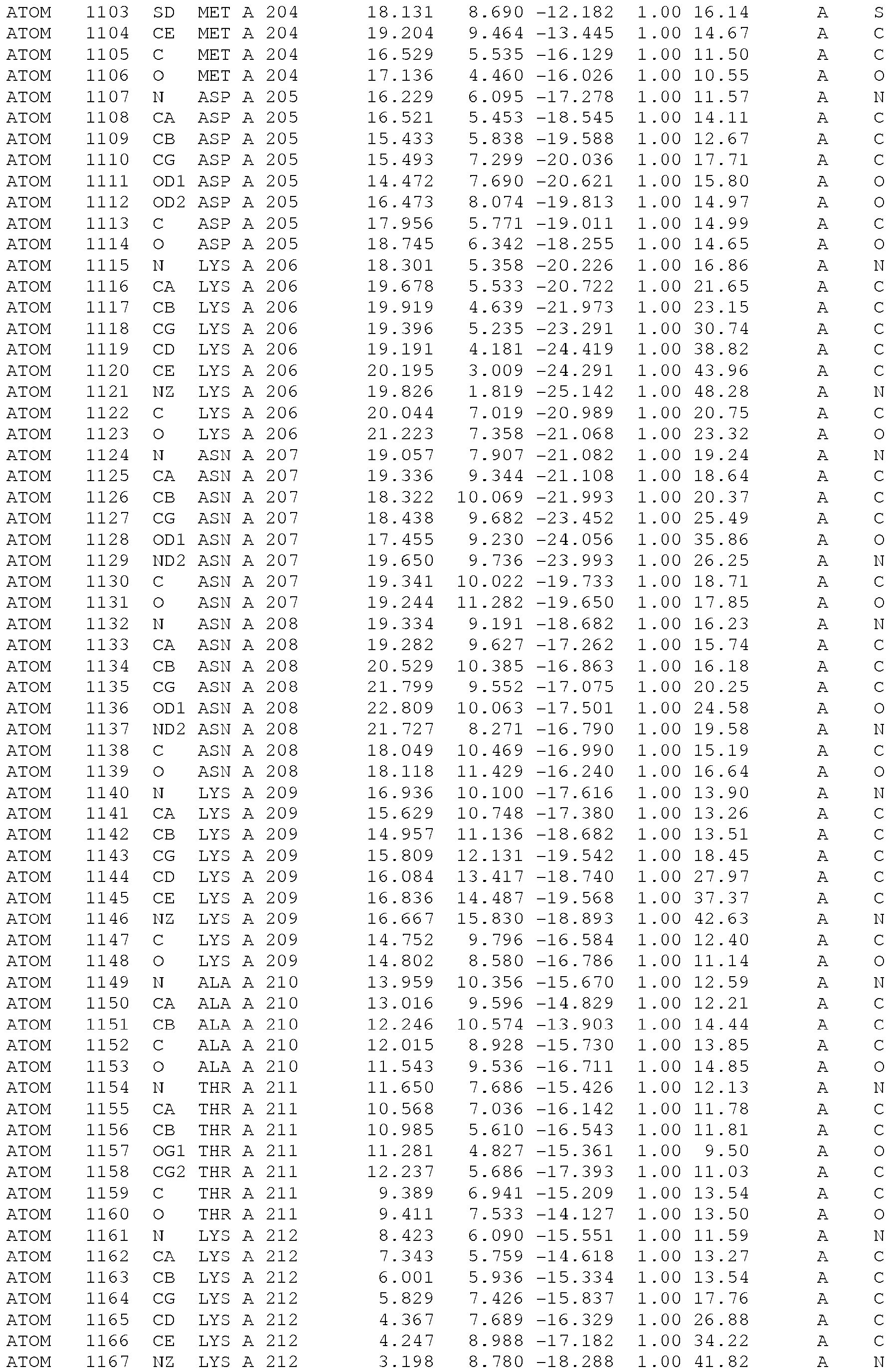

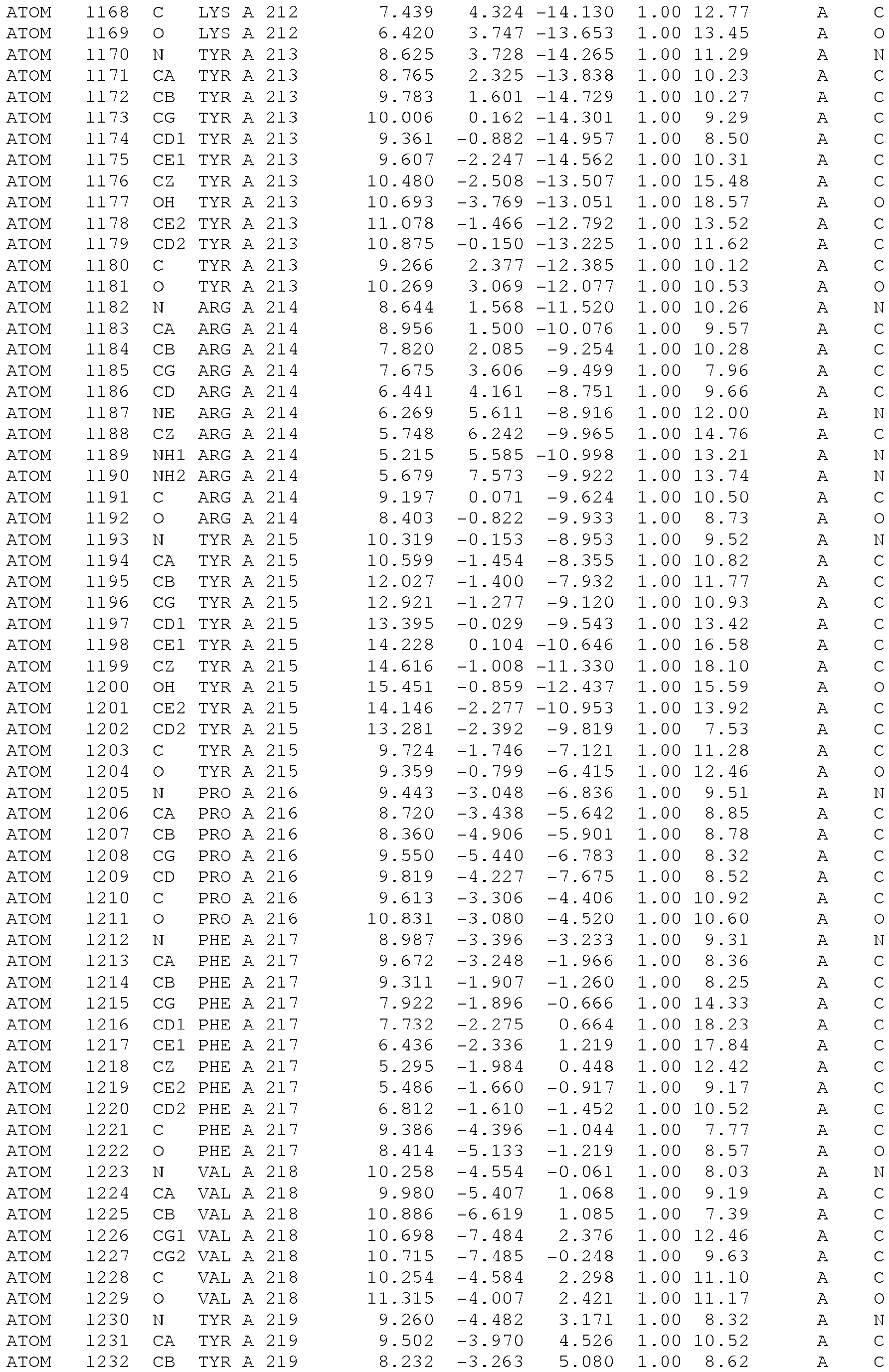

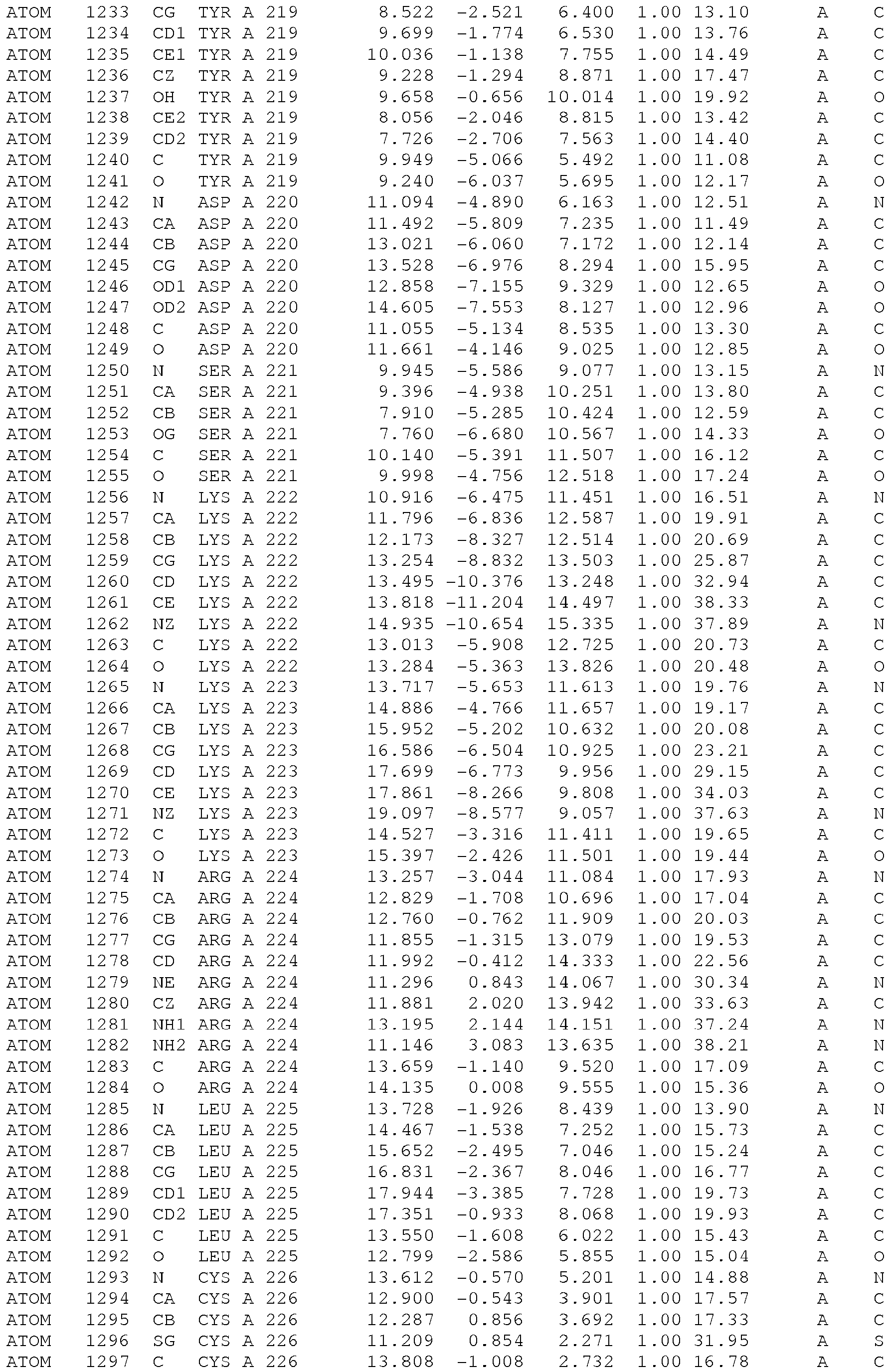

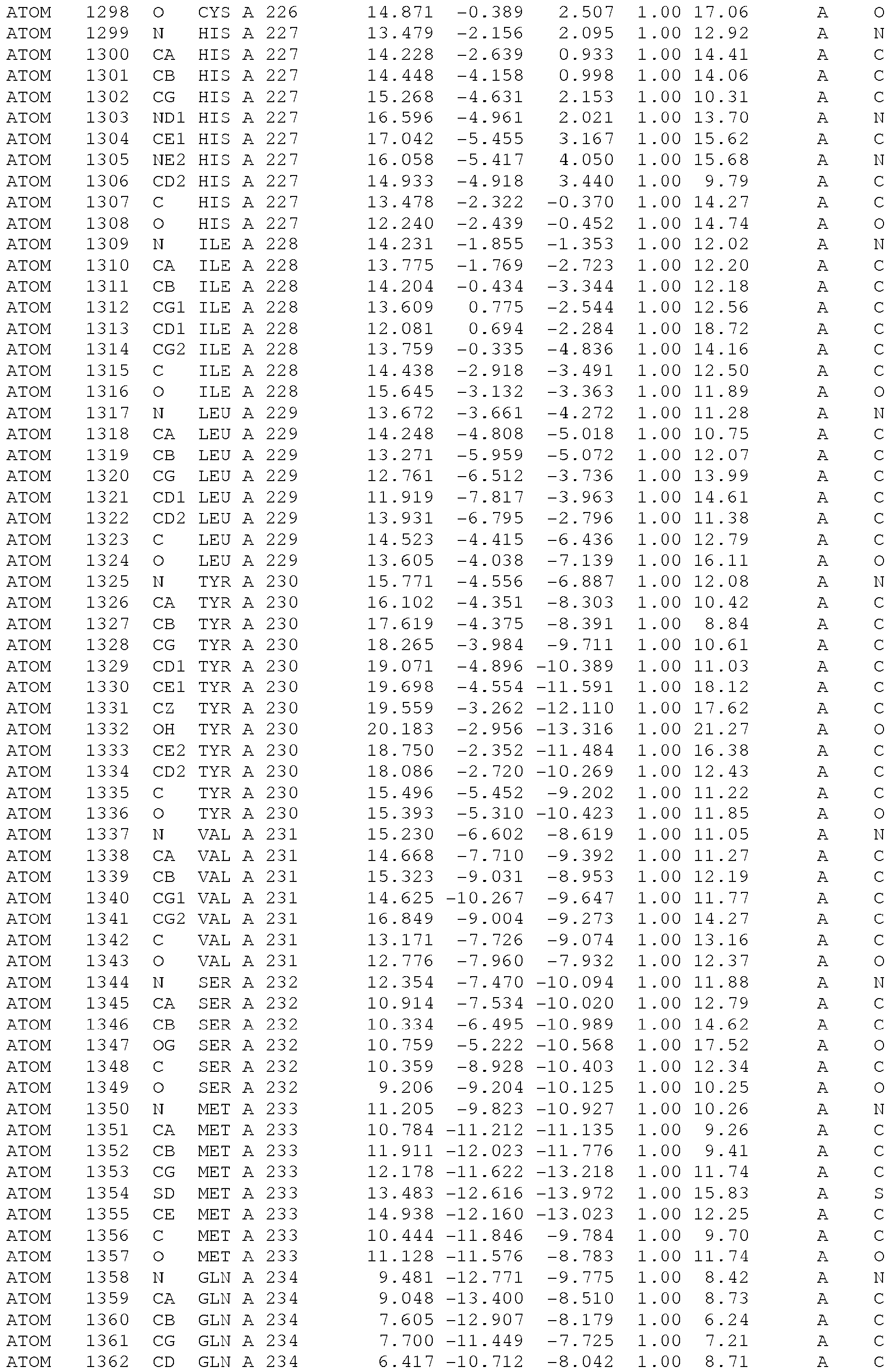

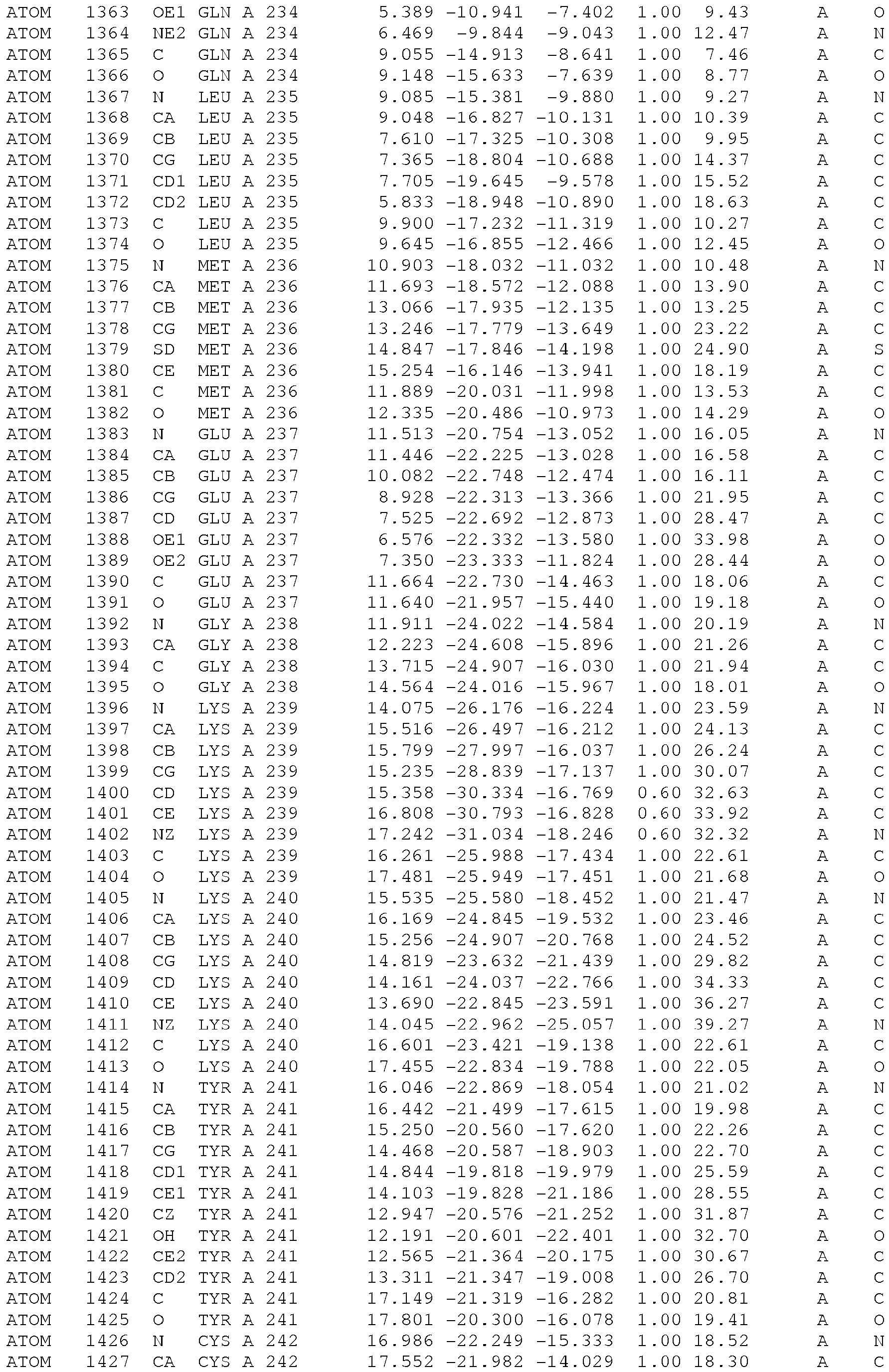

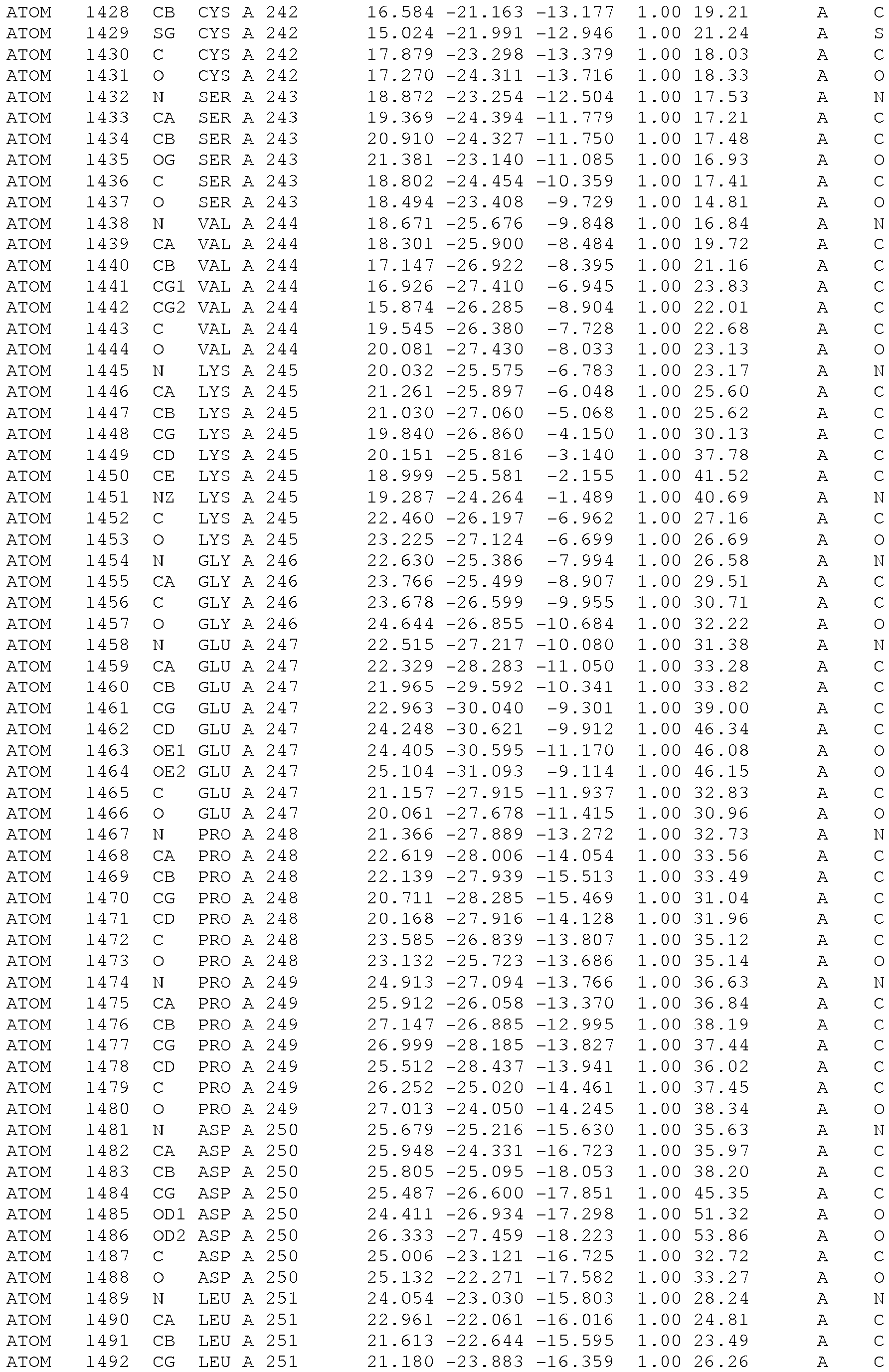

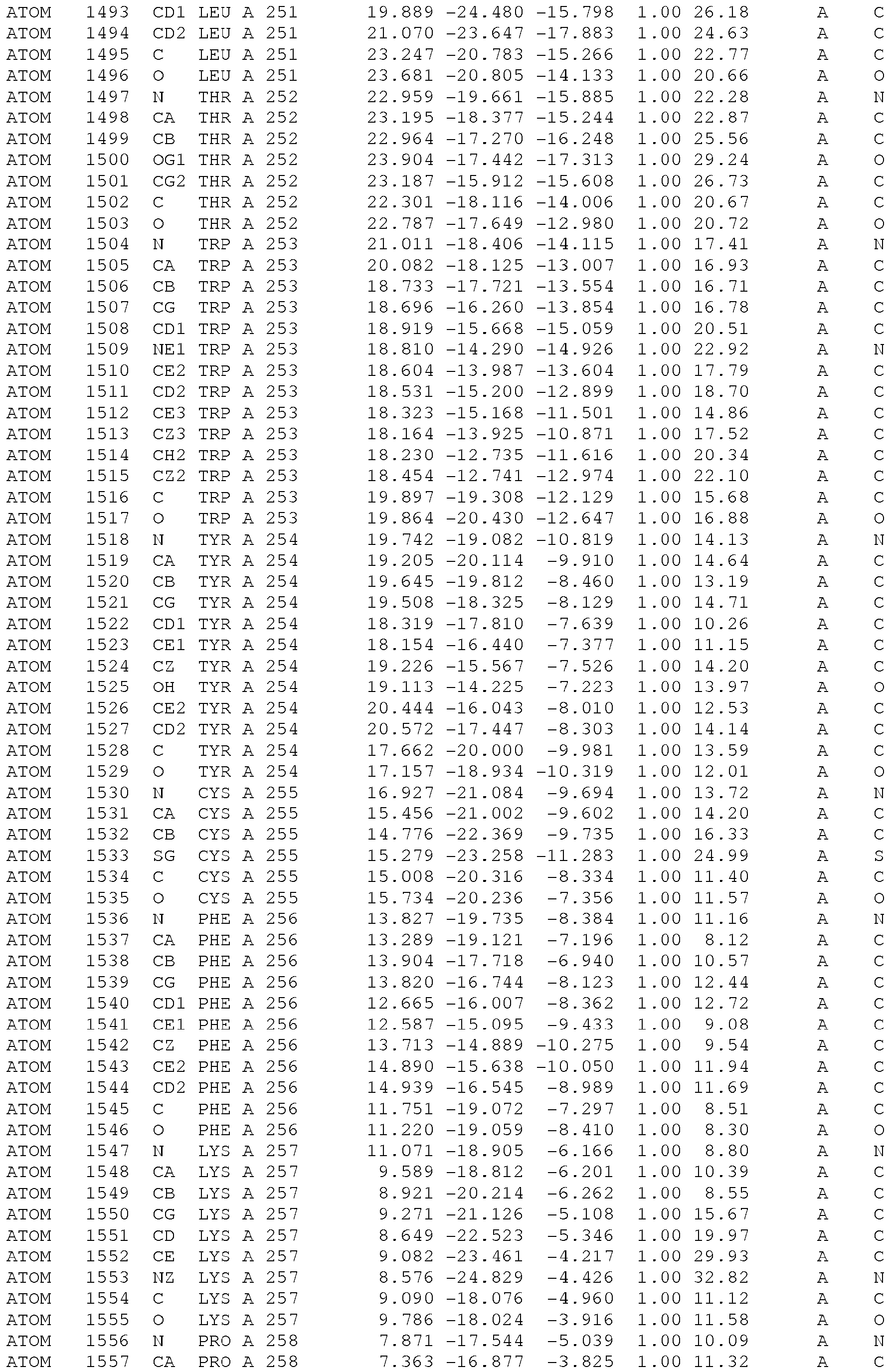

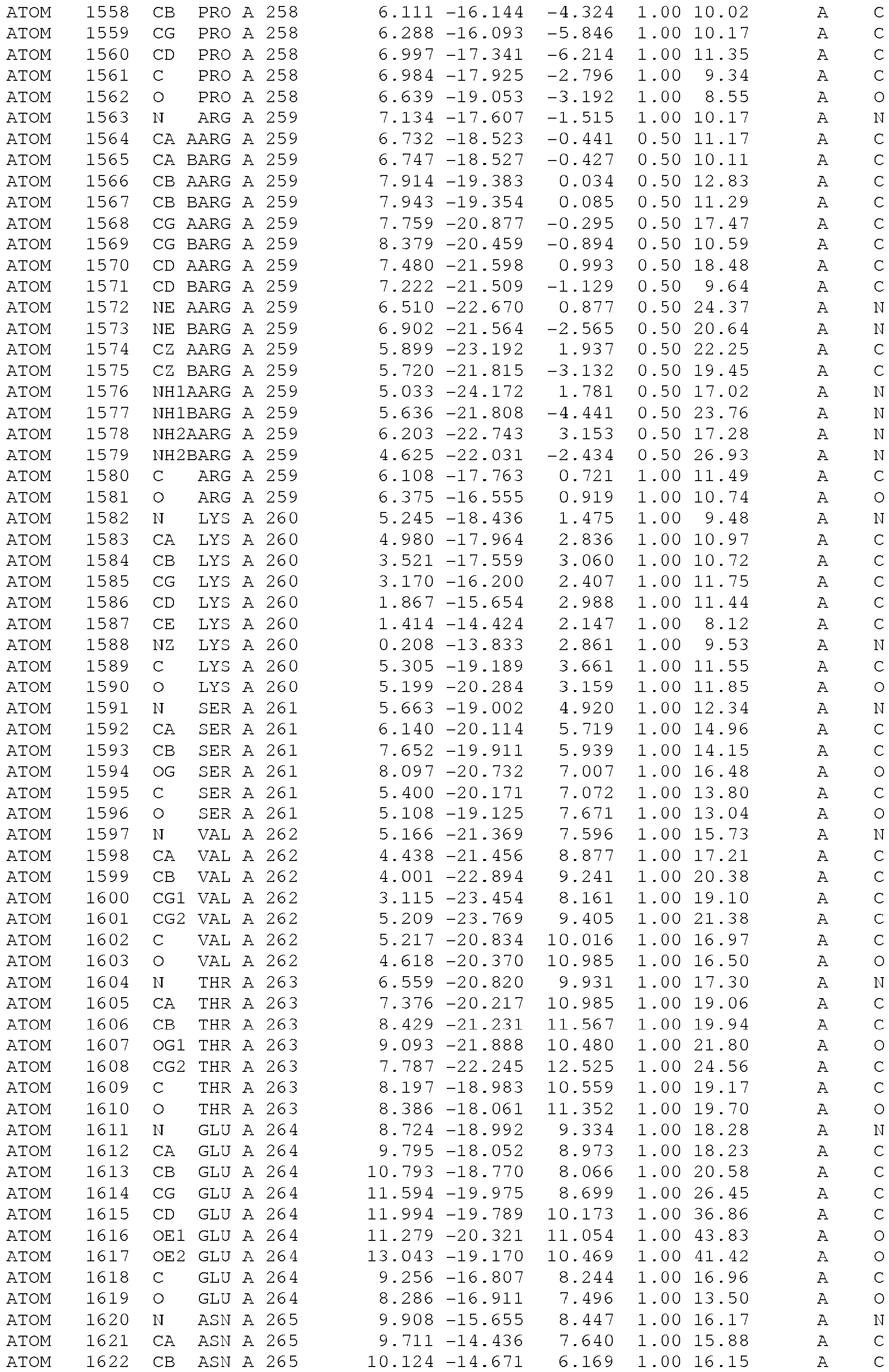

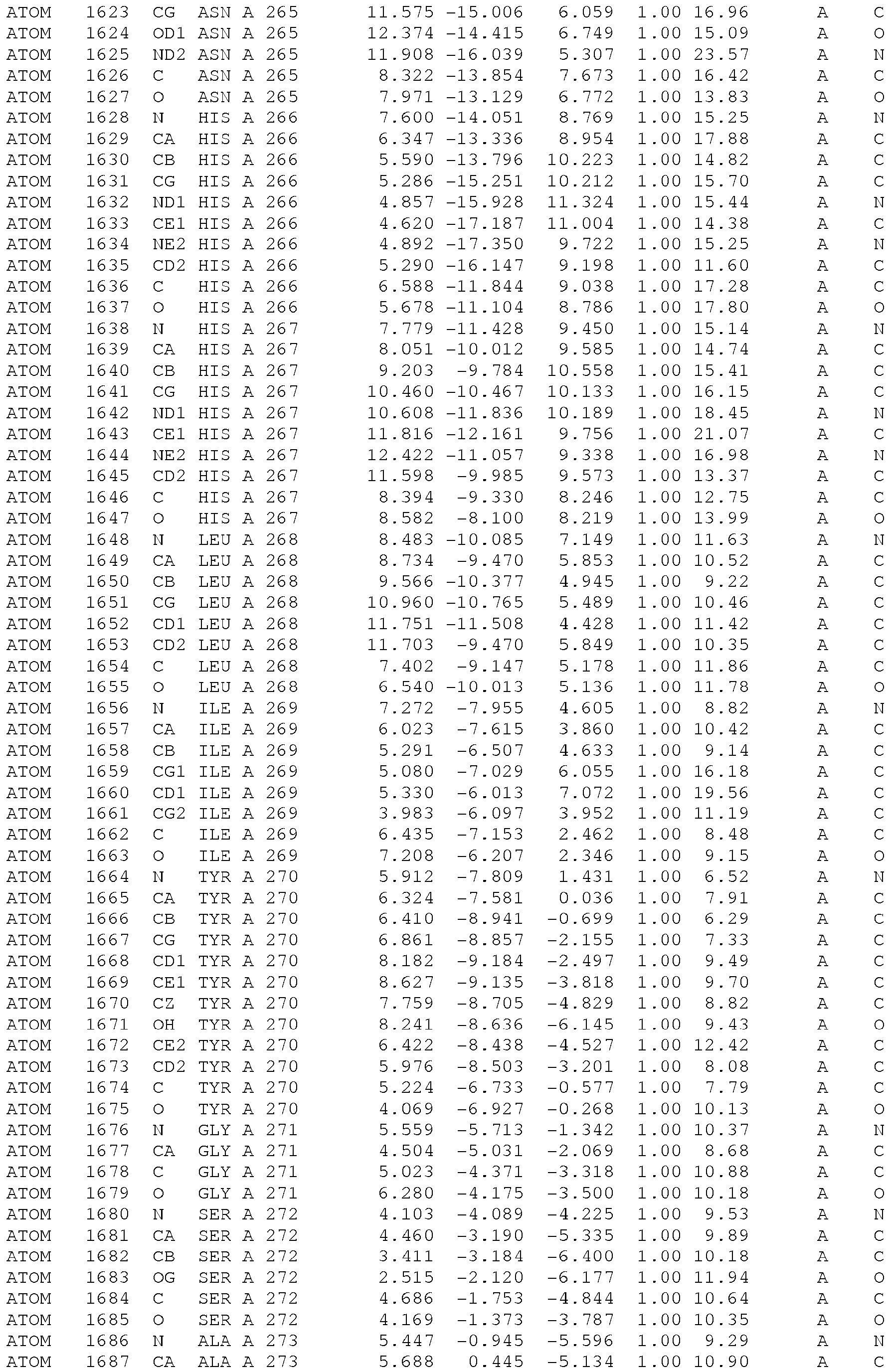

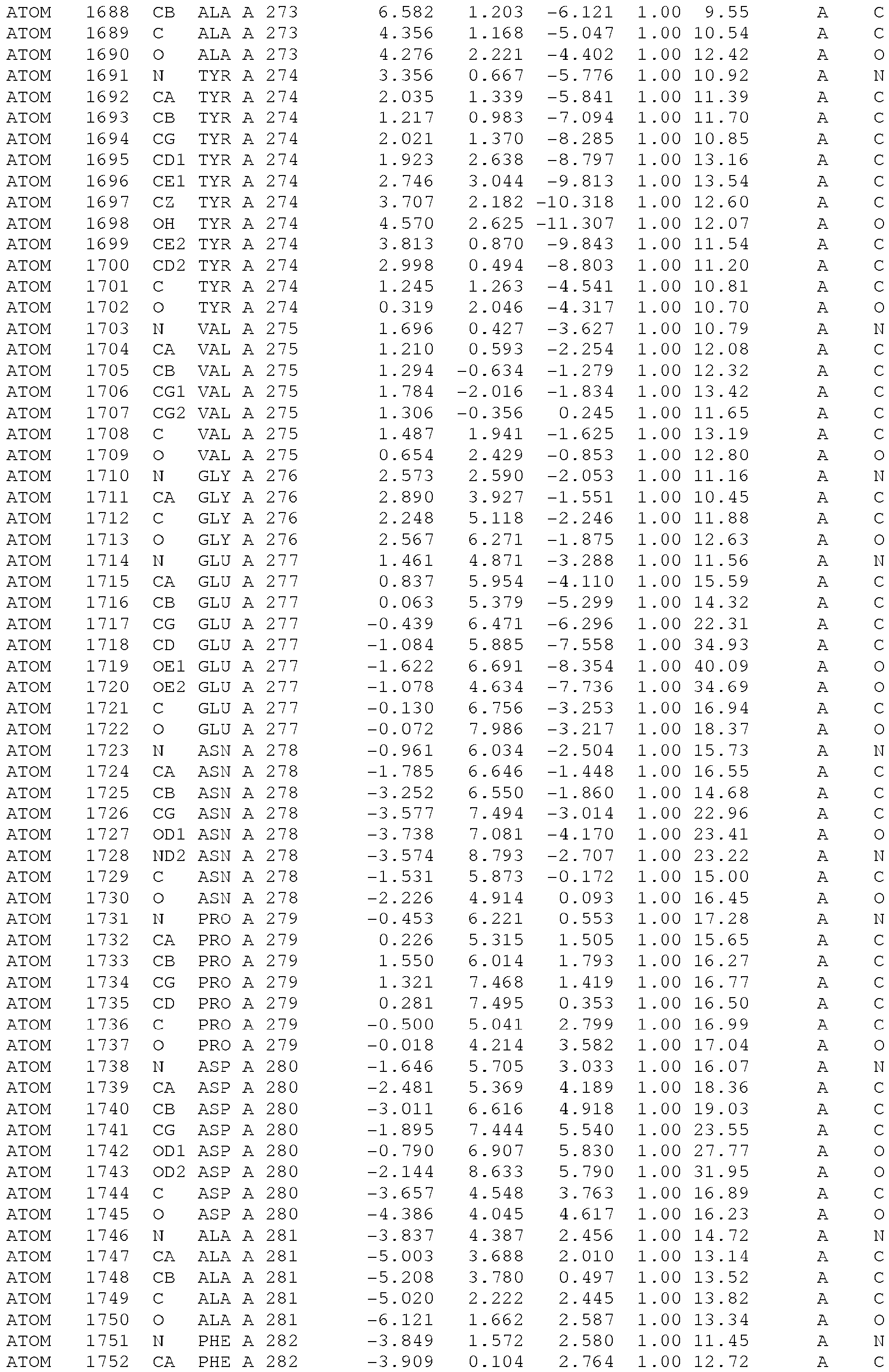

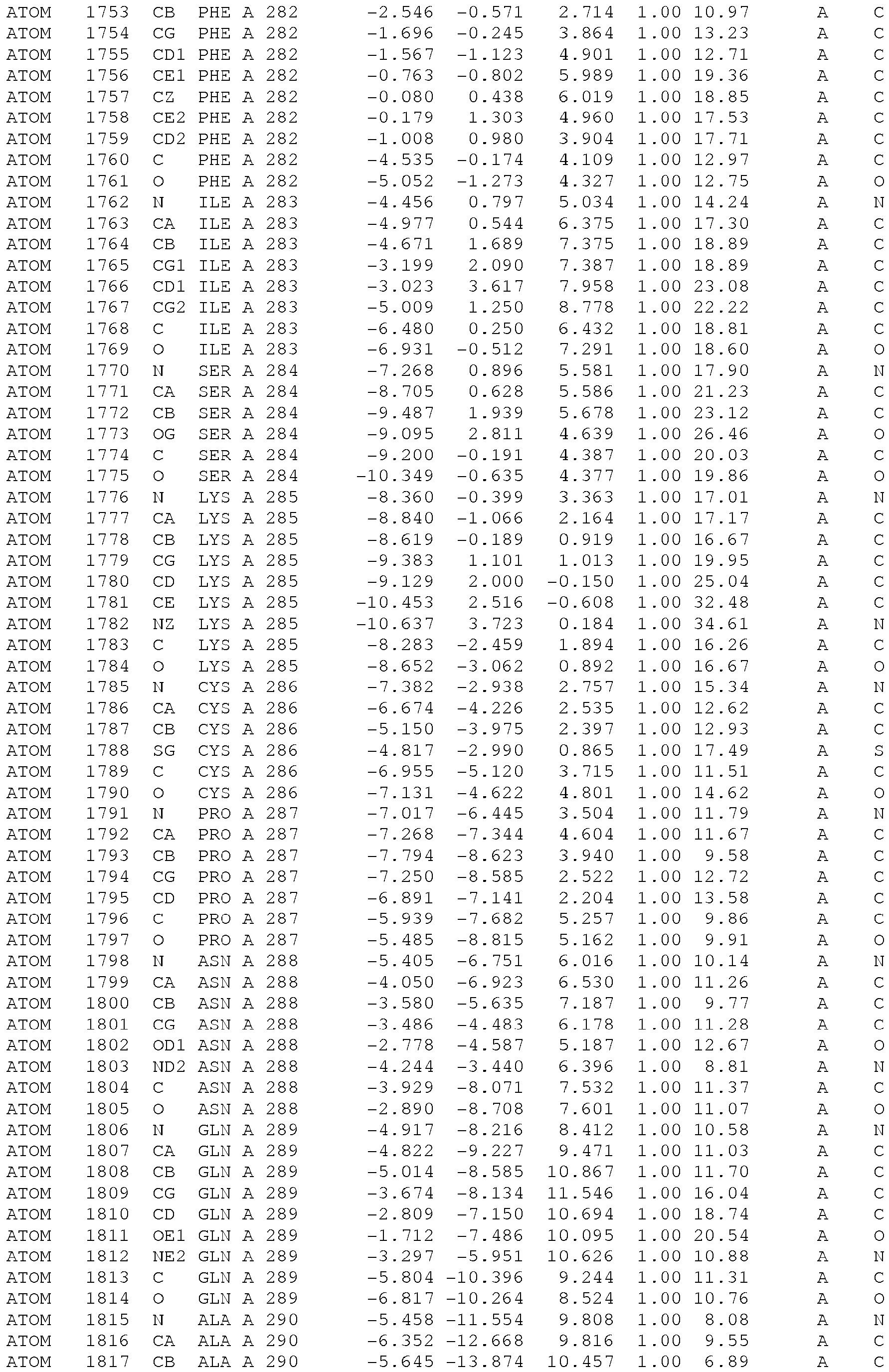

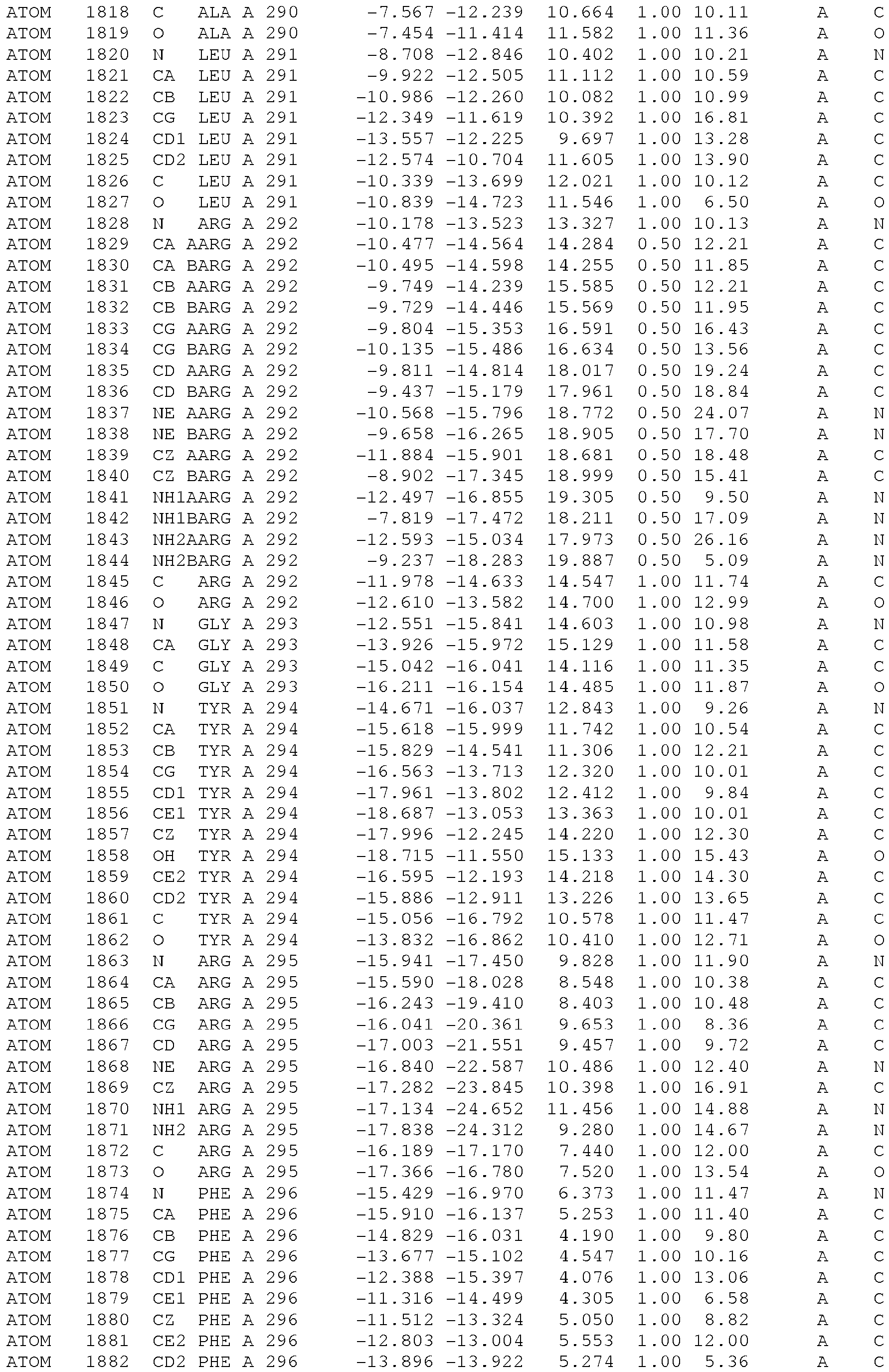

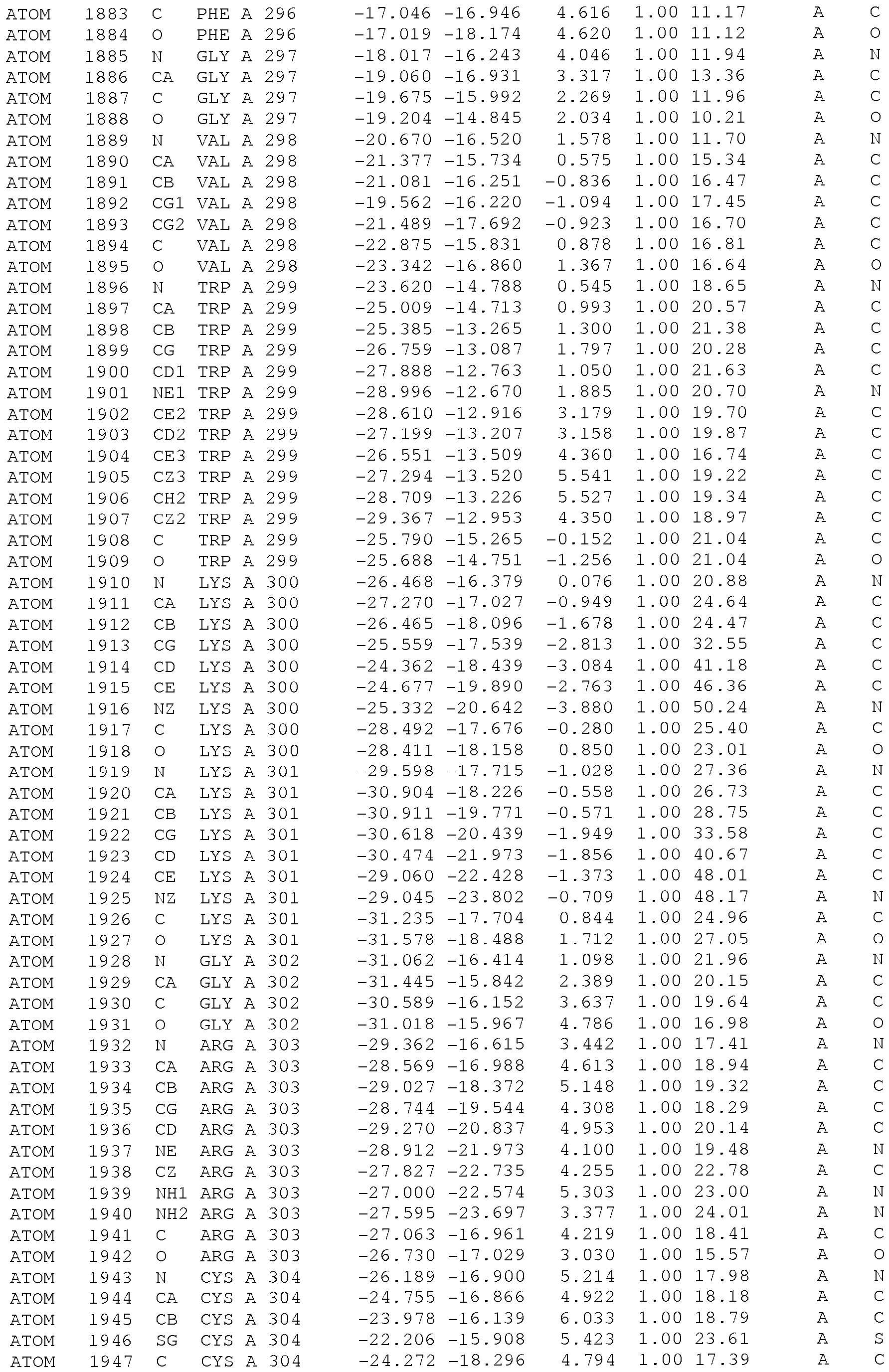

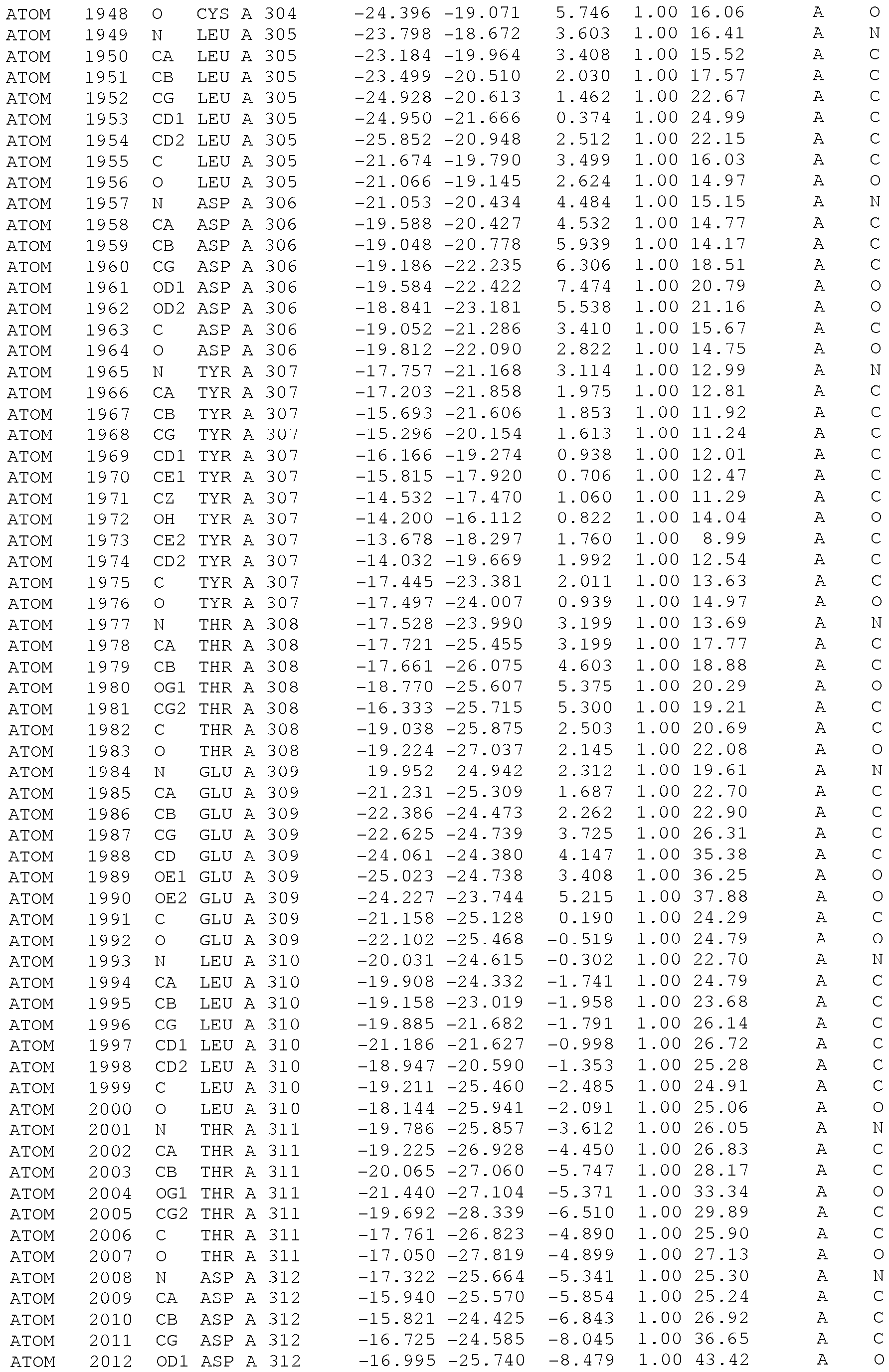

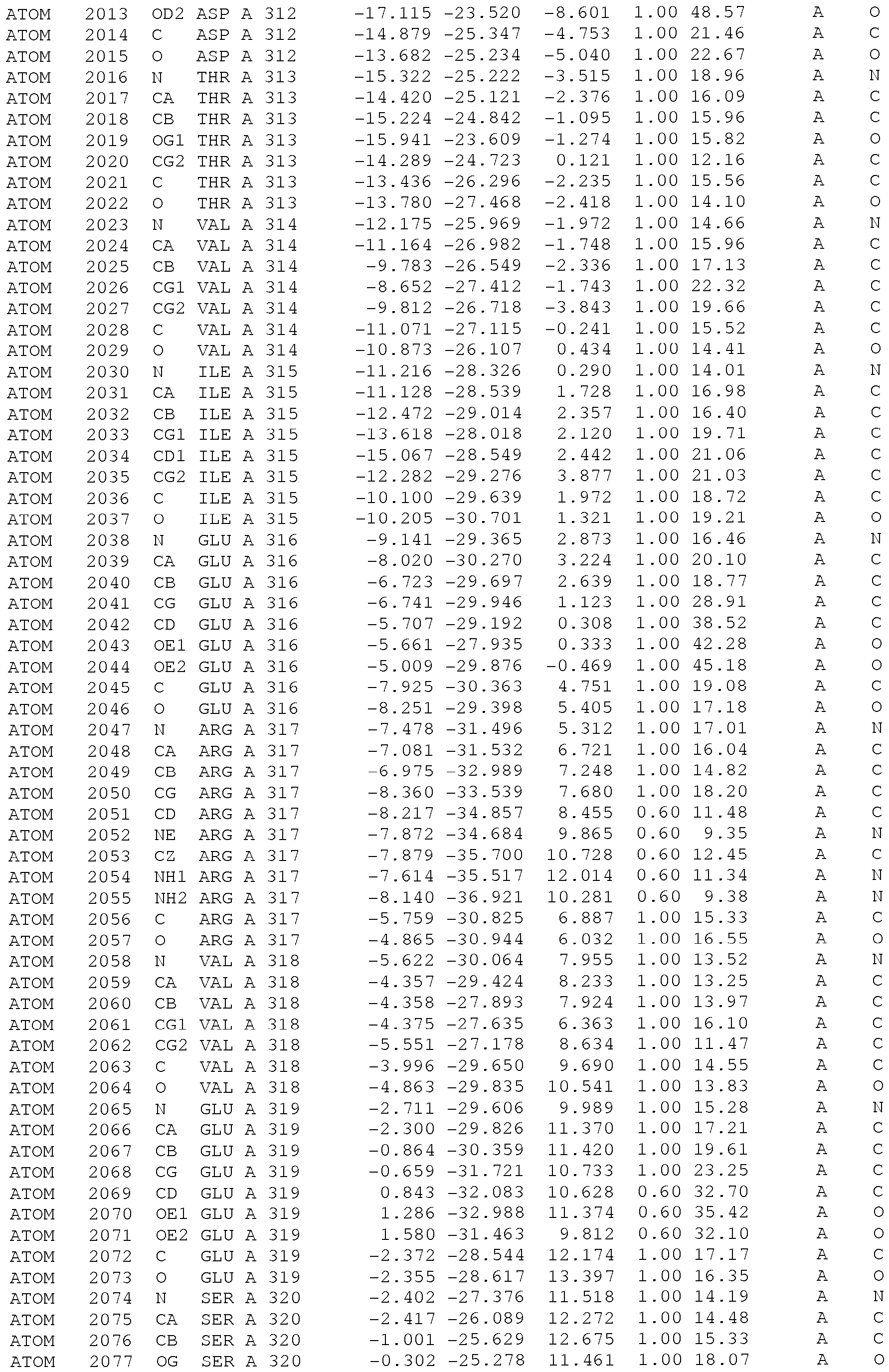

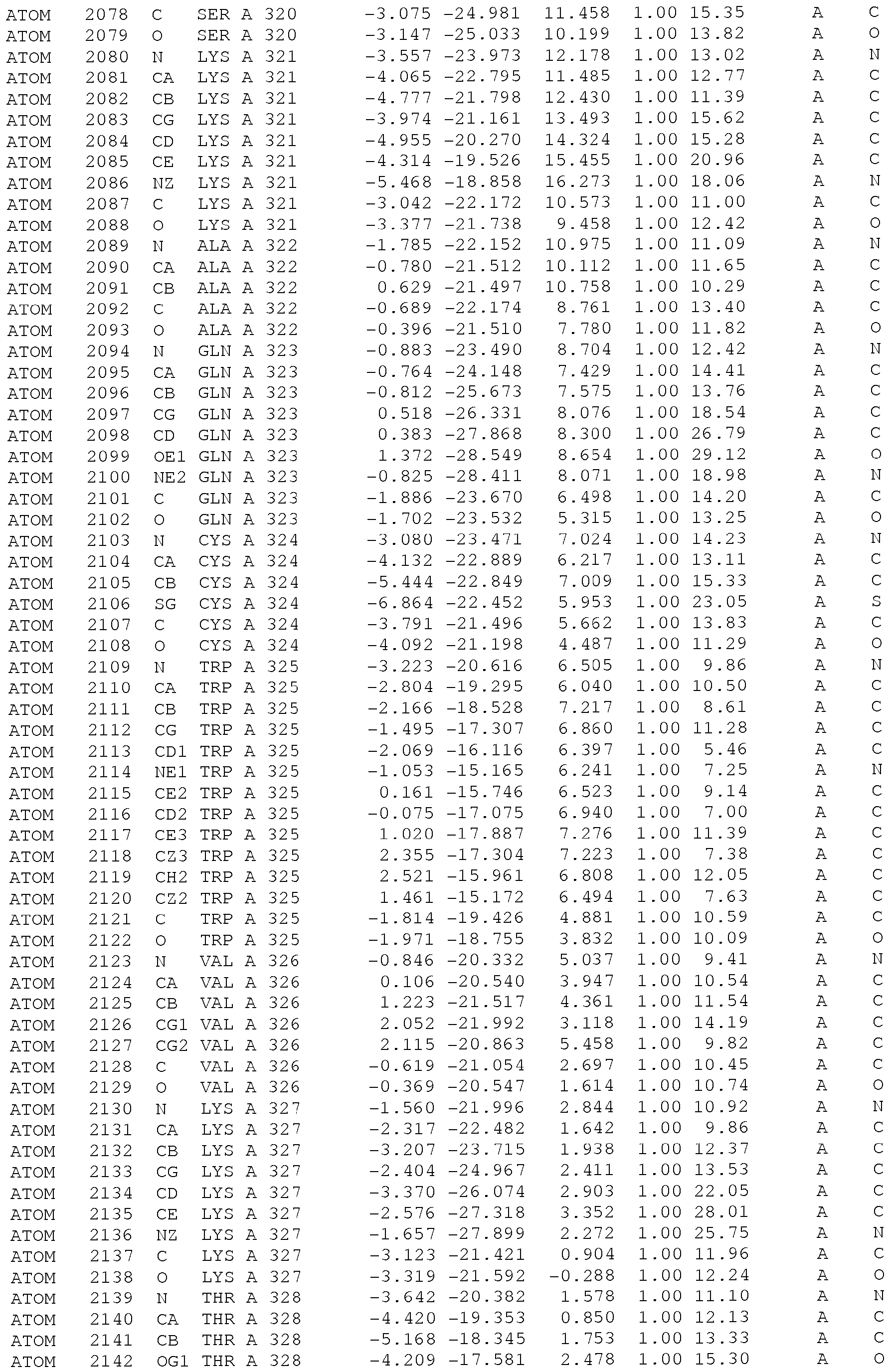

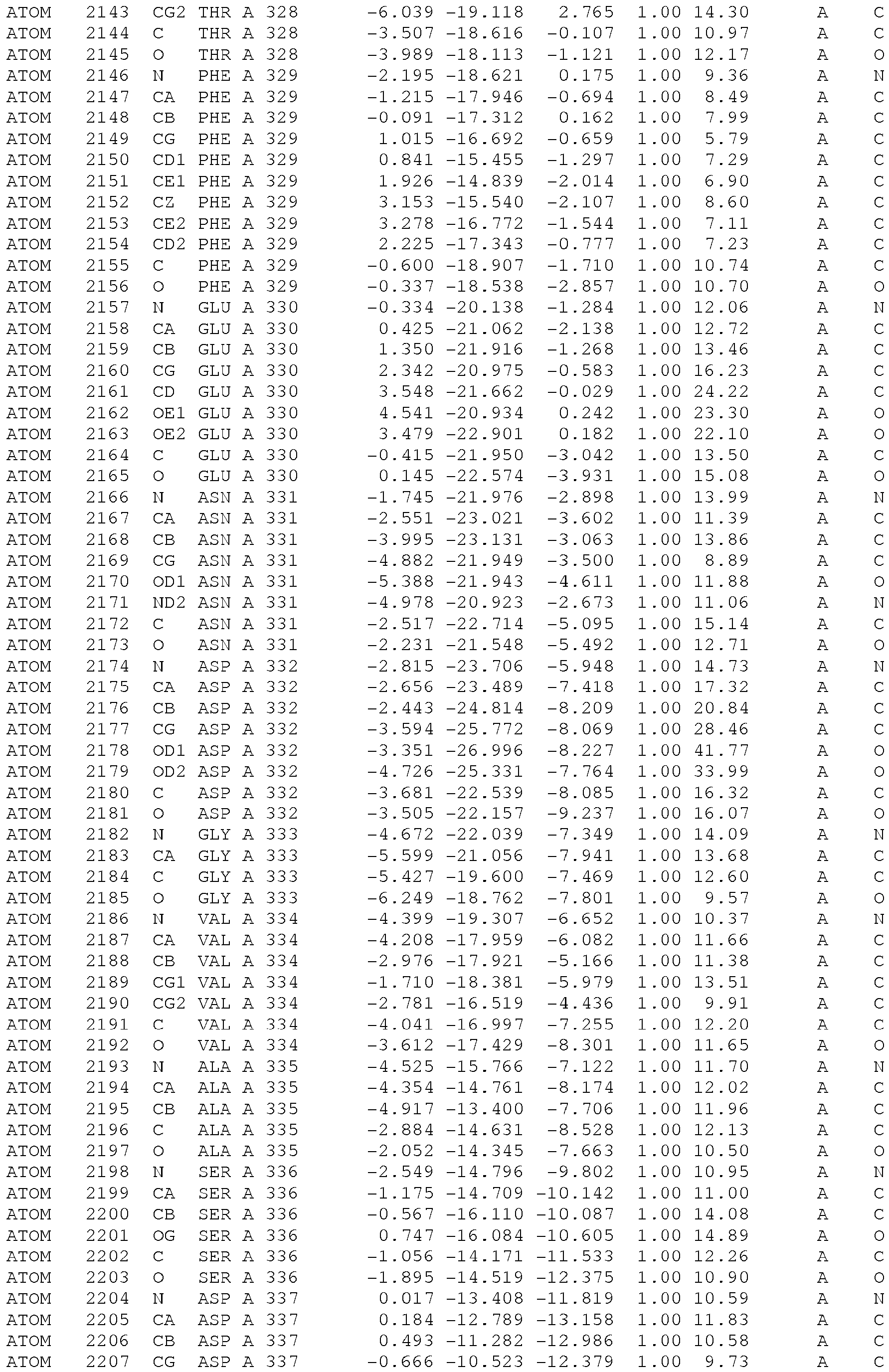

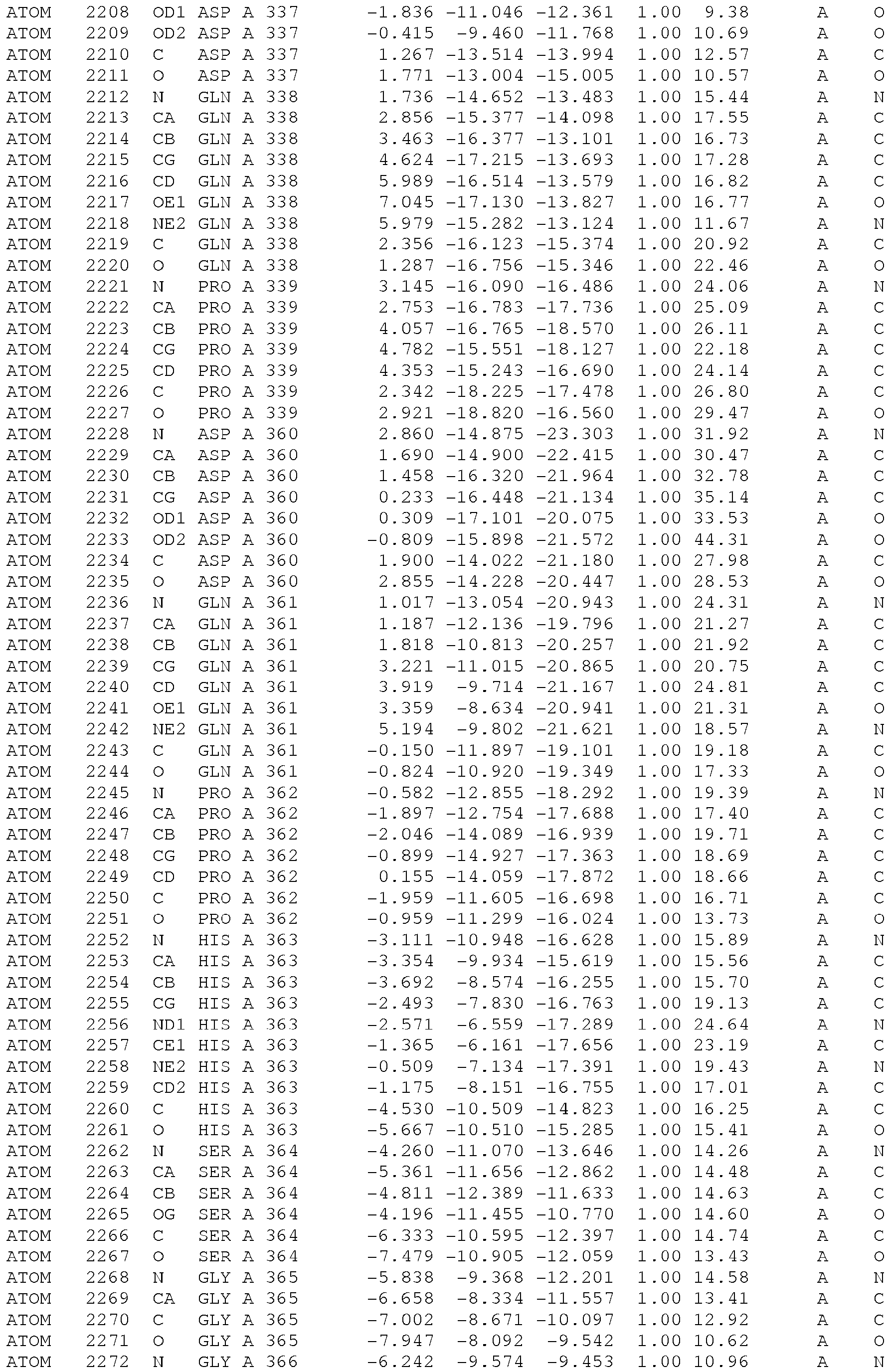

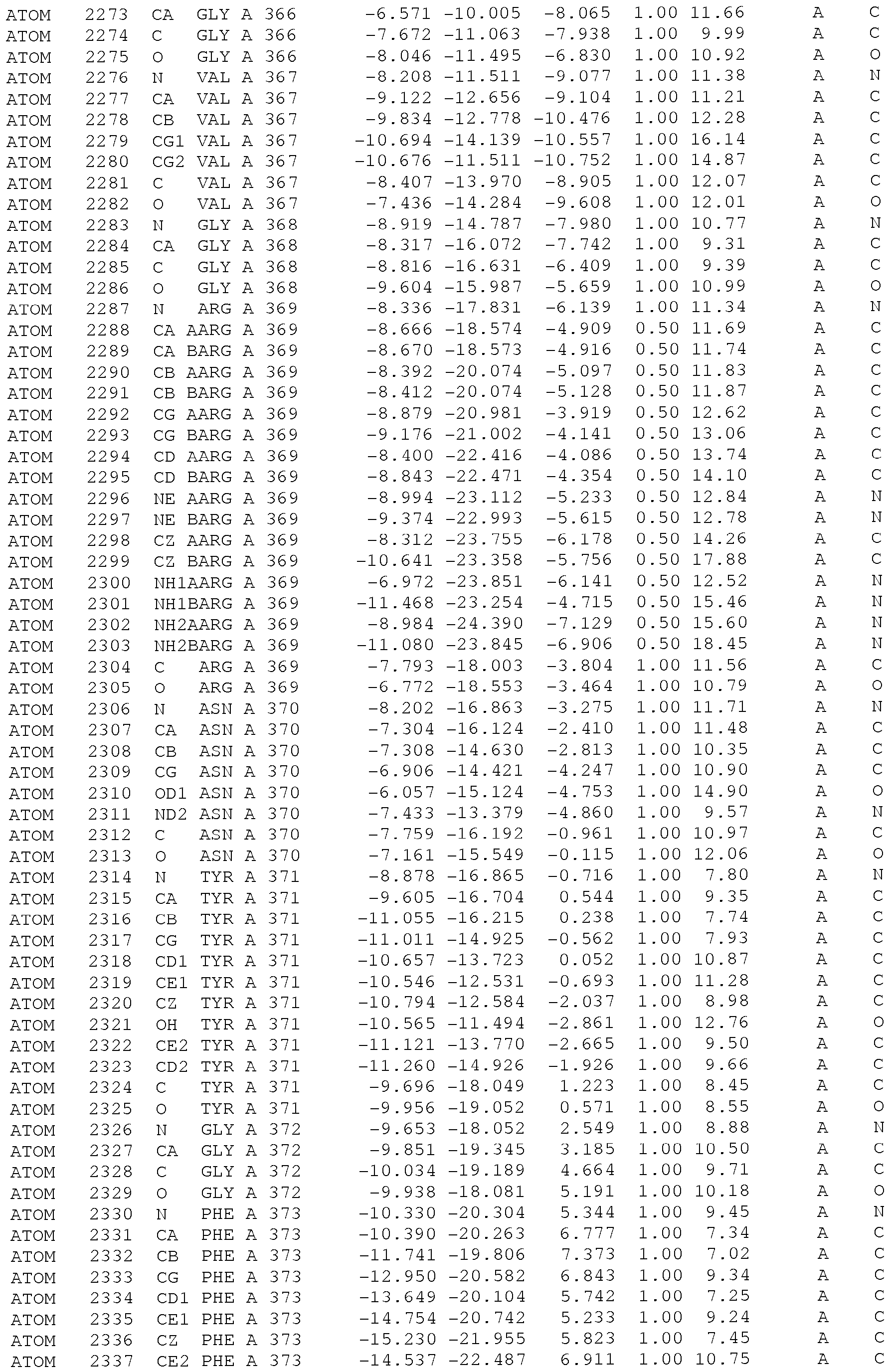

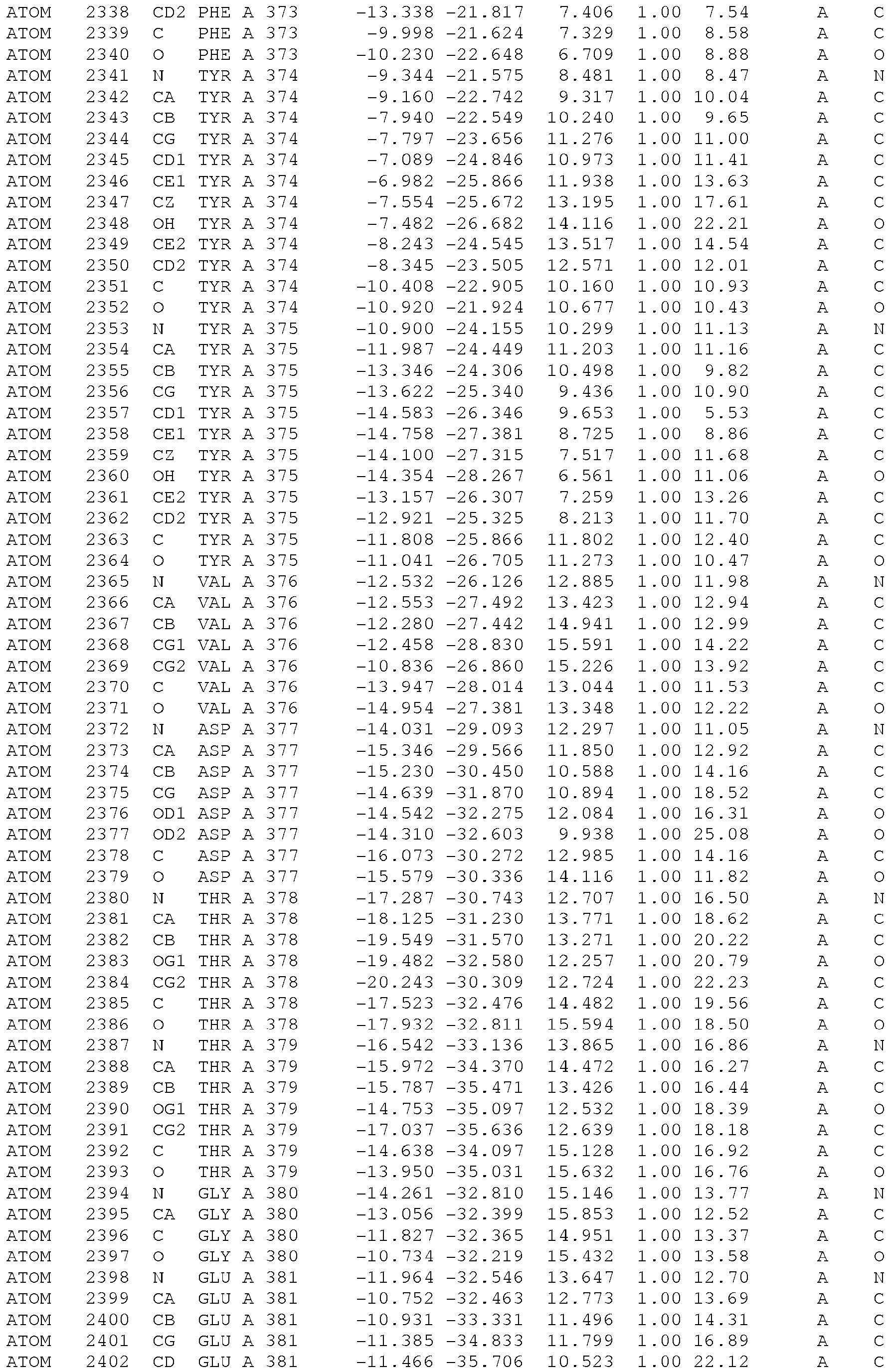

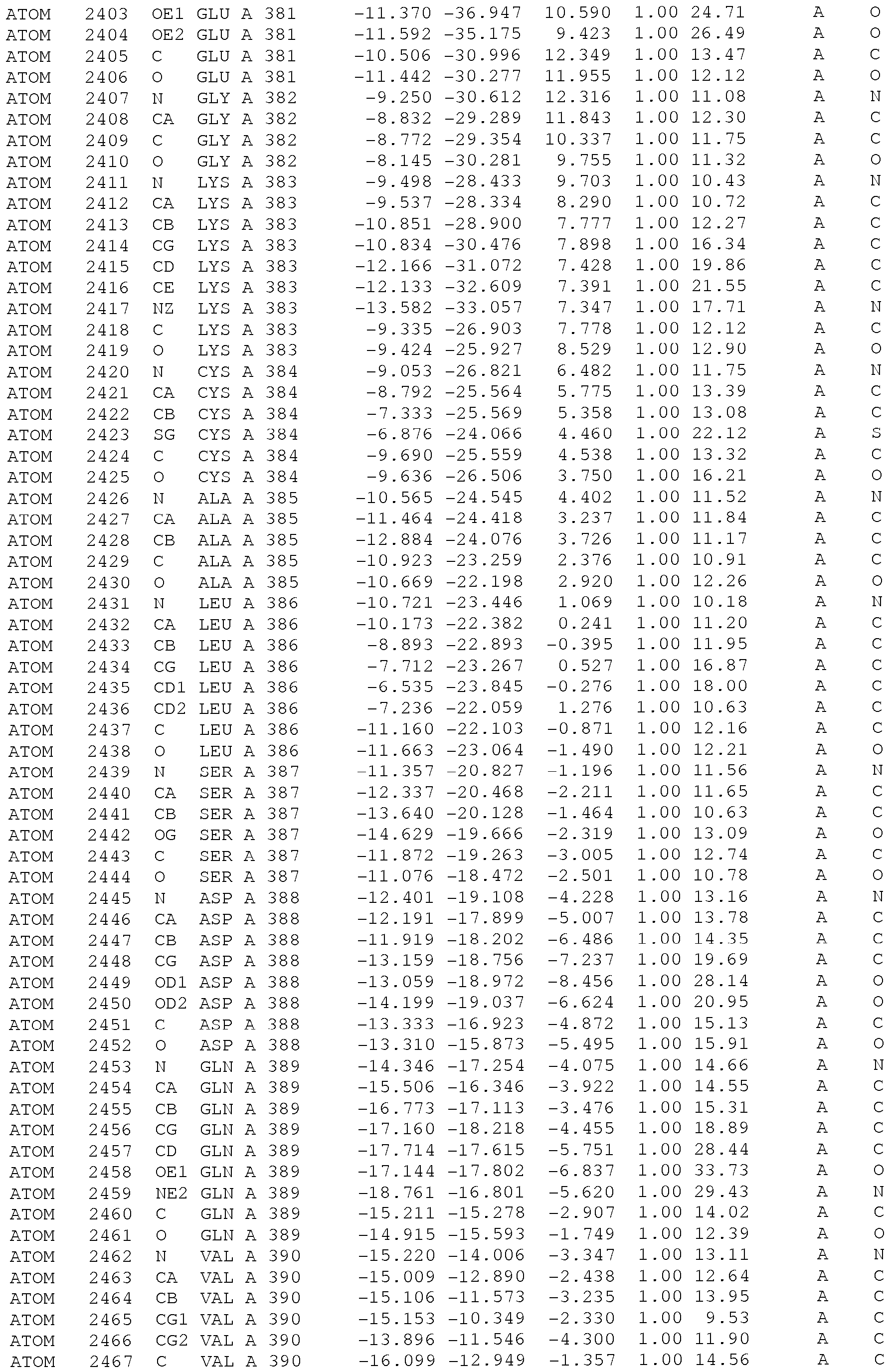

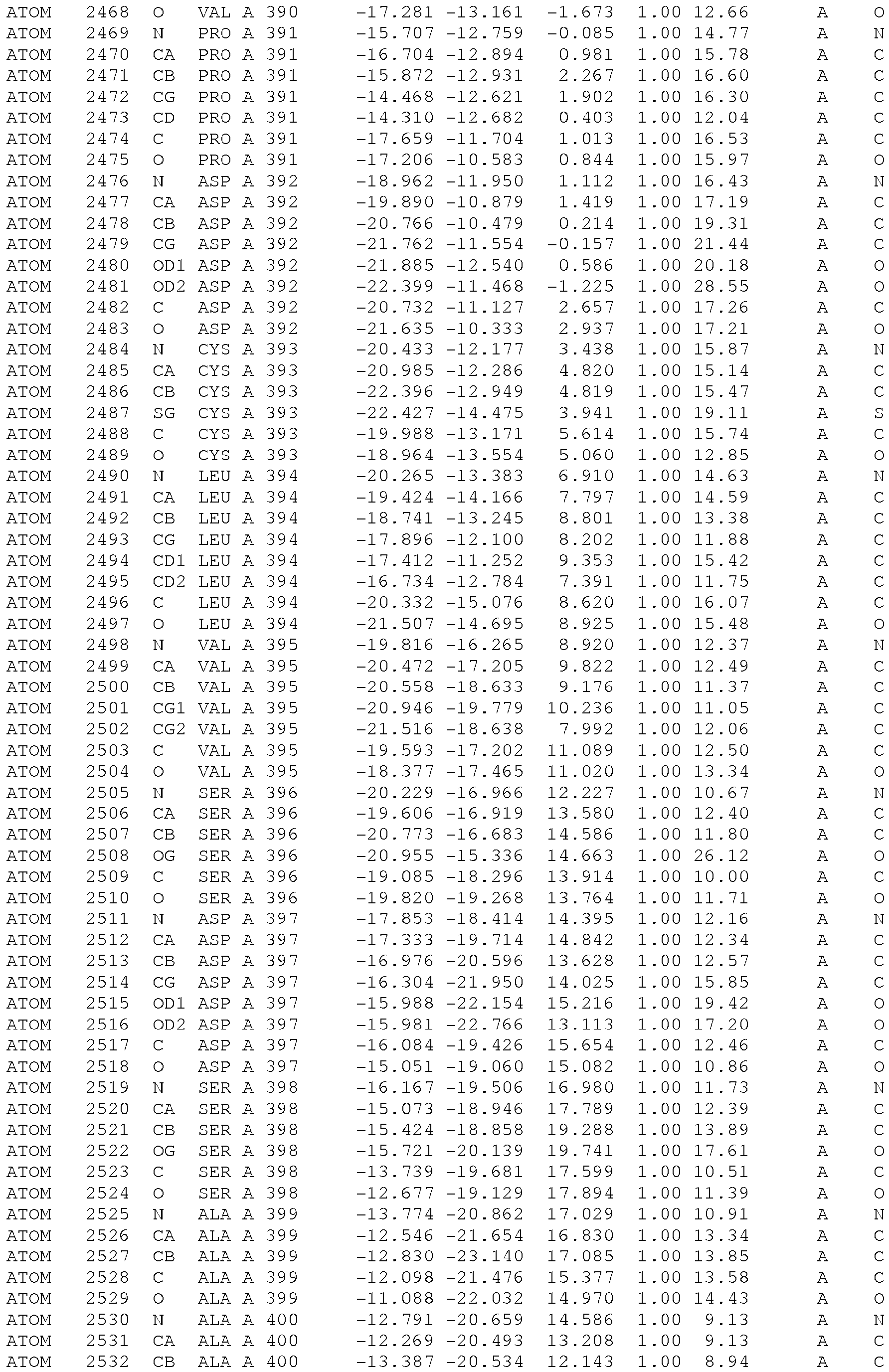

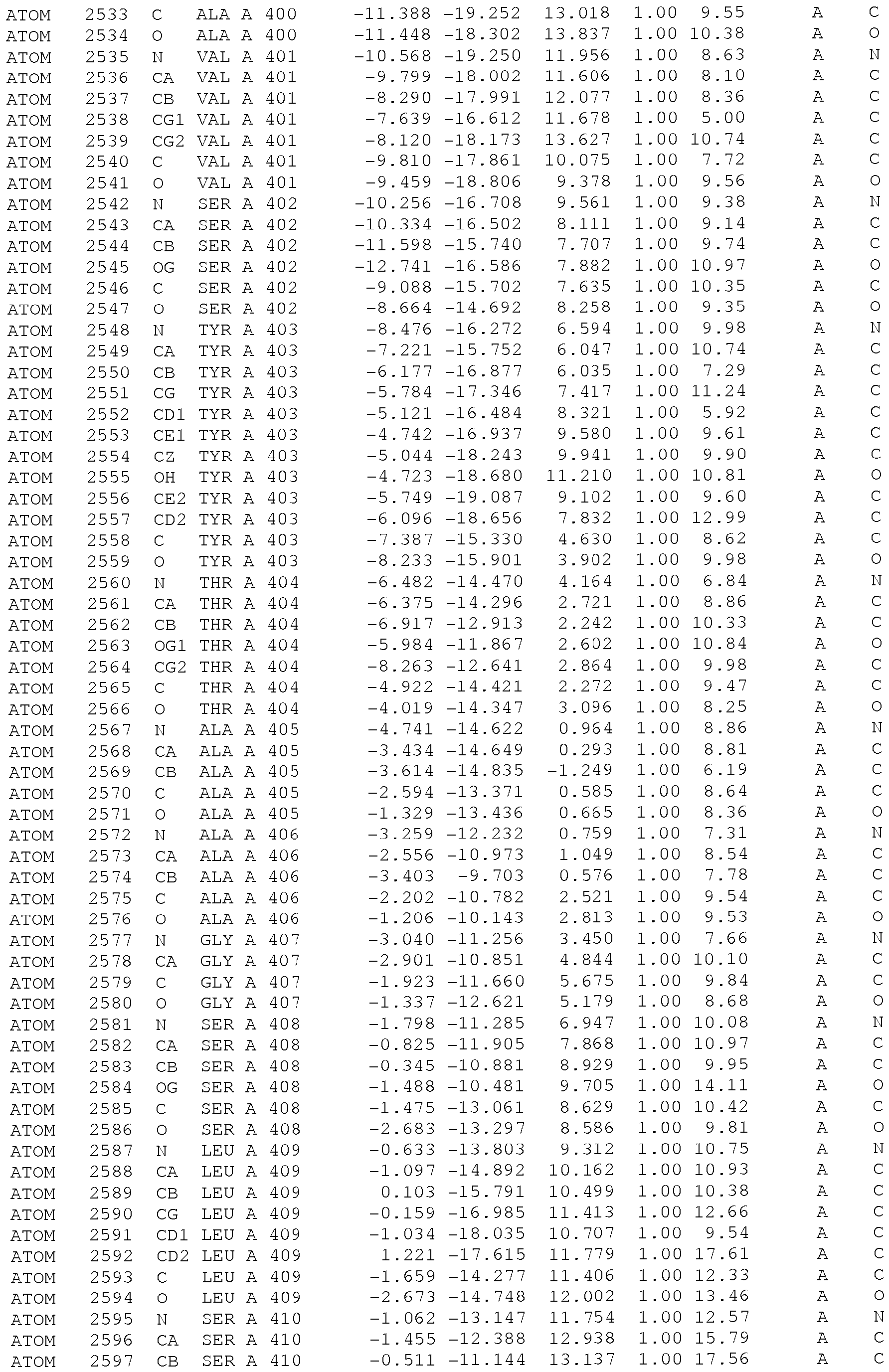

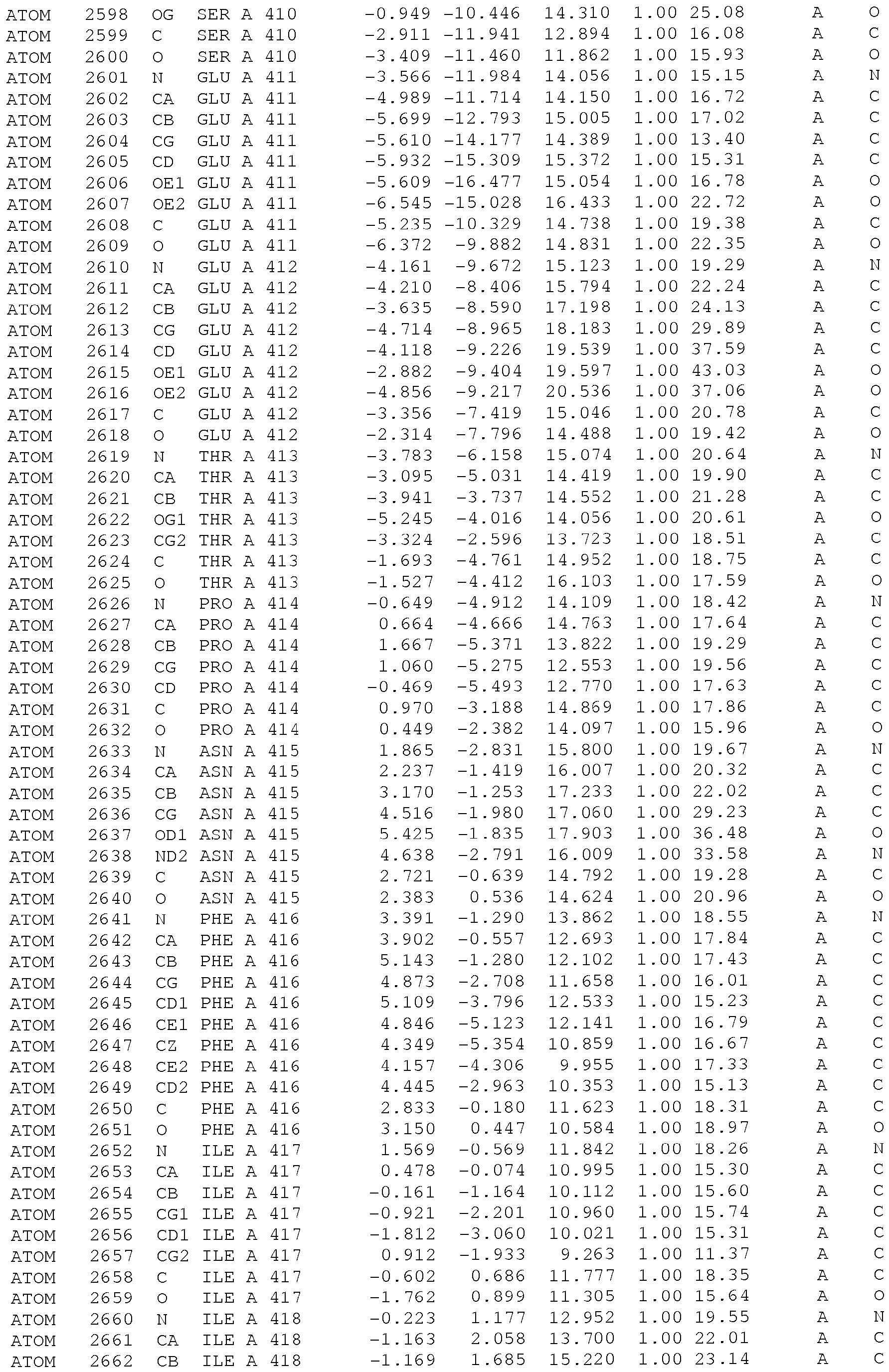

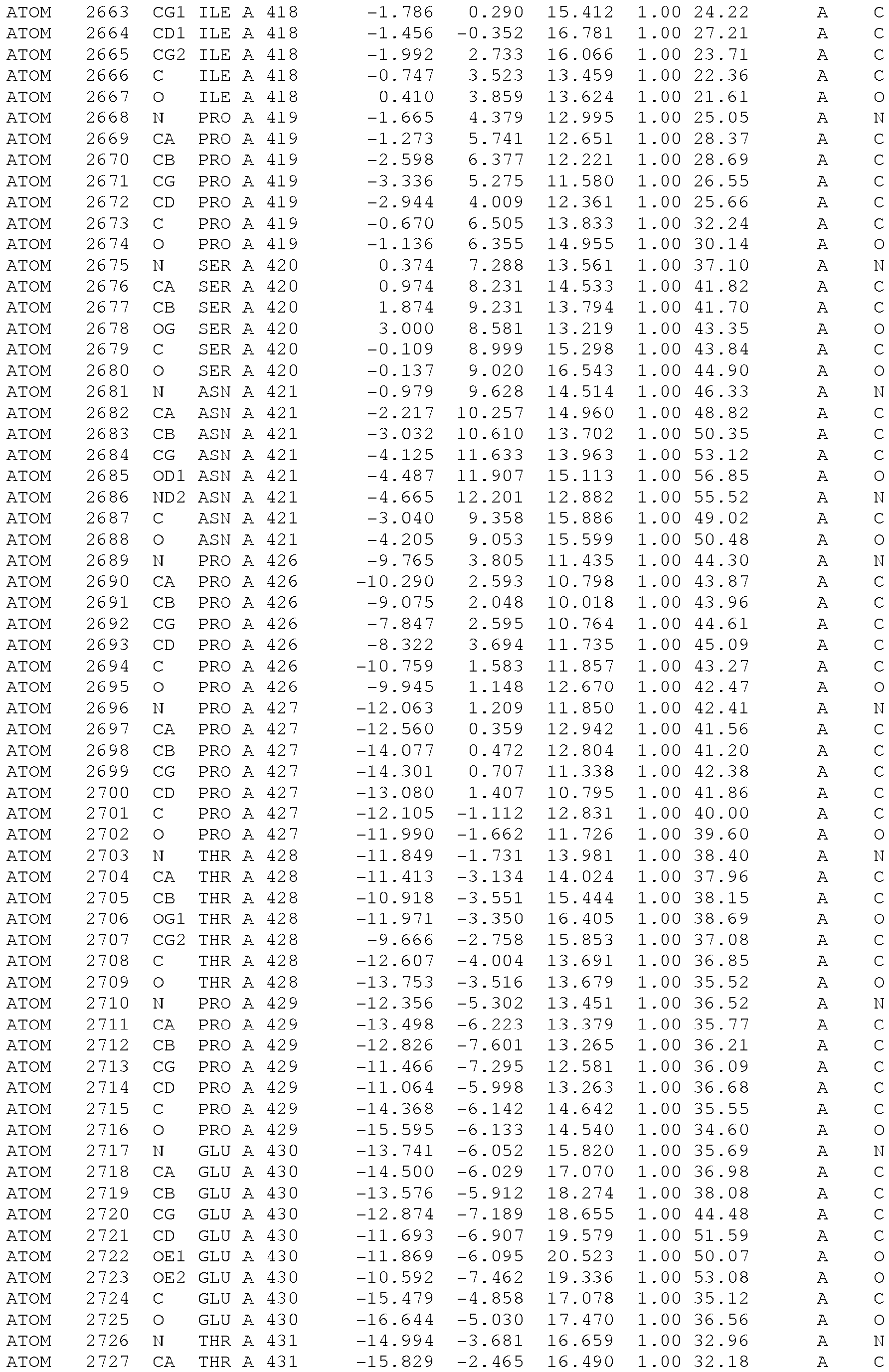

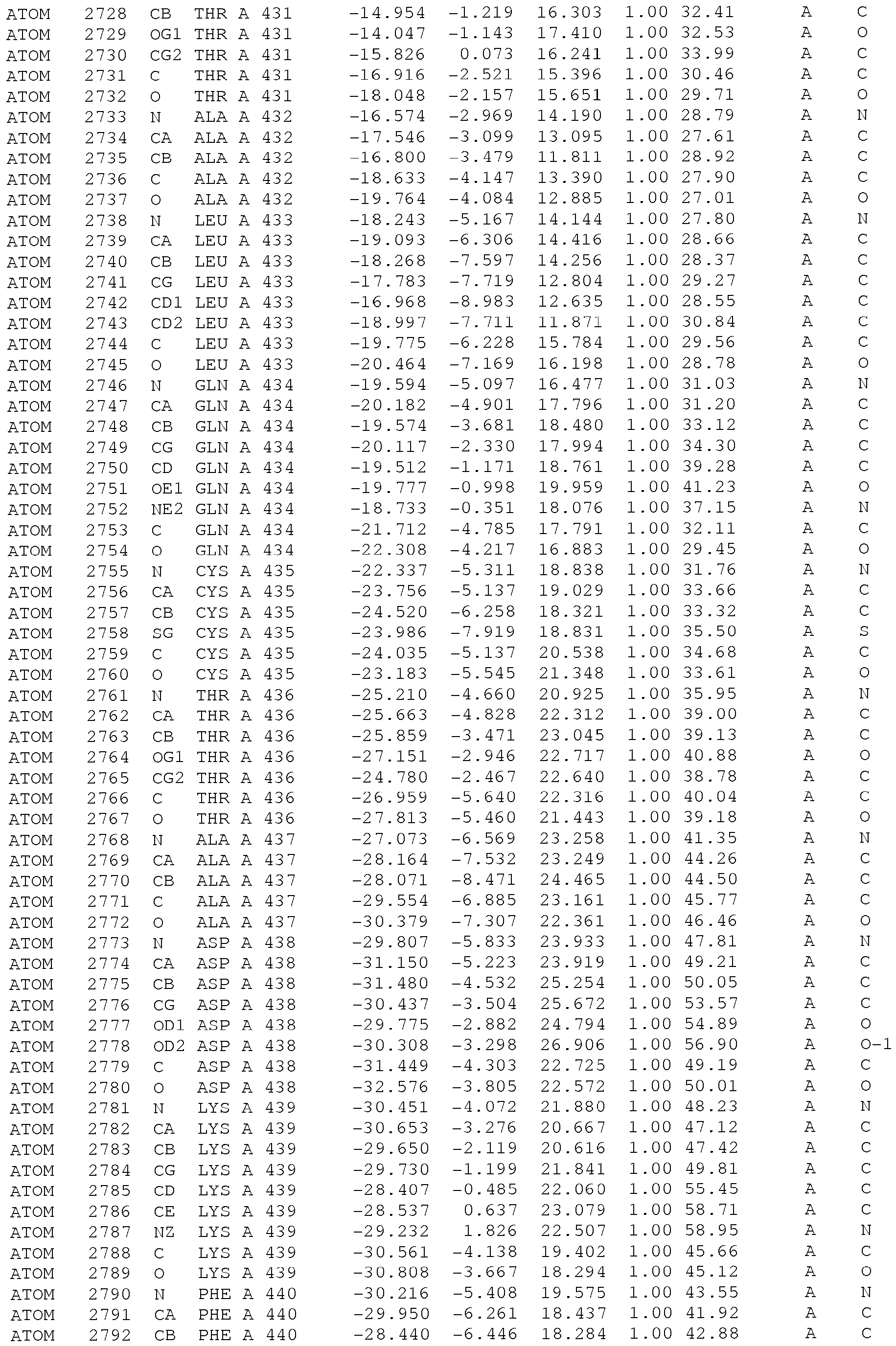

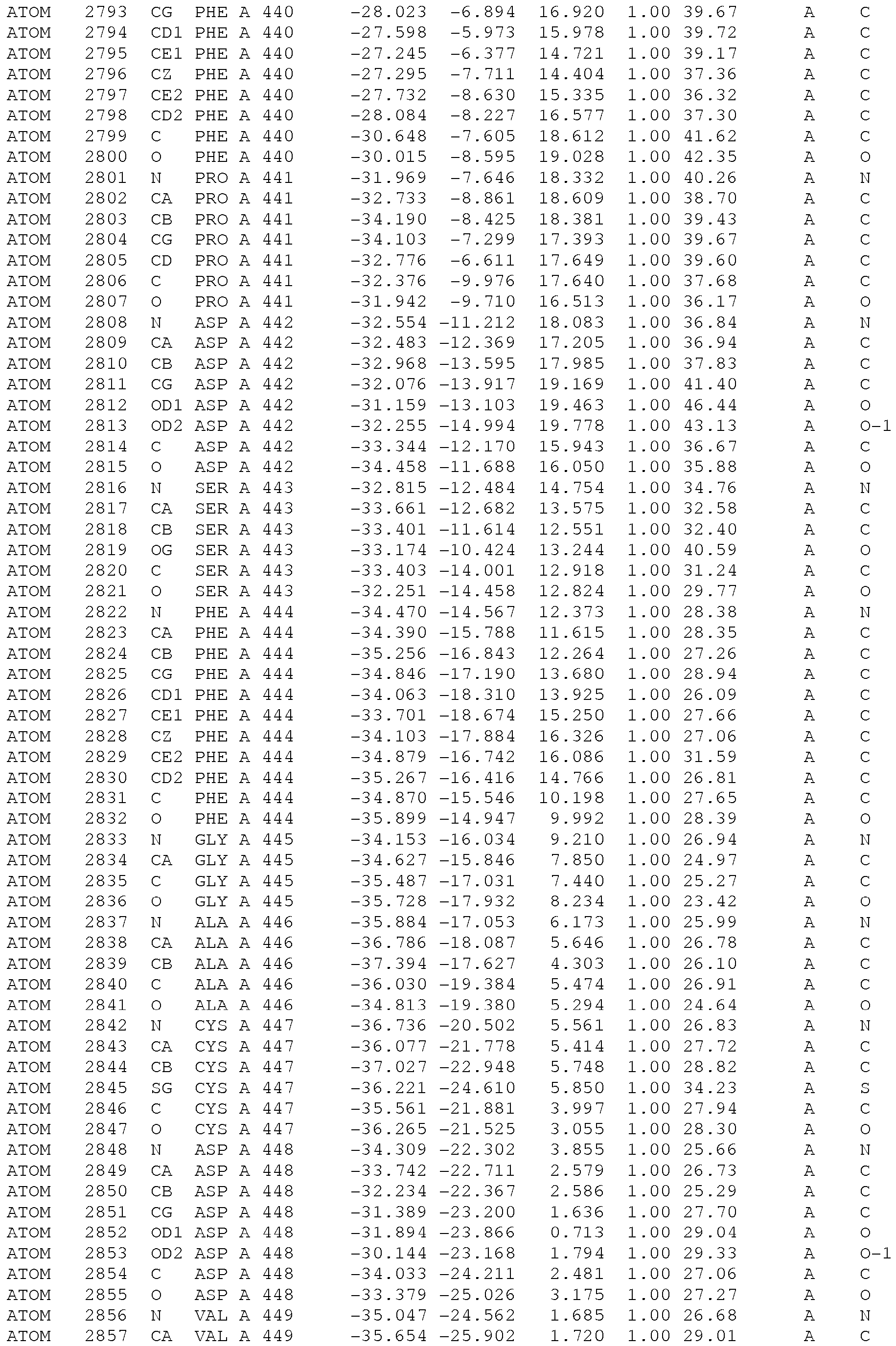

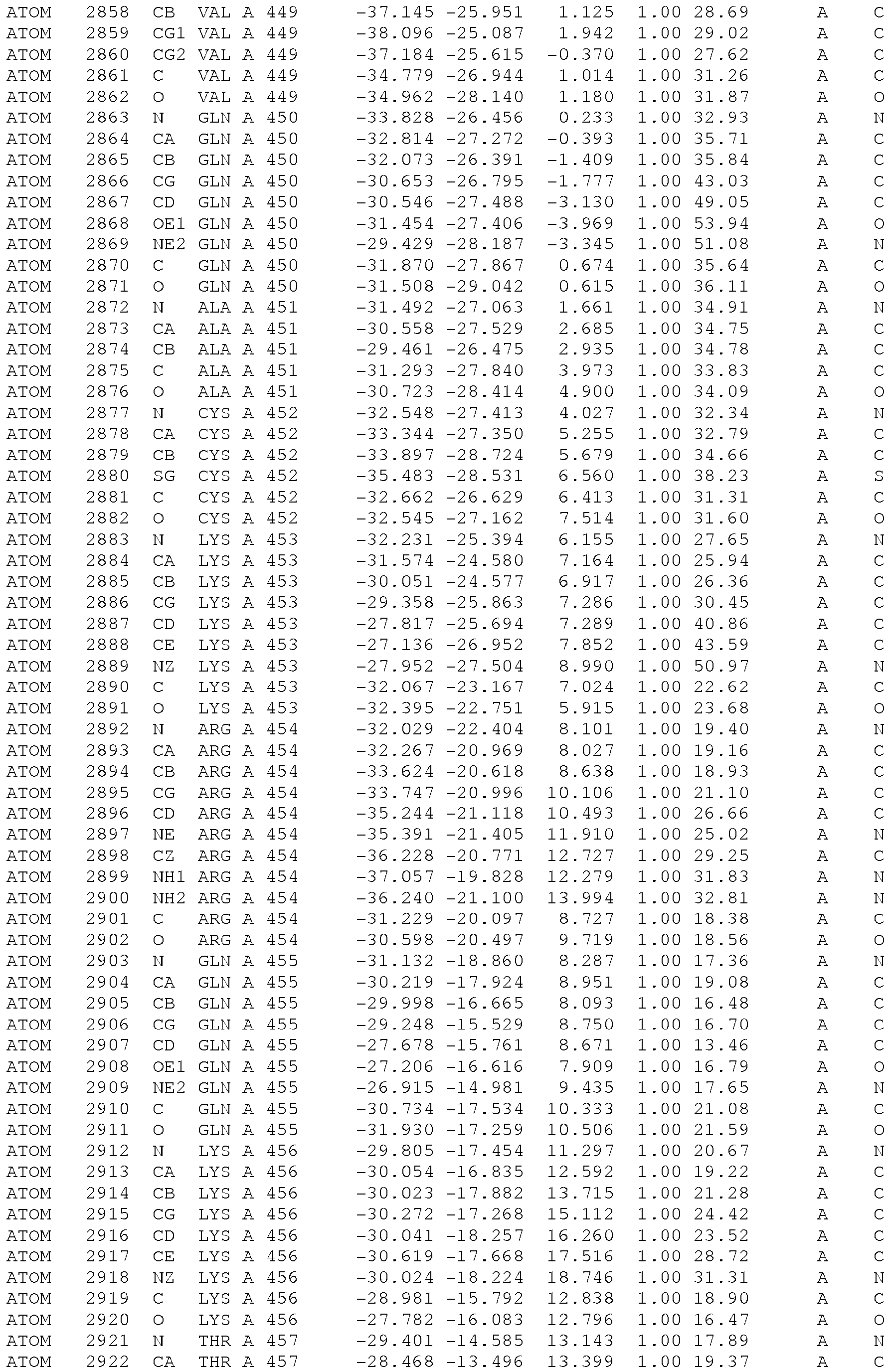

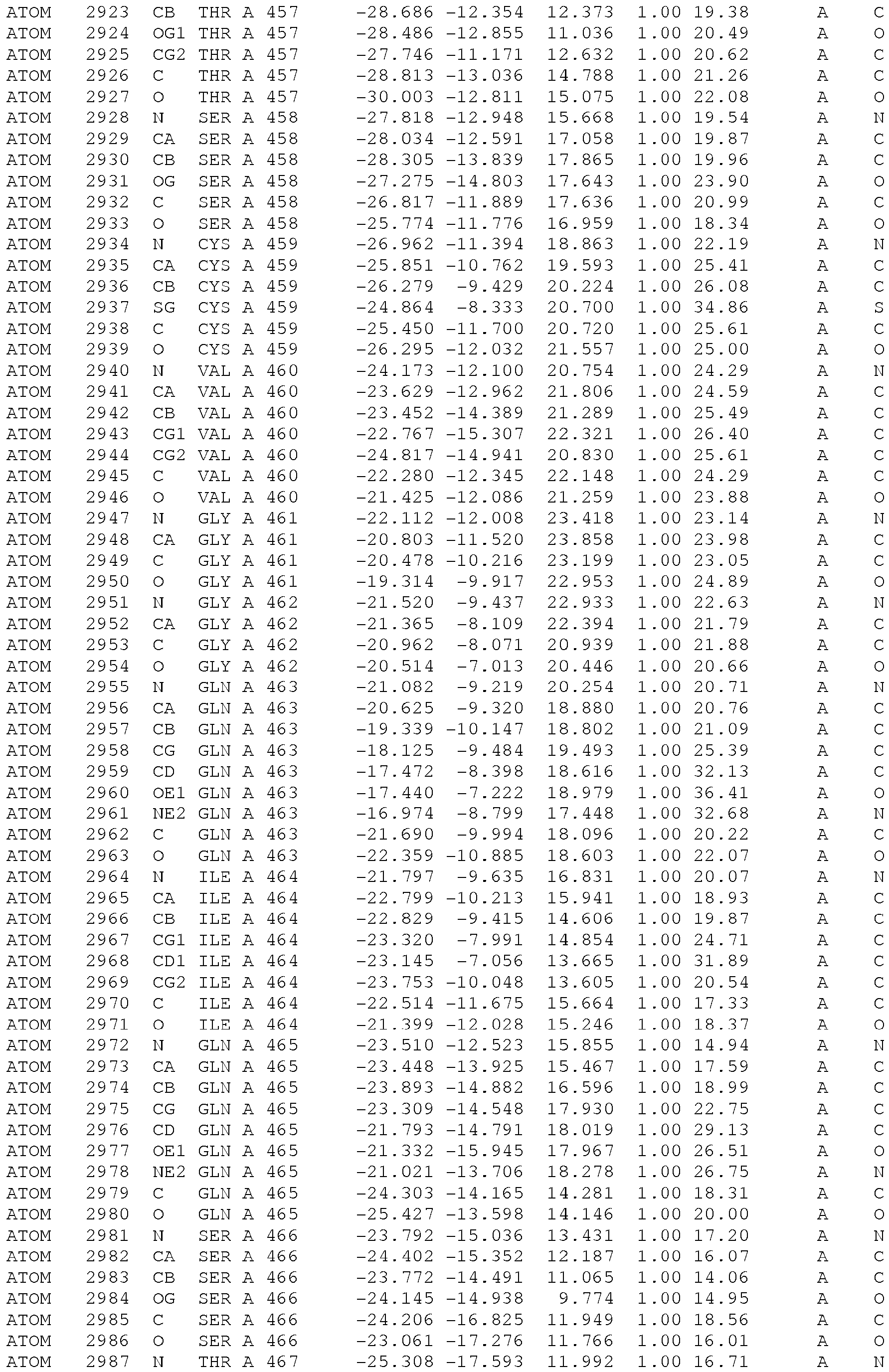

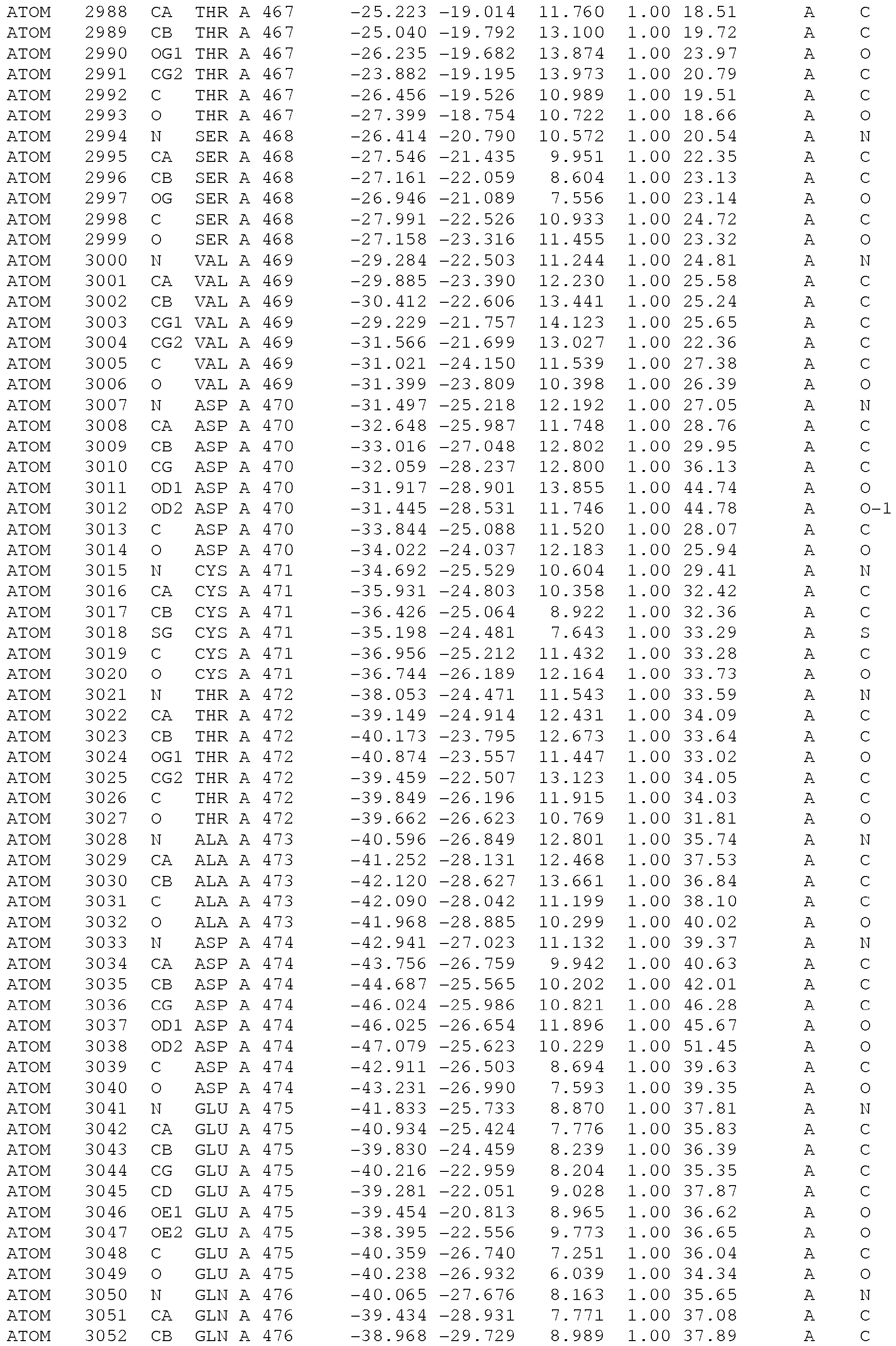

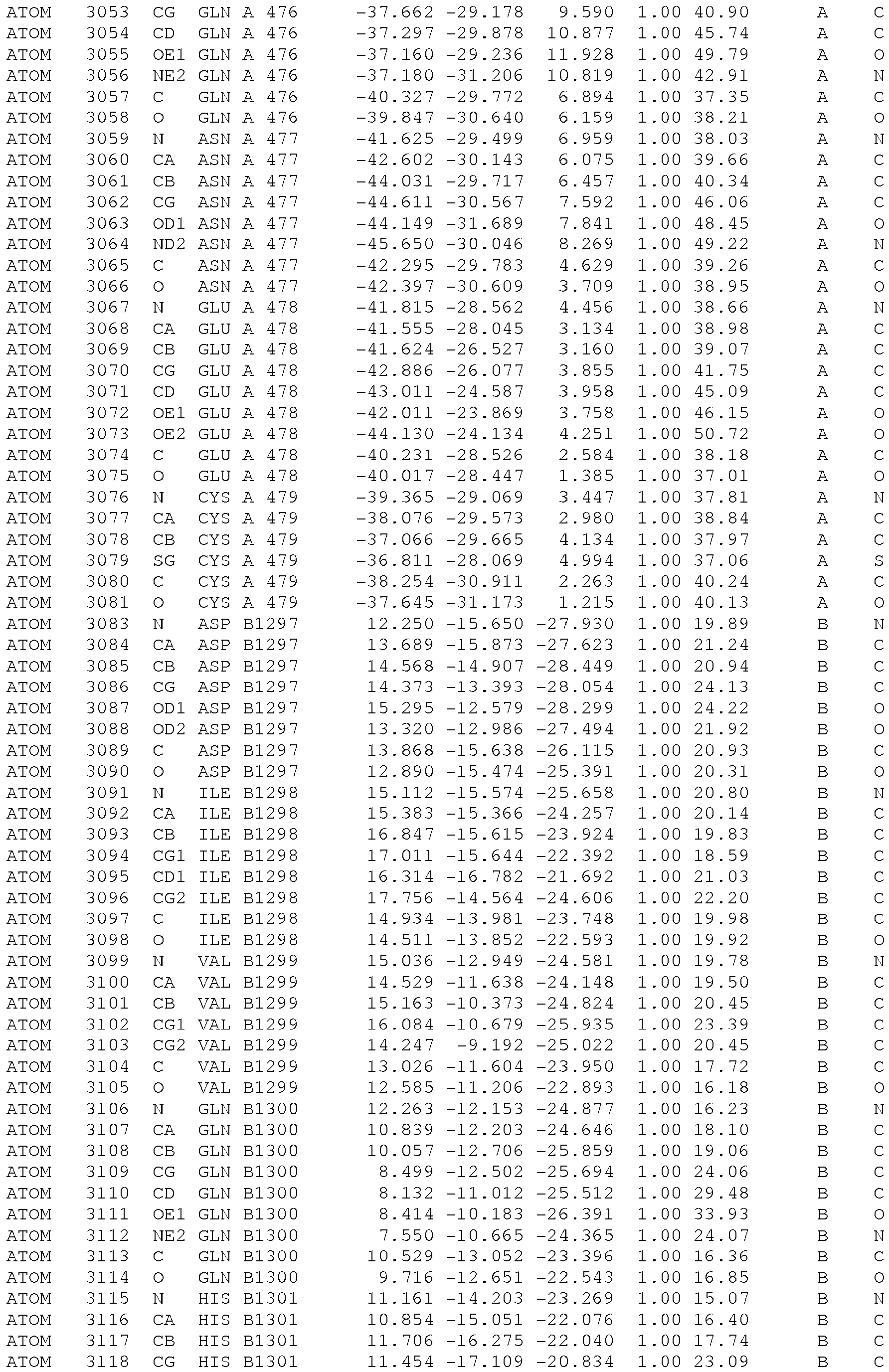

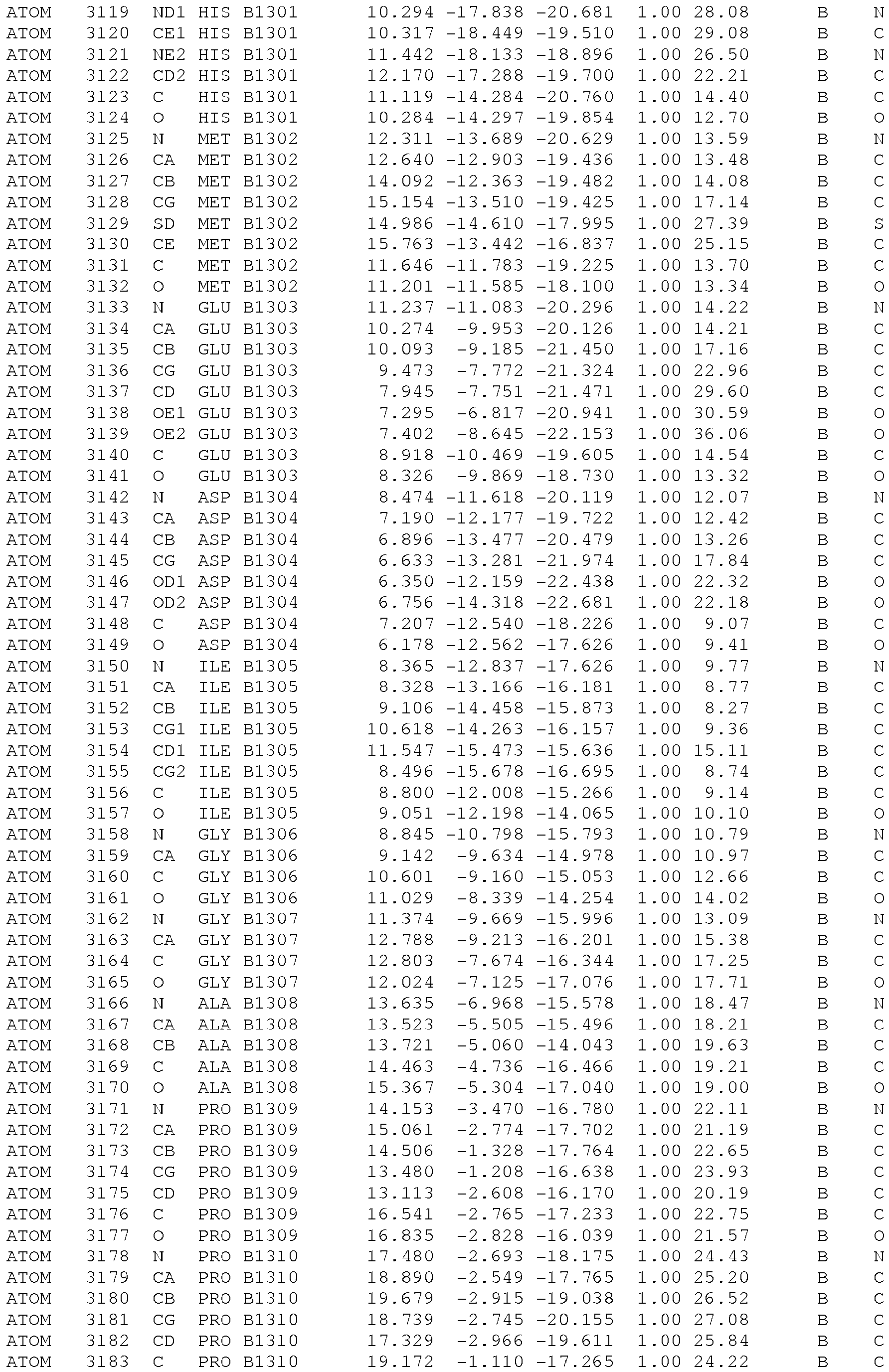

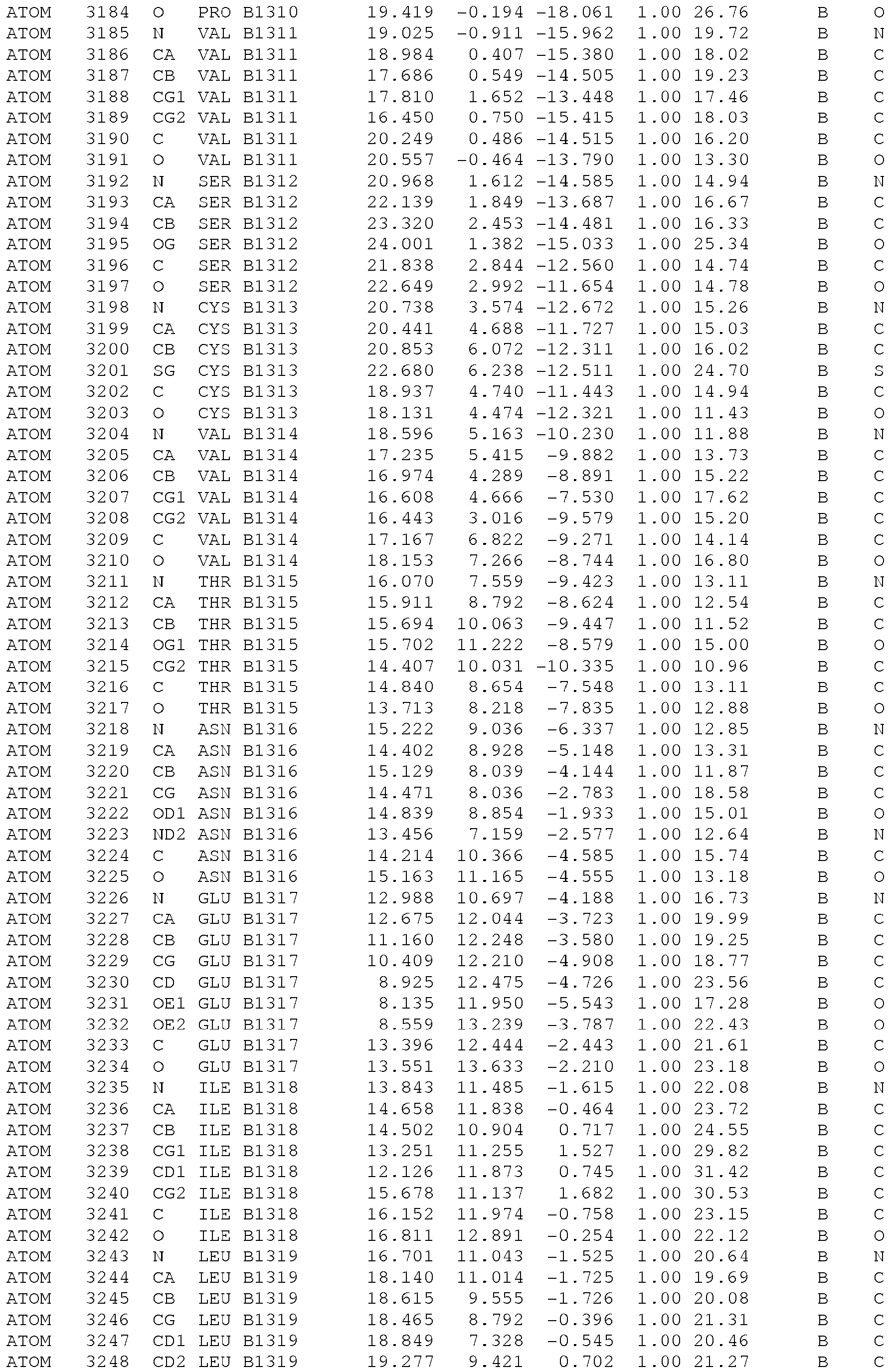

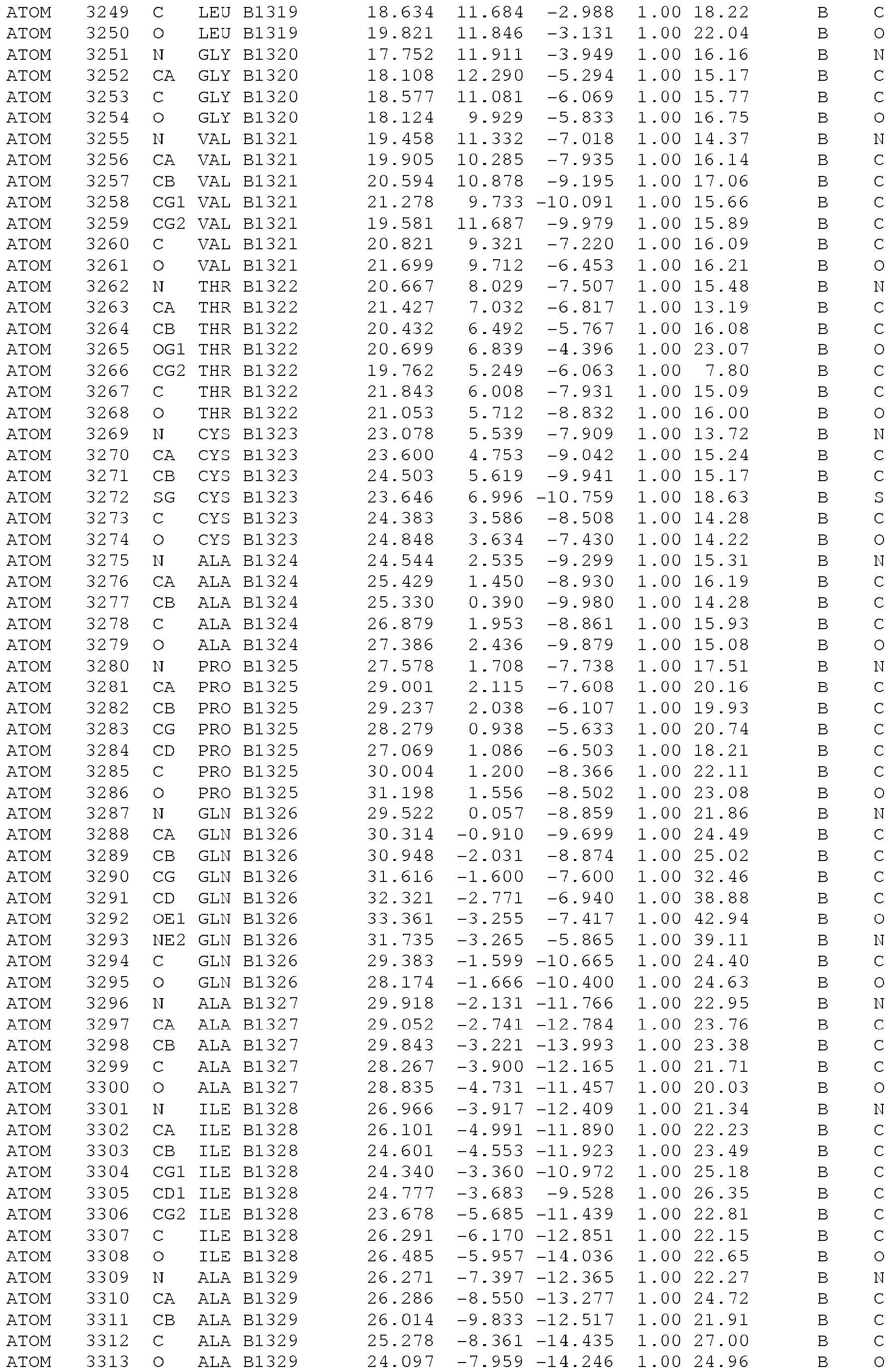

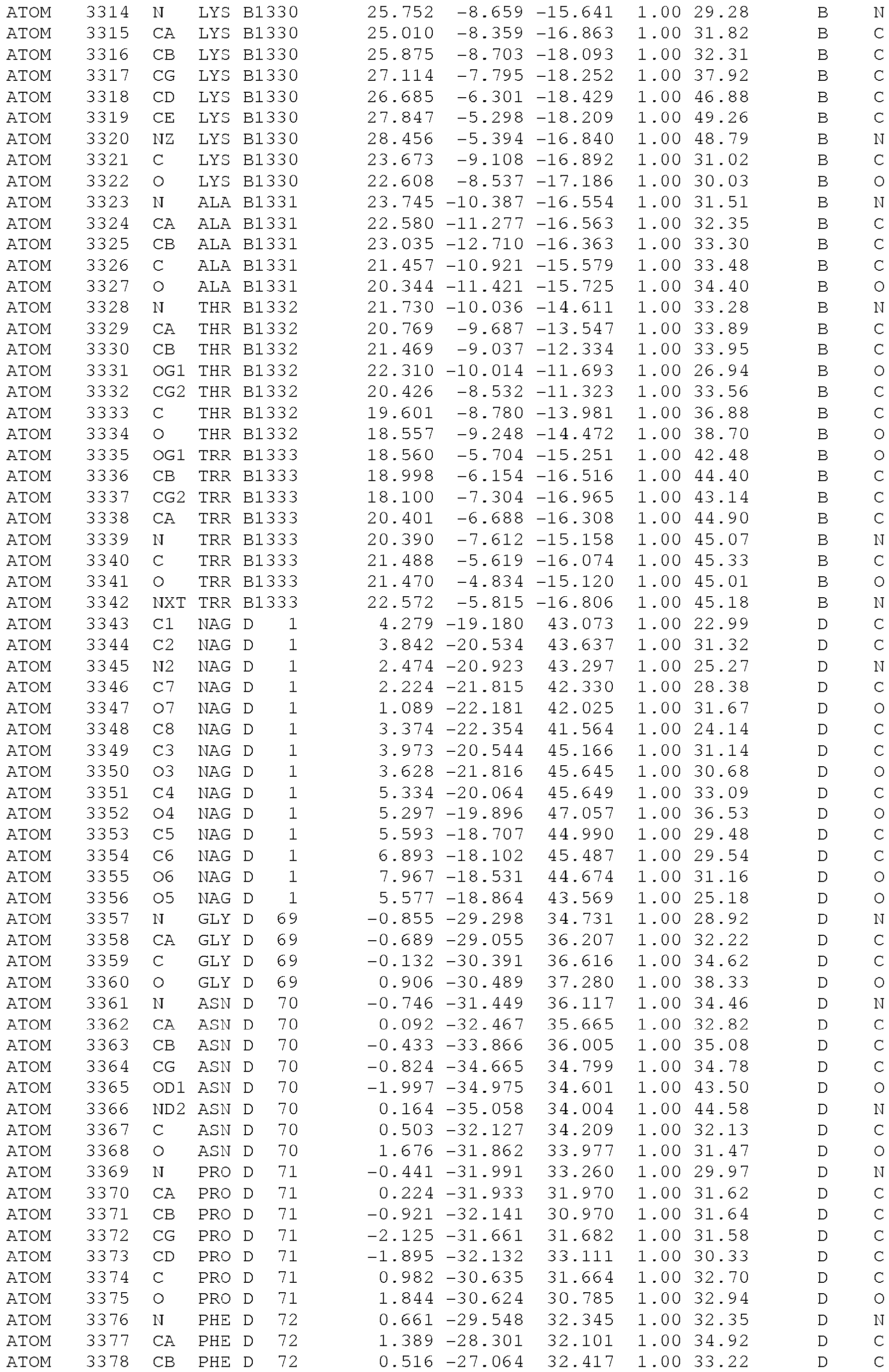

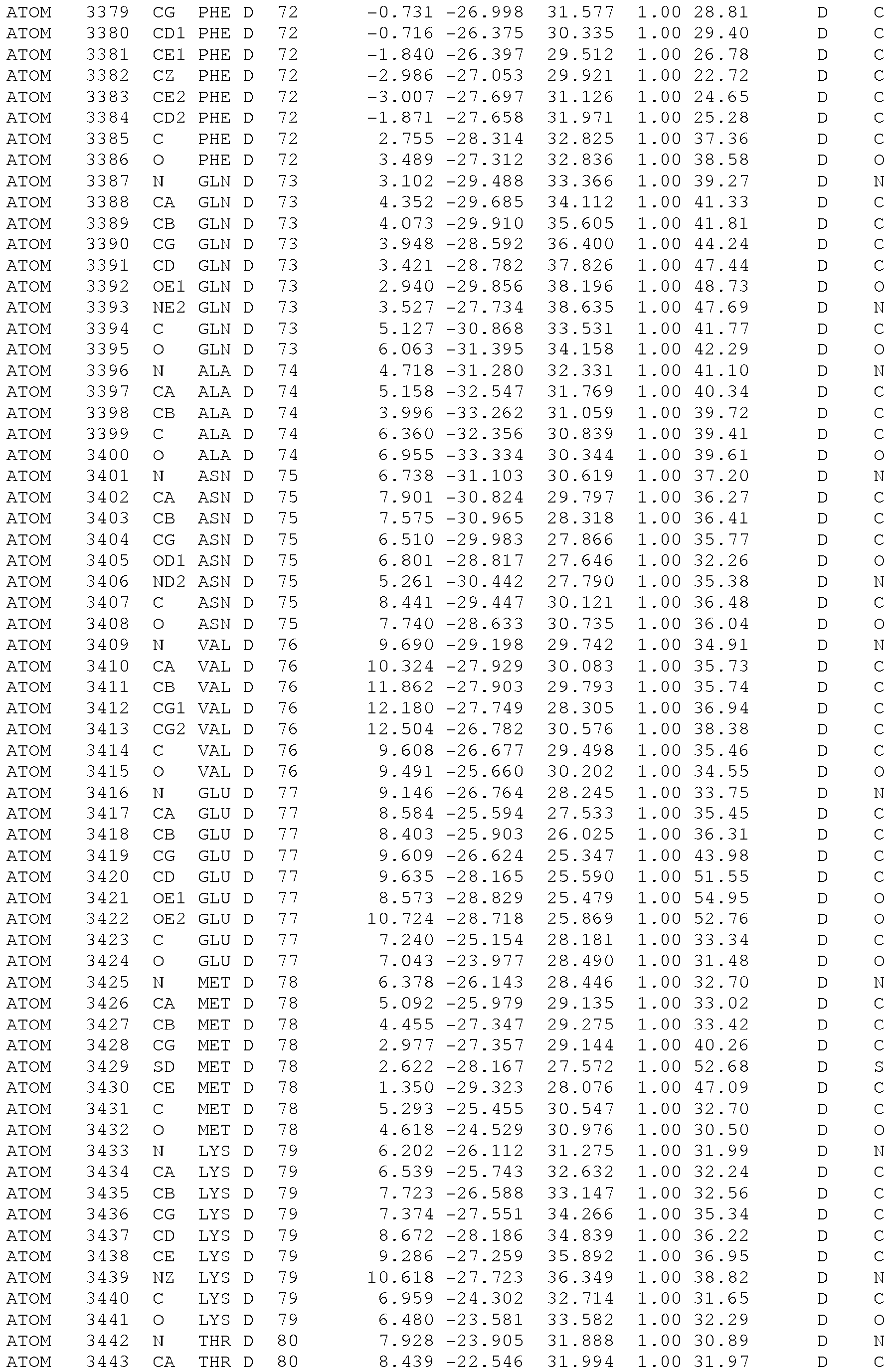

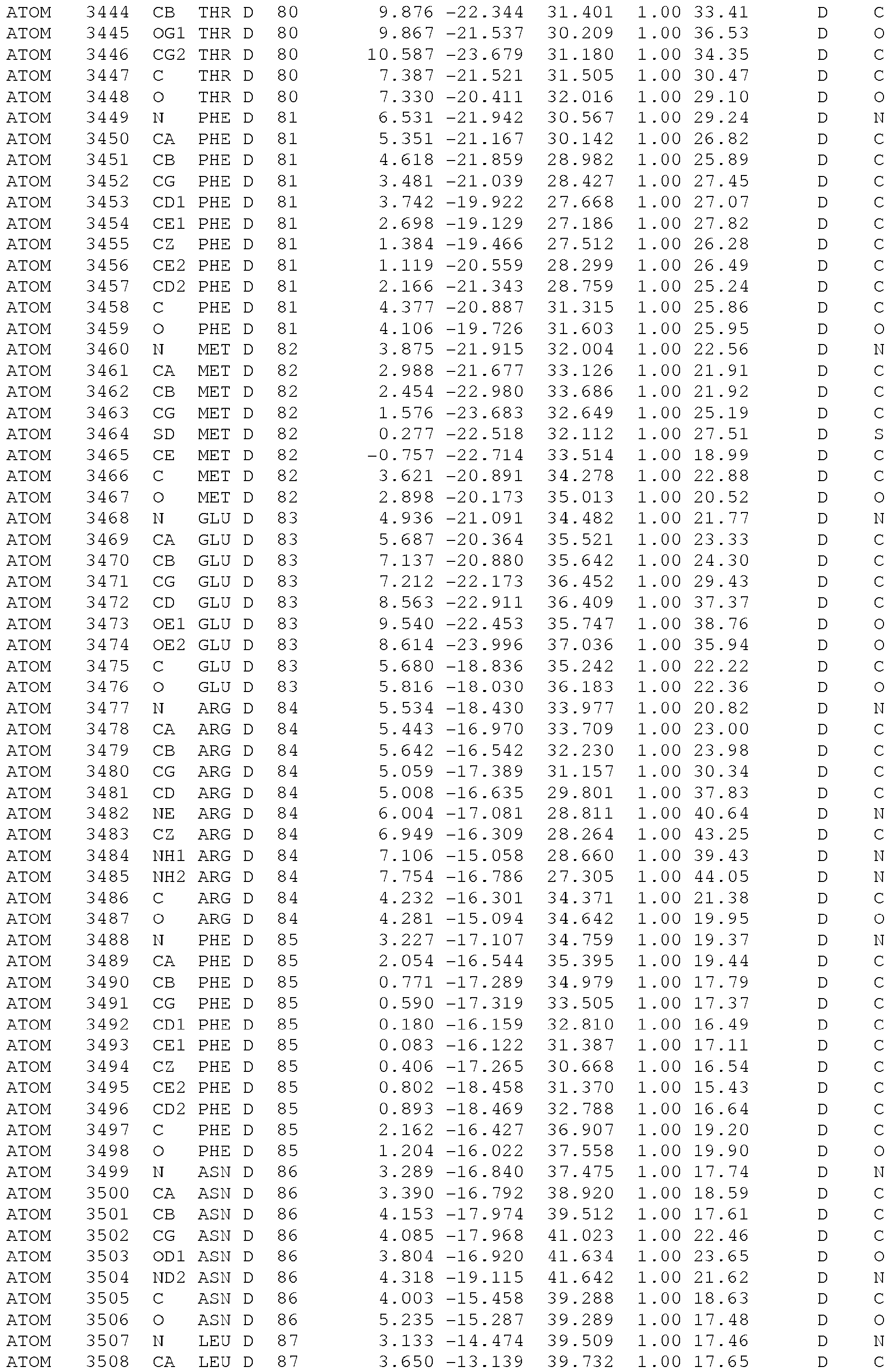

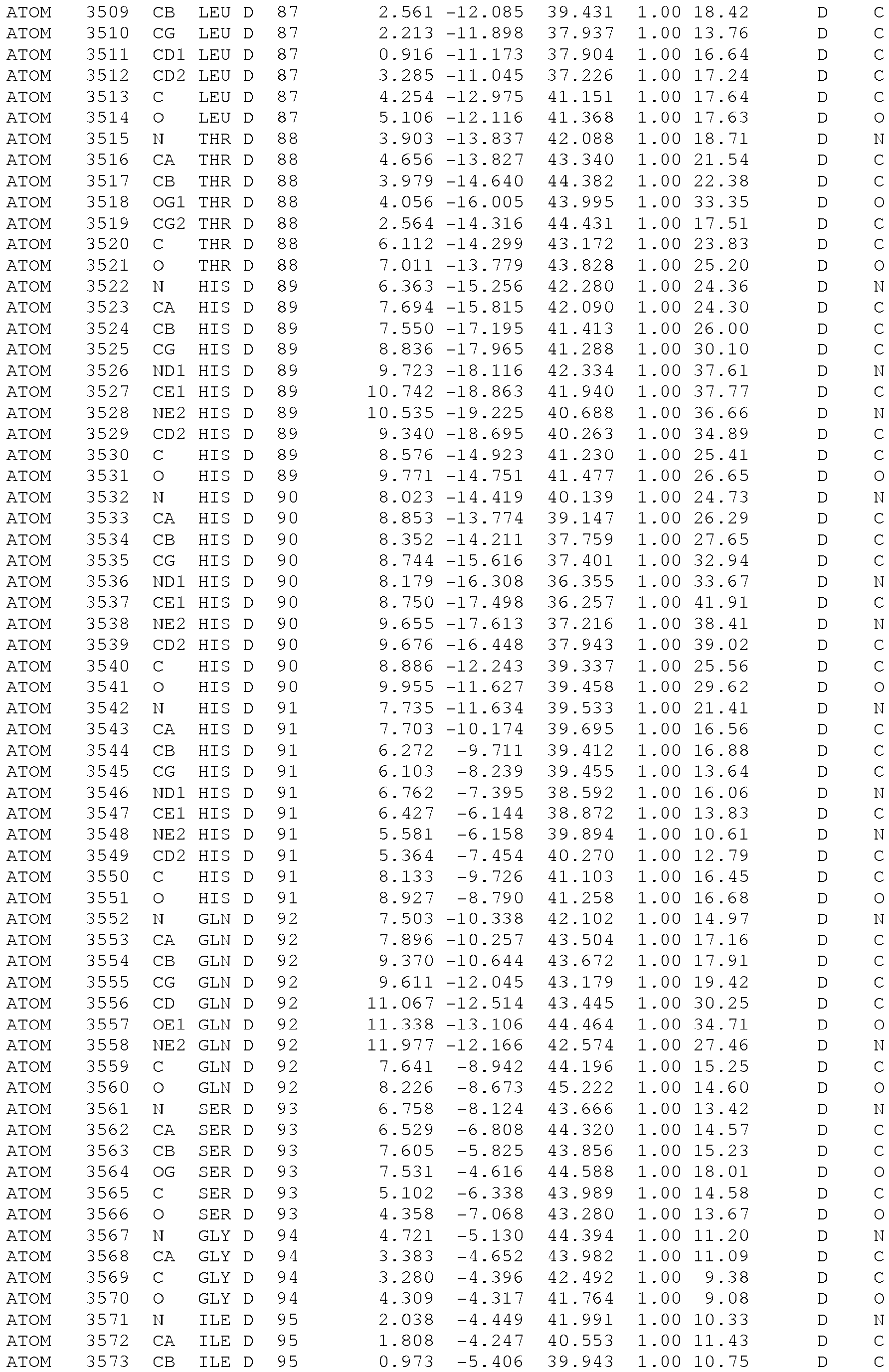

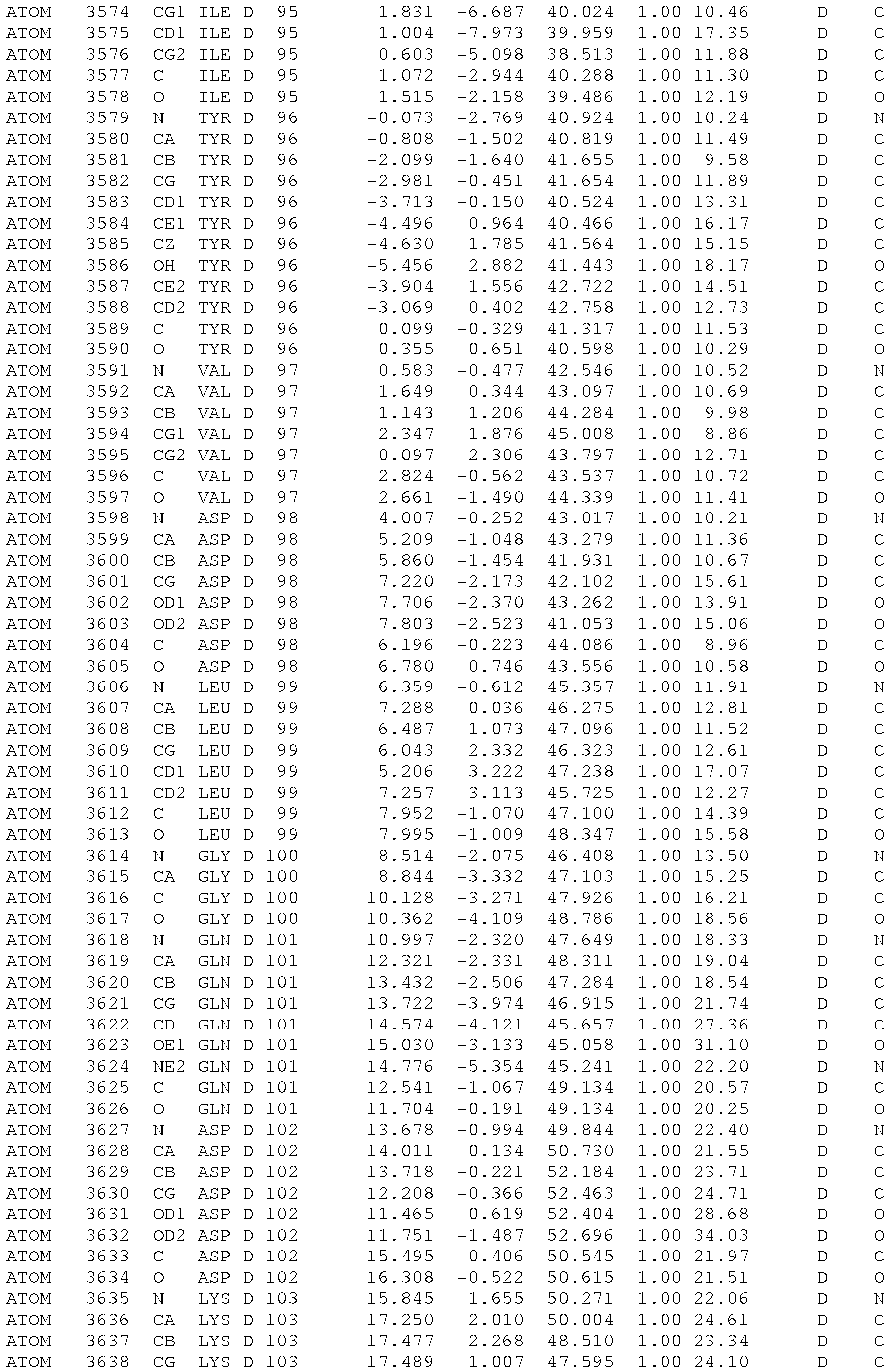

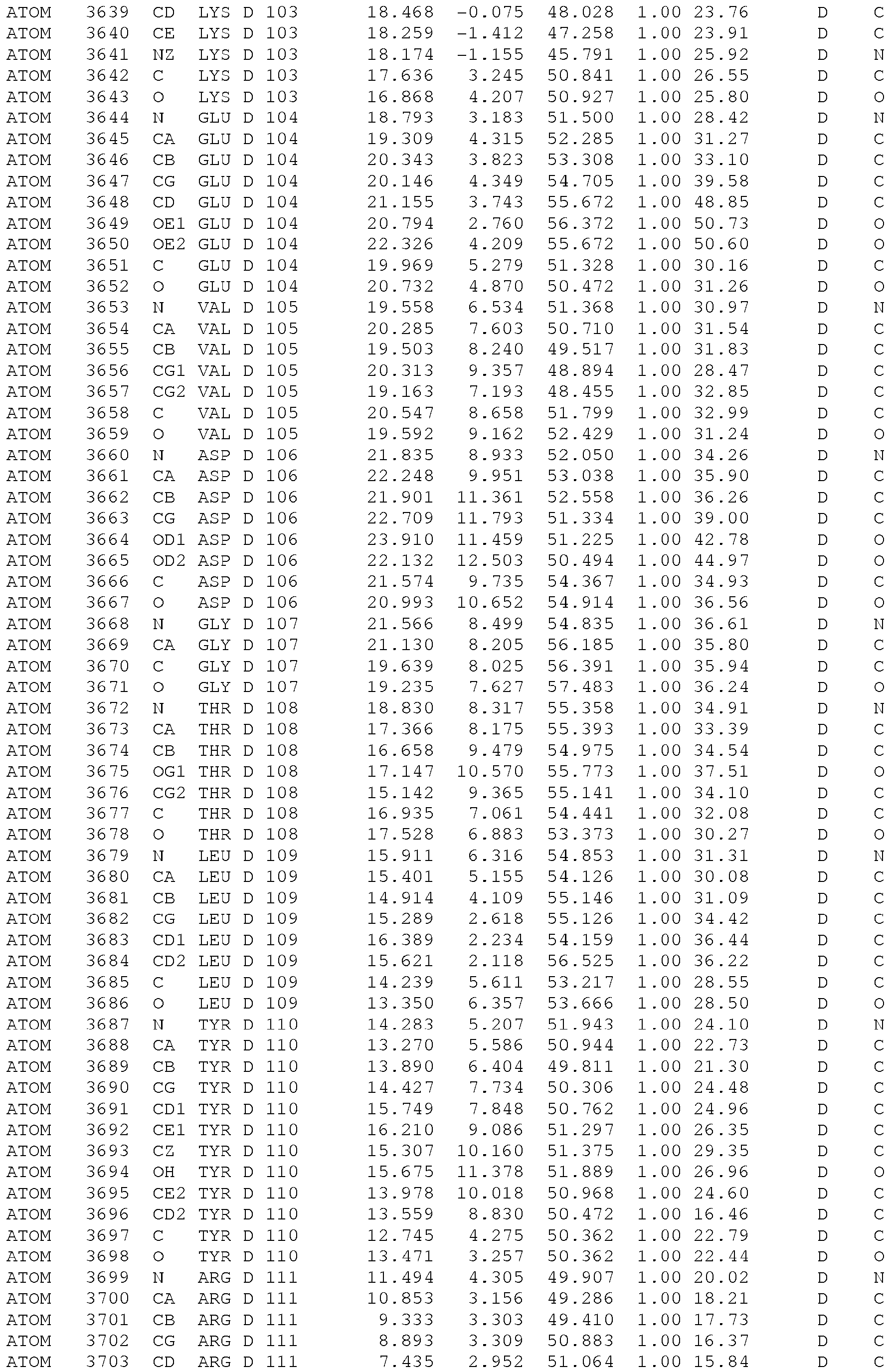

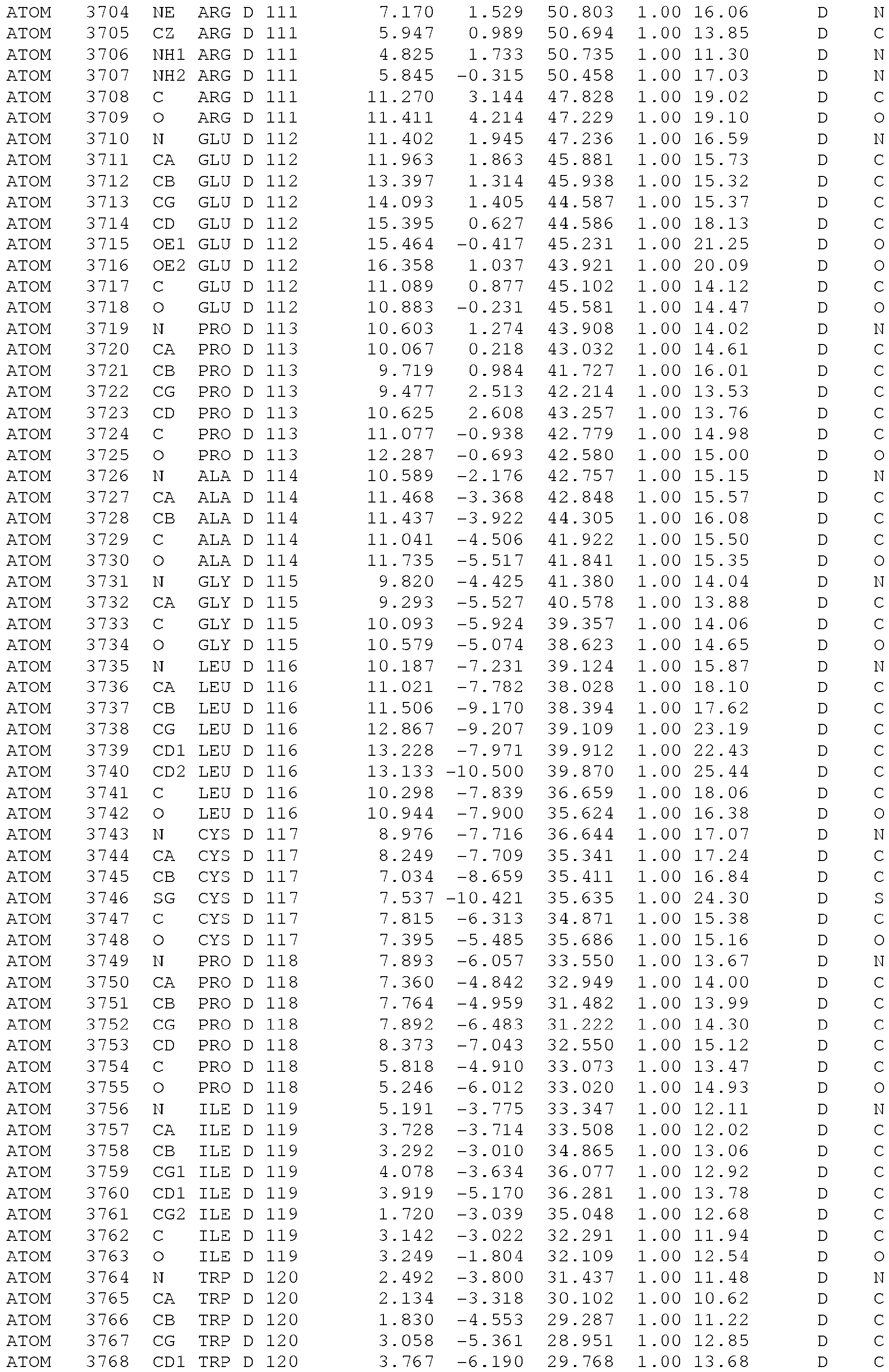

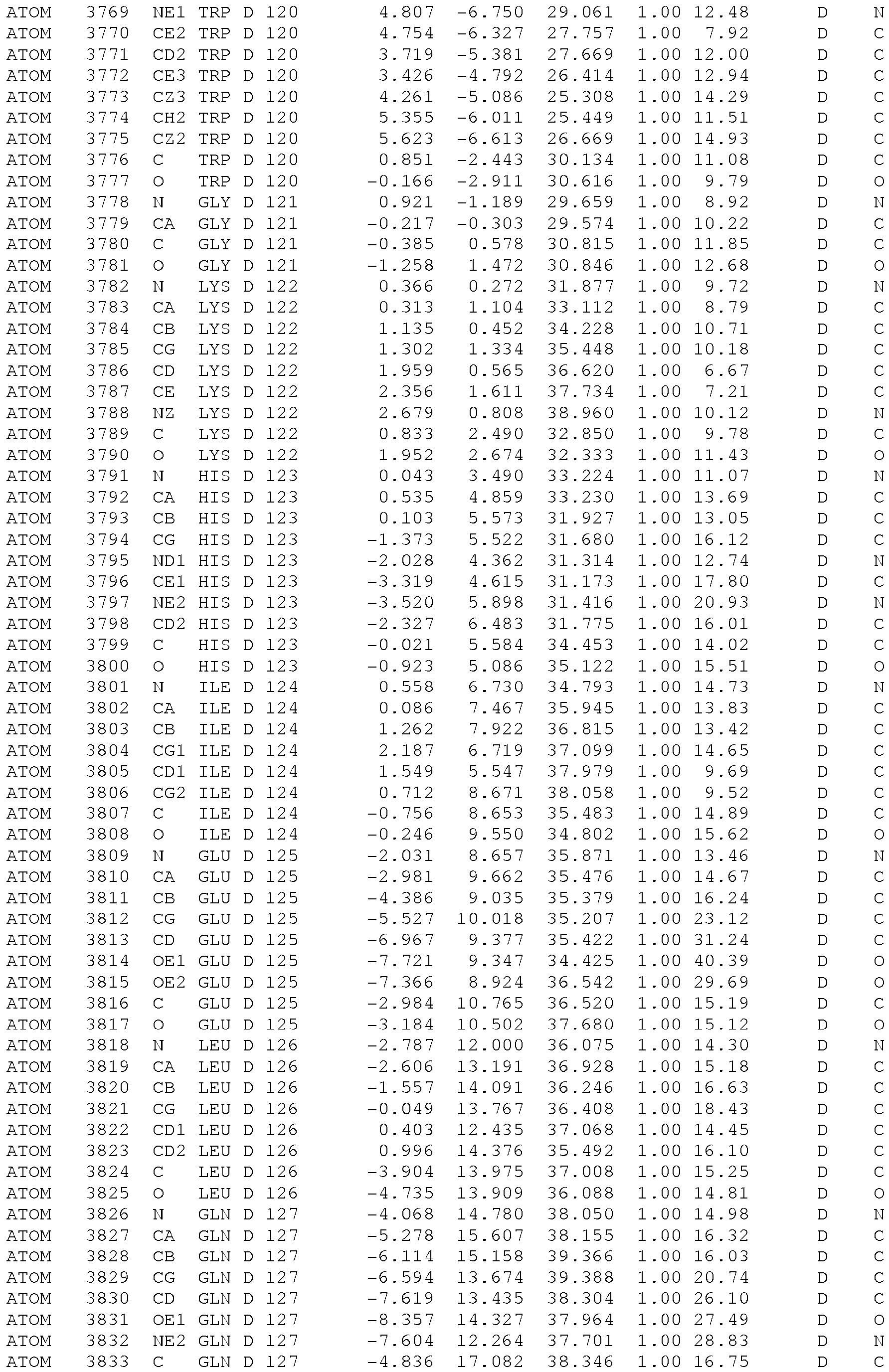

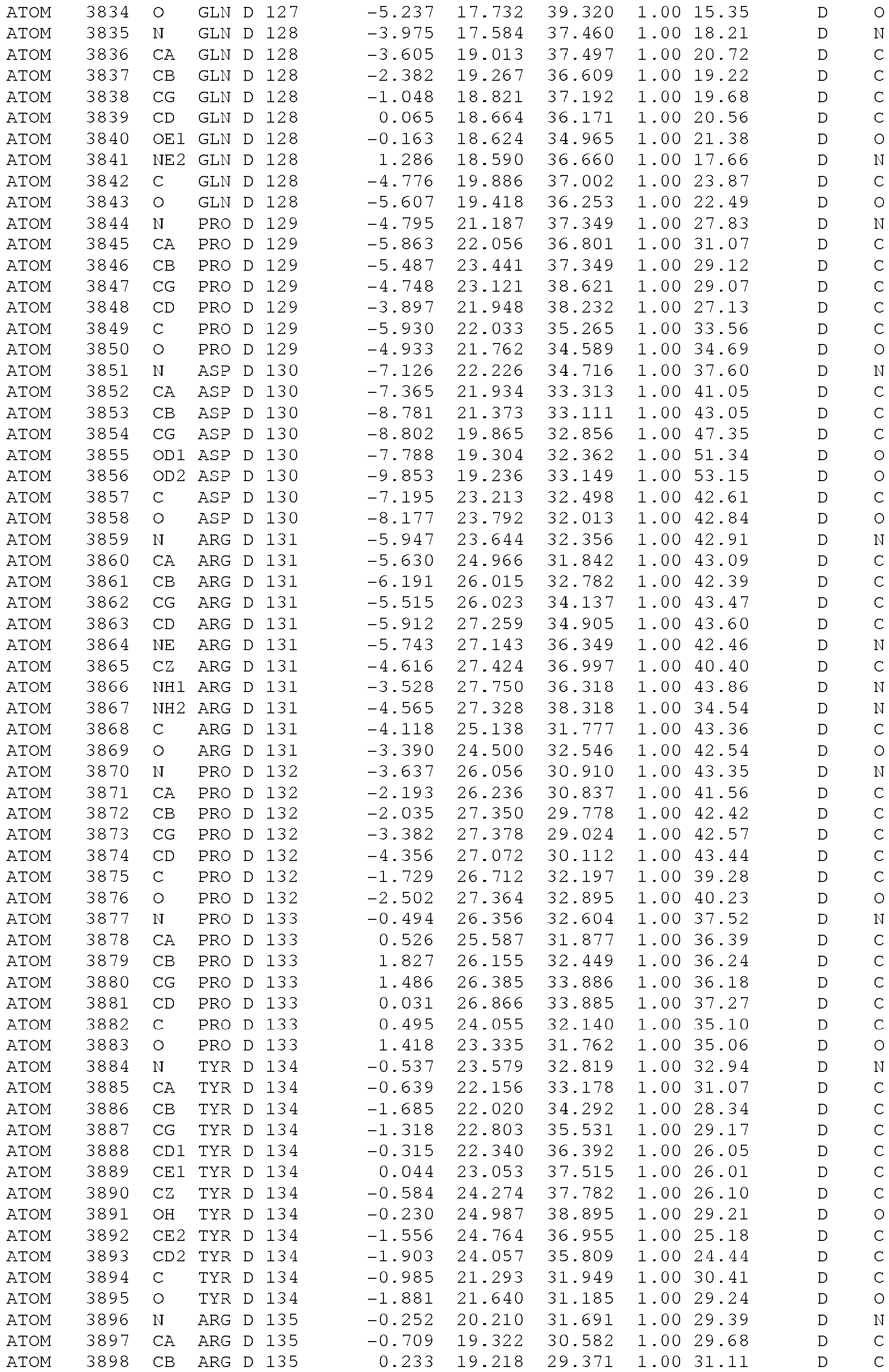

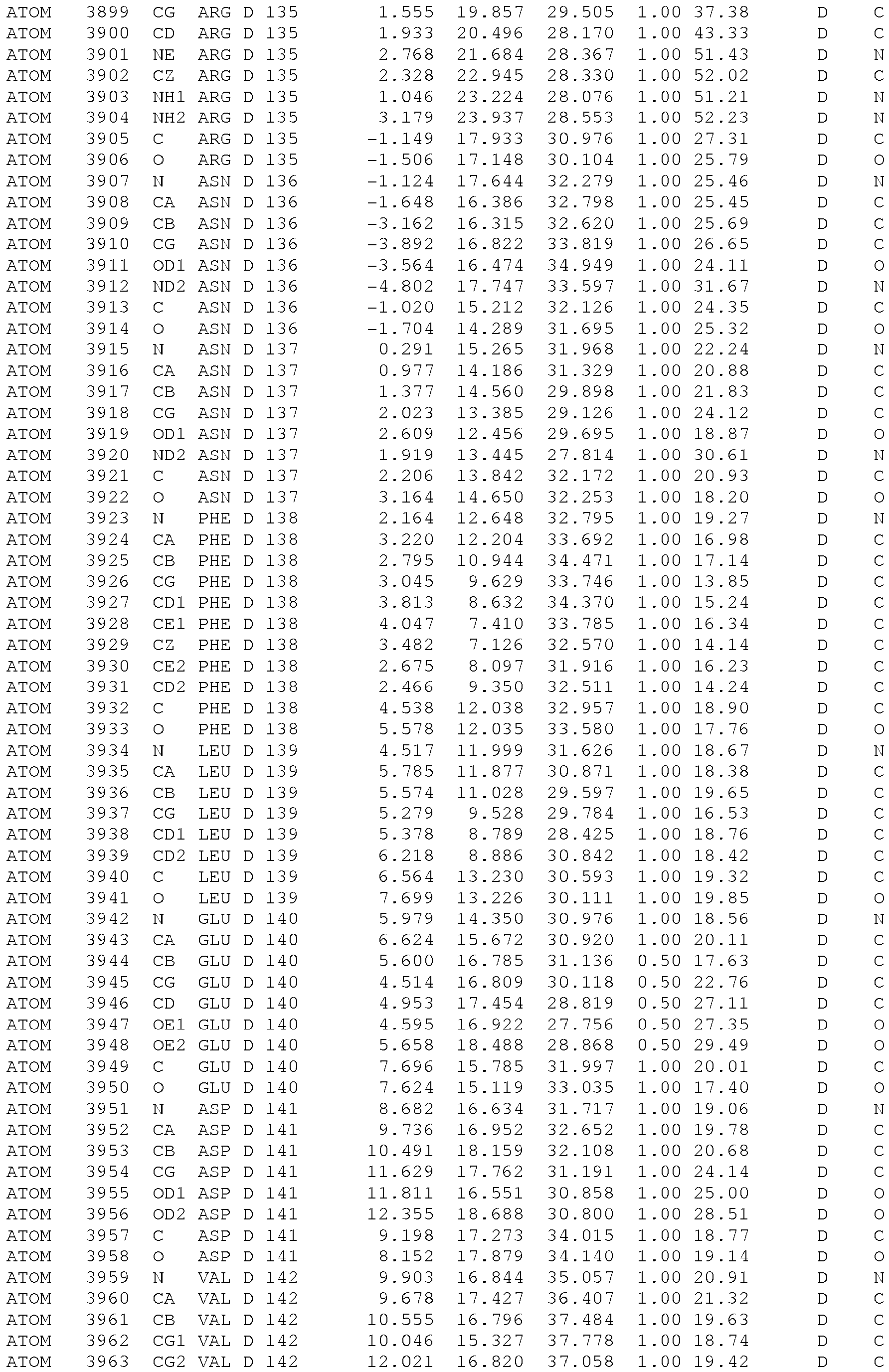

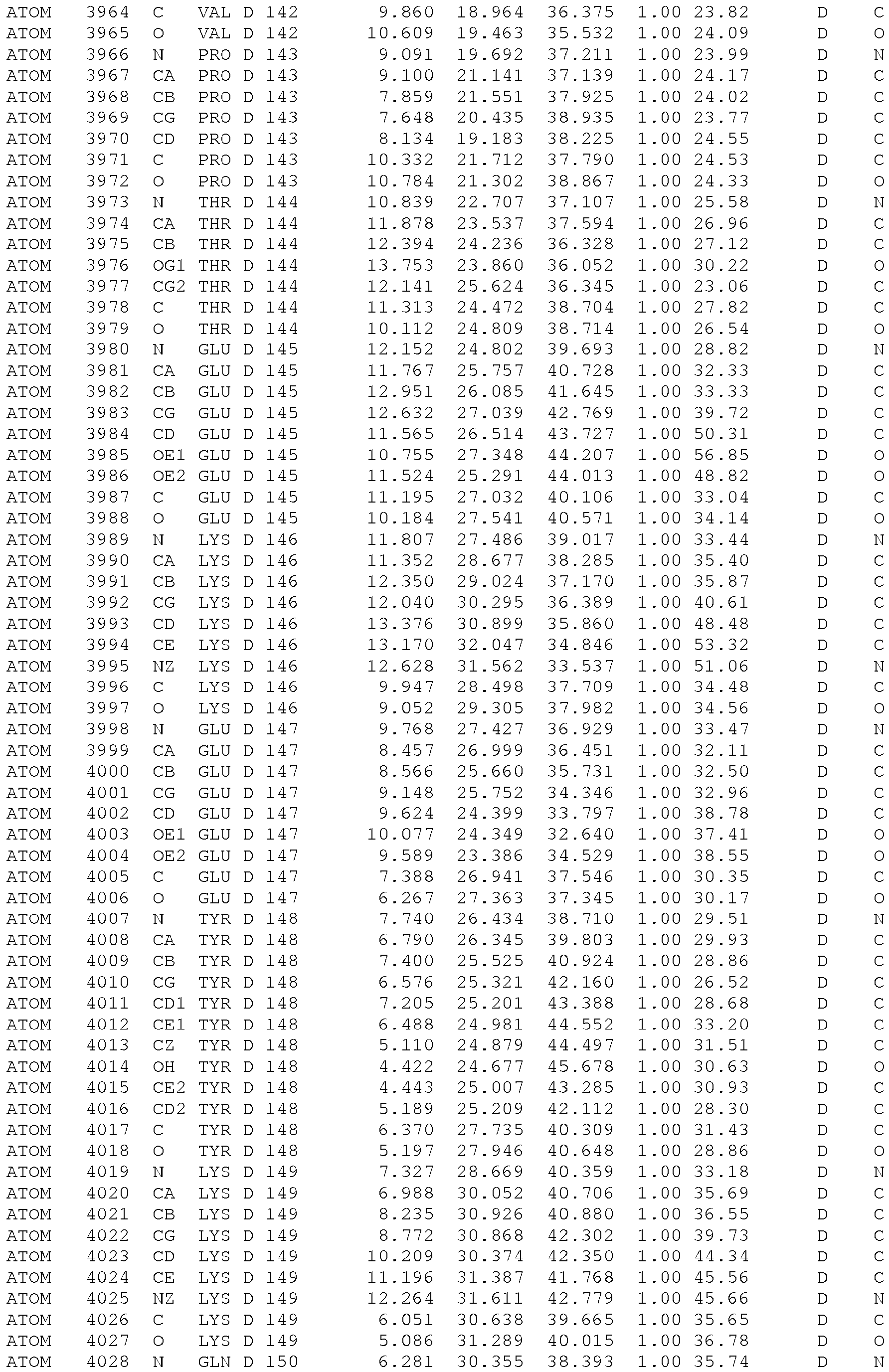

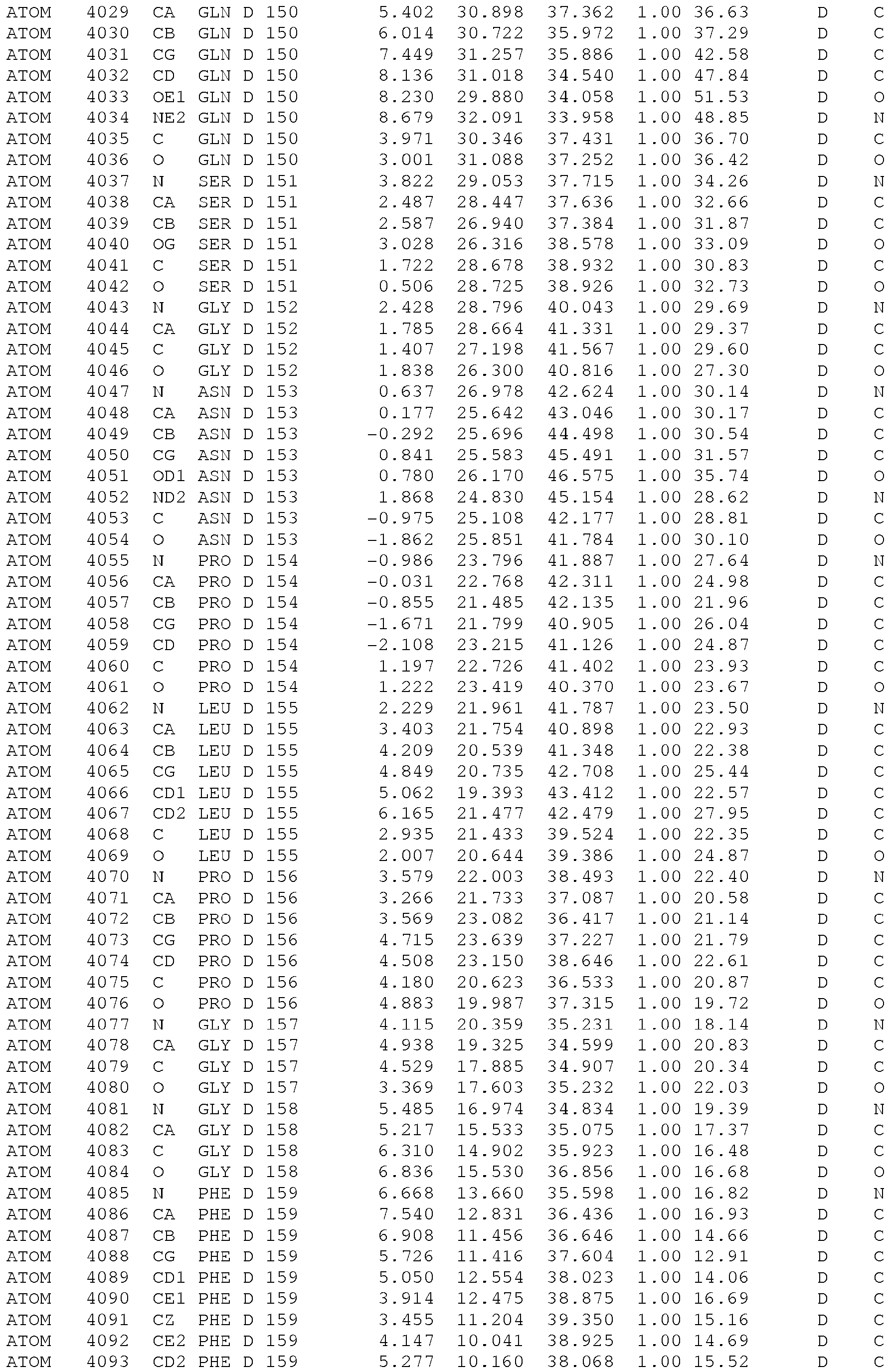

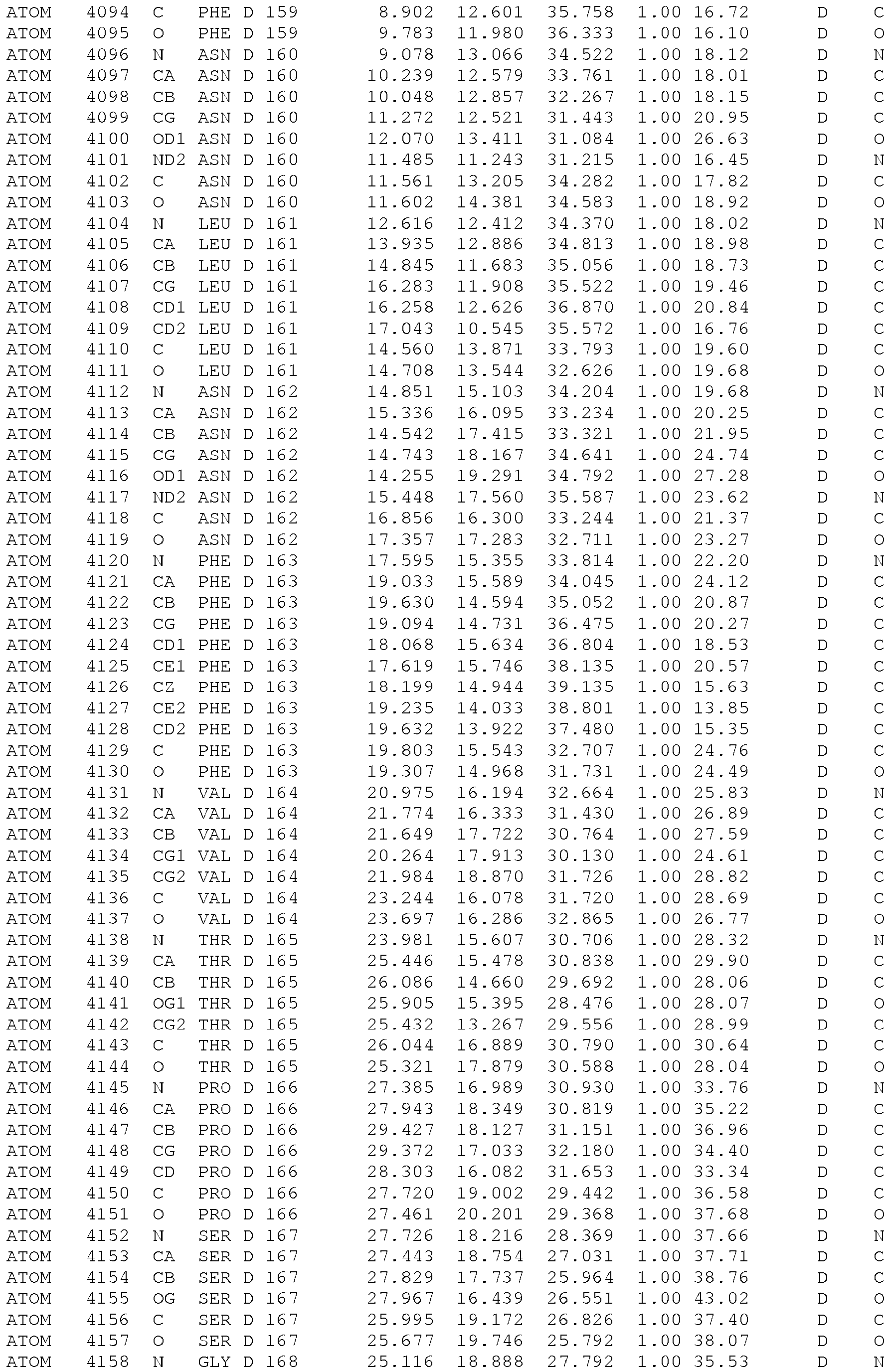

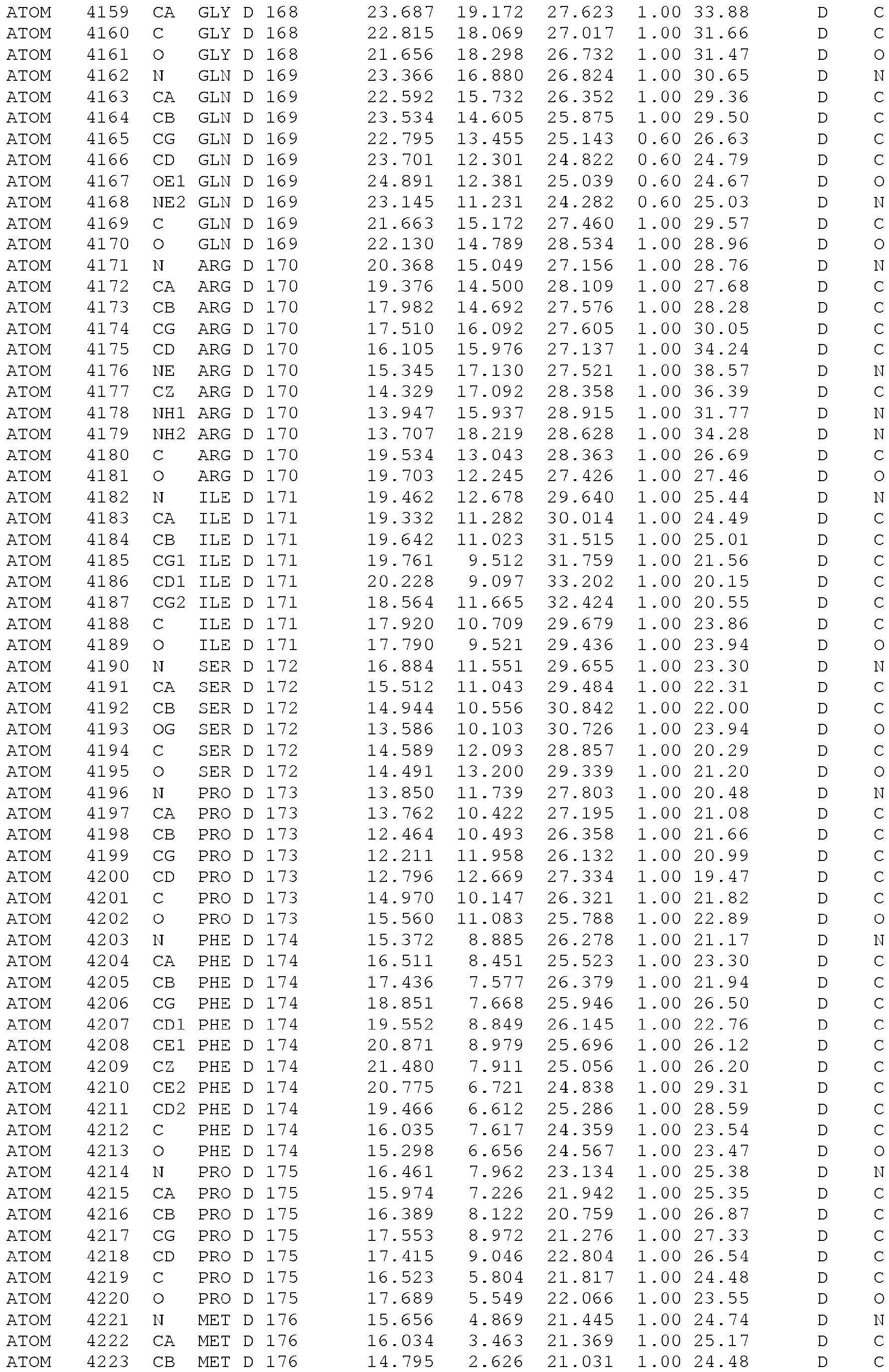

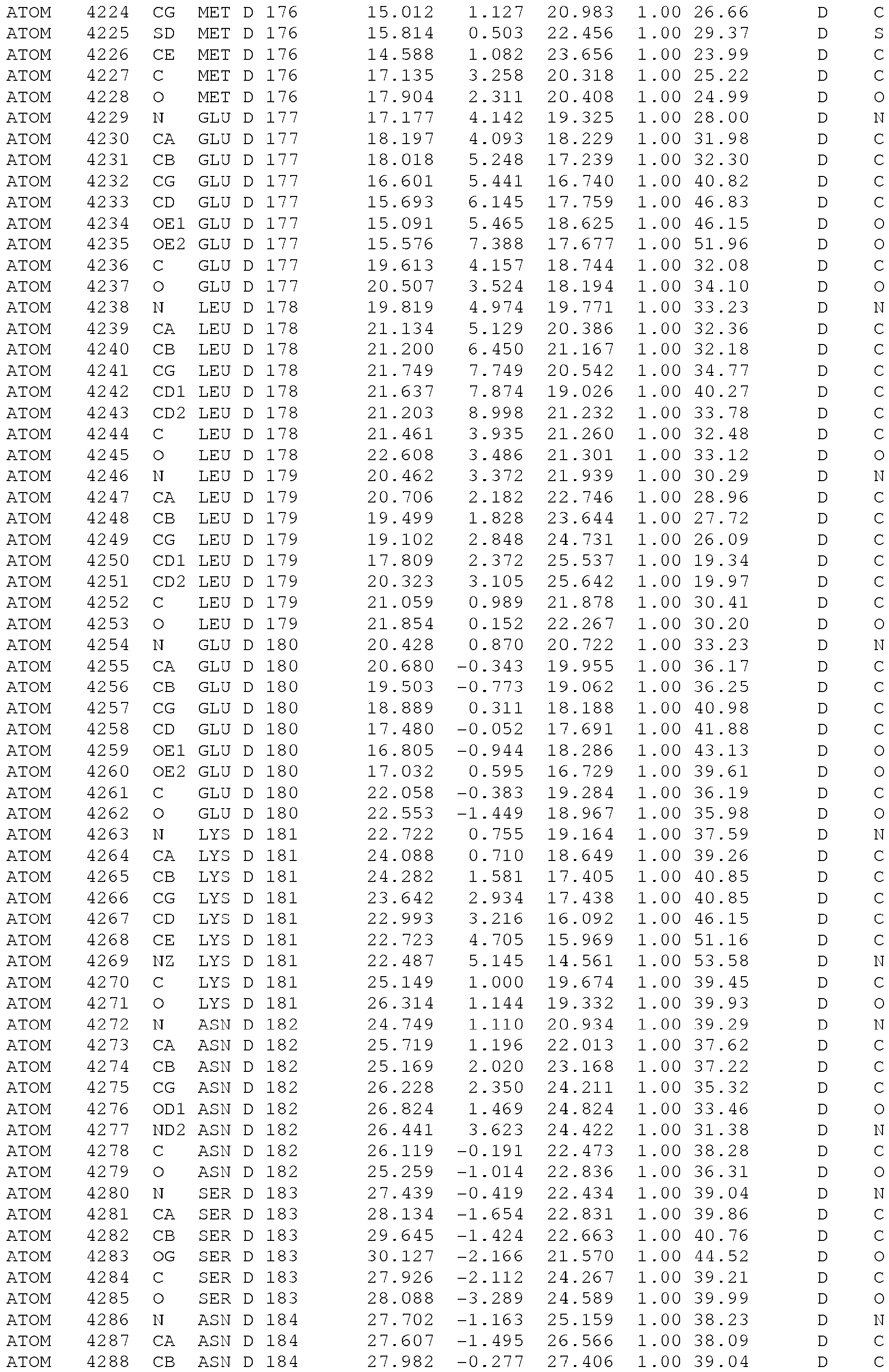

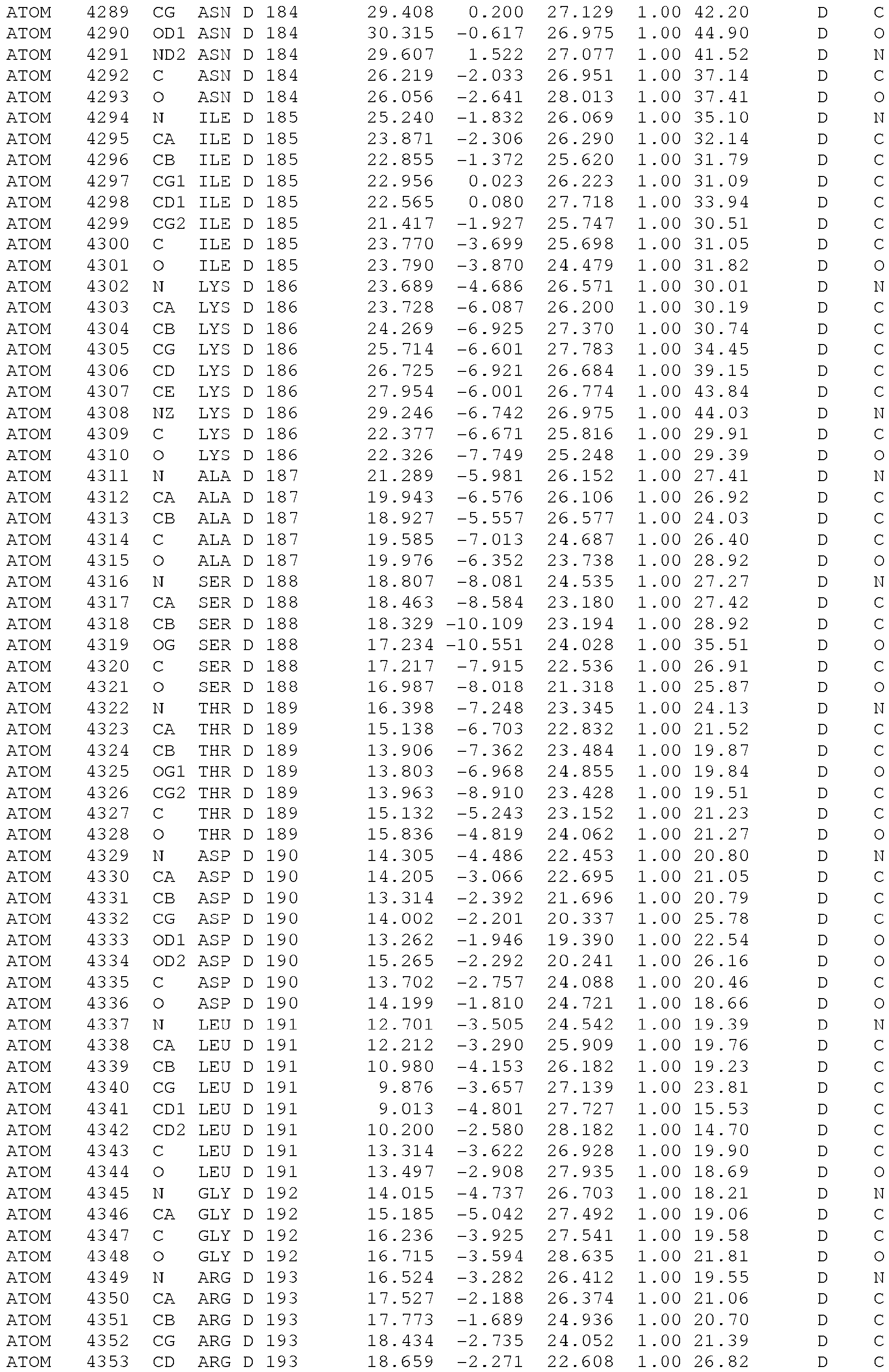

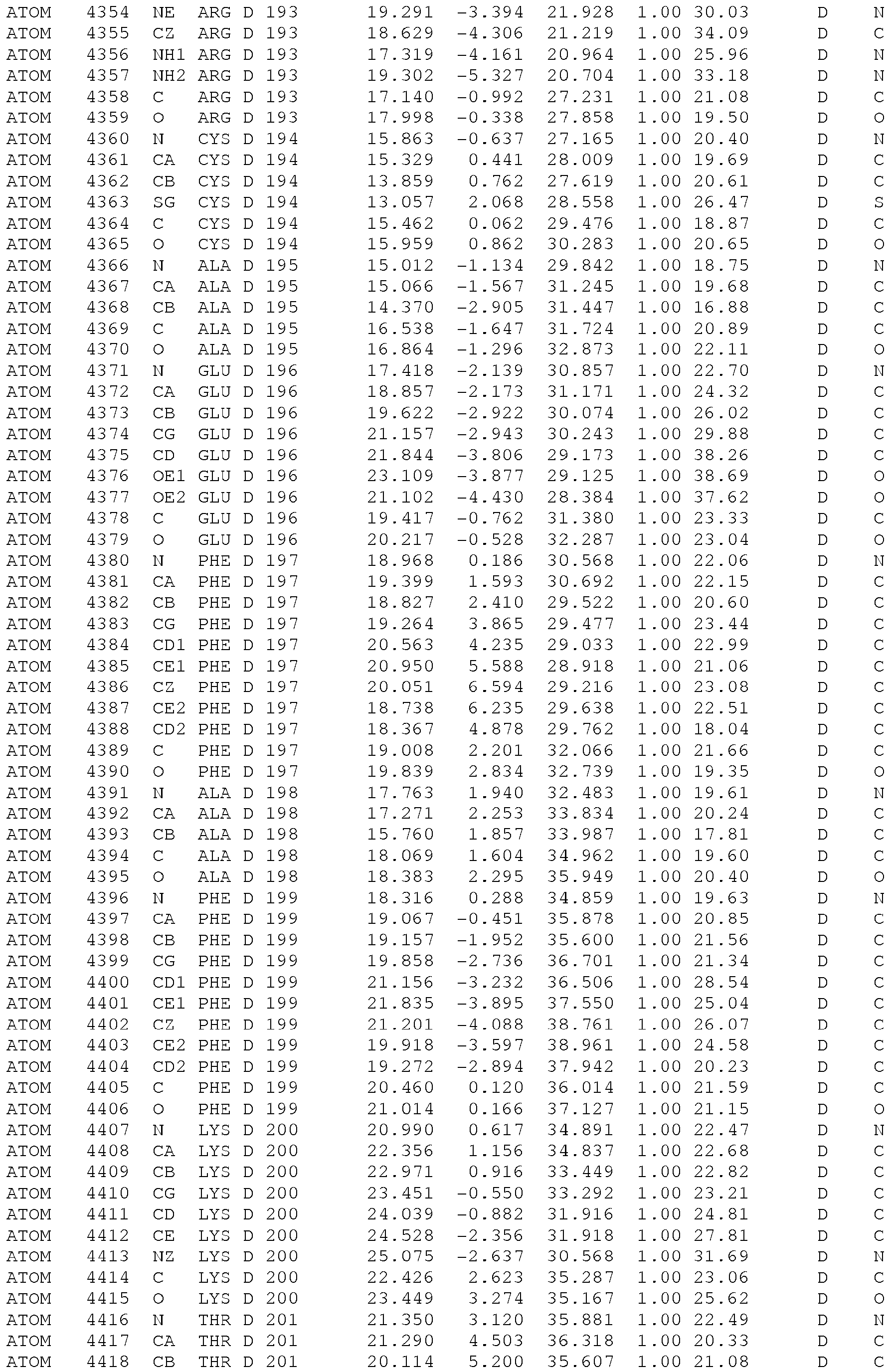

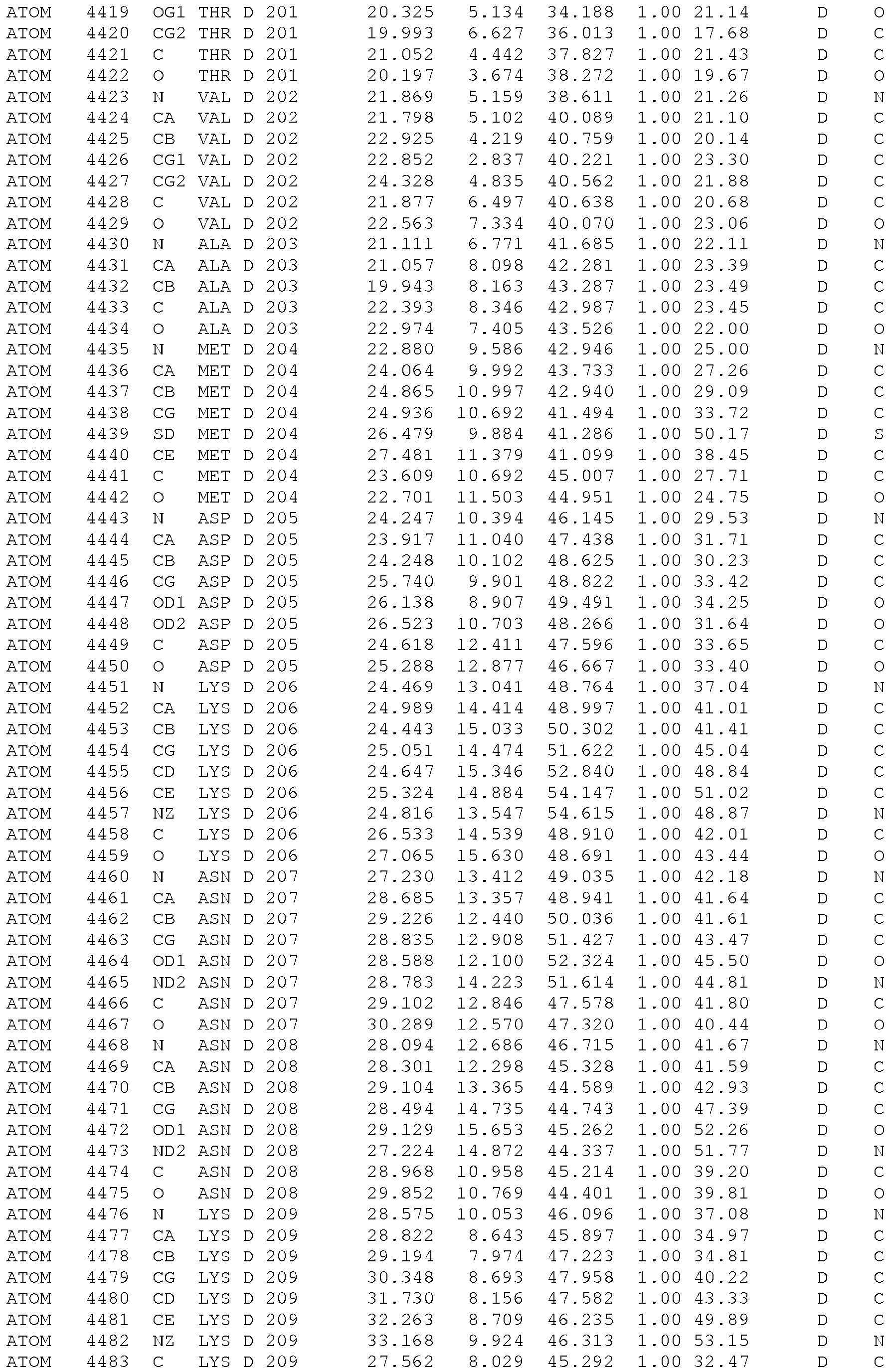

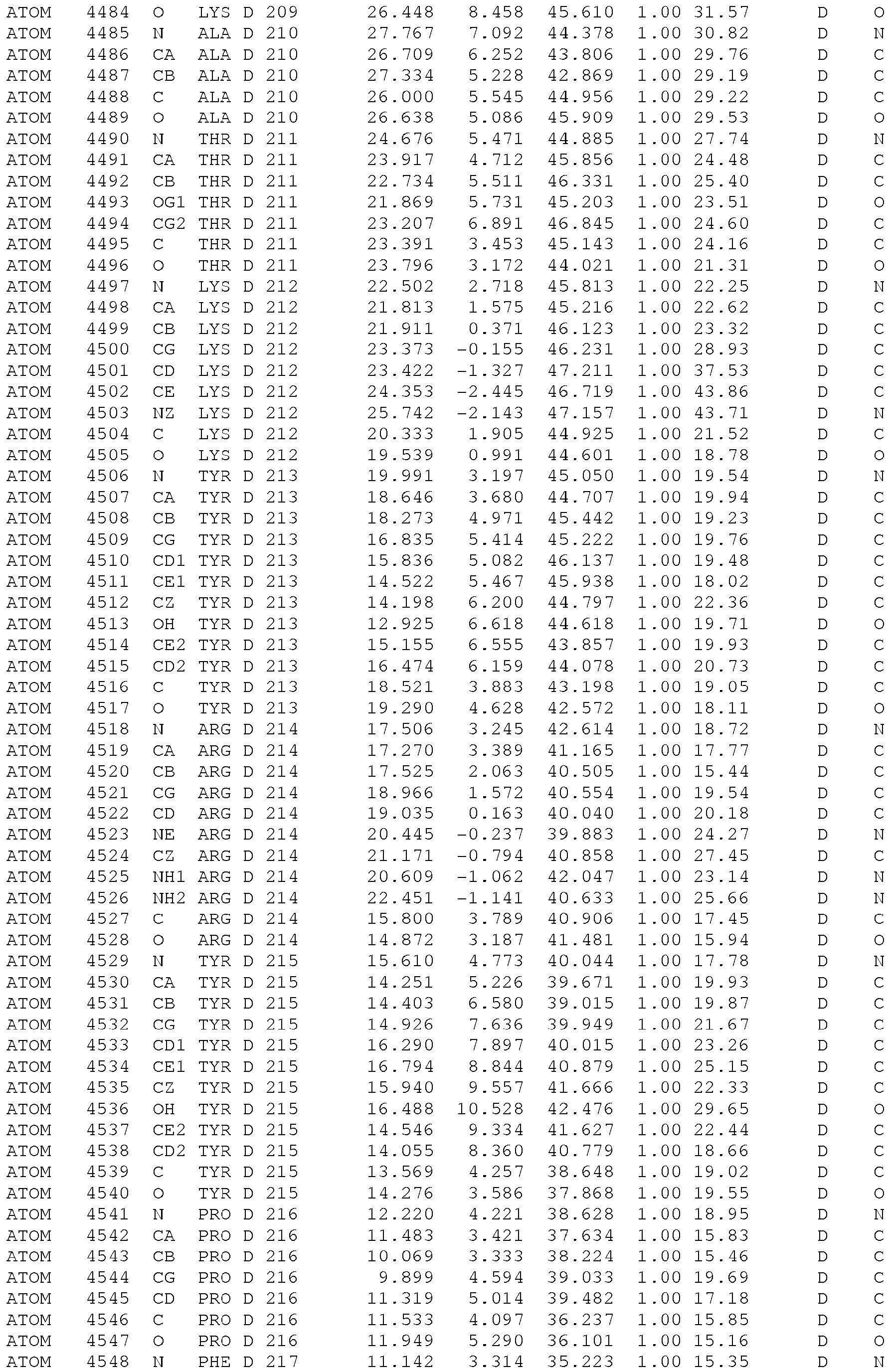

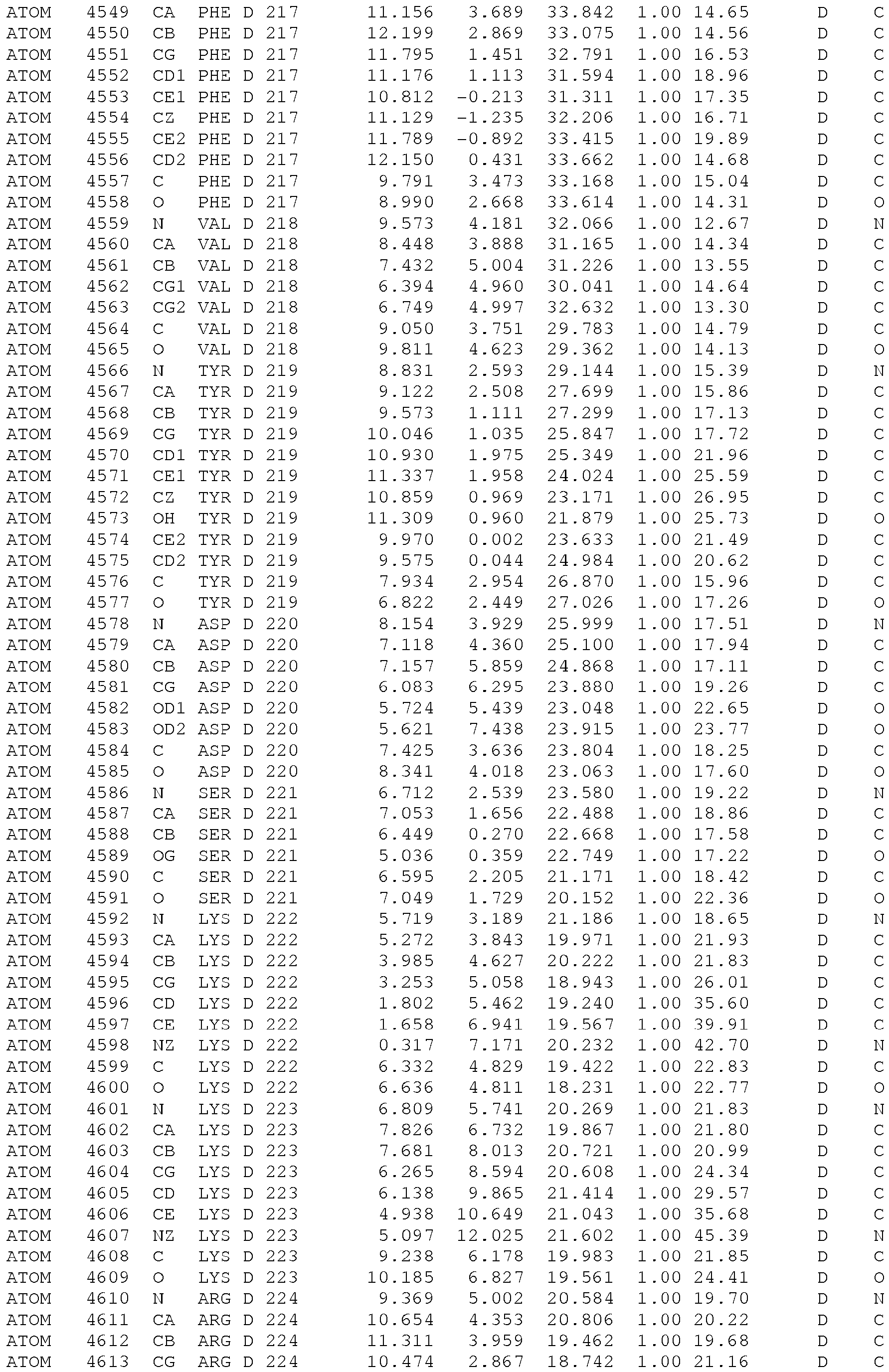

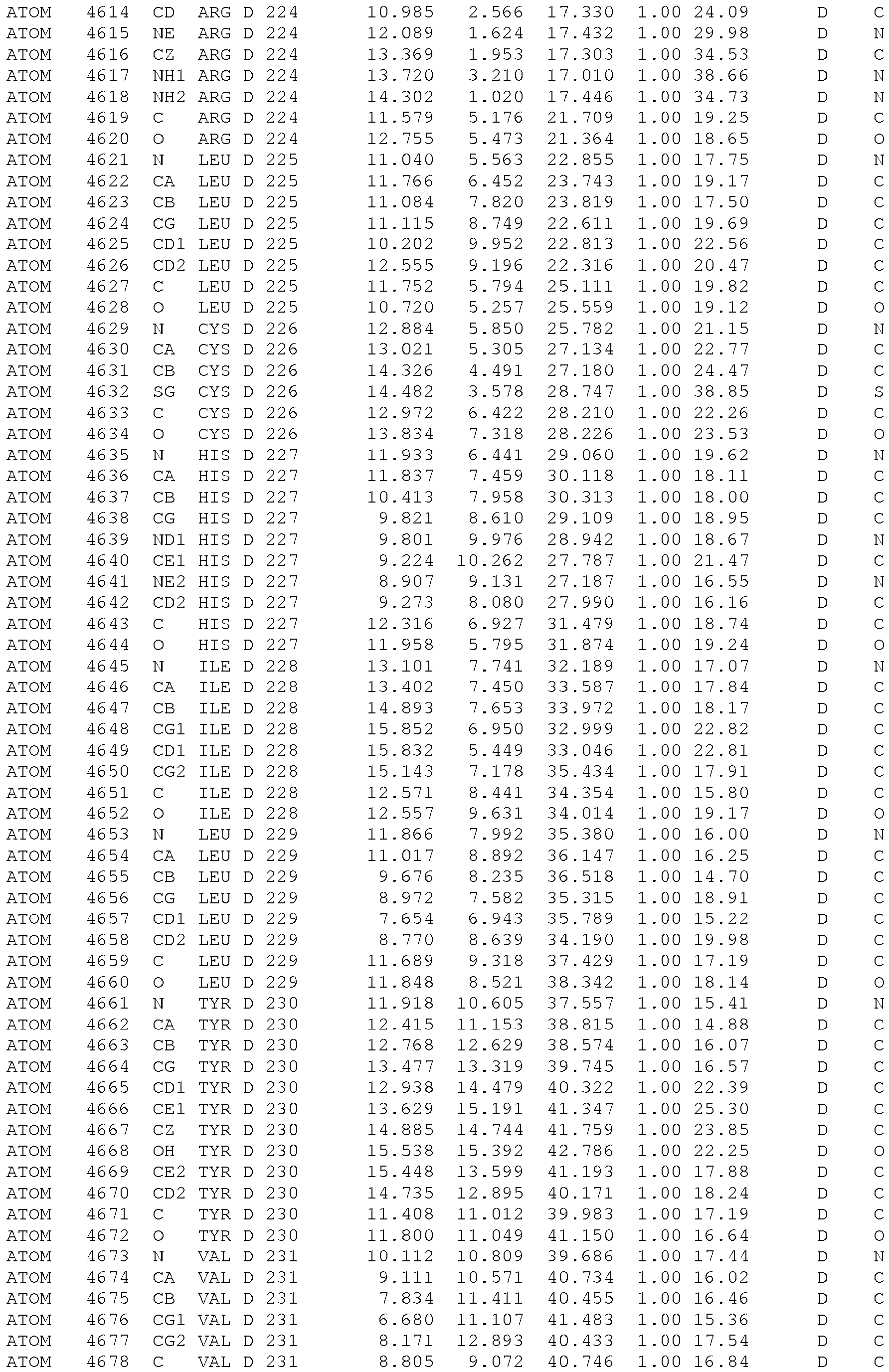

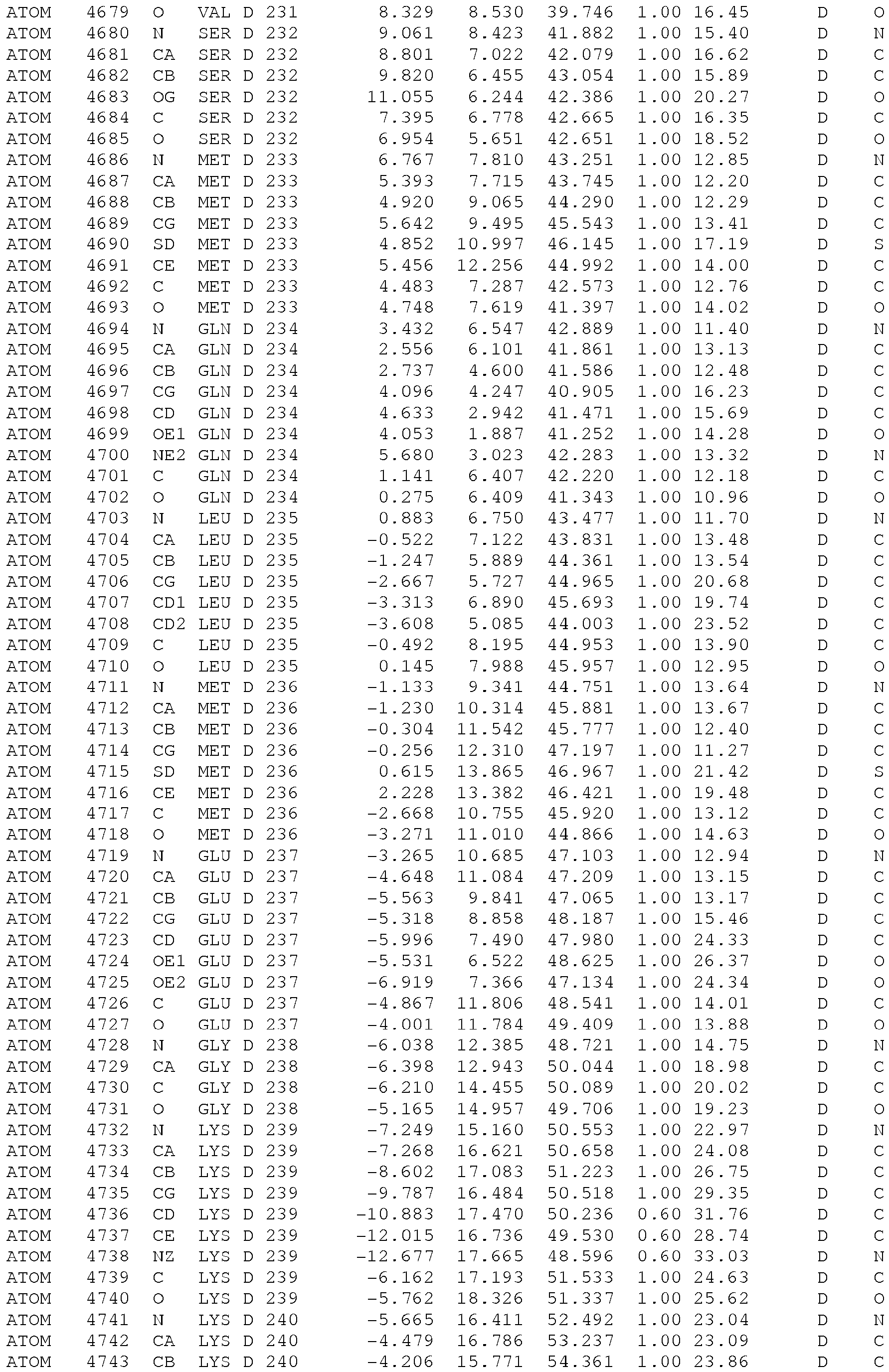

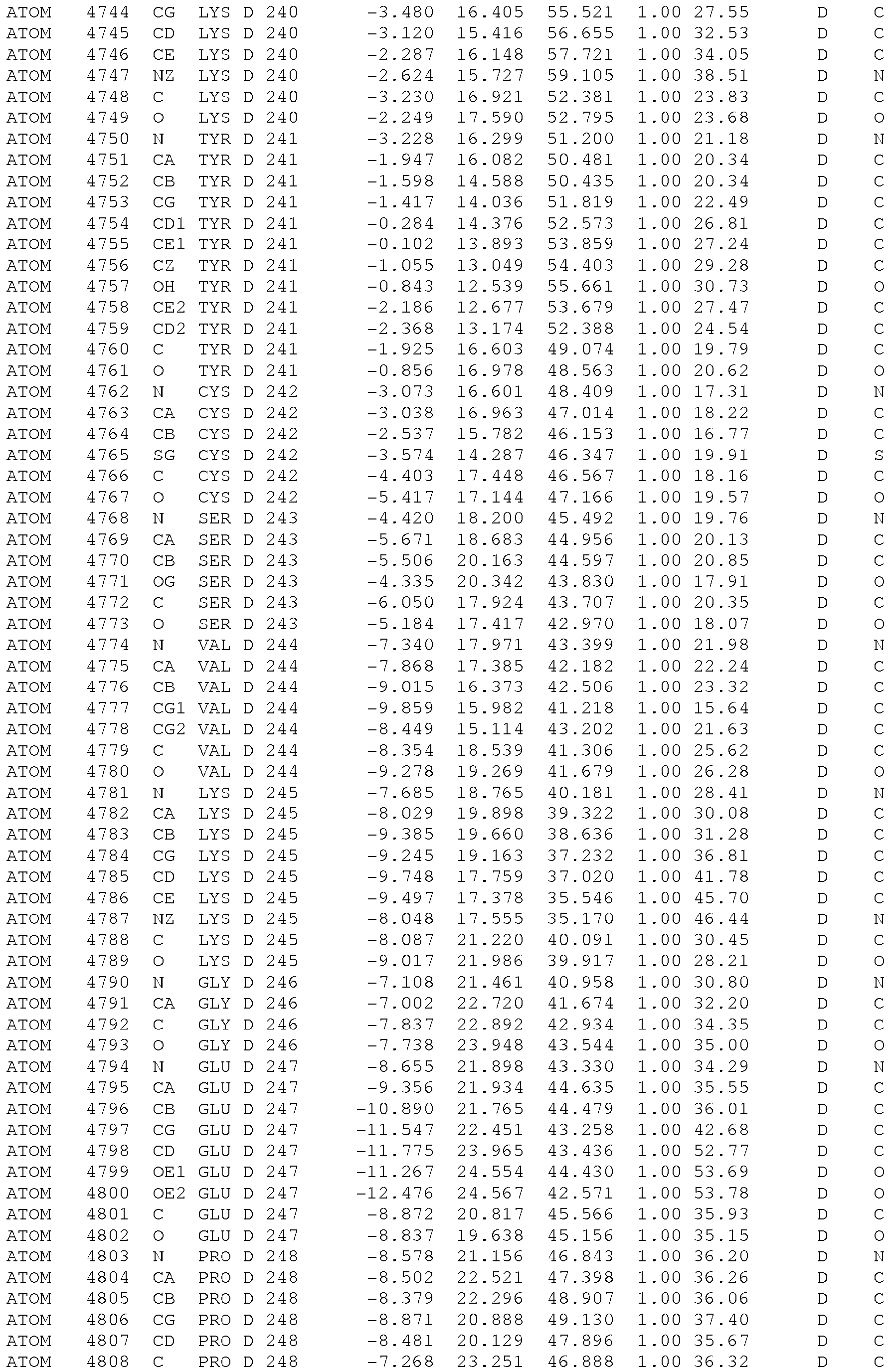

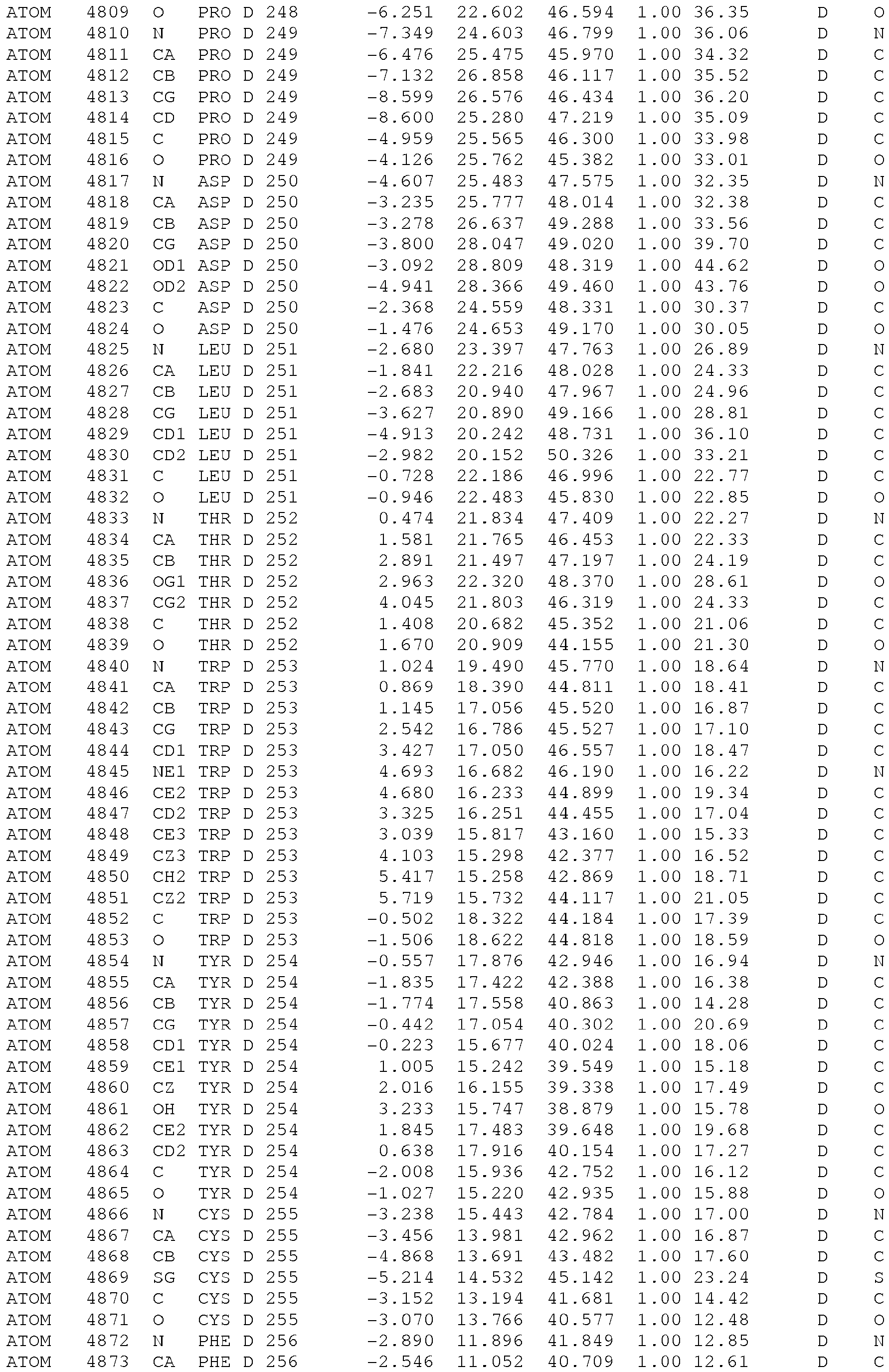

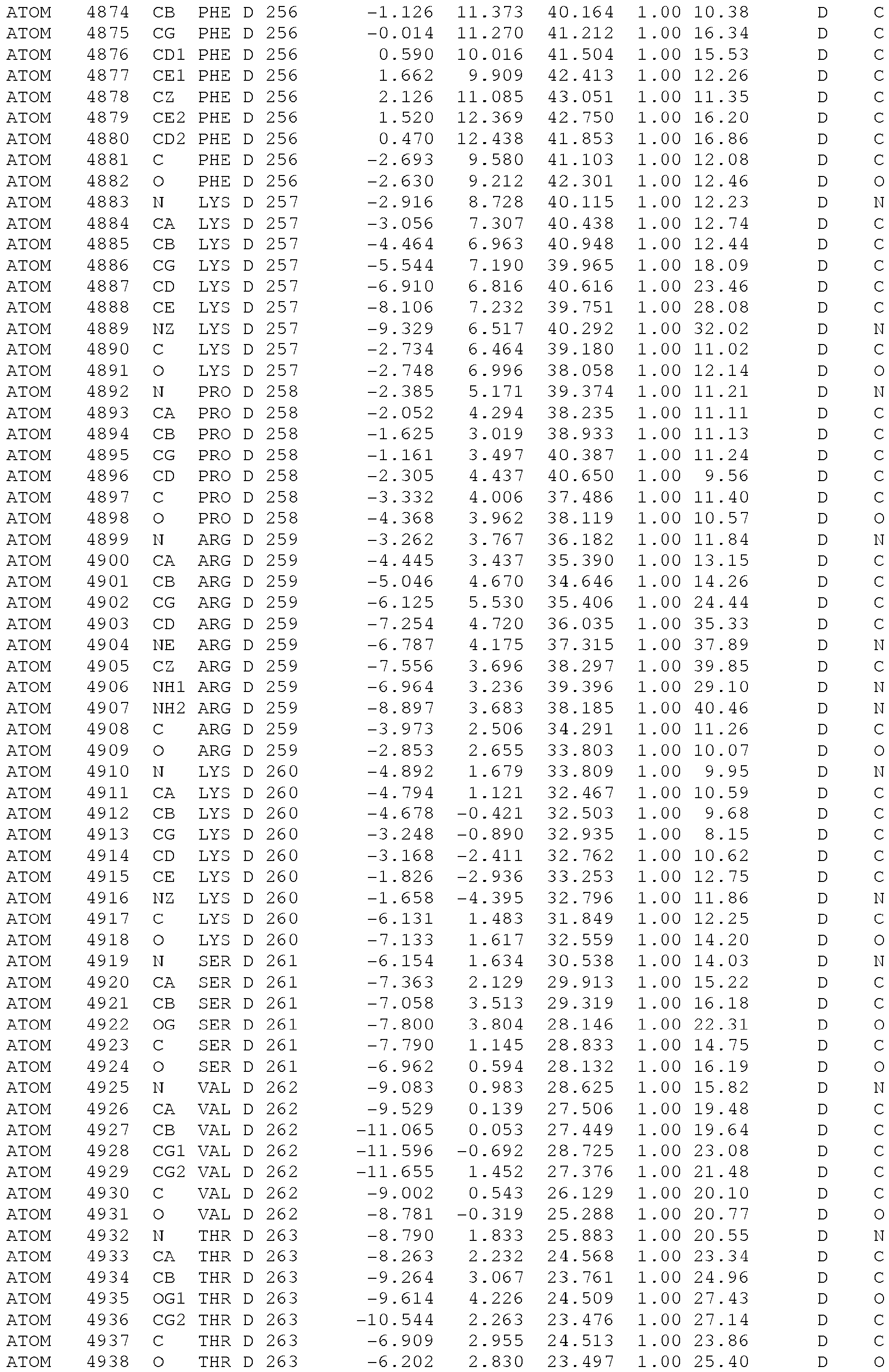

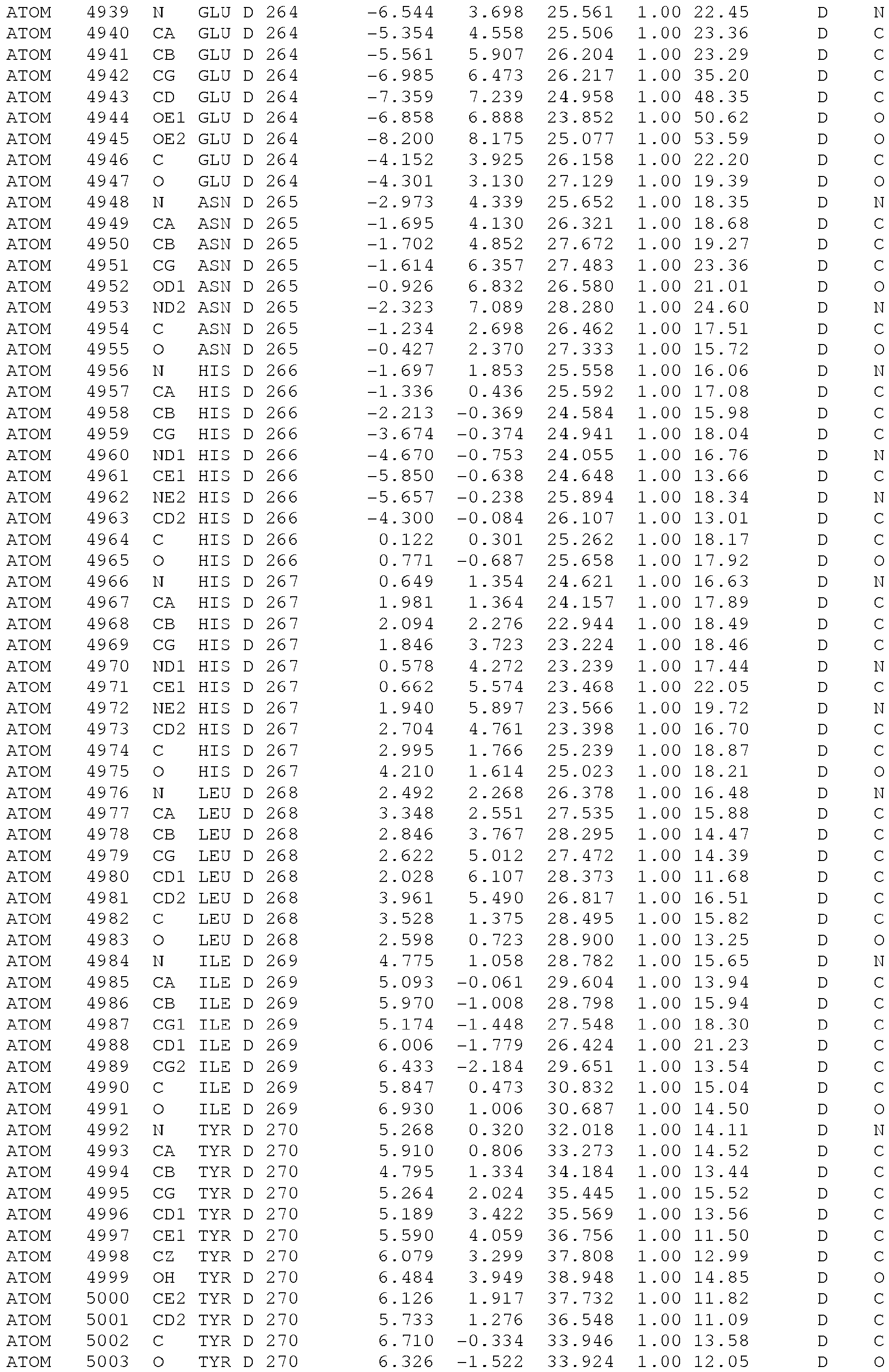

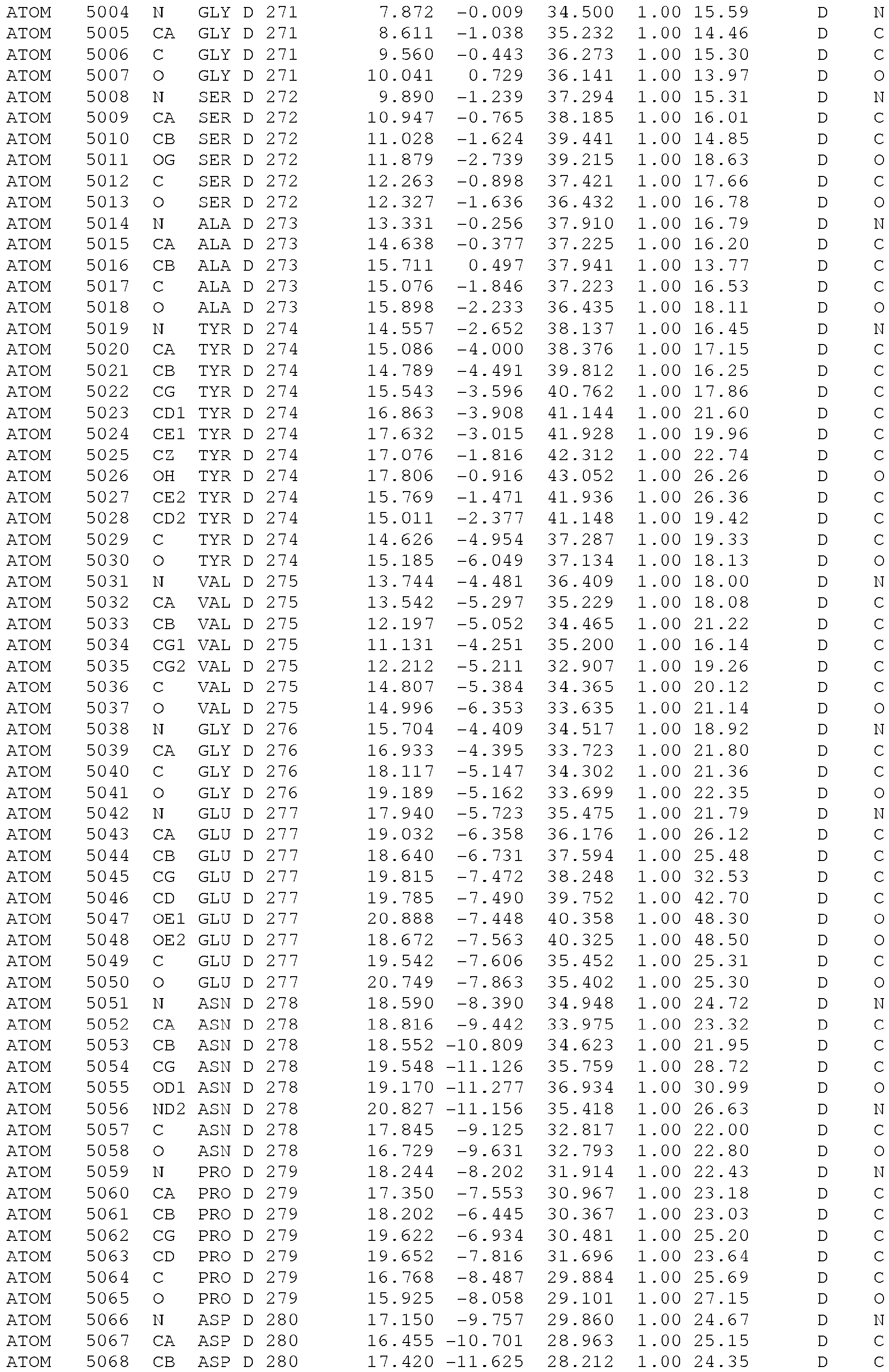

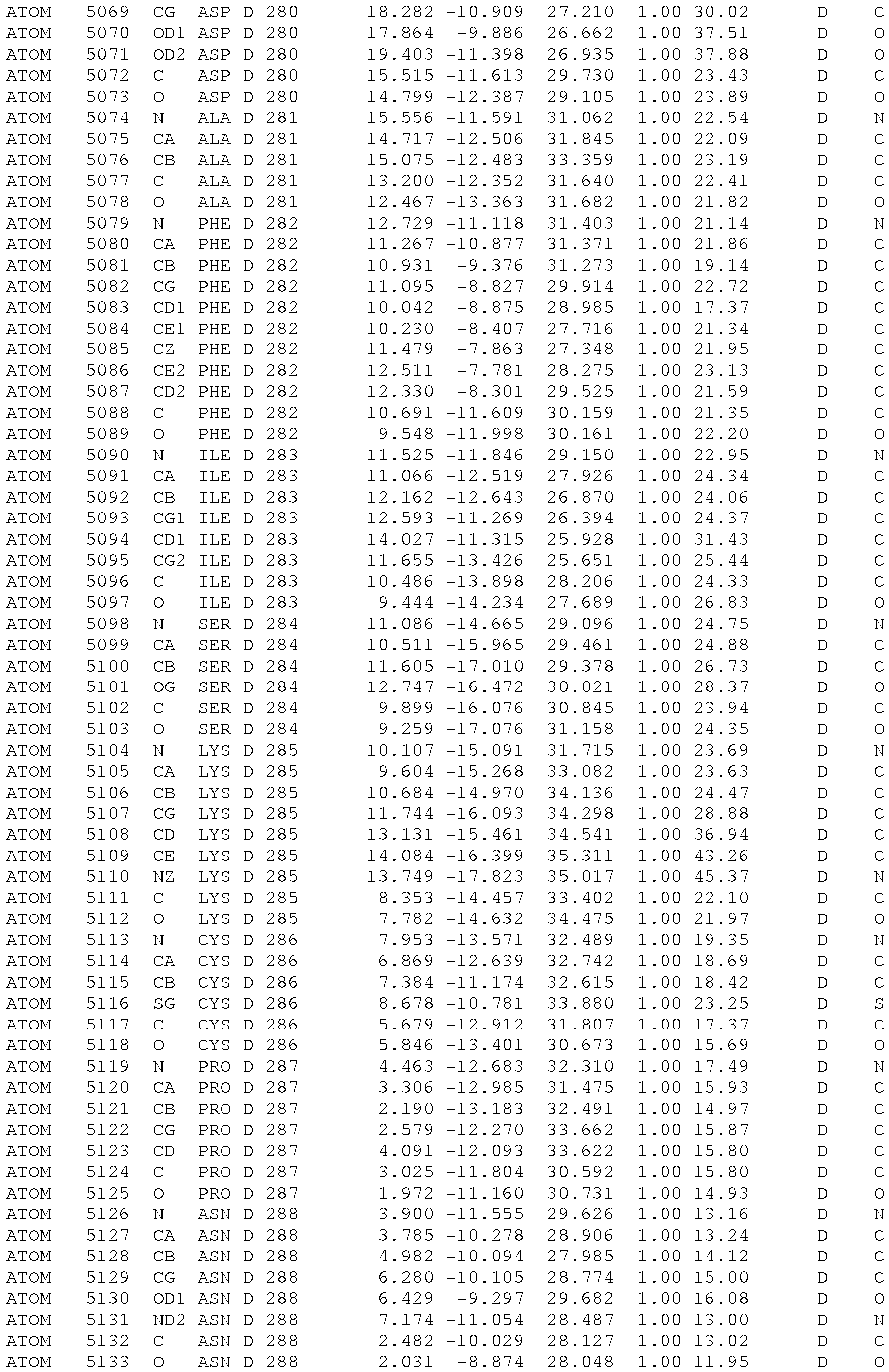

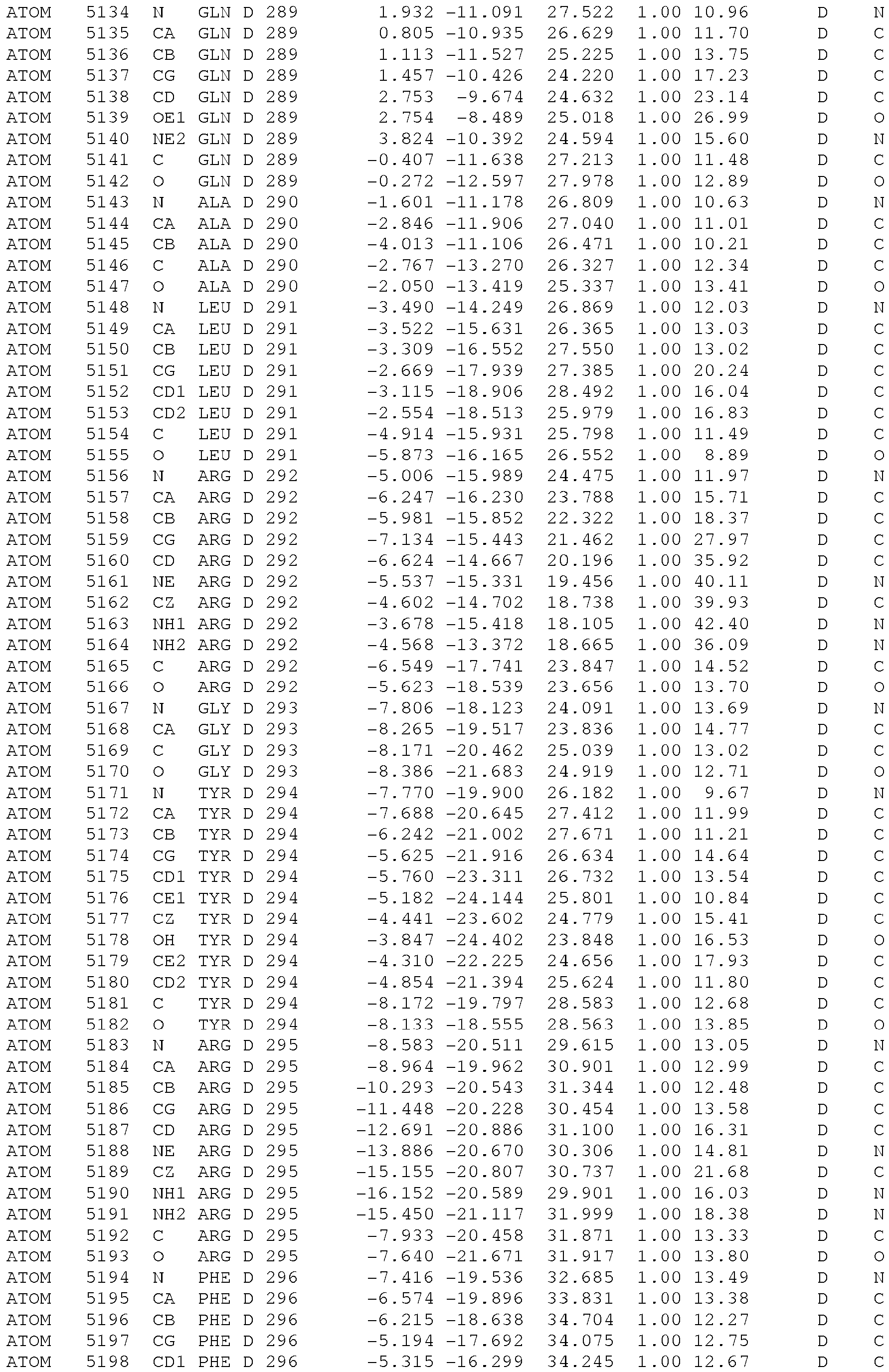

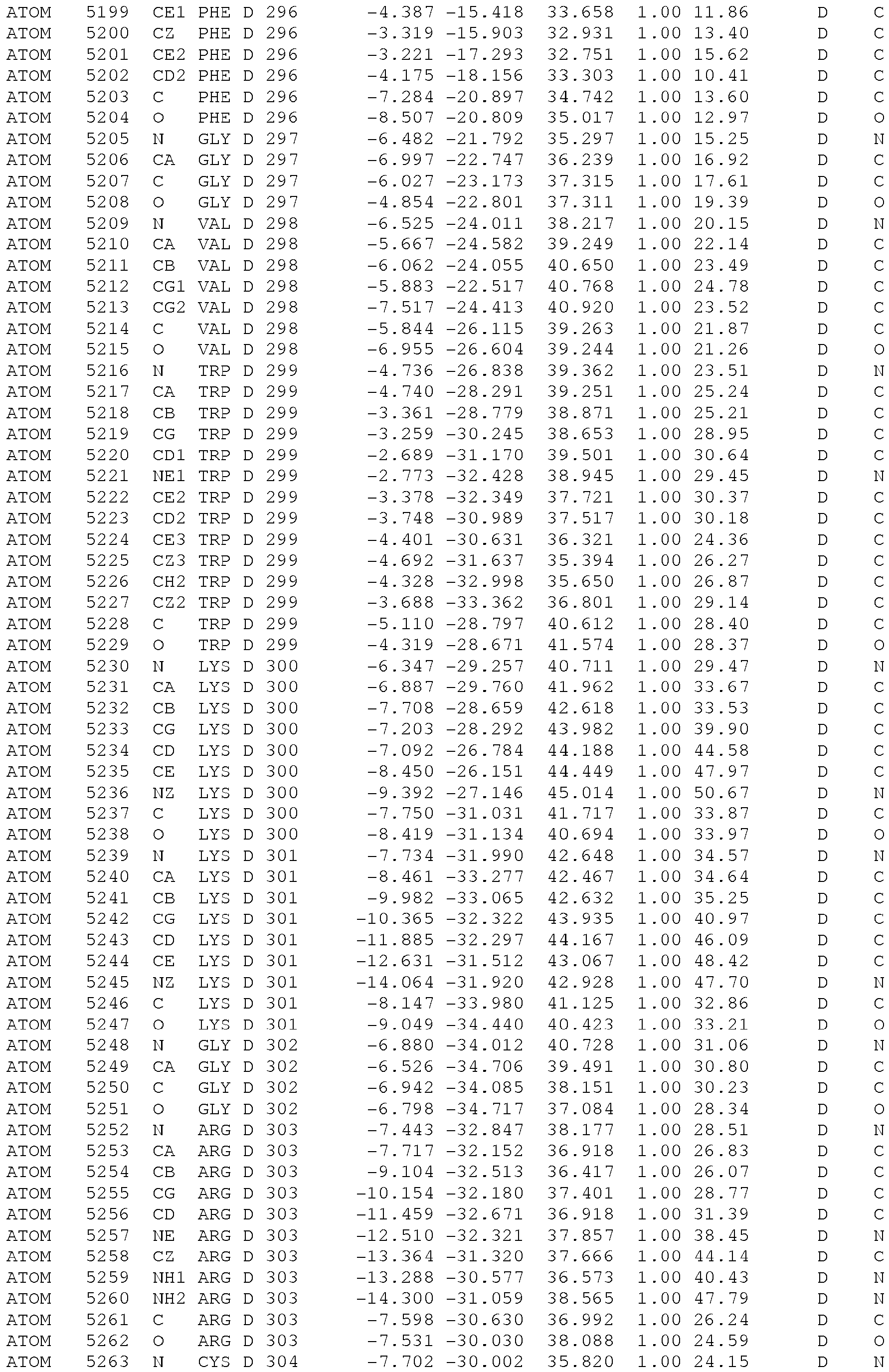

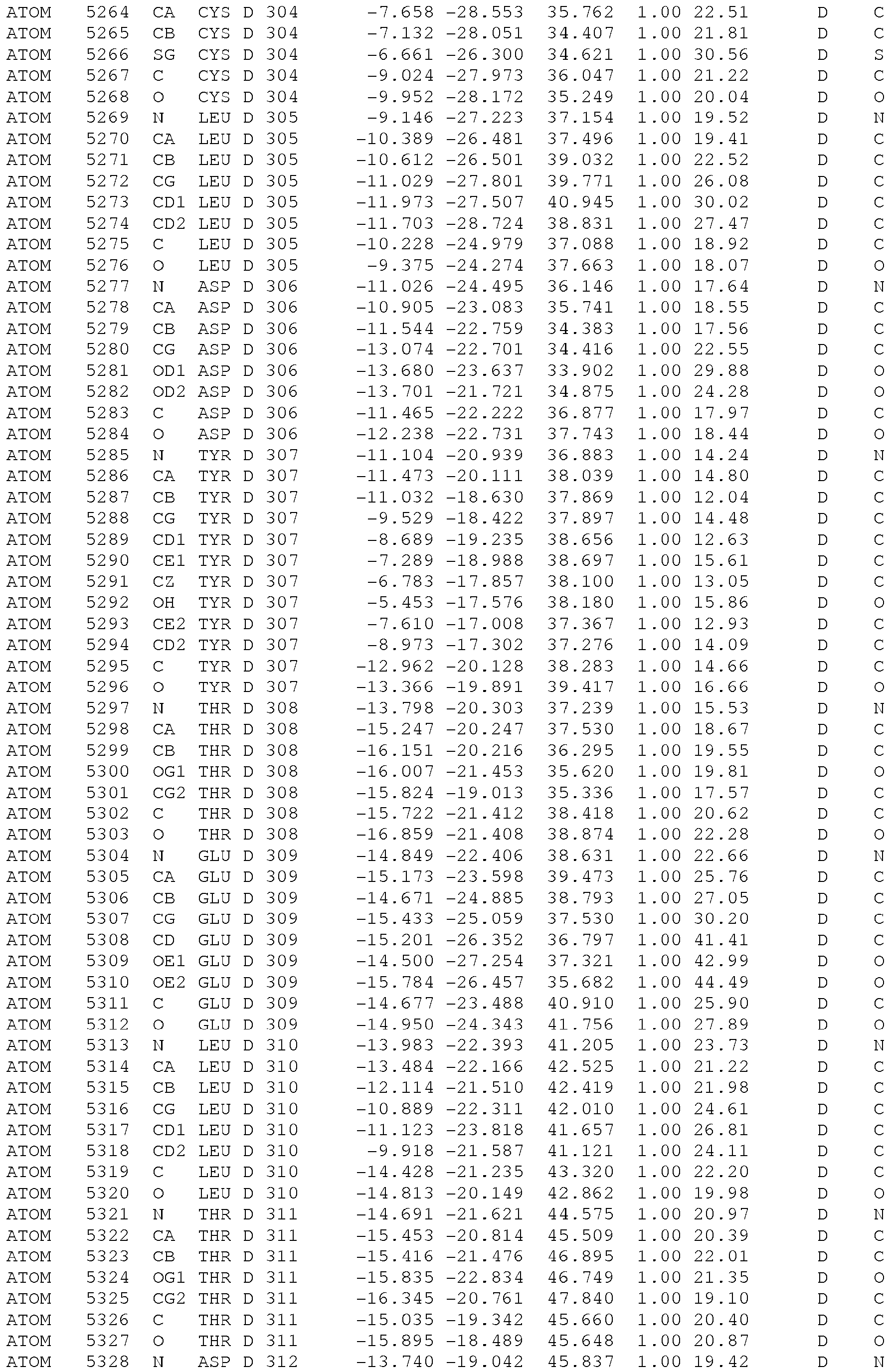

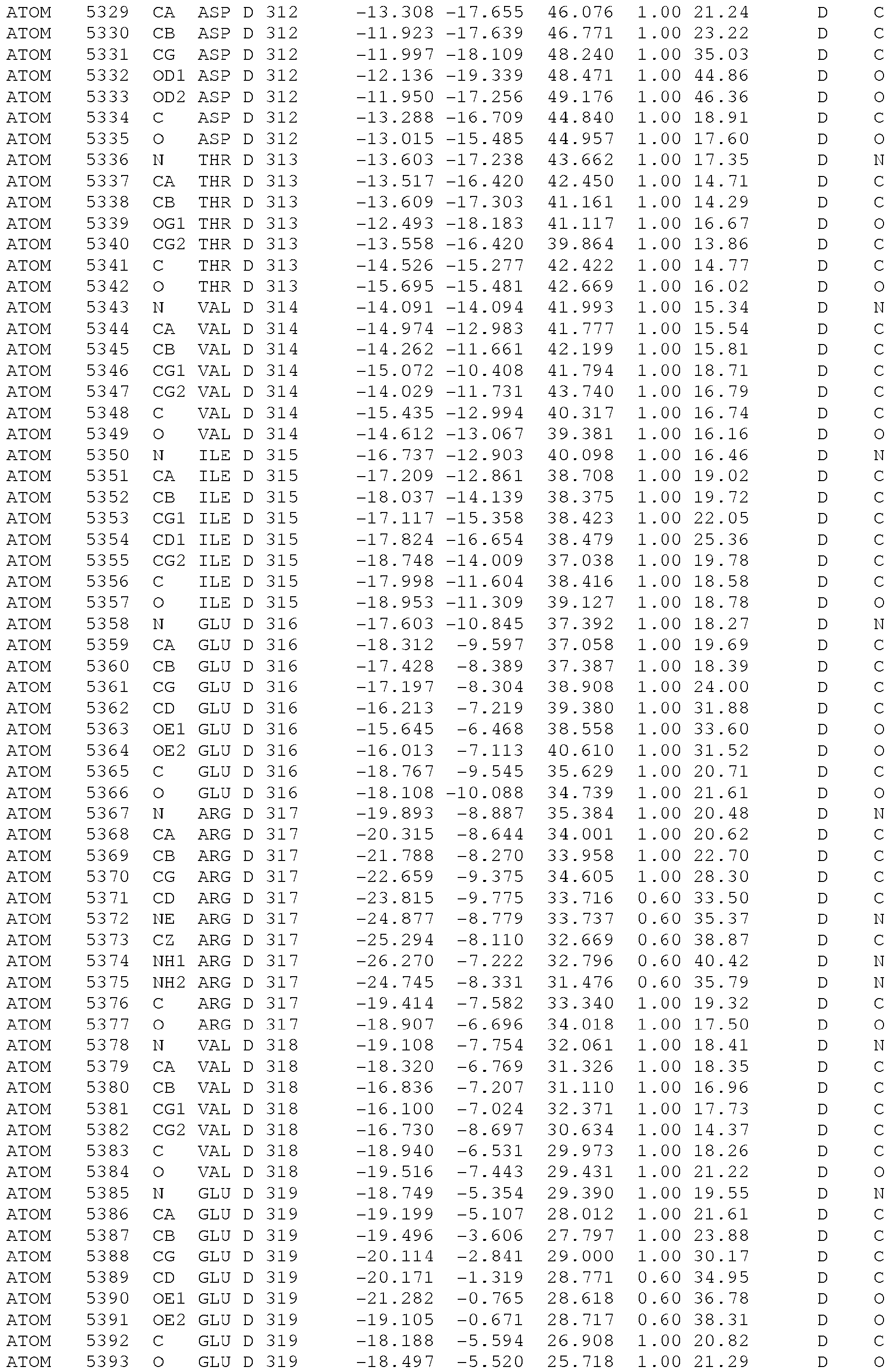

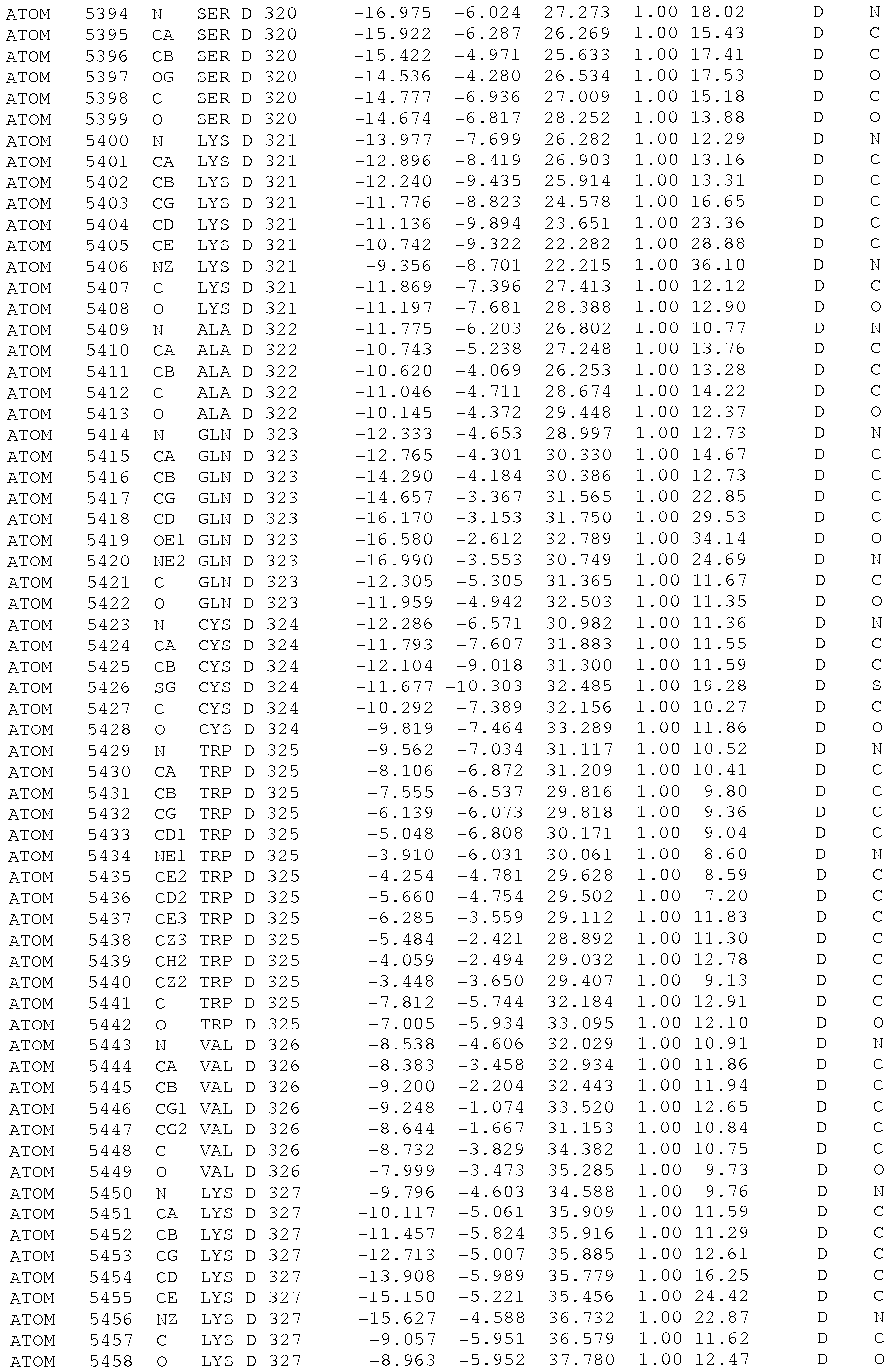

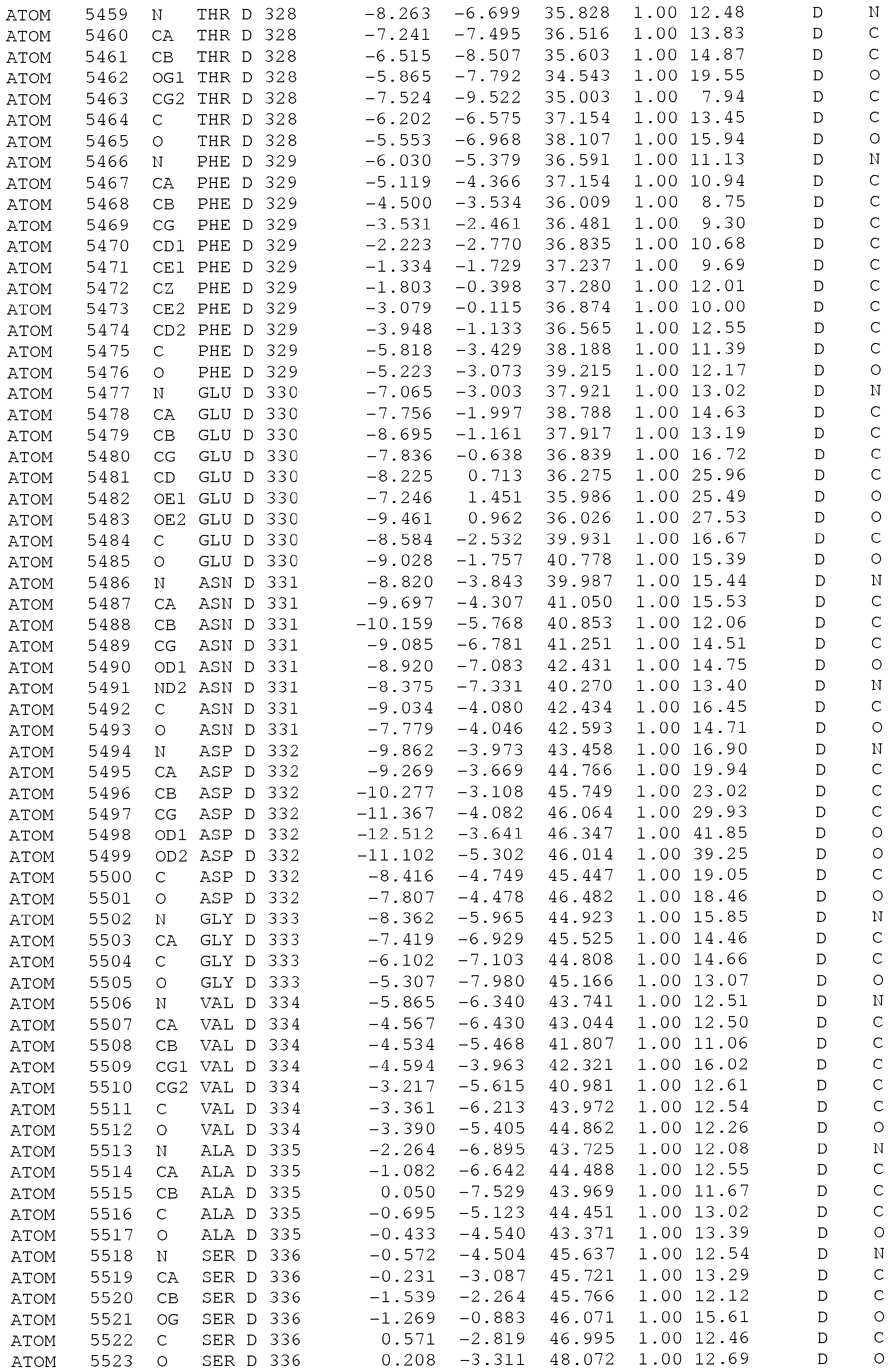

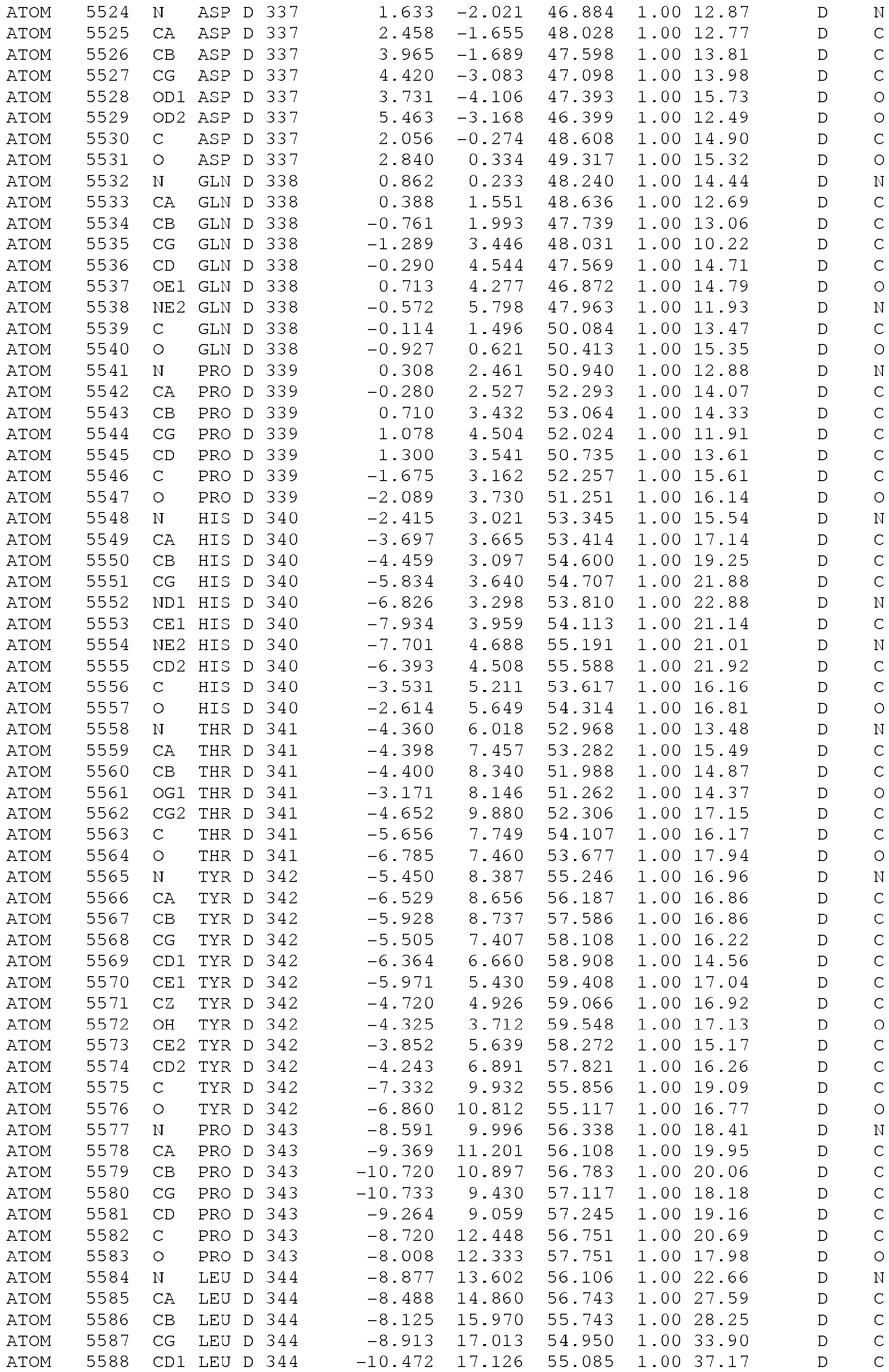

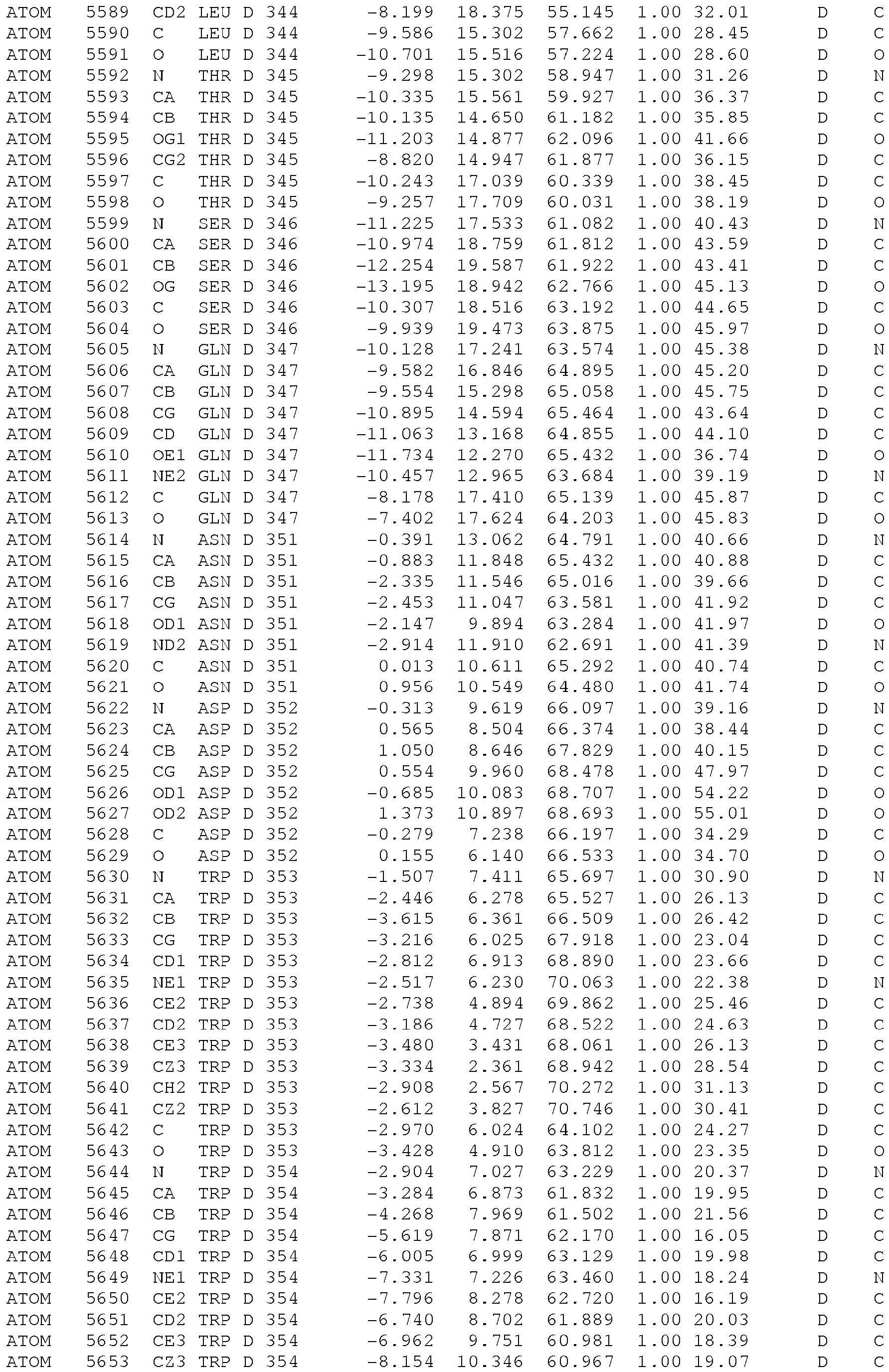

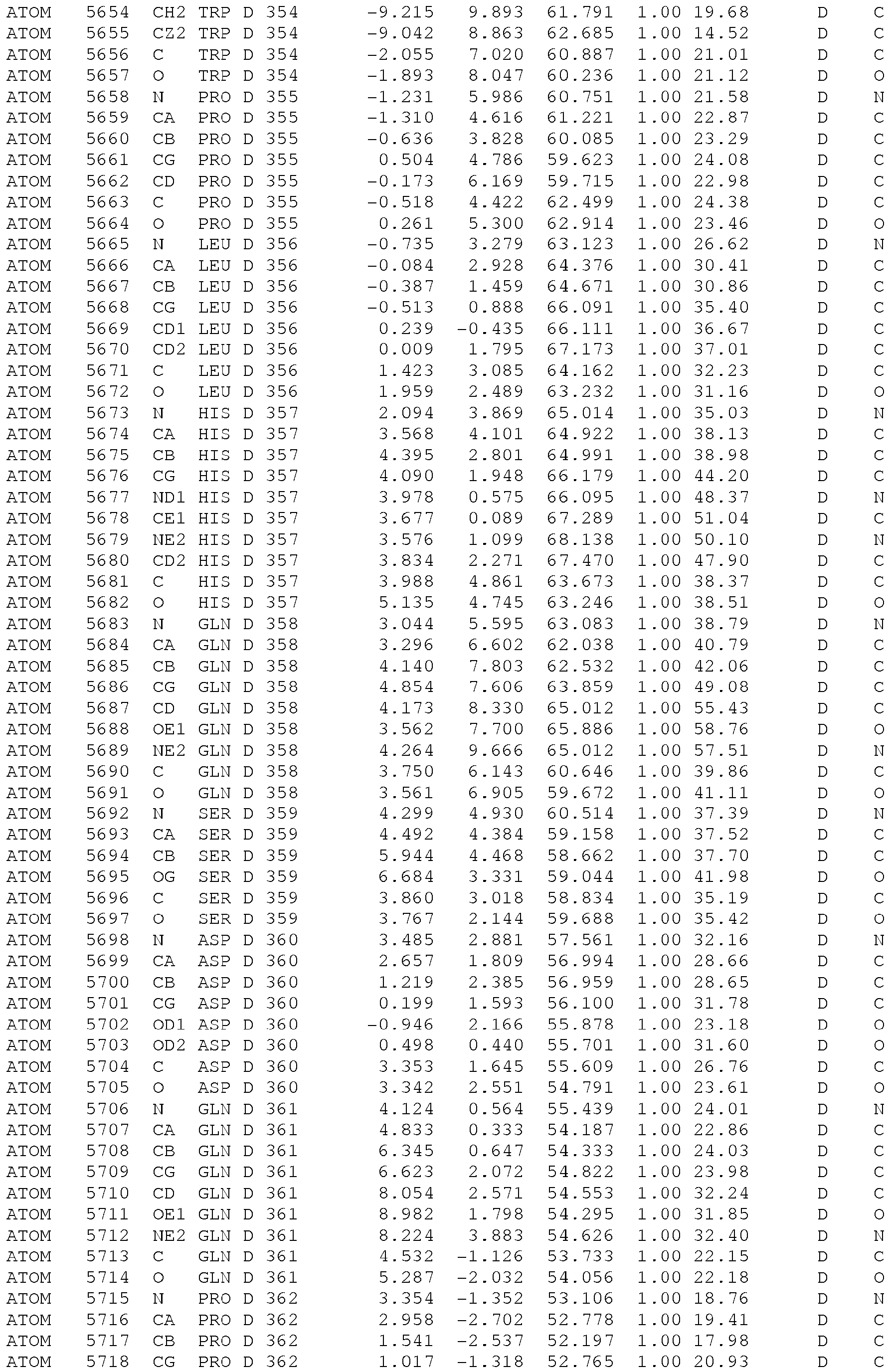

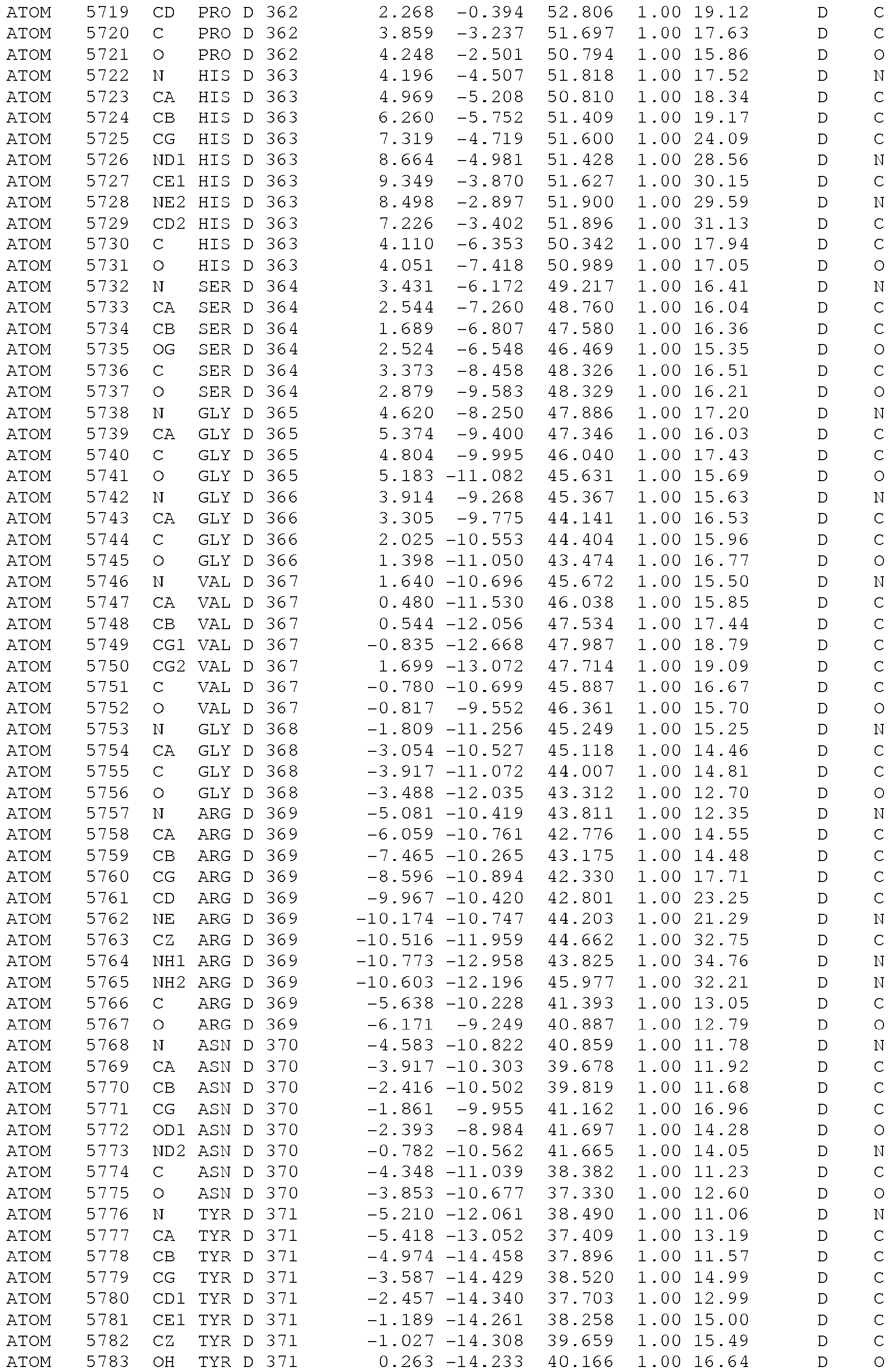

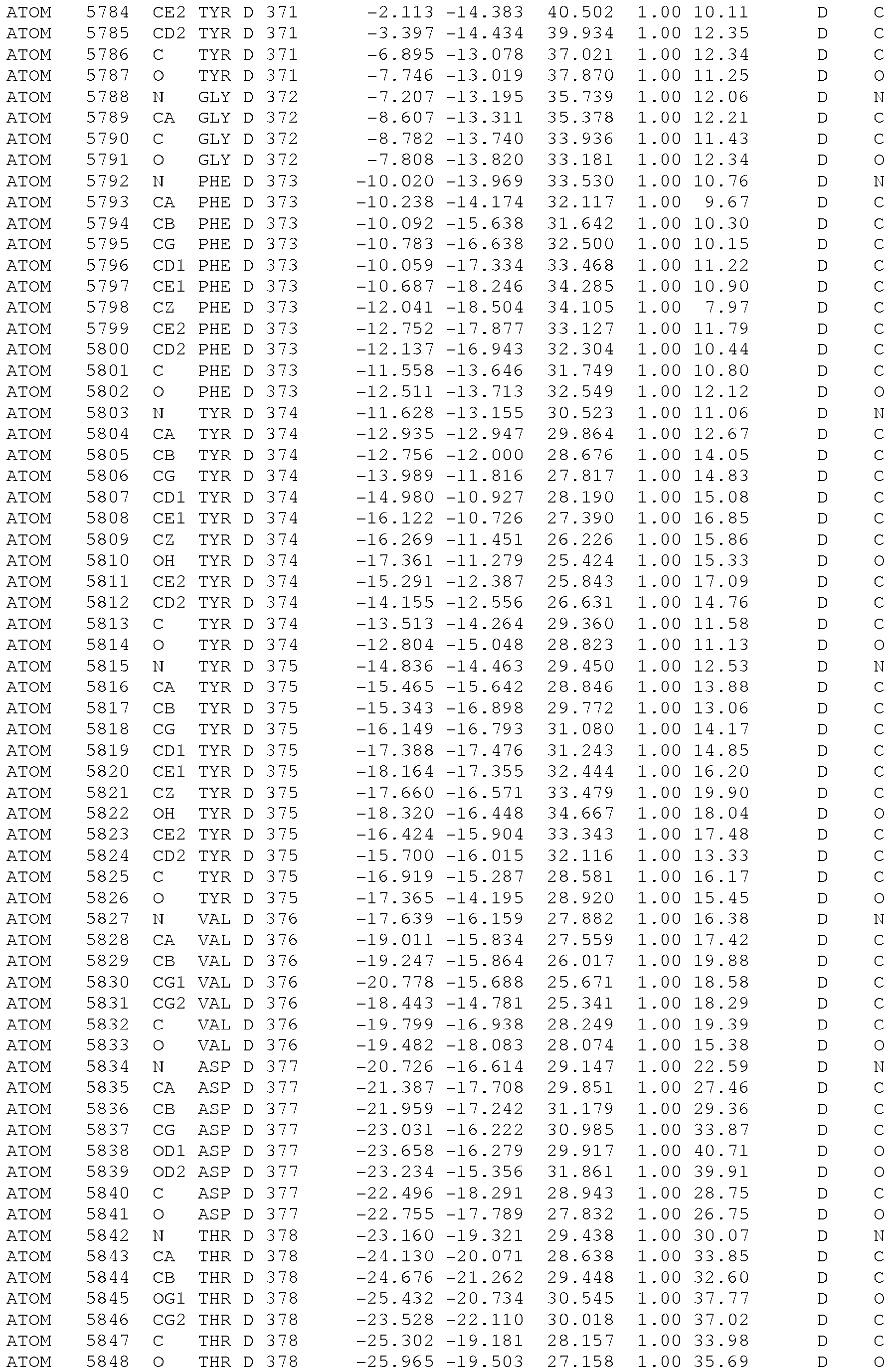

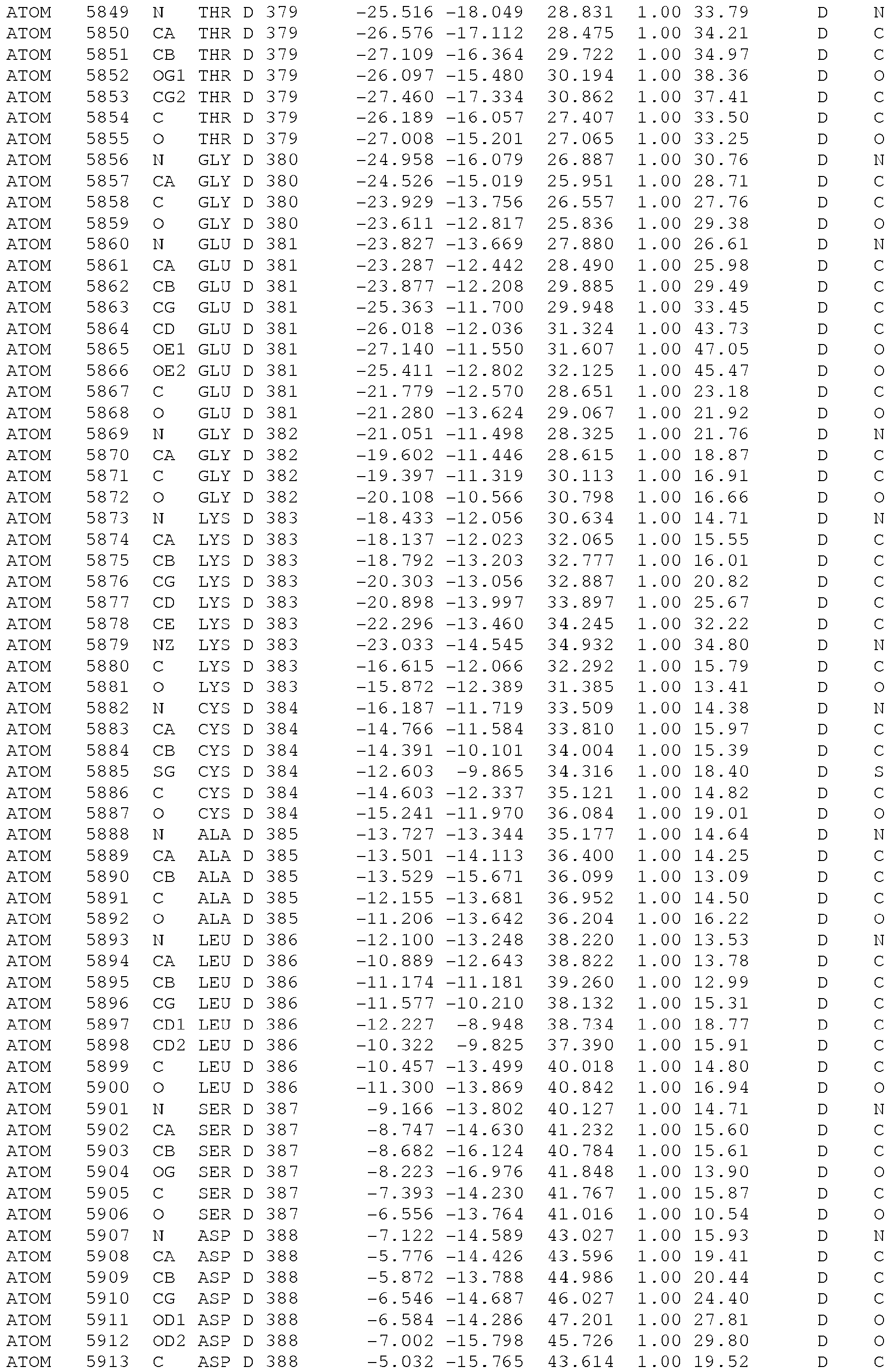

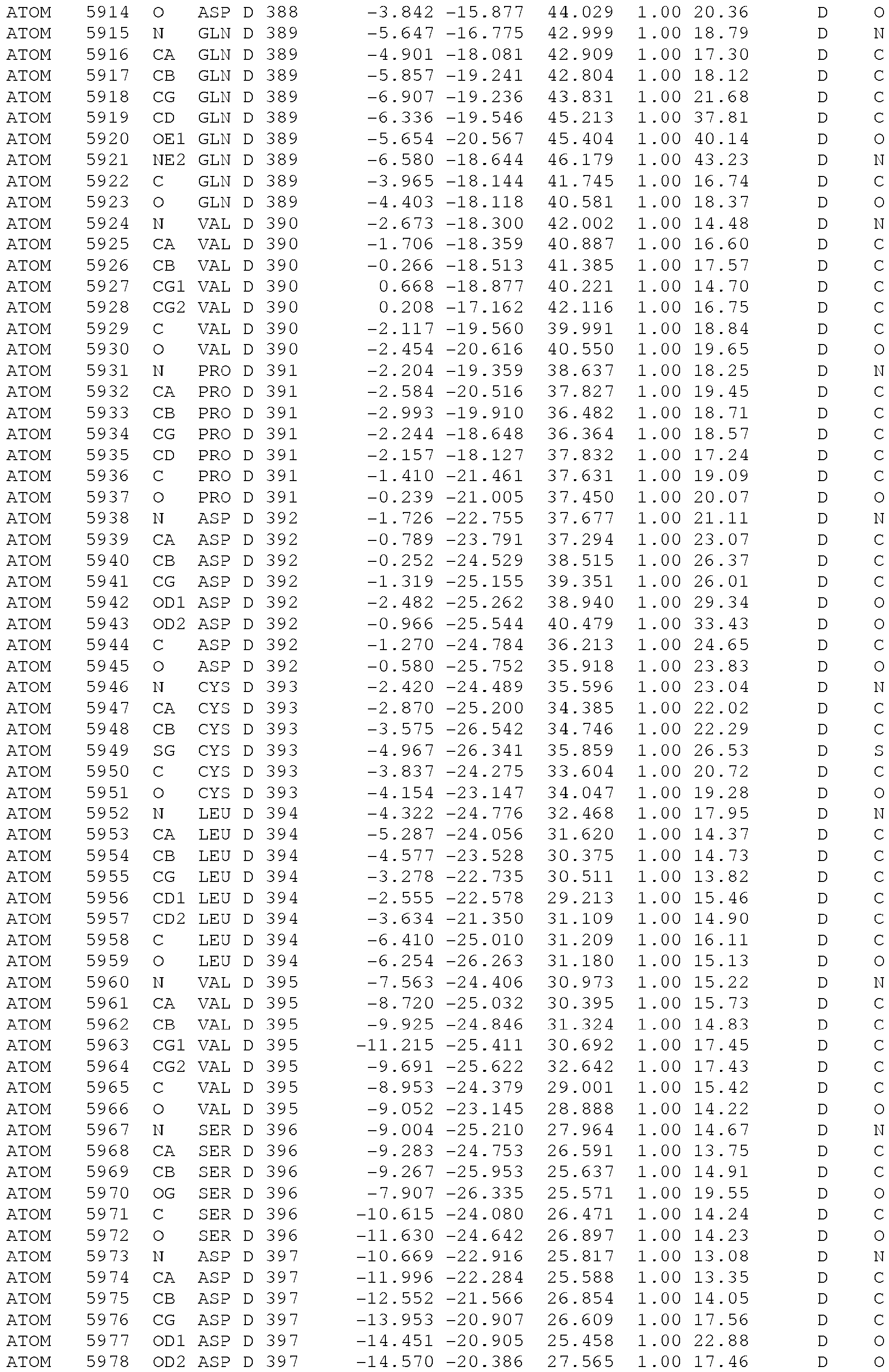

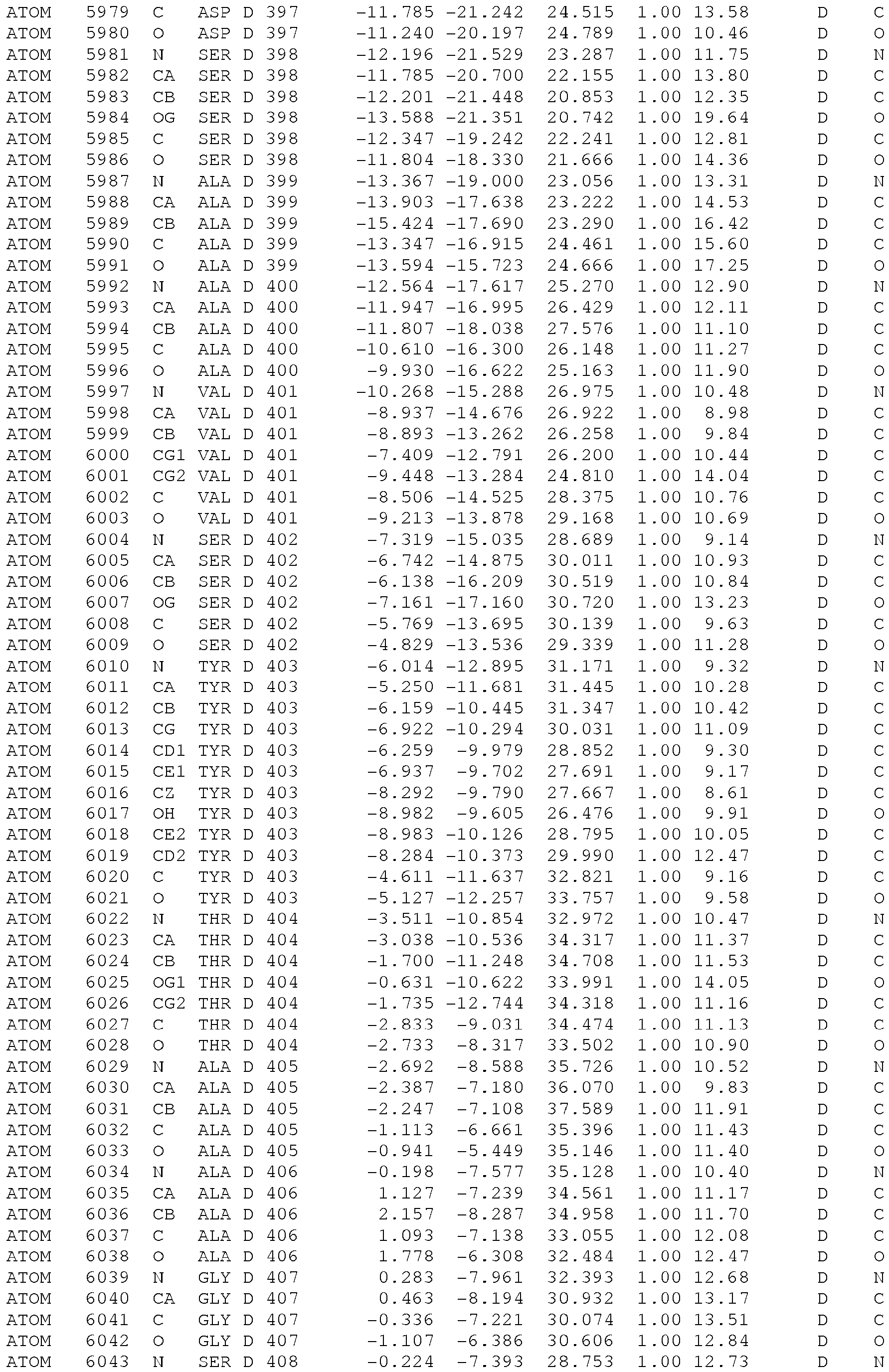

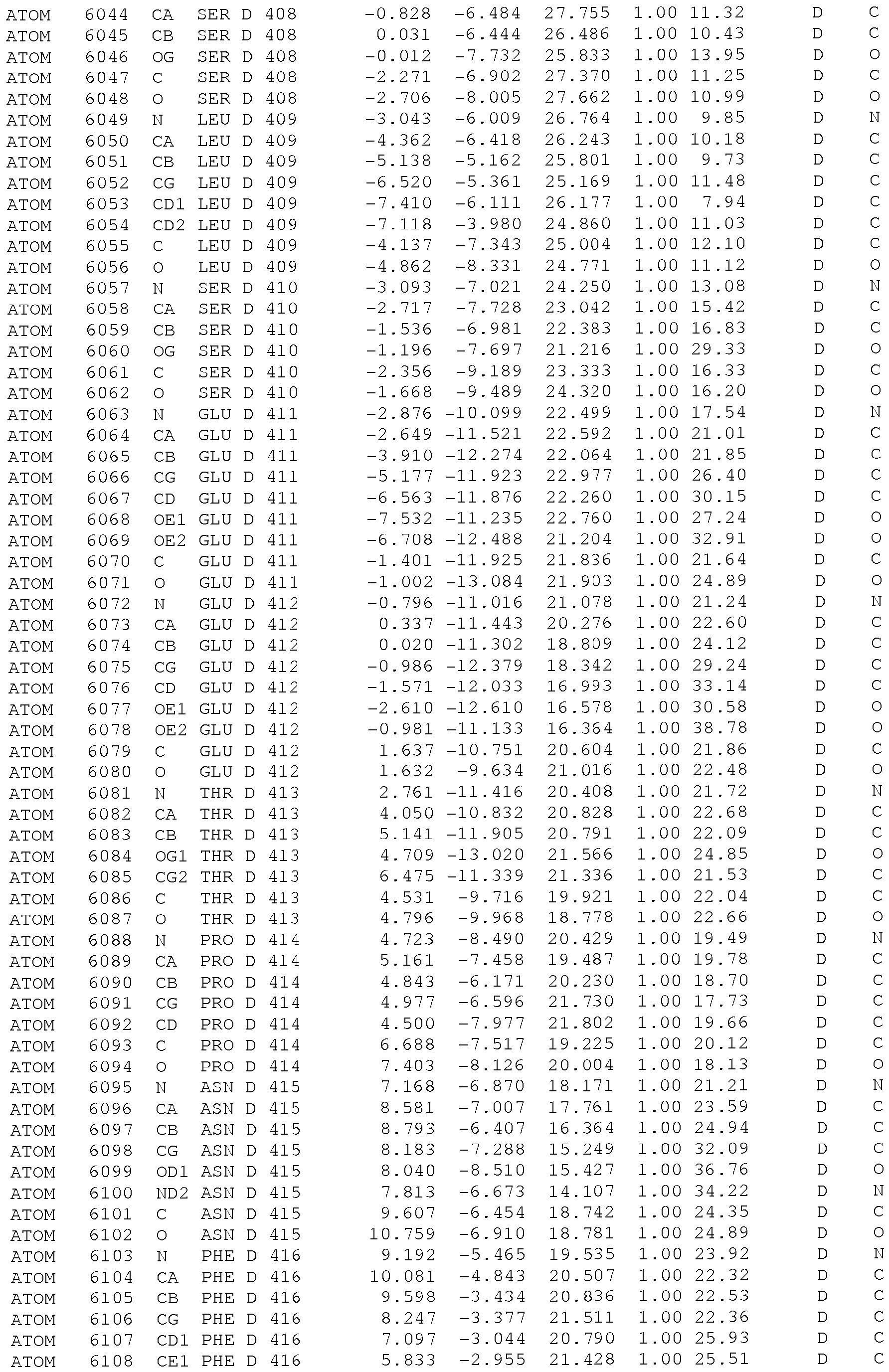

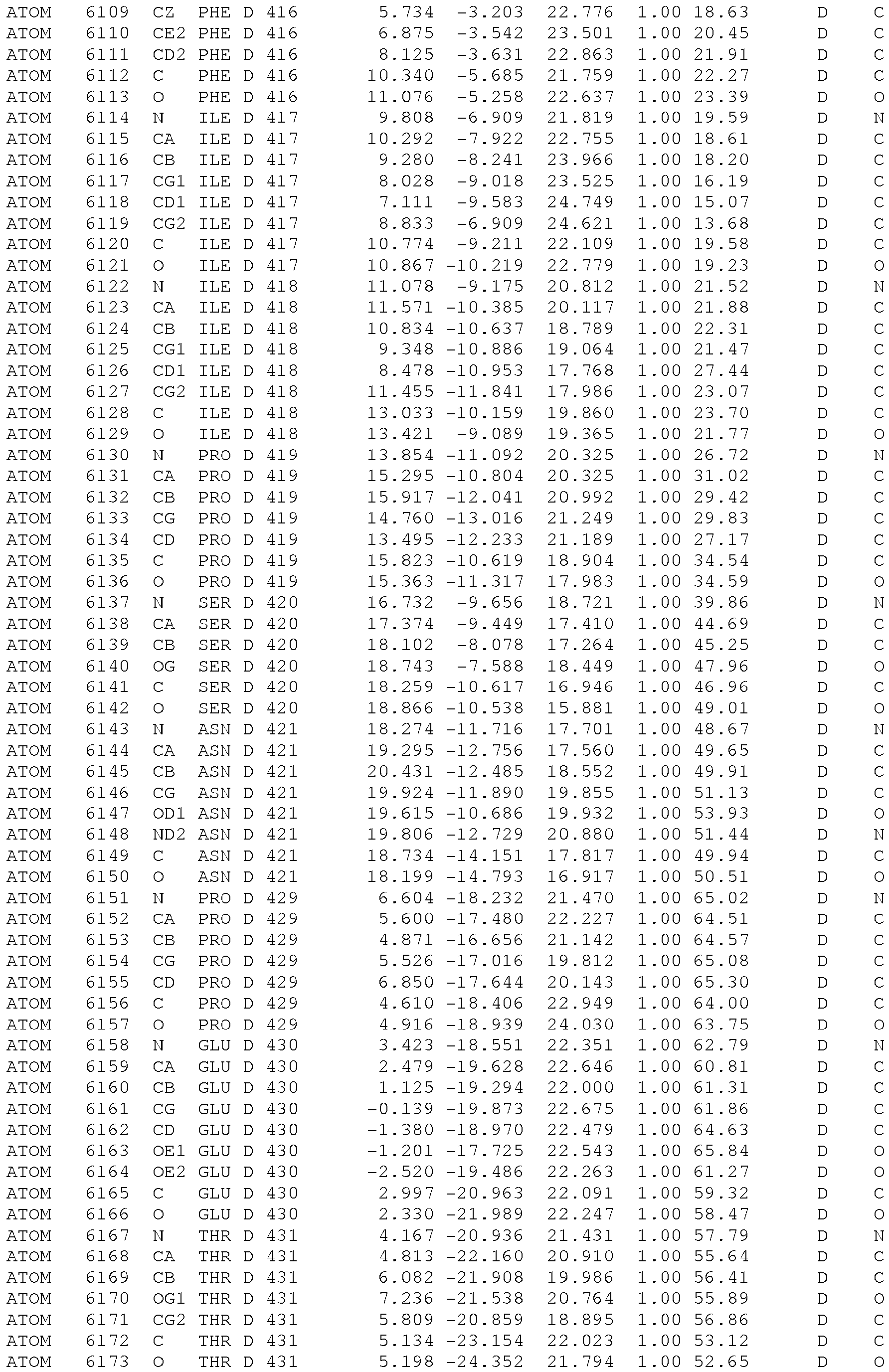

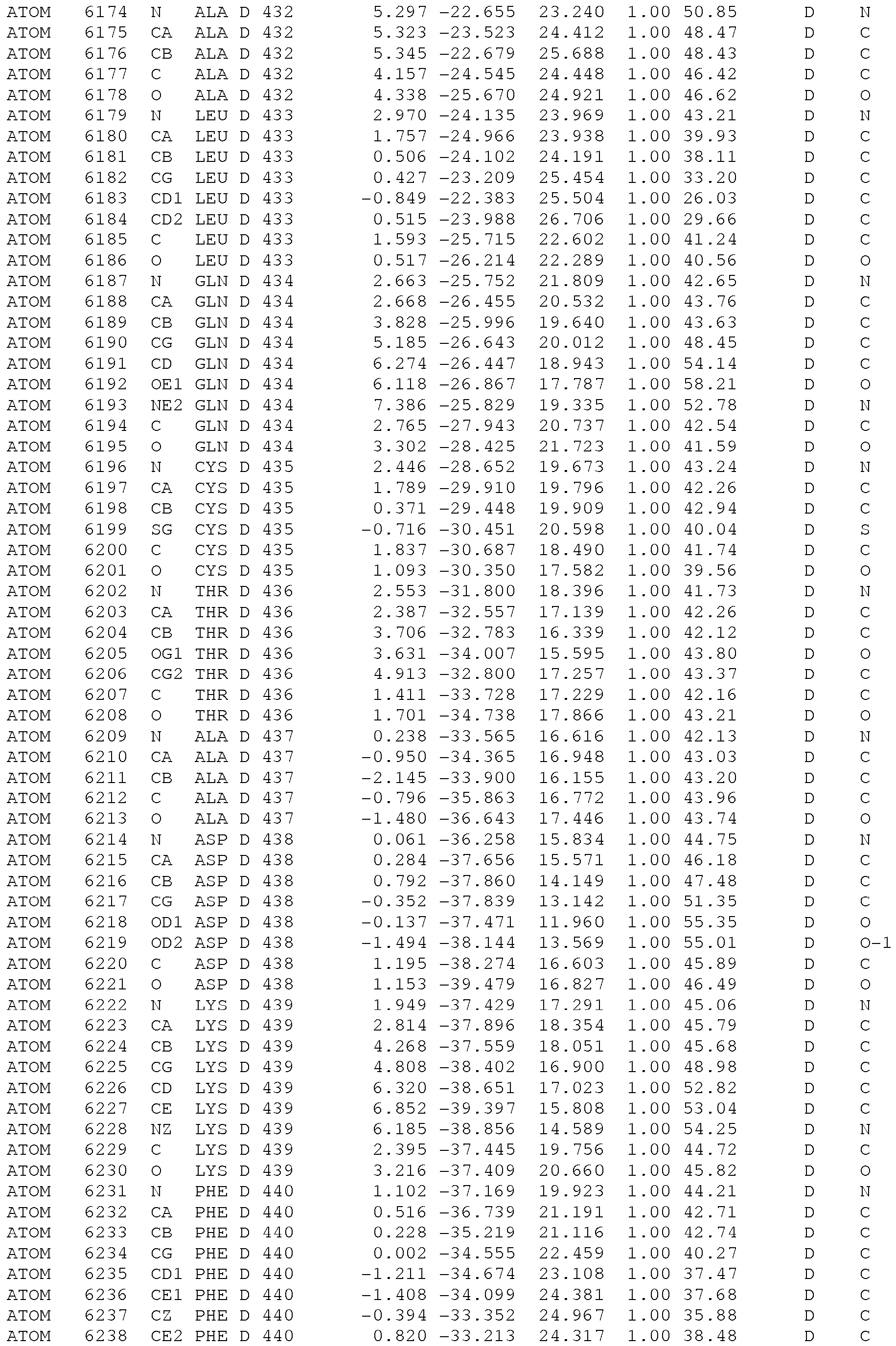

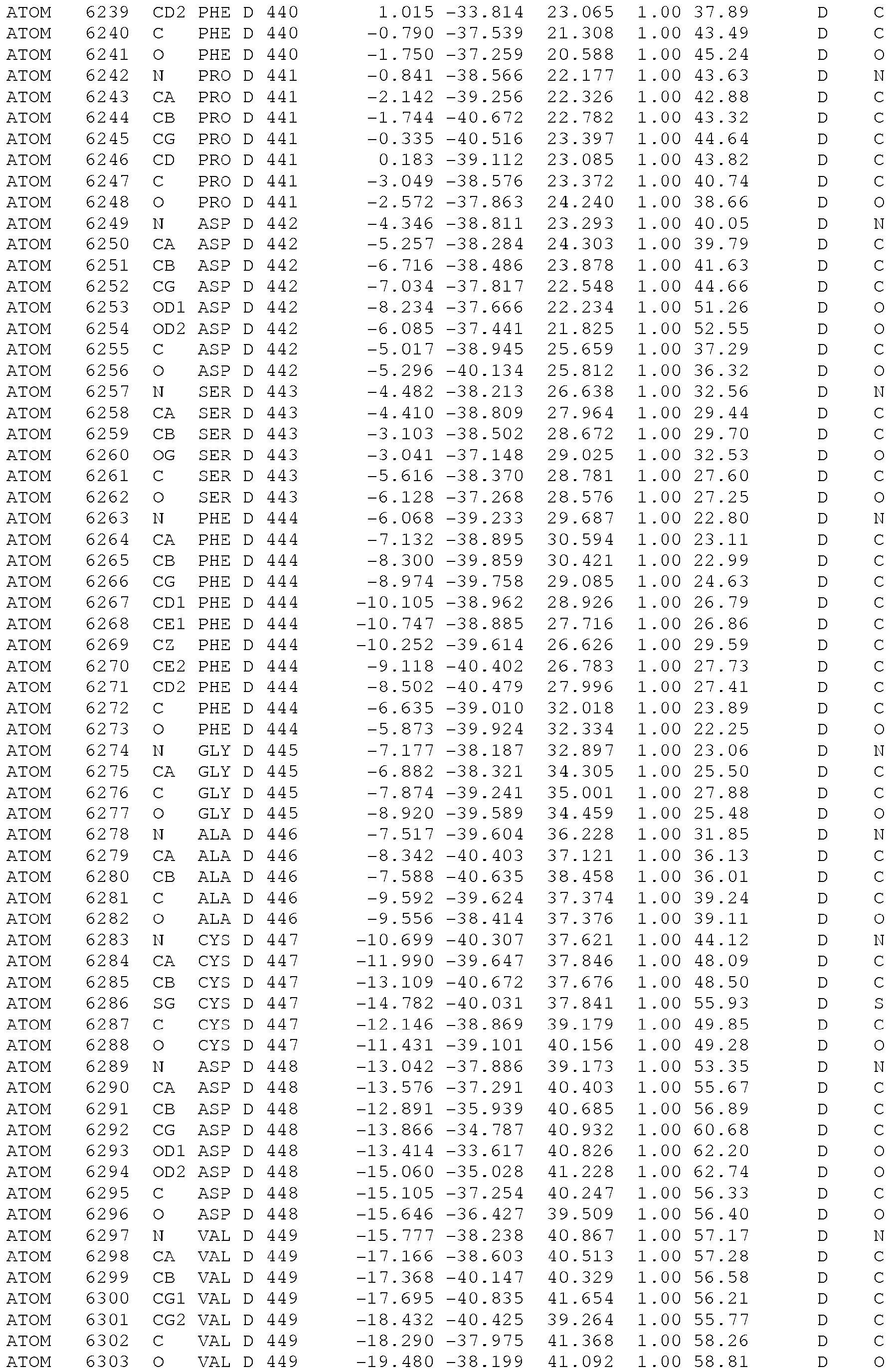

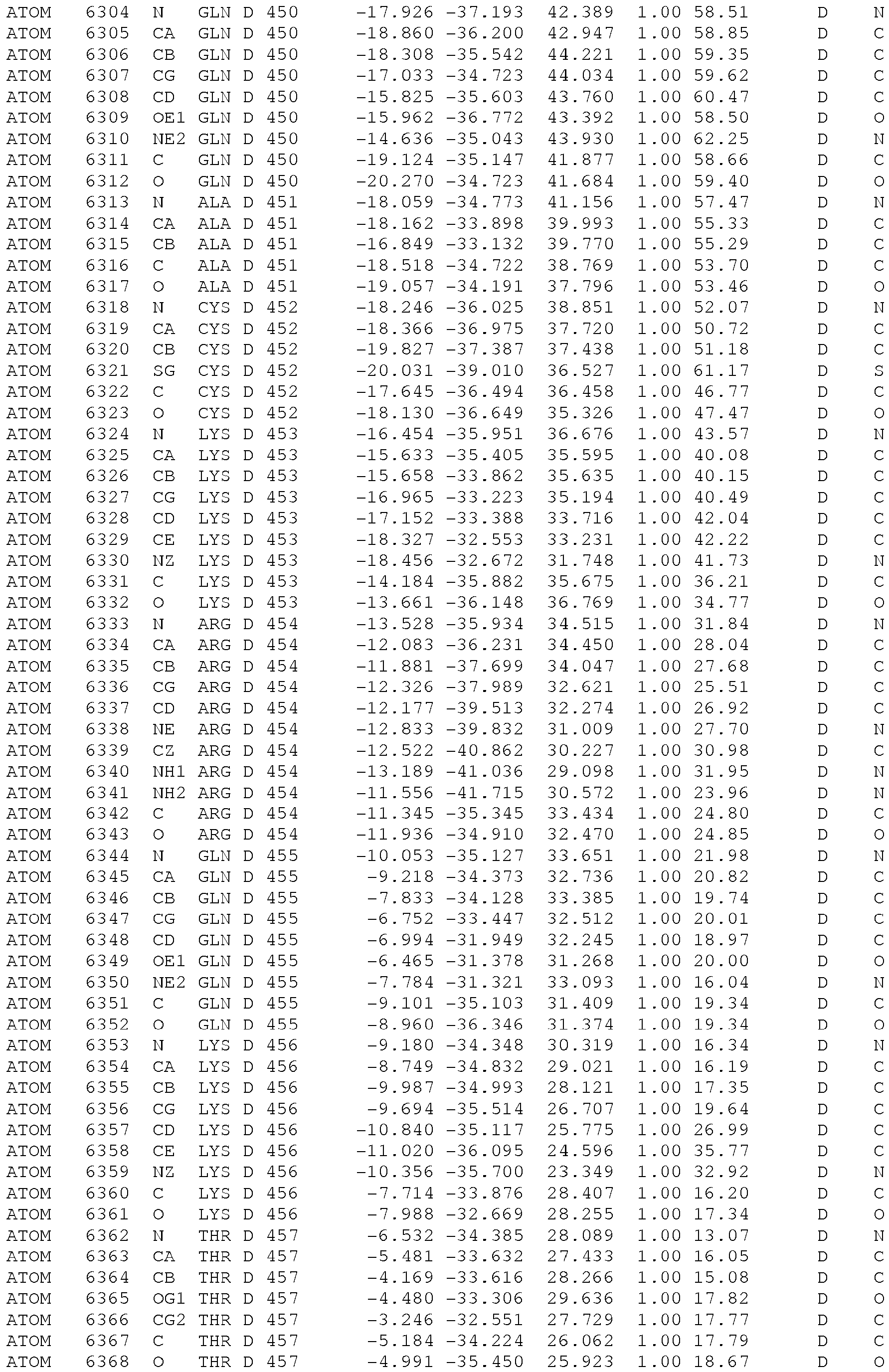

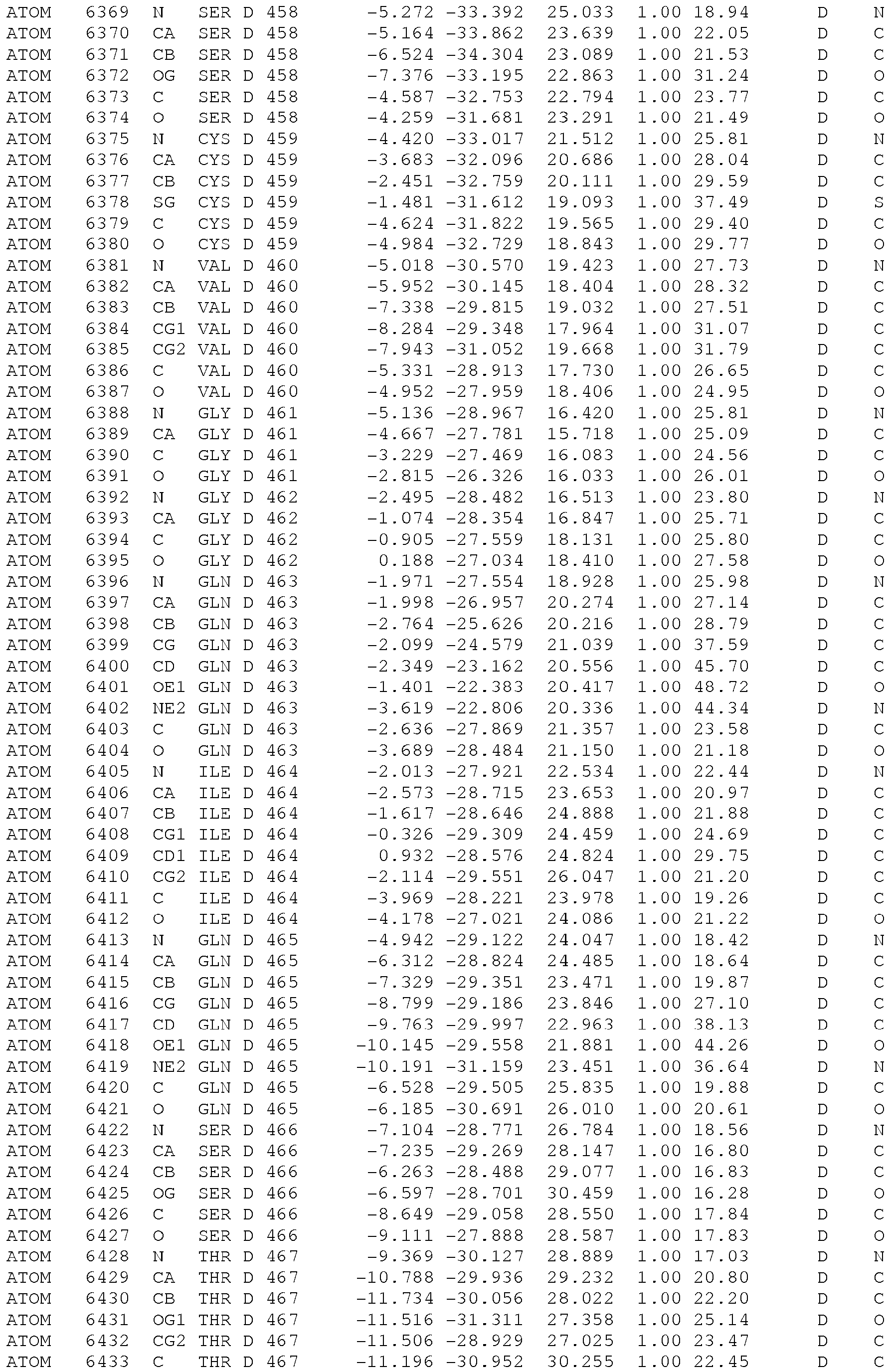

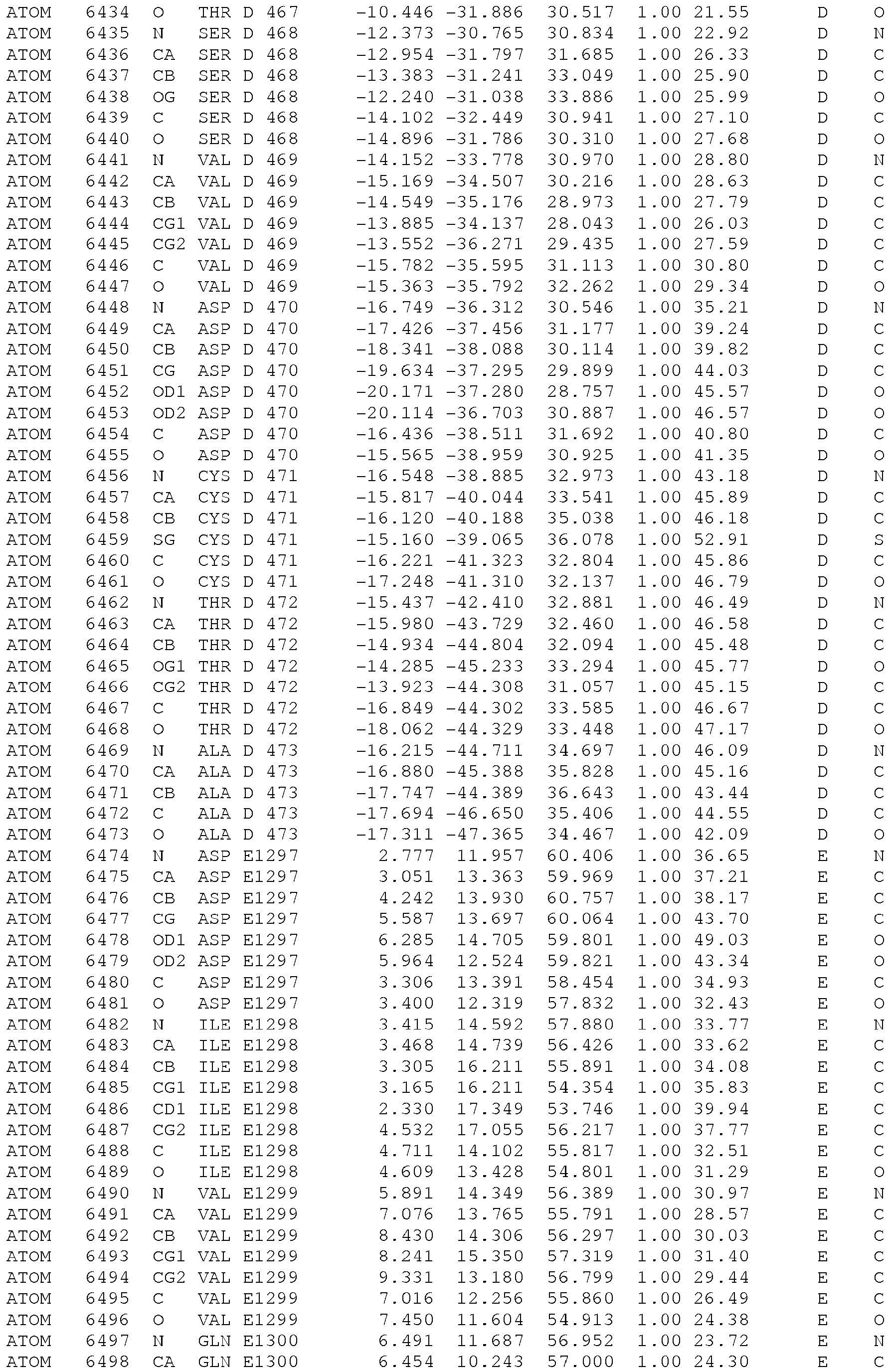

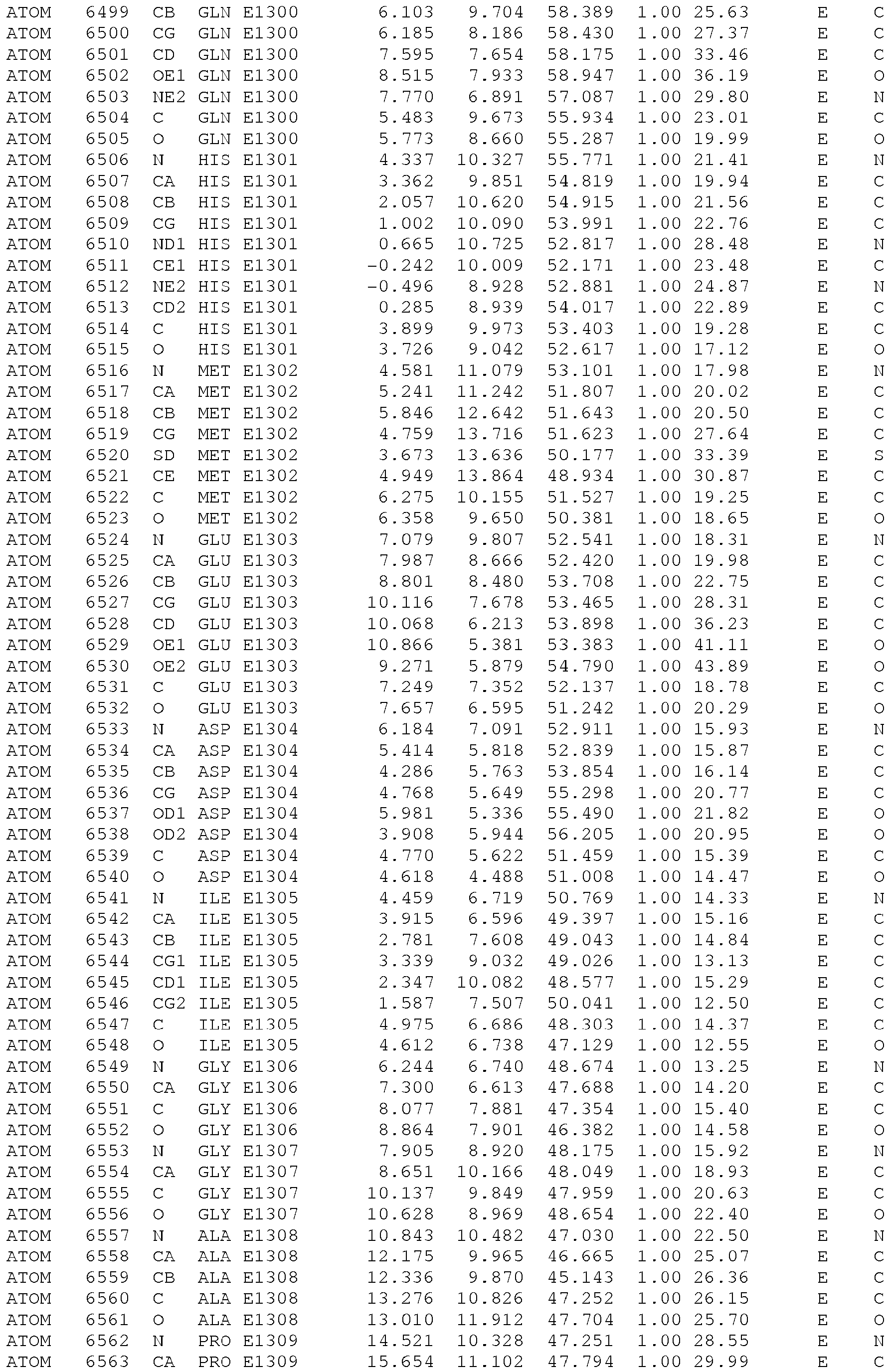

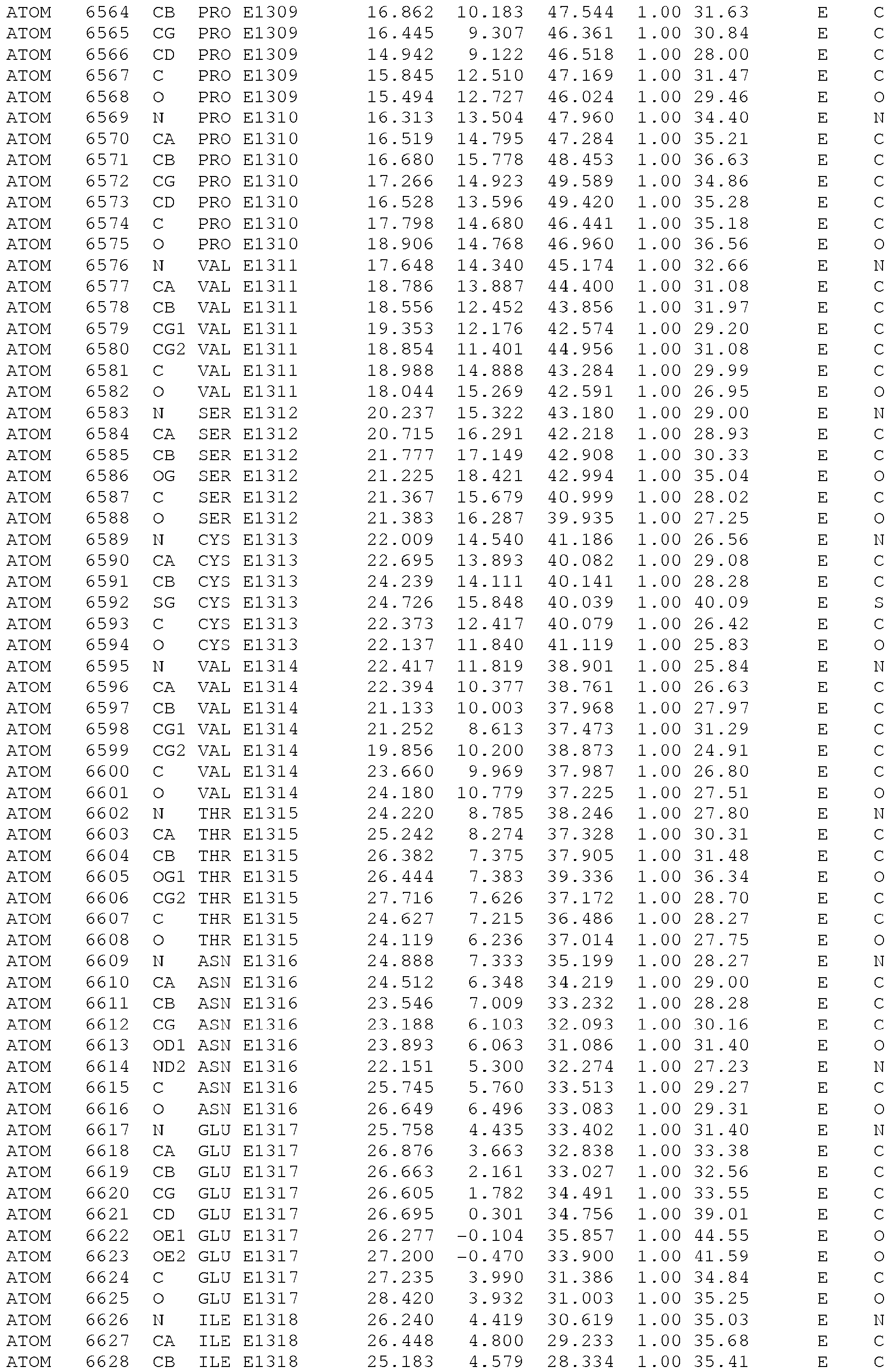

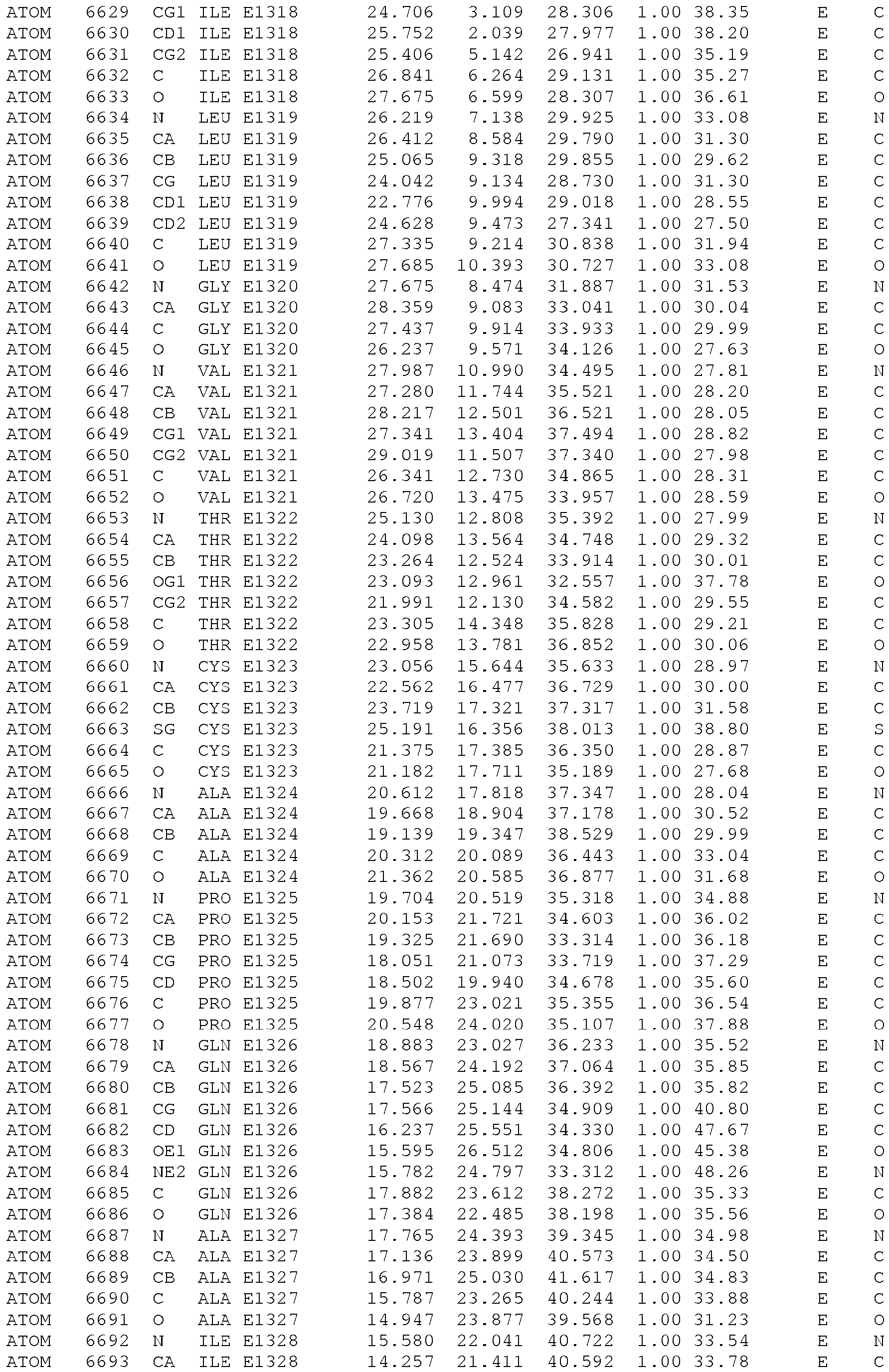

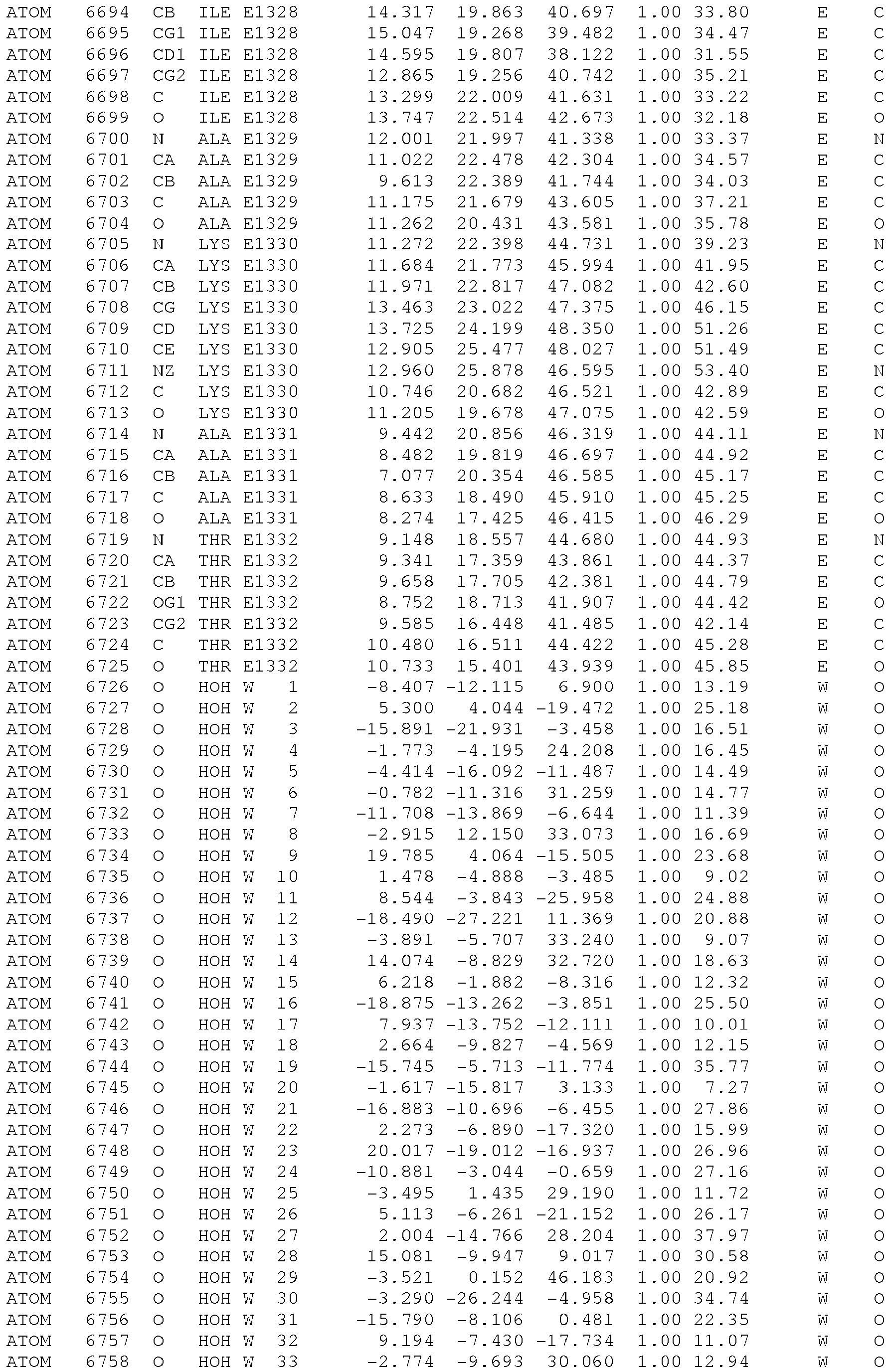

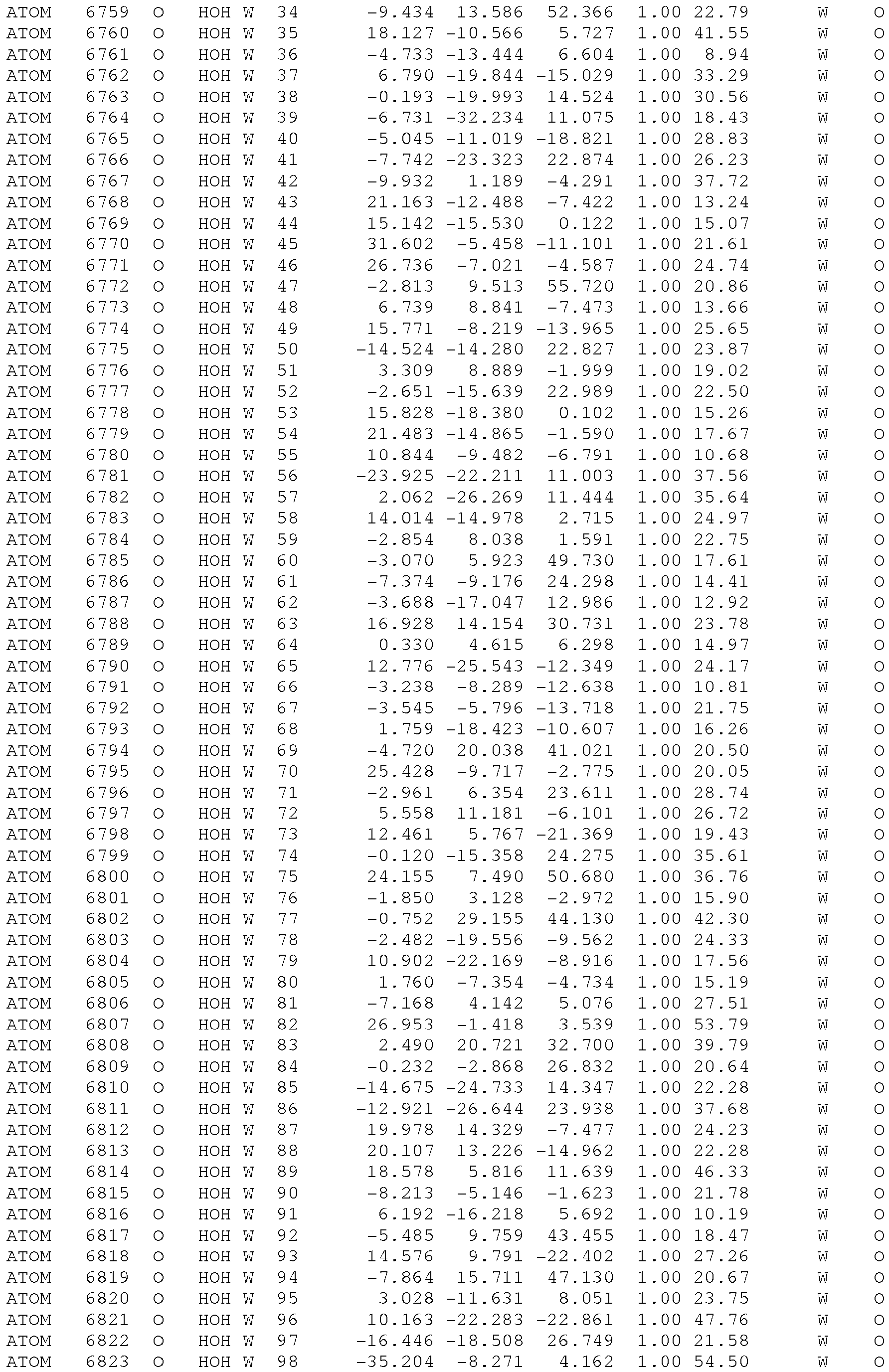

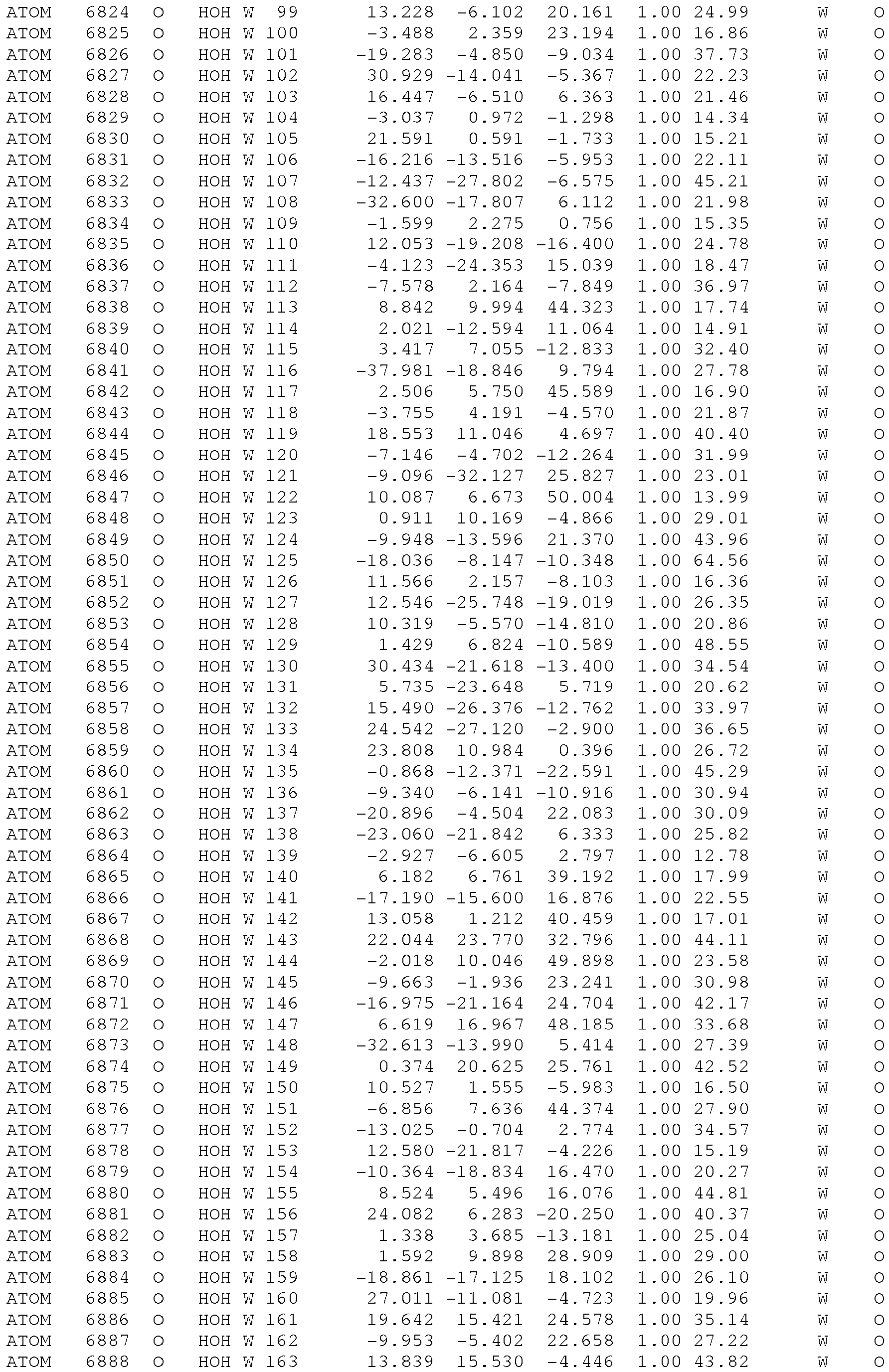

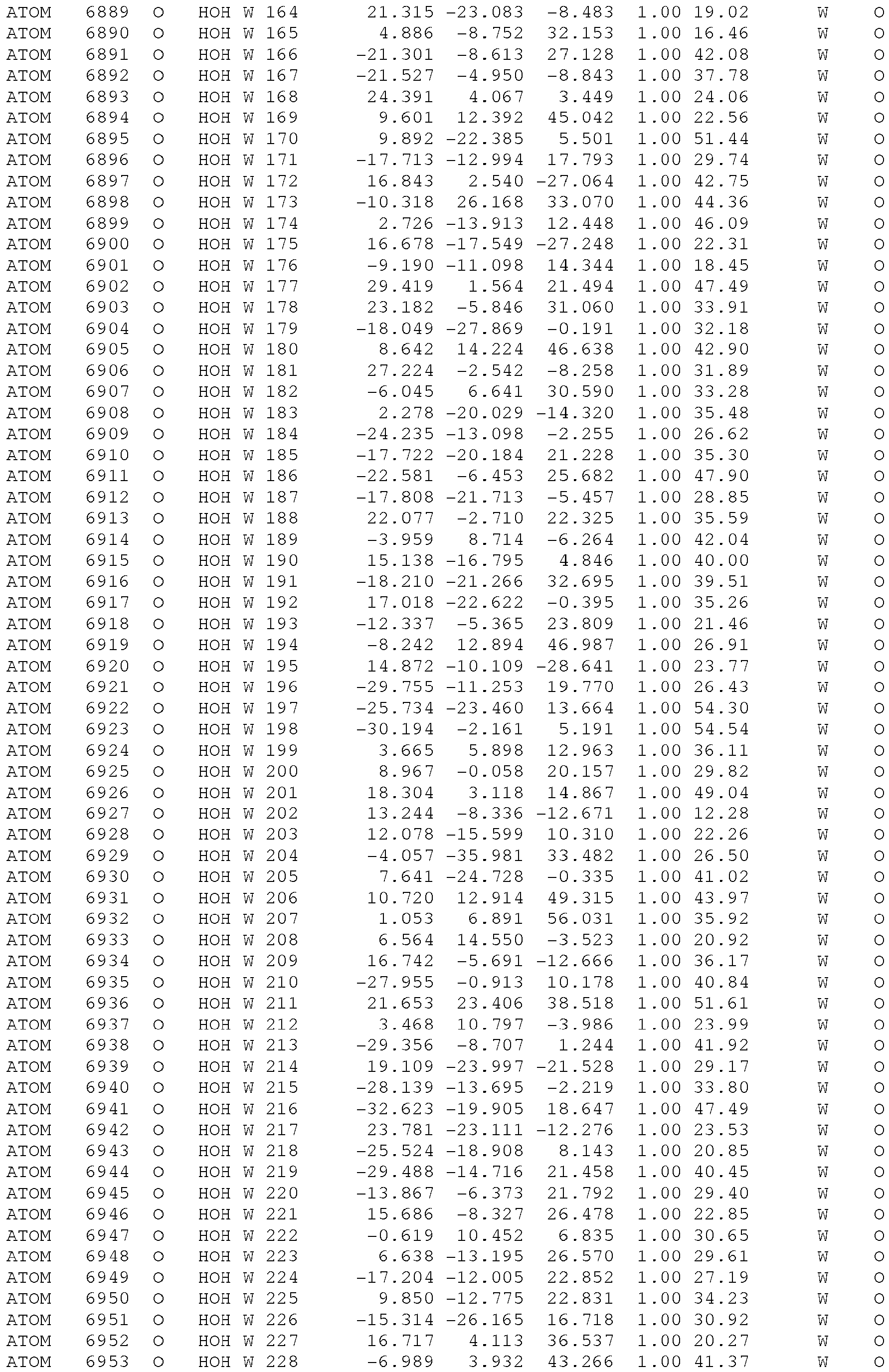

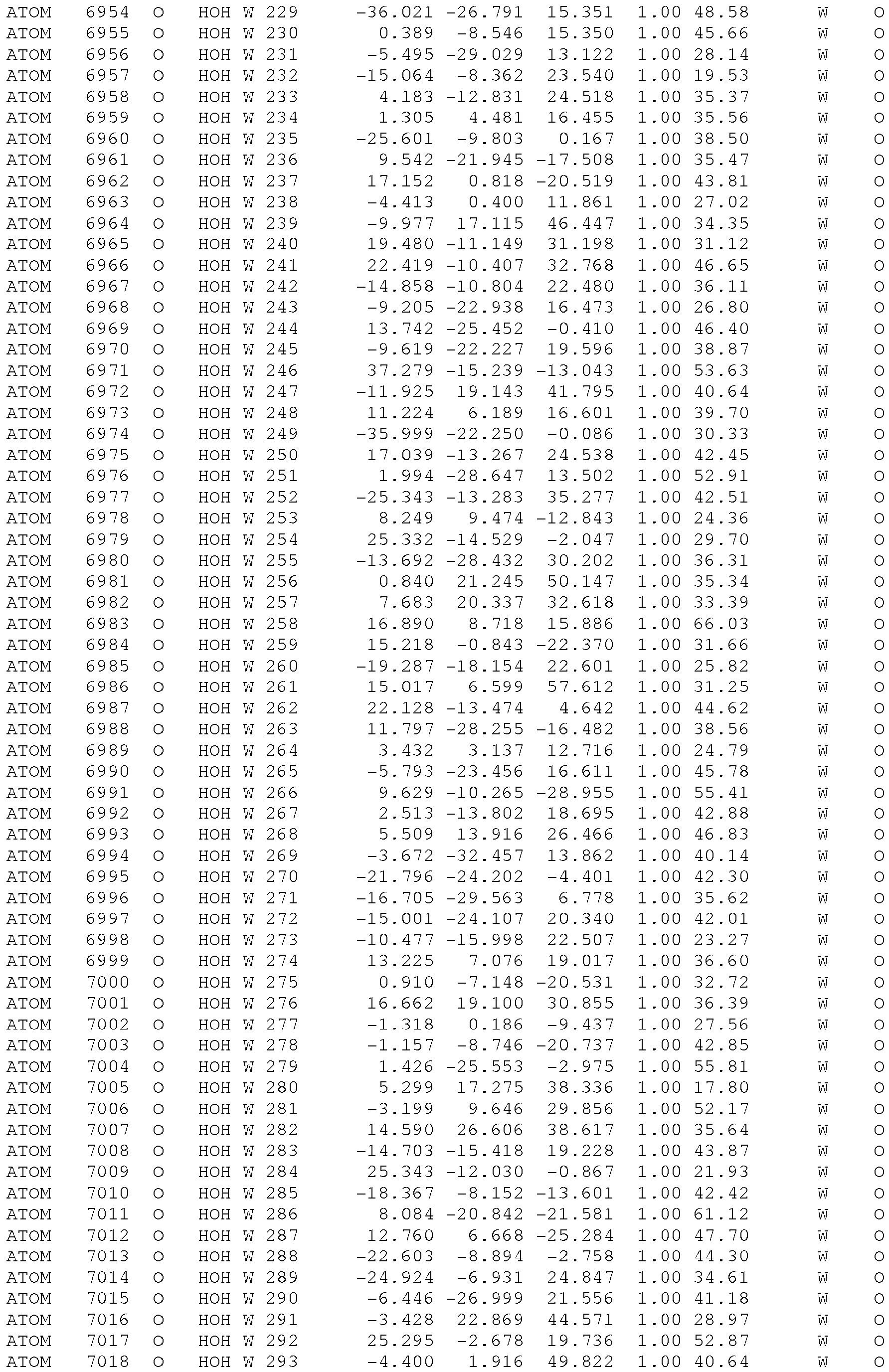

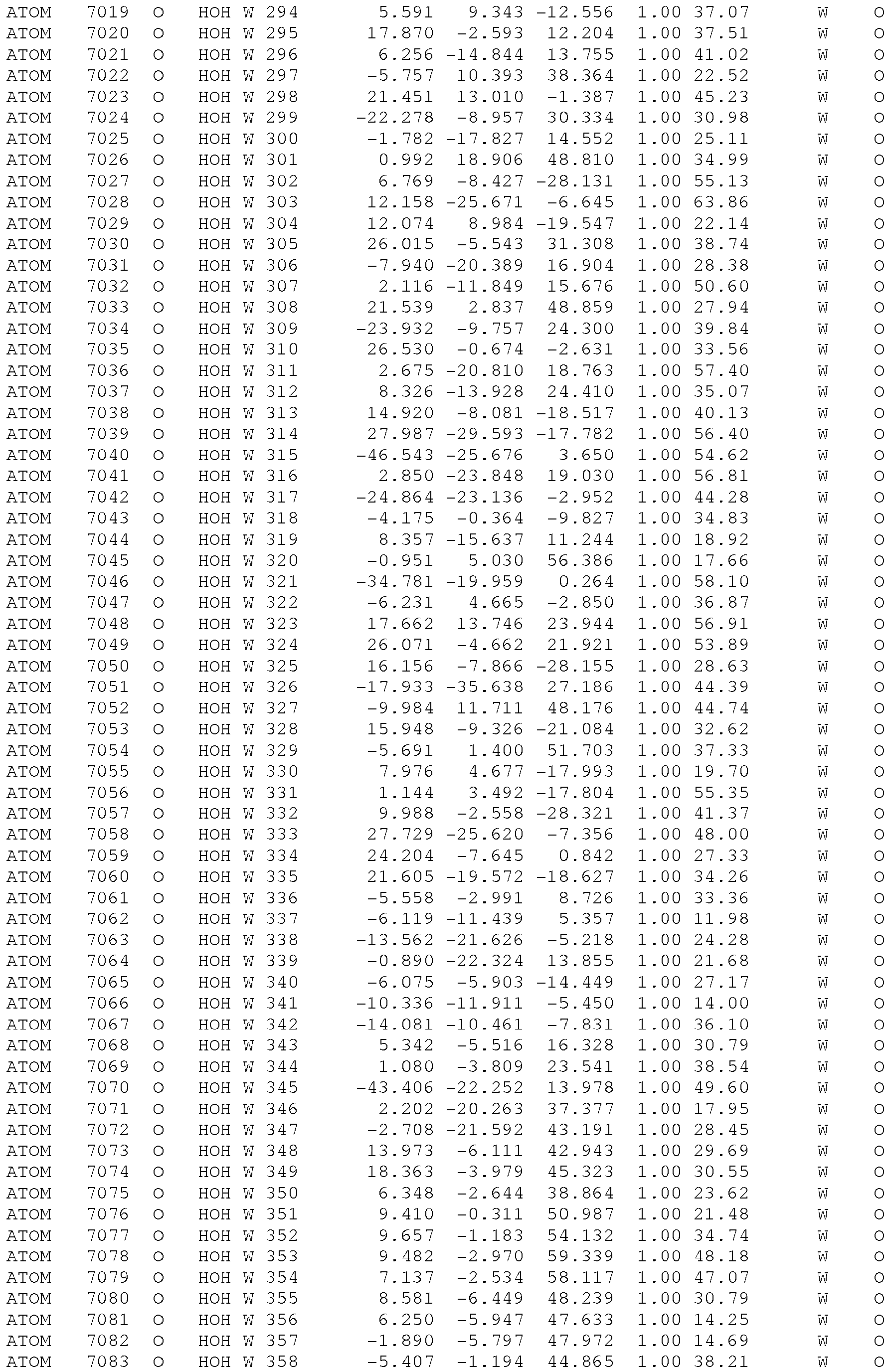

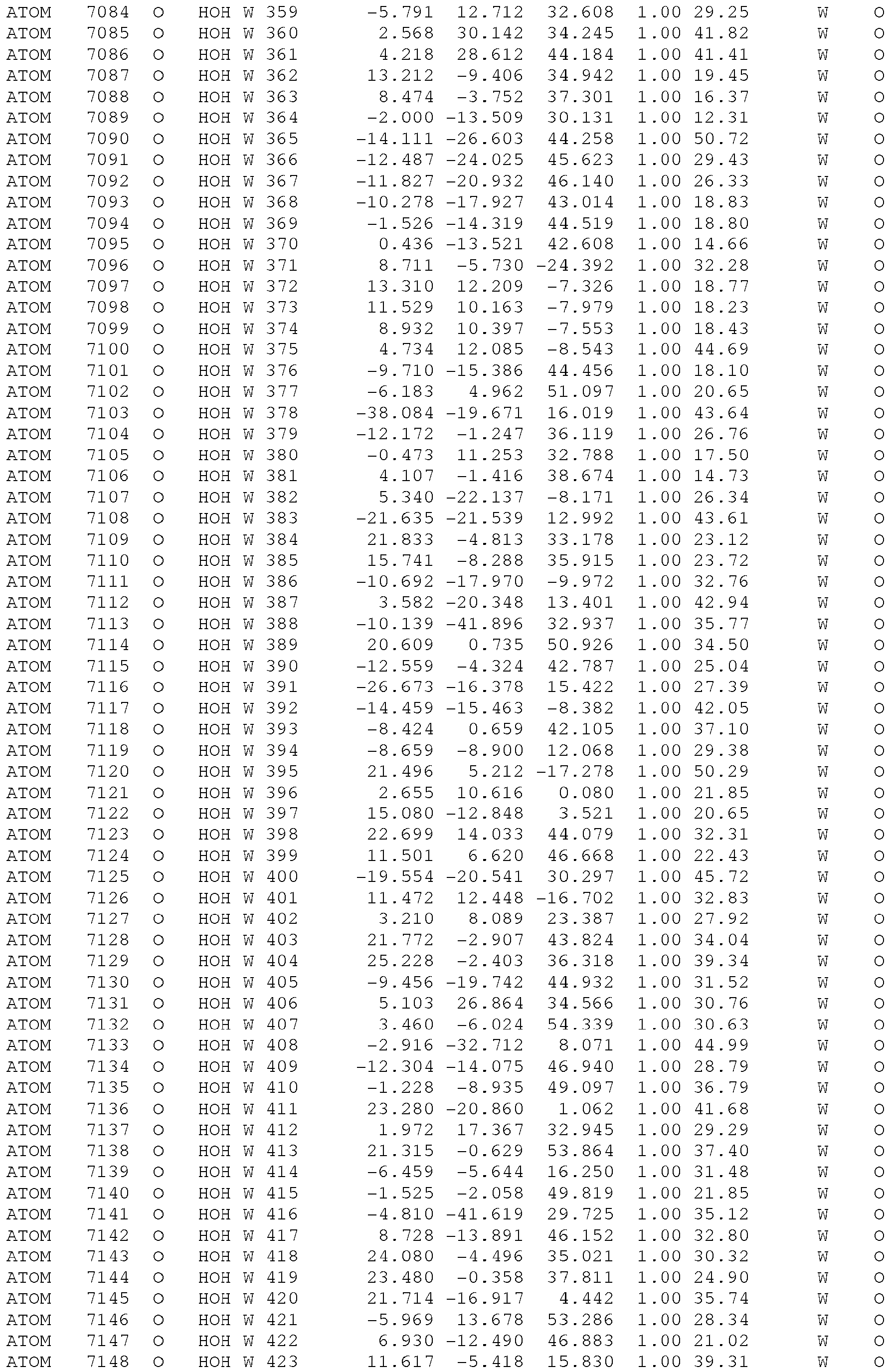

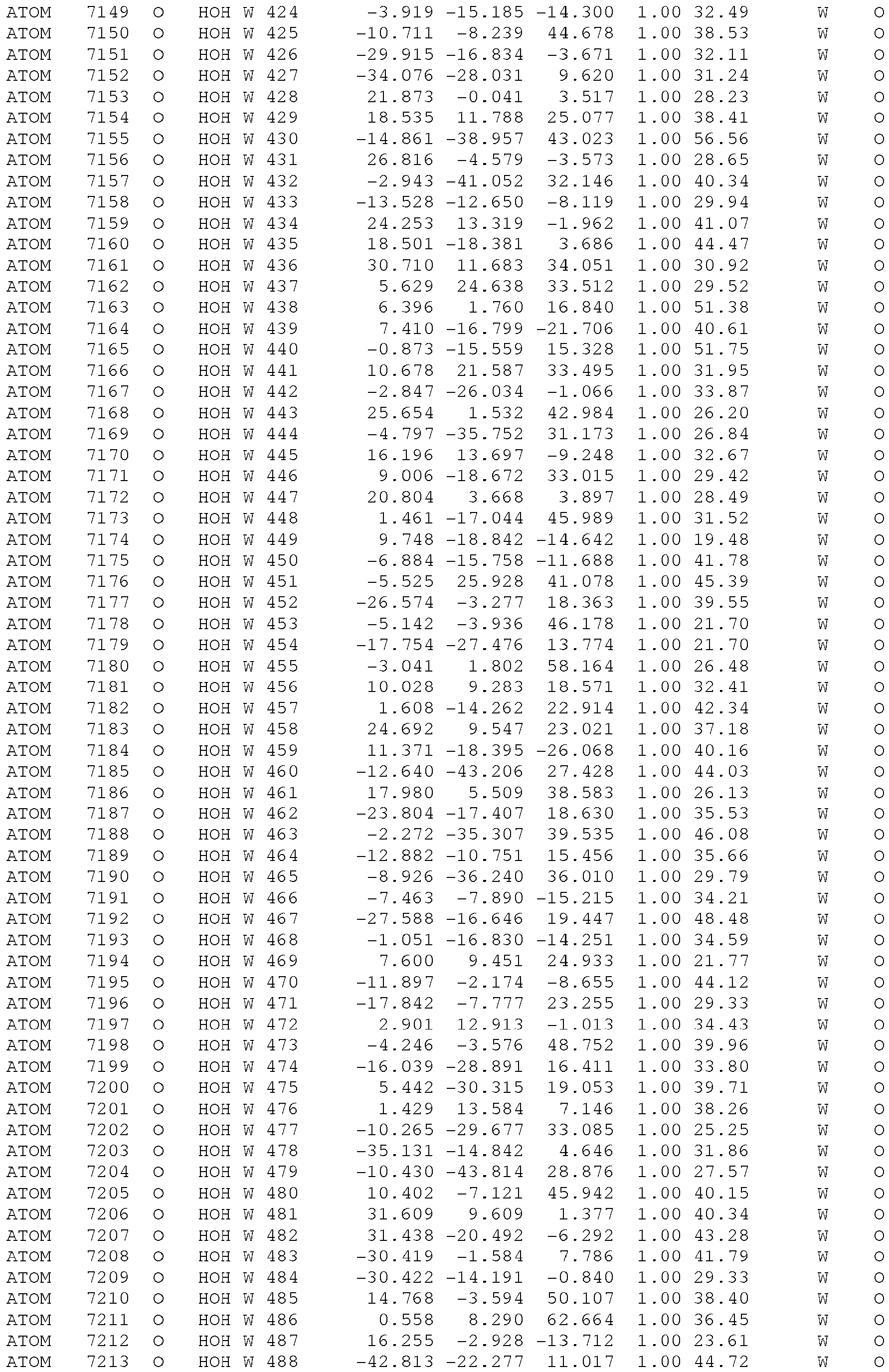

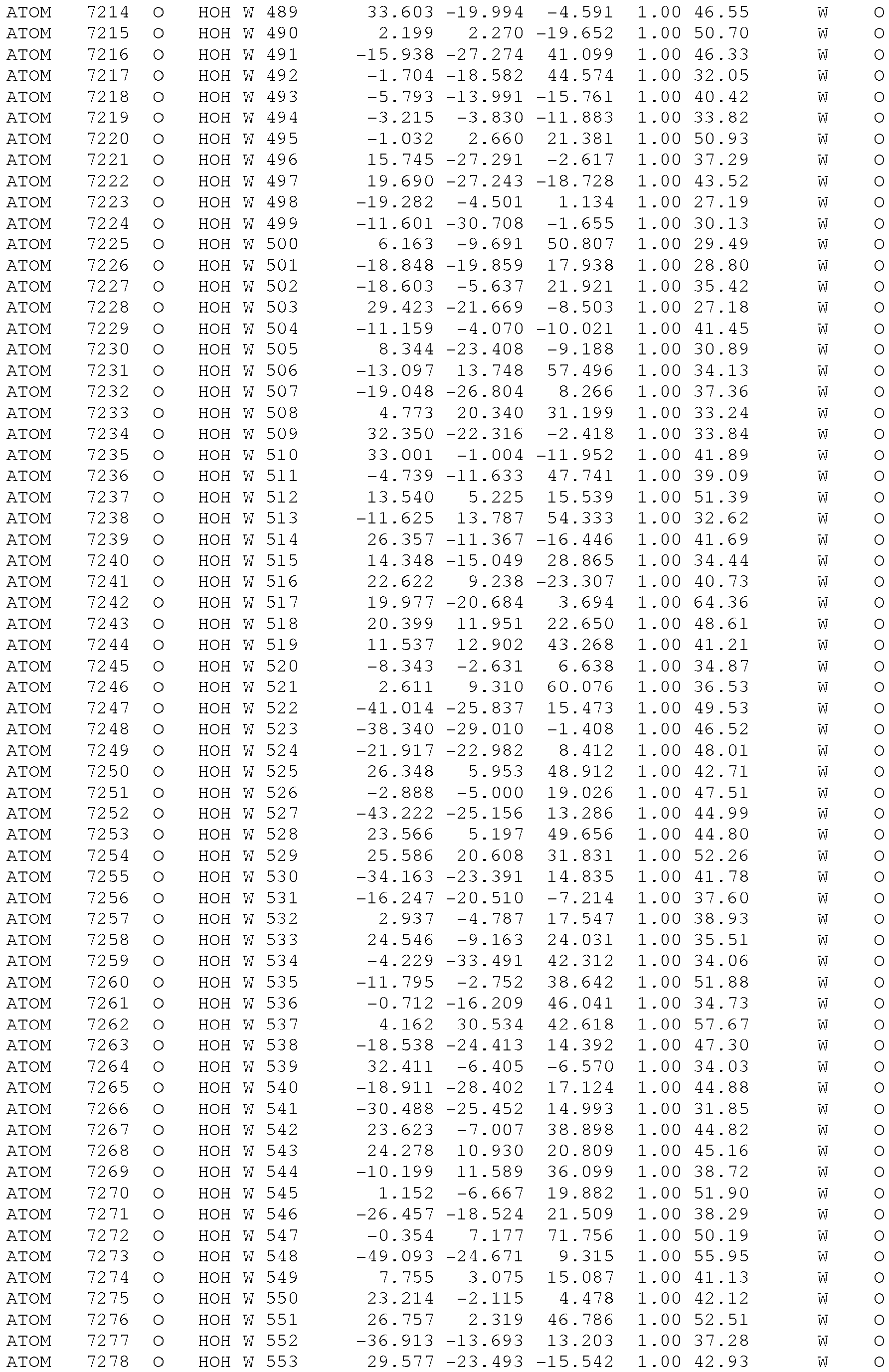

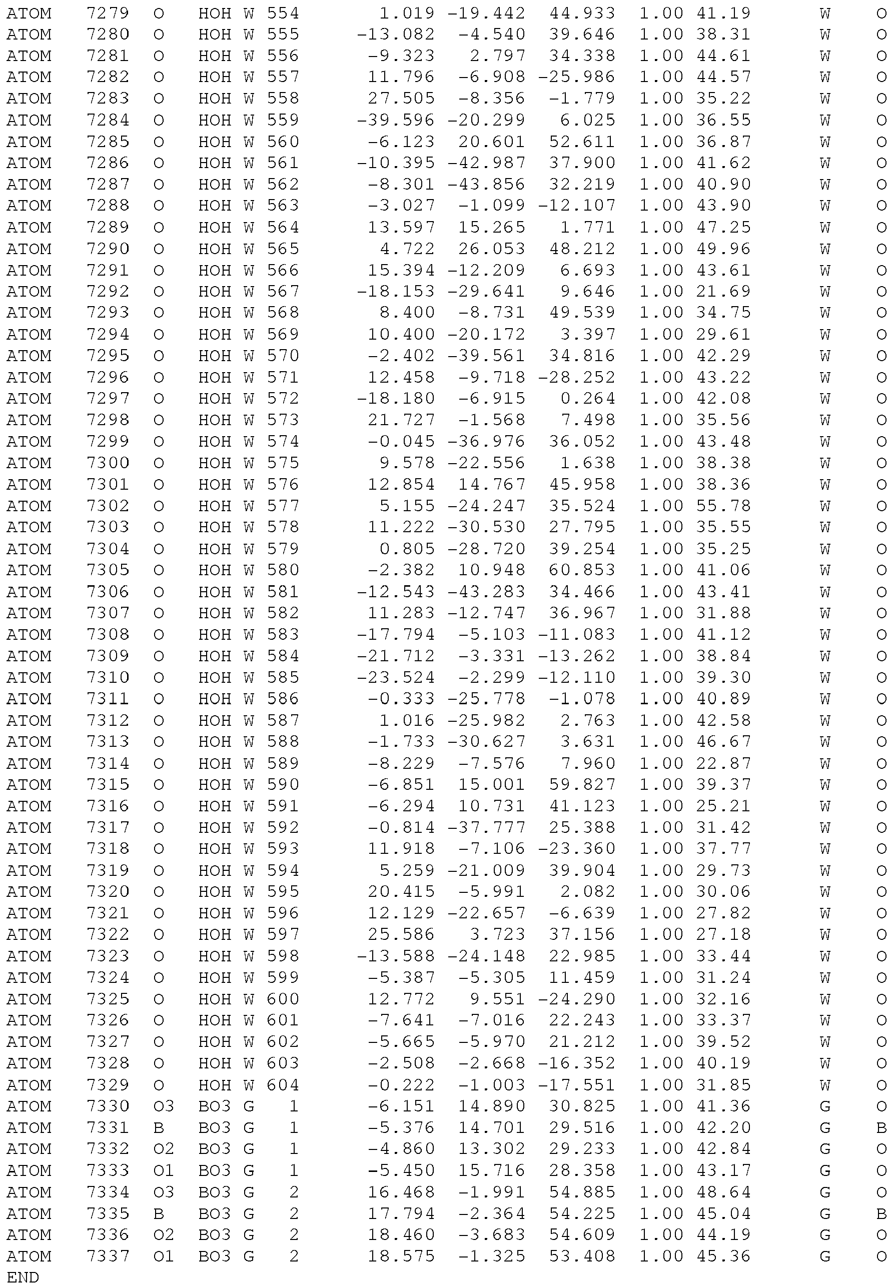

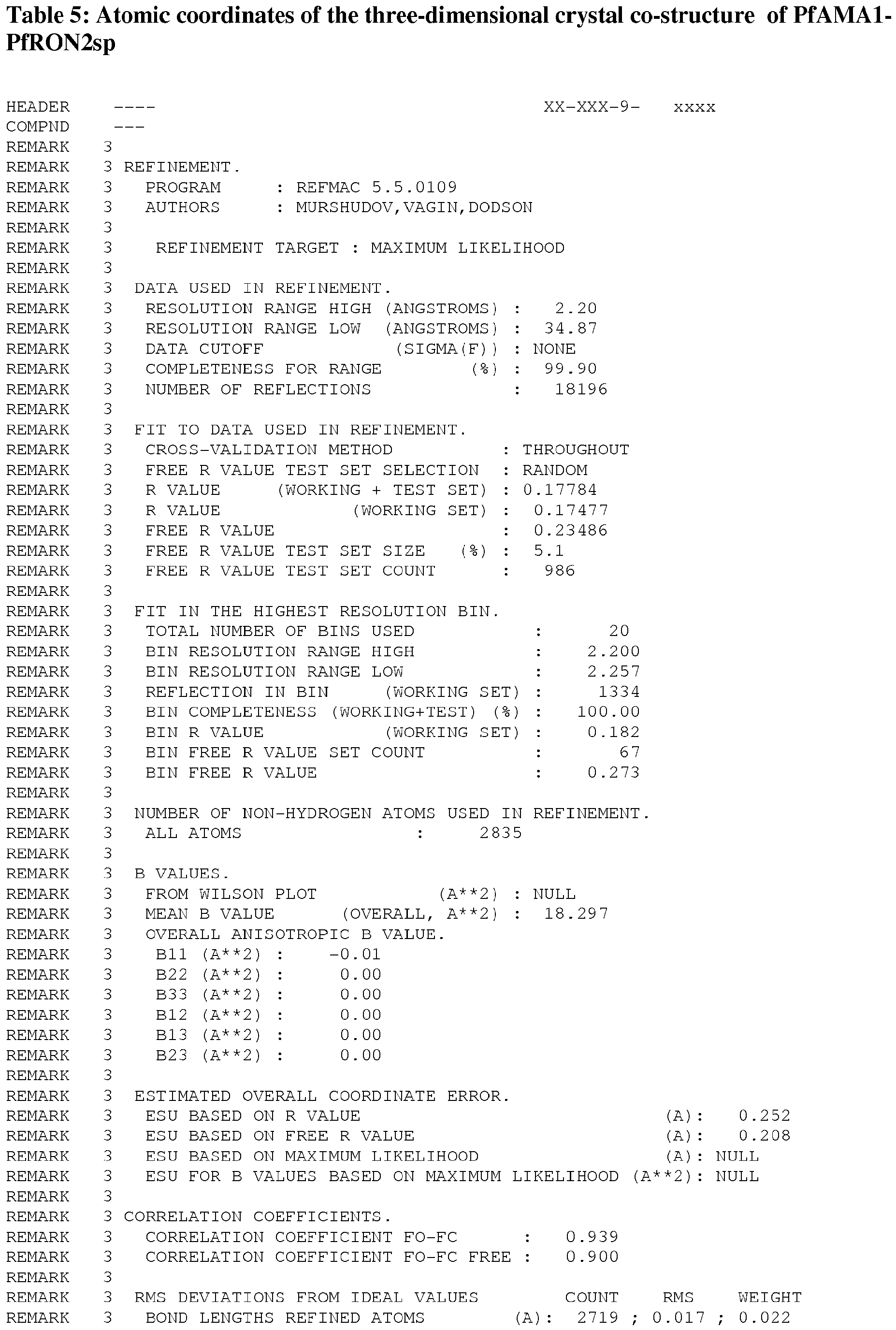

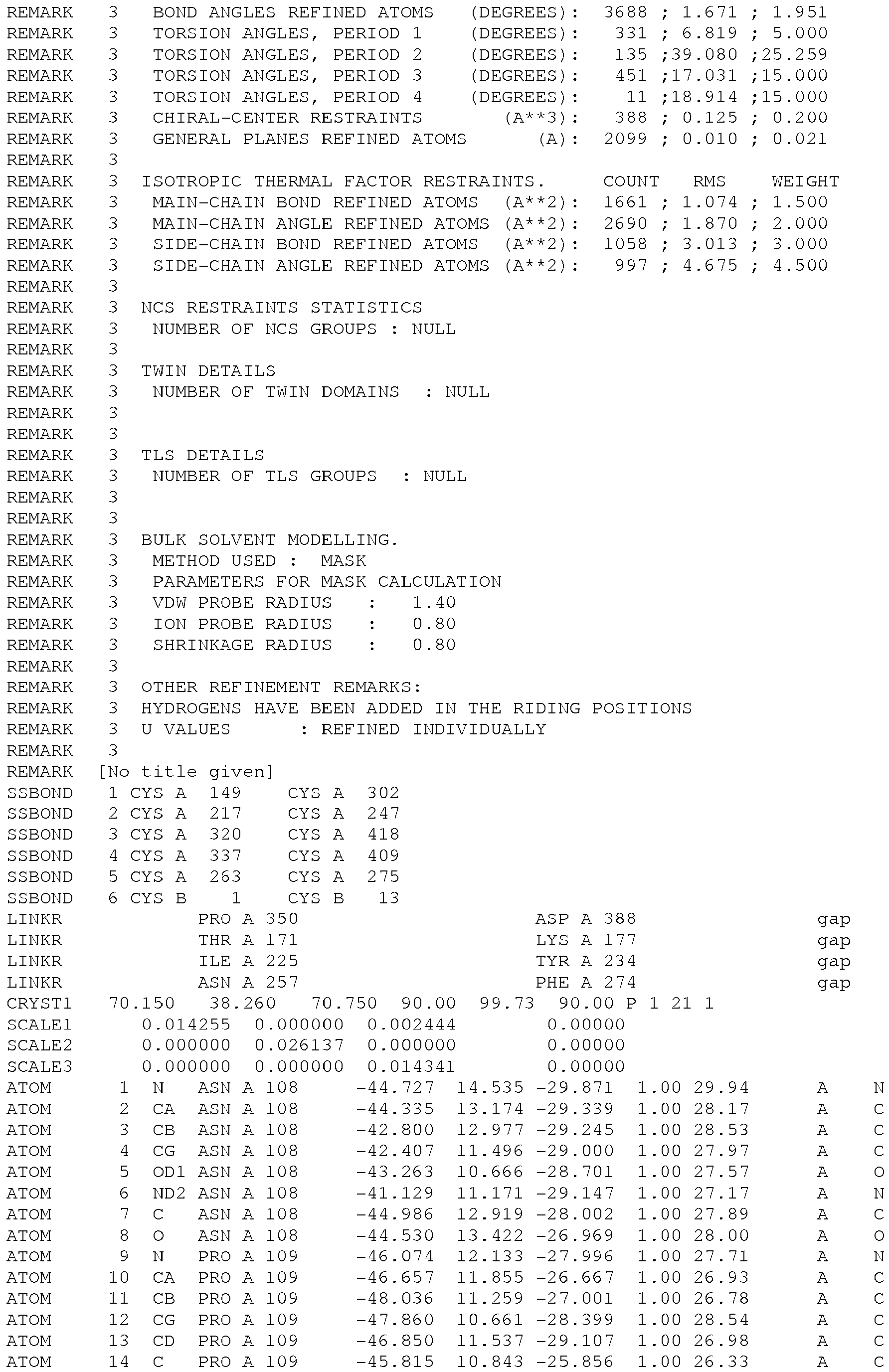

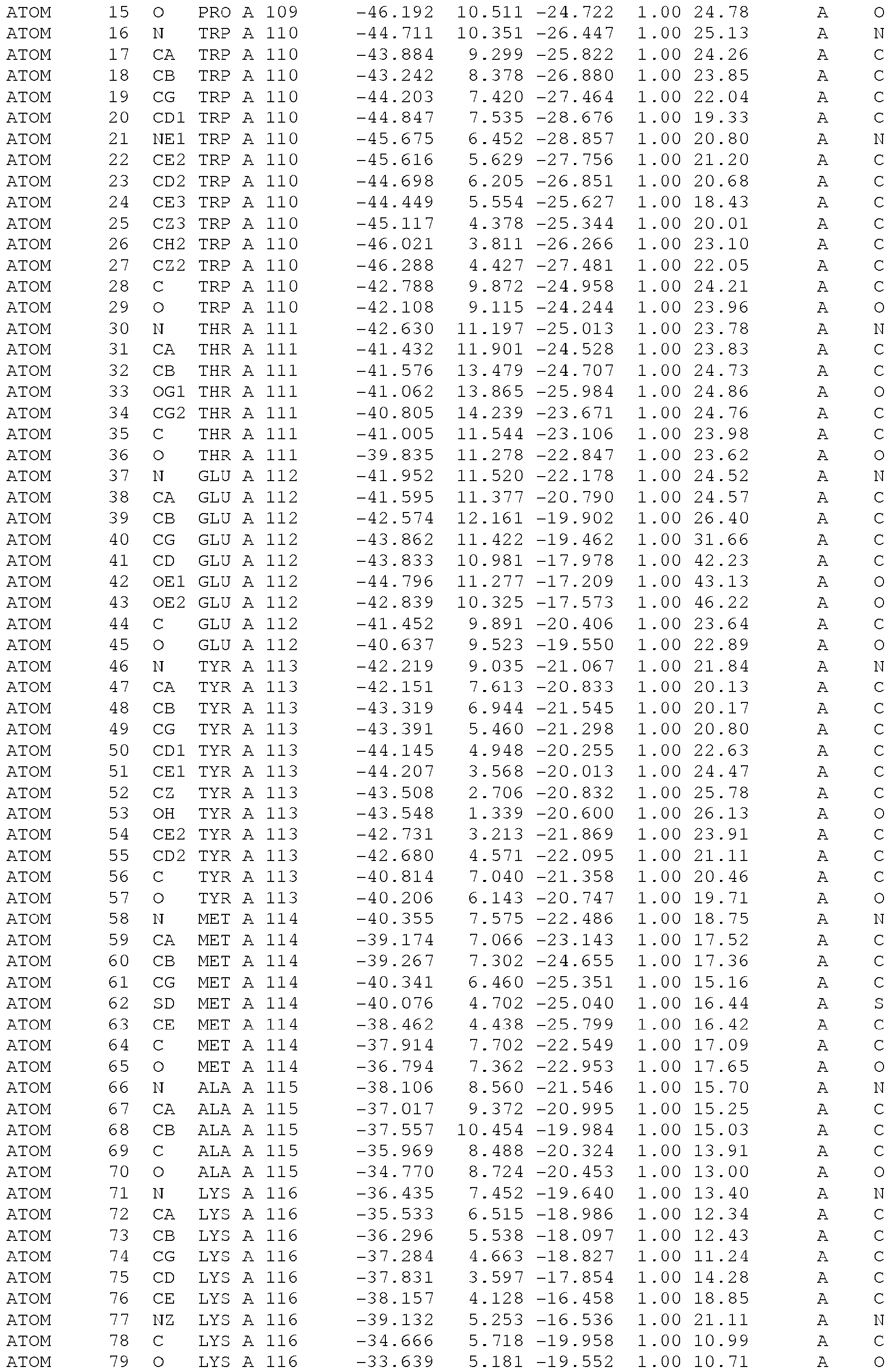

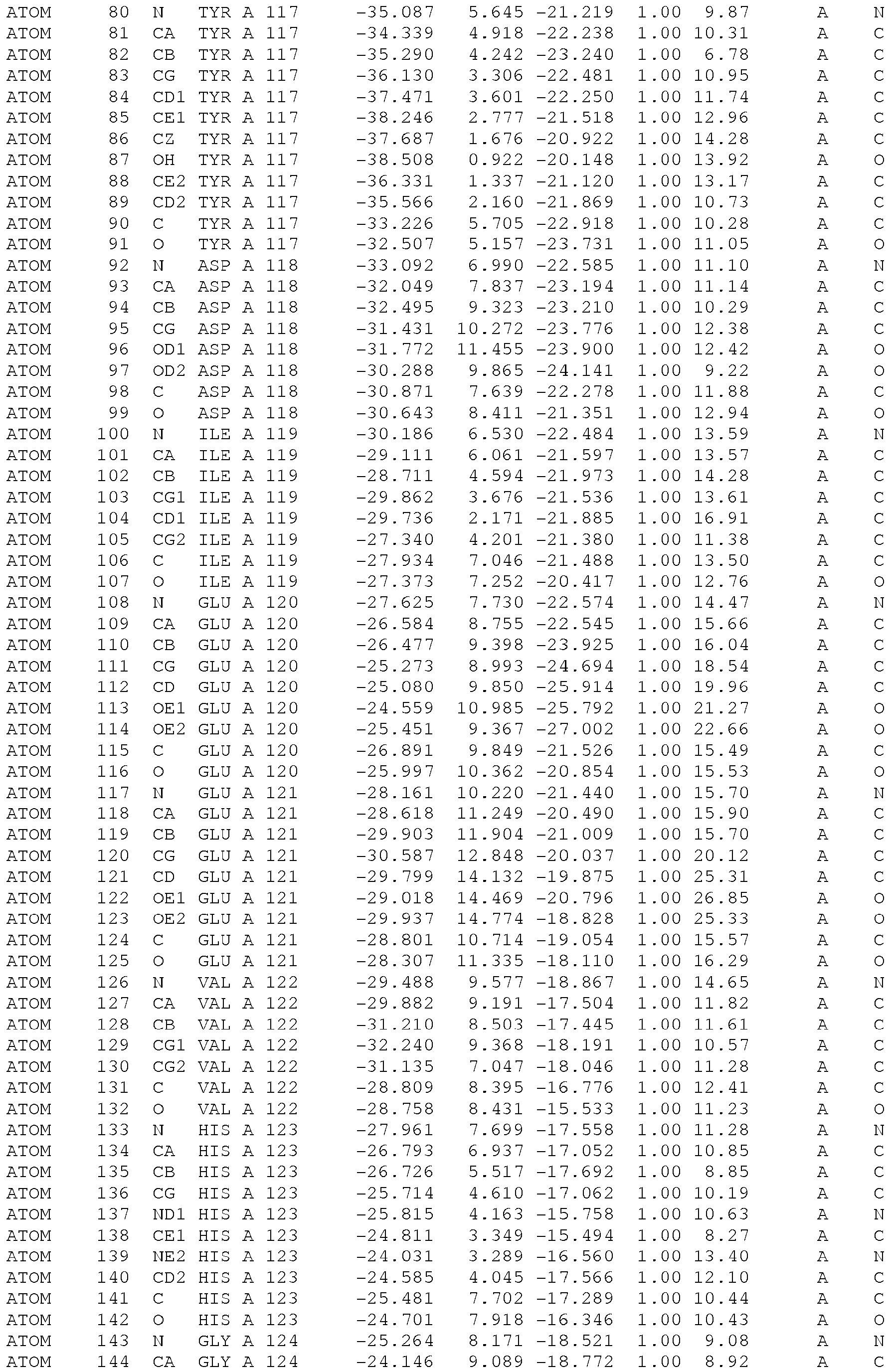

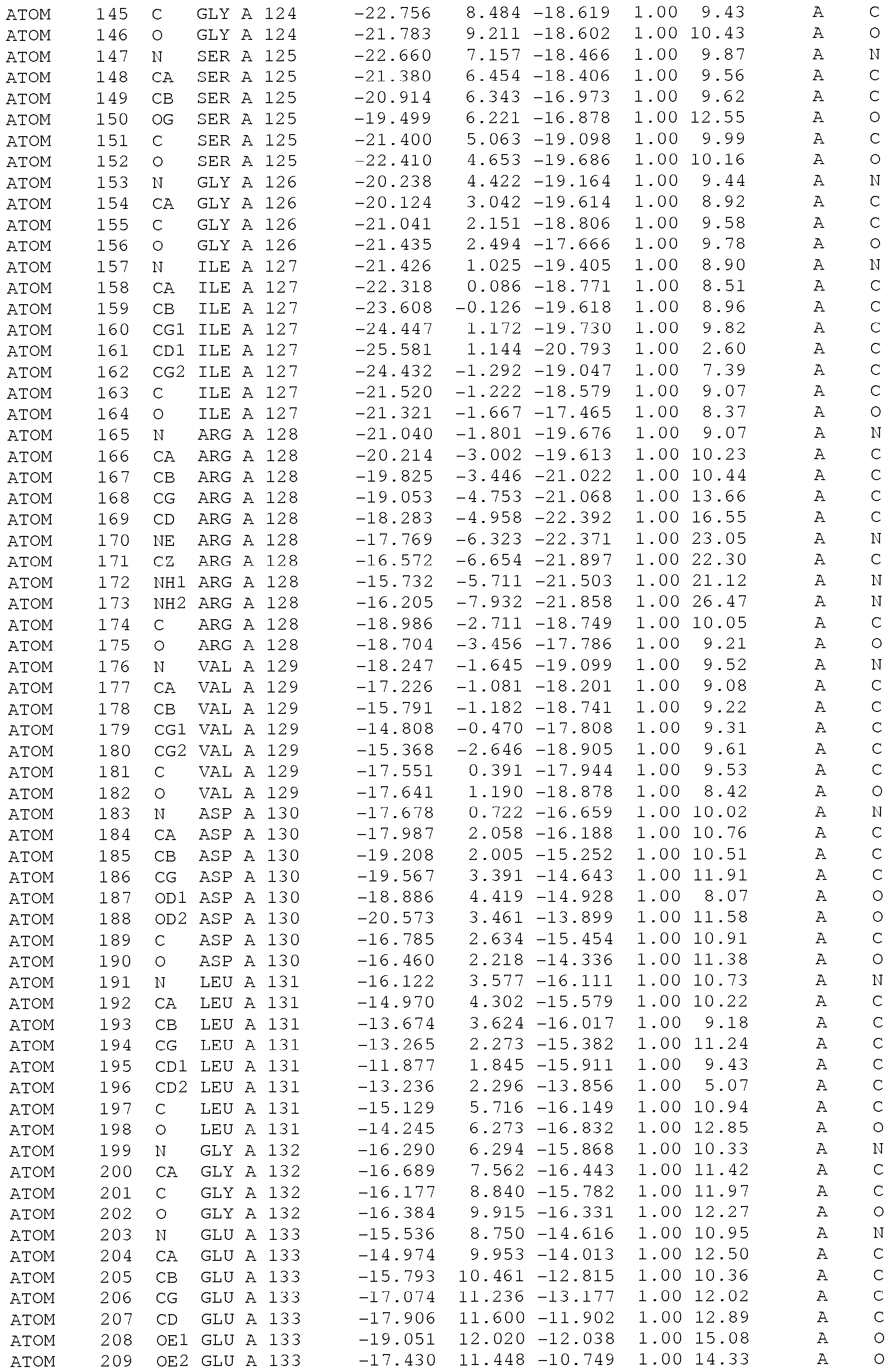

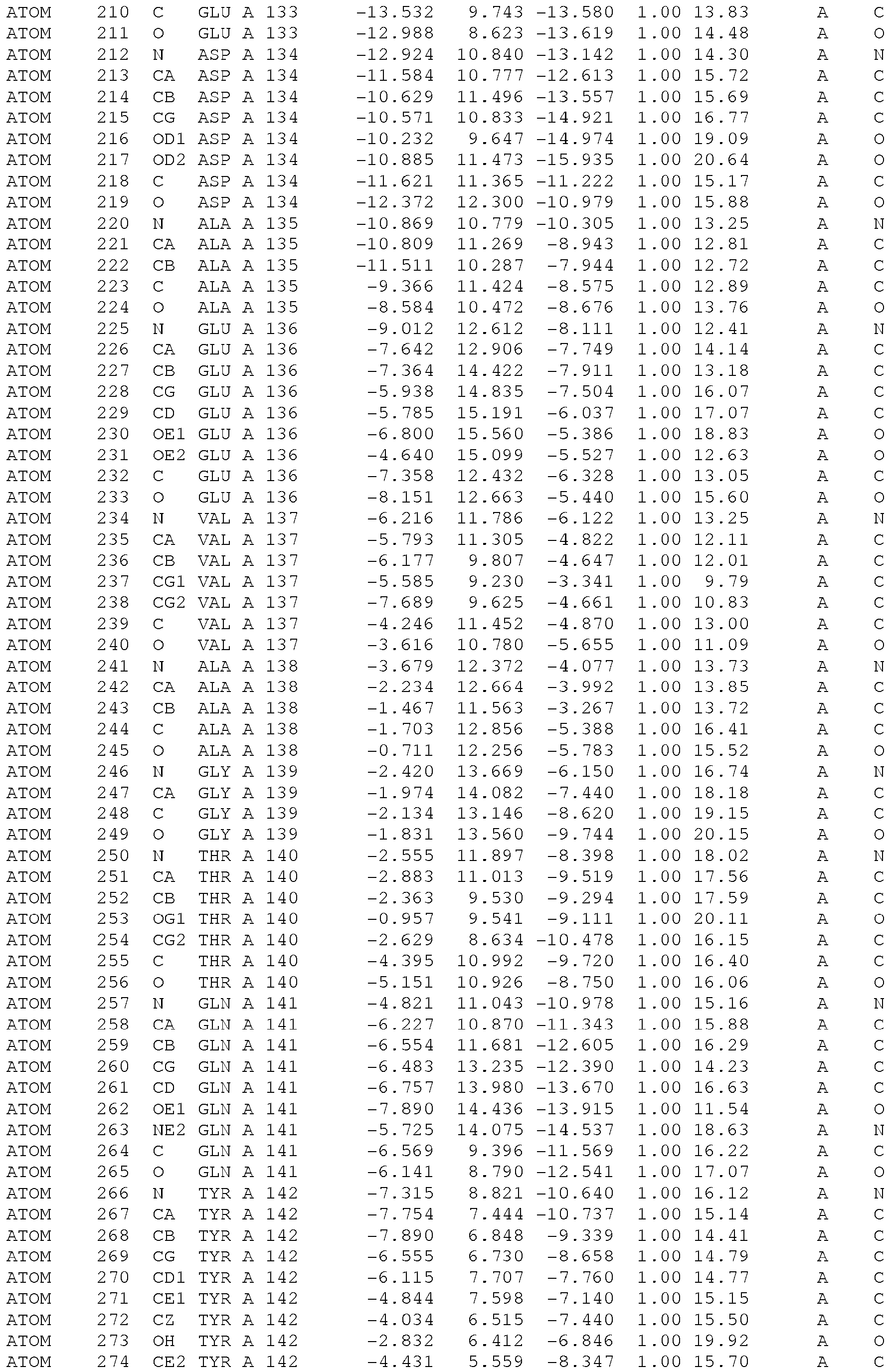

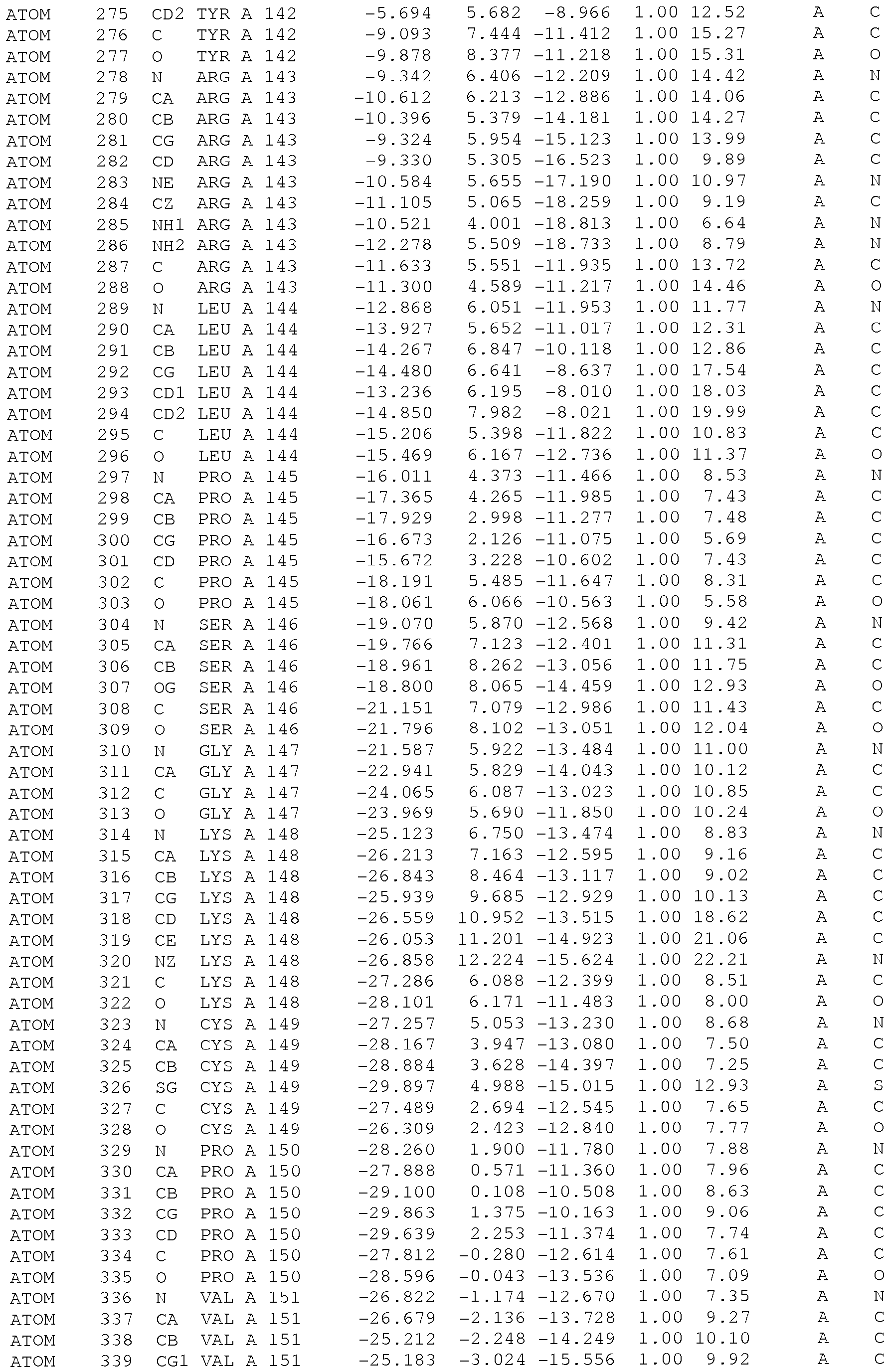

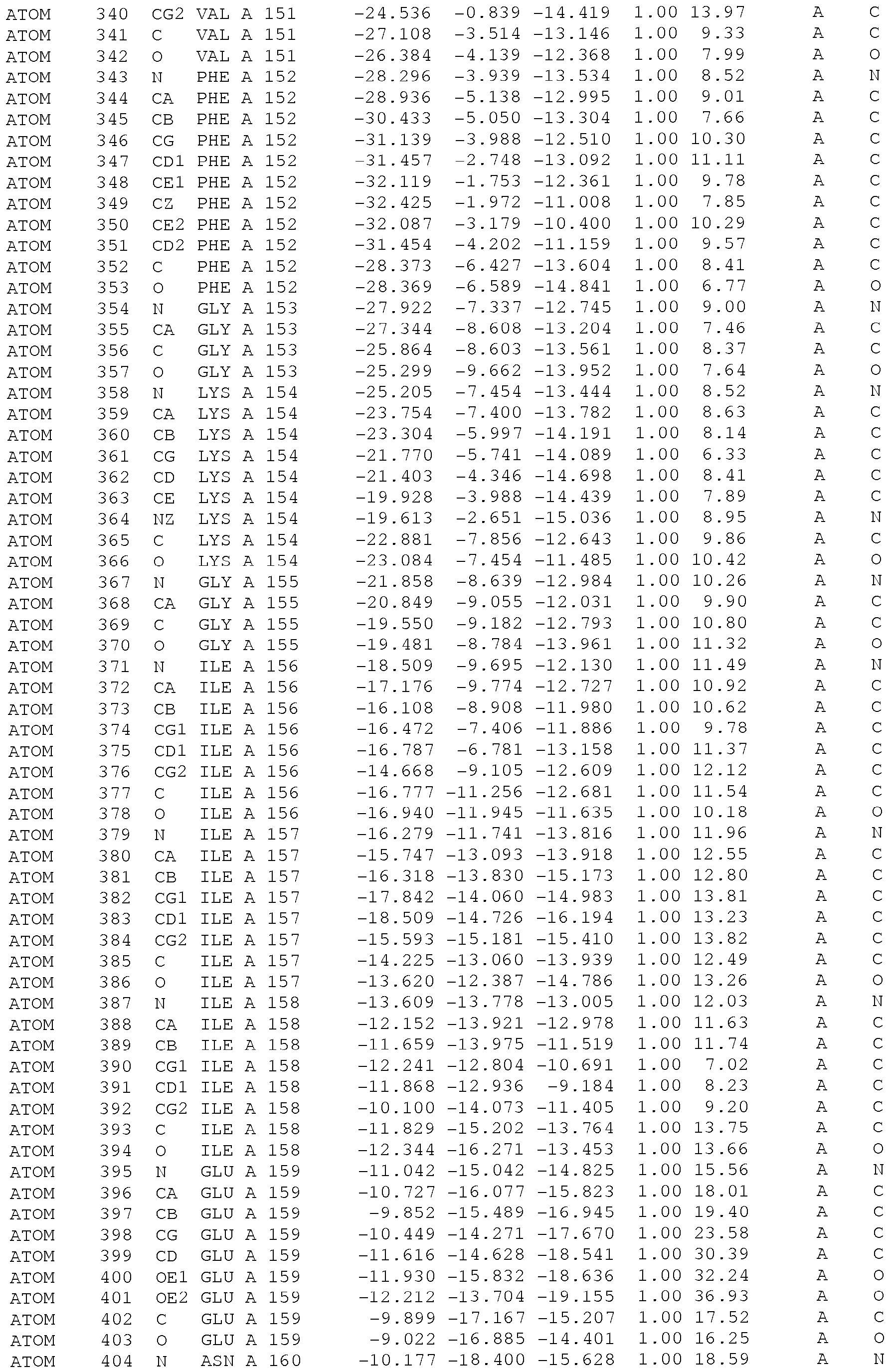

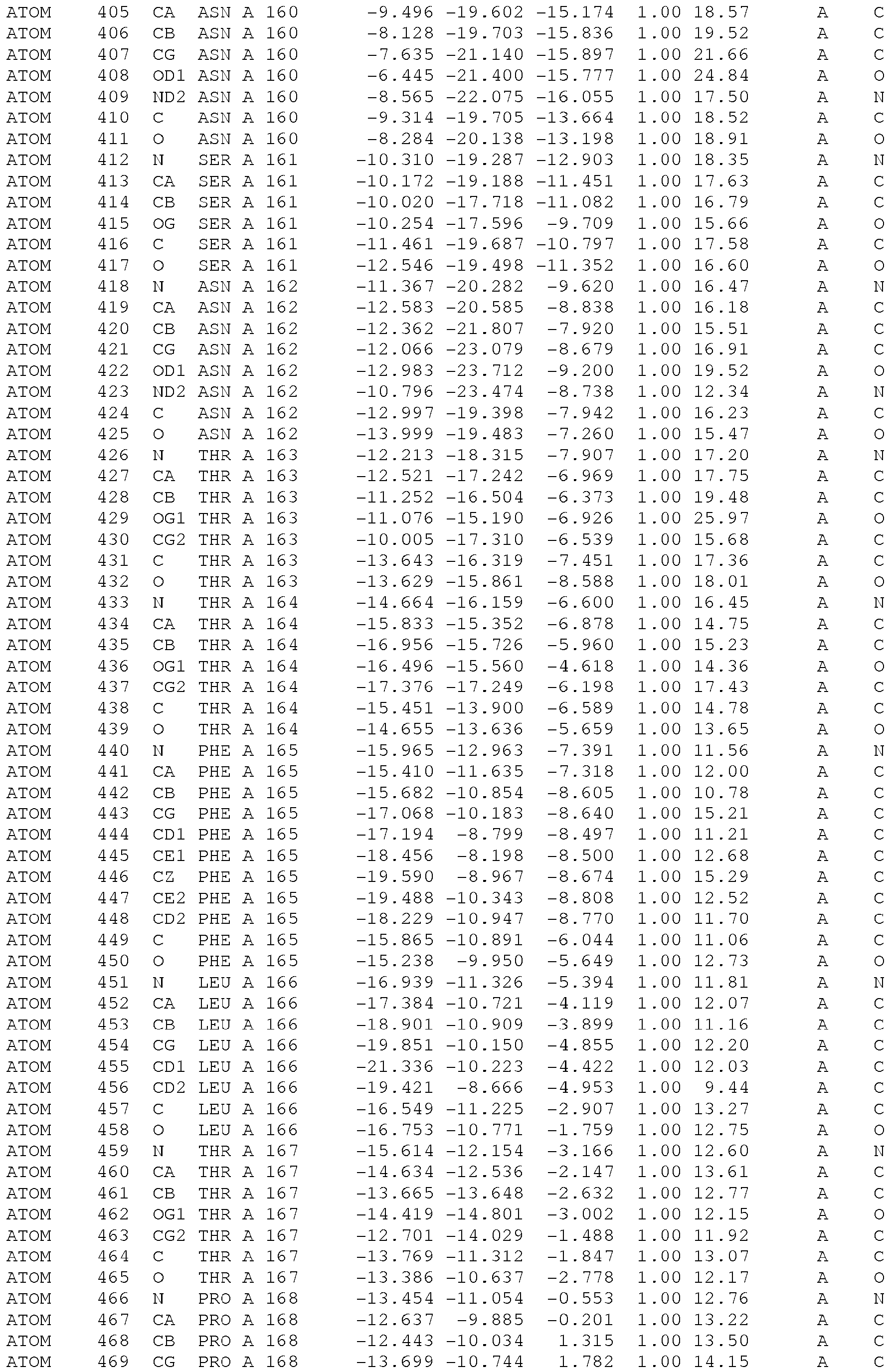

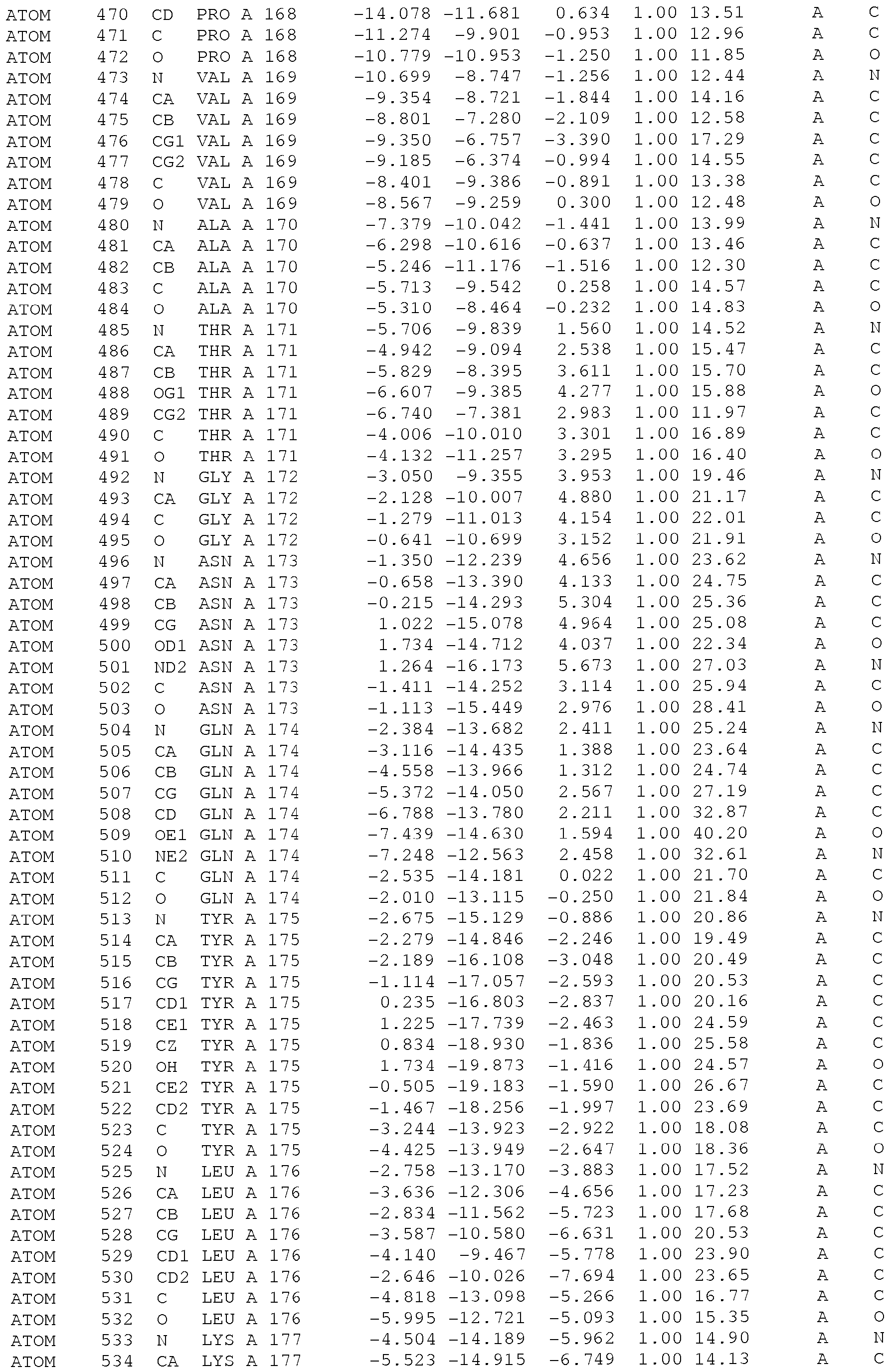

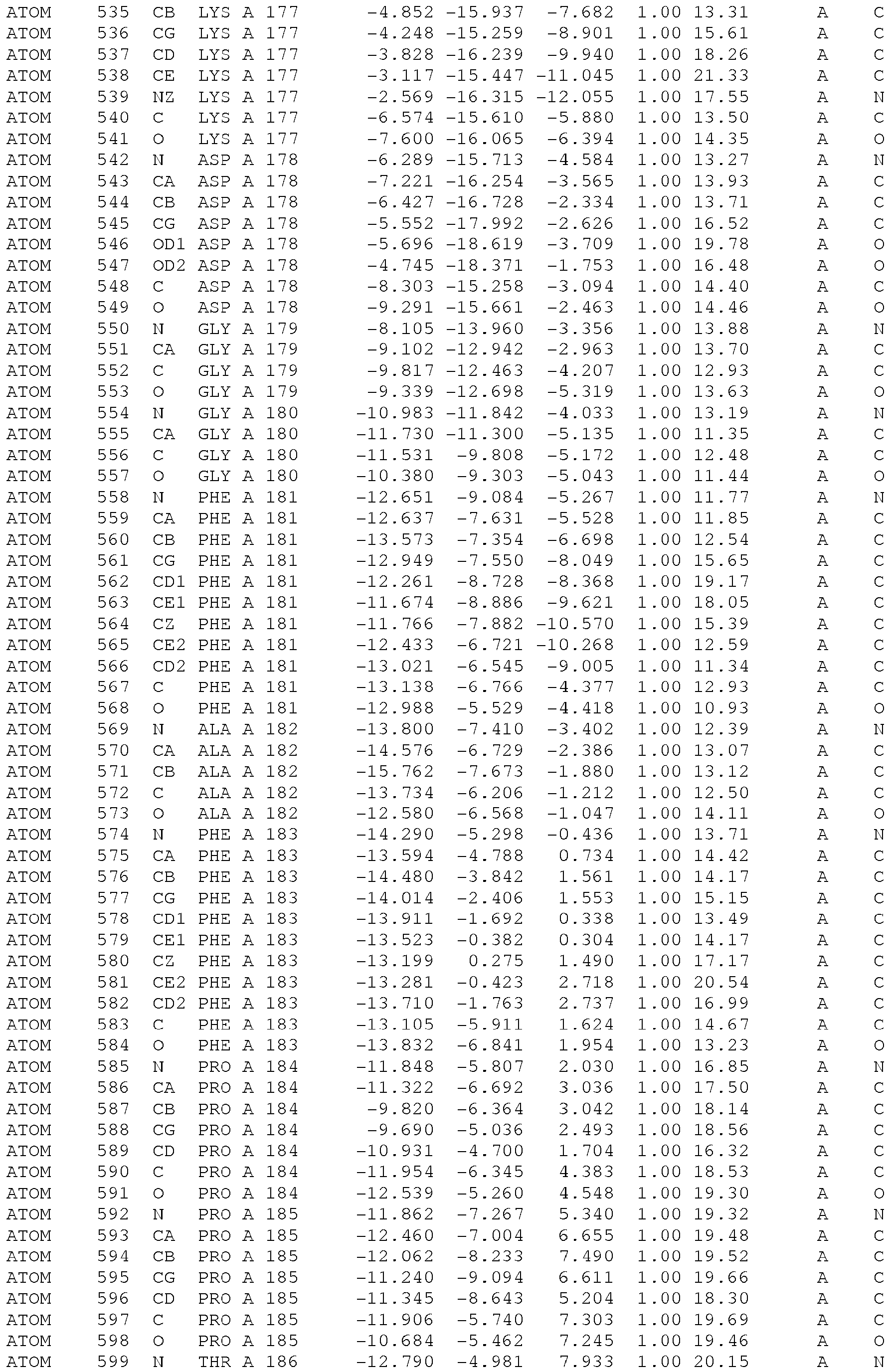

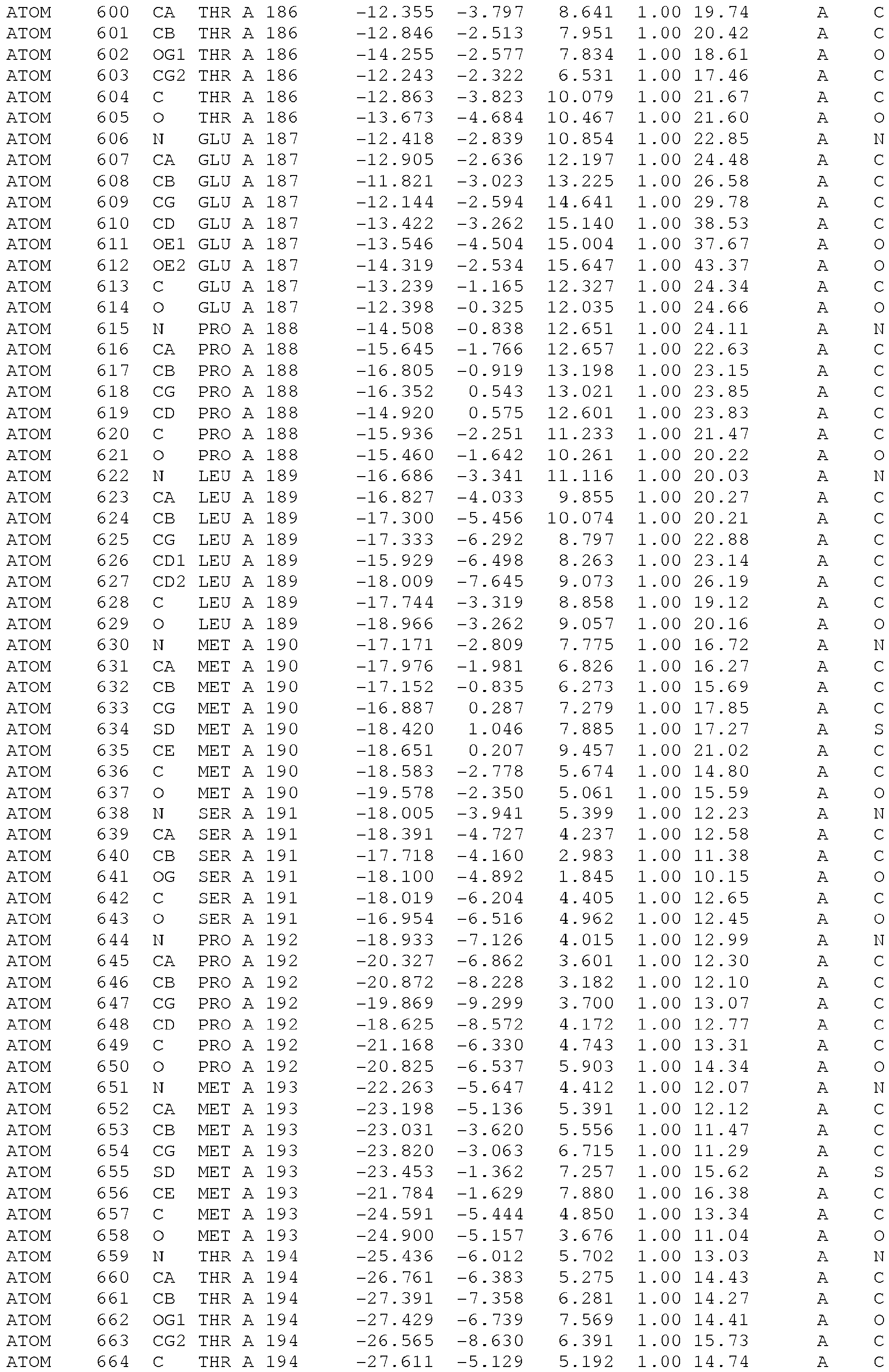

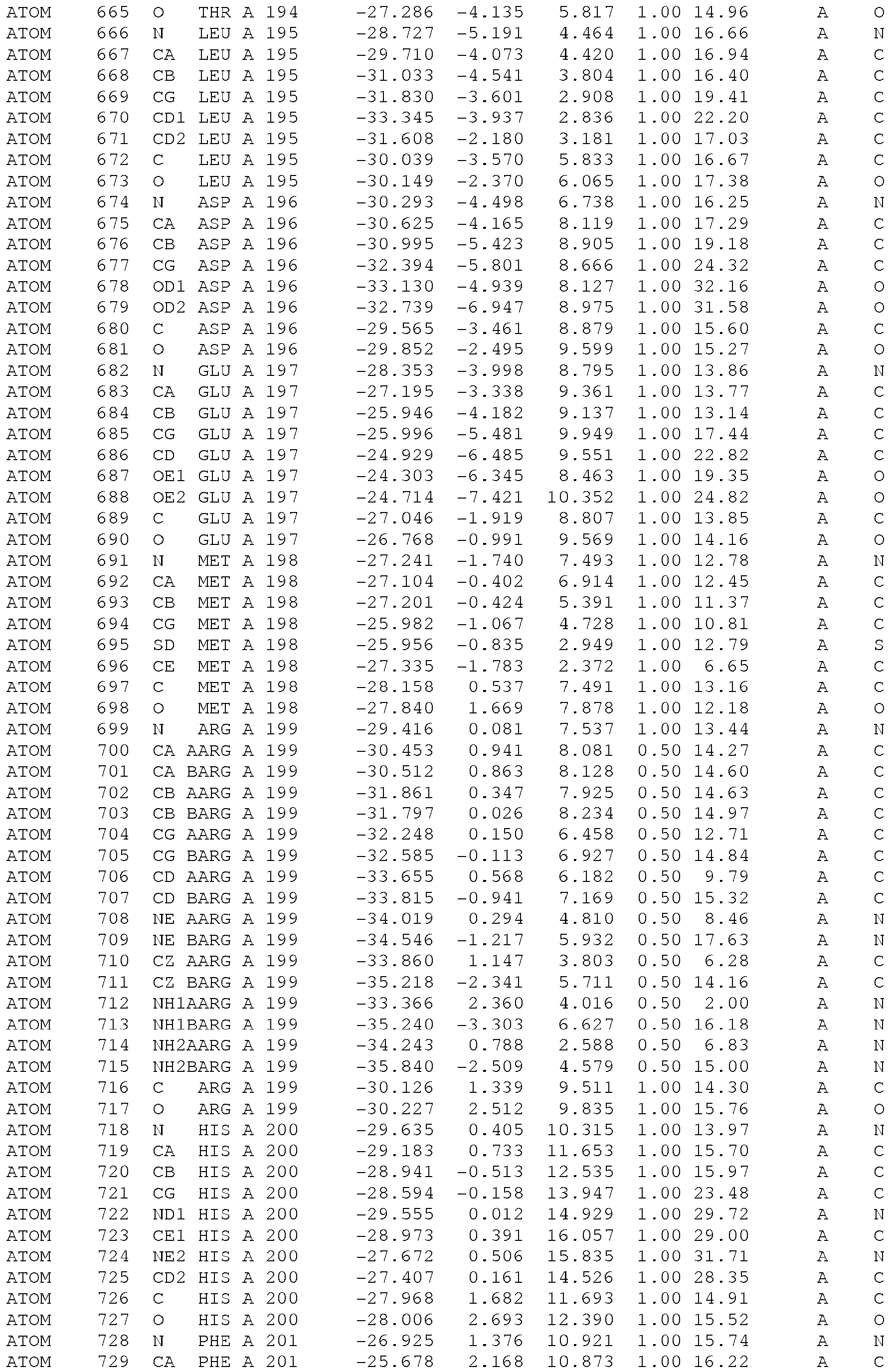

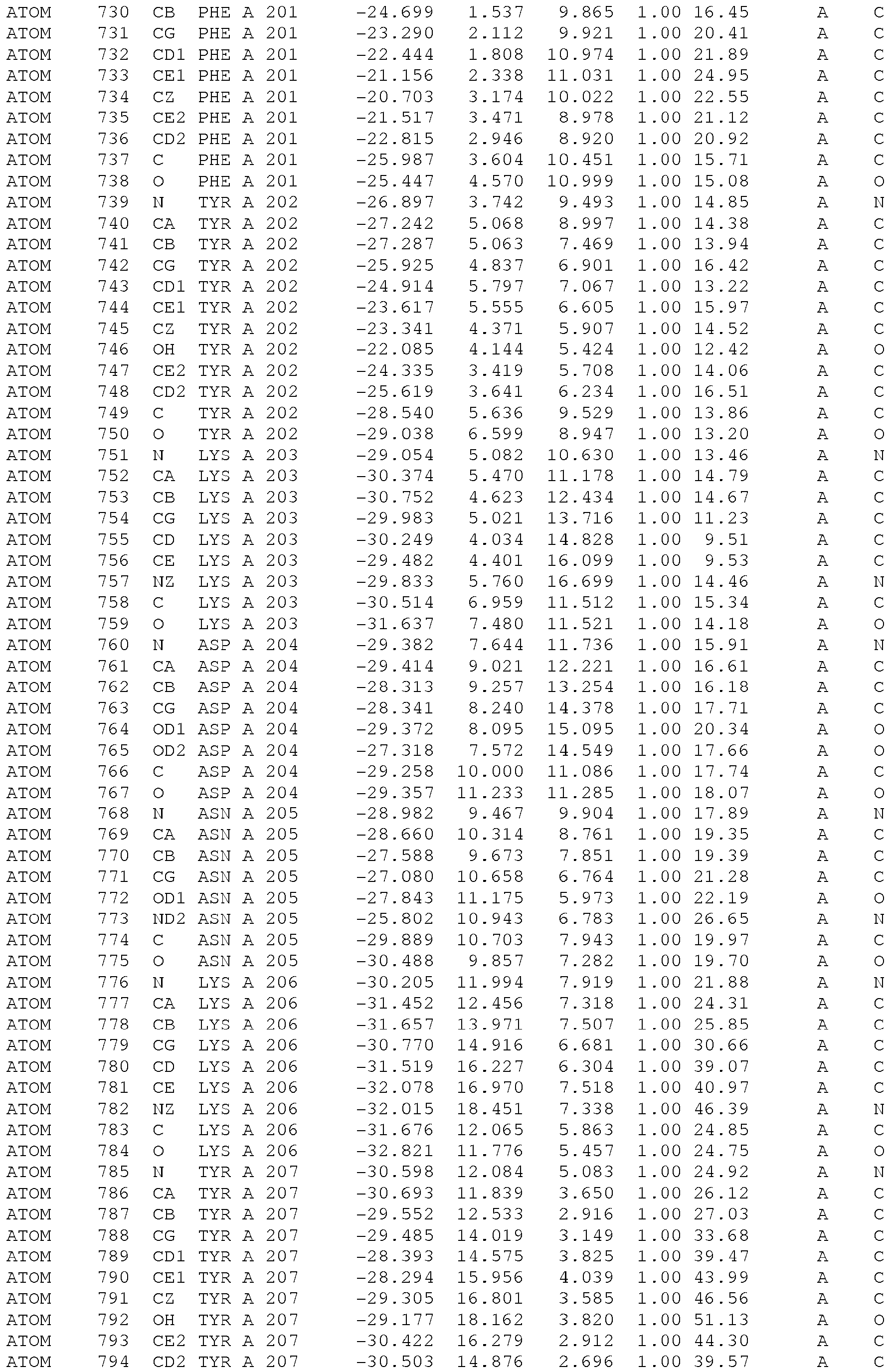

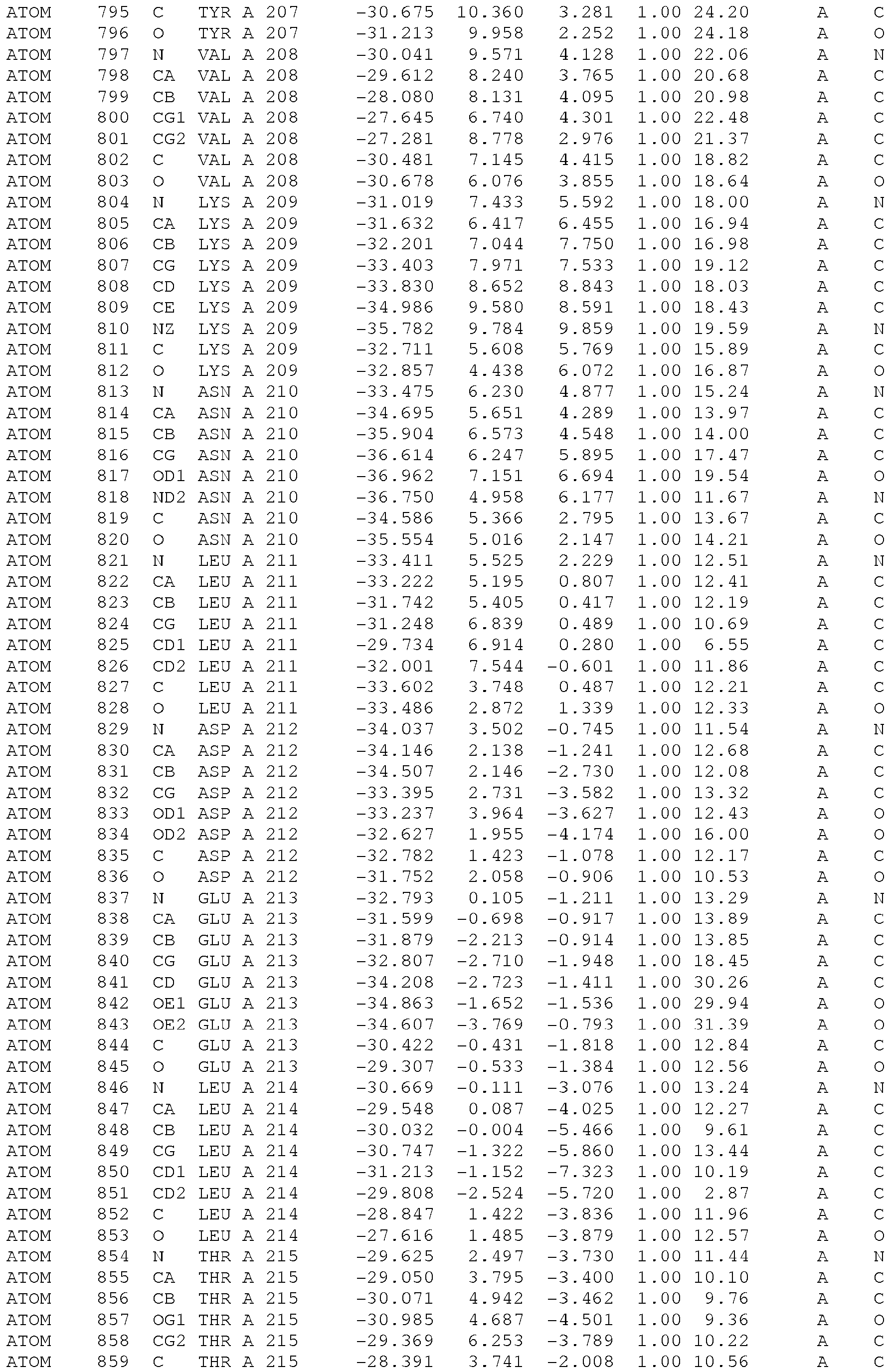

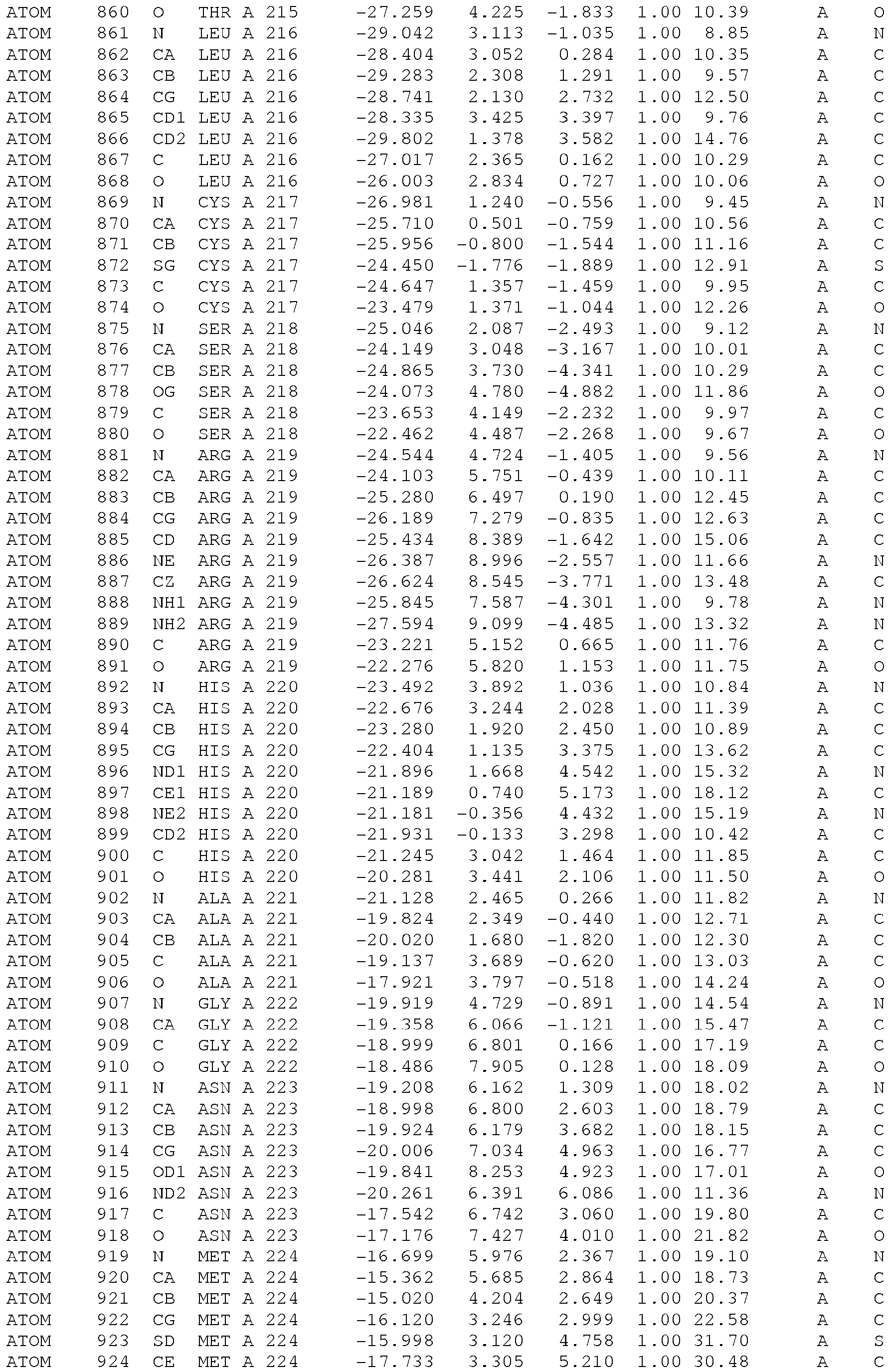

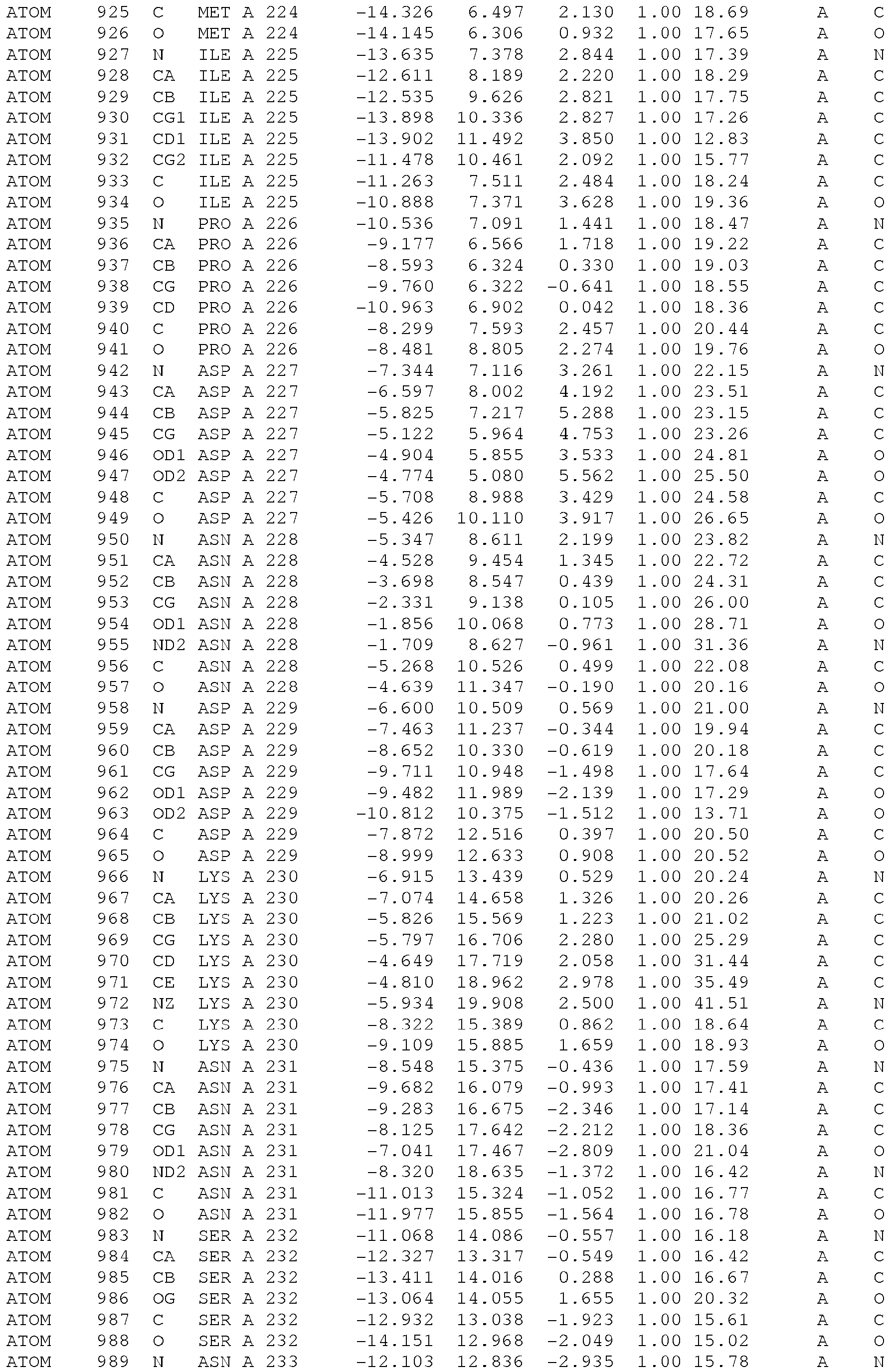

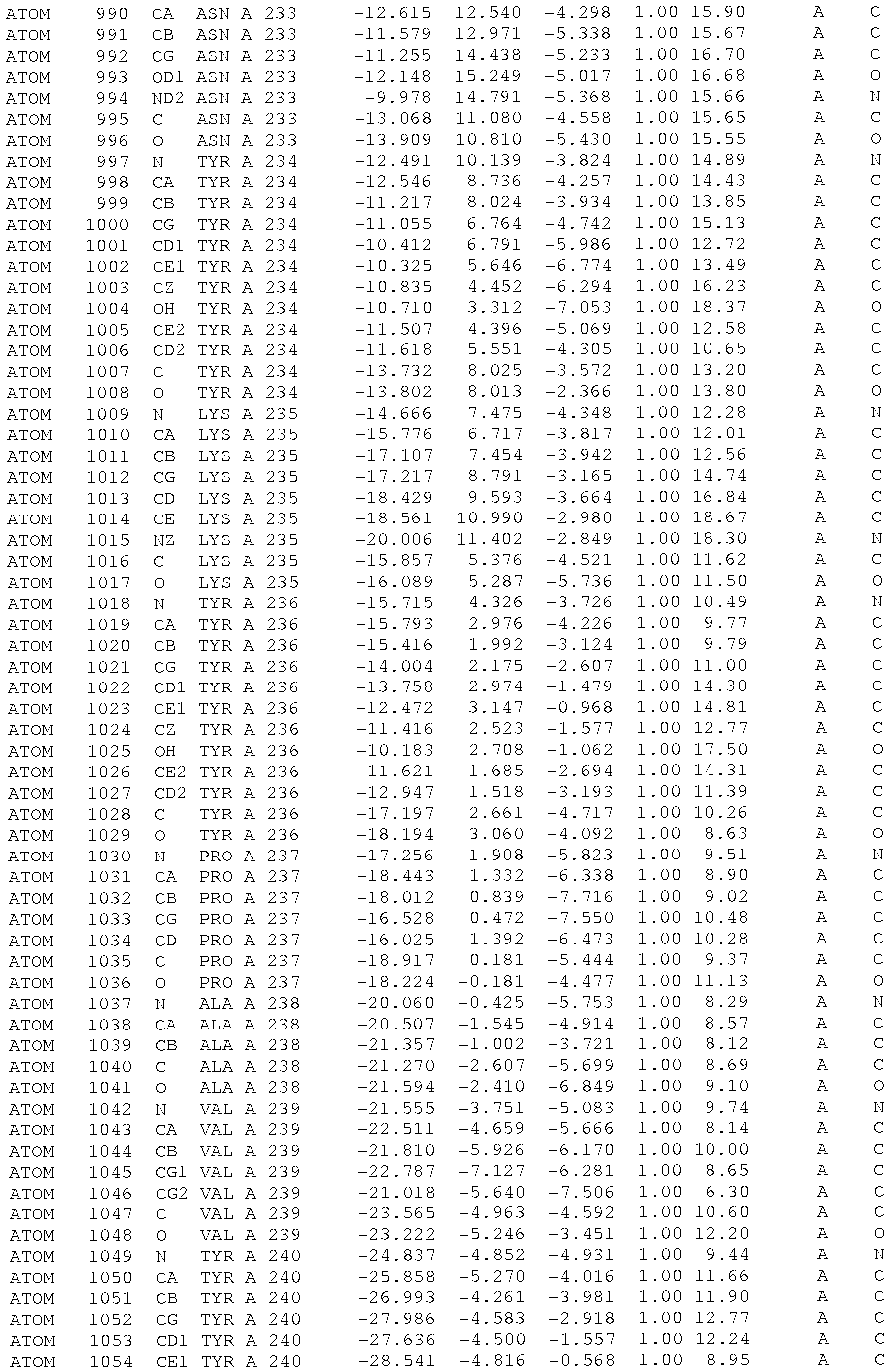

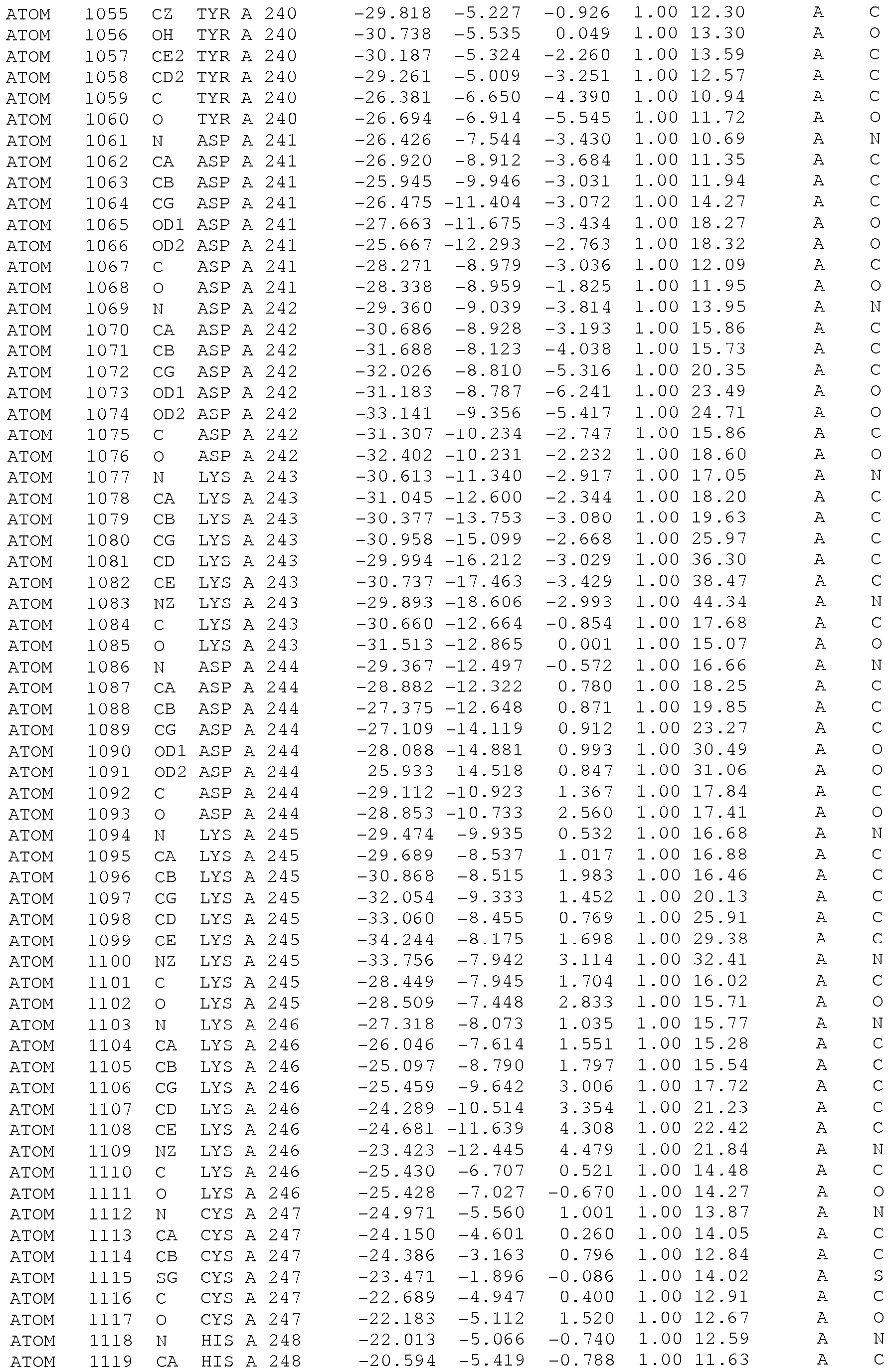

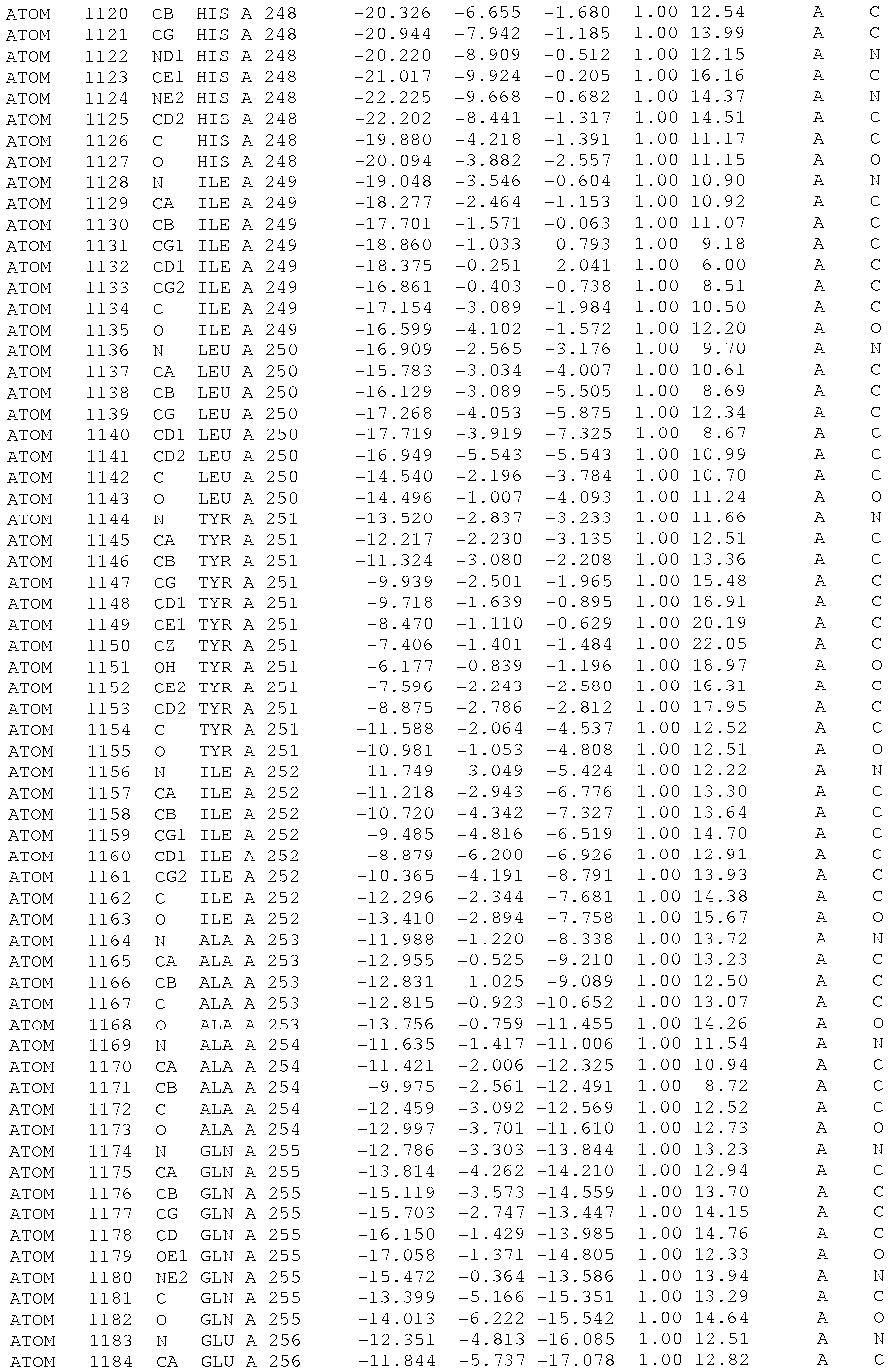

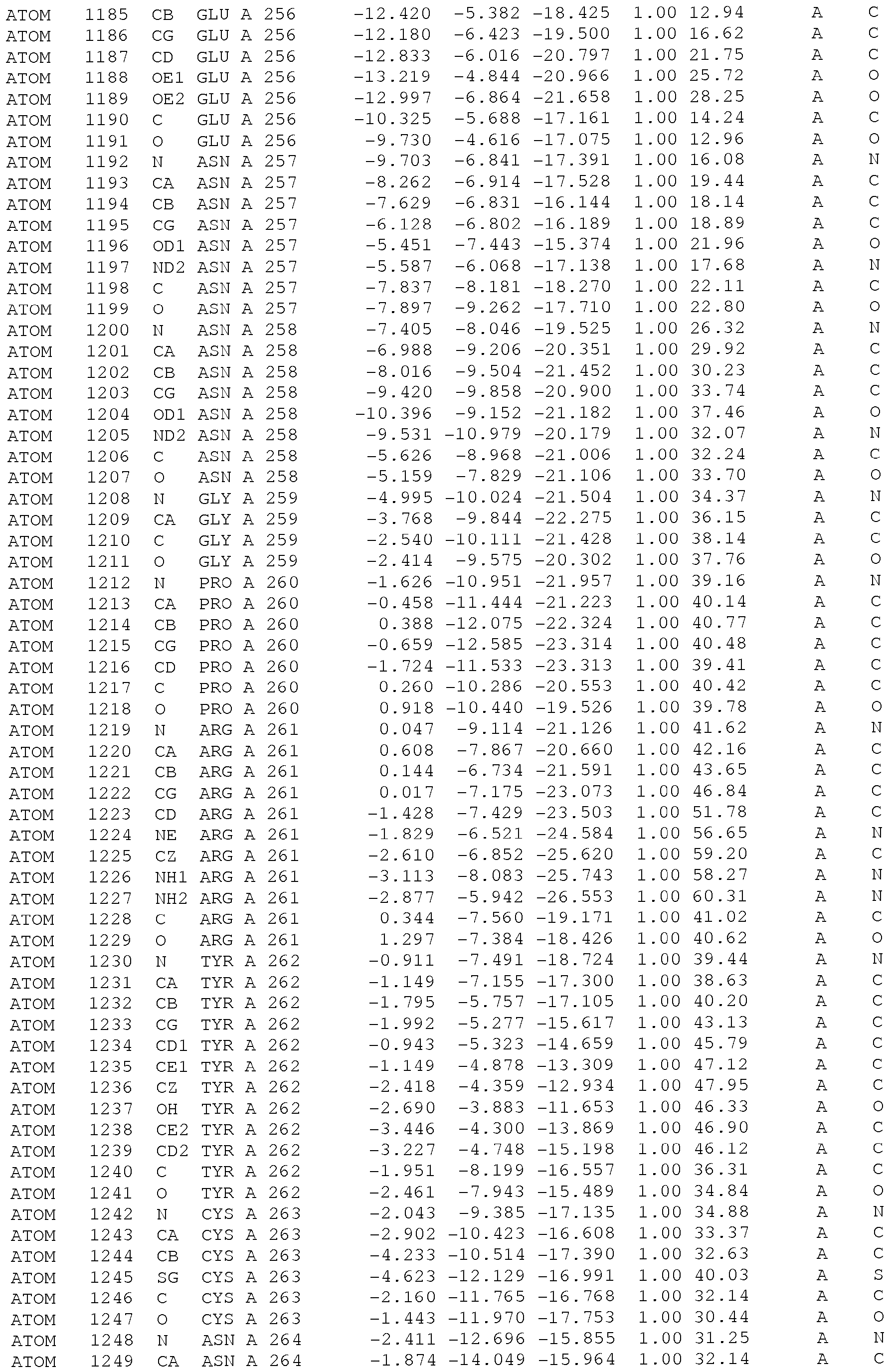

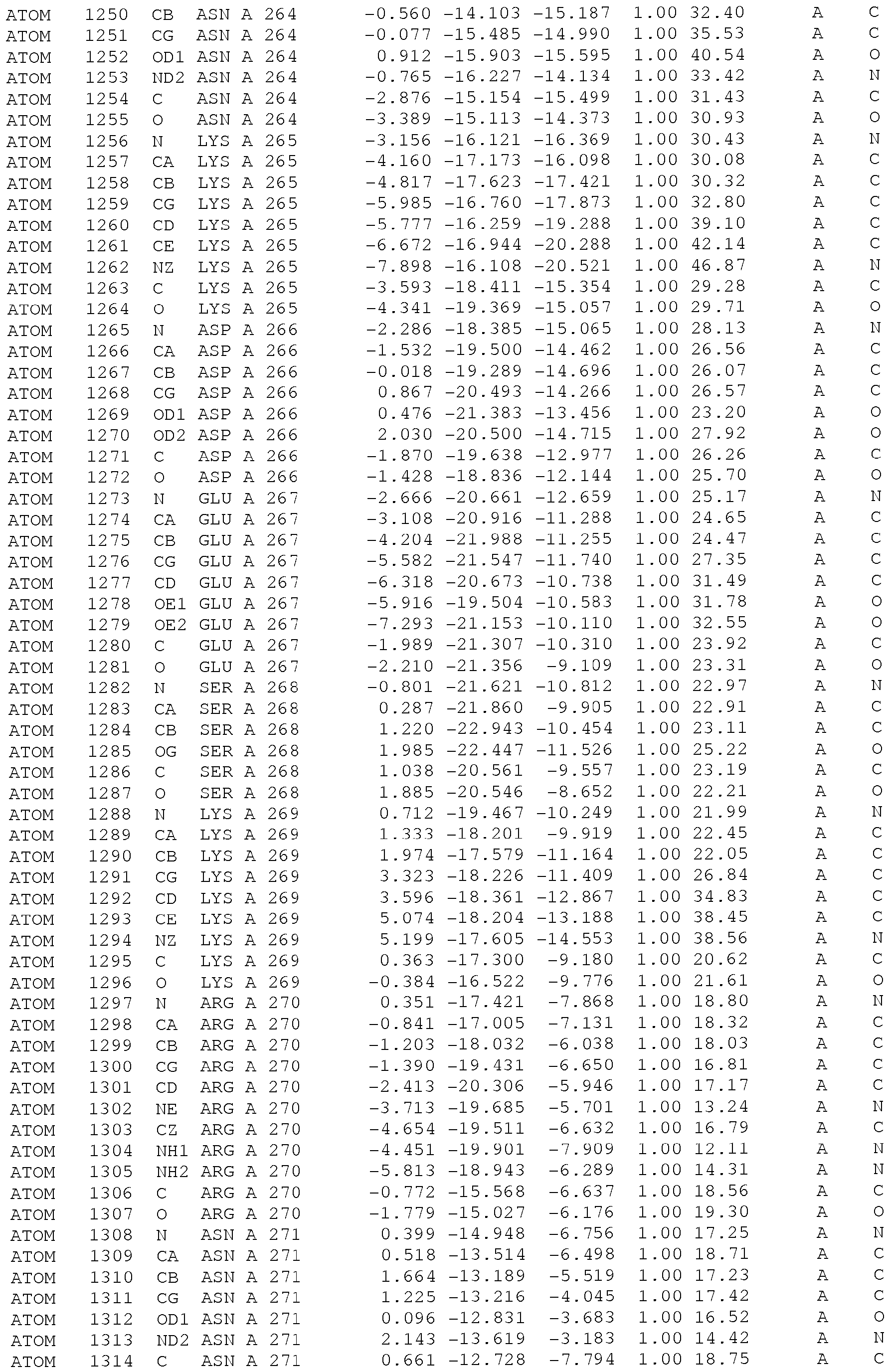

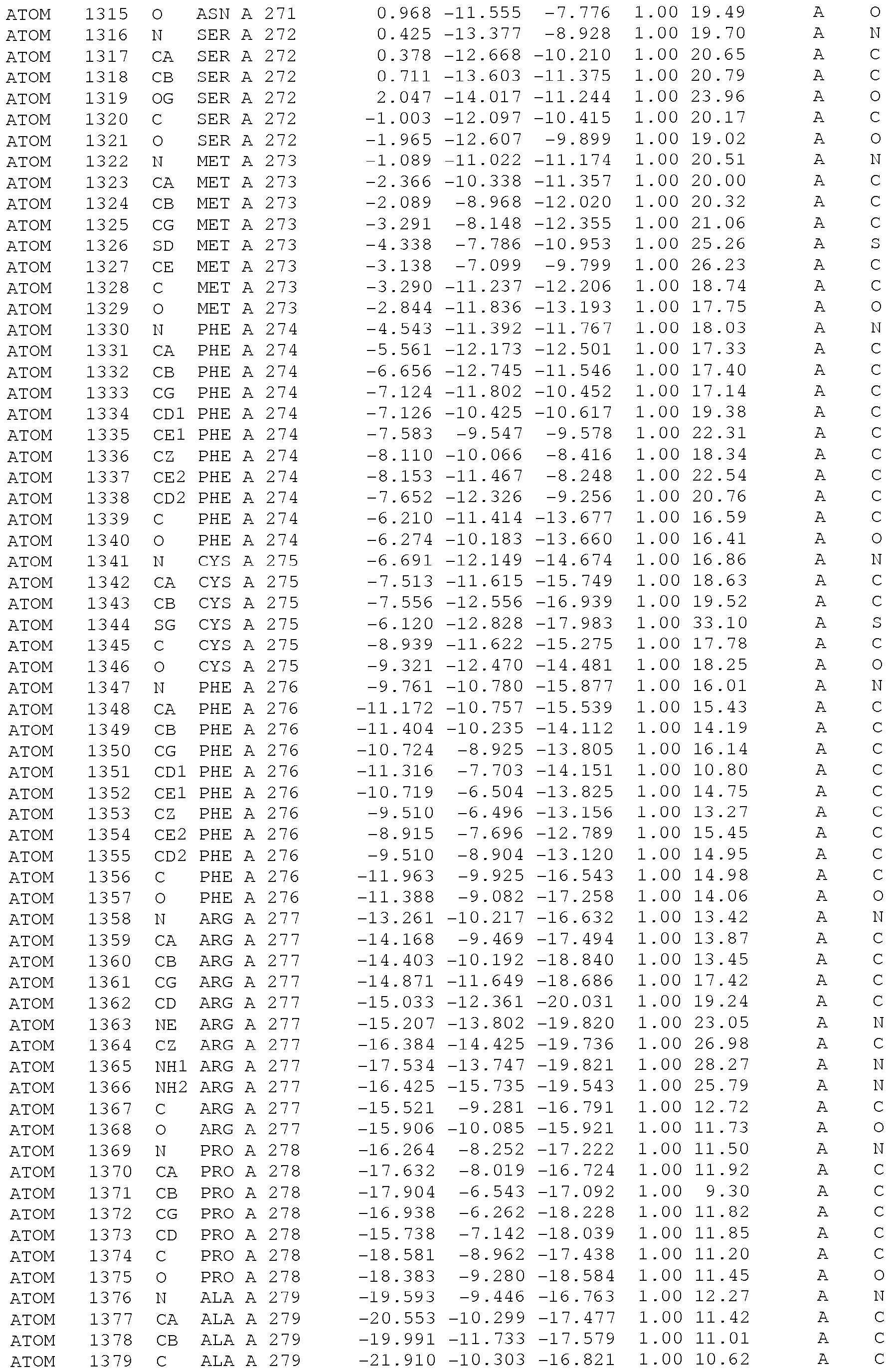

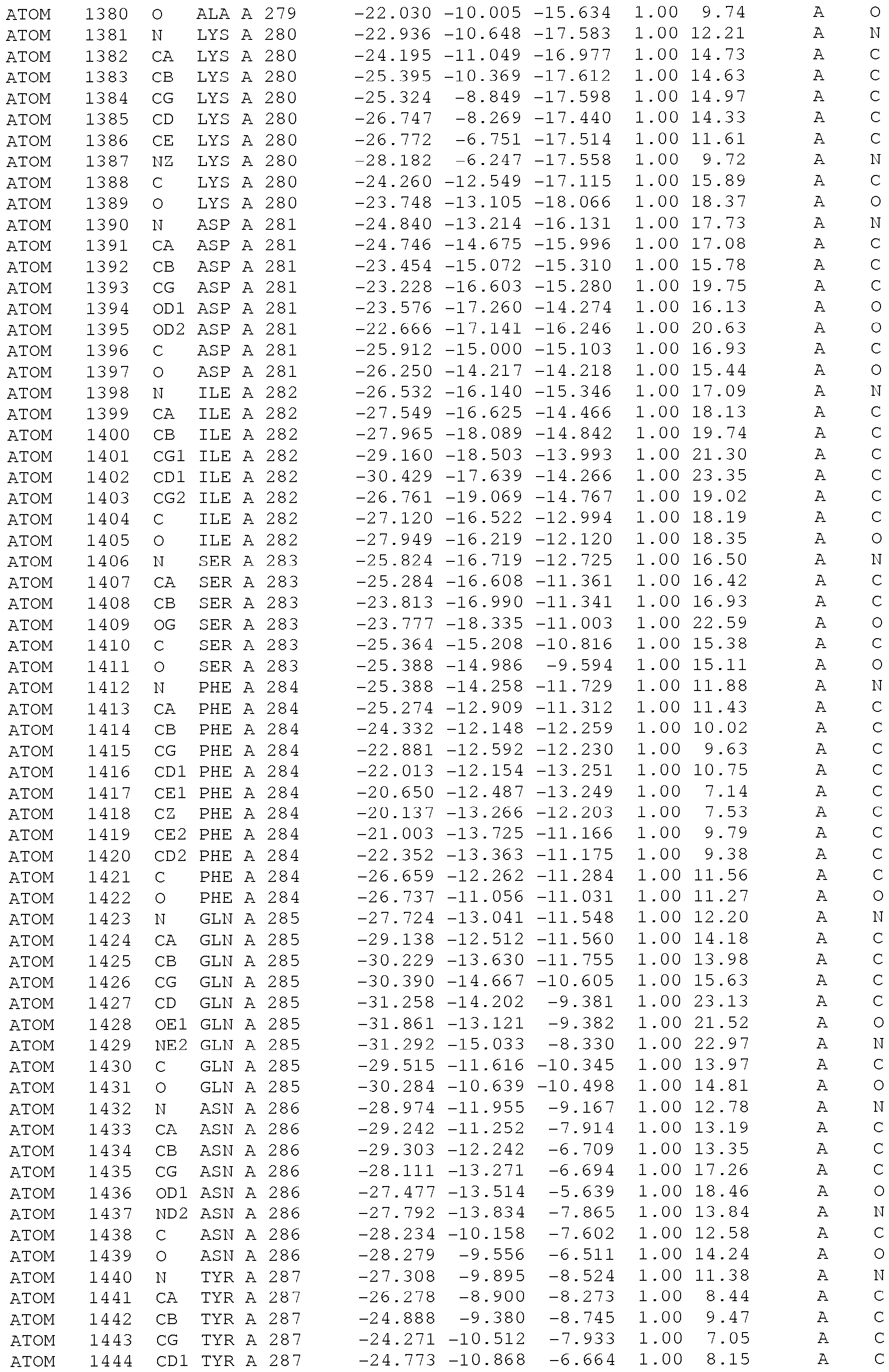

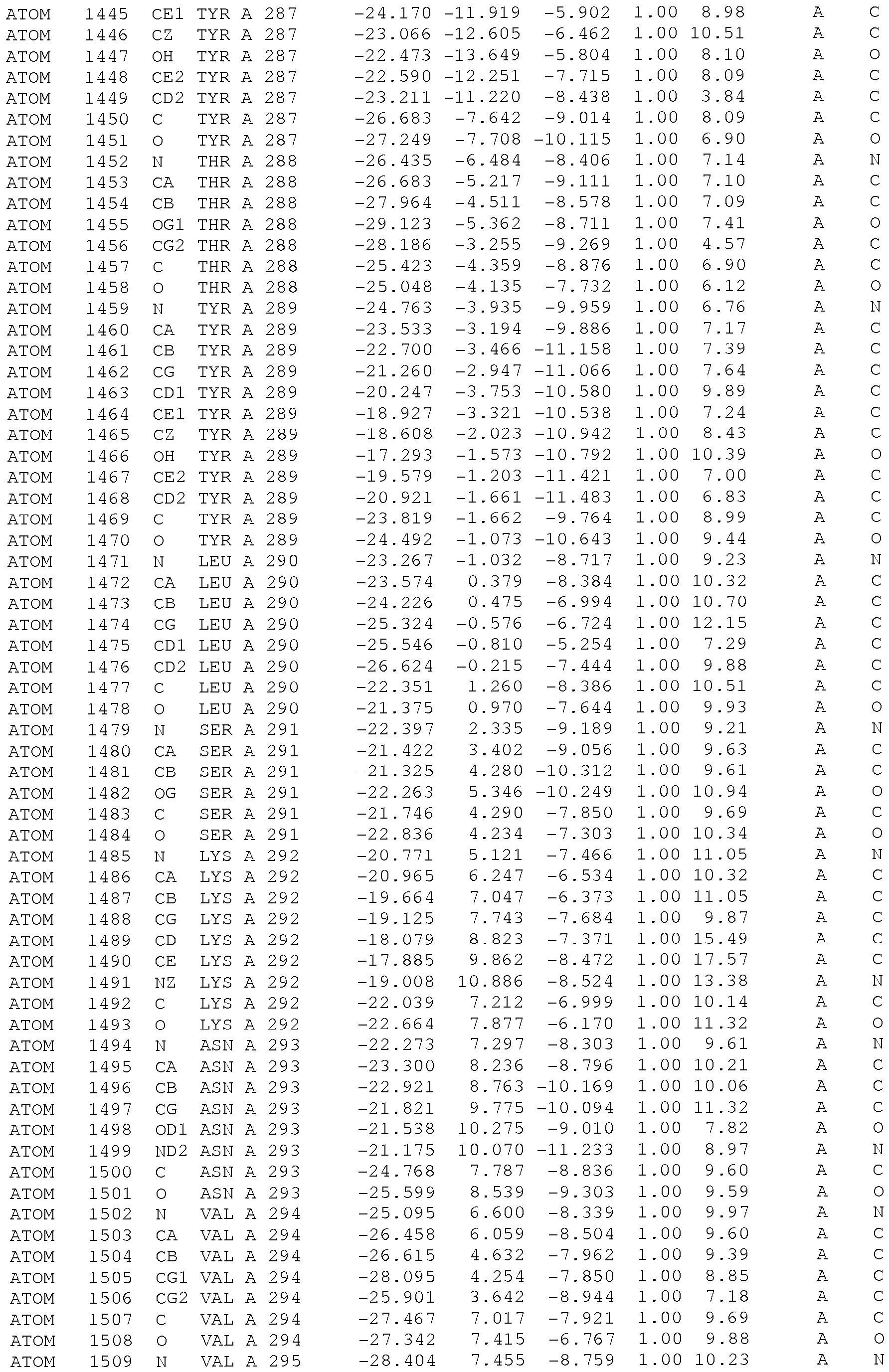

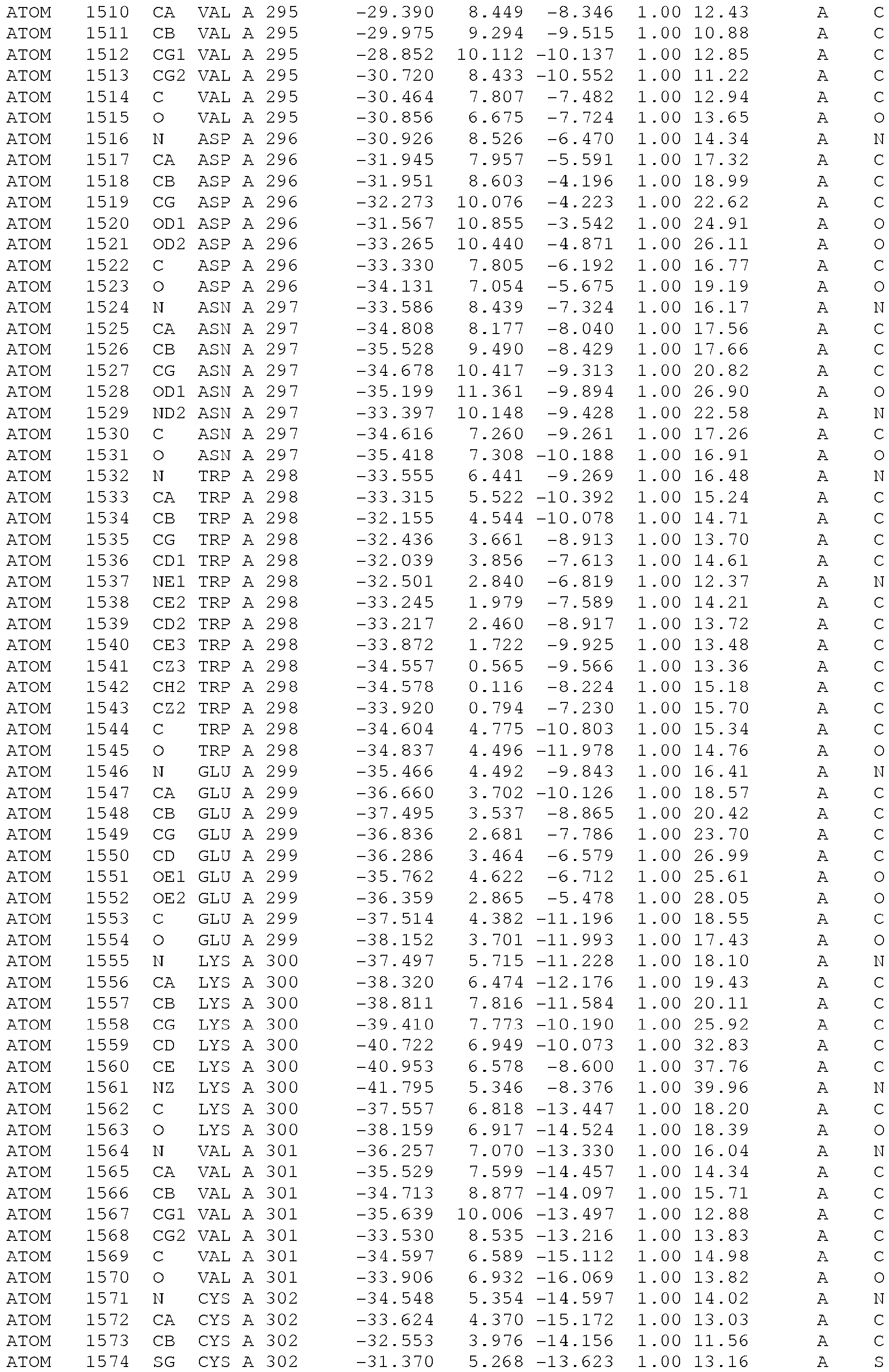

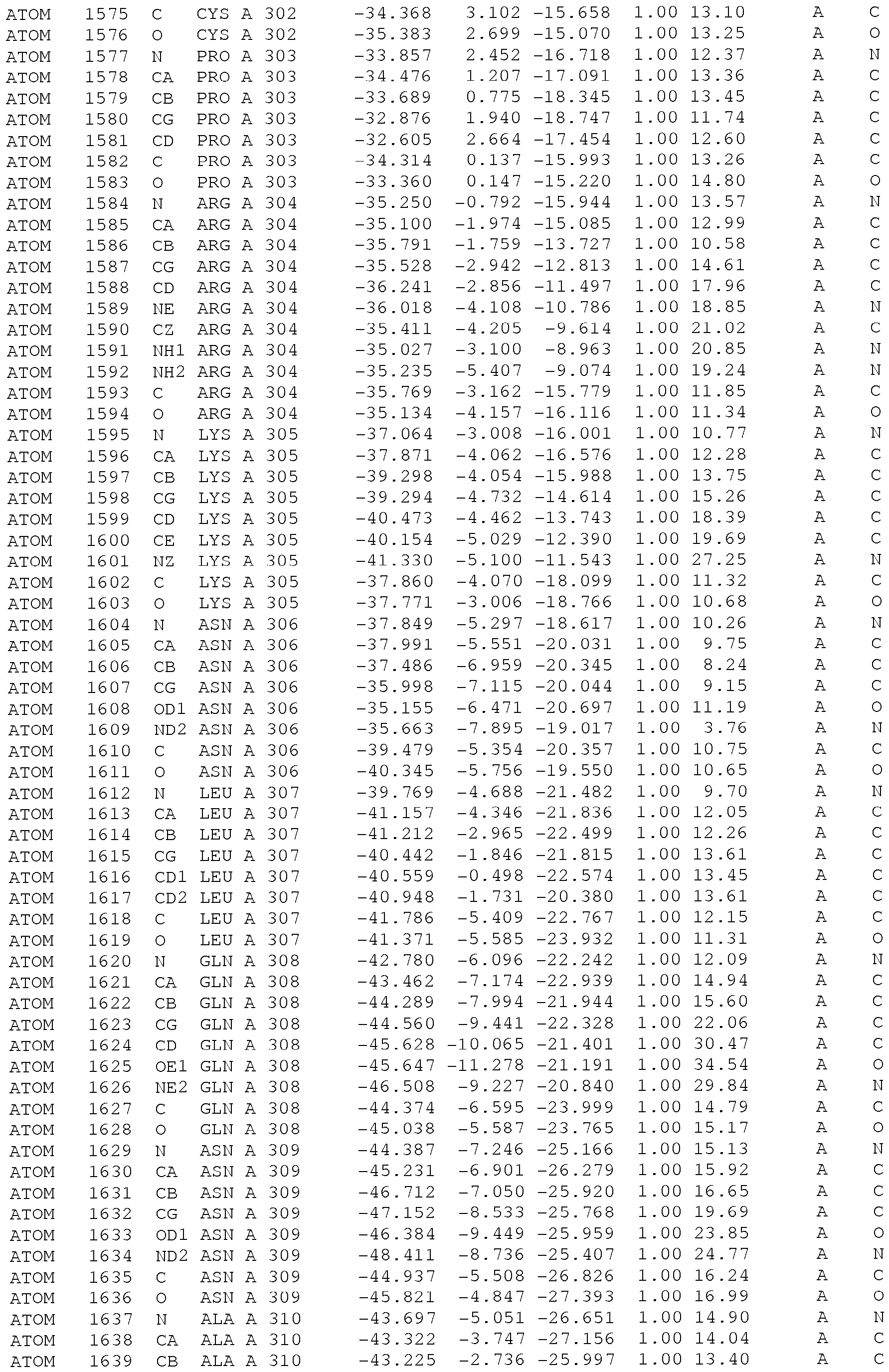

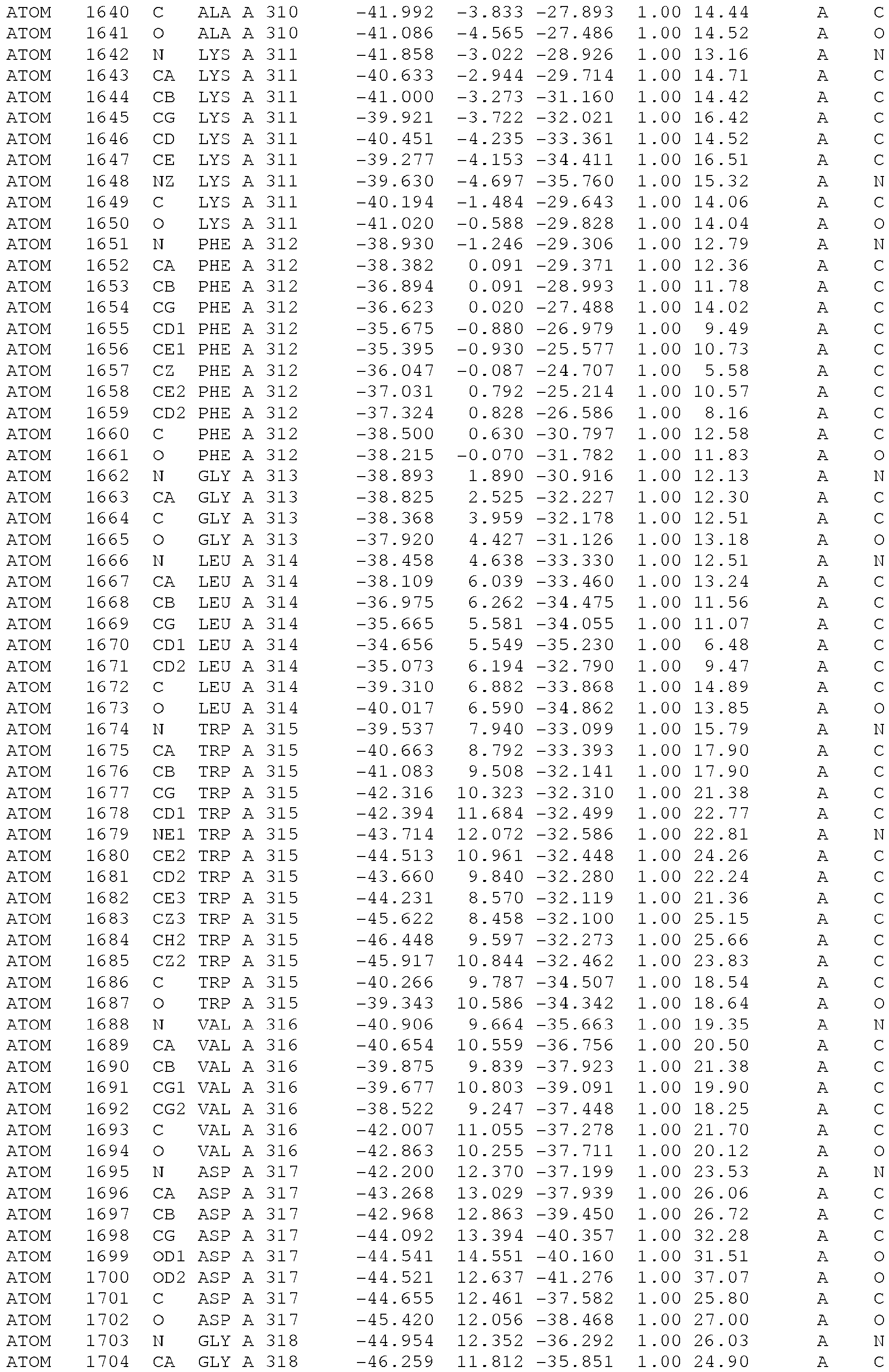

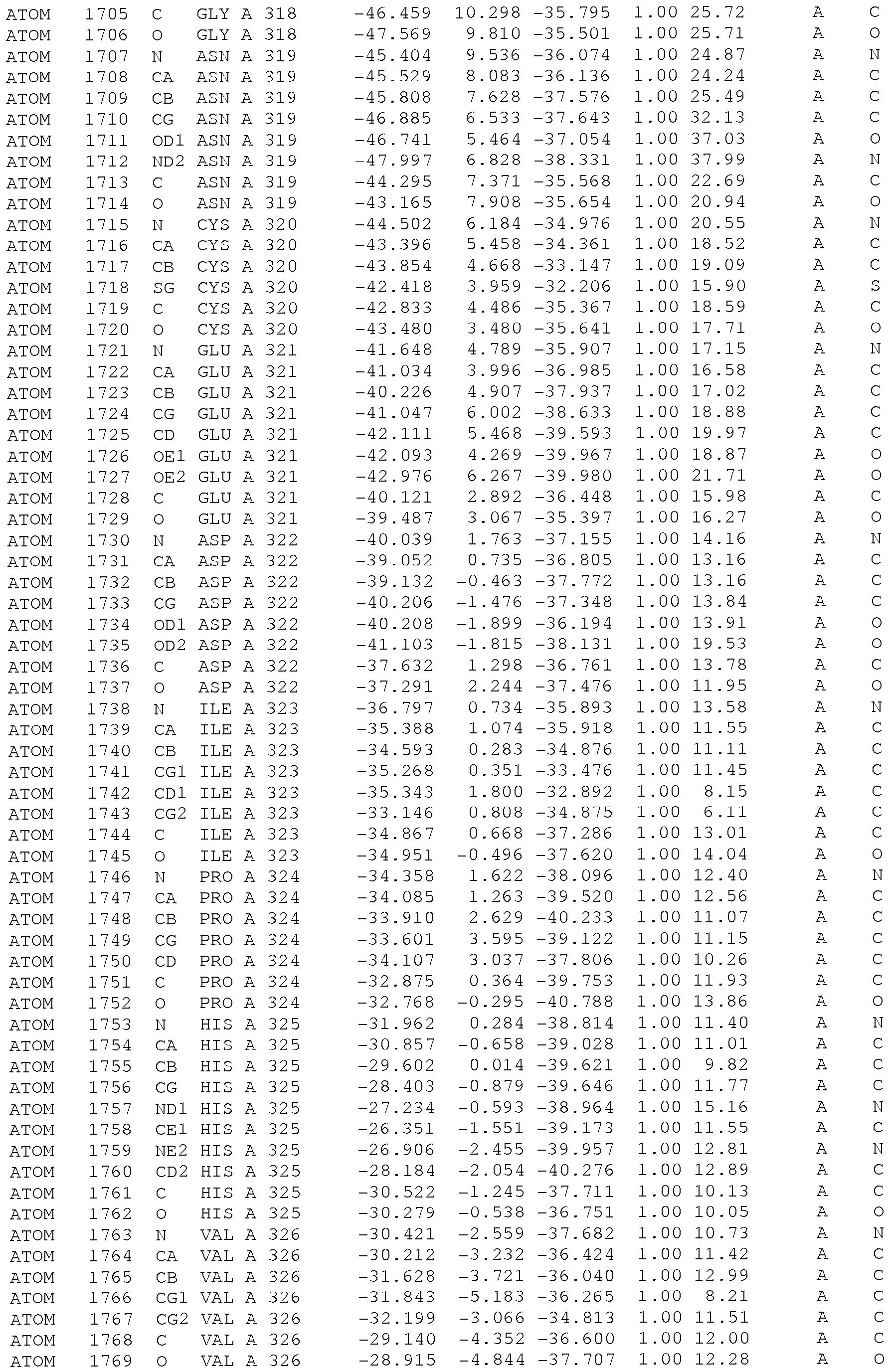

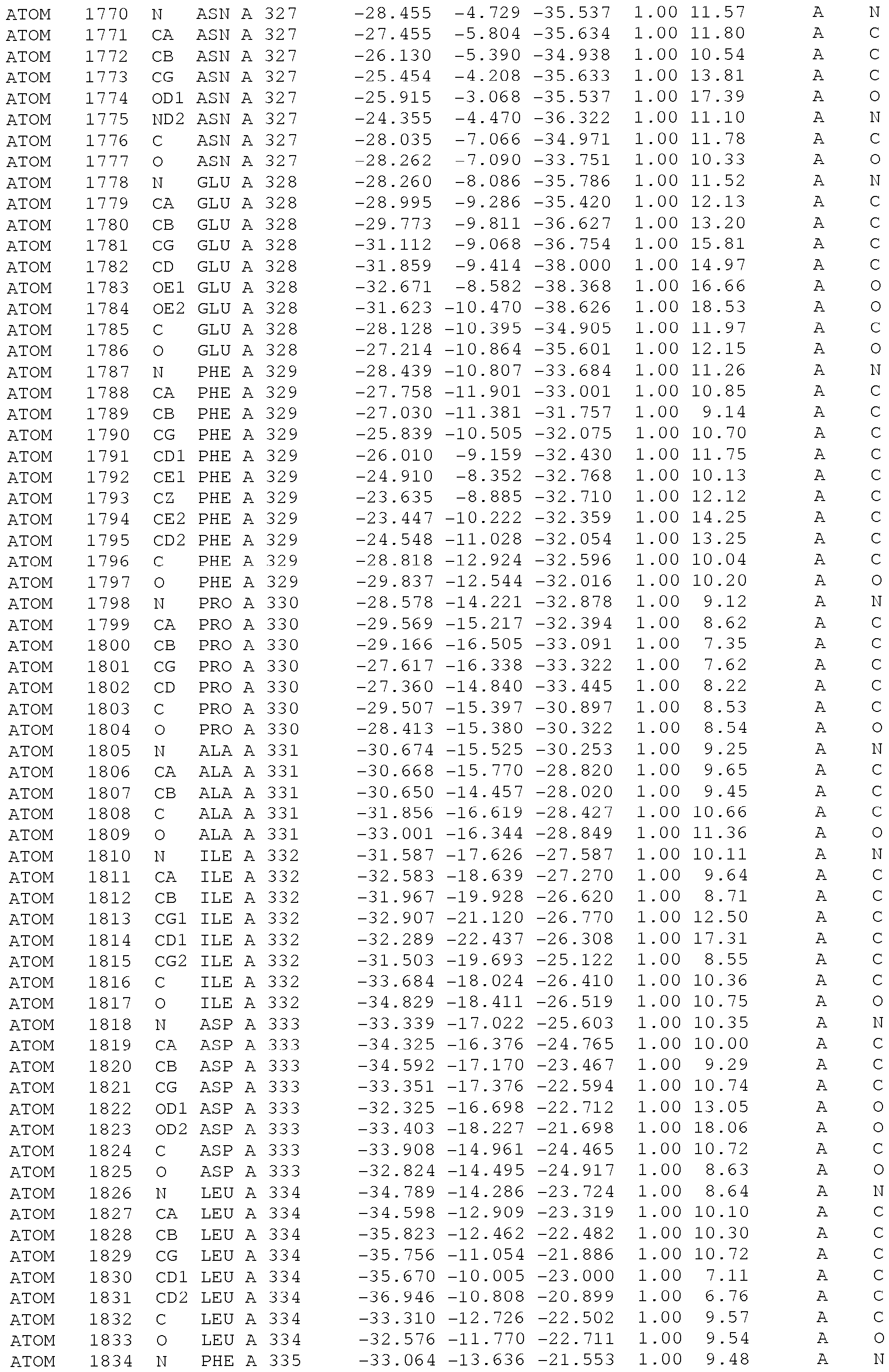

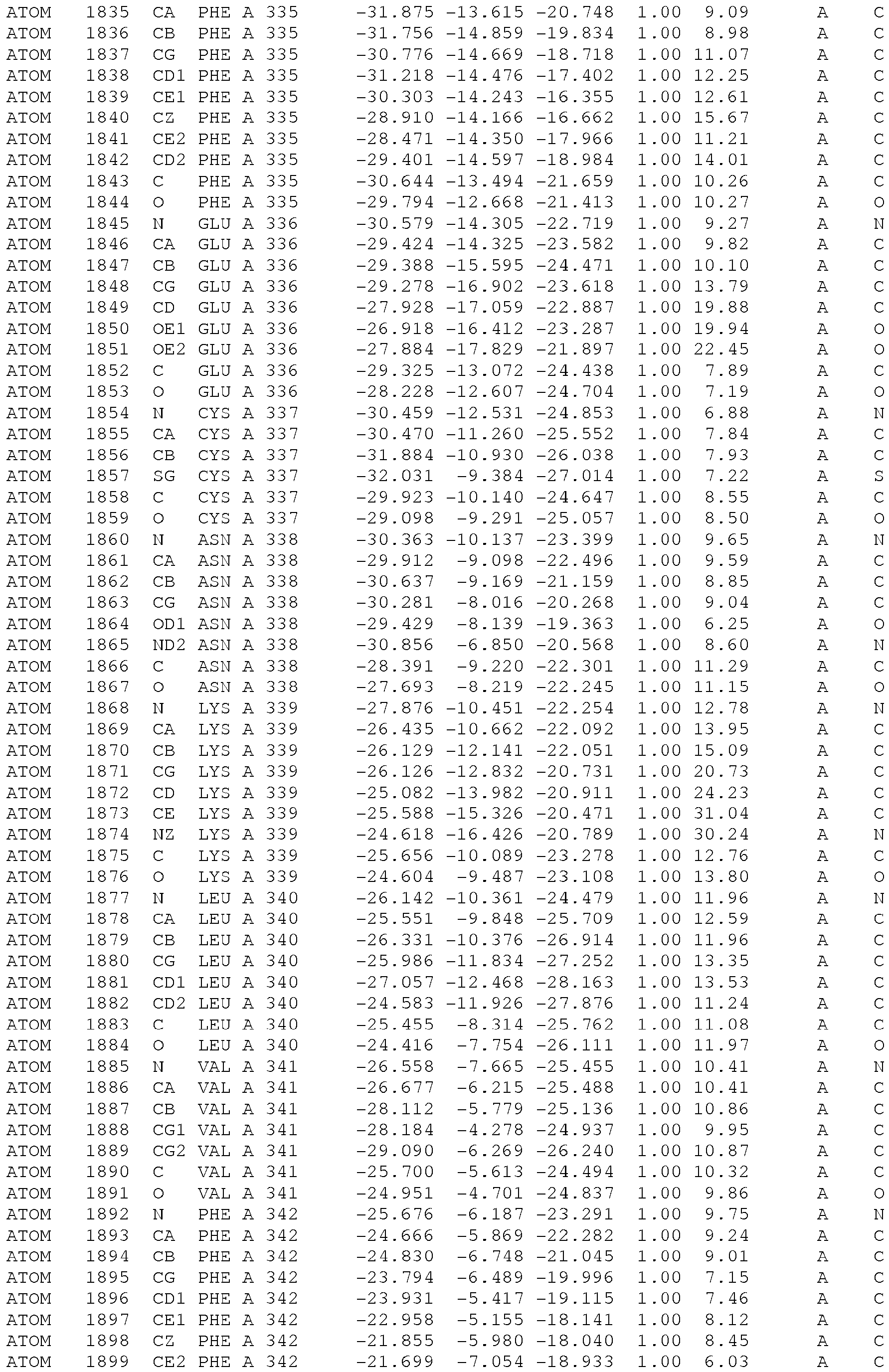

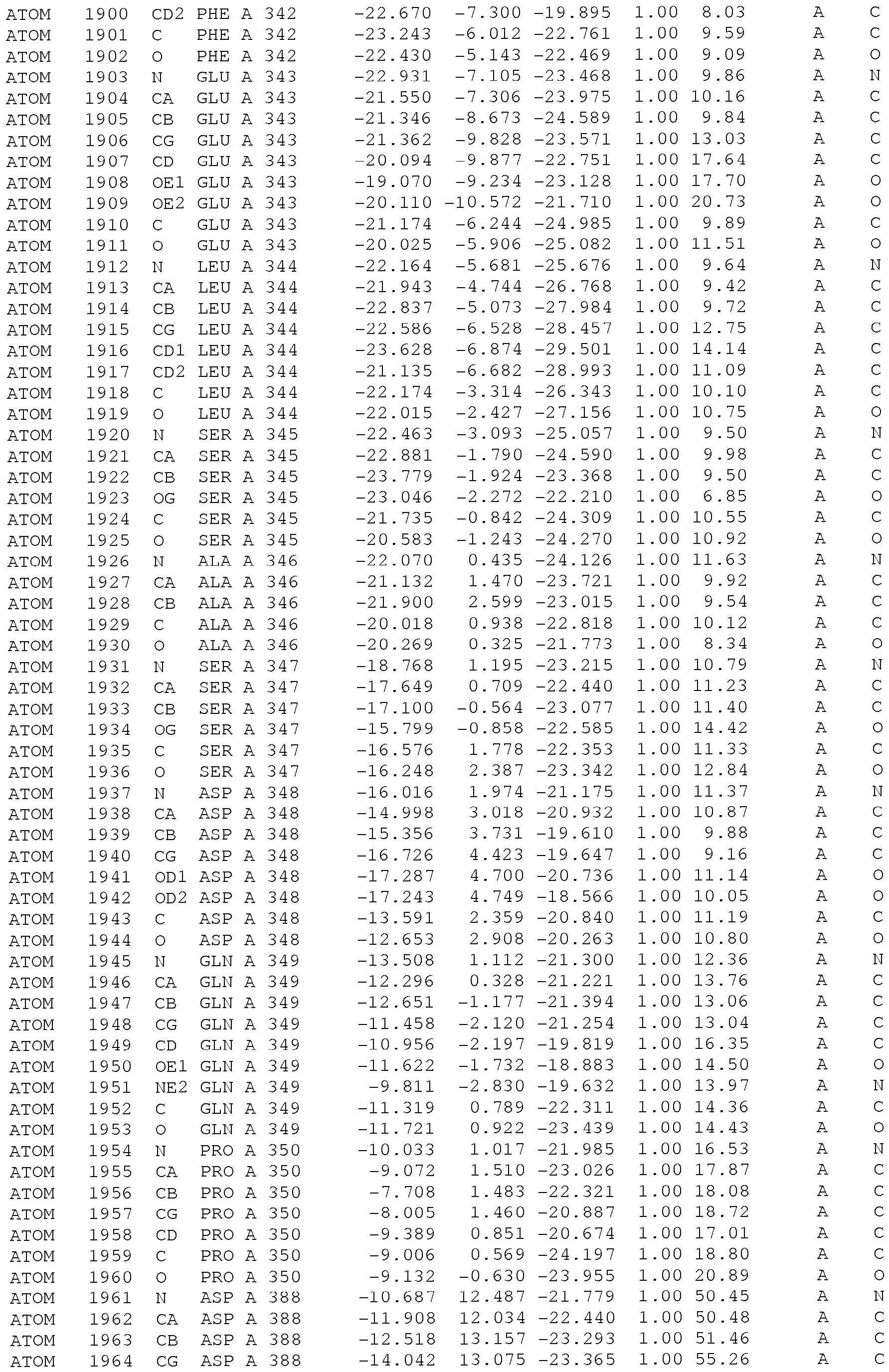

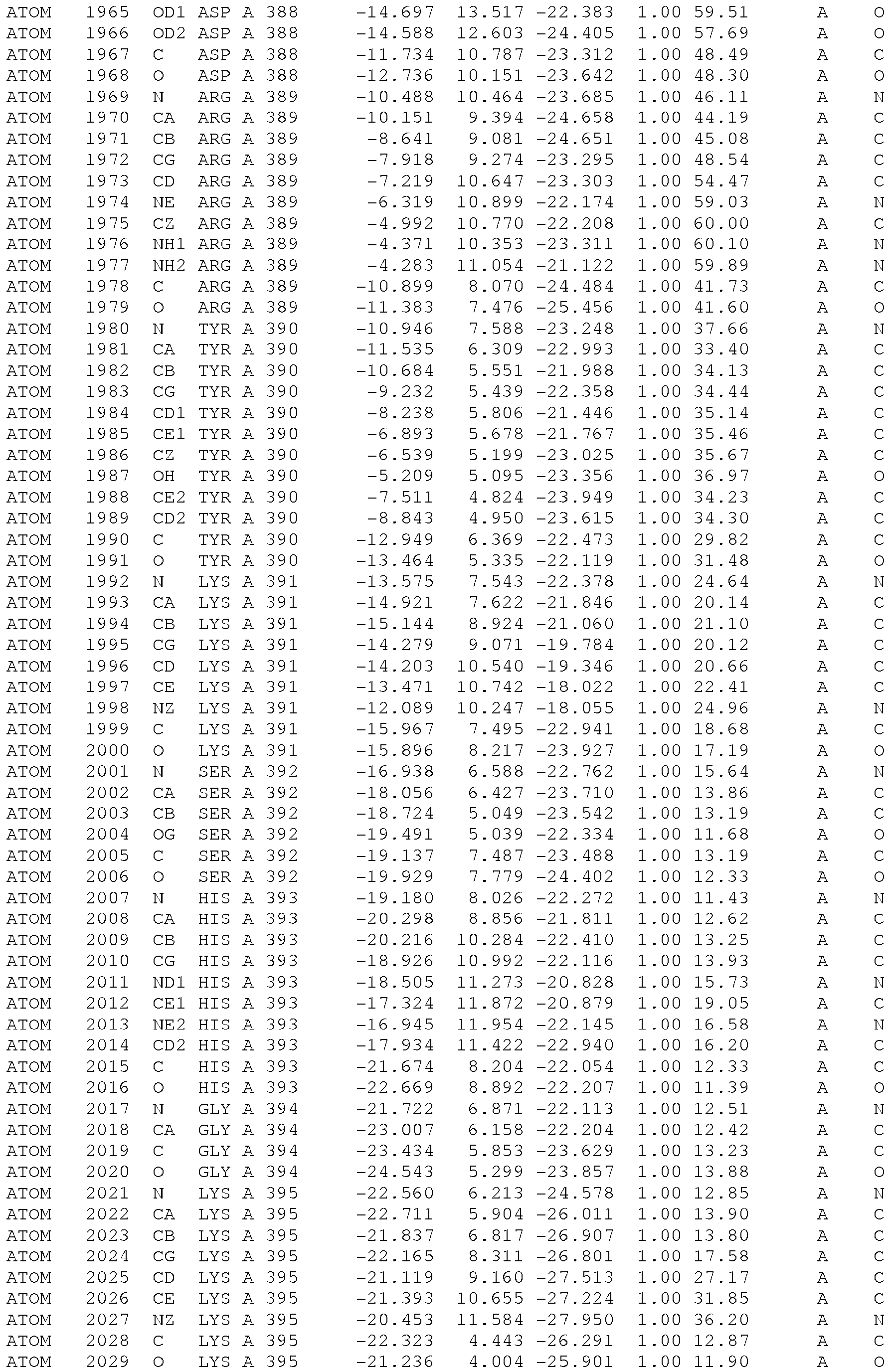

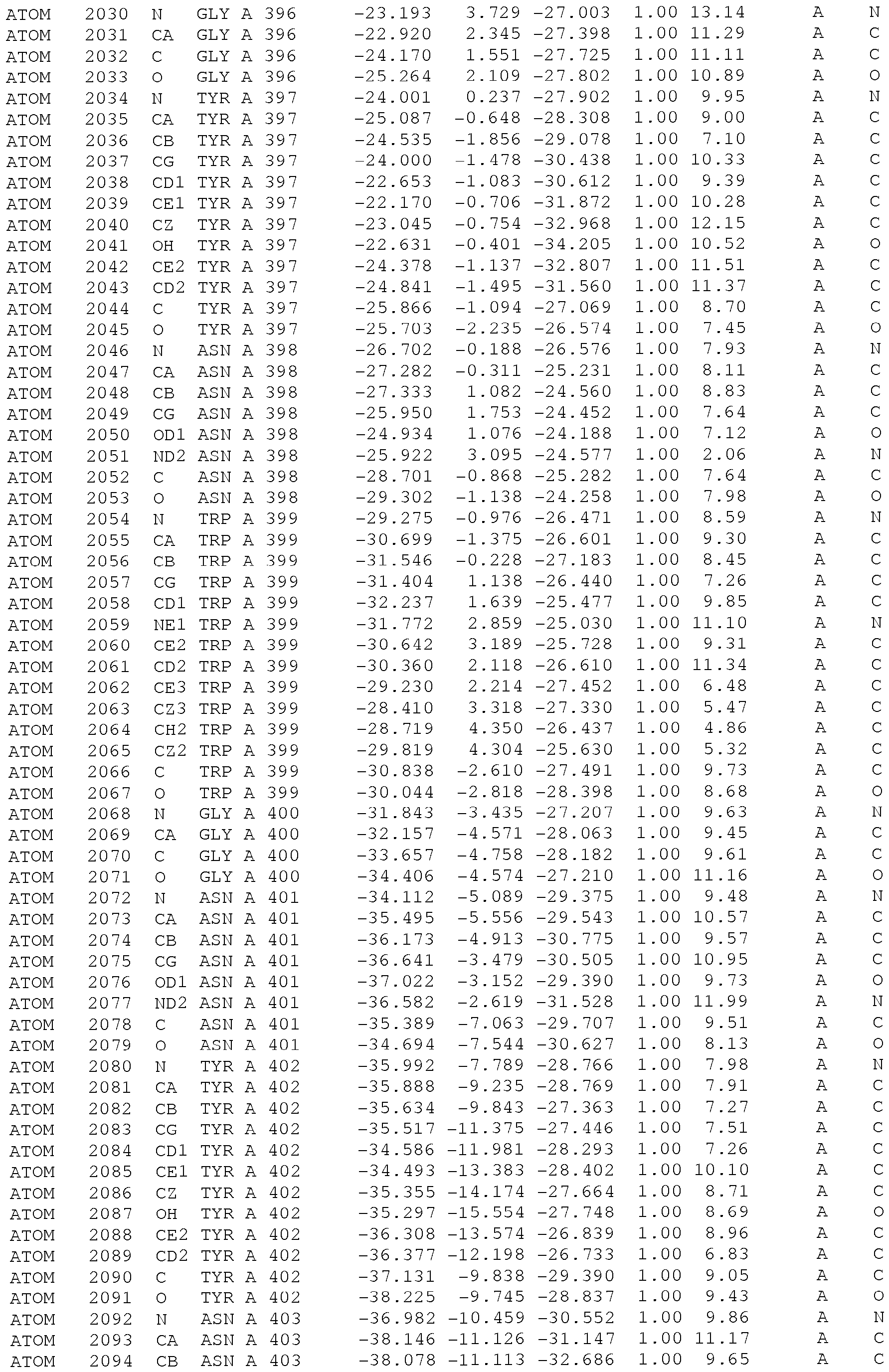

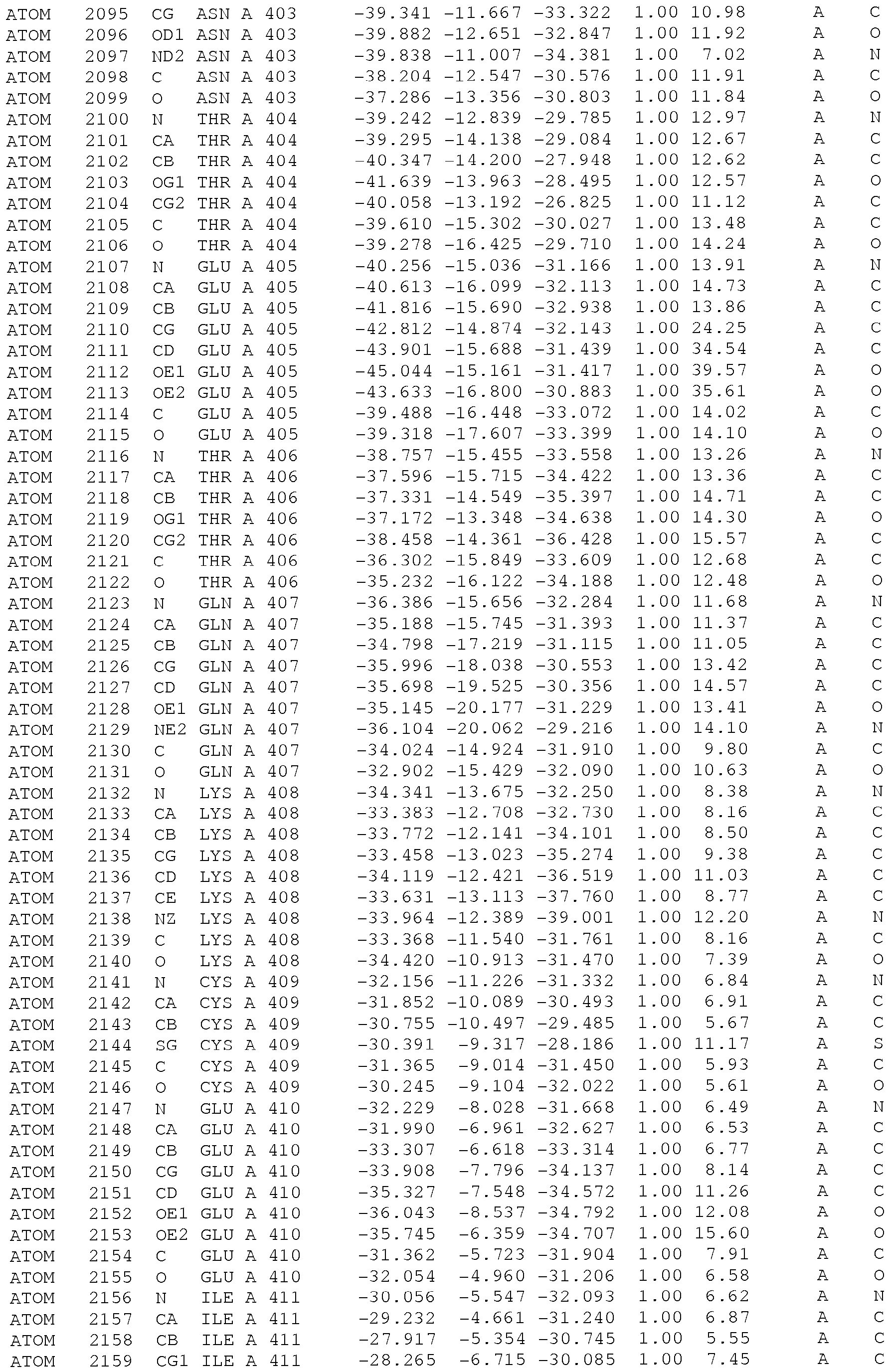

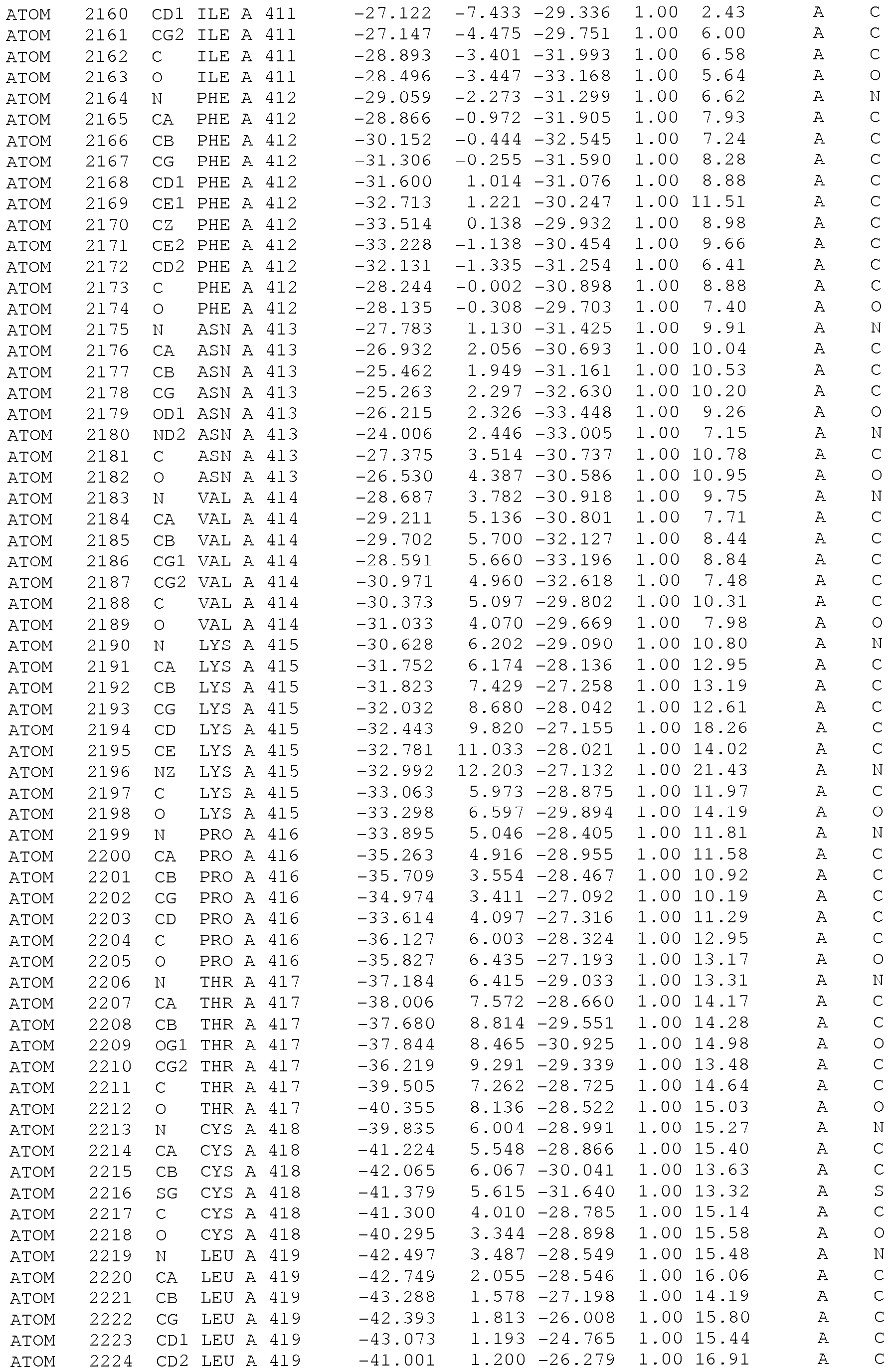

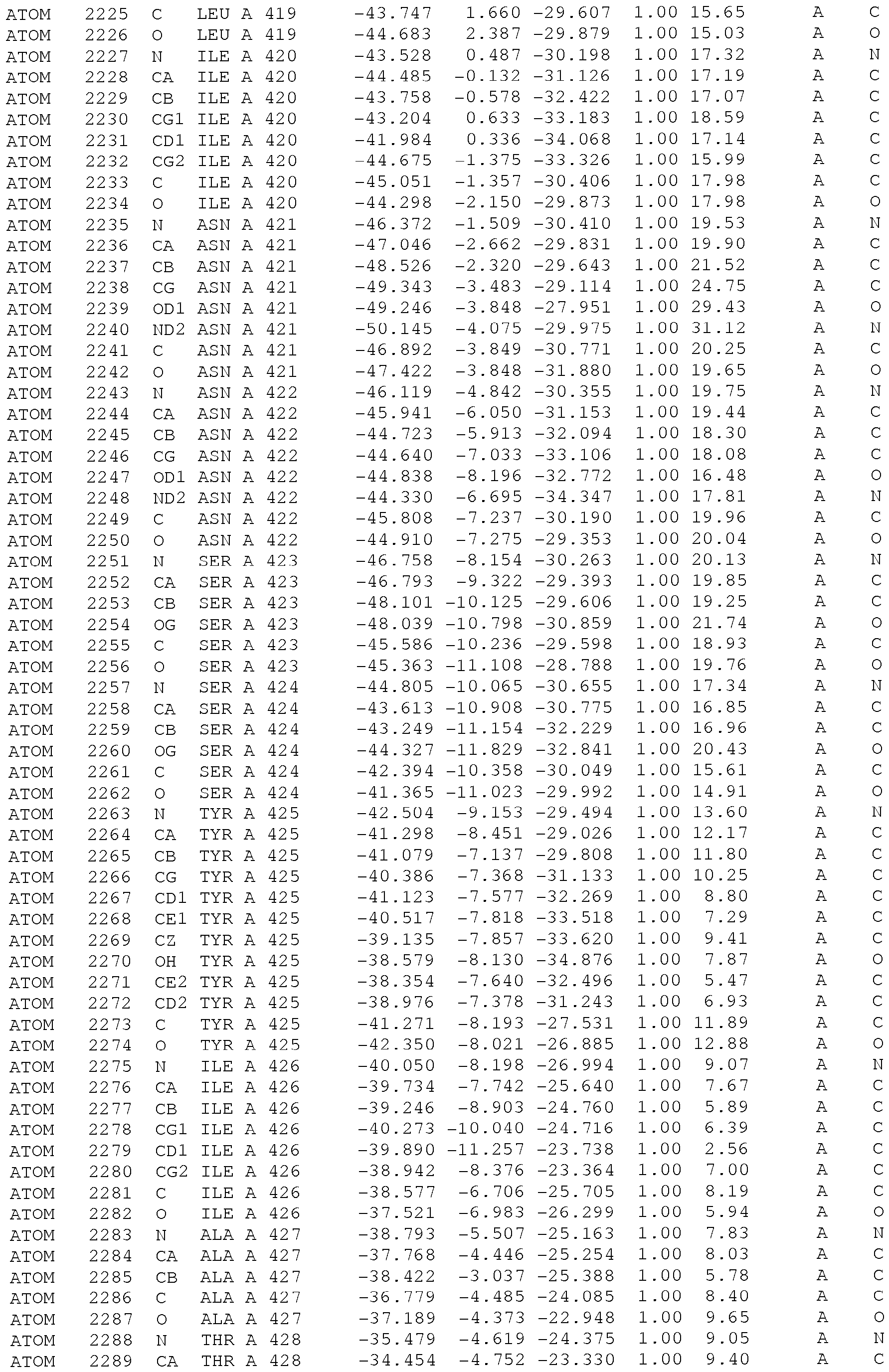

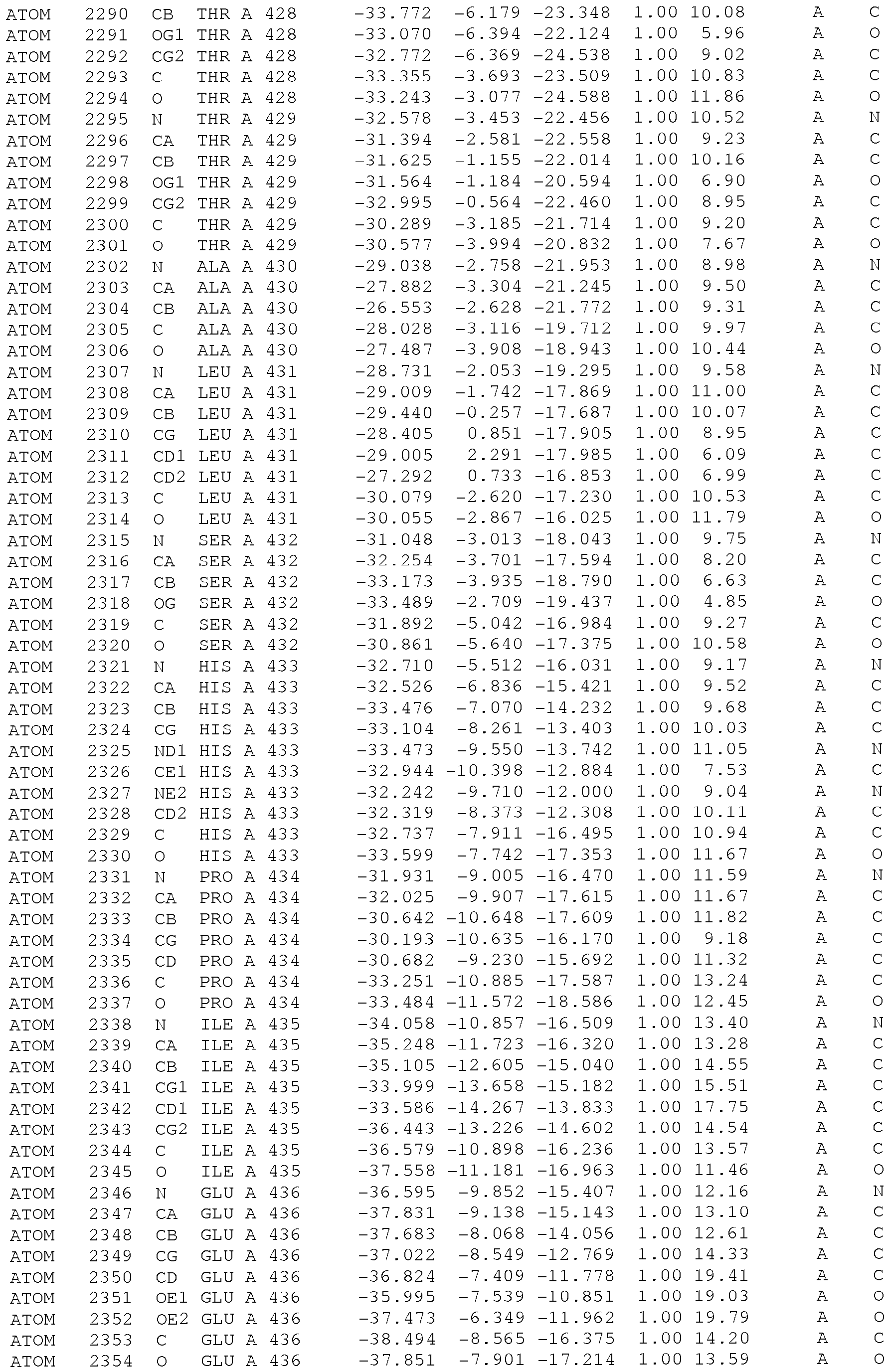

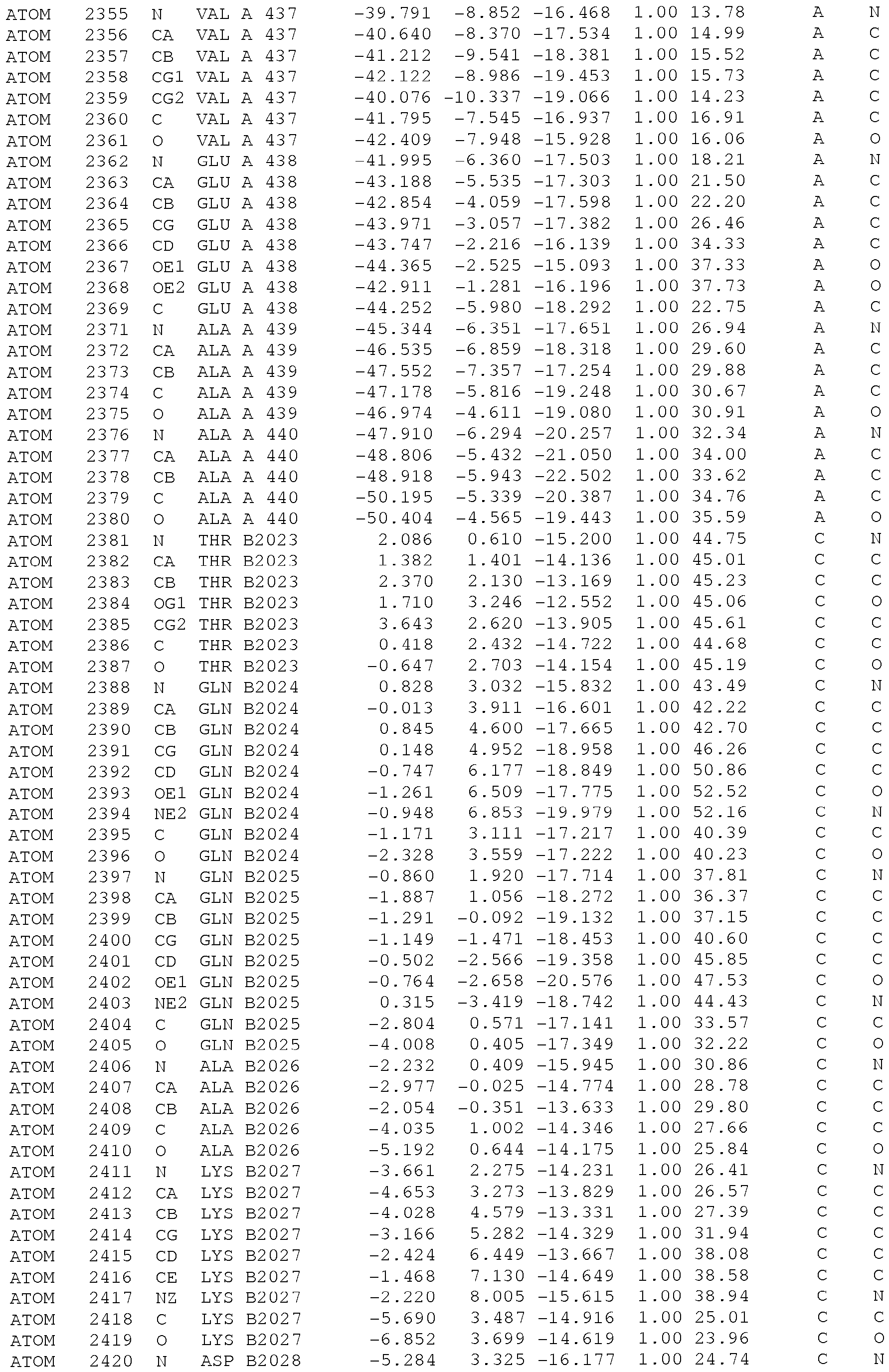

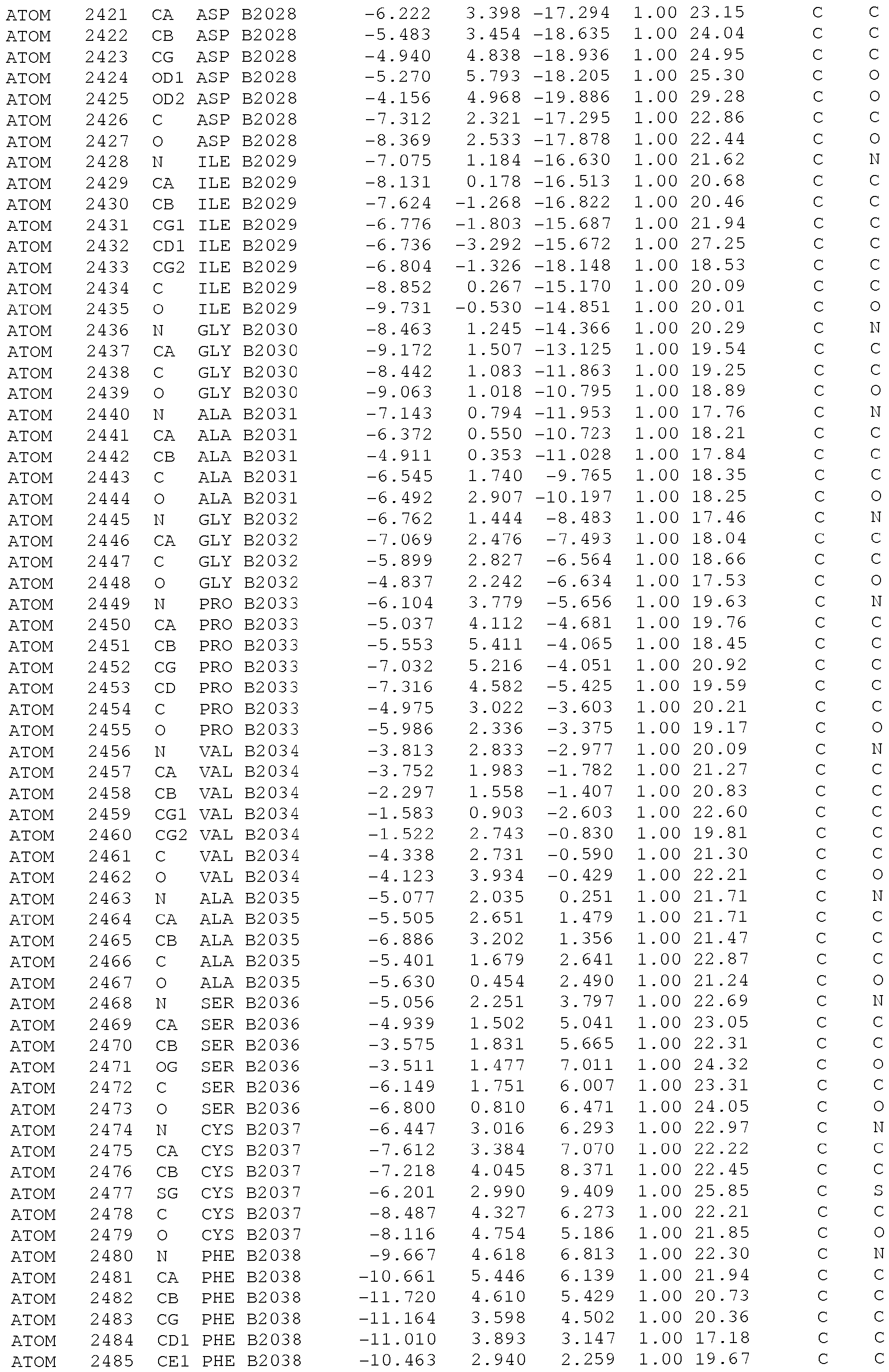

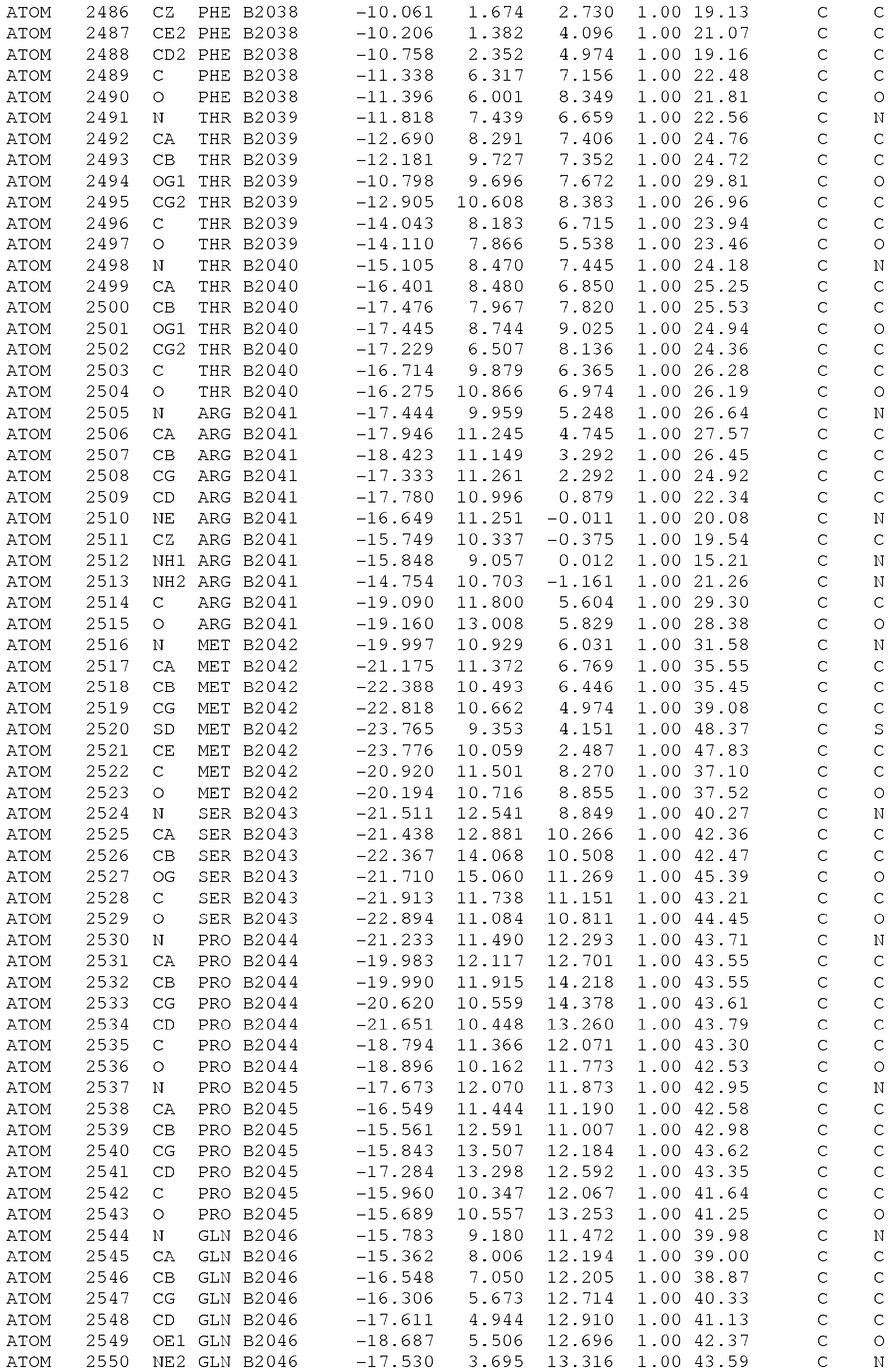

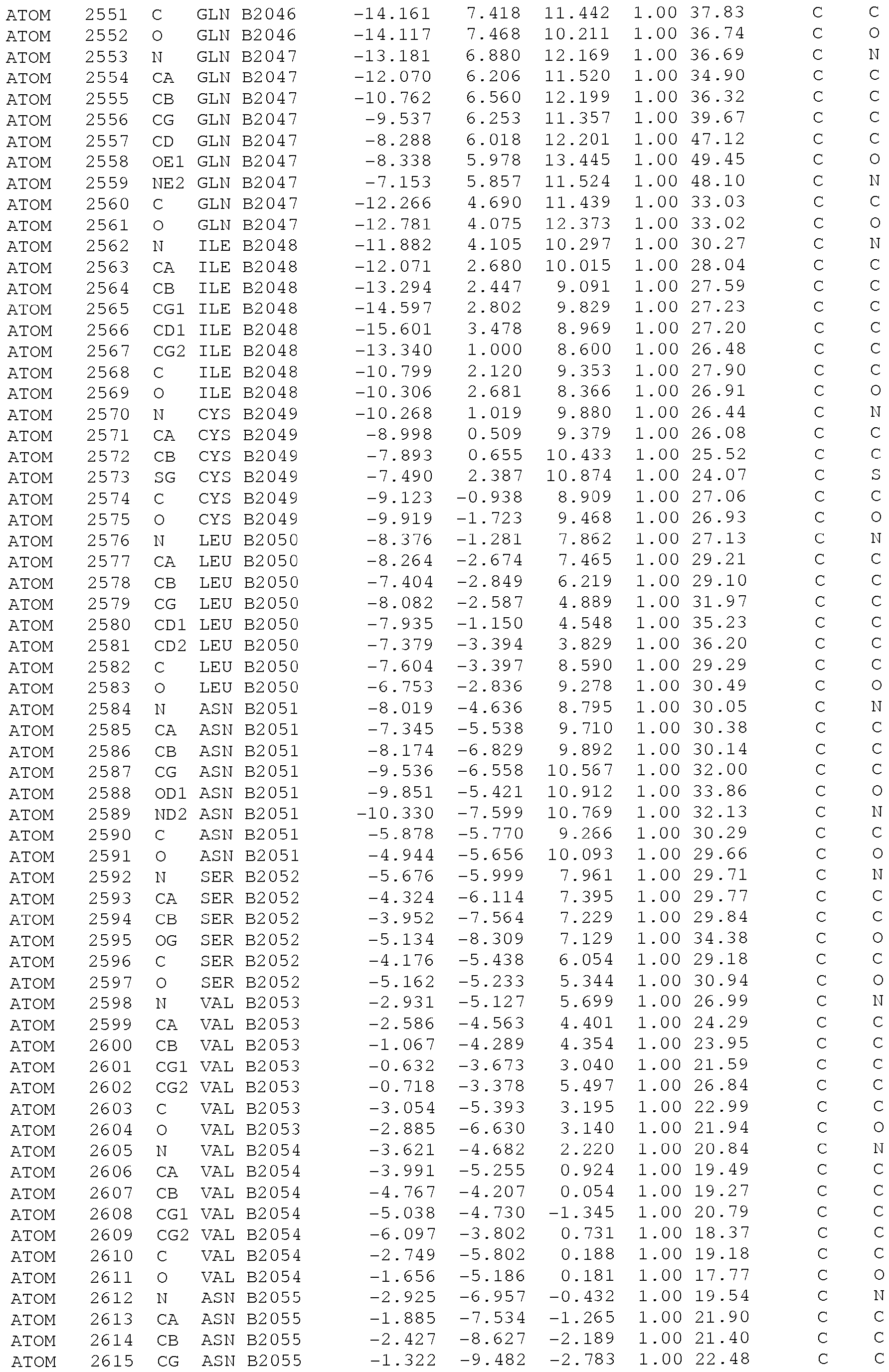

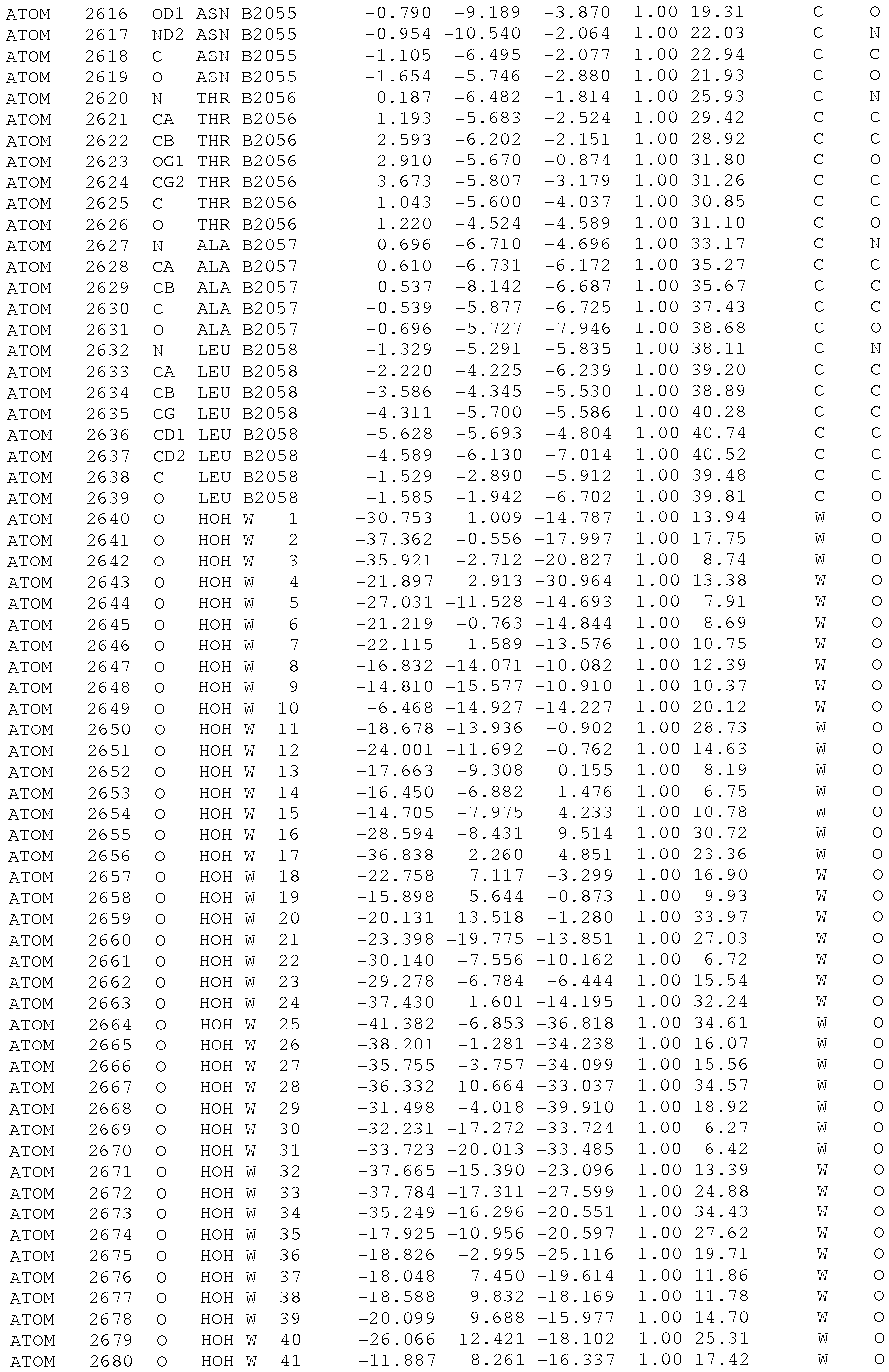

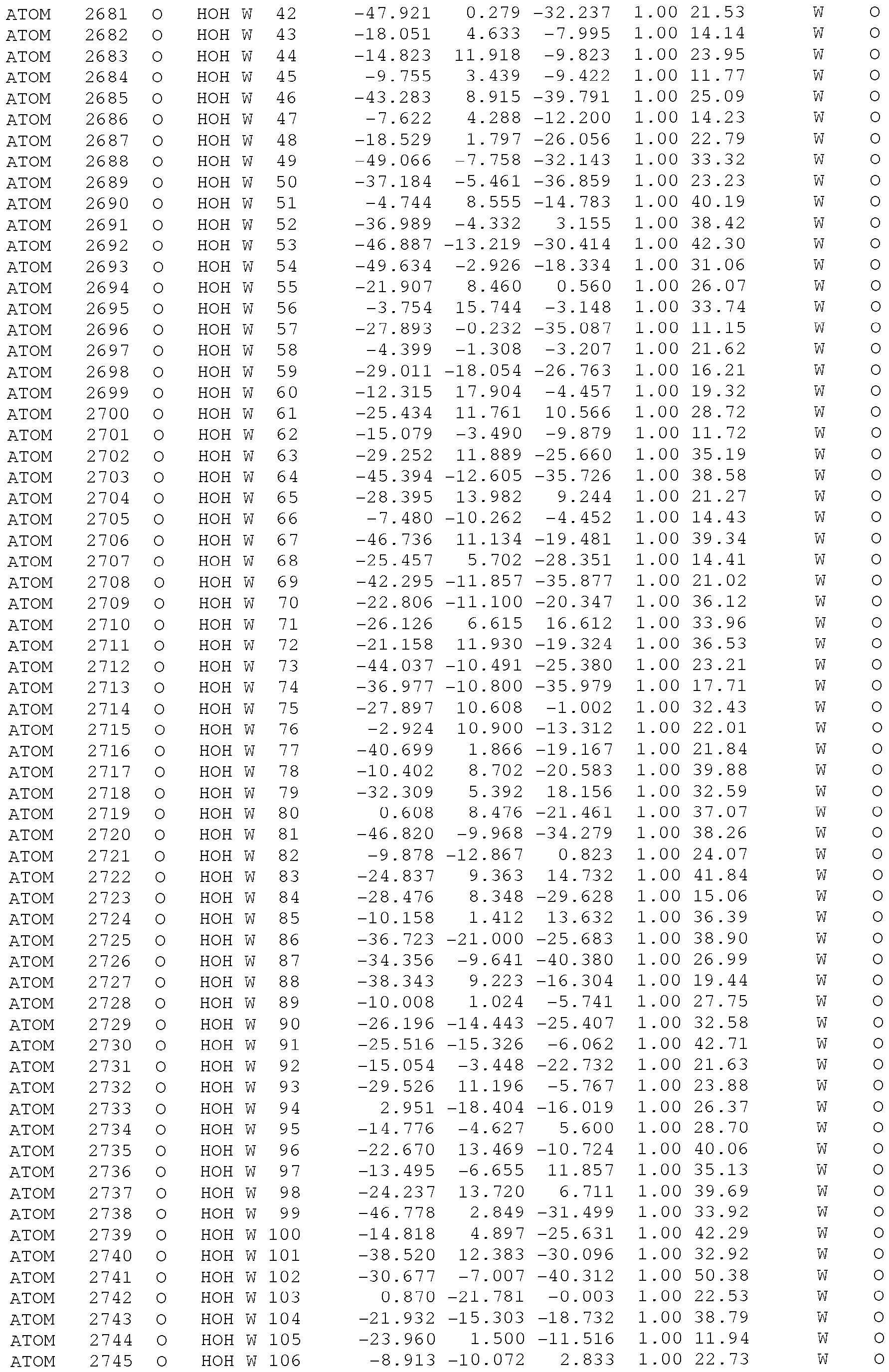

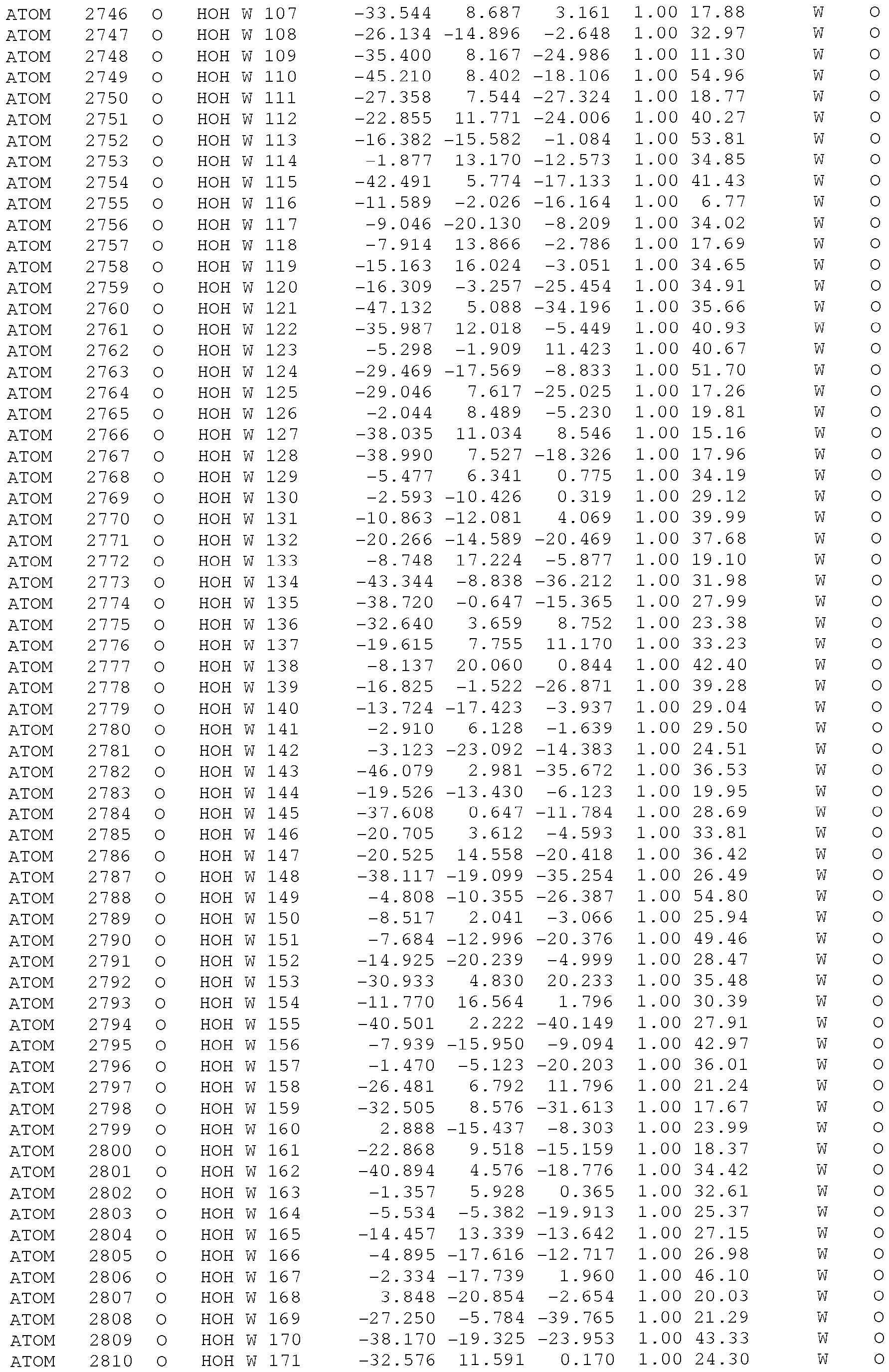

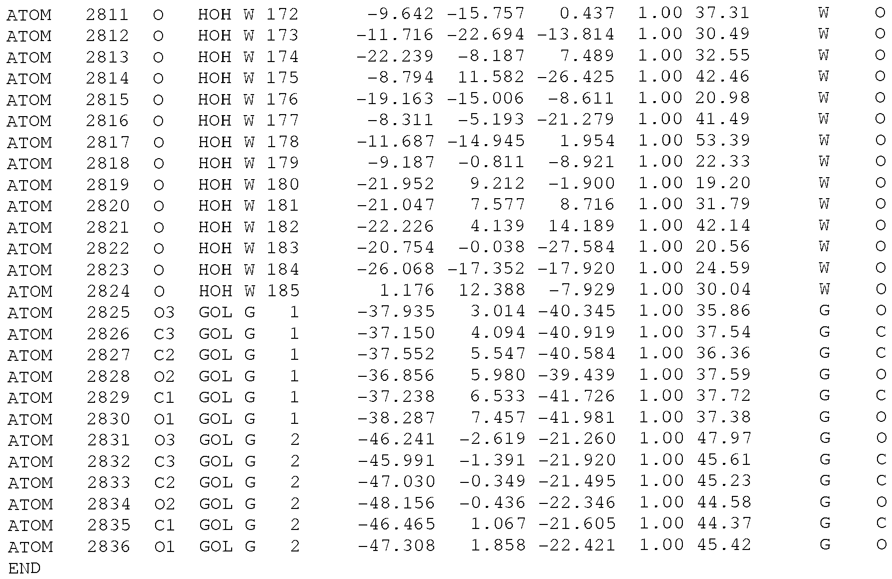

### References

1. Carruthers, V. & Boothroyd, J.C. Pulling together: an integrated model of Toxoplasma cell invasion. Current Opinion in Microbiology 10, 83-89 (2007).
2. Carruthers, V.B. & Sibley, L.D. Sequential protein secretion from three distinct organelles of Toxoplasma gondii accompanies invasion of human fibroblasts. Eur J Cell Biol73, 114-23 (1997).
3. Boothroyd, J.C. & Dubremetz, J.F. Kiss and spit: the dual roles of Toxoplasma rhoptries. Nat Rev Microbiol 6, 79-88 (2008).
4. Besteiro, S., Michelin, A., Poncet, J., Dubremetz, J.F. & Lebrun, M. Export of a Toxoplasma gondii rhoptry neck protein complex at the host cell membrane to form the moving junction during invasion. PLoS Pathog 5, e1000309 (2009).
5. Kenny, B. et al. Enteropathogenic E. coli (EPEC) transfers its receptor for intimate adherence into mammalian cells. Cell 91, 511-20 (1997).
6. Lamarque, M. et al. The RON2-AMA1 interaction is a critical step in moving junction-dependent invasion by Apicomplexan parasites. Plos Pathogens (2011).
7. Alexander, D.L., Mital, J., Ward, G.E., Bradley, P. & Boothroyd, J.C. Identification of the moving junction complex of Toxoplasma gondii: a collaboration between distinct secretory organelles. PLoS Pathog 1, e17 (2005).
8. Treeck, M. et al. Functional analysis of the leading malaria vaccine candidate AMA-1 reveals an essential role for the cytoplasmic domain in the invasion process. PLoS Pathog 5, e1000322 (2009).
9. Santos, J.M., Ferguson, D.J., Blackman, M.J. & Soldati-Favre, D. Intramembrane Cleavage of AMA1 Triggers Toxoplasma to Switch from an Invasive to a Replicative Mode. Science (2010).
10. Mital, J., Meissner, M., Soldati, D. & Ward, G.E. Conditional expression of Toxoplasma gondii apical membrane antigen-1 (TgAMAl) demonstrates that TgAMA1 plays a critical role in host cell invasion. Mol Biol Cell 16, 4341-9 (2005).
11. Kato, K., Mayer, D.C., Singh, S., Reid, M. & Miller, L.H. Domain III of Plasmodium falciparum apical membrane antigen 1 binds to the erythrocyte membrane protein Kx. Proc Natl Acad Sci U S A 102, 5552-7 (2005).
12. Waters, A.P. et al. A merozoite receptor protein from Plasmodium knowlesi is highly conserved and distributed throughout Plasmodium. J Biol Chem 265, 17974-9 (1990).
13. Aikawa, M., Miller, L.H., Johnson, J. & Rabbege, J. Erythrocyte entry by malarial parasites. A moving junction between erythrocyte and parasite. J Cell Biol 77, 72-82 (1978).
14. Mordue, D.G., Desai, N., Dustin, M. & Sibley, L.D. Invasion by Toxoplasma gondii establishes a moving junction that selectively excludes host cell plasma membrane proteins on the basis of their membrane anchoring. J Exp Med 190, 1783-92 (1999).
15. Morisaki, J.H., Heuser, J.E. & Sibley, L.D. Invasion of Toxoplasma gondii occurs by active penetration of the host cell. J Cell Sci 108 (Pt 6), 2457-64 (1995).
16. Gaffar, F.R., Yatsuda, A.P., Franssen, F.F. & de Vries, E. Erythrocyte invasion by Babesia bovis merozoites is inhibited by polyclonal antisera directed against peptides derived from a homologue of Plasmodium falciparum apical membrane antigen 1. Infect Immun 72, 2947-55 (2004).
17. Alexander, D.L., Arastu-Kapur, S., Dubremetz, J.F. & Boothroyd, J.C. Plasmodium falciparum AMA1 binds a rhoptry neck protein homologous to TgRON4, a component of the moving junction in Toxoplasma gondii. Eukaryot Cell 5, 1169-73 (2006).
18. Cao, J. et al. Rhoptry neck protein RON2 forms a complex with microneme protein AMA1 in Plasmodium falciparum merozoites. Parasitol Int 58, 29-35 (2009).
19. Proellocks, N.I., Coppel, R.L. & Waller, K.L. Dissecting the Apicomplexan rhoptry neck proteins. Trends Parasitol (2010).
20. Lebrun, M. et al. The rhoptry neck protein RON4 re-localizes at the moving junction during Toxoplasma gondii invasion. Cell Microbiol 7, 1823-33 (2005).
21. Chesne-Seck, M.L. et al. Structural comparison of apical membrane antigen 1 orthologues and paralogues in Apicomplexan parasites. Mol Biochem Parasitol 144, 55-67 (2005).
22. Crawford, J., Tonkin, M.L., Grujic, O. & Boulanger, M.J. Structural characterization of apical membrane antigen 1 (AMA1) from Toxoplasma gondii. J Biol Chem 285, 15644-52 (2010).
23. Pizarro, J.C. et al. Crystal structure of the malaria vaccine candidate apical membrane antigen 1. Science 308, 408-11 (2005).
24. Bai, T. et al. Structure of AMA1 from Plasmodium falciparum reveals a clustering of polymorphisms that surround a conserved hydrophobic pocket. Proc Natl Acad Sci U S A 102, 12736-41 (2005).
25. Gupta, A. et al. Refolding, purification, and crystallization of apical membrane antigen 1 from Plasmodium falciparum. Protein Expr Purif 41, 186-98 (2005).
26. Krissinel, E. & Henrick, K. Inference of macromolecular assemblies from crystalline state. J Mol Biol 372, 774-97 (2007).
27. Coley, A.M. et al. Structure of the malaria antigen AMA1 in complex with a growth-inhibitory antibody. PLoS Pathog 3, 1308-19 (2007).
28. Collins, C.R., Withers-Martinez, C., Hackett, F. & Blackman, M.J. An inhibitory antibody blocks interactions between components of the malarial invasion machinery. PLoS Pathog 5, e1000273 (2009).
29. Coley, A.M. et al. The most polymorphic residue on Plasmodium falciparum apical membrane antigen 1 determines binding of an invasion-inhibitory antibody. Infect Immun 74, 2628-36 (2006).
30. Henderson, K.A. et al. Structure of an IgNAR-AMA1 complex: targeting a conserved hydrophobic cleft broadens malarial strain recognition. Structure 15, 1452-66 (2007).
31. Harris, K.S. et al. Binding hot spot for invasion inhibitory molecules on Plasmodium falciparum apical membrane antigen 1. Infect Immun 73, 6981-9 (2005).
32. Richard, D. et al. Interaction between plasmodium falciparum apical membrane antigen 1 and the Rhoptry neck protein complex defines a key step in the erythrocyte invasion process of malaria parasites. J Biol Chem (2010).
33. Lee, E.F. et al. Peptide inhibitors of the malaria surface protein, apical membrane antigen 1: Identification of key binding residues. Biopolymers (2011).
34. Harris, K.S. et al. Rapid optimization of a peptide inhibitor of malaria parasite invasion by comprehensive N-methyl scanning. J Biol Chem 284, 9361-71 (2009).
35. Remarque, E.J., Faber, B.W., Kocken, C.H. & Thomas, A.W. A diversity-covering approach to immunization with Plasmodium falciparum apical membrane antigen 1 induces broader allelic recognition and growth inhibition responses in rabbits. Infect Immun 76, 2660-70 (2008).
36. Bowden, T.A. et al. Structural basis of Nipah and Hendra virus attachment to their cell-surface receptor ephrin-B2. Nat Struct Mol Biol 15, 567-72 (2008).
37. Diskin, R., Marcovecchio, P.M. & Bjorkman, P.J. Structure of a clade C HIV-1 gp120 bound to CD4 and CD4-induced antibody reveals anti-CD4 polyreactivity. Nat Struct Mol Biol 17, 608-13 (2010).
38. Luo, Y. et al. Crystal structure of enteropathogenic Escherichia coli intimin-receptor complex. Nature 405, 1073-7 (2000).
39. Schubert, W.D. et al. Structure of internalin, a major invasion protein of Listeria monocytogenes, in complex with its human receptor E-cadherin. Cell 111, 825-36 (2002).
40. Leslie, A.G.W. Recent changes to the MOSFLM package for processing film and image plate data. Joint CCP4 + ESF-EAMCB Newsletter on Protein Crystallography 26(1992).
41. Evans, P.R. Scaling and assessment of data quality. Acta Cryst D, 72-82 (2005).
42. Collaborative Computational Project Number 4, The CCP4 Suite- programs for protein crystallography. Acta Crystallog. sect. D 50, 760-763 (1994).
43. McCoy, A.J. et al. Phaser crystallographic software. J Appl Crystallogr 40, 658-674 (2007).
44. Emsley, P. & Cowtan, K. Coot: model-building tools for molecular graphics. Acta Crystallog. sect. D 60, 2126-2132 (2004).
45. Murshudov, G.N., Vagin, A.A. & Dodson, E.J. Refinement of macromolecular structures by the maximum-likelihood method. Acta Crystallog. sect. D 53, 240-255 (1997).
46. Thompson, J.D., Higgins, D.G. & Gibson, T.J. CLUSTAL W: improving the sensitivity of progressive multiple sequence alignment through sequence weighting, position-specific gap penalties and weight matrix choice. Nucleic Acids Res 22, 4673-80 (1994).
47. Gouet, P., Courcelle, E., Stuart, D.I. & Metoz, F. ESPript: multiple sequence alignments in PostScript. Bioinformatics 15, 305-308 (1999).

## Claims

1. A purified or isolated polypeptide selected from the group of polypeptides consisting of the following polypeptides:
a) a polypeptide having at most a 75 amino-acid length and comprising the sequence of the rhoptry neck protein RON2-2 domain of an Apicomplexan parasite, said RON2-2 domain designating the region located between the domain TM2 and TM3 of the RON2 peptide and extending into 14 amino-acids residues of TM3, or a fragment thereof comprising at least the subfragment of said RON2-2 peptide having the sequence comprised between the two cysteine residues located into said RON2-2 domain, cysteine residues inclusive; and
b) a homologous polypeptide therof having a sequence presenting an identity of at least 50 % with that RON2-2 domain and wherein at least 2 amino-acids residues capable of forming a disulfide bond are present.

2. The polypeptide according to claim 1, **characterized in that** said RON2-2 domain of an Apicomplexan parasite is a RON2-2 domain of an Apicomplexan parasite selected from the group consisting of Aconoidasida and Conoidasida class of Apicomplexan parasite, preferably selected from the group consisting of Plasmodium, Toxoplasma, Eimeria, Neospora or Theleiria, more preferably selected from the group of *Toxoplama gondii*, *Plamodium falciparum*, *Plasmodium bergehii*, *Plasmodium knowlesii, Plasmodium vivax, Plasmodium yoelii, Babesia bovis, Babesia canis, Eimeria tenella*, *Neospora caninum and Theileria parva* species.

3. The polypeptide according to claim 1 or 2, **characterized in that** it is a synthetic or a recombinant polypeptide.

4. The polypeptide according to one of claims 1 to 3, **characterized in that** it comprises a disulfide bond between the two amino-acids residues capable of forming a disulfide bound present in said subfragment.

5. The polypeptide according to one of claims 1 to 4, **characterized in that** it comprises the RON2-2 domain of the RON2 polypeptide having the sequence selected from the sequences SEQ ID NOs. 1, 3, 5, 7, 9, 11, 13, 15, 17 and 19, or a fragment or a homologous sequence thereof as defined in part a) and b) of claim 1.

6. The polypeptide according to one of claims 1 to 5, **characterized in that** it comprises the RON2-2 domain having the sequence selected from the sequences SEQ ID NOs. 2, 4, 6, 8, 10, 12, 14, 16, 18 and 20, or a fragment or a homologous sequence thereof as defined in part a) and b) of claim 1.

7. The polypeptide according to one of claims 1 to 6, **characterized in that** a disulfide bound is formed between two conserved cysteine amino-acids residues located into the RON2-2 domain.

8. The polypeptide according to one of claims 1 to 7, having at most 65, preferably 60, 55, 50, 45 and more preferably 40 amino-acids length.

9. The polypeptide according to one of claims 1 to 8, having at least 10 amino-acids length, preferably 11, 12, 13, 14, 15, 20, 25, 30 and more preferably 35 amino-acids length.

10. An isolated nucleic acid, DNA or RNA, coding for a polypeptide according to one of claims 1 to 8, or a complementary nucleic acid thereof.

11. Cloning or expression vector encoding the polypeptide according to one of claims 1 to 9.

12. Host cell transformed by a vector of claim 10.

13. Composition comprising a polypeptide according to one of claims 1 to 9, or a nucleic acid according to claim 10, for its use as a medicament, as an immunogen or as a vaccine.

14. Composition comprising a polypeptide according to one of claims 1 to 9, or a nucleic acid according to claim 10, for its use as a pharmaceutical composition for the prevention or the treatment of Apicomplexan parasite infection.

15. Composition according to claim 14, for the prevention or the treatment of toxoplasmosis, malaria, neosporosis, coccidiosis, babesiosis, theileriosis.

16. A method for the production of isolated or purified antibodies, or a functional fragment thereof, capable of inhibiting the interaction between the AMA1 polypeptide and the RON2 polypeptide during host cell invasion step by Apicomplexan parasite, **characterized in that** said method comprises the step of:
a) immunizing a suitable non-human mammal subject with an immunogen composition comprising a polypeptide according to one of claims 1 to 9, said immunogen composition optionally further includes an adjuvant; and
b) selecting the antibodies which are capable of specifically binding the RON2-2 domain of RON2 Apicomplexan parasite, preferably said RON2-2 domain exhibiting the epitope comprising a fragment of at least 10 amino-acids residues of the subfragment of said RON2-2 peptide having the sequence comprised between the two cysteine residues located into said RON2-2 domain, or homologous fragment thereof as defined in claim 1, part a) and b).

17. An isolated or purified antibody, or one of its functional fragments, susceptible to be obtained or obtained by the method according to claim 16, said antibody or one of its said fragments being capable of specifically binding the epitope formed by the subfragment of said RON2-2 peptide having the sequence comprised between the two cysteine residues located into said RON2-2 domain, or homologous fragment thereof as defined in claim 1, part a) and b).

18. The antibody susceptible to be obtained or obtained by the method according to claim 16, or as claimed in claim 17, **characterized in that** said functional fragment is selected from the from the fragment Fv, Fab, F(ab')2, Fab', scFv and scFv-Fc.

19. A method of *in vitro* diagnosis of Apicomplexan parasite infection from a biological sample, **characterized in that** said biological sample is contacted with:
- an antibody susceptible to be obtained or obtained by the method according to claim 16, or as claimed in claim 17; or
- a polypeptide according to one of claims 1 to 9,
optionally, said antibody or polypeptide is labeled.

20. A kit comprising the following elements:
a)
- an antibody susceptible to be obtained or obtained by the method according to claim 16, or as claimed in claim 17; or
- a polypeptide according to one of claims 1 to 9,
optionally, said antibody or polypeptide is labeled
b) optionally, the suitable reagents for the formation of a immunological reaction; and
c) optionally, the reagents allowing the demonstration of antigen/antibody and/or antibody/polypeptide complexes produced by the immunological reaction.

21. Construct for its use for crystallization of the Apicomplexan co-structure AMA1-RON2 complex, said construct comprising the following elements:
a) a polypeptide according to one of claims 1 to 9; and, preferably
b) an AMA1 polypeptide fragment comprising at least the subdomains DI and DII of the ectoplasmic domain of said AMA1 polypeptide, preferably said AMA1 polypeptide fragment is the AMA1 ectoplasmic domain comrising the DI, DII and, optionally DIII subdomains,
said RON2-2 and AMA1 polypeptide fragment being originating from the same species of Apicomplexan parasite.

22. Method for the preparation of a crystal of a polypeptide complex, preferably for diffraction measurements analysis, said method comprising contacting a polypeptide according to one of claims 1 to 9 and an AMA1 polypeptide fragment comprising at least the domains DI and DII of said AMA1 polypeptide, and wherein, said RON2 and AMA1 polypeptide fragment being originating from the same species of Apicomplexan parasite, preferably from *Toxoplasma gondii* or *Plasmodium falciparum* species.

23. A computer-readable data storage material encoded with computer-readable data comprising atomic structure coordinates of the polypeptide AMA1-RON2 complex obtained by the method according to claim 22.

24. A method for *in silico* screening a potential inhibitor of the host cell invasion of mammal by an Apicomplexan parasite comprising the step of:
- selecting a potential inhibitor by performing rational drug design with the three-dimensional structure of the Apicomplexan co-structure AMA1-RON2 polypeptidic complex, determined from the crystal obtained by the method of claim 22, , or by using the computer-readable data storage material according to claim 23, the said selecting being optionally performed in conjunction with computer modeling.

25. A method for screening a potential inhibitor of the host cell invasion of mammal by an Apicomplexan parasite, said method comprising:
a) selecting a potential inhibitor by performing the method of claim 24;
b) determining whether this potential inhibitor is capable to specifically bind a polypeptide according to one of claims 1 to 9, or to the AMA1 polypeptide, or fragment thereof comprisng at least the subdomains DI and DII of the ectoplasmic domain of said AMA1 polypeptide, or/and to inhibit the formation of the AMA1/RON2 complex resulting from the contact between a a polypeptide according to one of claims 1 to 9 and an AMA1 polypeptide fragment comprising at least the subdomains DI and DII of the ectoplasmic domain of said AMA1 polypeptide; and
c) optionally,
- administrating the potential inhibitor to a non-human animal model which has been infected by an Apicomplexan parasite or contacting *in vitro* the potential inhibitor with a cell model by using a cellular-based assay capable of reproducing the invasion of mammal host cell by apicomplexa parasite; and
d) optionally, detecting the ability of the potential inhibitor for inhibiting the host cell invasion and/or parasite growth.

26. An *in vitro* method for screening a potential inhibitor of the host cell invasion of mammal by an Apicomplexan parasite, said method comprising the steps of:
a) contacting said potential inhibitor with :
-i) a polypeptide according to one of claims 1 to 9, or with an AMA1 polypeptide, or a fragment thereof comprising at least the subdomains DI and DII of the ectoplasmic domain of said AMA1 polypeptide, or
-ii) an AMA1/RON2-2 complex resulting from the contact between a polypeptide according to the present invention and an AMA1 polypeptide fragment comprising at least the subdomains DI and DII of the ectoplasmic domain of said AMA1 polypeptide;
b) determining whether this potential inhibitor is capable to:
-i) specifically bind the polypeptide according to one of claims 1 to 9, or the AMA1 polypeptide, or a fragment thereof, or/and
-ii) specifically inhibit the formation of the AMA1/RON2 complex; and
c) optionally,
- administrating the potential inhibitor to a non-human animal model which has been infected by an Apicomplexan parasite or contacting *in vitro* the potential inhibitor with a cell model by using a cellular-based assay capable of reproducing the invasion of mammal host cell by Apicomplexan parasite; and
d) optionally, detecting the ability of the potential inhibitor for inhibiting the host cell invasion and/or parasite growth.


SEQUENCE LISTING

<110>  CENTRE NATIONAL DE LA RECHERCHE SCIENTIFIQUE (CNRS)
       UNIVERSITY OF VICTORIA INNOVATION AND DEVELOPMENT
       CORPORATION

<120>  Identification of region of RON2 of apicomplexan parasites
       suitable for targeting by vaccines and drugs

<130>  69136D29472

<160>  75

<170>  PatentIn version 3.3

<210>  1
<211>  1479
<212>  PRT
<213>  Toxoplasma gondii

<220>
<221>  misc_feature
<223>  RON2

<400>  1

Met Thr Lys Arg Ala Gly Leu Pro Leu Gly Arg Ala Phe Leu Val Leu
1               5                   10                  15

Ile Leu Leu Ser Ala Ala Asp Ser Leu Phe Phe Ser Ser Phe Pro Arg
            20                  25                  30

Ser Ala Leu Gln Leu Phe Ser Ser Val Leu Phe Thr Asp Ala Ala Glu
        35                  40                  45

Pro Asp Ser Asp Ala Thr Pro Gly Leu Arg Pro Gln Pro Ser Pro Arg
    50                  55                  60

Thr Phe Arg Pro Thr Gly Tyr Gln Arg Ile Glu Val Lys Thr Val Asp
65                  70                  75                  80

Glu Glu Leu Pro Glu Asp Leu Lys Val Tyr Thr Ala Ser Thr Arg Gly
                85                  90                  95

Ser Ser Ser Arg Thr Phe Glu Val Arg Asn Ala Gly Gly Arg Gln Glu
            100                 105                 110

Gly Phe Thr Leu Ser Val Leu Thr Ala Gly Gly Pro Leu Pro His Gly
        115                 120                 125

Ser Trp Ser Trp Ser Gly Thr Pro Pro Glu Val Gln Thr Thr Gly Gly
    130                 135                 140

Ser Gln Ile Ser Phe Gly Trp Val Pro Asp Thr Glu Thr Pro Ser Leu
145                 150                 155                 160

Pro Glu Arg Asn Leu Leu Gln Leu Lys Arg Met Leu Arg Asp Glu Gly
                165                 170                 175

Leu Ile Glu Ala Val Gln Leu Arg Ala Ala Glu Lys Gly Cys Pro Val
            180                 185                 190

Ala Val Leu His Asn Leu Arg Gln Leu Pro Val Asn Phe Arg Glu Val
        195                 200                 205

Leu His Glu Glu Tyr Glu Ser Arg Ser Asn Pro Ala Lys Met Tyr Glu
    210                 215                 220

Val Ala Asn Ser Tyr Val Gln Gln Arg Gly Ser Asp Ala Ala Arg Trp
225                 230                 235                 240

Ser Val Ser Gln Ser Val Glu Leu Ser Leu Leu Glu Met His Ala Thr
                245                 250                 255

Ser Thr Thr Asp Pro Arg Gly Ser Ser Ala Val Pro Ser Phe Leu Glu
            260                 265                 270

Thr Gly Pro Gln Val Arg Val Ala Met Thr Asp Ala Val Pro Ser Gly
        275                 280                 285

Ile Arg Val Tyr Ala Thr Pro Pro Ala Pro Arg Pro Val Pro Val Gln
    290                 295                 300

Ser Asn Gln Thr Glu Lys Glu Arg Ser Pro Thr Ser Lys Arg Leu Val
305                 310                 315                 320

Gly Met Gln Leu Gly Leu Tyr Leu Ile Cys Lys Leu Ala Ala Leu Phe
                325                 330                 335

Gly His Pro Thr Leu Phe Leu Asn Pro Tyr Tyr Thr Glu Gln Gln Leu
            340                 345                 350

Leu Glu Ala Val Ala Gln Ala Leu Gly Ile Ala Pro Pro His Arg Gly
        355                 360                 365

Asp Phe Glu Asn Glu Gly Asn Glu Ala Gln Ala Thr Ala Asn Gln His
    370                 375                 380

Asn Gly Ser Ala Asp Gln Leu Leu Ala Ala Ile Glu Ile Phe Arg Leu
385                 390                 395                 400

Gly Pro Asn Pro Tyr Thr Ile Gly His Val Leu Thr Leu Met Ile Ala
                405                 410                 415

Tyr Leu Asp Tyr Glu Ser Phe Phe Gly Ala Ser Pro Ser Lys Pro Phe
            420                 425                 430

His Ser Trp Val Ser Leu Ala Ala Ser Ala Gly Asn Asn Thr Gly Phe
        435                 440                 445

Ala Met Leu Asp Glu Met Cys Asp Asn His Arg Gly Pro Lys Arg Arg
    450                 455                 460

Gly Gln Lys His Trp Tyr Gln Thr Gly Gly Ala Arg Lys His Lys Asn
465                 470                 475                 480

Arg Asp Met Leu Pro Leu His Arg Gln Leu Cys Asp Ala Leu Glu Leu
                485                 490                 495

Val Leu Asn Gly Val Gln Gln Ile Gln Ile Asp Leu Met Asp Glu Leu
            500                 505                 510

Gly Lys Tyr Lys Thr Gly Val Glu Pro Leu Val Asp Pro Ala Thr Asn
        515                 520                 525

Ser Ala Arg Ile His Thr Arg Thr Cys Arg Gly Leu Ser Pro Val Cys
    530                 535                 540

Asp Tyr Glu Ala Thr Ile Leu Ala Pro Val Arg Ala Leu Glu Pro His
545                 550                 555                 560

Glu Gln Gln Asp Ser Leu Arg Thr Lys Lys Ala Phe Asn Leu Val Thr
                565                 570                 575

Gly Tyr Gly Ser Gly His Val Gly Gln Ile Thr Gly Ser Ile Ala Glu
            580                 585                 590

Pro Phe Ser His Ser Trp Arg Thr Arg Trp Gly Lys Val Val Ala Asp
        595                 600                 605

Pro Thr Ala Tyr Gly Glu Ile Phe Glu Arg Thr Leu Trp Phe Asp Asp
    610                 615                 620

Arg Glu Leu Met Ala Lys Ser Ser Gly Ala Leu Phe Arg Gln Tyr Asp
625                 630                 635                 640

Arg Ile Ala Lys Asp Ser Met Ser Phe Gly Val Phe Met Asn Val Glu
                645                 650                 655

Asn Gly Leu Leu Lys Lys Asp Met Arg Ser Lys Leu Glu Ala Tyr Ile
            660                 665                 670

Ser Gln Arg Lys Ser Phe Val Glu Lys Arg Gln Gln Ser Arg Phe Ala
        675                 680                 685

Lys Leu Arg Lys Lys Ile Pro Glu Asn Asp Pro Tyr Ala Leu Arg Ala
    690                 695                 700

Ala Ile Phe Leu Ala Leu Asn Ser Arg Thr Phe Cys Ala Gln Pro Thr
705                 710                 715                 720

Ser Phe Leu Ser Ser Phe Arg Thr Phe Leu Thr Asn Gln Tyr His Lys
                725                 730                 735

Leu Ser Gln Gly Arg Asn Leu Pro Arg Ser Gln Arg Ser Leu Met Ala
            740                 745                 750

Phe Met Arg Thr Gly Gln Val Lys Phe Phe Gln Glu Trp Cys Ser Phe
        755                 760                 765

Asp Pro Leu Ala Val Asn Ala Leu Phe Leu Phe Arg Phe Ala Val Ser
    770                 775                 780

Gly Thr Asp Pro Ala Ala Leu His Asp Arg Gln His Thr Arg Val Ser
785                 790                 795                 800

Arg Asn Lys Lys Thr Met Arg Ile Leu Asn Ser Lys Trp Thr Pro Ala
                805                 810                 815

Val Leu Lys Lys Leu Met Arg Lys Val Asn His Lys His Met Ala Arg
            820                 825                 830

Glu Ala Lys Ala Leu Leu Leu Arg Ser Leu Asp Pro Thr Val Leu Ser
        835                 840                 845

Ser Ile Val Thr Ala Phe Asp Phe Ile Thr His Thr Gln Ala Asn Leu
    850                 855                 860

Glu Val Asn Gln Asn Ala Phe Met Tyr His Glu Val Arg Ala Arg Glu

865                 870                 875                 880

Val Ser Arg Gln Ser Ala Ala Glu Lys Gly Ser His Arg Leu His Glu
                885                 890                 895

Arg Gly Leu Val Arg Glu Thr Asp Asp Met Ile Lys Arg Trp Ala Glu
            900                 905                 910

His Gly Ile Pro Gly Asp Ile Lys Arg Arg Leu Ala Arg Gly Glu Lys
        915                 920                 925

Leu Pro Glu Gly Met Ser Phe Gly Gly Ile Pro Ile Pro Asn Leu Thr
    930                 935                 940

Asn Trp Asp Ala Gln Leu Asn Ser Lys Trp Leu Glu Ala Tyr Asn Ala
945                 950                 955                 960

Tyr Leu Arg His Pro Tyr Gly Arg Ala Ala Leu Asn Ala Arg Asp Pro
                965                 970                 975

Val Ala Leu Leu Val Lys Asp Ser Arg Asp Arg Leu Gln Ala Glu Ala
            980                 985                 990

Glu Gly Thr Ile Phe Leu Gly Arg  Ile Ala Lys Arg Val  His Gln Ser
        995                 1000                1005

Lys Asn  Leu Leu Arg Arg Ala  Gly Arg Ala Leu Lys  Thr Phe Phe
    1010                1015                1020

Leu Ser  Leu Leu Arg Glu Asn  Glu Arg Ser Glu Tyr  Ala Val Trp
    1025                1030                1035

Phe Gly  Val Lys Val Asp Met  Arg Gln Val Ile Gln  Thr Cys Arg
    1040                1045                1050

Gln Ile  Asn Ser Val Ala Glu  Val Val Lys Asn Asp  Arg Leu Tyr
    1055                1060                1065

Asp Phe  Ile Thr Asp Gly Trp  Met Glu Leu Val Lys  Asp Val Val
    1070                1075                1080

Ala Gly  Tyr Thr Lys Ala Ser  Val Arg Val Pro Gly  Phe Asp Thr
    1085                1090                1095

Ile Ser  Ala Ala Asn Glu Gln  Leu Arg Lys Glu Gly  Val Ala Ala
    1100                1105                1110

Ala Thr Ala Arg Asn Gln Gly Phe Leu Ser Ile His Tyr Asp Tyr
    1115                1120                1125

Ala Asn Leu Pro Glu Glu Glu Arg Lys Lys Glu Phe Gln Arg Ser
    1130                1135                1140

Met Cys Met Glu Gln Cys Glu Ala Leu Trp Lys Leu Val Met Ala
    1145                1150                1155

Phe Val Met Pro Asn Leu Gln Asn Pro Lys Lys Leu Lys Gly Tyr
    1160                1165                1170

Glu Lys Asp Phe Ser Gly Ala Lys Glu Ile Glu Lys Leu Asn Ser
    1175                1180                1185

Pro His His Val Asn Ala Phe Arg Phe Ser Leu Ser Val Gln Ile
    1190                1195                1200

Asp Phe Phe Asp Asn Met Leu Asp Lys Thr Ser Lys Lys Asn Leu
    1205                1210                1215

Lys Ala Met Lys Phe Gly Ala Ser Thr Trp Phe Thr Tyr Ala Met
    1220                1225                1230

Lys Leu Ala Gly Gln Val Asn Ser Glu Met Gly Asn Pro Asn Leu
    1235                1240                1245

Gly Thr Ala Leu Tyr Met Gln Ala Ala Tyr Tyr Gly Asn Tyr Ile
    1250                1255                1260

Arg Lys Trp Met Glu Gln Arg Arg Lys Ser Arg Lys Gln Ala Ile
    1265                1270                1275

Ile Gly Val Leu Thr Leu Gly Met Met Gly Leu Tyr Ala Leu Leu
    1280                1285                1290

Asn Val Ala Asp Ile Val Gln His Met Glu Asp Ile Gly Gly Ala
    1295                1300                1305

Pro Pro Val Ser Cys Val Thr Asn Glu Ile Leu Gly Val Thr Cys
    1310                1315                1320

Ala Pro Gln Ala Ile Ala Lys Ala Thr Thr Ser Ala Ala Arg Val
    1325                1330                1335

Ala Thr Gln Asp Phe Leu Lys Val Gly Leu Phe Ala Gly Met Ala
    1340                1345                1350

Pro Tyr Leu Met Leu Pro Met Ala Val Val Ser Val Trp Asn Ile
    1355                1360                1365

Leu Lys Ser Glu Ile Lys Val Leu Leu Gln Phe Glu Met Ala Leu
    1370                1375                1380

Lys His Thr Phe Thr Arg Leu Lys Arg Trp Leu Ala Ala Pro Phe
    1385                1390                1395

Lys Asn Trp Trp Ala Lys Arg Gly Arg Leu Lys Asp Ala Leu Phe
    1400                1405                1410

Arg Arg Ala Ser Gln Thr Tyr Arg Lys Thr Glu Gln Glu Thr Lys
    1415                1420                1425

Gln Pro Pro Arg Pro Arg Asn Leu His Asn Pro Ser Ser Trp Gly
    1430                1435                1440

Asp Thr Glu Leu Asp Ser Leu Gly Val Pro Pro Glu Pro Phe Val
    1445                1450                1455

Gln Asp Phe Glu Ile Lys Tyr Thr Thr Pro Val Phe Pro Met Ser
    1460                1465                1470

Ala Pro Leu Ile Lys Ala
    1475

<210> 2
<211> 37
<212> PRT
<213> Artificial

<220>
<223> RON2 peptide (Toxoplasma gondii)

<400> 2

Asp Ile Val Gln His Met Glu Asp Ile Gly Gly Ala Pro Pro Val Ser
1               5                   10                  15

Cys Val Thr Asn Glu Ile Leu Gly Val Thr Cys Ala Pro Gln Ala Ile
            20                  25                  30

Ala Lys Ala Thr Thr
        35

<210>  3
<211>  2189
<212>  PRT
<213>  Plasmodium falciparum

<220>
<221>  misc_feature
<223>  RON2

<400>  3

Met Leu Lys Phe Phe Ile Phe Ile Leu His Ile Tyr Leu Tyr Ile Asp
1               5                   10                  15

Ser Ile Tyr Ser Ser Glu Leu Ser Lys His Val Lys His Asp Thr Asp
            20                  25                  30

Glu Leu Lys Tyr Ala Ser Pro Thr Tyr Asp Pro Lys Lys Gly Thr Lys
        35                  40                  45

Val Ile Phe Tyr Met Pro Gly Asn Glu Gln Gly Val Ile Pro Asn Asn
    50                  55                  60

Ile Gln Asn Lys Gln His Gly Thr Ser Ile Tyr Pro Ala Ile Glu Tyr
65                  70                  75                  80

Pro Thr Thr Lys Tyr Pro Ala Ile Glu Tyr Pro Gly Ser Glu Met Asn
                85                  90                  95

Asn Gly Lys Thr Gly Thr Thr Asn Ser Gly Ile Tyr Asn Lys Ala His
            100                 105                 110

Gly Ser Ser Asn Asp Tyr Asn Ala Ser Asn Ser Gln Asp Lys Thr Ile
        115                 120                 125

Asn Leu His Ile Asp Glu Asn Asn Ser Asn Asn Ser Tyr Gln Pro Tyr
    130                 135                 140

Asp Ser Asn Asn Thr Asn Asn Asn Thr Asn Ser Asn Asn Asn Ser Asn
145                 150                 155                 160

Asn Asn Ser Asn Asn Asn Ser Asn Asn Asn Ser Asn Asn Tyr Ser Asn
                165                 170                 175

Asn Asn Ser Asn Thr Asn Ser Asn Thr Asn Ser Asn Thr Asn Ser Asn
            180                 185                 190

Asn Asn Thr Thr Asn Tyr Asp Leu Asn Ser Glu Tyr Glu Lys Ile Arg
        195                 200                 205

Arg Lys Glu Glu Glu Ala Ala Arg Arg Ile Glu Arg Glu Arg Arg Ala
    210                 215                 220

Asp Ile Asn Arg Lys Asn Gln Asp Asn Asn Ser Asn Lys Tyr Asn Asn
225                 230                 235                 240

Glu Gln Asn Gly Gly Tyr Glu Ser Asp Gly Asn Ser Pro Asn Ser Arg
                245                 250                 255

Val Asn Ile Asn Ile Thr Asn Asn Gly Thr His Gly Asn Pro His Asn
            260                 265                 270

Asn Asn Ser Tyr Gly His Lys Asn Asn Met His Asn Thr Thr Asn Gly
        275                 280                 285

Asn Tyr Ala Asn Gly Asn Tyr Ala Asn Gly Asn Tyr Ser Lys Gly Asp
    290                 295                 300

Tyr Thr Asn Gly Asp Tyr Thr Asn Gly Asp Tyr Thr Asn Gly Asp Tyr
305                 310                 315                 320

Thr Asn Gly Ile Asn Asn Asn Met His Gly Lys Asn Asn Tyr Asn Thr
                325                 330                 335

Ala Asn Gly Glu Tyr Val Asn Gly Ala Tyr Asp Gly Leu Asn Asn Gly
            340                 345                 350

Ser Tyr Lys Leu Ile Gly Asn Leu Asn Asn Asn Gln Asn Val Asp Asn
        355                 360                 365

Ser Tyr Asn Gln Gly Asn Glu Asn Asp Lys Thr Tyr Thr Ser Asn Tyr
    370                 375                 380

Asn Ile Asn Leu Glu Asp Asn Lys Asn Ser Pro Asp Asn Asn Gln Asn
385                 390                 395                 400

Tyr Ile Ser Thr Phe Asn Lys Asp Ile Gly Pro Asn Lys Ser Glu Arg
                405                 410                 415

Ser Tyr Tyr Asp Val Tyr Gly Arg Glu Tyr Asp Asp Asn Lys Tyr Asn
            420                 425                 430

Pro Tyr Asn Lys Thr Asn Asn His Asn Thr Asn Gln Asn Gly Ser Thr
        435                 440                 445

Thr Tyr Gly Ser Asn Ala Asn Gly Thr Tyr Gly Pro Asn Gly Thr Tyr
    450                 455                 460

Gly Ser Asn Gly Thr Tyr Glu Pro Asn Glu Ser Tyr Gly Pro Asn Gly
465                 470                 475                 480

Ala Tyr Gly Pro Asn Gly Thr Tyr Gly His Asn Gly Lys Tyr Gly Ser
                485                 490                 495

Lys Gly Thr Tyr Gly Asn Asn Glu Thr Pro Tyr Tyr Val Glu His Pro
            500                 505                 510

Glu Tyr Asp Asn Gly Lys Ser Met Ser Asp Phe His Val Lys Asp Ser
        515                 520                 525

Lys Asp Asn Ile Gly Pro Gly Gly Asp Tyr Pro Asn Leu Tyr Gln Asn
    530                 535                 540

Ile Tyr Gly Asn Glu Lys Asn Pro Asn Ile Phe Pro Gly Ser Pro Arg
545                 550                 555                 560

Asn Ile Asn Val Tyr Ser Val His His Ile Pro Asn Asn Gly Ala Asn
                565                 570                 575

Gly Gly Leu Asn Ser Gly Ala Asn Gly Gly Leu Asn Asn Gly Ala Asn
            580                 585                 590

Asp Gly Leu Asn Asn Gly Ala Asn Gly Gly Leu Asn Asn Gly Ala Asn
        595                 600                 605

Gly Gly Leu Asn Asn Gly Ala Asn Gly Gly Leu Asn Asn Gly Met Asn
    610                 615                 620

Asn Gly Met Asn Asn Gly Met Asn Asn Gly Met Asn Asn Gly Met Asn
625                 630                 635                 640

Asn Gly Met Asn Asn Gly Met Asn Asn Gly Thr Asn Gly Gly Leu Asn
                645                 650                 655

Asn Gly Met Asn Asn Gly Met Asn Asn Gly Met Asn Asn Gly Met Asn
            660                 665                 670

Asn Gly Ile His Asp Asp Leu Tyr Asn Ser Glu Asn Ser Thr Phe Asn

675                 680                 685

Asn Gly Leu Asn Asn Ser Gly Arg Thr Gly Leu Asn Asn Ala Tyr Pro
    690                 695                 700

His Asn Gly Met Leu Asn Asn Gly Thr Glu Tyr Asn Val His Tyr Gly
705                 710                 715                 720

Asn Ser Asp Ser Asn Asn Thr Asn Asp Ser Met Leu Asn Glu Asn Tyr
                725                 730                 735

Tyr Ser Asp Ser Asp Tyr Asp Asp His Thr Pro Gly Asn Lys Lys Lys
            740                 745                 750

Val Tyr Lys Ser Val Ala Glu Arg Asn Lys Lys Ser Ala Ser Gln Asp
        755                 760                 765

Ser Leu Gly Ala Gly Phe Ser Asp Ser Asp Ser Asp Ser Glu Tyr Glu
    770                 775                 780

Val Val Asp Gly Glu Asn Lys Lys Tyr Lys Lys Lys Asn Lys Glu Asn
785                 790                 795                 800

Asn Glu Lys Asp Lys Tyr Glu Asn Asn Trp Asp Ser Asn Asn Tyr Asn
                805                 810                 815

Ser Asp Asn Glu Ile Lys Asp Gly Tyr Leu Ser Glu Ser Glu Arg Glu
            820                 825                 830

Tyr Ala Arg Asn Lys Ala Asn Glu Ile Glu Asp Lys Met Lys Lys Gly
        835                 840                 845

Glu Tyr Ser Arg Lys Tyr Lys Asn Ser Lys Ser Asn Glu Ser Gly Tyr
    850                 855                 860

Ala Ser Lys Gln Thr Ser Asp Ser Asp Asp Ser Asp Ile Glu Ala Asn
865                 870                 875                 880

Ala Phe Tyr Val Asp Asn Gly Gln Glu Met Leu Ile Lys Glu Lys Glu
                885                 890                 895

His Tyr Ser Ser Asp Ser Glu His His Lys Glu Glu Ser Ala Ser Ile
            900                 905                 910

Gly Asn Leu Asn Val Phe Phe Pro Ala Glu Asn Tyr His Phe Ser Thr
        915                 920                 925

Tyr Met Gly Phe Asp Arg Arg Ser Phe Leu Pro Ser Asn Glu Ile Glu
    930                 935                 940

Leu Glu Lys Met Ile Gly Ala Asn Phe Ser Asn Glu Val Lys Asn Tyr
945                 950                 955                 960

Cys Ser Arg Gln Asn Val Ala Gln Lys Ile Gly Asp Tyr Leu Asn Ile
                965                 970                 975

Ser Phe Glu Tyr Ser Arg Ala Leu Glu Glu Leu Arg Ser Glu Met Ile
            980                 985                 990

Leu Asp Phe Asn Lys Arg Lys His  Leu Thr Asn Asn Thr  Asp Asp Thr
        995                 1000                1005

Ile Leu  His Met Ile Glu Asn  Ala Glu Lys Arg Lys  Asn Asp Pro
    1010                1015                1020

Asn Tyr  Lys Glu Ala Tyr Glu  Asn Lys Asp Tyr Ala  Asn Asn Ala
    1025                1030                1035

Asn Ile  Phe Met Asn Glu Tyr  Ser Asn Pro Leu Ser  Thr Lys Tyr
    1040                1045                1050

Asn Lys  Ile Leu Lys Glu Tyr  Leu Cys His Leu Phe  Val Asn Asn
    1055                1060                1065

Pro Gly  Thr Lys Pro Leu Glu  Arg Leu Tyr Tyr Asn  Ser Leu Ala
    1070                1075                1080

Leu Gly  Glu Leu Val Glu Pro  Ile Arg Asn Lys Phe  Lys Ser Leu
    1085                1090                1095

Ala Ser  Ser Thr Ile Asp Phe  Asn Tyr Glu Ile His  Met Ala Ser
    1100                1105                1110

Ala Ser  Asn Ile Tyr Leu Leu  Ala His Phe Leu Val  Leu Ser Leu
    1115                1120                1125

Ala Tyr  Leu Ser Tyr Asn Glu  Tyr Phe Thr Thr Gly  Thr Lys Ser
    1130                1135                1140

Phe Tyr  Ser Leu Pro Thr Ile  Leu Thr Ala Asn Ser  Asp Asn Ser
    1145                1150                1155

Phe Phe Met Leu Asn Glu Met Cys Asn Ile His Tyr Asn Pro Asn
    1160                1165                1170

Lys Asn Phe Lys Lys Asp Ile Thr Phe Ile Pro Ile Glu Ser Arg
    1175                1180                1185

Pro Lys Arg Thr Thr Thr Phe Tyr Gly Glu Arg Arg Leu Thr Cys
    1190                1195                1200

Asp Leu Leu Glu Leu Val Leu Asn Ala Ile Met Leu Ile Asn Ile
    1205                1210                1215

Asn Glu Ile Asn Asn Val Phe Ser Asn Asn Asn Val Asp Gly Tyr
    1220                1225                1230

Glu Asn Ser Leu Ser Phe Ser His Asn Ala Ile Arg Ile Phe Ser
    1235                1240                1245

Lys Val Cys Pro Lys Ile Asn Asn Asp Asn Val Leu Lys Cys Glu
    1250                1255                1260

Phe Glu Glu Ser Ser Leu Tyr Asn Pro Lys Ile Ile Lys Asn Asp
    1265                1270                1275

Thr Ser Glu Lys Ser Ser Gln Lys Asn Leu Lys Lys Ala Phe Asp
    1280                1285                1290

Leu Leu Arg Thr Tyr Ala Glu Ile Glu Gly His Ser Ala Glu Gly
    1295                1300                1305

Ser Thr Ser Pro Tyr Tyr Val Ser Leu Ile Leu Asp Asp Met Lys
    1310                1315                1320

Tyr Asn Asp Phe Tyr Lys Tyr Thr Leu Trp Tyr Glu Pro Arg Glu
    1325                1330                1335

Leu Ile Tyr Gly Asp Ile Arg Gly Met Gln Met Lys Lys Lys Lys
    1340                1345                1350

Lys Thr Lys Tyr Ile Tyr Asn Asp Phe Met Lys Lys Ser Thr Gln
    1355                1360                1365

Leu Lys Lys Lys Leu Ile Lys Asn Asp Leu Lys Tyr Asn Leu Lys
    1370                1375                1380

Ser Lys Gly Leu Val Phe Leu Tyr Ala Met Ile Asp Lys Tyr Gly
    1385                1390                1395

Ser Ile Leu Asn Lys Ser Gln Lys Ala Lys Val Gln Phe Leu Asn
    1400                1405                1410

Ser Thr Ser Ser Ile Arg Tyr Tyr Leu Tyr Leu Asn Lys Val Ile
    1415                1420                1425

Phe Lys Ser Ala Lys Thr Tyr Leu Asp Ile Met Lys Arg Val Leu
    1430                1435                1440

Glu Glu Leu Gln Thr Ser Thr Asn Thr Pro Leu Lys Phe Leu Val
    1445                1450                1455

Arg Gly Asn Tyr Ile Glu Asn Ile Asn Asn Ile Ala Arg Asn Asp
    1460                1465                1470

Asn Met Phe Tyr Ala Asn Leu Phe Val Leu Thr Ala Leu Ser Arg
    1475                1480                1485

Arg Asp Pro Val Lys Asp Tyr Tyr Asn Asp Lys Arg Lys Met Leu
    1490                1495                1500

Ser Ala Thr Leu Ala Glu Lys Phe Ala Asn Ser Thr Ser Met Leu
    1505                1510                1515

Ile Pro His Lys Leu Arg Lys Leu Val Val Ser Met Lys Lys Gly
    1520                1525                1530

Leu Leu Lys Lys Lys Leu Leu Thr Ser Leu Ala Lys Val Lys Leu
    1535                1540                1545

Leu Gln His Ile Pro Ala His Met Leu Glu Asn Ile Thr Ser Ser
    1550                1555                1560

Ile Arg Phe Thr Thr His Thr Ile Ala Thr Met Gln Ile Ile Gln
    1565                1570                1575

Asn Ala Lys Tyr Met Ser Lys His Asn Phe Ser Gln Tyr Asp Ser
    1580                1585                1590

Lys Gly Met Leu Ala Arg Gln Ile Phe Thr Lys Gly Gly Phe Ala
    1595                1600                1605

Glu Tyr Ala Asp Asn Leu Met Ala Lys Trp Phe Ser Lys Gly Phe

1610                1615                1620

Glu Glu Tyr Lys Arg Glu Gln Ile Glu Asn Phe Lys Met Glu Asn
    1625                1630                1635

Ser Ile Asp Ser Glu Leu Lys Asp Ser Glu Arg Glu Asp Glu Asn
    1640                1645                1650

Asp Ser Ser Glu Glu Ser Ala Lys Lys Lys Leu Gln Asp Leu Gln
    1655                1660                1665

Leu Glu Glu Arg Glu Lys Met Lys Lys Glu Asn Ser Leu Leu Phe
    1670                1675                1680

Asn Gln Ser Asp Lys Trp Asp Gln Phe Ile Asn Lys Glu Leu Val
    1685                1690                1695

Arg Ala Leu Gly Leu Trp Leu Glu Phe Asn Asp Asn Pro Thr Asn
    1700                1705                1710

Ala Ser Ser Phe Val Tyr Lys Val Val Glu Asp Ser Lys His Leu
    1715                1720                1725

Leu Glu Asn Asn Ile Asp Asn Asn Ile Ile Phe Ser Arg Thr Val
    1730                1735                1740

Lys Pro Thr Lys Gln Thr Ala Phe Arg Arg Phe Phe Asn Lys Ile
    1745                1750                1755

Leu Ser Leu Gly Asn Met Leu Leu Arg Lys Pro Ser Phe Arg Val
    1760                1765                1770

Glu His Ala Leu Trp Phe Gly Ala Thr Ile Asp Ile Lys Lys Ala
    1775                1780                1785

Phe Ile Leu Leu Glu Lys Val Ser Glu Leu His Lys Met Leu Asn
    1790                1795                1800

Asn Gln Asp Glu Ser Trp Leu Ile Asn Glu Ala Phe Ile Glu Ile
    1805                1810                1815

Val Asp His Val Val Asp Leu Ser Thr Tyr Lys His Val Arg Glu
    1820                1825                1830

Pro Phe Gly Val Ala Arg Asn Pro Gly Met Met Ala Ile Asn Pro
    1835                1840                1845

Lys Tyr Ala Glu Leu Ser His Glu Asn Arg Leu Arg Glu Leu Gln
    1850                1855                1860

Asn Ser Met Cys Ala Asp His Cys Ser Ser Val Trp Lys Val Ile
    1865                1870                1875

Ser Ser Phe Ala Leu His His Leu Lys Asn Pro Asp Ser Leu His
    1880                1885                1890

Thr Tyr Glu Ser Lys Phe Ser Lys Asn Ser Phe Gly Asn Lys Ile
    1895                1900                1905

Asp Asp Lys Asp Phe Val His Asn Phe Lys Met Ile Leu Gly Gly
    1910                1915                1920

Asp Ala Val Leu His Tyr Phe Asp Asn Leu Leu Pro Lys Thr Met
    1925                1930                1935

Lys Lys Asp Leu Lys Ala Met Lys Tyr Gly Val Ser Leu Thr Ser
    1940                1945                1950

Ala Tyr Ser Leu Lys Leu Thr Lys Ile Ile Phe Ser Gln Met Gln
    1955                1960                1965

Leu Pro Tyr Leu Ser Gln Met Phe Tyr Met Gln Ala Pro Tyr Phe
    1970                1975                1980

Gly His Phe Ile Gly Lys Trp Gln Lys Lys Arg Gln Gln Ser Arg
    1985                1990                1995

Leu Lys Glu Ile Met Ser Phe Met Thr Leu Gly Ser Leu Ser Ala
    2000                2005                2010

Tyr Thr Leu Phe Ser Ala Met Asp Ile Thr Gln Gln Ala Lys Asp
    2015                2020                2025

Ile Gly Ala Gly Pro Val Ala Ser Cys Phe Thr Thr Arg Met Ser
    2030                2035                2040

Pro Pro Gln Gln Ile Cys Leu Asn Ser Val Val Asn Thr Ala Leu
    2045                2050                2055

Ser Thr Ser Thr Gln Ser Ala Met Lys Cys Val Phe Ser Val Gly
    2060                2065                2070

Leu Phe Ala Ser Ile Gly Pro Tyr Leu Phe Ala Pro Met Ala Gly
    2075                2080                2085

Leu Ala Val Trp Asn Ile Leu Lys Ser Glu Phe Lys Val Leu Gln
    2090                2095                2100

Arg Ile Asp Met Ala Leu Lys Asn Val Phe Lys Asn Met Trp Asn
    2105                2110                2115

Lys Phe Leu Ser Leu Lys Gly Ile Ser Lys Leu Arg Gly Ile Phe
    2120                2125                2130

Lys Arg Lys Lys Ala Met Lys Lys Lys Ile Ile Glu Asn Ala Thr
    2135                2140                2145

Arg Lys Met Asn Asp Met Lys Asn Asn Pro Glu Lys Ala Lys Ala
    2150                2155                2160

His Lys Met Ala Leu Lys Lys Ile Asn Asn Tyr Ser Lys Gly Ser
    2165                2170                2175

Tyr His Tyr Ile Ser Tyr Ala Lys Ile Arg Ile
    2180                2185

<210> 4
<211> 39
<212> PRT
<213> Artificial

<220>
<223> RON2 peptide (Plasmodium falciparum)

<400> 4

Asp Ile Thr Gln Gln Ala Lys Asp Ile Gly Ala Gly Pro Val Ala Ser
1               5                   10                  15

Cys Phe Thr Thr Arg Met Ser Pro Pro Gln Gln Ile Cys Leu Asn Ser
            20                  25                  30

Val Val Asn Thr Ala Leu Ser
        35

<210> 5
<211> 1365
<212> PRT
<213> Babesia Bovis

<220>
<221>  misc_feature
<223>  RON2

<400>  5

Met Phe Ala Val Thr Leu Ala Val Ile Thr Leu Val Ile His Asn Gly
1               5                   10                  15

Val Tyr Ala Ser Phe Ser Gln Lys Thr Ser Gly Val Phe Ser Arg Ser
            20                  25                  30

Gly Ala Leu Val Ala Pro His Pro Ser Thr Ile Ala Lys Leu Arg Leu
        35                  40                  45

Leu Asp Ala Leu Ser Gln Pro Pro Lys Asp Pro Asn Ala Thr Glu Asp
    50                  55                  60

Glu Ser Lys Val Ala Glu Lys Ser Pro Glu Ala Ser Thr Asn Ala Pro
65                  70                  75                  80

Glu Lys Pro Ala Glu Thr Ser Glu Pro Thr Val Ser Asn Gln Thr Ala
                85                  90                  95

Ala Ser Ser Glu Pro Asp Gln Asn Ser Val Asp Ala Ser Ala Gln Asn
            100                 105                 110

Ala Asp Gln Phe Gly Gly Phe Ser Asp Asn Leu Ser Met Pro Asn Gly
        115                 120                 125

Glu Ala Trp Gln Gly Val Ser Asp Ser Pro Leu Gln Glu His Ala Ala
    130                 135                 140

Ala Thr Asn Thr Ala Asn Leu Ala Val Leu Glu Lys Cys Ile Gly Ile
145                 150                 155                 160

Glu Gly Phe Ser Gln Pro Phe Thr Asn Gly Ile Ser Asp Glu Phe His
                165                 170                 175

Leu Leu Leu Gln Ser Arg Lys Thr Ala Ser Met Val Asp Ala Tyr Met
            180                 185                 190

Ser Tyr Ala Leu Lys Leu Glu Ala Ile Glu Lys Glu Ser Tyr Lys Arg
        195                 200                 205

Arg Val Lys Leu Val Glu Glu Lys Ser Asp Ala Ile Gly Phe Val Asp
    210                 215                 220

Phe Ser Glu Arg Gln Arg Gly Ser Leu Met Pro Glu Val Asn Lys Glu
225                 230                 235                 240

Ala Met Lys Val Ala Gly Val Thr Ser Leu Leu Gln Leu Gly Glu Tyr
                245                 250                 255

Ala Gly Pro Thr Pro Thr Ser Lys Ser Ala Asn Thr Ala Lys Ala Asp
            260                 265                 270

Ala Ile Gln Ala Asn Gln Thr Lys Ile Thr Thr Ser Gly Asn Gly Gln
        275                 280                 285

Gln Thr Thr Thr Glu Glu Pro Ile Thr Trp Lys Arg Asn Gly Ala Val
    290                 295                 300

Gln Glu Ala Ile Leu Ser Lys Ala Leu Leu Leu Leu Gly Gly Asp Ser
305                 310                 315                 320

Glu Pro Glu Thr Tyr Lys Arg Leu Thr Asn Met Ala Thr Ala Leu Gly
                325                 330                 335

Tyr His Thr Ala Glu Gly Lys Asp Lys Thr Pro Lys Lys Lys Thr Val
            340                 345                 350

Ala Leu Ser Leu Gly Met Glu Leu Phe Cys Val Cys Gly Asn Asn Pro
        355                 360                 365

Phe Leu Leu Gly His Leu Ala Thr Asn Met Leu Ala Tyr Thr Gln Tyr
    370                 375                 380

Asn Met Phe Phe Ala Gly Gly Ser Gly Arg Pro Phe Tyr Thr Trp Leu
385                 390                 395                 400

Asp Leu Val Ser Ser Gly Asn Leu Asp Met Leu Asp Arg Met Cys Gly
                405                 410                 415

Thr Lys Arg Gly Pro Lys Tyr Lys Arg Ser Ser Asp Gly Ser Val Thr
            420                 425                 430

Val Asn Lys Lys Arg Arg Arg Lys Asp Asp Pro Asp Leu Asp Ala Asp
        435                 440                 445

Gly Val Phe Leu Cys Asn Leu Leu Glu Ile Leu Leu Ile Ser Ile Asp
    450                 455                 460

Ala Ser Ile Asp Asp Met Gly Gln Leu Leu Ser Lys His Gly Val Pro
465                 470                 475                 480

Tyr Glu His His Ile Gly Pro Val Ala Asn Gly Arg Arg Met Gln Ser
                485                 490                 495

Val Leu Cys Ser Asn Lys Lys Asp Ala Ala Ile Ser Val Arg Cys Asp
            500                 505                 510

Phe Val Ser Ser Thr Leu Asn Thr Thr Lys Ile Phe Glu Gly Lys Ser
        515                 520                 525

Lys Asp Glu Gly Thr Asp Leu Trp Met Arg Leu Arg Glu Ala Phe Asp
    530                 535                 540

Phe Phe Lys Leu Leu Ser Asp Val Asn Leu Asp Thr Glu Gln Arg Thr
545                 550                 555                 560

Thr Trp Met Gln Met Ile Ser Asp Pro Arg Lys Tyr Thr Ser Ile Leu
                565                 570                 575

Glu Tyr Ala Ile Lys Phe Asp Asn Lys Met Phe Thr Gly Lys Lys Arg
            580                 585                 590

Thr Trp Met Leu Ala Tyr Lys Ser Val Glu Ser Lys Val Thr Pro Gly
        595                 600                 605

Leu Asp Asn Val Asn Leu Asp Ile His Ser Ala Ile Ser Met Leu Asp
    610                 615                 620

Ser Lys Ala Ala Asn Asn Ser Lys Lys Asn His Pro Leu Lys Arg Ala
625                 630                 635                 640

Leu Leu Tyr Met Ser Ala Ser Gly Ile Lys Gln Trp Val Val Asn Asn
                645                 650                 655

Leu Glu Asn Leu His Glu Arg Phe Gly Phe Pro Pro Ser Ala Leu Leu
            660                 665                 670

Gly Gly Asn Leu Ala Pro Tyr Phe Arg Gln Val Ala Gln Ser Thr Ser
        675                 680                 685

Gly Gly Leu Gly His Ile Val Leu Tyr His Met Leu Thr Ser Gln Asn
    690                 695                 700

Pro Ala Tyr Glu Leu Ile Ala Asp Met Asn Gly Phe Arg Gly Gly Asn

705                 710                 715                 720

Met Leu Tyr Asn Ile Val Glu Ser Ser Asn Met Phe Ile Pro Ala Ser
                725                 730                 735

Leu Lys Arg Gly Ile Lys Trp Met Leu Lys Gly Gly Leu Ala Gln Glu
            740                 745                 750

Phe Arg Arg Glu Lys Ala Lys His Thr Leu Leu Gln Leu Leu Pro Val
        755                 760                 765

Glu Leu Leu Arg Lys Ala Ile Ser Cys Ile Thr Phe Val Thr His Ser
    770                 775                 780

Leu Ala Asp Ile Gln Ile Asn Gln Asn Ala Glu Val Phe Gly Arg Gly
785                 790                 795                 800

Leu Leu Ala Asp Lys Asp Arg Ile Lys Lys His Phe Val Ala Gly Gly
                805                 810                 815

Tyr Ile Asn His Val Asp Ser Ile Ile Lys Glu Trp Ser Asp Glu Gly
            820                 825                 830

Tyr Thr Asp Ala Ile Ala Lys Lys Val Lys Gln Gly Glu Asp Leu Asn
        835                 840                 845

Lys Glu Asp Met Glu Asp Ala Asn Ile His Asn Ile Val His Thr Glu
    850                 855                 860

Ser Leu Arg Trp Glu Lys His Leu Asn Ser Leu Ile Leu Asp Gly Tyr
865                 870                 875                 880

Asn Ala Phe Leu Asp Leu Pro Ser Ile Lys Val Leu Asp Gly Arg His
                885                 890                 895

Ser Leu Ile Tyr Glu Ile Val Lys Asp Ser Arg Asp Asn Leu Glu Gln
            900                 905                 910

His Leu Asp Glu Thr Ile Phe Phe Gly Arg Val Val Asn Pro Pro Glu
        915                 920                 925

Tyr Asn Asn Lys Trp Lys Arg Ala Phe Asp Arg Ala Ala Lys Val Thr
    930                 935                 940

Lys Ser Ile Leu Asn Arg Thr Gln His Lys Val Glu His Ala Val Trp
945                 950                 955                 960

Phe Gly Val Lys Leu Asn Tyr Gln His Ile Ile Glu Val Val Glu Glu
                965                 970                 975

Leu Asn Arg Ile Ser Asn Val Ile Ser Pro Thr Glu Ser Tyr Asn Leu
            980                 985                 990

Gln Glu Ala Phe Gly His Ile Ile  Asp Asp Ala Val Ala  Val Val Ser
        995                 1000                1005

Asn Asp  Glu Phe Arg Arg Pro  Ser Ser Thr Gln Glu  Asn Leu Gly
    1010                 1015                 1020

Ile Pro  Gly Ile Asn Pro Leu  Tyr Thr Arg Met Ala  Pro Asp Glu
    1025                 1030                 1035

Arg Lys  Leu Glu Phe Gln Gln  Gly Met Cys Gly Gln  His Cys Gly
    1040                 1045                 1050

Ala Ile  Trp Arg Ala Leu Leu  Ala Phe Thr Met Asn  Ala Leu Arg
    1055                 1060                 1065

Ser Pro  Ala Ser Ile Lys Thr  Phe Glu Asn Thr Leu  Arg Gln Gly
    1070                 1075                 1080

Thr Ser  Leu Lys Asp Met Glu  Lys Pro Glu Phe Val  Asn Ser Leu
    1085                 1090                 1095

Arg Phe  Ile Leu Lys Gly Asp  Ala Met Leu His Met  Tyr Asp Ser
    1100                 1105                 1110

Met Leu  Pro Arg Lys Met Lys  Arg Glu Leu Arg Ala  Ile Lys Tyr
    1115                 1120                 1125

Gly Lys  Ala Phe Tyr Phe Ala  Asn Ile Met Lys Met  Ala Ser Thr
    1130                 1135                 1140

Leu Leu  Gly Met Ile Gly Phe  Arg Tyr Thr Ser Asn  Met Leu Arg
    1145                 1150                 1155

Ile Gln  Ala Pro Tyr Phe Gly  Asn Met Ile Val Arg  Trp Asp Arg
    1160                 1165                 1170

Glu Arg  Glu Lys Ser Arg Ser  Lys Ala Ile Phe Ser  Tyr Leu Ser
    1175                 1180                 1185

Ile Gly Thr Met Ala Thr Tyr Ser Ile Leu Gln Cys Ala Asp Ile
    1190                1195                1200

Ala Gln His Ala Ala Asp Val Gly Val Gly Pro Ala Glu Ser Cys
    1205                1210                1215

Phe Ile Met Val Lys Pro Pro Ala Leu His Cys Val Leu Lys Pro
    1220                1225                1230

Val Glu Thr Leu Met Lys Ser Ala Leu Thr Ile Gly Val Gln Asp
    1235                1240                1245

Thr Leu Ala Val Thr Val Leu Gly Leu Ile Gly Pro Tyr Phe Phe
    1250                1255                1260

Leu Pro Met Met Ala Tyr Ala Ser Trp Asn Ile Leu Lys His His
    1265                1270                1275

Phe Lys Val Leu His Arg Leu Asp Ile Ala Leu Ser Gly Thr Phe
    1280                1285                1290

Lys Arg Met Trp Ser Lys Ile Ser Ser Leu Ser Val Thr Lys Lys
    1295                1300                1305

Leu Thr Ser Trp Phe His Lys Arg Arg Glu Tyr Arg Lys Glu Ile
    1310                1315                1320

Glu Ser Lys Gly Leu Met Ala Met Lys Asn Arg Gln Ser Ala Ser
    1325                1330                1335

Gly Glu Lys Thr Gln Gly Gly Val Ala Val Asp Glu Glu Met Leu
    1340                1345                1350

Lys Lys Asp Asp Asn Phe Ser Tyr Thr Val Phe Ser
    1355                1360                1365

<210>  6
<211>  38
<212>  PRT
<213>  Artificial

<220>
<223>  RON2 peptide (Babesia Bovis)

<400>  6

Asp Ile Ala Gln His Ala Ala Asp Val Gly Val Gly Pro Ala Glu Ser
1               5                   10                  15

Cys Phe Ile Met Val Lys Pro Pro Ala Leu His Cys Val Leu Lys Pro
            20                  25                  30

Val Glu Thr Leu Met Lys
        35

<210>  7
<211>  1410
<212>  PRT
<213>  Eimeria tenella

<220>
<221>  misc_feature
<223>  RON2

<400>  7

Met Gly Val Phe Ser Arg Leu Leu Ser Ala Ala Leu Leu Cys Ala Ala
1               5                   10                  15

Ala Ser Pro Ala Leu Leu Ser Pro Gln Ala Ala Ala Ala Ala Ala Ala
            20                  25                  30

Pro Ala Glu Gly Ser Thr Ser Phe Thr Leu Pro Ser Gly Gly Ser Leu
        35                  40                  45

His Val Gln Val Leu Arg Gly Pro Gln Gln Gln Gln Gln Gln Gln Gln
    50                  55                  60

Gln Gly Pro Trp Gly Ser Thr Met Ser Phe Ala Gly Pro Thr Gly Ala
65                  70                  75                  80

Thr Gly Gly Tyr Ser Val Thr Asp Ser Ala Thr Leu Arg Ser Ser Ala
                85                  90                  95

Gly Pro Pro Ala Ala Ala Ala Ala Ala Ala Ala Ala Glu Gly Val Tyr
            100                 105                 110

Glu Leu Pro Thr Pro Pro Thr Pro Glu Asn Pro Ile Ser Lys Thr Gln
        115                 120                 125

Val Leu Phe Gly Trp Val Pro Glu Ala Ala Ala Ala Ala Ser Leu Gln
    130                 135                 140

Ala Val Asn Ala Glu Glu Leu Asn Asn Ala Leu Arg Ser Gln Ala Phe
145                 150                 155                 160

Val Arg Lys Leu Arg Thr Arg Ala Leu Val Glu Lys Ser Cys Met Ile
                165                 170                 175

Pro Leu Ala Ser Asp Arg Arg Ser Tyr Leu Glu Ala Phe Trp Ser Ala
            180                 185                 190

Ala Ala Ala Glu Arg Lys Phe Arg Ala Glu Ala Glu Ser Gln Arg Ala
        195                 200                 205

Ala Ile Glu Glu Glu Leu Lys Gln Gln Gln Met Ala Ala Pro Asp Met
    210                 215                 220

Ser Tyr Ala Arg Ser Val Ala Thr Arg Ala Asn Ile Glu Ser Gly Ala
225                 230                 235                 240

Ser Pro Ala Ala Ala Ala Ala Ala Ala Ala Gly Ala Ala Ala Ala Ala
                245                 250                 255

Ala Ala Gly Glu Glu Ala Ala Ala Ala Ala Leu Arg Tyr Gln Glu Thr
            260                 265                 270

Val Asp Arg Val Leu Ala Asn Arg Ile Glu Val Leu Met Thr Cys Lys
        275                 280                 285

Leu Ala Ala Leu Leu Gly Gln Pro Gly Ile Phe Leu Asn Asp Lys Ile
    290                 295                 300

Ala Ala Ala Glu Val Leu Asp Leu Val Ala Thr Ala Leu Gly Ile Gly
305                 310                 315                 320

Asn Thr Lys Glu Glu Ala Ala Ala Ala Ala Ala Ser Pro Ile Val Pro
                325                 330                 335

Met Phe Gly Gly Leu Glu Pro Phe Leu Leu Ala Ser Asn Pro Leu Ser
            340                 345                 350

Lys Leu His Pro Ala Ala Ala Ala Ala Ala Val Phe Val Leu Val Pro
        355                 360                 365

Pro Ala Ala Ala Ala Val Val Ala Ala Ala Ala Val Gly Val Gln Gln
    370                 375                 380

Asp Pro Lys Leu Arg Arg Arg Ser Cys Glu Leu Ser Ala Ala Ala Ala
385                 390                 395                 400

Ala Ala Ala Ala Ala Ala Ala Val Val Gly His Val Leu Thr Leu Leu
                405                 410                 415

Ile Ala His Ile Asp Arg Glu Ala Phe Phe Gly Glu Ala Ala Arg Lys
            420                 425                 430

Ala Phe Tyr Ser Phe Thr Ser Leu Ala Ala Ala Ala Gly Gly Glu Gly
        435                 440                 445

Ala Val Gly Met Leu Asp Glu Met Cys Asp Arg Asp Arg Gly Pro Arg
    450                 455                 460

Leu Ala Ala Pro Leu Leu Gly Lys Tyr Arg Pro Lys Gly Arg Gly Gly
465                 470                 475                 480

Lys Pro Arg Lys Arg Gly Ser Ser Ser Pro Trp Phe Val Arg Gly Ala
                485                 490                 495

Ser Arg Val His Arg Asn Lys Leu Gln Pro Glu Leu Leu Arg Ala Tyr
            500                 505                 510

Cys Asp Ala Thr Glu Met Ile Leu Asn Ala Leu Met Leu Ser Glu Leu
        515                 520                 525

Leu Leu Leu Leu Leu Leu Leu Leu Leu Leu Leu Leu Leu Arg Gly Glu
    530                 535                 540

Gln Glu Asp Val Gln Gln Glu Met Leu Lys Phe Ser Leu Pro Val Glu
545                 550                 555                 560

Pro Leu Val Asp Pro Ala Thr Asn Ala Ser Arg Ile Gln Thr Lys Thr
                565                 570                 575

Cys Arg Gly Gly Ala Pro Val Cys Glu Phe Glu Asn Ser Ile Leu Ser
            580                 585                 590

Pro Ile Ala Asn Pro Leu Asp Pro Gln Val Leu Glu Gln Asn Thr Asn
        595                 600                 605

Thr Arg Ala Ala Phe Asn Leu Tyr Ala Gly Leu Ala Ser Ala Gln Leu
    610                 615                 620

Gly Asn Leu Leu Glu Glu Thr Gly Ser Lys Tyr Tyr Asp Val Arg Ala
625                 630                 635                 640

Asp Gln Trp Leu Glu Ile Ile Ser Lys Pro Thr Ala His Ser Asp Ile

645                 650                 655

Leu Glu Lys Val Phe Phe Tyr Asp Asn Arg Glu Phe Leu Ala Ser Lys
            660                 665                 670

Arg Ser Lys Leu Ile Lys Ser Phe Glu Thr Asn Ala Lys Lys Ile Ala
        675                 680                 685

Ala Leu Ala Gly Lys Pro Ala Val Ser Ile Ser Glu Ala Ala Ala Ala
    690                 695                 700

Tyr Glu Glu Ile Gln Arg Ser Val Gly Ser Gln Thr Gly Lys Lys Gly
705                 710                 715                 720

Ala Ala Ala Ala Lys Lys Asn Leu Phe Pro Thr Ala Ala Thr Leu Leu
                725                 730                 735

Leu Lys Ser Thr Leu Ala Cys Asp Met Thr Ser Ser Phe Ala Ala Lys
            740                 745                 750

Leu Glu Ser Phe Thr Gln Phe Met Phe Lys Ala Lys Ala Ala Ser Gly
        755                 760                 765

Arg Arg Gln Ala Pro Leu Gln Arg Thr Leu Met Ala Phe Ile Arg Thr
    770                 775                 780

Gly Lys Gly Ser Ala Leu Gln Ser Met Cys Ser Tyr Asp Pro Leu Thr
785                 790                 795                 800

Phe Asn Tyr Leu Phe Leu Tyr Arg Phe Val Leu Leu Gly Ser Asp Pro
                805                 810                 815

Ala Lys Thr His Asp Lys Gln His Ala Ala Val Ser Lys Thr Pro Leu
            820                 825                 830

Thr Thr Arg Leu Leu Gly Ser Thr Trp Thr Pro Ser Ile Leu Lys Arg
        835                 840                 845

Val Leu Arg Gly Val Lys Arg Lys Ser Ser Ile Leu Lys Ala Lys Gln
    850                 855                 860

Leu Leu Leu Glu Ser Leu Asp Ala Gly Val Phe Pro Leu Leu Leu Thr
865                 870                 875                 880

Ser Phe Glu Trp Ile Val His Thr Gln Ala Ala Leu Gln Val Asn Gln
                885                 890                 895

Asn Ser Glu Met Phe His Glu Leu Glu Ala Gln Gln Pro Arg Ala Leu
            900                 905                 910

Pro Ala Glu Pro Gln Lys Ala Ala Asp Ala Asn Leu His Asp Gly Gly
        915                 920                 925

Leu Val Lys Arg Thr Asp Glu Gln Leu Ala Glu Trp Ala Glu Phe Gly
    930                 935                 940

Val Pro Ala Ala Leu Arg Glu Gln Leu Arg Arg Gly Glu Lys Leu Pro
945                 950                 955                 960

Lys Ala Val Ala Leu Glu Arg Ile Pro Thr Pro Asp Leu Thr Gln Trp
                965                 970                 975

Glu Gln Gln Leu Asn Arg Lys Trp Leu Ala Ala Leu Glu Ala Tyr Leu
            980                 985                 990

Arg His Pro Tyr Gly Ala Ala Ala Ala Ala Ala Arg Asp Pro Val Ala
        995                 1000                1005

Leu Leu Val Gln Arg Ser Arg Asp Arg Leu Glu Ala Glu Leu Asp
    1010                1015                1020

Ser Thr Ile Phe Leu Gly Arg Ile Val Gly Ala Pro Arg Gly Pro
    1025                1030                1035

Arg Arg Gly Arg Arg Ala Leu Arg Ala Val Gly Gly Leu Phe Arg
    1040                1045                1050

Ala Leu Leu Arg Ala Pro Arg Gln Ser Glu Phe Ala Val Trp Met
    1055                1060                1065

Gly Val Lys Val His Ala Asp Arg Val Leu Arg Val Leu His Ala
    1070                1075                1080

Val His Ala Ala Ala Glu Val Ala Lys Thr Ala Gly Leu Phe Glu
    1085                1090                1095

His Val Arg Glu Ala Phe Leu Glu Leu Val Arg Asp Ala Val Leu
    1100                1105                1110

Gly Ser Leu Gln Asn Gln Leu Arg Val Pro Gly Phe Asp Thr Phe
    1115                1120                1125

Ala Ala Val Glu Ala Gly Leu Arg Gly Gly Glu Val Ser Ala Ala
    1130                1135                1140

Ala Ala Ala Ala Ala Ala Ala Ala Ala Ala Ala Arg Arg Ser Ala
    1145                1150                1155

Gly Phe Leu Ser Val His Pro Glu Leu Ala Ala Leu Gly Pro Ala
    1160                1165                1170

Ala Arg Glu Ala Glu Phe Gln Asn Ser Met Cys Met Asp His Cys
    1175                1180                1185

Glu Ala Leu Trp Thr Leu Val Thr Ser Leu Val Phe Ser Ala Leu
    1190                1195                1200

Gln Asn Pro Arg Lys Trp Arg Asp Tyr Glu Lys Glu Val Gly Gly
    1205                1210                1215

Ala Ala Ala Ala Ala Ala Leu Ala Asp Pro Arg Arg Val Asn Ser
    1220                1225                1230

Phe Arg Leu Gly Leu Ser Val Gln Thr Asp Phe Phe Glu Asn Val
    1235                1240                1245

Leu Asp Lys Lys Ser Lys Arg Asn Ile Gln Lys Met Lys Phe Gly
    1250                1255                1260

Gly Gly Ser Trp Phe Ala Tyr Ala Leu Leu Leu Ala Ala Arg Leu
    1265                1270                1275

His Arg Gly Leu Gly His Ser Asp Leu Ala Thr Phe Phe Ser Phe
    1280                1285                1290

Gln Ala Pro Tyr Leu Gly His Phe Val Leu Gln Trp Gln Gln Gln
    1295                1300                1305

Arg Arg Glu Ala Arg Arg Lys Ala Leu Leu Ser Met Leu Ser Leu
    1310                1315                1320

Gly Phe Phe Phe Ala Tyr Thr Phe Ile Ser Val Ser Asp Ile Thr
    1325                1330                1335

Gln His Leu Asn Asp Ser Gly Leu Gly Pro Ala Val Glu Cys Leu
    1340                1345                1350

Glu Asn  Leu Val Val Gly Pro  Val Cys Pro Ala Ala  Val Val Ala
    1355                 1360                 1365

Pro Ala  Val Arg Ser Ala Ala  Ala Ala Ala Ala Ala  Asp Val Phe
    1370                 1375                 1380

Lys Val  Gly Leu Phe Trp Leu  Leu Thr Pro Tyr Leu  Val Trp Pro
    1385                 1390                 1395

Met Ala  Ala Ala Ala Ala Trp  Ala Leu Leu Arg Asn
    1400                 1405                 1410

<210>  8
<211>  37
<212>  PRT
<213>  Artificial

<220>
<223>  RON2 peptide (Eimeria tenella)

<400>  8

Asp Ile Thr Gln His Leu Asn Asp Ser Gly Leu Gly Pro Ala Val Glu
1               5                   10                  15

Cys Leu Glu Asn Leu Val Val Gly Pro Val Cys Pro Ala Ala Val Val
            20                  25                  30

Ala Pro Ala Val Arg
        35

<210>  9
<211>  1479
<212>  PRT
<213>  Neospora caninum

<220>
<221>  misc_feature
<223>  RON2

<400>  9
   Met Thr Arg Glu Cys Ala Asn Leu Pro Leu Gly Arg Thr Phe Leu Leu
   1               5                   10                  15
   Leu Val Leu Leu Ser Ala Ala Thr Asp Ser Pro Val Ala Ser Arg Ser
               20                  25                  30
   Arg Val Leu Gln Phe Phe Pro Ser Phe Ser Val Ile Pro Ser Glu Ala
           35                  40                  45
   Ala Gln Pro Asp Ala Ser Gly Asn Trp Gln Pro Gly Gln Gly Gly Gly
       50                  55                  60
   Phe Arg Pro Thr Gly Tyr Gln His Ile Val Thr Lys Thr Val Asn Glu
   65                  70                  75                  80
   Glu Leu Pro Glu Asp Leu Lys Val Tyr Thr Ser Gly Ser Pro Gly Ser
                   85                  90                  95
   Ser Val Gly Ala Thr Val Asp Phe Gly Ser Gly Gly Tyr Phe Phe Ala
               100                 105                 110
   Asn Thr Gly Gly Arg Pro Asp Glu Ser Thr Leu Thr Val Phe Thr Ala
           115                 120                 125
   Gly Gly Pro Leu Pro His Ala Ser Trp Ser Trp Ala Gly Ser Pro Pro
       130                 135                 140
   Glu Ile Gln Thr Thr Gly Ala Ser Gln Ile Ser Phe Gly Trp Val Pro
   145                 150                 155                 160
   Glu Thr Glu Thr Pro Ser Leu Ser Glu Arg Asn Ser Leu Glu Leu Lys
                   165                 170                 175
   Lys Met Leu Arg Asp Glu Gly Leu Val Glu Ala Leu Gln Leu Arg Ala
               180                 185                 190
   Ala Glu Lys Gly Cys Pro Val Thr Val Leu His Asn Leu Arg Gln Ile
           195                 200                 205
   Gln Val Ser Phe Arg Glu Ile Leu His Glu Glu Ile Glu Ser Arg Ser
       210                 215                 220
   Asn Pro Ala Lys Leu Tyr Gln Val Ala Asn Ser Tyr Val Arg His Gly
   225                 230                 235                 240
   Val Gly Ser Gly Ala Thr Ser Trp Gly Thr Ala Gln Ser Val Glu Phe
                   245                 250                 255
   Ser Leu Leu Asp Met Gln Ala Ser Ser Thr Ala Asn Arg Asn Pro Ser
               260                 265                 270
   Ser Ala Ser Ser Phe Phe Glu Thr Arg Ala Gly Ala Ala Pro Glu Ser
           275                 280                 285
   Glu Gly Ile Arg Val Ser Arg Thr Pro Pro Pro Pro Arg Ala Val Pro
       290                 295                 300
   Leu Gln Arg Asp Gln Ser Glu Gly Asp Arg Asn Pro Thr Ala Lys Arg
   305                 310                 315                 320
   Leu Val Gly Thr Gln Leu Gly Leu Tyr Leu Glu Cys Lys Leu Ala Ala
                   325                 330                 335
   Leu Leu Gly His Pro Thr Leu Phe Leu Asn Pro Tyr Tyr Thr Glu Gln
               340                 345                 350
   Gln Leu Leu Glu Ala Val Ala Glu Ala Leu Gly Leu Ala Pro Pro Asn
           355                 360                 365
   Arg Arg Glu Thr Glu Ala Gly Ala Ser Ser Glu Ala Glu Gln His Asn
       370                 375                 380
   Gly Ser Val Asp Gln Leu Leu Gly Ala Ile Glu Ile Phe Arg Leu Gly
   385                 390                 395                 400
   Pro Asn Pro Tyr Thr Leu Gly His Val Leu Ser Leu Met Ile Gly Tyr
                   405                 410                 415
   Leu Asp Tyr Asn Ala Phe Phe Gly Asn Ser Pro Ser Lys Pro Phe Tyr
               420                 425                 430
   Ser Trp Val Thr Leu Ala Ala Ser Ala Gly Asn Asp Thr Gly Phe Ala
           435                 440                 445
   Met Leu Asp Glu Met Cys Asp Asn His Arg Gly Pro Lys Arg Arg Gly
       450                 455                 460
   Ser Lys His Trp Tyr Gln Arg Gly Gly Ser Arg Lys His Lys Ser Glu
   465                 470                 475                 480
   Thr Met Ser Pro Leu Gln Arg His Leu Cys Asp Ala Leu Glu Ile Val
                   485                 490                 495
   Leu Asn Gly Val Gln Gln Ile Gln Ile Glu Leu Met Asp Glu Leu Gly
               500                 505                 510
   Lys Tyr Gln Thr Gly Val Glu Pro Leu Val Asp Pro Ser Thr Asn Ser
           515                 520                 525
   Ala Arg Ile His Thr Arg Val Cys Arg Gly Leu Ser Pro Val Cys Asp
   530                 535                 540
   Tyr Glu Ala Thr Ile Leu Ser Pro Val Arg Pro Leu Glu Ser His Glu
   545                 550                 555                 560
   Gln Gln Asp Ser Leu Lys Thr Lys Lys Ala Phe Asn Leu Ile Thr Gly
                   565                 570                 575
   Tyr Gly Ser Gly His Val Gly Gln Ile Ser Gly Ser Ile Ala Glu Pro
               580                 585                 590
   Phe Ser His Thr Trp Arg Thr Arg Trp Gly Lys Val Val Ala Asp Pro
           595                 600                 605
   Thr Ala Tyr Pro Glu Ile Leu Glu Arg Ala Leu Trp Phe Asp Asp Arg
       610                 615                 620
   Glu Leu Met Ala Lys Gln His Gly Ala Leu Phe Lys Gln Tyr Asp Lys
   625                 630                 635                 640
   Ile Thr Lys Asp Ala Met Ser Phe Gly Ser Phe Ala Ala Val Glu Asn
                   645                 650                 655
   Gly Leu Ala Lys Arg Asp Ile Arg Ser Lys Leu Glu Gly Tyr Thr Ser
               660                 665                 670
   Gln Arg Lys Glu Phe Val Val Lys Arg Gln Gln Ser Arg Leu Ser Arg
           675                 680                 685
   Phe Arg Lys Lys His Ser Glu Gln Asp Pro Leu Ala Phe Arg Thr Ala
       690                 695                 700
   Ile Leu Leu Ser Leu Asn Ser Arg Thr Tyr Cys Thr Gln Thr Lys Ser
   705                 710                 715                 720
   Phe Ile Ser Ser Phe Arg Ala Phe Leu Ser Arg Gln Phe Gln Lys Leu
                   725                 730                 735
   Ser Gln Gly Arg Asn Leu Pro Lys Thr Gln Arg Ser Leu Met Ala Phe
               740                 745                 750
   Leu Arg Thr Gly Gln Val Lys Phe Tyr His Glu Trp Cys Ser Phe Asp
           755                 760                 765
   Pro Leu Ala Thr Asn Ala Leu Phe Met Leu Arg Phe Ala Leu Ser Gly
       770                 775                 780
   Asn Asp Pro Ala Ala Leu Asn Asp Arg Gln His Thr Arg Val Gly Arg
   785                 790                 795                 800
   Ser Lys Lys Thr Leu Arg Ile Leu Gln Ser Lys Trp Thr Pro Ala Met
                   805                 810                 815
   Leu Lys Lys Leu Met Arg Gly Ile Asn Gln Lys Lys Met Ala Gln Glu
               820                 825                 830
   Ala Lys Ala Leu Leu Tyr Arg Ser Leu Asp Ser Ser Val Leu Ser Ser
           835                 840                 845
   Ile Leu Thr Ser Phe Asp Phe Ile Thr His Thr Gln Ala Asn Leu Glu
       850                 855                 860
   Val Asn Gln Asn Ala Phe Met Tyr His Glu Val Gln Ala Ser Ala Val
   865                 870                 875                 880
   Ser Arg Lys Ser Ser Ala Asp Lys Ala Lys His Gln Leu His Ala Glu
                   885                 890                 895
   Gly Leu Val Arg His Thr Asp Asp Val Ile Lys Lys Trp Ala Asn Tyr
               900                 905                 910
   Gly Ile Pro Gly Asp Ile Lys Arg Arg Leu Ala Arg Gly Glu Lys Leu
           915                 920                 925
   Pro Glu Gly Thr Ser Phe Gly Gly Ile Pro Ile Pro Asp Leu Thr Asn
       930                 935                 940
   Trp Asp Ala Gln Leu Asn Gln Lys Trp Leu Asp Ala Tyr Asn Ala Tyr
   945                 950                 955                 960
   Leu Lys His Pro Tyr Gly Arg Ala Ala Leu Ser Ala Lys Asp Pro Val
                   965                 970                 975
   Ala Met Leu Val Arg Asp Ser Arg Asp Arg Leu Gln Ala Glu Gly Glu
               980                 985                 990
   Gly Thr Ile Phe Leu Gly Arg Ile  Ala Lys Arg Val His  Arg Ser Arg
           995                 1000                1005
   Asn Leu  Phe Arg Arg Ala Gly  Arg Ala Leu Lys Thr  Phe Phe Leu
       1010                1015                1020
   Ser Leu Leu Arg Glu Asn Glu Gln Ser Glu Tyr Ala Val Trp Phe
   1025 1030 1035
   Gly Val Lys Val Asp Met Arg Gln Val Ile Gln Thr Cys Arg Ala
   1040 1045 1050
   Val Asn Ala Val Ala Glu Val Val Lys Asn Asp Arg Leu Tyr Gly
   1055 1060 1065
   Tyr Val Thr Asp Gly Trp Leu Glu Leu Val Lys Asp Val Val Ala
   1070 1075 1080
   Gly Tyr Thr Arg Ala Pro Leu Arg Val Pro Gly Tyr Asp Ala Ile
   1085 1090 1095
   Ser Ala Ala Ser Glu Gln Met Arg Lys Lys Gly Glu Ala Ala Ala
   1100 1105 1110
   Thr Ala Arg Asn Gln Gly Phe Leu Ser Leu His Tyr Asp Tyr Ala
   1115 1120 1125
   His Leu Ser Glu Ala Asp Arg Arg Lys Glu Phe Gln Gln Ser Met
   1130 1135 1140
   Cys Met Glu His Cys Glu Ala Ile Trp Lys Leu Ile Met Ala Phe
   1145 1150 1155
   Val Met Pro Asn Leu Gln Asn Pro Lys Lys Leu Lys Gly Tyr Glu
   1160 1165 1170
   Lys Asp Phe Ser Asp Ala Lys Glu Ile Glu Arg Leu Asn Ser Pro
   1175 1180 1185
   His His Val Asn Ala Phe Arg Phe Gly Leu Ser Val Gln Ile Asp
   1190 1195 1200
   Phe Phe Asp Asn Met Leu Asp Lys Thr Ser Lys Lys Asn Leu Lys
   1205 1210 1215
   Ala Met Lys Tyr Gly Ala Ser Thr Trp Phe Ser Tyr Ala Met Lys
   1220 1225 1230
   Phe Ala Gly Gln Val Asn Ser Glu Met Gly Asn Pro Asn Leu Gly
   1235 1240 1245
   Thr Ala Leu Tyr Met Gln Ala Pro Tyr Tyr Gly Asp Tyr Ile Arg
   1250 1255 1260
   Lys Trp Met Glu Glu Arg Arg Ala Ala Arg Lys Gln Ala Ile Ile
   1265 1270 1275
   Gly Val Leu Thr Leu Gly Met Met Gly Leu Tyr Ser Leu Leu Ser
   1280 1285 1290
   Val Thr Asp Ile Val Gln His Met Glu Asp Ile Gly Gly Ala Pro
   1295 1300 1305
   Pro Ala Ser Cys Val Thr Asn Glu Ile Leu Gly Val Thr Cys Ala
   1310 1315 1320
   Pro Gln Ala Ile Ala Lys Ala Thr Thr Ser Ala Ala Gln Val Ala
   1325 1330 1335
   Thr Gln Asp Phe Leu Lys Val Gly Leu Phe Ala Gly Leu Ala Pro
   1340 1345 1350
   Tyr Leu Met Leu Pro Met Ala Ile Val Ser Val Trp Asn Ile Leu
   1355 1360 1365
   Lys Ser Glu Ile Lys Ile Leu Leu Gln Phe Glu Met Ala Val Lys
   1370 1375 1380
   His Met Phe Ser Arg Leu Lys Arg Trp Leu Ala Ala Pro Phe Lys
   1385 1390 1395
   Asn Trp Trp Ala Lys Arg Gly Arg Leu Lys Asp Ala Leu Phe Arg
   1400 1405 1410
   Arg Ala Ser Gln Thr Tyr Lys Lys Thr Glu Ala Glu Thr Lys Lys
   1415 1420 1425
   Glu Pro Arg Pro Arg Asn Leu His Asn Pro Ser Ser Trp Gly Gly
   1430 1435 1440
   Ser Glu Leu Asp Ser Leu Gly Val Pro Pro Glu Ser Phe Val Gln
   1445 1450 1455
   Glu Phe Glu Ile Arg Tyr Thr Thr Pro Val Phe Pro Met Ser Ala
   1460 1465 1470
   Pro Leu Ile Lys Thr Ser
   1475

<210>  10
<211>  37
<212>  PRT
<213>  Artificial

<220>
<223>  RON2 peptide (Neospora caninum)

<400>  10

Asp Ile Val Gln His Met Glu Asp Ile Gly Gly Ala Pro Pro Ala Ser
1               5                   10                  15

Cys Val Thr Asn Glu Ile Leu Gly Val Thr Cys Ala Pro Gln Ala Ile
            20                  25                  30

Ala Lys Ala Thr Thr
        35

<210>  11
<211>  1726
<212>  PRT
<213>  Plasmodium bergehii

<220>
<221>  misc_feature
<223>  RON2

<400>  11

Met Gly Ser Asp Asn Tyr Ala Gly His Pro Gln Leu Val Asn Gly Asn
1               5                   10                  15

Asn Met Gly Ser Asp Asn Tyr Ala Arg His Pro Gln Leu Val Asn Gly
            20                  25                  30

Asn Asn Met Gly Pro Asp Asn Ser Ala Glu His Pro Gln Leu Val Asn
        35                  40                  45

Gly Asn Asn Met Gly Ser Asp Asn Ser Ala Gly Tyr Pro Lys Leu Val
    50                  55                  60

Asn Gly Asn Asn Met Gly Ser Asp Asn Tyr Ala Gly His Pro Gln Leu
65                  70                  75                  80

Val Asn Gly Asn Asn Met Gly Ser Asp Asn Tyr Ala Gly His Pro Gln
                85                  90                  95

Leu Val Asn Gly Asn Asn Met Gly Pro Asp Asn Ser Ala Glu His Pro
            100                 105                 110

Gln Leu Val Asn Gly Asn Asn Met Gly Ser Glu Asn Ser Ala Glu Ser
        115                 120                 125

Pro Ile Met Ile Asn Gly Asn Asn Met Gly Ser Asp Asn Ser Ala Gly
    130                 135                 140

Tyr Pro Lys Leu Val Asn Gly Asn Asn Met Gly Ser Asp Asn Ser Ala
145                 150                 155                 160

Gly Tyr Pro Gln Leu Val Asn Gly Asn Asn Met Gly Ser Asp Asn Ser
                165                 170                 175

Ala Glu Ser Pro Ile Met Ile Asn Gly Asn Asn Met Gly Ser Asp Asn
            180                 185                 190

Ser Ala Gly Tyr Pro Gln Leu Val Asn Gly Asn Asn Met Gly Ser Asp
        195                 200                 205

Asn Ser Ala Glu Ser Pro Ile Met Ile Asn Gly Asn Asn Met Gly Ser
    210                 215                 220

Asp Asn Ser Ala Gly Tyr Pro Gln Leu Val Asn Gly Asn Asn Met Gly
225                 230                 235                 240

Ser Asp Asn Ser Ala Glu Ser Pro Arg Met Ile Asn Gly Asn Asn Ser
                245                 250                 255

Val Met Pro Tyr Phe Thr Gly Asn Asp Gln Gln Ile Phe Asn Ser Ile
            260                 265                 270

Ser Ser Leu Lys Pro Val Asn His Glu Glu Leu Tyr Lys Asn Lys Met
        275                 280                 285

Asn Pro Ile Ile Tyr Asn Ala His Glu Thr Lys Ser Gly Asn Lys Asn
    290                 295                 300

Gly Ala Pro Asn Ser Thr Tyr Asp Tyr Ser Ser Asp Tyr Asn Ser Asp
305                 310                 315                 320

Ser Glu Ser Asn Glu Ser Asn Glu Ser Asn Glu Ser Asn Glu Ser Asn
                325                 330                 335

Glu Ser Asn Val Tyr Ile Ile Lys Asn Thr Lys Leu Glu Asn Asp Asn

340                 345                 350

Lys Asp Gly Asn Gly Asn Val Asn Asp Thr Asn Tyr Tyr Lys Asn Gln
        355                 360                 365

Ile Asp Ile Ala Asn Lys Asn Gln Gly Asn Gly Tyr Asn Ser Glu Asn
    370                 375                 380

Ile Tyr Gly Asp Ser Met His Ser Asp Ser Ala Leu Ser Tyr Glu Ser
385                 390                 395                 400

Asp Leu Ser Ser Glu Asn Lys Tyr Ala Lys Glu Gly Ser Asn Asp Glu
                405                 410                 415

Ala Asn Ile Phe Tyr Arg Asp Asn Ala Gln Asp Arg Glu Thr Lys Glu
            420                 425                 430

Lys Leu Met Asn Ser Ser Glu Ser Asp Lys Asp Val Glu Asn Pro Leu
        435                 440                 445

His Val Lys Ile Pro Glu Glu Asn Val Asn Tyr His Phe Ser Asn Tyr
    450                 455                 460

Met Asn Phe Asp Lys Lys Asn Ile Leu Ile Ser Asn Glu Ser Glu Leu
465                 470                 475                 480

Leu Lys Met Ile Gly Pro Asp Phe Ser Lys Glu Val Asn Asn Tyr Cys
                485                 490                 495

Ser Lys Lys Ser Ile Phe Pro Ser Lys Gly Asn Tyr Leu Asp Ile Ser
            500                 505                 510

Phe Glu Tyr Ser Lys Lys Leu Gly Lys Leu Arg Glu Lys Ile Val Asn
        515                 520                 525

Gly Leu Phe Lys Lys Arg Gly Lys Leu Val Thr Lys Glu Asn Asn Ile
    530                 535                 540

Leu Lys Gln Ile Glu Asn Ser Leu Lys Lys Tyr Tyr Val Glu Lys Arg
545                 550                 555                 560

Gln Glu Tyr Leu Asn Ser Ala Ser Glu Ser Ser Glu Leu Lys Asn Asp
                565                 570                 575

Glu Glu Ser Met Leu Ser Asn Glu Tyr Asn Lys Leu Leu Glu Glu Tyr
            580                 585                 590

Ile Cys His Ile Phe Ser Asn Asn Pro Gly Thr Thr Gln Phe Glu Lys
        595                 600                 605

Leu Tyr Tyr His Asn Leu Ala Leu Gly Glu Ile Met Lys Pro Ile Lys
    610                 615                 620

Thr Lys Tyr Lys Asn Val Ala Pro Leu Ser Ile Ala Leu Asn Tyr Glu
625                 630                 635                 640

Ile Tyr Ile Val Ser Ser Ser Asn Ile Tyr Leu Phe Gly His Met Leu
                645                 650                 655

Leu Leu Ser Leu Ala Tyr Leu Ser Tyr Asn Ser Tyr Phe Thr Lys Gly
            660                 665                 670

Thr Lys Ser Phe Tyr Ser Met Glu Thr Met Leu Leu Ala Asn Ser Asp
        675                 680                 685

Tyr Ser Phe Phe Met Tyr Asn Glu Met Cys Asn Val Tyr Tyr Arg Pro
    690                 695                 700

Asn Lys Ser Phe Lys Lys Asp Leu Thr Phe Ile Pro Ile Glu Leu Arg
705                 710                 715                 720

Pro Gly Arg Tyr Thr Thr Tyr Val Gly Glu Arg Lys Ile Ile Cys Asp
                725                 730                 735

Thr Leu Glu Leu Ile Leu Asn Ala Val Ser Leu Ile Asn Ile Asn Glu
            740                 745                 750

Val Tyr Asn Val Phe His Lys Asn Asn Val Tyr Gly Tyr Glu Asn Ser
        755                 760                 765

Val Ser Phe Ser Asn Asn Ala Ile Arg Val Phe Ser Gln Val Cys Pro
    770                 775                 780

Arg Asn Met Glu Lys Asn Ile Ile Asn Cys Ser Phe Glu Lys Ser Thr
785                 790                 795                 800

Leu Tyr Lys Gly Asn Ala Pro Glu Asn Ile Asp Gln Lys Asp Leu Lys
                805                 810                 815

Lys Ala Phe Asp Leu Leu Asn Thr Phe Ser Gly Ile Glu Ser Phe Ser
            820                 825                 830

His Asn Asn Tyr Gln Asn Ser Tyr Tyr Ile Lys Leu Ile Met Glu Gln
        835                 840                 845

Asn Leu Tyr Ala Asp Phe Tyr Lys Tyr Leu Phe Trp Tyr Asp Asn Arg
    850                 855                 860

Glu Leu Ile Lys Thr Arg Glu Leu Ser Gly Lys Lys Lys Thr Ser Lys
865                 870                 875                 880

Tyr Ile Tyr Asp Gln Tyr Ile Lys Thr Asn Lys Leu Leu Glu Lys Lys
                885                 890                 895

Phe Asn Val Leu Ser Lys Tyr Asn Leu Lys Ser Lys Gly Leu Leu Ala
            900                 905                 910

Phe His Ser Leu Ile Asp Lys Tyr Ser Glu Phe Val Lys Asn Lys Lys
        915                 920                 925

Ile Arg Asn Leu Tyr Leu Lys Phe Val Ser Tyr Ala Arg His Phe Leu
    930                 935                 940

Phe Met Asn Asn Thr Met Asn Ser Pro Asn Lys Ser Tyr Leu Asp Phe
945                 950                 955                 960

Met Asn Met Val Leu Glu Glu Leu Gln Asn Glu Thr Lys Ala Pro Leu
                965                 970                 975

Lys Leu Ile Val Arg Gly Asn Tyr Met Met His Leu Asn Asp Ile Ala
            980                 985                 990

Lys Lys Glu Ser Ile Phe Tyr Ile  Asn Leu Phe Ile Leu  Ser Leu Phe
        995                 1000                 1005

Ser Asn  Lys Asn Pro Val Lys  Asn Phe Tyr Asn Gly  Lys Arg Glu
    1010                 1015                 1020

Met Leu  Lys Ala Ser Leu Ser  Glu Lys Phe Ala Thr  Ser Thr Ser
    1025                 1030                 1035

Ala Phe  Ile Pro His Lys Leu  Arg Arg Ile Val Ile  Gly Met Lys
    1040                 1045                 1050

Lys Gly  Phe Leu Lys Lys Lys  Leu Leu Lys Thr Leu  Met Lys Asn
    1055                 1060                 1065

Arg Leu Leu Gln His Ile Pro Ile Asn Leu Leu Glu Asn Ile Met
    1070                1075                1080

Thr Thr Phe Gln Phe Thr Thr His Ala Ile Ala Thr Thr Glu Leu
    1085                1090                1095

Ala Gln Asn Ala His Arg Thr Ser Lys Tyr Leu Asn Ser Lys Asn
    1100                1105                1110

Thr Ser Asn Pro Glu Phe Ala Lys Ala Ile Phe Ser Lys Gly Gly
    1115                1120                1125

Phe Pro Gln Tyr Ala Asp Lys Leu Met Glu Glu Trp Phe Ser Lys
    1130                1135                1140

Gly Phe Glu Glu Tyr Lys Lys Glu Lys Ile Asp Tyr His Asn Met
    1145                1150                1155

Glu Asn Glu Val Asp Lys Glu Leu Asn Lys Leu Glu Thr Leu Ile
    1160                1165                1170

Pro Gly Leu Asn Gly Asn Arg Asn Ser Asn Thr Pro His His Leu
    1175                1180                1185

Ile Glu His Thr Asp Thr Asp Ala His Asn Ser Leu Asp Thr Gln
    1190                1195                1200

Asn Lys Asn Asp Asn Thr Leu Gly Lys Gln Arg Ile Asp Lys Leu
    1205                1210                1215

Ile Tyr Asn Glu His Asp Lys Trp Asp His Tyr Ile Asn Lys Glu
    1220                1225                1230

Tyr Val Lys Ala Leu Ser Ala Trp Ile Asp Ile His Lys Lys Ser
    1235                1240                1245

Asn Asn Val Met Glu Asn Thr Leu Gln Ala Ala Glu Asp Ser Lys
    1250                1255                1260

Tyr Leu Leu Glu Asn Asn Ile Glu Asp Ser Ile Phe Phe Ser Arg
    1265                1270                1275

Thr Phe Lys Pro Thr Lys Gln Ser Ala Phe Arg Asn Val Leu Asn
    1280                1285                1290

Lys Thr Leu Ser Leu Gly Lys Met Leu Leu Arg Lys Pro Ser Phe

1295                 1300                 1305

Lys Val  Asp His Ala Leu Trp  Phe Gly Ala Thr Ile  Asn Met Lys
    1310                 1315                 1320

Lys Gly  Phe Ala Leu Leu Glu  Lys Val Ser Glu Leu  His Lys Leu
    1325                 1330                 1335

Ile Arg  His Glu Asp Glu Ser  Trp Leu Ile Asn Glu  Ala Phe Ile
    1340                 1345                 1350

Glu Ile  Val Asp His Ile Ile  Ser Met Ser Thr Pro  Ser Ser Ile
    1355                 1360                 1365

Ser Phe  Gln Ala Gly Tyr Leu  Arg Asn Pro Gly Met  Phe His Val
    1370                 1375                 1380

Asn Pro  Phe Tyr His Tyr Leu  Pro His Glu Glu Arg  Leu Lys Glu
    1385                 1390                 1395

Leu Gln  Gln Tyr Met Cys Tyr  Asp His Cys Ser Ala  Leu Trp Lys
    1400                 1405                 1410

Met Leu  Ser Thr Phe Ala Leu  His His Leu Lys Asn  Pro Asp Ser
    1415                 1420                 1425

Leu Gln  Thr Tyr Glu Asp Lys  Phe Ser Lys Asn Ser  Leu Gly Asn
    1430                 1435                 1440

Lys Met  Thr Asp Lys Asp Phe  Val Asn Asn Phe Lys  Met Ile Leu
    1445                 1450                 1455

Gly Gly  Asp Ala Ala Leu His  Phe Tyr Asp Asn Leu  Leu Pro Lys
    1460                 1465                 1470

Ser Met  Lys Lys Glu Leu Lys  Ser Met Lys Tyr Gly  Val Ser Leu
    1475                 1480                 1485

Ser Phe  Ser Phe Ser Leu Lys  Leu Ala Lys Met Val  Phe Gly Glu
    1490                 1495                 1500

Met Gln  Leu Pro His Leu Ser  Tyr Met Phe Tyr Ala  Gln Ala Pro
    1505                 1510                 1515

Tyr Phe  Gly His Phe Ile Gly  Lys Trp Gln Lys Glu  Arg Gln Gln
    1520                 1525                 1530

Gly Arg Leu Lys Glu Ile Leu Gly Ala Met Thr Leu Gly Thr Leu
    1535                1540                1545

Ser Thr Tyr Thr Ile Leu Ser Ala Met Asp Ile Thr Gln His Ala
    1550                1555                1560

Thr Asp Ile Gly Met Gly Pro Ser Thr Ser Cys Tyr Thr Ser Leu
    1565                1570                1575

Val Pro Pro Pro Lys Ser Ile Cys Ile Gln Gln Thr Val Lys Ala
    1580                1585                1590

Val Leu Thr Asn Ser Thr Leu Ala Ser Met Lys Ser Val Phe Ser
    1595                1600                1605

Val Gly Leu Phe Ala Ala Ile Thr Pro Tyr Met Phe Ala Pro Met
    1610                1615                1620

Ala Gly Leu Ala Val Trp Ser Val Leu Lys Ser Gln Phe Lys Val
    1625                1630                1635

Val Asn Arg Ile Asp Met Ala Leu Lys Gly Ala Phe Lys Asn Met
    1640                1645                1650

Trp Asn Lys Phe Met Ser Leu Lys Gly Ile Arg Arg Leu Lys Asn
    1655                1660                1665

Val Phe Lys Lys Ile Lys Thr Ile Lys Lys Lys Met Ile Gln Lys
    1670                1675                1680

Thr Glu Lys Asn Leu Ala Glu Ile Gln Gln Asn Pro Glu Ala Glu
    1685                1690                1695

Lys Asn His Lys Ala Ala Val Asn Glu Ile His Asn His Thr Lys
    1700                1705                1710

Gly Asn Tyr His Tyr Val Ser Tyr Ala Lys Ile Val Val
    1715                1720                1725

<210>  12
<211>  39
<212>  PRT
<213>  Artificial

<220>
<223>  RON2 peptide (Plasmodium bergehii)

<400>  12

Asp Ile Thr Gln His Ala Thr Asp Ile Gly Met Gly Pro Ser Thr Ser
1               5                   10                  15

Cys Tyr Thr Ser Leu Val Pro Pro Pro Lys Ser Ile Cys Ile Gln Gln
            20                  25                  30

Thr Val Lys Ala Val Leu Thr
        35

<210>  13
<211>  2143
<212>  PRT
<213>  Plasmodium knowlesii

<220>
<221>  misc_feature
<223>  RON2

<400>  13

Met Leu Lys Ile Ile Phe Leu Ile Leu Ile Ile Phe Ala Arg Ile Asp
1               5                   10                  15

Ser Ile Ser Thr Lys Glu Val Thr Gly Ser Ala Gln Asp Gly Lys Gln
            20                  25                  30

Tyr Val Lys Thr Glu Ser Pro Thr Tyr Thr Pro Lys Lys Lys Thr Lys
        35                  40                  45

Val Ile Phe Tyr Met Pro Gly Gln Glu Gln Glu Glu Glu Glu Asp Asp
    50                  55                  60

Asn Asp Pro Asn Gly Ser Lys Lys Asn Gly Lys Ser Asp Pro Arg Ser
65                  70                  75                  80

Asn Lys Gly Thr His Leu Gly Ser Arg Thr Asp Ala Gly Asn Ser Pro
                85                  90                  95

Gly Gly Phe Asn Lys Gly Ser Ala Val Gly Ser Glu Pro Lys Thr Ala
            100                 105                 110

Ser Asn Asn Tyr Asn Gly Gly Ala Gly Gly Gly Ile Asn Ile Asp Met
        115                 120                 125

Thr His His Gly Asp Asn Gly Asn Lys Gly Val Gln Gly Asn Gly Gly
    130                 135                 140

Phe Asn Lys Asn Gln Gln Asp Arg Leu Lys Asp Glu Tyr Asp Lys Ile
145                 150                 155                 160

Arg Lys Gln Glu Glu Glu Glu Glu Glu Arg Ile Asn Asn Gln Arg Arg
                165                 170                 175

Ala Asp Met Lys Lys Asn Gln Lys Gly Lys Asn Gln Phe Gly His Asp
            180                 185                 190

Lys Gly Val Gln Asp Ser Leu Pro Asn Thr Gln Tyr Glu Leu Ser Asp
        195                 200                 205

Pro His Ser Gly Asn Thr Thr Pro Asn Ala Lys Asn Arg Gly Thr Thr
    210                 215                 220

Gly Ile Tyr Asp Glu Leu Gly Asn Gly Pro Tyr Asn Leu Val Gly Lys
225                 230                 235                 240

Gln Ser Glu Gly Val Thr Pro Thr Ala Pro Pro Tyr Glu His Tyr Asp
                245                 250                 255

Lys His Gly Ala Gly Gly Lys Gly Tyr Gly Pro Tyr Gly Gly Ser Asp
            260                 265                 270

Gly Lys Ser Tyr Gly Pro Tyr Gly Gly Ser Asp Gly Lys Asp Tyr Gly
        275                 280                 285

Leu Tyr Gly Gly Ser Asp Gly Lys Gly Tyr Gly Pro Tyr Gly Gly Ser
    290                 295                 300

Gly Gly Lys Gly Ser Gly Pro Tyr Gly Gly Ser Asp Gly Lys Ala Tyr
305                 310                 315                 320

Gly Pro Tyr Gly Gly Ser Asp Gly Lys Gly Thr Trp Pro Asp Gly Thr
                325                 330                 335

Gly Gly Asn Ala Tyr His Gly Gly Tyr Asp Lys Glu Gly Thr Asn Pro
            340                 345                 350

Thr Tyr Thr Ser His Leu Asn Ile Asn Leu Gly Asp Gly Lys Gly Asp
        355                 360                 365

His Gly Arg Arg Asn Gly Met Glu Gly Tyr Pro Asn Tyr Phe Lys Lys
    370                 375                 380

His Phe Arg Pro Gly Asp Ala Lys Gly Thr Gly Thr Asp Gly Tyr Glu
385                 390                 395                 400

Pro Gly Thr Asn Gly Thr Gln His Gly Gly Ser Gly Val Ala Pro Gly
                405                 410                 415

Gly Gly Asp Tyr Gly Gln Asn Gly Ser Gly Ala Asn Gly Gln Asn Ile
            420                 425                 430

Tyr Gly Pro His Gly Asn Asn Gly Val Gly Pro Ala Ser Tyr Gly Ile
        435                 440                 445

Asp Phe Gly Lys Asn Gly Asn Gly Lys Lys Asn Gly Gly Gly Pro Gly
    450                 455                 460

Glu Asp Gly Ala Asn Thr Thr Asn Pro Ser Tyr Asp Glu Gly Gly Tyr
465                 470                 475                 480

Glu Lys Asn Asn Gly Pro Glu Arg Tyr Asp Gly Asn Gly Thr Pro Tyr
                485                 490                 495

Tyr Val Glu His Pro Asp Asp Asn Lys Gly Arg Gly Thr Thr Thr Tyr
            500                 505                 510

Gly Leu Asp Gln Gly Lys Asn Gly Gly Thr Asn Gly Gly His Gly Asn
        515                 520                 525

Gly Gly Asn Gly Gly Ser Gly Gly Asn Gly Gln Gly Gly Tyr Gly Glu
    530                 535                 540

Ala Gly His Gly Lys Gly Gly Tyr Gly Gln Gly Gly Tyr Gly Gly Ala
545                 550                 555                 560

Gly His Gly Asn Gly Gly Tyr Gly Gln Gly Gly Tyr Gly Gly Ala Gly
                565                 570                 575

His Gly Asn Gly Gly Tyr Gly Pro Asp Gly Asn Gly Gln Asn Tyr Pro
            580                 585                 590

Gly Ser Ala Asn Lys Ala Gly Thr His Ser Met His Asn Asn Glu Lys
        595                 600                 605

Ser Asn Ser Pro Met Leu Asp Tyr Ile His Gly Leu Arg Pro Val Asn
    610                 615                 620

Thr Gly Glu Gly Gln Gly Ile Tyr Gly Gly Asn Gly Ile Asn Ser Pro
625                 630                 635                 640

Leu Val Tyr His Val Gln His Gly Gly Asn Gly Arg Lys Asn Ser Ser
                645                 650                 655

Asp Lys Gly Thr Ser Asp Glu Thr Ser Asp Glu Glu Asn Asp Thr Tyr
            660                 665                 670

Asp Arg Met Arg Asn Lys Arg Arg Val Lys Arg Asn Pro Gly Gly Arg
        675                 680                 685

Asn Arg Ser Ser Asn Ser Ser Gln Asp Gly Asp Met Asp Ser Glu Asp
    690                 695                 700

Thr Glu Tyr Glu Leu Asp Glu Gly Gly Val Lys Arg Leu Lys Pro Lys
705                 710                 715                 720

Asn Lys Gly Ser Ala Ser Ser Glu Glu Ser Asp Gly Tyr Pro Tyr Ala
                725                 730                 735

Glu Gly Thr Asn His Ser Glu Phe Pro Arg Met Asn Gly Arg Gly Val
            740                 745                 750

Gly Asn Tyr Gly Thr His Ser Val Gly Ser Gly Tyr Asn Lys Asn Asn
        755                 760                 765

Asp Glu Asn Gly Phe Thr Pro Ile Thr Val Lys Tyr Asp Asn Thr His
    770                 775                 780

Ala Lys Arg Arg Ala Asn Glu Ile Glu Glu Asn Leu Asn Lys Gly Glu
785                 790                 795                 800

Tyr Ser Arg Thr Lys Met Ala Asn Arg Lys Asn Gly Asp Lys Ser Gly
                805                 810                 815

Gly Ser Glu Ser Asp Ala Glu Glu Ser Asp Val Asp Pro Ser Asn Val
            820                 825                 830

Phe Tyr Val Asp Asn Gly Gln Asp Met Leu Ile Lys Glu Lys Met Ser
        835                 840                 845

Arg Ser Glu Gly Ser Asp Glu Met Ser Glu Glu Gly Leu Asn Val Lys
    850                 855                 860

Tyr Lys Thr Glu Lys Gly Pro Val Asn Tyr His Phe Ser Asn Tyr Met

865                 870                 875                 880

Asn Leu Asp Lys Lys Asn Thr Leu Ser Ser Asn Glu Ile Glu Leu Glu
                885                 890                 895

Lys Met Ile Gly Ser Lys Phe Ser Glu Glu Val Asp Lys Tyr Cys Arg
            900                 905                 910

Leu Asn Glu Pro Leu Gln Lys Lys Gly Asp Phe Leu Asn Leu Ser Phe
        915                 920                 925

Glu Tyr Ser Arg Ala Leu Glu Glu Leu Arg Ser Glu Met Ile Lys Glu
    930                 935                 940

Leu Gln Arg Arg Lys Ala Val Gly Tyr Asn Asn Tyr Asn Asn Ile Leu
945                 950                 955                 960

Lys Ala Ile Tyr Asn Pro Val Asn Arg Lys Asn Thr Asn Leu Gly Arg
                965                 970                 975

Asp Ala Tyr Glu Asp Lys Asn Phe Ile Ser Gln Ala Asn Glu Phe Arg
            980                 985                 990

Asn Glu Gln Ile Gln Pro Leu Ser  Ala Lys Tyr Asn Lys  Ile Leu Arg
        995                 1000                1005

Gln Tyr  Leu Cys His Val Phe  Val Asn Asn Pro Gly  Thr Asn Gln
    1010                1015                1020

Leu Glu  Arg Leu Tyr Tyr His  Asn Leu Ala Leu Gly  Glu Leu Ile
    1025                1030                1035

Gln Pro  Ile Arg Lys Lys Tyr  Ser Lys Gln Ala Ser  Ser Ser Val
    1040                1045                1050

Gly Leu  Asn Tyr Glu Ile Tyr  Ile Ala Ala Ser Ser  Asn Ile Tyr
    1055                1060                1065

Leu Leu  Gly His Leu Leu Met  Leu Ser Leu Ala Tyr  Leu Ser Tyr
    1070                1075                1080

Asn His  Tyr Phe Val Gln Gly  Leu Lys His Phe Tyr  Ser Leu Glu
    1085                1090                1095

Thr Met  Leu Met Ala Asn Ser  Asp Tyr Thr Phe Phe  Met Tyr Asn
    1100                1105                1110

Glu Val Cys Asn Val Tyr Tyr Gln Pro Lys Lys Leu Phe Asp Lys
    1115                1120                1125

Asp Ile Thr Phe Ile Pro Ile Glu Ser Arg Pro Gly Arg His Ser
    1130                1135                1140

Thr Tyr Val Gly Glu Arg Lys Val Thr Cys Asp Leu Leu Glu Leu
    1145                1150                1155

Ile Leu Asn Ala Tyr Thr Leu Ile Asn Ile His Glu Ile Gln Lys
    1160                1165                1170

Val Phe Asn Asn Ser Asp Thr Tyr Gly Tyr Glu Asn Ser Ile Ser
    1175                1180                1185

Phe Ala His Asn Ala Val Arg Ile Phe Ser Gln Val Cys Pro Arg
    1190                1195                1200

Asp Asp Ala Lys Asn Thr Leu Ser Cys Asp Phe Glu Lys Ser Thr
    1205                1210                1215

Leu Tyr Asn Pro Lys Ile Leu Lys Ile Asp Gln Glu Asp Lys Glu
    1220                1225                1230

Asn Gln Arg Arg Leu Lys Arg Ala Phe Asp Met Leu Arg Thr Phe
    1235                1240                1245

Ala Glu Ile Glu Asn Ala Ser Tyr Gln Pro Glu Pro Asn Pro Asn
    1250                1255                1260

Tyr Ile Asn Leu Ile Phe Glu Gln Asn Leu Tyr Thr Asp Phe Tyr
    1265                1270                1275

Lys Tyr Leu Phe Trp Tyr Asp Asn Arg Glu Phe Ile Asn Val Gln
    1280                1285                1290

Ile Arg Asn Ser Gly Gly Lys Lys Lys Gly Arg Lys Thr Lys Tyr
    1295                1300                1305

Val Tyr Asp Asp Phe Val Lys Arg Gly Lys Gln Leu Lys Asp Lys
    1310                1315                1320

Leu Ile Lys Met Asp Ala Lys Tyr Asn Ala Arg Ser Lys Ala Leu
    1325                1330                1335

Leu Val Phe Tyr Ala Leu Val Asp Lys Tyr Ser Asn Ile Phe Arg
    1340                1345                1350

Lys Ser Glu Asn Val Arg Lys Phe Phe Leu Asn Asp Val Ser Ser
    1355                1360                1365

Ile Arg His His Leu Tyr Leu Asn Ser Val Leu Ser Lys Ser Pro
    1370                1375                1380

Lys Ser Asn Leu Asp Ser Met Lys Lys Thr Leu Glu Glu Leu Gln
    1385                1390                1395

Ser Leu Thr Asn Ser Pro Leu Lys Phe Met Val Arg Gly Asn Asn
    1400                1405                1410

Leu Lys Phe Ile Asn Ser Val Ala Arg Phe Glu Asn Leu Phe Tyr
    1415                1420                1425

Val Asn Leu Phe Ile Met Ser Ser Leu Ser Arg Lys Asp Pro Val
    1430                1435                1440

Lys Ser Tyr Tyr Asn Glu Lys Lys Lys Met Leu Thr Ala Thr Met
    1445                1450                1455

Ser Glu Lys Phe Ala Thr Ser Thr Ser Ala Leu Ile Pro His Lys
    1460                1465                1470

Leu Arg Lys Leu Val Val Thr Met Lys Lys Gly Leu Leu Lys Lys
    1475                1480                1485

Arg Leu Leu Thr Ala Leu Ala Lys Met Lys Leu Leu Gln His Ile
    1490                1495                1500

Pro Ala His Leu Leu Glu Asn Ile Thr Thr Thr Ile Arg Phe Thr
    1505                1510                1515

Thr His Ser Ile Ala Ser Arg Glu Ile Ile Gln Asn Ala Lys Phe
    1520                1525                1530

Met Pro Lys Asn Val Asn Phe Tyr Arg Asn Arg Asn Leu Glu Leu
    1535                1540                1545

Ala Lys Gln Ile Phe Thr Asp Gly Gly Phe Ala Lys Tyr Ala Asp
    1550                1555                1560

Asn Leu Met Ser Thr Trp Phe Asn Lys Gly Phe Glu Glu Tyr Lys
    1565                1570                1575

Arg Lys Lys Ile Glu Thr Gln Arg Met Glu Tyr Ser Ile Glu Lys
    1580                1585                1590

Gln Leu Lys Glu Tyr Ser Asn Asn Asp Ile Ser Ala Ser Glu Gly
    1595                1600                1605

Met Thr Glu Gln Glu Arg Leu Glu Gln Glu Glu Asn Asp Leu Asn
    1610                1615                1620

Glu Glu Lys Glu Lys Gln Trp Gln Glu Asn Lys Leu Ile Phe Asn
    1625                1630                1635

Gln Asn Asp Lys Trp Asp His Tyr Ile Asn Lys Glu Phe Ala Lys
    1640                1645                1650

Ala Leu Gly Ile Trp Leu Glu Leu Ser Asp Asn Gly Tyr Asn Arg
    1655                1660                1665

Asp Ser Phe Ile Tyr Lys Val Val Glu Asp Ser Lys Tyr Leu Leu
    1670                1675                1680

Glu Asn Asn Ile Glu Asp Ser Ile Met Phe Ser Arg Thr Val Lys
    1685                1690                1695

Pro Thr Lys Gln Thr Val Phe Arg Lys Phe Phe Lys Lys Ile Thr
    1700                1705                1710

Ser Leu Gly Asn Met Leu Leu Arg Lys Pro Ser Phe Lys Val Glu
    1715                1720                1725

His Ala Ile Trp Phe Gly Ala Thr Ile Asn Met Lys Lys Ala Met
    1730                1735                1740

Met Leu Leu Glu Lys Val Ala Glu Leu His Lys Leu Leu Arg Asn
    1745                1750                1755

Glu Asp Glu Ser Trp Leu Ile Asn Glu Ala Phe Ile Glu Ile Val
    1760                1765                1770

Asp His Val Val Gln Ile Ser Thr Glu Arg Arg Leu Arg Glu Pro
    1775                1780                1785

Phe Ser Val Ala Arg Asn Pro Gly Met Met Ala Ile Ser Pro Ala

1790                1795                1800

Tyr Ala Gln Leu Asn Asn Glu Asp Arg Met Lys Glu Leu Gln Asn
    1805                1810                1815

Ser Met Cys Ala Asp His Cys Ser Ala Leu Trp Lys Thr Ile Ser
    1820                1825                1830

Thr Phe Ala Leu Gln His Leu Lys Asn Pro Glu Ser Leu His Ser
    1835                1840                1845

Tyr Glu Ser Lys Phe Ser Lys Asn Ser Phe Gly Asn Lys Ile Asp
    1850                1855                1860

Asp Gln Asn Phe Val Asn Asp Phe Lys Met Ile Leu Gly Gly Asp
    1865                1870                1875

Ala Val Leu His Tyr Phe Asp Val Leu Leu Pro Lys Ser Met Lys
    1880                1885                1890

Lys Glu Leu Lys Ala Met Lys Asn Gly Val Ser Leu Ser Ser Ala
    1895                1900                1905

Phe Ser Leu Lys Leu Thr Lys Leu Ile Phe Ser Glu Leu Gln Leu
    1910                1915                1920

Pro Tyr Leu Ser Gln Met Phe Tyr Thr Gln Ala Pro Tyr Phe Gly
    1925                1930                1935

His Phe Ile Gly Lys Trp Gln Lys Glu Arg Glu Lys Ser Arg Met
    1940                1945                1950

Lys Glu Ile Leu Gly Phe Met Thr Leu Gly Thr Leu Ser Ala Tyr
    1955                1960                1965

Thr Leu Leu Ser Ala Met Asp Ile Thr Gln His Ala Ser Asp Ile
    1970                1975                1980

Gly Met Gly Pro Val Thr Ser Cys Tyr Thr Ser Thr Ile Pro Pro
    1985                1990                1995

Pro Lys Gln Val Cys Ile Gln Gln Ala Val Lys Val Thr Leu Thr
    2000                2005                2010

Asn Ser Thr Gln Ala Cys Met Lys Ser Val Phe Ser Val Gly Leu
    2015                2020                2025

Phe Ala Ser Ile Gly Pro Tyr Leu Phe Ala Pro Met Ala Gly Leu
    2030                2035                2040

Ala Leu Trp Asn Val Leu Lys Ser Glu Phe Lys Val Ile Gln Arg
    2045                2050                2055

Val Asp Met Ala Leu Lys Ser Val Phe Lys Asn Met Trp Asn Lys
    2060                2065                2070

Phe Leu Ser Ile Lys Gly Ile Arg Lys Leu Lys His Ile Phe Lys
    2075                2080                2085

Arg Lys Lys Thr Met Lys Lys Lys Ile Ile Glu Lys Ala Glu Gln
    2090                2095                2100

Lys Met Leu Glu Met Lys Asn Asn Pro Gln Met Ala Lys Asn His
    2105                2110                2115

Lys Leu Met Val Gln Lys Leu His Lys Asn Val Ser Gly Ser Tyr
    2120                2125                2130

His Tyr Ile Ser Tyr Ala Arg Ile Gln Met
    2135                2140

<210> 14
<211> 39
<212> PRT
<213> Artificial

<220>
<223> RON2 peptide (Plasmodium knowlesii)

<400> 14

Asp Ile Thr Gln His Ala Ser Asp Ile Gly Met Gly Pro Val Thr Ser
1               5                   10                  15

Cys Tyr Thr Ser Thr Ile Pro Pro Pro Lys Gln Val Cys Ile Gln Gln
            20                  25                  30

Ala Val Lys Val Thr Leu Thr
        35

<210> 15
<211> 2203
<212> PRT
<213> Plasmodium vivax

<220>
<221>  misc_feature
<223>  RON2

<400>  15

Met Leu Lys Ile Ile Phe Leu Ile Leu Leu Ile Phe Ala Arg Ile Asp
1               5                   10                  15

Ser Ile Ser Thr Arg Glu Ala Lys Gly Ser Val Arg Asp Gly Lys Gln
            20                  25                  30

Tyr Val Lys Thr Lys Ser Pro Thr Tyr Thr Pro Gln Lys Lys Thr Lys
        35                  40                  45

Val Ile Phe Tyr Met Pro Gly Gln Glu Gln Glu Glu Glu Glu Asp Asp
    50                  55                  60

Asn Asp Pro Asn Gly Ser Lys Lys Asn Gly Lys Ser Asp Thr Gly Ala
65                  70                  75                  80

Asn Lys Gly Thr His Met Gly Ser Lys Thr Asp Ala Gly Asn Ser Pro
                85                  90                  95

Ser Gly Leu Asn Lys Gly Ser Gly Val Gly Ser Gly Ser Arg Pro Ala
            100                 105                 110

Ser Asn Asn Tyr Lys Gly Asn Ala Gly Gly Gly Ile Asn Ile Asp Met
        115                 120                 125

Ser Pro His Gly Asp Asn Ser Asn Lys Gly Gln Gln Gly Asn Ala Gly
    130                 135                 140

Leu Asn Lys Asn Gln Glu Asp Thr Leu Arg Asp Glu Tyr Glu Lys Ile
145                 150                 155                 160

Arg Lys Gln Glu Glu Glu Glu Glu Glu Arg Ile Asn Asn Gln Arg Arg
                165                 170                 175

Ala Asp Met Lys Arg Ala Gln Arg Gly Lys Asn Lys Phe Gly Asp Asp
            180                 185                 190

Lys Gly Val Gln Asp Ser Leu Pro His Thr Lys Tyr Glu Leu Ser Asn
        195                 200                 205

Pro Gln Ala Gly Pro Thr Asn Pro Asn Ala Lys Asn Arg Gly Thr Gly
    210                 215                 220

Val Tyr Asp Glu Phe Gly Asn Gly Pro Tyr Asn Leu Val Gly Lys Gln
225                 230                 235                 240

Ser Gly Gly Leu Thr Pro Thr Ala Pro Pro Tyr Glu His Tyr Gly Glu
                245                 250                 255

His Gly Ala Glu Gly Lys Gly Tyr Gly Pro Tyr Gly Gly Ala Glu Gly
            260                 265                 270

Lys Gly Tyr Gly Pro Tyr Gly Gly Ser Asp Gly Lys Gly Tyr Gly Thr
        275                 280                 285

Tyr Gly Gly Ala Asp Gly Lys Gly Tyr Gly Pro Tyr Gly Gly Ser Asp
    290                 295                 300

Gly Lys Gly Tyr Gly Pro Tyr Gly Gly Ser Asp Gly Lys Gly Tyr Gly
305                 310                 315                 320

Pro Tyr Gly Gly Ser Asp Gly Lys Gly Tyr Gly Pro Tyr Gly Gly Ala
                325                 330                 335

Asp Arg Lys Gly Tyr Gly Pro Asp Gly Thr Ala Gly Ser Ala Tyr His
            340                 345                 350

Gly Gly Tyr Asp Asp Gly Thr Asn Pro Thr Tyr Arg Ser His Leu Asn
        355                 360                 365

Val Asn Leu Arg Asp Gly Lys Gly Asp His Asp Gly Lys Asn Gly Met
    370                 375                 380

Glu Gly Tyr Pro Asn Tyr Phe Lys Lys His Phe Arg Pro Gly Asp Ala
385                 390                 395                 400

Lys Gly Ala Gly Thr Asp Gly Asn Glu Ser Gly Thr Tyr Val Val Ala
                405                 410                 415

Pro Gly Glu Gly Gly Tyr Gly Lys Asn Lys Gly Thr Gly Ala Asp Gly
            420                 425                 430

Gln Asn Gly Thr Glu Ala Gly Gly Gln Asn Gly Ser Gly Thr Asp Ala
        435                 440                 445

Gln Asn Gly Ser Gly Pro His Gly His Ser Gly Val Gly Pro Thr Thr
    450                 455                 460

Tyr Gly Val Asp Phe Gly Lys Ser Gly Gly Gly Arg Gly Asn Gly Gly
465                 470                 475                 480

Gly Pro Gly Glu Glu Gly Glu Asn Lys Thr Asn Pro Ser Tyr Asp Gly
                485                 490                 495

Gly Tyr Glu Lys Asn Ser Gly Pro Glu Lys His Gly Glu Asn Gly Thr
            500                 505                 510

Pro Tyr Tyr Val Glu His Pro Asp Glu Asn Asp Gly Ser Gly Thr Thr
        515                 520                 525

Thr Tyr Gly Leu Asp His Gly Lys Asn Gly Gly Thr Gln Gly Gly His
    530                 535                 540

Gly Gln Asp Gly Leu Gly Glu Gly Gly His Gly Gln Asp Gly His Gly
545                 550                 555                 560

Gln Gly Gly Tyr Gly Gln Gly Gly Tyr Gly Lys Gly Gly His Gly Glu
                565                 570                 575

Gly Gly Tyr Gly Asn Gly Gly His Gly Glu Gly Gly Tyr Gly Asn Gly
            580                 585                 590

Gly His Gly Glu Gly Gly Tyr Gly Asn Gly Gly Pro Gly Glu Gly Gly
        595                 600                 605

Tyr Gly Asn Gly Gly His Gly Glu Gly Gly Tyr Gly Gln Gly Gly His
    610                 615                 620

Gly His Gly Gly His Gly Gln Gly Gly Tyr Gly Gln Ser Gly His Gly
625                 630                 635                 640

Gln Gly Gly Tyr Gly His Gly Gly Tyr Gly Gln Asn His Pro Gly Ser
                645                 650                 655

Ala Asn Gly Ala Gly Thr His Pro Met His Ser Asn Gly Arg Ser Ser
            660                 665                 670

Val Pro Ser Leu Glu Tyr Ile His Gly Leu Arg Pro Val Asn Ala Gly
        675                 680                 685

Asp Gly Gln Gly Val Tyr Gly Gly Asn Gly Ile Asn Asn Pro Leu Val
    690                 695                 700

Tyr His Val Gln His Gly Val Asn Ile Pro Asn Ser Asn Ser Asp Lys
705                 710                 715                 720

Lys Ala Ser Asp His Thr Pro Asp Glu Asp Glu Asp Thr Tyr Gly Arg
                725                 730                 735

Thr Arg Asn Lys Arg Tyr Met His Arg Asn Pro Gly Glu Lys Tyr Lys
            740                 745                 750

Gly Ser Asn Ser Pro His Asp Ser Asn Asp Asp Ser Gly Asp Thr Glu
        755                 760                 765

Tyr Glu Leu Asn Glu Gly Asp Val Lys Arg Leu Thr Pro Lys Asn Lys
    770                 775                 780

Lys Gly Ala Thr Thr Glu Glu Val Asp Thr Tyr Pro Tyr Gly Lys Lys
785                 790                 795                 800

Thr Asn Gly Ser Glu Phe Pro Arg Met Asn Gly Ser Glu Thr Gly His
                805                 810                 815

Tyr Gly Tyr Asn Asn Thr Gly Ser Gly Gly His Asn Asp Glu Asn Gly
            820                 825                 830

Tyr Thr Pro Ile Ile Val Lys Tyr Asp Asn Thr His Ala Lys Asn Arg
        835                 840                 845

Ala Asn Glu Ile Glu Glu Asn Leu Asn Lys Gly Glu Tyr Ser Arg Ile
    850                 855                 860

Lys Met Ala Lys Gly Lys Lys Gly Gln Lys Ser Gly Gly Tyr Glu Ser
865                 870                 875                 880

Asp Gly Glu Asp Ser Asp Val Asp Ser Ser Asn Val Phe Tyr Val Asp
                885                 890                 895

Asn Gly Gln Asp Met Leu Ile Lys Glu Lys Met Ser Arg Ser Glu Gly
            900                 905                 910

Pro Asp Glu Met Ser Glu Glu Gly Leu Asn Val Lys Tyr Lys Ala Gln
        915                 920                 925

Arg Gly Pro Val Asn Tyr His Phe Ser Asn Tyr Met Asn Leu Asp Lys
    930                 935                 940

Arg Asn Thr Leu Ser Ser Asn Glu Ile Glu Leu Gln Lys Met Ile Gly

945                 950                 955                 960

Pro Lys Phe Ser Glu Glu Val Asn Lys Tyr Cys Arg Leu Asn Glu Pro
                965                 970                 975

Ser Ser Lys Lys Gly Glu Phe Leu Asn Val Ser Phe Glu Tyr Ser Arg
            980                 985                 990

Ala Leu Glu Glu Leu Arg Ser Glu  Met Ile Asn Glu Leu  Gln Lys Arg
        995                 1000                1005

Lys Ala  Val Gly Ser Asn Tyr  Tyr Asn Asn Ile Leu  Asn Ala Ile
    1010                 1015                 1020

Tyr Thr  Ser Met Asn Arg Lys  Asn Ala Asn Phe Gly  Arg Asp Ala
    1025                 1030                 1035

Tyr Glu  Asp Lys Ser Phe Ile  Ser Glu Ala Asn Ser  Phe Arg Asn
    1040                 1045                 1050

Glu Glu  Met Gln Pro Leu Ser  Ala Lys Tyr Asn Lys  Ile Leu Arg
    1055                 1060                 1065

Gln Tyr  Leu Cys His Val Phe  Val Gly Asn Pro Gly  Val Asn Gln
    1070                 1075                 1080

Leu Glu  Arg Leu Tyr Phe His  Asn Leu Ala Leu Gly  Glu Leu Ile
    1085                 1090                 1095

Glu Pro  Ile Arg Arg Lys Tyr  Asn Lys Leu Ala Ser  Ser Ser Val
    1100                 1105                 1110

Gly Leu  Asn Tyr Glu Ile Tyr  Ile Ala Ser Ser Ser  Asn Ile Tyr
    1115                 1120                 1125

Leu Met  Gly His Leu Leu Met  Leu Ser Leu Ala Tyr  Leu Ser Tyr
    1130                 1135                 1140

Asn Ser  Tyr Phe Val Gln Gly  Leu Lys Pro Phe Tyr  Ser Leu Glu
    1145                 1150                 1155

Thr Met  Leu Met Ala Asn Ser  Asp Tyr Ser Phe Phe  Met Tyr Asn
    1160                 1165                 1170

Glu Val  Cys Asn Val Tyr Tyr  His Pro Lys Gly Thr  Phe Asn Lys
    1175                 1180                 1185

Asp Ile Thr Phe Ile Pro Ile Glu Ser Arg Pro Gly Arg His Ser
    1190                1195                1200

Thr Tyr Val Gly Glu Arg Lys Val Thr Cys Asp Leu Leu Glu Leu
    1205                1210                1215

Ile Leu Asn Ala Tyr Thr Leu Ile Asn Val His Glu Ile Gln Lys
    1220                1225                1230

Val Phe Asn Thr Ser Glu Ala Tyr Gly Tyr Glu Asn Ser Ile Ser
    1235                1240                1245

Phe Gly His Asn Ala Val Arg Ile Phe Ser Gln Val Cys Pro Arg
    1250                1255                1260

Asp Asp Ala Lys Asn Thr Phe Gly Cys Asp Phe Glu Lys Ser Thr
    1265                1270                1275

Leu Tyr Asn Ser Lys Val Leu Lys Met Asp Glu Gly Asp Lys Glu
    1280                1285                1290

Asn Gln Arg Ser Leu Lys Arg Ala Phe Asp Met Leu Arg Thr Phe
    1295                1300                1305

Ala Glu Ile Glu Ser Thr Ser His Leu Gly Asp Pro Ser Pro Asn
    1310                1315                1320

Tyr Ile Ser Leu Ile Phe Glu Gln Asn Leu Tyr Thr Asp Phe Tyr
    1325                1330                1335

Lys Tyr Leu Phe Trp Tyr Asp Asn Arg Glu Leu Ile Asn Val Gln
    1340                1345                1350

Ile Arg Asn Ala Gly Arg Arg Lys Lys Gly Lys Lys Val Lys Phe
    1355                1360                1365

Val Tyr Asp Glu Phe Val Lys Arg Gly Lys Gln Leu Lys Asp Lys
    1370                1375                1380

Leu Ile Lys Ile Asp Ala Lys Tyr Asn Ala Arg Ser Lys Ala Leu
    1385                1390                1395

Leu Val Phe Tyr Ala Leu Val Asp Lys Tyr Ala Asn Ile Phe Arg
    1400                1405                1410

Lys Ser Glu Asn Val Arg Lys Phe Phe Leu Asn Asp Val Ser Ser
    1415                1420                1425

Ile Arg His His Leu Tyr Leu Asn Ser Val Leu Thr Lys Ser Pro
    1430                1435                1440

Lys Ser Asn Leu Asp Ser Met Lys Lys Thr Leu Glu Glu Leu Gln
    1445                1450                1455

Ser Leu Thr Asn Ala Pro Leu Lys Phe Ile Val Arg Gly Asn Asn
    1460                1465                1470

Leu Lys Phe Leu Asn Asn Val Ala Lys Phe Glu Asn Leu Phe Tyr
    1475                1480                1485

Val Asn Leu Phe Ile Met Ser Ser Leu Ser Arg Lys Asp Pro Val
    1490                1495                1500

Lys Ser Tyr Tyr Ser Glu Lys Lys Lys Met Leu Thr Ala Thr Arg
    1505                1510                1515

Ala Glu Lys Phe Ala Thr Ser Thr Ser Ala Leu Ile Pro His Lys
    1520                1525                1530

Leu Arg Lys Leu Val Val Thr Met Lys Lys Gly Leu Leu Lys Lys
    1535                1540                1545

Lys Leu Leu Thr Ala Leu Ala Lys Met Lys Leu Leu Gln His Ile
    1550                1555                1560

Pro Ala Asn Leu Leu Glu Asn Ile Leu Thr Thr Ile Arg Phe Thr
    1565                1570                1575

Thr His Thr Ile Ala Thr Arg Glu Ile Ile Gln Asn Ala Lys Tyr
    1580                1585                1590

Met Pro Lys Asn Leu Thr Phe Tyr Gln Asn Arg Asn Val Glu Leu
    1595                1600                1605

Ala Lys Gln Val Phe Ala Asp Gly Gly Phe Ala Lys Tyr Ala Asp
    1610                1615                1620

Asn Leu Met Ser Thr Trp Phe Thr Lys Gly Phe Glu Glu Phe Lys
    1625                1630                1635

Arg Glu Lys Ile Glu Lys Gln Lys Met Glu Tyr Ser Ile Glu Lys
    1640                1645                1650

Glu Leu Lys Glu Ala Ser Asn Asn Asp Ile Ser Ala Ser Glu Glu
    1655                1660                1665

Ile Thr Glu Gln Glu Lys Leu Gln Gln Glu Gln Asn Glu Leu Asn
    1670                1675                1680

Glu Glu Lys Glu Arg Gln Arg Gln Glu Asn Lys Leu Ile Phe Asn
    1685                1690                1695

Gln Asn Asp Lys Trp Asp Gln Tyr Ile Asn Lys Glu Phe Ala Lys
    1700                1705                1710

Ala Leu Gly Ile Trp Leu Glu Leu Ser Asp Asn Ala Tyr Asn Lys
    1715                1720                1725

Asp Ser Phe Ile Tyr Arg Val Val Glu Asp Ser Lys Tyr Leu Leu
    1730                1735                1740

Glu Ser Asn Ile Glu Asp Asn Ile Met Phe Ser Arg Thr Val Lys
    1745                1750                1755

Pro Thr Lys Gln Thr Ala Phe Arg Lys Phe Phe Lys Lys Ile Ile
    1760                1765                1770

Ser Leu Gly Asn Met Leu Leu Arg Lys Pro Ser Phe Lys Val Glu
    1775                1780                1785

His Ala Ile Trp Phe Gly Ala Thr Ile Asn Met Lys Lys Ala Met
    1790                1795                1800

Met Leu Leu Glu Lys Val Ala Glu Leu His Lys Leu Leu Arg Asn
    1805                1810                1815

Glu Asp Glu Ser Trp Leu Ile Asn Glu Ala Phe Ile Glu Ile Val
    1820                1825                1830

Asp His Val Val Leu Ile Ser Ser Glu Lys Arg Leu Arg Glu Pro
    1835                1840                1845

Phe Ser Val Ala Arg Asn Pro Gly Met Val Ala Ile Asn Pro Ala
    1850                1855                1860

Tyr Ala Gln Leu Asn Asn Glu Glu Arg Met Lys Glu Leu Gln Asn

1865                1870                1875

Ser Met Cys Ala Asp His Cys Ser Ala Leu Trp Lys Thr Ile Ser
    1880                1885                1890

Thr Phe Ala Leu Gln His Leu Lys Asn Pro Glu Ser Leu His Ser
    1895                1900                1905

Tyr Glu Ser Lys Phe Ser Lys Asn Ser Phe Gly Asn Lys Ile Asp
    1910                1915                1920

Asp Gln Asn Phe Val Asn Asn Phe Lys Met Ile Leu Gly Gly Asp
    1925                1930                1935

Ala Val Leu His Tyr Phe Asp Val Leu Leu Pro Lys Ser Met Lys
    1940                1945                1950

Lys Glu Leu Lys Ala Met Lys Asn Gly Val Ser Leu Ser Ser Ala
    1955                1960                1965

Phe Ser Leu Lys Leu Thr Lys Ile Ile Phe Ser Glu Leu Gln Leu
    1970                1975                1980

Pro Tyr Leu Ser Gln Met Phe Tyr Thr Gln Ala Pro Tyr Phe Gly
    1985                1990                1995

His Phe Ile Gly Lys Trp Gln Lys Glu Arg Glu Lys Ser Arg Met
    2000                2005                2010

Lys Glu Ile Leu Gly Phe Met Thr Leu Gly Thr Leu Ser Ala Tyr
    2015                2020                2025

Thr Ile Leu Ser Ala Met Asp Ile Ser Gln His Ala Thr Asp Ile
    2030                2035                2040

Gly Met Gly Pro Ala Thr Ser Cys Tyr Thr Ser Thr Ile Pro Pro
    2045                2050                2055

Pro Lys Gln Val Cys Ile Gln Gln Ala Val Lys Ala Thr Leu Thr
    2060                2065                2070

Ser Ser Thr Gln Ala Cys Met Lys Ser Val Phe Ser Val Gly Leu
    2075                2080                2085

Phe Ala Ser Ile Gly Pro Tyr Leu Phe Ala Pro Met Ala Gly Leu
    2090                2095                2100

Ala Val Trp Asn Val Leu Lys Ser Glu Phe Lys Val Leu Gln Arg
    2105                2110                2115

Val Asp Met Ala Leu Lys Ser Val Phe Lys Asn Met Trp Arg Lys
    2120                2125                2130

Phe Leu Ser Ile Lys Gly Ile Arg Lys Leu Lys Tyr Ile Phe Lys
    2135                2140                2145

Arg Arg Lys Thr Met Lys Lys Lys Ile Ile Glu Lys Ala Glu His
    2150                2155                2160

Lys Met Leu Glu Met Lys Asn Asn Pro Glu Met Ala Lys Asn His
    2165                2170                2175

Lys Leu Ala Val Gln Lys Ile His Lys His Ala Ser Gly Ser Tyr
    2180                2185                2190

His Tyr Ile Ser Tyr Ala Arg Ile Gln Val
    2195                2200

<210> 16
<211> 39
<212> PRT
<213> Artificial

<220>
<223> RON2 peptide (Plasmodium vivax)

<400> 16

Asp Ile Ser Gln His Ala Thr Asp Ile Gly Met Gly Pro Ala Thr Ser
1               5                   10                  15

Cys Tyr Thr Ser Thr Ile Pro Pro Pro Lys Gln Val Cys Ile Gln Gln
            20                  25                  30

Ala Val Lys Ala Thr Leu Thr
        35

<210> 17
<211> 2232
<212> PRT
<213> Plasmodium Yoelii

<220>
<221> misc_feature
<223> RON2

<220>
<221>  misc_feature
<222>  (1194)..(1194)
<223>  Xaa can be any naturally occurring amino acid

<400>  17

Met Leu Lys Ala Thr Ile Gly Ile Ile Leu Phe Ile Cys Val Asn Ile
1               5                   10                  15

Asp Ile Ile Arg Thr Thr Ser Arg Asp Asn Val Val Asn Thr Lys Phe
            20                  25                  30

Val Arg Gln Lys Ser Pro Thr Tyr Asp Pro Asn Lys Lys Gly Asp Val
        35                  40                  45

Ile Phe Tyr Met Pro Glu His Lys Asp Glu Ile His Arg Ala Asn Asn
    50                  55                  60

Lys Ser Ala Asn Ile Asn Leu Lys Asn Asn Pro Pro Arg Asn Ile Asn
65                  70                  75                  80

Ile Gly Tyr Asn Thr Ala Pro Gly Val Asn His Gly Phe Gly Gln Phe
                85                  90                  95

Ser Gly Lys Ser Ser Asn Met Asn Asn Gly Ile Asn Thr Tyr Lys Asn
            100                 105                 110

Gln Pro Lys His Phe Gln Ser Met Asn Tyr Gln Lys Asn Gly Pro Phe
        115                 120                 125

Gly Asn Lys Leu Glu Gln Met Asn Ile Pro Ala Asn Leu Tyr Asn Asn
    130                 135                 140

Lys Asn Asn Ser Tyr Tyr Ser Gly Ser Asn Asn Gly Asn Asn Asn Ser
145                 150                 155                 160

Tyr Gly Leu His Gly Asn Ile Tyr Asp Lys Ile Asn Ser Ser Val Tyr
                165                 170                 175

Asn Asn Ser Lys Tyr Asn Asn Ser Asn Phe Asn Thr Asp Lys Asn Asn
            180                 185                 190

Asn Asp Asn Glu Asn Asn Lys Thr Tyr Lys Ser Tyr Leu Asn Leu Tyr
        195                 200                 205

Val Ser Asp Asn Lys Ile Ser Pro Ile Gly Asn Asn Gly Arg Pro Gly

210                 215                 220

His Leu Ile Ser His Phe Asn Asn Pro Asn Gln Lys Leu Glu Phe Leu
225                 230                 235                 240

His Gly Phe Asp Gly Leu Phe Asn Gln Asn Ile Pro Gly Met Asn His
                245                 250                 255

Lys Gly Asn Tyr Gly Phe Asn Gly Val Asn Ile Lys Gly Gln Gly Lys
            260                 265                 270

Asn Glu Lys Phe Asp Asn Tyr Gly Gln Asn Leu Gly Leu Asn Lys Thr
        275                 280                 285

Asn Glu Tyr Gln Gln Met Ile Asn Gly Asn Asn Met Gly Ser Asp His
    290                 295                 300

Ile Tyr Glu Ser Pro Asn Phe Val Asn Gly Asn Asn Met Gly Ser Asp
305                 310                 315                 320

Asn Ala Gly Glu Tyr Gln Arg Leu Val Asn Gly Asn Asn Met Gly Ser
                325                 330                 335

Asp His Ile Tyr Glu Ser Pro Asn Phe Val Asn Gly Asn Asn Met Gly
            340                 345                 350

Ser Asp Asn Ala Gly Glu Ser Gln Arg Leu Val Asn Gly Asn Asn Met
        355                 360                 365

Gly Ser Asp Asn Ala Gly Glu Ser Pro Lys Leu Val Asn Gly Asn Asn
    370                 375                 380

Met Gly Ser Asp Asn Ala Gly Glu Ser Pro Lys Leu Val Asn Gly Asn
385                 390                 395                 400

Asn Met Gly Ser Asp Asn Ala Gly Glu Ser Gln Arg Leu Val Asn Gly
                405                 410                 415

Asn Asn Met Gly Ser Asp Asn Ala Gly Glu Ser Pro Lys Leu Val Asn
            420                 425                 430

Gly Asn Asn Met Gly Ser Asp Asn Ala Gly Glu Ser Gln Arg Leu Val
        435                 440                 445

Asn Gly Asn Asn Met Gly Ser Asp Asn Ser Gly Glu Tyr Gln Arg Leu
    450                 455                 460

Val Asn Gly Asn Asn Met Gly Ser Asp Asn Ser Gly Glu Tyr Gln Arg
465                 470                 475                 480

Leu Val Asn Gly Asn Asn Met Gly Ser Asp His Ile Tyr Glu Ser Pro
                485                 490                 495

Asn Phe Val Asn Gly Asn Asn Met Gly Ser Asp Asn Ala Gly Glu Tyr
            500                 505                 510

Gln Arg Leu Ile Asn Gly Asn Asn Met Gly Ser Asp Asn Ala Gly Glu
        515                 520                 525

Tyr Gln Arg Leu Ile Asn Gly Asn Asn Met Gly Ser Asp His Ile Tyr
    530                 535                 540

Glu Ser Pro Asn Phe Val Asn Gly Asn Asn Met Gly Ser Asp Asn Ala
545                 550                 555                 560

Gly Glu Tyr Gln Arg Leu Ile Asn Gly Asn Asn Met Gly Ser Asp Asn
                565                 570                 575

Ala Gly Glu Tyr Gln Arg Leu Ile Asn Gly Asn Asn Met Gly Ser Asp
            580                 585                 590

His Ile Tyr Glu Ser Pro Asn Phe Val Asn Gly Asn Asn Met Gly Ser
        595                 600                 605

Asp Asn Ala Gly Glu Tyr Gln Arg Leu Val Asn Gly Asn Asn Met Gly
    610                 615                 620

Ser Asp Asn Ala Gly Glu Tyr Gln Arg Leu Val Asn Gly Asn Asn Met
625                 630                 635                 640

Gly Ser Asp His Ile Tyr Glu Ser Pro Asn Leu Val Asn Gly Asn Asn
                645                 650                 655

Met Gly Ser Asp Asn Ser Gly Glu Tyr Gln Arg Leu Val Asn Gly Asn
            660                 665                 670

Asn Met Gly Ser Asp Asn Ala Gly Glu Tyr Gln Arg Leu Val Asn Gly
        675                 680                 685

Asn Asn Met Gly Ser Asp His Ile Tyr Glu Ser Pro Asn Leu Val Asn
    690                 695                 700

Gly Asn Asn Met Gly Ser Asp Asn Ser Gly Glu Tyr Gln Arg Leu Val
705                 710                 715                 720

Asn Gly Asn Asn Met Gly Ser Asp Asp Ser Gly Glu Tyr Gln Lys Leu
                725                 730                 735

Val Asn Gly Asn Asn Gly Val Ile Pro Asn Phe Thr Gly Asn Asp Gln
            740                 745                 750

Glu Ile Phe Lys Asn Ile Arg Gly Leu Arg Pro Val Asn His Glu Glu
        755                 760                 765

Leu Tyr Lys Asn Lys Met Asn Pro Leu Ile Tyr Asn Ala His Gly Met
    770                 775                 780

Gln Ser Gly Asn Lys Asn Gly Ala Pro Asn Ser Thr Ser Asp Tyr Val
785                 790                 795                 800

Ser Asp Tyr Asn Ser Asp Ser Asp Thr Asp Ser Asp Ser Asp Ser Asp
                805                 810                 815

Ser Asp Leu Asp Tyr Asp Ser Glu Ser Asn Glu Ser Asn Val Tyr Lys
            820                 825                 830

Ile Lys Thr Asn Lys Leu Glu Asn Asp Asn Lys Asp Gly Asn Gly Asn
        835                 840                 845

Val Asn Asp Ala Asn Tyr Tyr Lys Asn Gln Ile Asp Ile Ala Asn Lys
    850                 855                 860

Asn His Gly Asn Arg Tyr Asn Ser Glu Asn Asp Tyr Gly Asp Ser Met
865                 870                 875                 880

His Ser Asp Ser Ala Leu Ser Tyr Glu Arg Asp Leu Ser Ser Gly Asn
                885                 890                 895

Arg His Val Lys Glu Gly Ser Asn Asp Glu Ile Asn Ile Phe Tyr Arg
            900                 905                 910

Asp Asn Ala Gln Asp Arg Glu Thr Lys Glu Lys Leu Met Asn Ser Ser
        915                 920                 925

Glu Ser Asp Lys Asp Val Glu Asn Pro Leu Asn Ile Thr Ile Pro Glu
    930                 935                 940

Glu Asn Val Asn Tyr His Phe Ser Asn Tyr Met Asn Phe Asp Lys Lys
945                 950                 955                 960

Asn Ile Leu Thr Ser Asn Glu Glu Glu Leu Leu Lys Met Ile Gly Pro
                965                 970                 975

Asp Phe Ser Lys Glu Val Ser Asn Tyr Cys Ser Lys Lys Ser Ile Phe
            980                 985                 990

Pro Ser Asn Gly Lys Tyr Leu Asp  Val Ser Phe Glu Tyr  Ser Lys Glu
        995                 1000                1005

Leu Gly  Lys Leu Arg Glu Lys  Met Met Ser Gly Leu  Phe Lys Lys
    1010                1015                1020

Lys Gly  Lys Leu Val Thr Lys  Glu Asn Asn Ile Leu  Lys Gln Ile
    1025                1030                1035

Glu Asn  Ser Leu Lys Met Asp  Tyr Leu Glu Arg Gln  Gln Gly Tyr
    1040                1045                1050

Val Asn  Tyr Gly Ser Lys Ser  Asn Glu Leu Lys Asn  Asp Glu Glu
    1055                1060                1065

Ser Met  Leu Ser Asn Glu Tyr  Asn Lys Leu Leu Glu  Glu Tyr Ile
    1070                1075                1080

Cys His  Ile Leu Ser Asn Asn  Pro Gly Lys Thr Gln  Phe Glu Lys
    1085                1090                1095

Leu Tyr  Tyr His Asn Leu Ala  Leu Gly Glu Ile Met  Lys Pro Ile
    1100                1105                1110

Lys Thr  Lys Tyr Lys Asn Ala  Ala Thr Leu Ser Ile  Ala Leu Asn
    1115                1120                1125

Tyr Glu  Ile Tyr Ile Val Ser  Ser Ser Asn Ile Tyr  Leu Phe Gly
    1130                1135                1140

His Met  Leu Leu Leu Ser Leu  Ala Tyr Leu Ser Tyr  Asn Ser Tyr
    1145                1150                1155

Phe Thr  Lys Gly Thr Lys Ser  Phe Tyr Ser Met Glu  Thr Met Leu
    1160                1165                1170

Leu Ala  Asn Ser Asp Tyr Ser  Phe Phe Met Tyr Asn  Glu Met Cys

1175                1180                1185

Asn Val Tyr Tyr Arg Xaa Asn Lys Ser Phe Lys Lys Asp Leu Thr
    1190                1195                1200

Phe Ile Pro Ile Glu Leu Arg Pro Gly Arg Tyr Thr Thr Tyr Val
    1205                1210                1215

Gly Glu Arg Lys Ile Ile Cys Asn Thr Leu Glu Leu Ile Leu Asn
    1220                1225                1230

Ala Ile Ser Leu Ile Asn Ile Asn Glu Ile Tyr Asn Val Phe His
    1235                1240                1245

Lys Asn Asn Val Tyr Gly Tyr Glu Asn Ser Val Ser Phe Ser Asn
    1250                1255                1260

Asn Ala Ile Arg Val Phe Ser Gln Val Cys Pro Arg Asn Met Glu
    1265                1270                1275

Lys Asn Ile Ile Asn Cys Ser Phe Glu Lys Ser Thr Leu Tyr Lys
    1280                1285                1290

Ala Asn Ala Pro Glu Asp Thr Asn Gln Asn Glu Tyr Gln Arg Gln
    1295                1300                1305

Asn Gln Ile Lys Asn Gln Asn Glu Leu Lys Lys Ala Phe Asp Leu
    1310                1315                1320

Leu Asn Thr Phe Ser Glu Ile Glu Ser Phe Ser Asn Asn Asn Tyr
    1325                1330                1335

Gln Asn Ser Tyr Tyr Ile Lys Leu Ile Met Glu Gln Asn Leu Tyr
    1340                1345                1350

Thr Asp Phe Tyr Lys Tyr Leu Phe Trp Tyr Asp Asn Arg Glu Leu
    1355                1360                1365

Ile Lys Thr His Glu Ile Asn Gly Lys Lys Asn Thr Lys Lys Thr
    1370                1375                1380

Ser Asn Tyr Ile Tyr Asp Gln Tyr Ile Lys Thr Asn Arg Leu Leu
    1385                1390                1395

Glu Lys Lys Phe Asn Val Leu Ser Lys His Asn Leu Lys Ser Lys
    1400                1405                1410

Gly Leu Leu Ala Phe His Ser Leu Ile Asp Arg Tyr Ser Glu Phe
    1415                1420                1425

Val Lys Asn Lys Lys Ile Arg Asn Leu Tyr Leu Lys Phe Val Ser
    1430                1435                1440

Tyr Ala Arg His Phe Leu Phe Met Asn Asn Thr Met Lys Ser Leu
    1445                1450                1455

Asn Lys Ser Asp Leu Asp Phe Met Lys Met Ile Phe Glu Glu Leu
    1460                1465                1470

Gln Asn Glu Thr Lys Val Pro Leu Lys Leu Ile Val Arg Gly Asn
    1475                1480                1485

Tyr Met Lys Ser Met Asn Asp Ile Ala Lys Lys Glu Asn Leu Phe
    1490                1495                1500

Phe Ile Asn Leu Phe Ile Leu Ser Leu Phe Ser Asn Lys Asn Pro
    1505                1510                1515

Val Lys Asn Phe Tyr Asn Gly Lys Arg Glu Met Leu Lys Ala Ser
    1520                1525                1530

Leu Ser Glu Lys Phe Ala Thr Ser Thr Ser Ala Phe Ile Pro His
    1535                1540                1545

Lys Leu Arg Arg Ile Val Val Gly Met Lys Lys Gly Phe Leu Lys
    1550                1555                1560

Arg Lys Leu Leu Lys Thr Leu Met Lys Asn Arg Leu Leu Gln His
    1565                1570                1575

Ile Pro Ile Asp Leu Leu Glu Asn Ile Met Thr Thr Phe Arg Phe
    1580                1585                1590

Thr Thr His Ala Ile Ala Thr Ser Glu Leu Ala Gln Asn Ala His
    1595                1600                1605

Arg Thr Ser Lys Tyr Leu Asn Ser Asn Asn Thr Ser Lys Leu Glu
    1610                1615                1620

Phe Ala Lys Thr Ile Phe Ser Lys Gly Gly Phe Pro Gln Tyr Ala
    1625                1630                1635

Asp Lys Leu Met Glu Lys Trp Phe Ser Lys Gly Phe Glu Glu Tyr
    1640                1645                1650

Lys Lys Glu Lys Ile Asp Asn Gln Asn Met Glu Asn Glu Val Asp
    1655                1660                1665

Lys Glu Leu Asp Gln Ile Lys Glu Met Phe Ile Pro Gly Ser Asn
    1670                1675                1680

Gly Lys His Asn Ser Asn Thr Pro Pro His Leu Ile Glu Asn Ile
    1685                1690                1695

Asn Thr Asp Val Asn Asn Ser Leu Asp Asn Gln Asp Lys Tyr Asp
    1700                1705                1710

Asn Thr Leu Gly Lys Gln Arg Val Asp Lys Leu Ile Tyr Asn Glu
    1715                1720                1725

His Asp Lys Trp Asp His Tyr Ile Asn Lys Glu Tyr Val Lys Ala
    1730                1735                1740

Leu Gly Ala Trp Ile Glu Ile His Lys Lys Ser Asn Asn Val Met
    1745                1750                1755

Glu Asn Ile Leu Gln Ala Val Glu Asp Ser Lys Tyr Leu Leu Glu
    1760                1765                1770

Asn Asn Ile Glu Asp Ser Ile Phe Phe Ser Arg Thr Phe Lys Ala
    1775                1780                1785

Thr Lys Gln Ser Ala Phe Arg Asn Val Leu Asn Lys Thr Leu Ser
    1790                1795                1800

Leu Gly Lys Met Leu Leu Arg Lys Pro Ser Phe Lys Val Asp His
    1805                1810                1815

Ala Leu Trp Phe Gly Ala Thr Ile Asn Met Lys Lys Gly Phe Ala
    1820                1825                1830

Leu Leu Glu Lys Val Ser Glu Leu His Lys Leu Ile Arg His Glu
    1835                1840                1845

Asp Glu Ser Trp Leu Ile Asn Glu Ala Phe Ile Glu Ile Val Asp
    1850                1855                1860

His Ile Ile Ala Ile Ser Thr Pro Ser Ser Ile Ser Ser Arg Ala
    1865                1870                1875

Gly Tyr Leu Ser Asn Pro Gly Met Phe His Ile Asn Pro Phe Tyr
    1880                1885                1890

His Arg Leu Ser Asn Glu Glu Arg Leu Lys Glu Leu Gln Gln Tyr
    1895                1900                1905

Met Cys Tyr Asp His Cys Ser Ser Leu Trp Lys Met Leu Ser Thr
    1910                1915                1920

Phe Ala Leu His His Leu Lys Asn Pro Asp Ser Leu Gln Thr Tyr
    1925                1930                1935

Glu Asp Lys Phe Ser Lys Asn Ser Leu Gly Asn Lys Met Thr Asp
    1940                1945                1950

Lys Asp Phe Val Asn Asn Phe Lys Met Ile Leu Gly Gly Asp Ala
    1955                1960                1965

Ala Leu His Phe Tyr Asp Asn Leu Leu Pro Lys Ser Met Lys Lys
    1970                1975                1980

Glu Leu Lys Ser Met Lys Tyr Gly Val Ser Leu Ser Phe Ser Phe
    1985                1990                1995

Ser Leu Lys Leu Ala Lys Met Val Phe Gly Glu Met Gln Leu Pro
    2000                2005                2010

His Leu Ser His Met Phe Tyr Ala Gln Ala Pro Tyr Phe Gly His
    2015                2020                2025

Phe Ile Gly Lys Trp Gln Lys Glu Arg Gln Gln Gly Arg Leu Lys
    2030                2035                2040

Glu Ile Leu Gly Ala Met Thr Leu Gly Thr Leu Ser Thr Tyr Thr
    2045                2050                2055

Val Leu Ser Ala Met Asp Ile Thr Gln His Ala Thr Asp Ile Gly
    2060                2065                2070

Met Gly Pro Ser Thr Ser Cys Tyr Thr Ser Leu Leu Pro Pro Pro
    2075                2080                2085

Lys Ser Ile Cys Ile Gln Gln Thr Val Lys Thr Val Leu Thr Asn

2090                2095                2100

Ser Thr Leu Ala Ser Met Lys Ser Val Phe Ser Val Gly Leu Phe
    2105                2110                2115

Ala Ala Ile Thr Pro Tyr Met Phe Ala Pro Met Ala Gly Leu Ala
    2120                2125                2130

Val Trp Ser Val Leu Lys Ser Gln Phe Lys Val Val Asn Arg Ile
    2135                2140                2145

Asp Met Ala Leu Lys Gly Ala Leu Lys Asn Met Trp Asn Lys Phe
    2150                2155                2160

Met Ser Leu Lys Gly Ile Arg Arg Leu Lys Asn Val Phe Lys Lys
    2165                2170                2175

Ile Lys Thr Ile Lys Lys Lys Met Ile Gln Lys Thr Glu Lys Asn
    2180                2185                2190

Leu Ala Glu Ile Gln Gln Asn Pro Glu Ala Glu Gln Asn His Lys
    2195                2200                2205

Ala Ala Val Asn Glu Ile His Asn Asn Thr Arg Gly Asn Tyr His
    2210                2215                2220

Tyr Ile Ser Tyr Ala Lys Ile Val Val
    2225                2230

<210>  18
<211>  39
<212>  PRT
<213>  Artificial

<220>
<223>  RON2 peptide (Plasmodium Yoelii)

<400>  18

Asp Ile Thr Gln His Ala Thr Asp Ile Gly Met Gly Pro Ser Thr Ser
1               5                   10                  15

Cys Tyr Thr Ser Leu Leu Pro Pro Pro Lys Ser Ile Cys Ile Gln Gln
            20                  25                  30

Thr Val Lys Thr Val Leu Thr
        35

<210>  19
<211>  788
<212>  PRT
<213>  Theileria parva

<220>
<221>  misc_feature
<223>  RON2

<400>  19

Met Thr Leu Met Trp Asp His Arg Met Phe Thr Gly Lys Lys Lys Gly
1               5                   10                  15

Thr Trp Phe Ala Asp Tyr Asn Lys Ile Glu Lys Lys Arg Ile Gly Arg
            20                  25                  30

Asn Leu Ser Ala Glu Ile Val Pro Pro Tyr Asn Ala Val Gln Tyr Tyr
        35                  40                  45

Asn Lys Leu Pro Tyr Glu Leu Gly Ile Glu Lys Lys Lys Lys Lys Lys
    50                  55                  60

Arg Ile Leu Arg Tyr Arg Ala Ser His Arg Phe Tyr Ile Tyr Met Ala
65                  70                  75                  80

Ser Ser Gly Ile Lys Lys Trp Ile Glu Asp Asn Met Thr Leu Met His
                85                  90                  95

Glu Thr Tyr Asn Tyr Ser Pro Ser Val Leu Leu Gly Gly Ser Ile Ser
            100                 105                 110

Asn Arg Phe Arg Glu Phe Tyr Asp Ser Ser Ser Val Gly Leu Ala His
        115                 120                 125

Leu Phe Leu Tyr Asn Ile Leu Ser Lys Lys Tyr Pro Ala Gln Asp Tyr
    130                 135                 140

Leu Lys Glu Leu Glu Lys Leu Gly Lys Ala Asp Pro Phe Ser Arg Phe
145                 150                 155                 160

Val Asp Ala Ser Ser Ile Phe Ala Pro Glu Lys Leu Arg Lys Val Val
                165                 170                 175

Lys Trp Phe Leu Lys Gly Ser Phe Val Lys Gln Phe Met Arg Glu Lys
            180                 185                 190

Thr Lys Leu Thr Leu Lys Gln Leu Val Arg Ser Glu Val Leu Arg Asp

195                 200                 205

Ala Leu Glu Ser Ile Thr Phe Val Thr His Ser Met Ala Asn Leu Gln
    210                 215                 220

Val Ile Gln Asn Ala Glu Tyr Trp Gly Arg Ala Tyr Pro Asn Ser Ser
225                 230                 235                 240

Phe Tyr Phe Arg Arg Gly Gly Val Ile Ser Val Ile Asp Asn Thr Ile
                245                 250                 255

Lys Asn Trp Ser Asp Asp Gly Tyr Thr Arg Ser Ile Ser Glu Lys Leu
            260                 265                 270

Lys Asn His Val Asp Leu Asn Glu Asp Asp Leu Lys Ile Ala Asp Phe
        275                 280                 285

Gln His Ile His Asn Met Glu Ser Val Lys Trp Asp Lys Ser Leu Asn
    290                 295                 300

Thr Glu Ile Leu Lys Ser Phe Asn Glu Phe Leu Glu Leu Gly Ser Val
305                 310                 315                 320

Lys Ala Leu Glu Gly Arg Asn His Ile Leu Tyr Glu Val Val Lys Asp
                325                 330                 335

Ser Arg Asp Asn Leu Glu Gln Asn Ile Glu Asn Thr Ile Phe Phe Gly
            340                 345                 350

Arg Ile Leu Asn Ser Pro Lys Ile Glu Ser Asn Leu Arg Lys Tyr Leu
        355                 360                 365

Arg Arg Ala Val Asn Phe Gly Asn Phe Ile Leu Asp Arg Ala Glu Lys
    370                 375                 380

Asn Val Asp His Ala Ile Trp Phe Gly Ile Lys Leu Asn Leu Asp Lys
385                 390                 395                 400

Val Met Arg Val Val Asp Glu Leu His Lys Leu Gln Arg Lys Leu Ser
                405                 410                 415

Asn Asn Glu Ser Trp Asn Leu Thr Gly Ala Phe Ile Asp Val Ile Glu
            420                 425                 430

Asp Leu Val Glu Val Leu Thr Asn Arg Thr Ser Arg Asn Pro Ile Asp
        435                 440                 445

Val Pro Leu Asn Tyr Gly Met Pro Thr Ile Ser Asn Leu Tyr Gly Arg
    450                 455                 460

Met Ser Val Asp Glu Arg Arg Ile Glu Phe Gln Gln Ser Met Cys Ser
465                 470                 475                 480

Glu His Cys Gly Ala Ile Trp Lys Ala Ile Leu Ala Phe Thr Ile Ser
                485                 490                 495

Thr Leu Arg Asn Pro Gly Ser Ile Lys Ser Tyr Glu Lys Asn Leu Ser
            500                 505                 510

Ser Thr Thr Ser Leu Ser Glu Leu Asn Ser Pro Asn Tyr Val Asn Asn
        515                 520                 525

Val Arg Phe Ile Leu Lys Gly Asp Ala Trp Thr Gly Phe Tyr Asp Thr
    530                 535                 540

Leu Leu Pro Lys Ser Met Lys Lys Glu Leu Glu Val Met Glu Phe Gly
545                 550                 555                 560

Lys Ser Phe Tyr Ile Ala Asn Ile Leu Lys Leu Ala Ser Val Leu Met
                565                 570                 575

Asn Arg Met Gly Tyr Val Tyr Thr Ala Thr Thr Met Lys Val Gln Ala
            580                 585                 590

Pro Tyr Phe Gly Asn Phe Thr Thr Glu Trp Met Lys Glu Arg Lys Lys
        595                 600                 605

Asn Arg Thr Lys Ile Leu Phe Ser Ala Leu Ala Leu Gly Thr Met Ala
    610                 615                 620

Thr Tyr Thr Val Leu Glu Cys Met Asp Ile Ala Gln His Ala Val Asp
625                 630                 635                 640

Met Gly His Pro Pro Val Glu Thr Cys Trp Tyr Leu Val Lys Pro Pro
                645                 650                 655

Ser Met His Cys Ala Ile Glu Pro Ile Ser Asn Leu Ala Ile Ser Ala
            660                 665                 670

Ser Ser Val Ala Ile Arg Asp Val Phe Ser Ser Thr Ile Leu Ala Leu
        675                 680                 685

Thr Gly Pro Tyr Met Met Ile Pro Met Gly Ile Tyr Ala Gly Trp Thr
    690                 695                 700

Leu Leu Lys Arg Gln Phe Lys Ile Leu His Arg Leu Asp Met Ala Ile
705                 710                 715                 720

Ser Ser Val Phe Ser Arg Leu Trp Arg Arg Val Asn Thr Lys Asp Met
                725                 730                 735

Ile Arg Ser Ile Ala Asn Leu Phe Ser Asn Arg Lys Gln Tyr Arg Lys
            740                 745                 750

Glu Ile Glu Ala Ala Ala Leu Glu Ser Lys Gln Thr Gly Asn Glu Glu
        755                 760                 765

Val Lys Thr Gln Thr Gly Phe Ala Asp Asp Gly Asn Thr Phe Ser Tyr
    770                 775                 780

Thr His Met Asp
785

<210>  20
<211>  38
<212>  PRT
<213>  Artificial

<220>
<223>  RON2 peptide (Theileria parva)

<400>  20

Asp Ile Ala Gln His Ala Val Asp Met Gly His Pro Pro Val Glu Thr
1               5                   10                  15

Cys Trp Tyr Leu Val Lys Pro Pro Ser Met His Cys Ala Ile Glu Pro
            20                  25                  30

Ile Ser Asn Leu Ala Ile
        35

<210>  21
<211>  347
<212>  PRT
<213>  artificial

<220>
<223>  AMA1 peptide with cleavage sites

<400>  21

Gly Ser Ala Met Gly Asn Tyr Met Gly Asn Pro Trp Thr Glu Tyr Met
1               5                   10                  15

Ala Lys Tyr Asp Ile Glu Glu Val His Gly Ser Gly Ile Arg Val Asp
            20                  25                  30

Leu Gly Glu Asp Ala Glu Val Ala Gly Thr Gln Tyr Arg Leu Pro Ser
        35                  40                  45

Gly Lys Cys Pro Val Phe Gly Lys Gly Ile Ile Ile Glu Asn Ser Asn
    50                  55                  60

Thr Thr Phe Leu Thr Pro Val Ala Thr Gly Asn Gln Tyr Leu Lys Asp
65                  70                  75                  80

Gly Gly Phe Ala Phe Pro Pro Thr Glu Pro Leu Met Ser Pro Met Thr
                85                  90                  95

Leu Asp Glu Met Arg His Phe Tyr Lys Asp Asn Lys Tyr Val Lys Asn
            100                 105                 110

Leu Asp Glu Leu Thr Leu Cys Ser Arg His Ala Gly Asn Met Ile Pro
        115                 120                 125

Asp Asn Asp Lys Asn Ser Asn Tyr Lys Tyr Pro Ala Val Tyr Asp Asp
    130                 135                 140

Lys Asp Lys Lys Cys His Ile Leu Tyr Ile Ala Ala Gln Glu Asn Asn
145                 150                 155                 160

Gly Pro Arg Tyr Cys Asn Lys Asp Glu Ser Lys Arg Asn Ser Met Phe
                165                 170                 175

Cys Phe Arg Pro Ala Lys Asp Ile Ser Phe Gln Asn Tyr Thr Tyr Leu
            180                 185                 190

Ser Lys Asn Val Val Asp Asn Trp Glu Lys Val Cys Pro Arg Lys Asn
        195                 200                 205

Leu Gln Asn Ala Lys Phe Gly Leu Trp Val Asp Gly Asn Cys Glu Asp
    210                 215                 220

Ile Pro His Val Asn Glu Phe Pro Ala Ile Asp Leu Phe Glu Cys Asn
225                 230                 235                 240

Lys Leu Val Phe Glu Leu Ser Ala Ser Asp Gln Pro Lys Gln Tyr Glu

245                 250                 255

Gln His Leu Thr Asp Tyr Glu Lys Ile Lys Glu Gly Phe Lys Asn Lys
            260                 265                 270

Asn Ala Ser Met Ile Lys Ser Ala Phe Leu Pro Thr Gly Ala Phe Lys
        275                 280                 285

Ala Asp Arg Tyr Lys Ser His Gly Lys Gly Tyr Asn Trp Gly Asn Tyr
    290                 295                 300

Asn Thr Glu Thr Gln Lys Cys Glu Ile Phe Asn Val Lys Pro Thr Cys
305                 310                 315                 320

Leu Ile Asn Asn Ser Ser Tyr Ile Ala Thr Thr Ala Leu Ser His Pro
                325                 330                 335

Ile Glu Val Glu Ala Ala Ala Leu Val Pro Arg
            340                 345

<210>  22
<211>  335
<212>  PRT
<213>  artificial

<220>
<223>  AMA1 peptide

<400>  22

Asn Tyr Met Gly Asn Pro Trp Thr Glu Tyr Met Ala Lys Tyr Asp Ile
1               5                   10                  15

Glu Glu Val His Gly Ser Gly Ile Arg Val Asp Leu Gly Glu Asp Ala
            20                  25                  30

Glu Val Ala Gly Thr Gln Tyr Arg Leu Pro Ser Gly Lys Cys Pro Val
        35                  40                  45

Phe Gly Lys Gly Ile Ile Ile Glu Asn Ser Asn Thr Thr Phe Leu Thr
    50                  55                  60

Pro Val Ala Thr Gly Asn Gln Tyr Leu Lys Asp Gly Gly Phe Ala Phe
65                  70                  75                  80

Pro Pro Thr Glu Pro Leu Met Ser Pro Met Thr Leu Asp Glu Met Arg
                85                  90                  95

His Phe Tyr Lys Asp Asn Lys Tyr Val Lys Asn Leu Asp Glu Leu Thr
            100                 105                 110

Leu Cys Ser Arg His Ala Gly Asn Met Ile Pro Asp Asn Asp Lys Asn
        115                 120                 125

Ser Asn Tyr Lys Tyr Pro Ala Val Tyr Asp Asp Lys Asp Lys Lys Cys
    130                 135                 140

His Ile Leu Tyr Ile Ala Ala Gln Glu Asn Asn Gly Pro Arg Tyr Cys
145                 150                 155                 160

Asn Lys Asp Glu Ser Lys Arg Asn Ser Met Phe Cys Phe Arg Pro Ala
                165                 170                 175

Lys Asp Ile Ser Phe Gln Asn Tyr Thr Tyr Leu Ser Lys Asn Val Val
            180                 185                 190

Asp Asn Trp Glu Lys Val Cys Pro Arg Lys Asn Leu Gln Asn Ala Lys
        195                 200                 205

Phe Gly Leu Trp Val Asp Gly Asn Cys Glu Asp Ile Pro His Val Asn
    210                 215                 220

Glu Phe Pro Ala Ile Asp Leu Phe Glu Cys Asn Lys Leu Val Phe Glu
225                 230                 235                 240

Leu Ser Ala Ser Asp Gln Pro Lys Gln Tyr Glu Gln His Leu Thr Asp
                245                 250                 255

Tyr Glu Lys Ile Lys Glu Gly Phe Lys Asn Lys Asn Ala Ser Met Ile
            260                 265                 270

Lys Ser Ala Phe Leu Pro Thr Gly Ala Phe Lys Ala Asp Arg Tyr Lys
        275                 280                 285

Ser His Gly Lys Gly Tyr Asn Trp Gly Asn Tyr Asn Thr Glu Thr Gln
    290                 295                 300

Lys Cys Glu Ile Phe Asn Val Lys Pro Thr Cys Leu Ile Asn Asn Ser
305                 310                 315                 320

Ser Tyr Ile Ala Thr Thr Ala Leu Ser His Pro Ile Glu Val Glu
                325                 330                 335

<210>  23

<211>  541
<212>  PRT
<213>  Toxoplasma gondii

<220>
<221>  misc_feature
<223>  AMA1

<400>  23

Met Gly Leu Val Gly Val Gln Val Leu Leu Val Leu Val Ala Asp Cys
1               5                   10                  15

Thr Ile Phe Ala Ser Gly Leu Ser Ser Ser Thr Arg Ser Arg Glu Ser
            20                  25                  30

Gln Thr Leu Ser Ala Ser Thr Ser Gly Asn Pro Phe Gln Ala Asn Val
        35                  40                  45

Glu Met Lys Thr Phe Met Glu Arg Phe Asn Leu Thr His His His Gln
    50                  55                  60

Ser Gly Ile Tyr Val Asp Leu Gly Gln Asp Lys Glu Val Asp Gly Thr
65                  70                  75                  80

Leu Tyr Arg Glu Pro Ala Gly Leu Cys Pro Ile Trp Gly Lys His Ile
                85                  90                  95

Glu Leu Gln Gln Pro Asp Arg Leu Pro Tyr Arg Asn Asn Phe Leu Glu
            100                 105                 110

Asp Val Pro Thr Glu Lys Glu Tyr Lys Gln Ser Gly Asn Pro Leu Pro
        115                 120                 125

Gly Gly Phe Asn Leu Asn Phe Val Thr Pro Ser Gly Gln Arg Ile Ser
    130                 135                 140

Pro Phe Pro Met Glu Leu Leu Glu Lys Asn Ser Asn Ile Lys Ala Ser
145                 150                 155                 160

Thr Asp Leu Gly Arg Cys Ala Glu Phe Ala Phe Lys Thr Val Ala Met
                165                 170                 175

Asp Lys Asn Asn Lys Ala Thr Lys Tyr Arg Tyr Pro Phe Val Tyr Asp
            180                 185                 190

Ser Lys Lys Arg Leu Cys His Ile Leu Tyr Val Ser Met Gln Leu Met
        195                 200                 205

Glu Gly Lys Lys Tyr Cys Ser Val Lys Gly Glu Pro Pro Asp Leu Thr
    210                 215                 220

Trp Tyr Cys Phe Lys Pro Arg Lys Ser Val Thr Glu Asn His His Leu
225                 230                 235                 240

Ile Tyr Gly Ser Ala Tyr Val Gly Glu Asn Pro Asp Ala Phe Ile Ser
                245                 250                 255

Lys Cys Pro Asn Gln Ala Leu Arg Gly Tyr Arg Phe Gly Val Trp Lys
            260                 265                 270

Lys Gly Arg Cys Leu Asp Tyr Thr Glu Leu Thr Asp Thr Val Ile Glu
        275                 280                 285

Arg Val Glu Ser Lys Ala Gln Cys Trp Val Lys Thr Phe Glu Asn Asp
    290                 295                 300

Gly Val Ala Ser Asp Gln Pro His Thr Tyr Pro Leu Thr Ser Gln Ala
305                 310                 315                 320

Ser Trp Asn Asp Trp Trp Pro Leu His Gln Ser Asp Gln Pro His Ser
                325                 330                 335

Gly Gly Val Gly Arg Asn Tyr Gly Phe Tyr Tyr Val Asp Thr Thr Gly
            340                 345                 350

Glu Gly Lys Cys Ala Leu Ser Asp Gln Val Pro Asp Cys Leu Val Ser
        355                 360                 365

Asp Ser Ala Ala Val Ser Tyr Thr Ala Ala Gly Ser Leu Ser Glu Glu
    370                 375                 380

Thr Pro Asn Phe Ile Ile Pro Ser Asn Pro Ser Val Thr Pro Pro Thr
385                 390                 395                 400

Pro Glu Thr Ala Leu Gln Cys Thr Ala Asp Lys Phe Pro Asp Ser Phe
                405                 410                 415

Gly Ala Cys Asp Val Gln Ala Cys Lys Arg Gln Lys Thr Ser Cys Val
            420                 425                 430

Gly Gly Gln Ile Gln Ser Thr Ser Val Asp Cys Thr Ala Asp Glu Gln
        435                 440                 445

Asn Glu Cys Gly Ser Asn Thr Ala Leu Ile Ala Gly Leu Ala Val Gly
450                 455                 460

Gly Val Leu Leu Leu Ala Leu Leu Gly Gly Gly Cys Tyr Phe Ala Lys
465                 470                 475                 480

Arg Leu Asp Arg Asn Lys Gly Val Gln Ala Ala His His Glu His Glu
                485                 490                 495

Phe Gln Ser Asp Arg Gly Ala Arg Lys Lys Arg Pro Ser Asp Leu Met
            500                 505                 510

Gln Glu Ala Glu Pro Ser Phe Trp Asp Glu Ala Glu Glu Asn Ile Glu
        515                 520                 525

Gln Asp Gly Glu Thr His Val Met Val Glu Gly Asp Tyr
    530                 535                 540

<210>  24
<211>  622
<212>  PRT
<213>  Plasmodium falciparum

<220>
<221>  misc_feature
<223>  AMA1

<400>  24

Met Arg Lys Leu Tyr Cys Val Leu Leu Leu Ser Ala Phe Glu Phe Thr
1               5                   10                  15

Tyr Met Ile Asn Phe Gly Arg Gly Gln Asn Tyr Trp Glu His Pro Tyr
            20                  25                  30

Gln Asn Ser Asp Val Tyr Arg Pro Ile Asn Glu His Arg Glu His Pro
        35                  40                  45

Lys Glu Tyr Glu Tyr Pro Leu His Gln Glu His Thr Tyr Gln Gln Glu
    50                  55                  60

Asp Ser Gly Glu Asp Glu Asn Thr Leu Gln His Ala Tyr Pro Ile Asp
65                  70                  75                  80

His Glu Gly Ala Glu Pro Ala Pro Gln Glu Gln Asn Leu Phe Ser Ser
                85                  90                  95

Ile Glu Ile Val Glu Arg Ser Asn Tyr Met Gly Asn Pro Trp Thr Glu
            100                 105                 110

Tyr Met Ala Lys Tyr Asp Ile Glu Glu Val His Gly Ser Gly Ile Arg
        115                 120                 125

Val Asp Leu Gly Glu Asp Ala Glu Val Ala Gly Thr Gln Tyr Arg Leu
    130                 135                 140

Pro Ser Gly Lys Cys Pro Val Phe Gly Lys Gly Ile Ile Ile Glu Asn
145                 150                 155                 160

Ser Asn Thr Thr Phe Leu Thr Pro Val Ala Thr Gly Asn Gln Tyr Leu
                165                 170                 175

Lys Asp Gly Gly Phe Ala Phe Pro Pro Thr Glu Pro Leu Met Ser Pro
            180                 185                 190

Met Thr Leu Asp Glu Met Arg His Phe Tyr Lys Asp Asn Lys Tyr Val
        195                 200                 205

Lys Asn Leu Asp Glu Leu Thr Leu Cys Ser Arg His Ala Gly Asn Met
    210                 215                 220

Ile Pro Asp Asn Asp Lys Asn Ser Asn Tyr Lys Tyr Pro Ala Val Tyr
225                 230                 235                 240

Asp Asp Lys Asp Lys Lys Cys His Ile Leu Tyr Ile Ala Ala Gln Glu
                245                 250                 255

Asn Asn Gly Pro Arg Tyr Cys Asn Lys Asp Glu Ser Lys Arg Asn Ser
            260                 265                 270

Met Phe Cys Phe Arg Pro Ala Lys Asp Ile Ser Phe Gln Asn Tyr Thr
        275                 280                 285

Tyr Leu Ser Lys Asn Val Val Asp Asn Trp Glu Lys Val Cys Pro Arg
    290                 295                 300

Lys Asn Leu Gln Asn Ala Lys Phe Gly Leu Trp Val Asp Gly Asn Cys
305                 310                 315                 320

Glu Asp Ile Pro His Val Asn Glu Phe Pro Ala Ile Asp Leu Phe Glu
                325                 330                 335

Cys Asn Lys Leu Val Phe Glu Leu Ser Ala Ser Asp Gln Pro Lys Gln

340                 345                 350

Tyr Glu Gln His Leu Thr Asp Tyr Glu Lys Ile Lys Glu Gly Phe Lys
        355                 360                 365

Asn Lys Asn Ala Ser Met Ile Lys Ser Ala Phe Leu Pro Thr Gly Ala
    370                 375                 380

Phe Lys Ala Asp Arg Tyr Lys Ser His Gly Lys Gly Tyr Asn Trp Gly
385                 390                 395                 400

Asn Tyr Asn Thr Glu Thr Gln Lys Cys Glu Ile Phe Asn Val Lys Pro
                405                 410                 415

Thr Cys Leu Ile Asn Asn Ser Ser Tyr Ile Ala Thr Thr Ala Leu Ser
            420                 425                 430

His Pro Ile Glu Val Glu Asn Asn Phe Pro Cys Ser Leu Tyr Lys Asp
        435                 440                 445

Glu Ile Met Lys Glu Ile Glu Arg Glu Ser Lys Arg Ile Lys Leu Asn
    450                 455                 460

Asp Asn Asp Asp Glu Gly Asn Lys Lys Ile Ile Ala Pro Arg Ile Phe
465                 470                 475                 480

Ile Ser Asp Asp Lys Asp Ser Leu Lys Cys Pro Cys Asp Pro Glu Met
                485                 490                 495

Val Ser Asn Ser Thr Cys Arg Phe Phe Val Cys Lys Cys Val Glu Arg
            500                 505                 510

Arg Ala Glu Val Thr Ser Asn Asn Glu Val Val Val Lys Glu Glu Tyr
        515                 520                 525

Lys Asp Glu Tyr Ala Asp Ile Pro Glu His Lys Pro Thr Tyr Asp Lys
    530                 535                 540

Met Lys Ile Ile Ile Ala Ser Ser Ala Ala Val Ala Val Leu Ala Thr
545                 550                 555                 560

Ile Leu Met Val Tyr Leu Tyr Lys Arg Lys Gly Asn Ala Glu Lys Tyr
                565                 570                 575

Asp Lys Met Asp Glu Pro Gln Asp Tyr Gly Lys Ser Asn Ser Arg Asn
            580                 585                 590

Asp Glu Met Leu Asp Pro Glu Ala Ser Phe Trp Gly Glu Glu Lys Arg
        595                 600                 605

Ala Ser His Thr Thr Pro Val Leu Met Glu Lys Pro Tyr Tyr
    610                 615                 620

<210>  25
<211>  540
<212>  PRT
<213>  Plasmodium vivax

<220>
<221>  misc_feature
<223>  AMA1

<400>  25

Met Asn Lys Ile Tyr Tyr Ile Ile Phe Leu Ser Ala Gln Cys Leu Val
1               5                   10                  15

His Ile Gly Lys Cys Gly Arg Asn Gln Lys Pro Ser Arg Leu Thr Arg
            20                  25                  30

Ser Ala Asn Asn Val Leu Leu Glu Lys Gly Pro Ser Val Glu Arg Ser
        35                  40                  45

Thr Arg Met Ser Asn Pro Trp Lys Ala Phe Met Glu Lys Tyr Asp Ile
    50                  55                  60

Glu Arg Thr His Ser Ser Gly Val Arg Val Asp Leu Gly Glu Asp Ala
65                  70                  75                  80

Glu Val Glu Asn Ala Lys Tyr Arg Ile Pro Ala Gly Arg Cys Pro Val
                85                  90                  95

Phe Gly Lys Gly Ile Val Ile Glu Asn Ser Ala Val Ser Phe Leu Thr
            100                 105                 110

Pro Val Ala Thr Gly Asp Gln Arg Leu Lys Asp Gly Gly Phe Ala Phe
        115                 120                 125

Pro Asn Ala Asn Asp His Ile Ser Pro Met Thr Leu Glu Asn Leu Lys
    130                 135                 140

Ala Arg Tyr Lys Asp Asn Val Glu Met Met Lys Leu Asn Asp Ile Ala
145                 150                 155                 160

Leu Cys Arg Thr His Ala Ala Ser Phe Val Met Ala Gly Asp Gln Asn
                165                 170                 175

Ser Ser Tyr Arg His Pro Ala Val Tyr Asp Glu Lys Glu Lys Thr Cys
            180                 185                 190

His Met Leu Tyr Leu Ser Ala Gln Glu Asn Met Gly Pro Arg Tyr Cys
        195                 200                 205

Ser Pro Asp Ala Gln Asn Arg Asp Ala Val Phe Cys Phe Lys Pro Asp
    210                 215                 220

Lys Asn Glu Ser Phe Glu Asn Leu Val Tyr Leu Ser Lys Asn Val Arg
225                 230                 235                 240

Asn Asp Trp Asp Lys Lys Cys Pro Arg Lys Asn Leu Gly Asn Ala Lys
                245                 250                 255

Phe Gly Leu Trp Val Asp Gly Asn Cys Glu Glu Ile Pro Tyr Val Lys
            260                 265                 270

Glu Val Glu Ala Lys Asp Leu Arg Glu Cys Asn Arg Ile Val Phe Gly
        275                 280                 285

Ala Ser Ala Ser Asp Gln Pro Thr Gln Tyr Glu Glu Glu Met Thr Asp
    290                 295                 300

Tyr Gln Lys Ile Gln Gln Gly Phe Arg Gln Asn Asn Arg Glu Met Ile
305                 310                 315                 320

Lys Ser Ala Phe Leu Pro Val Gly Ala Phe Asn Ser Asp Asn Phe Lys
                325                 330                 335

Ser Lys Gly Arg Gly Phe Asn Trp Ala Asn Phe Asp Ser Val Lys Lys
            340                 345                 350

Lys Cys Tyr Ile Phe Asn Thr Lys Pro Thr Cys Leu Ile Asn Asp Lys
        355                 360                 365

Asn Phe Ile Ala Thr Thr Ala Leu Ser His Pro Lys Glu Val Asp Pro
    370                 375                 380

Glu Phe Pro Cys Ser Ile Tyr Lys Asp Glu Ile Glu Arg Glu Ile Lys
385                 390                 395                 400

Lys Gln Ser Arg Asn Met Asn Leu Tyr Ser Val Asp Gly Glu Arg Ile
                405                 410                 415

Val Leu Pro Arg Ile Phe Ile Ser Asn Asp Lys Glu Ser Ile Lys Cys
            420                 425                 430

Pro Cys Glu Pro Glu Arg Ile Ser Asn Ser Thr Cys Asp Phe Tyr Val
        435                 440                 445

Cys Asn Cys Val Glu Lys Arg Ala Glu Ile Lys Glu Asn Asn Gln Val
    450                 455                 460

Val Ile Lys Glu Glu Phe Arg Asp Tyr Tyr Glu Asn Gly Glu Glu Lys
465                 470                 475                 480

Ser Asn Lys Gln Met Leu Leu Ile Ile Ile Gly Ile Thr Gly Gly Val
                485                 490                 495

Cys Val Val Ala Leu Ala Ser Met Ala Tyr Phe Arg Lys Lys Ala Asn
            500                 505                 510

Asn Asp Lys Tyr Asp Lys Met Asp Gln Ala Glu Gly Tyr Gly Lys Pro
        515                 520                 525

Thr Thr Arg Lys Asp Glu Met Leu Asp Pro Glu Ala
    530                 535                 540

<210>  26
<211>  33
<212>  DNA
<213>  artificial

<220>
<223>  ML567 primer

<400>  26
gatggcacat tatacgcgga gcctgcgggg ttg                                33

<210>  27
<211>  33
<212>  DNA
<213>  artificial

<220>
<223>  ML568 primer

<400>  27
caaccccgca ggctccgcgt ataatgtgcc atc                                33

<210>  28

<211>  40
<212>  DNA
<213>  artificial

<220>
<223>  ML559 primer

<400>  28
ctgatggagg gtaaaaaggc ctgttcagtc aagggcgaac                              40


<210>  29
<211>  40
<212>  DNA
<213>  artificial

<220>
<223>  ML560 primer

<400>  29
gttcgccctt gactgaacag gcctttttac cctccatcag                              40


<210>  30
<211>  35
<212>  DNA
<213>  artificial

<220>
<223>  ML593 primer

<400>  30
gttgatggca cattagcccg ggagcctgcg gggtt                                   35


<210>  31
<211>  35
<212>  DNA
<213>  artificial

<220>
<223>  ML594 primer

<400>  31
aaccccgcag gctcccgggc taatgtgcca tcaac                                   35


<210>  32
<211>  40
<212>  DNA
<213>  artificial

<220>
<223>  ML557 primer

<400>  32
caataaggcg acgaaggccc gttacccatt tgtttatgac                              40


<210>  33
<211>  40

<212>  DNA
<213>  artificial

<220>
<223>  ML558 primer

<400>  33
gtcataaaca aatgggtaac gggccttcgt cgccttattg                              40


<210>  34
<211>  33
<212>  DNA
<213>  artificial

<220>
<223>  ML533 primer

<400>  34
gcgacgaagt accgtgcccc atttgtttat gac                                     33


<210>  35
<211>  33
<212>  DNA
<213>  artificial

<220>
<223>  ML534 primer

<400>  35
gtcataaaca aatggggcac ggtacttcgt cgc                                     33


<210>  36
<211>  33
<212>  DNA
<213>  artificial

<220>
<223>  ML591 primer

<400>  36
gaggcttcaa cttgaatgcc gtgacgccta gcg                                     33


<210>  37
<211>  33
<212>  DNA
<213>  artificial

<220>
<223>  ML592 primer

<400>  37
cgctaggcgt cacggcattc aagttgaagc ctc                                     33


<210>  38
<211>  33
<212>  DNA

<213>  artificial

<220>
<223>  ML563 primer

<400>  38
cctagcgggc agcgagcttc accatttccg atg                                    33


<210>  39
<211>  33
<212>  DNA
<213>  artificial

<220>
<223>  ML564 primer

<400>  39
catcggaaat ggtgaagctc gctgcccgct agg                                    33


<210>  40
<211>  33
<212>  DNA
<213>  artificial

<220>
<223>  ML561 primer

<400>  40
ggaggtgcgc cgaggctgcc tttaagacgg tcg                                    33


<210>  41
<211>  33
<212>  DNA
<213>  artificial

<220>
<223>  ML562 primer

<400>  41
cgaccgtctt aaaggcagcc tcggcgcacc tcc                                    33


<210>  42
<211>  37
<212>  DNA
<213>  artificial

<220>
<223>  ML589 primer

<400>  42
cctttaagac ggtcgctgcg gataaaaaca ataaggc                                37


<210>  43
<211>  37
<212>  DNA
<213>  artificial

<220>
<223>  ML590 primer

<400>  43
gccttattgt ttttatccgc agcgaccgtc ttaaagg                              37


<210>  44
<211>  42
<212>  DNA
<213>  artificial

<220>
<223>  ML565 primer

<400>  44
cttggaagat gttccgactg caaaagaata caaacagtca gg                        42


<210>  45
<211>  42
<212>  DNA
<213>  artificial

<220>
<223>  ML566 primer

<400>  45
cctgactgtt tgtattcttt tgcagtcgga acatcttcca ag                        42


<210>  46
<211>  33
<212>  DNA
<213>  artificial

<220>
<223>  ML457 primer

<400>  46
ctgtgccaca tcctcgccgt atcgatgcag ctg                                  33


<210>  47
<211>  33
<212>  DNA
<213>  artificial

<220>
<223>  ML458 primer

<400>  47
cagctgcatc gatacggcga ggatgtggca cag                                  33


<210>  48
<211>  42
<212>  DNA
<213>  artificial

<220>
<223>  ML525 primer

<400>  48
gttccgcgtg gatccgttgc accggtctcc tgcgtgacga ac                          42


<210>  49
<211>  42
<212>  DNA
<213>  artificial

<220>
<223>  ML526 primer

<400>  49
gttcgtcacg caggagaccg gtgcaacgga tccacgcgga ac                          42


<210>  50
<211>  42
<212>  DNA
<213>  artificial

<220>
<223>  ML531 primer

<400>  50
caggccatcg ccaaggccac cttcgcaggc atggcgccgt ac                          42


<210>  51
<211>  42
<212>  DNA
<213>  artificial

<220>
<223>  ML532 primer

<400>  51
gtacggcgcc atgcctgcga aggtggcctt ggcgatggcc tg                          42


<210>  52
<211>  33
<212>  DNA
<213>  artificial

<220>
<223>  ML459 primer

<400>  52
gcgccgccgg tctccgccgt gacgaacgag atc                                    33


<210>  53
<211>  33
<212>  DNA
<213>  artificial

<220>

<223>  ML460 primer

<400>  53
gatctcgttc gtcacggcgg agaccggcgg cgc                                    33


<210>  54
<211>  33
<212>  DNA
<213>  artificial

<220>
<223>  ML498 primer

<400>  54
atcctcggag tcaccgccgc gccgcaggcc atc                                    33


<210>  55
<211>  33
<212>  DNA
<213>  artificial

<220>
<223>  ML543 primer

<400>  55
gatggcctgc ggcgcggcgg tgactccgag gat                                    33


<210>  56
<211>  33
<212>  DNA
<213>  artificial

<220>
<223>  ML569 primer

<400>  56
cgtggatccg ttgcagccat cgtgcaacat atg                                    33


<210>  57
<211>  33
<212>  DNA
<213>  artificial

<220>
<223>  ML570 primer

<400>  57
catatgttgc acgatggctg caacggatcc acg                                    33


<210>  58
<211>  33
<212>  DNA
<213>  artificial

<220>
<223>  ML571 primer

<400>  58
gcagacatcg tgcaagctat ggaagacatt ggc                                    33


<210>  59
<211>  33
<212>  DNA
<213>  artificial

<220>
<223>  ML572 primer

<400>  59
gccaatgtct tccatagctt gcacgatgtc tgc                                    33


<210>  60
<211>  33
<212>  DNA
<213>  artificial

<220>
<223>  ML463 primer

<400>  60
gtgcaacata tggaagccat tggcggcgcg ccg                                    33


<210>  61
<211>  33
<212>  DNA
<213>  artificial

<220>
<223>  ML464 primer

<400>  61
cggcgcgccg ccaatggctt ccatatgttg cac                                    33


<210>  62
<211>  33
<212>  DNA
<213>  artificial

<220>
<223>  ML573 primer

<400>  62
caacatatgg aagacgctgg cggcgcgccg ccg                                    33


<210>  63
<211>  33
<212>  DNA
<213>  artificial

<220>
<223>  ML574 primer

<400>  63
cggcggcgcg ccgccagcgt cttccatatg ttg                                    33


<210>  64
<211>  33
<212>  DNA
<213>  artificial

<220>
<223>  ML461 primer

<400>  64
gacattggcg gcgcggcgcc ggtctcctgc gtg                                    33


<210>  65
<211>  33
<212>  DNA
<213>  artificial

<220>
<223>  ML462 primer

<400>  65
cacgcaggag accggcgccg cgccgccaat gtc                                    33


<210>  66
<211>  33
<212>  DNA
<213>  artificial

<220>
<223>  ML575 primer

<400>  66
tgcgtgacga acgaggccct cggagtcacc tgc                                    33


<210>  67
<211>  33
<212>  DNA
<213>  artificial

<220>
<223>  ML576 primer

<400>  67
gcaggtgact ccgagggcct cgttcgtcac gca                                    33


<210>  68
<211>  33
<212>  DNA
<213>  artificial

<220>
<223>  ML577 primer

<400>  68

gtgacgaacg agatcgccgg agtcacctgc gcg                                    33

<210>  69
<211>  33
<212>  DNA
<213>  artificial

<220>
<223>  ML578 primer

<400>  69
cgcgcaggtg actccggcga tctcgttcgt cac                                    33

<210>  70
<211>  33
<212>  DNA
<213>  artificial

<220>
<223>  ML579 primer

<400>  70
gccatcgcca aggccgccac gagcgccgcg aga                                    33

<210>  71
<211>  33
<212>  DNA
<213>  artificial

<220>
<223>  ML580 primer

<400>  71
tctcgcggcg ctcgtggcgg ccttggcgat ggc                                    33

<210>  72
<211>  37
<212>  PRT
<213>  Artificial

<220>
<223>  RON2-2 domain

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(9)

<223>  Xaa can be any naturally occurring amino acid

<220>
<221>  misc_feature
<222>  (11)..(12)
<223>  Xaa can be any naturally occurring amino acid

<220>
<221>  misc_feature
<222>  (14)..(16)
<223>  Xaa can be any naturally occurring amino acid

<220>
<221>  misc_feature
<222>  (18)..(26)
<223>  Xaa can be any naturally occurring amino acid

<220>
<221>  misc_feature
<222>  (28)..(37)
<223>  Xaa can be any naturally occurring amino acid

<400>  72

Asp Ile Xaa Gln Xaa Xaa Xaa Asp Xaa Gly Xaa Xaa Pro Xaa Xaa Xaa
1               5                   10                  15


Cys Xaa Xaa Xaa Xaa Xaa Xaa Xaa Xaa Xaa Cys Xaa Xaa Xaa Xaa Xaa
            20                  25                  30


Xaa Xaa Xaa Xaa Xaa
        35



<210>  73
<211>  38
<212>  PRT
<213>  Artificial

<220>
<223>  RON2-2 domain



<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(9)
<223>  Xaa can be any naturally occurring amino acid

<220>

<221>  misc_feature
<222>  (11)..(12)
<223>  Xaa can be any naturally occurring amino acid

<220>
<221>  misc_feature
<222>  (14)..(16)
<223>  Xaa can be any naturally occurring amino acid

<220>
<221>  misc_feature
<222>  (18)..(27)
<223>  Xaa can be any naturally occurring amino acid

<220>
<221>  misc_feature
<222>  (29)..(38)
<223>  Xaa can be any naturally occurring amino acid

<400>  73

Asp Ile Xaa Gln Xaa Xaa Xaa Asp Xaa Gly Xaa Xaa Pro Xaa Xaa Xaa
1               5                   10                  15

Cys Xaa Xaa Xaa Xaa Xaa Xaa Xaa Xaa Xaa Xaa Cys Xaa Xaa Xaa Xaa
            20                  25                  30

Xaa Xaa Xaa Xaa Xaa Xaa
        35

<210>  74
<211>  39
<212>  PRT
<213>  Artificial

<220>
<223>  RON2-2 domain

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (11)..(12)
<223>  Xaa can be any naturally occurring amino acid

<220>
<221>  misc_feature
<222>  (14)..(16)
<223>  Xaa can be any naturally occurring amino acid

<220>
<221>  misc_feature
<222>  (18)..(28)
<223>  Xaa can be any naturally occurring amino acid

<220>
<221>  misc_feature
<222>  (30)..(39)
<223>  Xaa can be any naturally occurring amino acid

<400>  74

Asp Ile Xaa Gln Xaa Xaa Xaa Asp Xaa Gly Xaa Xaa Pro Xaa Xaa Xaa
1               5                   10                  15

Cys Xaa Xaa Xaa Xaa Xaa Xaa Xaa Xaa Xaa Xaa Xaa Cys Xaa Xaa Xaa
            20                  25                  30

Xaa Xaa Xaa Xaa Xaa Xaa Xaa
        35

<210>  75
<211>  431
<212>  PRT
<213>  Artificial

<220>
<223>  AMA1 fragment

<400>  75

Gly Ser Ala Met Gly Ser Ala Ser Thr Ser Gly Asn Pro Phe Gln Ala
1               5                   10                  15

Asn Val Glu Met Lys Thr Phe Met Glu Arg Phe Asn Leu Thr His His
            20                  25                  30

His Gln Ser Gly Ile Tyr Val Asp Leu Gly Gln Asp Lys Glu Val Asp
        35                  40                  45

Gly Thr Leu Tyr Arg Glu Pro Ala Gly Leu Cys Pro Ile Trp Gly Lys
    50                  55                  60

His Ile Glu Leu Gln Gln Pro Asp Arg Pro Pro Tyr Arg Asn Asn Phe
65                  70                  75                  80

Leu Glu Asp Val Pro Thr Glu Lys Glu Tyr Lys Gln Ser Gly Asn Pro

85                  90                  95

Leu Pro Gly Gly Phe Asn Leu Asn Phe Val Thr Pro Ser Gly Gln Arg
            100                 105                 110

Ile Ser Pro Phe Pro Met Glu Leu Leu Glu Lys Asn Ser Asn Ile Lys
        115                 120                 125

Ala Ser Thr Asp Leu Gly Arg Cys Ala Glu Phe Ala Phe Lys Thr Val
    130                 135                 140

Ala Met Asp Lys Asn Asn Lys Ala Thr Lys Tyr Arg Tyr Pro Phe Val
145                 150                 155                 160

Tyr Asp Ser Lys Lys Arg Leu Cys His Ile Leu Tyr Val Ser Met Gln
                165                 170                 175

Leu Met Glu Gly Lys Lys Tyr Cys Ser Val Lys Gly Glu Pro Pro Asp
            180                 185                 190

Leu Thr Trp Tyr Cys Phe Lys Pro Arg Lys Ser Val Thr Glu Asn His
        195                 200                 205

His Leu Ile Tyr Gly Ser Ala Tyr Val Gly Glu Asn Pro Asp Ala Phe
    210                 215                 220

Ile Ser Lys Cys Pro Asn Gln Ala Leu Arg Gly Tyr Arg Phe Gly Val
225                 230                 235                 240

Trp Lys Lys Gly Arg Cys Leu Asp Tyr Thr Glu Leu Thr Asp Thr Val
                245                 250                 255

Ile Glu Arg Val Glu Ser Lys Ala Gln Cys Trp Val Lys Thr Phe Glu
            260                 265                 270

Asn Asp Gly Val Ala Ser Asp Gln Pro His Thr Tyr Pro Leu Thr Ser
        275                 280                 285

Gln Ala Ser Trp Asn Asp Trp Trp Pro Leu His Gln Ser Asp Gln Pro
    290                 295                 300

His Ser Gly Gly Val Gly Arg Asn Tyr Gly Phe Tyr Tyr Val Asp Thr
305                 310                 315                 320

Thr Gly Glu Gly Lys Cys Ala Leu Ser Asp Gln Val Pro Asp Cys Leu
                325                 330                 335

Val Ser Asp Ser Ala Ala Val Ser Tyr Thr Ala Ala Gly Ser Leu Ser
            340                 345                 350

Glu Glu Thr Pro Asn Phe Ile Ile Pro Ser Asn Pro Ser Val Thr Pro
        355                 360                 365

Pro Thr Pro Glu Thr Ala Leu Gln Cys Thr Ala Asp Lys Phe Pro Asp
    370                 375                 380

Ser Phe Gly Ala Cys Asp Val Gln Ala Cys Lys Arg Gln Lys Thr Ser
385                 390                 395                 400

Cys Val Gly Gly Gln Ile Gln Ser Thr Ser Val Cys Thr Ala Asp Glu
                405                 410                 415

Gln Asn Glu Cys Gly Ser Asn Thr Ala Ala Ala Leu Val Pro Arg
            420                 425                 430